(19)

Europäisches
Patentamt
European
Patent Office
Office européen
des brevets

(11) **EP 4 741 515 A2**

(12) **EUROPEAN PATENT APPLICATION**

(43) Date of publication:
**13.05.2026 Bulletin 2026/20**

(21) Application number: **26151907.8**

(22) Date of filing: **18.02.2019**

(51) International Patent Classification (IPC):
***C12Q 1/6881*** (2018.01)

(52) Cooperative Patent Classification (CPC):
**C12Q 1/6881;** C12Q 2600/154; G16B 20/00

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.02.2018 US 201862631791 P
23.04.2018 US 201862661179 P**

(62) Document number(s) of the earlier application(s) in
accordance with Art. 76 EPC:
**19712298.9 / 3 752 641**

(71) Applicants:
• **Yissum Research Development Company of the
Hebrew University of Jerusalem Ltd.**
**91390 Jerusalem (IL)**
• **Hadasit Medical Research Services
and Development Ltd.**
**9112001 Jerusalem (IL)**

(72) Inventors:
• **DOR, Yuval**
**9774415 Jerusalem (IL)**

• **SHEMER, Ruth**
**9078910 Mevasseret Zion (IL)**
• **GLASER, Benjamin**
**9692852 Jerusalem (IL)**
• **KAPLAN, Tomer**
**9626902 Jerusalem (IL)**
• **MOSS, Joshua**
**9319004 Jerusalem (IL)**

(74) Representative: **Murgitroyd & Company**
**165-169 Scotland Street
Glasgow G5 8PL (GB)**

Remarks:
•This application was filed on 14-01-2026 as a
divisional application to the application mentioned
under INID code 62.
•Claims filed after the date of filing of the application/
after the date of receipt of the divisional application
(Rule 68(4) EPC).

(54) **CELL FREE DNA DECONVOLUTION AND USE THEREOF**

(57)    Methods of determining the origin of cell free
DNA (cfDNA) and for detecting death of a cell type or
tissue in a subject by determining the origin of cfDNA in
the subject are provided. Computer program products,
computerized methods and computerized systems for
doing same, as well as methods for constructing a methy-
lome atlas, are also provided.

EP 4 741 515 A2

(Cont. next page)

**Tissues**

Figure 1C

Figure 1C Continued

Figure 1D

Figure 1E

4

**Description**

**CROSS REFERENCE TO RELATED APPLICATIONS**

This application claims the benefit of priority of U.S. Provisional Patent Application No. 62/631,791, filed February 18, 2018, and U.S. Provisional Patent Application No. 62/661,179, filed April 23, 2018, the contents of which are all incorporated herein by reference in their entirety.

**FIELD OF INVENTION**

The present invention is in the field of cell free DNA methylome analysis.

**BACKGROUND OF THE INVENTION**

Small fragments of DNA circulate freely in the peripheral blood of healthy and diseased individuals. These cell-free DNA (cfDNA) molecules are thought to derive from dying cells and are thus a reflection of ongoing cell death taking place in the body. In recent years, this understanding has led to the emergence of diagnostic tools which are impacting multiple areas of medicine. Specifically, next generation sequencing of fetal DNA circulating in maternal blood has allowed non-invasive prenatal testing of fetal chromosomal abnormalities; detection of donor-derived DNA in the circulation of organ transplant recipients can be used for early identification of graft rejection; and the evaluation of mutated DNA in the circulation can be used to detect, genotype and monitor cancer. These technologies are powerful at identifying genetic anomalies in circulating DNA yet are not informative when cfDNA does not carry mutations. A key limitation is that sequencing does not reveal the tissue origins of cfDNA, precluding the identification of tissue-specific cell death. The latter is critical in many settings such as neurodegenerative, inflammatory or ischemic diseases, not involving DNA mutations. Even in oncology, it is often important to determine the tissue origins of the tumor in addition to determining its mutational profile, for example in cancers of unknown primary (CUP) and in the setting of early cancer diagnosis. Finally, identification of collateral tissue damage (e.g. toxicity of drugs in genetically normal tissues) is a key element in drug development and monitoring of response to therapy.

Several approaches have been proposed recently for tracing the tissue sources of cfDNA, all based on tissue-specific epigenetic signatures. Nucleosome positioning in different tissues has been used to infer the origins of cfDNA, based on the idea that nucleosome-free regions are more likely to be degraded upon cell death and hence will be under-represented in cfDNA. An alternative approach is based on DNA methylation patterns. Methylation of cytosine adjacent to guanine (CpG sites) is an essential component of cell type-specific gene regulation, and hence is a fundamental mark of cell identity. It was recently shown that the detection of cfDNA molecules with a methylation pattern unique to a certain tissue can be used to identify cell death derived from that tissue. Others have taken a genome-wide approach to the problem and used the plasma methylome to assess the origins of cfDNA. It has been showed that the deconvolution of cfDNA methylation profiles can be used to infer relative contributions of four different tissues, using a low-depth whole genome bisulfite sequencing (WGBS). It was further demonstrated that the potential exists of using cfDNA methylation in detecting cancer DNA in circulation, as well as identifying its tissue of origin in two cancer types, using a reduced representation bisulfite sequencing (RRBS) approach. Nevertheless, a method of reliably determining the tissue of origin of all cfDNA found in a subject, and not just the most abundant is greatly in need. Such a method would allow for early diagnosis of conditions that may be unknown to the physician or patient, and not just a directed assessment of an already predicted condition.

**SUMMARY OF THE INVENTION**

The present invention provides methods of determining the origin of cell free DNA (cfDNA) and for detecting death of a cell type or tissue in a subject by determining the origin of cfDNA in the subject are provided. Computer program products for doing same and methods of constructing a methylome atlas are also provided.

According to a first aspect, there is provided a method of determining the cell type or tissue of origin of cell free DNA (cfDNA) comprising:

a. providing cfDNA;
b. measuring DNA methylation of the cfDNA; and
c. assigning a cfDNA molecule from the cfDNA to a cell type or tissue of origin by comparing the methylation of the molecule to a methylome atlas of at least I cell type or tissue, wherein the atlas comprises at least 25 of the 100 most uniquely methylated sites and at least 25 of the 100 most uniquely unmethylated sites in each of the at least 1 cell type or tissue;

thereby determining the cell or tissue of origin of cfDNA.

According to another aspect, there is provided a method of detecting death of a cell type or tissue in a subject comprising:

a. providing cfDNA from the subject;
b. measuring DNA methylation of the cfDNA; and
c. assigning a cfDNA molecule from the cfDNA to a cell type or tissue of origin by comparing the methylation of the molecule to a methylome atlas of at least 1 cell type or tissue, wherein the atlas comprises at least 25 of the 100 most uniquely methylated sites and at least 25 of the 100 most uniquely unmethylated sites in each of the at least l cell type or tissue;

thereby detecting death of a cell type or tissue in a subject.

According to some embodiments, at least 50 ng of cfDNA are provided. According to some embodiments, the providing comprises providing a bodily fluid and isolating the cfDNA from the bodily fluid.

According to some embodiments, the measuring DNA methylation comprises bisulfite conversion of the cfDNA. According to some embodiments, the measuring further comprises performing a methylome array or chip on the bisulfite converted cfDNA.

According to some embodiments, the methylome atlas comprises only data from purified cell types. According to some embodiments, the atlas comprises only data from non-blood derived purified cell types. According to some embodiments, the methylome atlas comprises methylation data from at least 5 of the following 34 tissues or cell types: monocytes, B-cells, CD4+ T-cells, NK-cells, CD8+ T-cells, eosinophils, neutrophils, erythrocyte progenitors, adipocytes, neurons, hepatocytes, lung alveolar cells, pancreatic beta cells, pancreatic acinar cells, pancreatic duct cells, vascular endothelial cells, left atrium, bladder, breast, cervix, colon, esophagus, oral cavity, kidney, prostate, rectum, stomach, thyroid, uterus, lung bronchial cells, cholangiocytes, muscle, oligodendrocytes, and ovary. According to some embodiments, the methylome atlas comprises data from all of the 34 tissues or cell types.

According to some embodiments, the methylome atlas comprises at least the 100 most uniquely methylated or unmethylated sites in each tissue or cell type. According to some embodiments, the methylome atlas further comprises any CpG sites within at least 150 base pairs upstream and downstream of the most uniquely methylated and most uniquely unmethylated sites in each tissue or cell type. According to some embodiments, the CpG sites within at least 150 base pairs upstream and downstream are selected from Tables 1 and 2. According to some embodiments, the methylome atlas further comprises at least one of the 500 CpG sites that best differentiate between the most similar pairs of tissues and cell types. According to some embodiments, the 500 CpG sites that best differentiate between the most similar pairs of tissues and cell types are selected from Table 3. According to some embodiments, the most uniquely methylated sites are selected from Table 1. According to some embodiments, the most uniquely hypomethylated sites are selected from Table 2.

According to some embodiments, cfDNA of the tissue or cell type comprises as little 1% of all of the cfDNA.

According to some embodiments, the methods of the invention are for use in detecting a disease state in a subject in need thereof and wherein the cfDNA is from the subject. According to some embodiments, the disease state is selected from organ transplantation, sepsis, and cancer. According to some embodiments, the disease is cancer, and the method determines the cell or tissue of origin of the cancer.

According to another aspect, there is provided a computer program product for determining the cell or tissue of origin of cell free DNA (cfDNA), comprising a non-transitory computer-readable storage medium having program code embodied thereon, the program code executable by at least one hardware processor to

a. measure or access measurements of DNA methylation of cfDNA;
b. assign a cfDNA molecule from the cfDNA to a cell type or tissue of origin by comparing the methylation of the molecule to a methylome atlas of at least 5 cell types or tissues, wherein the atlas comprises at least 25 of the 100 most uniquely methylated sites and at least 25 of the 100 most uniquely unmethylated sites in each of the 5 cell types or tissues; and
c. provide an output regarding the cell or tissue of origin of cfDNA.

According to another aspect, there is provided a method of constructing a methylome atlas, comprising:

a. providing DNA methylation data from at least 5 tissues and/or cell types;
b. selecting at least the top 100 uniquely hyper-methylated and at least the top 100 uniquely hypomethylated CpG sites per a tissue and/or cell type as compared to the other tissues and/or cell types; and
c. combining the uniquely hyper-methylated and uniquely hypo-methylated sites for all the tissues and/or cell types;

thereby constructing a methylome atlas.

According to some embodiments, the methods of the invention further comprise:

d. selecting all neighboring CpG sites within at least 150 base pairs upstream and/or downstream of each of the uniquely hyper-methylated and uniquely hypomethylated CpG site; and

e. combining the neighboring CpG sites with the uniquely hyper-methylated and uniquely hypomethylated CpG sites.

According to some embodiments, the methods of the invention further comprise:

f. selecting at least the top 100 CpG sites that best differentiate between the most similar pair of tissues and/or cell types; and

g. combining the CpG sites that best differentiate with the uniquely hyper-methylated and uniquely hypomethylated CpG sites.

According to another aspect, there is provided a computerized method of determining the cell type or tissue of origin of cell free DNA (cfDNA) comprising:
using at least one hardware processor to:

a. receive a methylation sequencing data acquired from a cfDNA sample and a methylome atlas, wherein the methylome atlas comprises (i) a first set of uniquely methylated sites, (ii) a plurality of neighboring methylated sites, wherein at least some of the plurality of neighboring methylated sites are within 500 base units to each element of the first set of uniquely methylated sites, and (iii) a second set of uniquely methylated sites comprising more than double the number of sites of the first set, and comprising less than half the number of tissue types in each comparison as the first-set;

b. compare the methylation sequencing data with a methylome atlas to determine a set of candidate tissues, wherein the comparing comprises identifying at least some elements of the first set of uniquely methylated sites in both the methylation sequencing data and the methylome atlas;

c. compare the plurality of neighboring methylated sites of the methylation sequencing data with the atlas entries of the set of candidate tissues to determine a first subset of candidate tissues;

d. compare the second set of uniquely methylated sites of the methylome atlas within the methylation sequencing data to determine a probability ranking of candidate tissues;

e. assign at least one of the highest-ranking tissues of the probability ranking of candidate tissues to the cfDNA sample.

According to another aspect, there is provided a computer program product for determining the cell or tissue of origin of cell free DNA (cfDNA), comprising a non-transitory computer-readable storage medium having program code embodied thereon, the program code executable by at least one hardware processor to:

a. receive a methylation sequencing data acquired from a cfDNA sample and a methylome atlas, wherein the methylome atlas comprises (i) a first set of uniquely methylated sites, (ii) a plurality of neighboring methylated sites, wherein at least some of the plurality of neighboring methylated sites are within 500 base units to each element of the first set of uniquely methylated sites, and (iii) a second set of uniquely methylated sites comprising more than double the number of sites of the first set, and comprising less than half the number of tissue types in each comparison as the first set;

b. compare the methylation sequencing data with a methylome atlas to determine a set of candidate tissues, wherein the comparing comprises identifying at least some elements of the first set of uniquely methylated sites in both the methylation sequencing data and the methylome atlas;

c. compare the plurality of neighboring methylated sites of the methylation sequencing data with the atlas entries of the set of candidate tissues to determine a first subset of candidate tissues;

d. compare the second set of uniquely methylated sites of the methylome atlas within the methylation sequencing data to determine a probability ranking of candidate tissues;

e. assign at least one of the highest-ranking tissues of the probability ranking of candidate tissues to the cfDNA sample.

According to another aspect, there is provided a computerized system for determining the cell or tissue of origin of cell free DNA (cfDNA), comprising: at least one hardware processor; and a non-transitory computer-readable storage medium having program code embodied thereon. The program code executable by the at least one hardware processor to:

a. receive a methylation sequencing data acquired from a cfDNA sample and a methylome atlas, wherein the methylome atlas comprises (i) a first set of uniquely methylated sites, (ii) a plurality of neighboring methylated sites, and (iii) a second set of uniquely methylated sites comprising more than double the number of sites of the first set. The second set of uniquely methylated sites comprise at less than half the number of tissue types in each comparison as the first set of the first set. At least some of the plurality of neighboring methylated sites are within 500 base units to each element of the first set of uniquely methylated sites,

b. compare the methylation sequencing data with a methylome atlas to determine a set of candidate tissues, wherein the comparing comprises identifying at least some elements of the first set of uniquely methylated sites in both the methylation sequencing data and the methylome atlas.

c. compare the plurality of neighboring methylated sites of the methylation sequencing data with the atlas entries of the set of candidate tissues to determine a first subset of candidate tissues;

d. compare the second set of uniquely methylated sites of the methylome atlas within the methylation sequencing data to determine a probability ranking of candidate tissues;

e. assign at least one of the highest-ranking tissues of the probability ranking of candidate tissues to the cfDNA sample.

According to some embodiments, the first set comprises between 25 and 100 most uniquely methylated sites.
According to some embodiments, the first set comprises a plurality of most uniquely methylated sites and wherein the plurality of neighboring methylated sites comprises any CpG sites within between 150 and 500 base pairs upstream and downstream of said most uniquely methylated sites in each tissue or cell type.
According to some embodiments, the second set comprises between 100 and 500 most uniquely methylated sites.
According to some embodiments, the second set of uniquely methylated sites compares a plurality of specific pairs or triplets of tissue types, such as similar tissue types, or the like.
According to some embodiments, the first set of uniquely methylated sites are uniquely methylated as compared to all cell types and tissues of the atlas, and wherein the second set of uniquely methylated sites are uniquely methylated in one cell type or tissue as compared to a second most similar cell type or tissue.
According to some embodiments, the comparing comprises using a latent probabilistic model.
Further embodiments and the full scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

## BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description together with the drawings makes apparent to those skilled in the art how embodiments of the invention may be practiced.

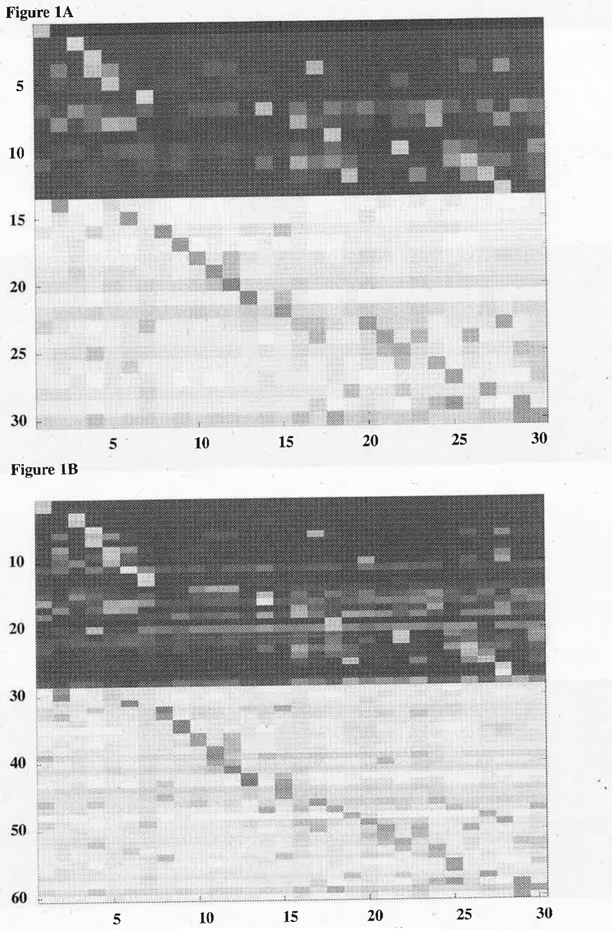

**Figures 1A-E: Feature selection results using 30 Illumina Infinium 450K/EPIC arrays. (1A)** Heat map of 30 CpGs (rows) selected using Compressed Sensing algorithm. **(1B)** Heat map of 60 CpGs selected using two consecutive rounds of Compressed Sensing. **(1C)** Heat map of 1000 CpGs selected by first using Compressed Sensing (same as 1A, 1B), followed by 940 iterations of feature selection using error-correcting code. **(1D)** A line graph, with shaded area showing the improvement in the minimal distance between two tissues, for 1000 iterations of the algorithm. The red line shows the minimal tissue distance for the variance-based algorithm. The horizontal yellow line shows the minimal tissue distance obtained by selecting the 30 most specific CpGs per tissue (900 CpGs overall). **(1E)** A bar graph showing repeating the feature selection algorithms 100 times (each time removing previously selected CpGs), which demonstrates the efficiency of the algorithm as well as the limited number of informative CpG sites.

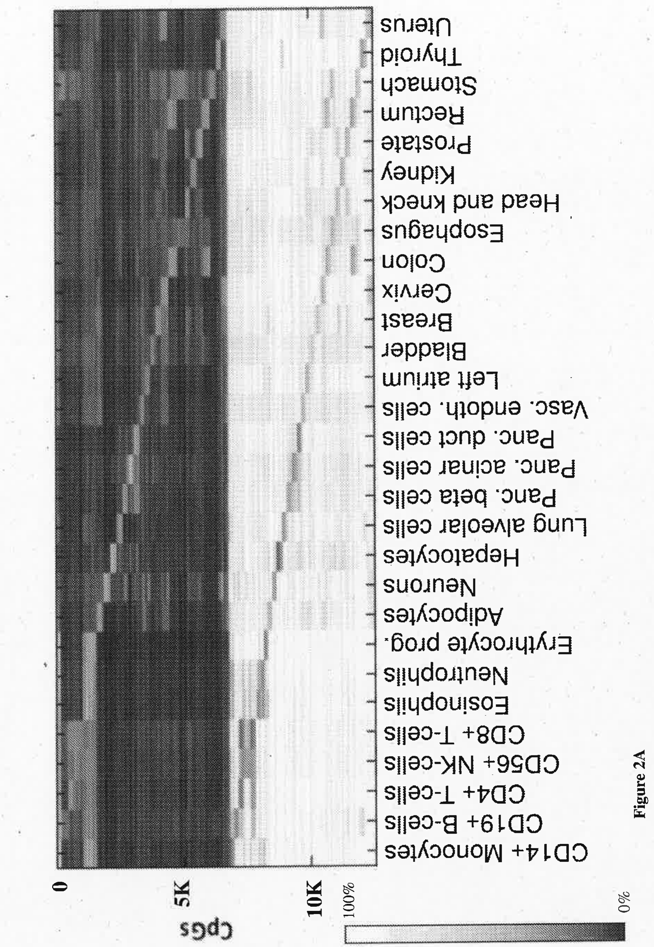

**Figures 2A-C: Comprehensive reference methylation matrix. (2A)** A heatmap showing the 100 most uniquely hyper-methylated and 100 hypomethylated sites for each cell type. Selection of CpGs is described in the Materials and Methods section. **(2B)** Bar chart estimations of specificity (false positive rate) and sensitivity (detection rate) calculated for various CpG selection strategies. **(2C)** A cartoon schematic of a method of the invention.

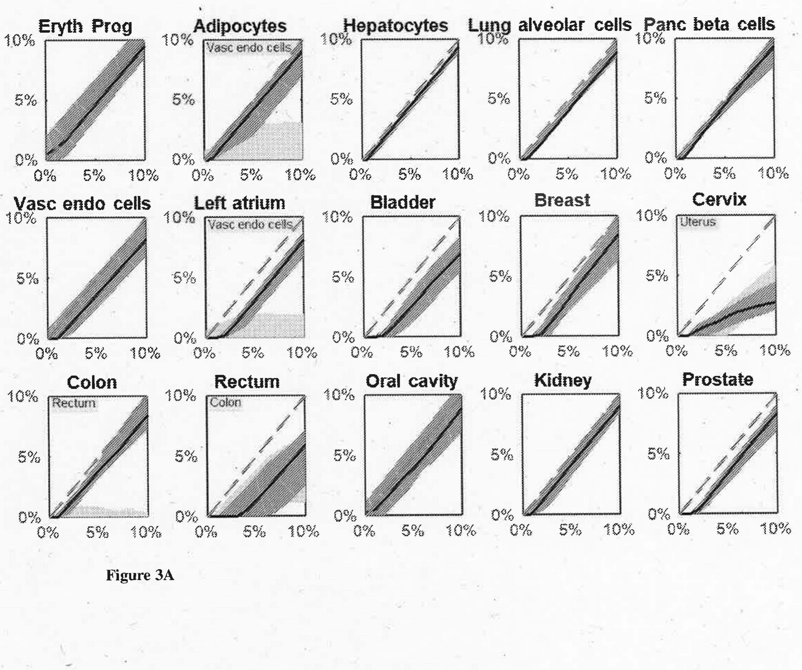

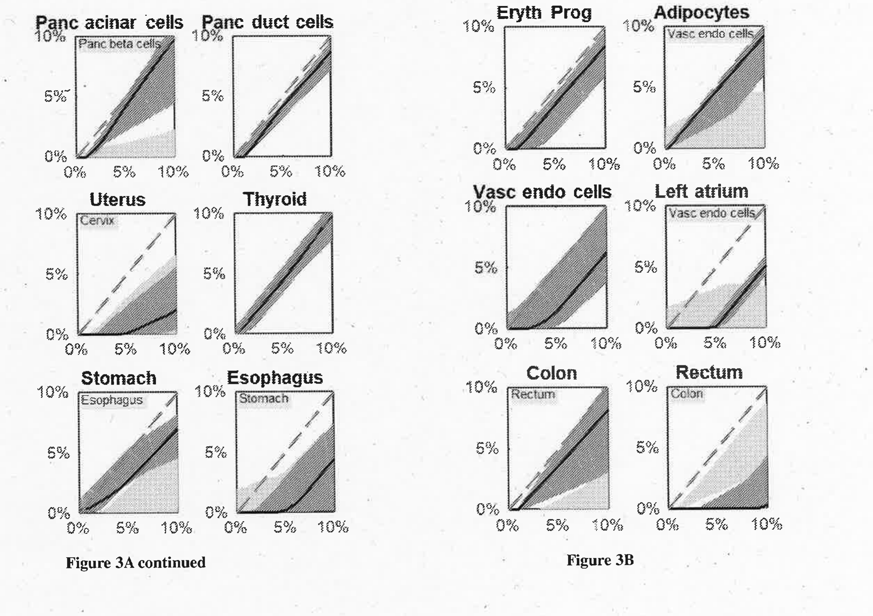

**Figures 3A-E: Deconvolution of simulated mixed samples. (3A-B)** Line graphs showing the actual and predicted contribution of methylomes of indicated cell types, whole tissues and cell cultures after mixing in silico with the whole blood methylome, and deconvolution of the resulting mix **(3A)** with and **(3B)** without feature selection. Solid black lines represent the median predicted percent contributed by each cell type for each actual contribution. The blue shaded area represents the 25%-75% confidence interval. **(3C-D)** Line graph comparing the performance of a reference matrix containing either purified cell types, cultured cells or whole tissue methylomes. For each cell type indicated, the solid green line represents the predicted percent contributed by this cell type at the relevant actual contribution, using the comprehensive reference matrix. **(3C)** The blue lines represent the predicted contribution of these cells, when pancreatic cells in the reference matrix are replaced by whole pancreas methylome **(3D)** The blue/red solid lines represent the predicted contribution of these cells, when hepatocytes in the reference matrix are replaced with whole liver or HepG2 culture cells methylome respectively. **(3E)** A line graph as in 3C but using a reference atlas composed of an average healthy cfDNA methylome and three pancreas cell types (acinar, beta, and duct cells).

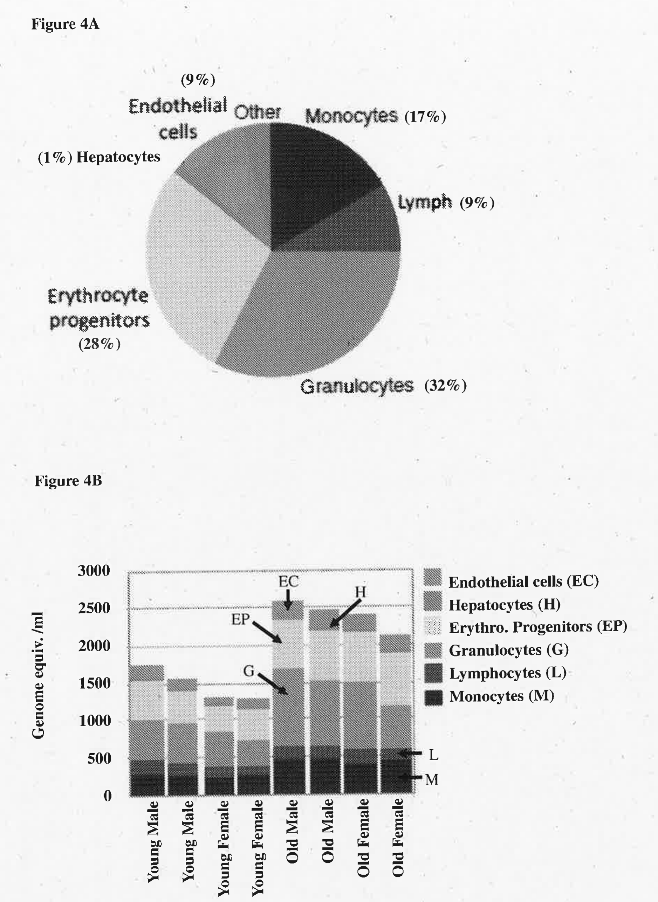

**Figures 4A-C: Cellular contributors to cfDNA in healthy individuals. (4A)** A pie chart of the average predicted distributions of contributors to cfDNA, across 8 pooled samples. **(4B)** A bar chart showing results of deconvolution of 8 pooled DNA samples in absolute levels of DNA (genome equivalents/ml, derived by multiplying the fraction contribution of each tissue by the total concentration of cfDNA). Cell types which contributed less than 1.5% were included in "Other". Young <30 years old, old >75 years old. **(4C)** Spread plots showing comparisons of proportions predicted by deconvolution of cfDNA or leukocyte methylome for erythrocyte progenitors, vascular endothelial cells and hepatocytes. In all cases, the contribution predicted for cfDNA was higher than for leukocytes (p<0.05). Also shown, for control, are spread plots from lymphocytes and granulocytes (which are the prevalent cells that make up leukocytes, as expected).

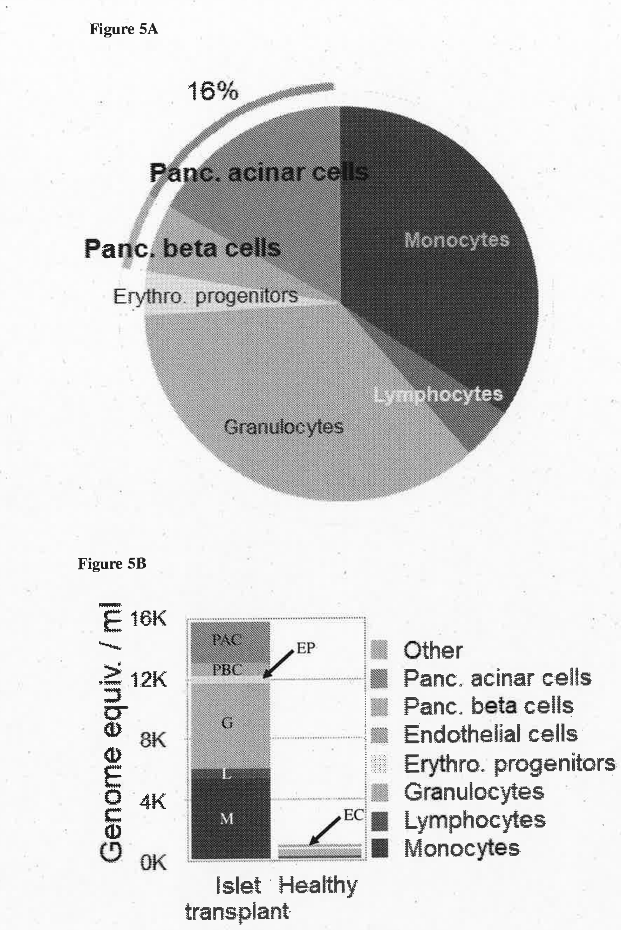

**Figures 5A-E: Cellular contributors to cfDNA in pancreatic islet transplantation.** (**5A**) Pie chart showing result of deconvolution of pooled sample of cfDNA from 5 patients 1 hour after islet transplantation. Cell types which contributed less than 1% were included in "Other". **(5B)** A bar graphs showing the result of deconvolution of pooled post-transplant sample and an average of healthy pools, shown in absolute levels of DNA (genome equivalents/ml). **(5C)** A line graph of the amount of pancreatic cfDNA predicted for three individuals before, 1 hour after and 2 hours after transplantation. **(5D)** A line graph showing a comparison of pancreatic cfDNA measurements using methylome deconvolution vs targeted methylation assay. Results are highly similar (r=0.988, p-value=8.5e-08). **(5E)** A line graph showing the benefit of using purified cell types in reference. Amount of pancreatic cfDNA predicted is shown for three individuals before, 1 hour after and 2 hours after transplantation, using a reference matrix including methylomes from either purified pancreatic cells (solid lines, also displayed in **5C**) or whole pancreas (dotted lines).

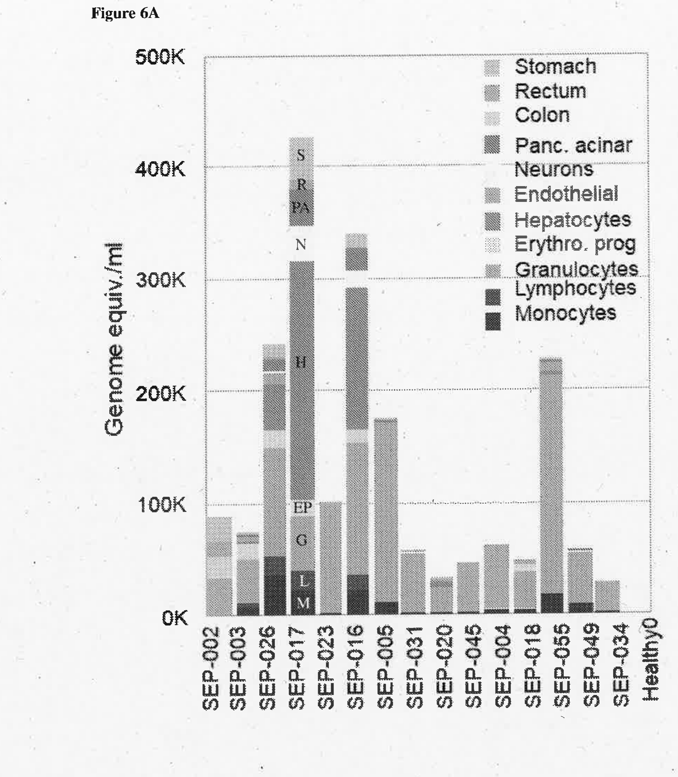

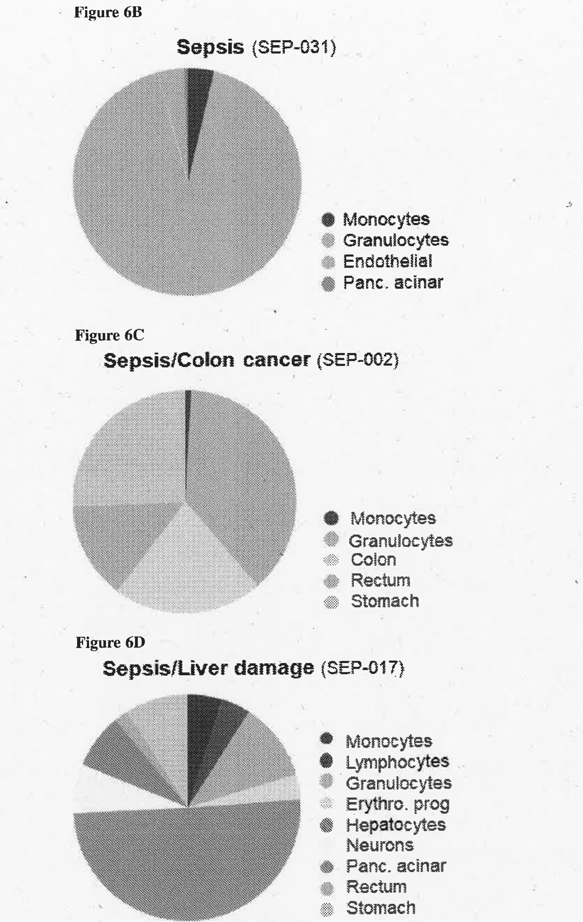

**Figures 6A-E: Cellular contributors to cfDNA in sepsis. (6A)** A bar chart of predicted cellular contributions for 15 samples of cfDNA from patients with sepsis. Cell types which contributed less than 1% were included in "Other". Average of healthy pools is shown at the right. **(6B-D)** Pie charts representing predicted distribution of cell types contributing to cfDNA from 3 of the sepsis samples shown, including a subject with only sepsis **(6B),** a subject whose sepsis developed after extraction of a colon cancer that had metastasized to the liver **(6C)** and a subject with liver damage in addition to sepsis **(6D). (6E)** A line graph showing a comparison of predicted levels of hepatocyte cfDNA to serum levels of Alanine Aminotransferase, a standard biomarker for hepatocyte damage (r=0.926, p-value=7.1e-07.

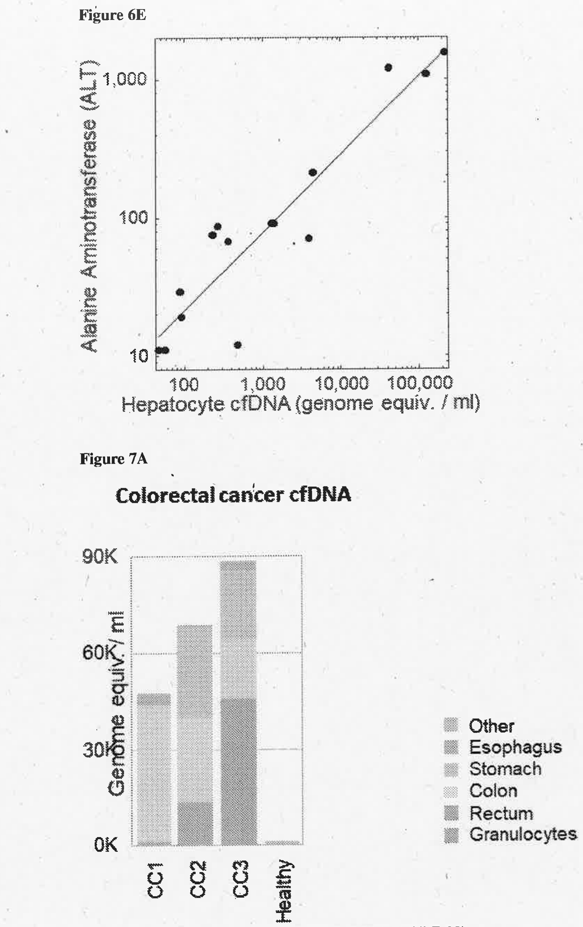

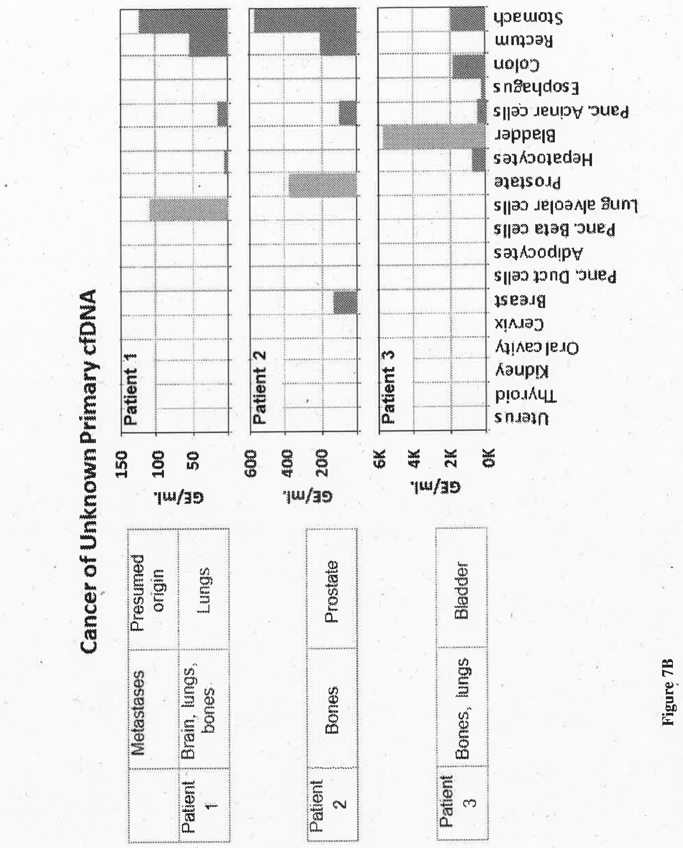

**Figure 7A-B: Cellular contributors to cfDNA in cancer. (7A)** A bar chart showing the predicted contributions of cell types for 3 samples of cfDNA from patients with colon cancer. Sample CC3 is the same as sample S 1 from **Figure 6A.** Average of healthy pools is shown as well. **(7B)** A heat map of the predicted cellular contributors for 3 cfDNA samples obtained from patients diagnosed with a Cancer of Unknown Primary (CUP). Cell types which contributed less than 1% were considered as 0. The contribution of blood cell types is not shown. For each patient, the location of metastases and the predicted tissue source of cancer according to clinical history are listed.

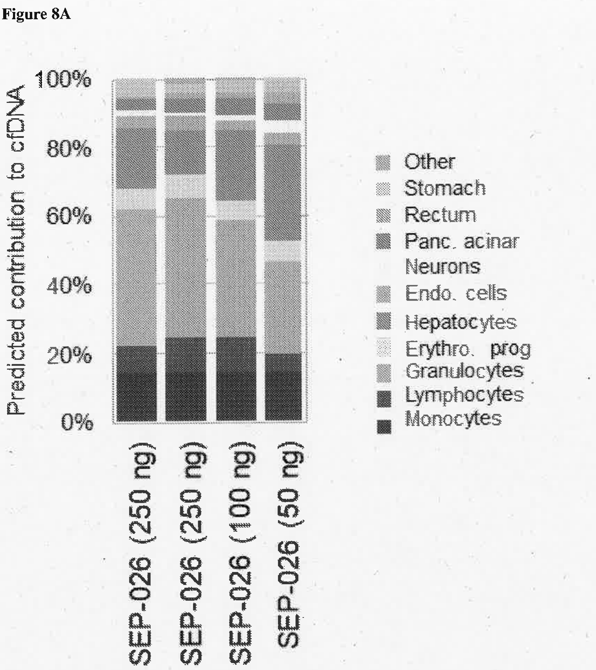

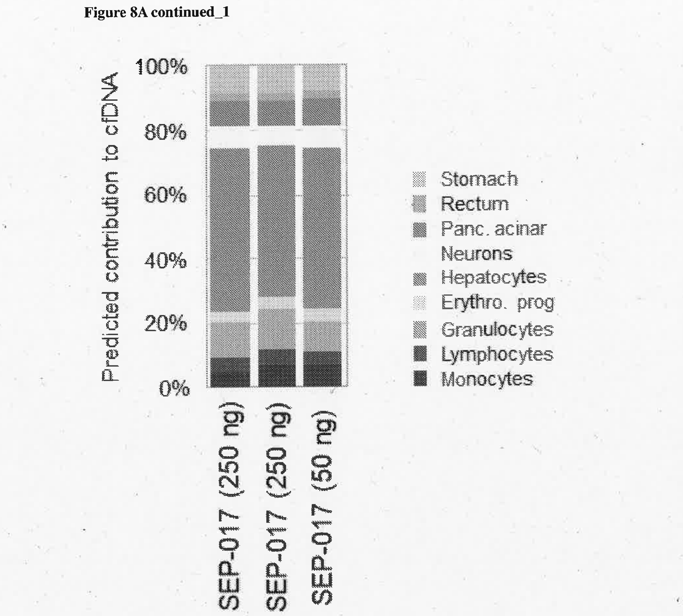

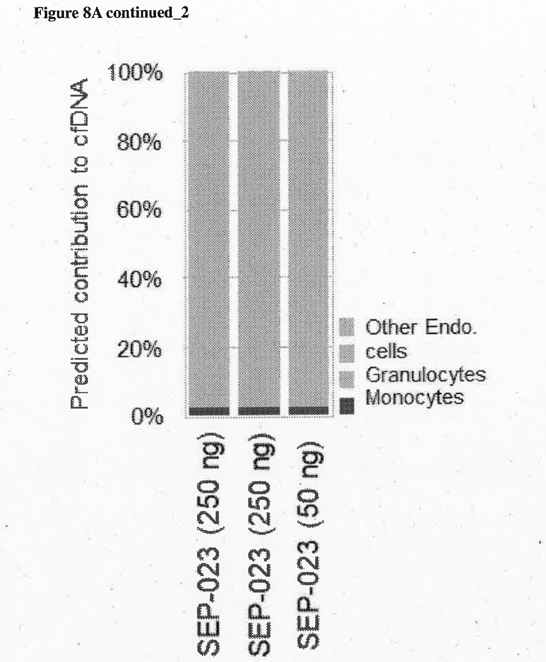

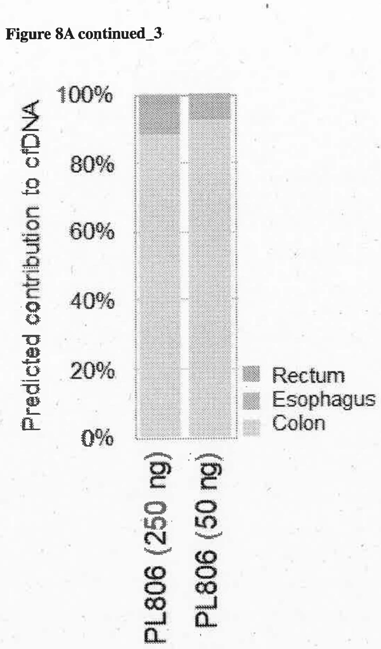

**Figures 8A-B: Reproducibility of deconvolution results. (8A)** Bar charts of the predicted distribution of cellular contributors from 4 samples using different amounts of cfDNA (50 ng, 100 ng or 250 ng). **(8B)** Pearson correlation coefficients shown for different samples analyzed from the same individual with varying amounts of cfDNA used.

## DETAILED DESCRIPTION OF THE INVENTION

The present invention provides methods of determining the origin of cell free DNA (cfDNA) and detecting death of a cell type or tissue in a subject by determining the origin of cfDNA in the subject. The methods of the invention are based on the surprising finding that by generating an atlas of informative methylation sites for various tissues and cell types cfDNA methylation sequencing can be deconvoluted to accurately identify the origin of cfDNA molecules even when they are a very small percentage of the total DNA sampled. The methods of the invention are further based on the surprising findings that use of purified cell types in place of whole tissues and an atlas comprising the most uniquely methylated and unmethylated sites between the different tissues/cell types provided superior deconvolution results.

By a first aspect, there is provided a method of determining the cell or tissue of origin of a cell free DNA (cfDNA) comprising:

a. providing cfDNA;

b. measuring DNA methylation of the cfDNA; and

c. assigning a cfDNA molecules from the cfDNA to a cell type or tissue of origin by comparing the methylation of the molecule to a methylome atlas of at least 1 cell type or tissue, wherein the atlas comprises at least 25 of the 100 most uniquely methylated sites and at least 25 of the 100 most uniquely unmethylated sites in each of the at least 1 cell type or tissue; .

thereby determining the cell or tissue of origin of cfDNA.

In some embodiments, the cfDNA is from a subject and assigning a cfDNA molecule to a cell or tissue of origin indicates detection of death of that cell or tissue. In some embodiments, the subject is suspected of having increased cell death. In some embodiments, the subject is not suspected of having increased cell death. In some embodiments, the subject appears healthy and/or does not suffer from a disease or condition.

By another aspect, there is provided a method of detecting death of a cell type or tissue in a subject comprising:

a. providing cfDNA from the subject;

b. measuring DNA methylation of the cfDNA; and

c. assigning a cfDNA molecules from the cfDNA to a cell type or tissue of origin by comparing the methylation of the molecule to a methylome atlas of at least 1 cell type or tissue, wherein the atlas comprises at least 25 of the 100 most uniquely methylated sites and at least 25 of the 100 most uniquely unmethylated sites in each of the at least l cell type or tissue;

thereby detecting death of a cell type or tissue in a subject.

As used herein, "cfDNA" refers to any DNA obtained from an organism which existed in the organism outside of a cell. In some embodiments, the cfDNA is DNA obtained from an organism which existed in the organism outside of any vesicle. Cell-free DNA is well known in the art, and generally refers to DNA that is free floating within a bodily fluid. This DNA is generally not enclosed in a vesicle and thus DNA in transport, such as by exosomes or other vesicular transporters, in not considered cfDNA. In some embodiments, cfDNA is DNA from a dying and/or dead cell. When a cell dies the DNA is generally fragmented and released from the cell as it lyses. This DNA however, is not all immediately removed or cleaned up and thus persists in the organism. Frequently the DNA from the dead cell enters the bloodstream.

Since cfDNA has a short half-life in the organism, it provides a snap shot of the cell death occurring in the organism at that moment. In some embodiments, the methods of the invention detect cell death that has occurred within the last 5, 10, 15, 20, 25, 30, 35, 40, 45, 50, 55, or 60 minutes from the time of providing the cfDNA. Each possibility represents a separate embodiment of the invention.

In some embodiments, the cfDNA is mammalian cfDNA. In some embodiments, the cfDNA is human cfDNA. In some embodiments, the cfDNA is extracted from bodily fluid. In some embodiments, the providing comprises providing a bodily fluid and isolating the cfDNA from the bodily fluid. In some embodiments, the bodily fluid is blood. In some embodiments, the bodily fluid is selected from at least one of: blood, serum, gastric fluid, intestinal fluid, saliva, bile, tumor fluid, interstitial fluid, and stool. Standard techniques for cell-free DNA extraction are known to a skilled artisan, a non-limiting example of which is the QIAamp Circulating Nucleic Acid kit (QIAGEN).

In some embodiments, at least 10, 20, 30, 40, 50, 60, 70, 80, 90 or 100 ng of cfDNA are provided. Each possibility represents a separate embodiment of the invention. In some embodiments, as little as 10, 20, 30, 40, 50, 60, 70, 80, 90 or 100 ng of cfDNA are provided. Each possibility represents a separate embodiment of the invention. In some embodiments, at least 50 ng are provided. In some embodiments, as little as 50 ng are provided.

In some embodiments, the providing comprises providing a bodily fluid and isolating the cfDNA from the bodily fluid. In some embodiments, the bodily fluid is selected from: blood, serum, gastric fluid, intestinal fluid, saliva, bile, tumor fluid, interstitial fluid, breast milk and . stool. In some embodiments, the bodily fluid is any bodily fluid that contains cfDNA. In some embodiments, the bodily fluid is blood. In some embodiments, the bodily fluid is any one of whole blood, partially lysed whole blood, plasma, or partially processed whole blood.

The sample of blood can be obtained by standard techniques, such as using a needle and syringe. In another embodiment, the blood sample is a peripheral blood sample. Alternatively, the blood sample can be a fractionated portion of peripheral blood, such as a plasma sample. In another embodiment, once the blood sample is obtained, total DNA can be extracted from the sample using standard techniques known to one skilled in the art. In some embodiments, intact cells are removed before DNA extraction, so that only free-floating DNA is extracted. Intact cells can be removed by any method known in the art, such as for non-limiting example by centrifugation or by gradient separation, such as by Ficol gradient separation. A non-limiting example for DNA extraction is the FlexiGene DNA kit (QIAGEN). In another embodiment, maternal plasma may be further separated from peripheral blood by centrifugation, such as exemplified herein, at 1,900 x g for 10 minutes at 4°C. The plasma supernatant may be recentrifuged at 16,000 x g for 10 minutes at 4°C. In another embodiment, a fraction of the resulting supernatant is used for cell-free DNA extraction, to thereby receive plasma DNA extracts. Standard techniques for receiving cell-free DNA extraction are known to a skilled artisan, a non-limiting example of which is the QIAamp Circulating Nucleic Acid kit (QIAGEN). In some embodiments, the total cfDNA is subsequently fragmented, such as to sizes of approximately 300 bp - 800 bp. For example, the total DNA can be fragmented by sonication.

Measuring DNA methylation may be performed by any method known in the art. Non-limiting examples include deep sequencing following bisulfite conversion, ELISA-based methylation kits, methylation sensitive PCR, and the luminometric methylation assay (LUMA). In some embodiments, measuring DNA methylation comprises bisulfite conversion. In some embodiments, measuring DNA methylation further comprises next generation sequencing. In some embodiments, measuring DNA methylation further comprises next generation sequencing. In some embodiments, only the loci present in the atlas are sequenced. Next generation sequencing, also known as high-throughput sequencing is any sequencing method that allows for rapid high-throughput sequencing of base pairs from DNA or RNA samples. Such sequencing is well known in the art and can include Illumina arrays and ion torrent as non-limiting examples. Next generation sequencing of DNA methylation works on a similar principle and may be performed with arrays such as the Illumina EPIC array and the Illumina 450k array, for example. In some embodiments, data from the whole genome is used. In some embodiments, data from chips or arrays are used. Such chip/array data may decrease background, lower costs, and provide more reliable cleaner data.

In some embodiments, the methylome atlas comprises data from at least 1, 3, 5, 10, 15, 20, or 25 tissues. Each possibility represents a separate embodiment of the invention. In some embodiments, the methylome atlas comprises data from at least 1, 3, 5, 10, 15, 20, or 25 cell types. Each possibility represents a separate embodiment of the invention. In some embodiments, the methylome atlas comprises data from at least 1, 3, 5, 10, 15, 20, or 25 tissues and/or cell types. Each possibility represents a separate embodiment of the invention. It will be understood by one skilled in the art that only tissues and/or cell types can be identified as the origin of the cfDNA is they are included in the atlas. Thus, for example, if only 10 tissues are selected than cfDNA can only be identified as coming from one of those tissues. Similarly, if it is predicted that the cfDNA may be from a particular source it may be less time consuming and cheaper to use a smaller atlas. For example, if hepatotoxicity is being examined, the atlas may comprise only the hepatocyte methylome, or only methylomes from liver cell types. In such a case the readout would be X% of the cfDNA is from dead hepatocytes/liver cells and the rest is unknown. If the possible source of cfDNA is unknown, or if a subject is healthy (or appears healthy) than a broader atlas comprising CpGs from more tissues would be preferred. In some embodiments, the atlas comprises data from at least 5 cell types and/or tissues.

In some embodiments, the atlas comprises data only from tissues. In some embodiments, the atlas comprises data only from cell types. In some embodiments, the atlas comprises data from tissues and cell types. In some embodiments, the cell types are purified cell populations. In some embodiments, the cell types comprise blood-derived purified cell populations. In some embodiments, the cell types comprise tissue-derived purified cell populations. In some embodiments, the atlas does not comprise data from blood-derived purified cell population. In some embodiments, the atlas consists of only tissue-derived purified cell population data. In some embodiments, atlas comprises data from blood-derived and tissue-derived purified cell populations. As used here, "blood-derived" and "tissue-derived" cell types or cell populations refer to a cell type or population whose source is either blood or a tissue or organ. Blood-derived population are well known, and may be red blood cells, monocytes, b-cells, t-cells, or the like. They may express specific markers, such as CD4-positive or CD-8 positive T cells, for non-limiting example. Tissue-derived cells are from a tissue or organ and not blood. All organs are made up for multitudes of cells that may be identified by markers, such as protein expression, surface expression, secretion or morphology. Examples include different neurons in the brain, and beta/duct/acinar cells in the pancreas.

In some embodiments, the methylome atlas comprises methylation data from at least 1, 2, 3, 4, 5, 10, 15, 20, 25, 30 or 34 of the following 34 tissues or cell types: monocytes, B-cells, CD4+ T-cells, NK-cells, CD8+ T-cells, eosinophils, neutrophils, erythrocyte progenitors, adipocytes, neurons, hepatocytes, lung alveolar cells, pancreatic beta cells, pancreatic -acinar cells, pancreatic duct cells, vascular endothelial cells, left atrium, bladder, breast, cervix, colon, esophagus, oral cavity, kidney, prostate, rectum, stomach, thyroid, uterus, lung bronchial cells, cholangiocytes, muscle, oligodendrocytes, and ovary. Each possibility represents a separate embodiment of the invention. In some embodiments, the methylome atlas comprises methylation data from at least 1, 2, 3, 4, 5, 10, 15, 20, 25, 29, 30, 31, 32, 33 or 34 of the following 34 tissues or cell types: monocytes, B-cells, CD4+ T-cells, NK-cells, CD8+ T-cells, eosinophils, neutrophils, erythrocyte progenitors, adipocytes, neurons, hepatocytes, lung alveolar cells, pancreatic beta cells, pancreatic acinar cells, pancreatic duct cells, vascular endothelial cells, left atrium, bladder, breast, cervix, colon, esophagus, oral cavity, kidney, prostate, rectum, stomach, thyroid, uterus, lung bronchial cells, cholangiocytes, muscle, oligodendrocytes, and ovary. Each possibility represents a separate embodiment of the invention. In some embodiments, the methylome atlas comprises methylation data from at least 1, 2, 3, 4, 5, 10, 15, 20, 25, 30, 34 or 35 of the following 35 tissues or cell types: monocytes, B-cells, CD4+ T-cells, NK-cells, CD8+ T-cells, eosinophils, neutrophils, erythrocyte progenitors, adipocytes, neurons, hepatocytes, lung alveolar cells, pancreatic beta cells, pancreatic acinar cells, pancreatic duct cells, vascular endothelial cells, left atrium, bladder, breast, cervix, colon, esophagus, oral cavity, head and neck, kidney, prostate, rectum, stomach, thyroid, uterus, lung bronchial cells, cholangiocytes, muscle, oligodendrocytes, and ovary. Each possibility represents a separate embodiment of the invention. In some embodiments, the methylome atlas comprises methylation data from at least 1, 2, 3, 4, 5, 10, 15, 20, 25, 29, 30, 31, 32, 33, 34 or 35 of the following 35 tissues or cell types: monocytes, B-cells, CD4+ T-cells, NK-cells, CD8+ T-cells, eosinophils, neutrophils, erythrocyte progenitors, adipocytes, neurons, hepatocytes, lung alveolar cells, pancreatic beta cells, pancreatic acinar cells, pancreatic duct cells, vascular endothelial cells, left atrium, bladder, breast, cervix, colon, esophagus, oral cavity, head and neck, kidney, prostate, rectum, stomach, thyroid, uterus, lung bronchial cells, cholangiocytes, muscle, oligodendrocytes, and ovary. Each possibility represents a separate embodiment of the invention. In some embodiments, the monocytes are CD14+ monocytes. In some embodiments, the B-cells are CD19+ B-cells. In some embodiments, the NK-cells are CD56+ NK-cells. In some embodiments, oral cavity cells are head and neck cells.

In some embodiments, the atlas comprises at least the 10, 20, 25, 30, 40, 50, 60, 70, 75, 80, 90, 100, 200, 300, 400, 500, 1000, 2000, 3000, 4000 or 5000 most uniquely methylated sites in a tissue or in each tissue. Each possibility represents a separate embodiment of the invention. In some embodiments, the atlas comprises at least 10, 20, 25, 30, 40, 50, 60, 70, 75, 80, 90, or 100 of the 100 most uniquely methylated sites in a tissue or in each. Each possibility represents a separate embodiment of the invention. In some embodiments, the atlas comprises at least the 100 most uniquely methylated sites in a tissue. In some embodiments, the methylome atlas further comprises any CpG sites within at least 50, 100, 150, 200, 250, 300, 400, 500, 1000, 1500, or 2000 base pairs upstream and/or downstream of the most uniquely methylated sites in a tissue or in each tissue. Each possibility represents a separate embodiment of the invention.

In some embodiments, the atlas comprises at least the 10, 20, 25, 30, 40, 50, 60, 70, 75, 80, 90, 100, 200, 300, 400, 500, 1000, 2000, 3000, 4000 or 5000 most uniquely unmethylated sites in a tissue or in each tissue. Each possibility represents a separate embodiment of the invention. In some embodiments, the atlas comprises at least 10, 20, 25, 30, 40, 50, 60, 70, 75, 80, 90, or 100 of the 100 most uniquely unmethylated sites in a tissue or in each tissue. Each possibility represents a separate embodiment of the invention. In some embodiments, the atlas comprises at least the 100 most uniquely unmethylated sites in a tissue. In some embodiments, the methylome atlas further comprises any CpG sites within at least 50, 100, 150, 200, 250, 300, 400, 500, 1000, 1500, or 2000 base pairs upstream and/or downstream of the most uniquely unmethylated sites in a tissue or in each tissue. Each possibility represents a separate embodiment of the invention.

Selection of hyper/hypomethylated sites can be performed as follows: For finding cell-type specific CpGs, after obtaining a matrix X of $N$ CpGs by $K$ cell types, where each column represents the average methylation of all samples of that cell type at each CpG, a matrix X' is then created, where each row $X_i$ was calculated as $X_i' = X_i \times \frac{1}{\sum_k^K X_{i,k}}$. For each cell type k, the rows with the highest value in column k as uniquely hyper-methylated CpGs can be identified. To identify uniquely hypomethylated CpGs, a similar process is performed: a matrix X" = 1 - X was defined, then a matrix

$X_i''' = X_i'' \times \frac{1}{\sum_k^K X''_{i,k}}$ was defined and again the rows with the highest value in column k as uniquely hypomethylated CpGs were identified. For each cell type, the top hypomethylated CpGs and unmethylated CpGs can be included in the reference matrix. A more detail description of an algorithm that may be used for site selection follows.

Optionally, an efficient algorithm for selecting a small number of CpGs for inference of cell-of-origin for circulating cell-free DNA using DNA methylation patterns and Compressed Sensing.

Given an expression atlas X, composed of $n$ CpGs over $d$ cell types, a matrix A will denote a normalized version of the matrix X. For rows $Xi$ whose average methylation < 0.5 we will set $Ai=Xi$, and set $Ai=1-Xi$ for all other rows. Next, we **will** normalize each row Ai to have a Euclidean norm of one: $Ai = Ai / \|Ai\|$. We are now ready to set the Compressed Sensing problem. Given a matrix A, we wish to find a column-sparse matrix W such that WxA is approximately the identity matrix, and:

$$\min_{W}\|WA - I\|^2 \text{ such that } \|1^T W\|_0 \leq s$$

where $s$ is the number of non-zero columns in W.

Once W is found such that $WxA \approx I$, for every vector $Y=A\beta$ that is a linear combination of columns in A, it applies that $WY = W(A\beta) = (WA)\beta = I\beta = \beta$ or in other words, multiplying a mixed vector Y by W would immediately result in the mixture coefficients $\beta$.

Optionally, a greedy algorithm is used for finding W. First, assume that a set S of the CpGs (|S|=s) is provided. Given such a partial set S, a projection of W to the S columns is denoted by $Ws$, and a projection of A to the S rows is denoted by $As$.

$$W_S = \arg\min_{B}\|BA_S - I\|^2$$

Thus, finding the non-zeros columns of W, denoted $Ws$, is equivalent to finding a matrix B which is the pseudo-inverse of the S rows of A, and may be achieved with standard tools (e.g. the pinv function in MATLAB or numpy.linalg.pinv in Python). Next, the set S may be increased by adding the next, i'th, feature that minimizes:

$$i = \arg\min_{i \notin S} \ \min_{B}\|BA_{S+\{i\}} - I\|^2$$

This is equivalent to using the current base Ws, and finding a new orthogonal vector v, such that:

$$i = \arg\min_{i \notin S} \ \min_{v}\|vA_i + W_S A_S - I\|^2$$

EQN 1

Let one define the matrix $C= I - WsAs$ and a function $f(v)=\|vAi-C\|^2$. By deriving $f$ with respect to v, one may find the optimal value $v = C Ai / \|Ai\|^2$ for each i. Next, one may search for the next feature i minimizing f(v). By substituting $v$ in EQN 1 and some manipulations, one obtains:

$$i = \arg\max_{i}\|A_i C^T\|^2$$

where the $n$ x d matrix $AC^T$ may be computed and the row $i$ chosen with the maximal $L_2$ norm.

By repeating this procedure $d$ times, with $d$ denoting the number of cell types in the atlas, and the results given that the rank of $A>d$, an optimal subset of d CpG sites may be found that reveal the mixture coefficients $\beta$.

To obtain a more robust solution, this procedure may be repeated $J$ times (each time, the (j) is used after excluding all features S from previous (j) runs), yielding several sparse matrices W. By averaging over these matrices $W'=E[W]$, a sparse $W'$ matrix may be obtained which is defined over a superset of |SxJ| CpG features. The multiplication of the W' matrix with any mixture data Y may robustly estimate the mixture coefficient $\beta$.

Due to measurement noise, this approach may return erroneous results from specific samples of very similar cell types. Such cell types may have very similar methylation patterns, with few separating CpGs. To correct the erroneous results, one may choose to focus on a subset of CpG sites using a feature selection approach, that would construct an "Error-correcting code" as follows.

Another set S may explicitly include CpGs that are differentially methylated between overall similar cell types. Given a current set S of CpG sutes, one could consider the distance between all pairs of cell types <*ij*> when projected onto the current set S of CpGs, and identifying the most similar pair <i,j >. Then, one can identify the CpG site <k> that is the most differentially methylated among cell types i and *j*, and add the CpG <k> into the set S. By repeating this process iteratively, one can identify a large set of CpGs S whose methylation pattern may differentiate between similar tissues. Namely, at each stage, one would identify the most similar pair of cell types/tissues <i,j> given the currnet set S,

$$\arg\min_{i,j}\|X_i - X_j\|_S^2$$

and then find the k'th feature that would further separate them the most:

$$\arg\max_k \left( \|X_i - X_j\|_{S+\{k\}}^2 - \|X_i - X_j\|_S^2 \right) = \arg\max_k \|X_i - X_j\|_{\{k\}}^2$$

Pairwise distances may be computed among all cell types and used to separate the current pair of tissues. This procedure may be iteratively applied until all pairs of cell types are differentiated.

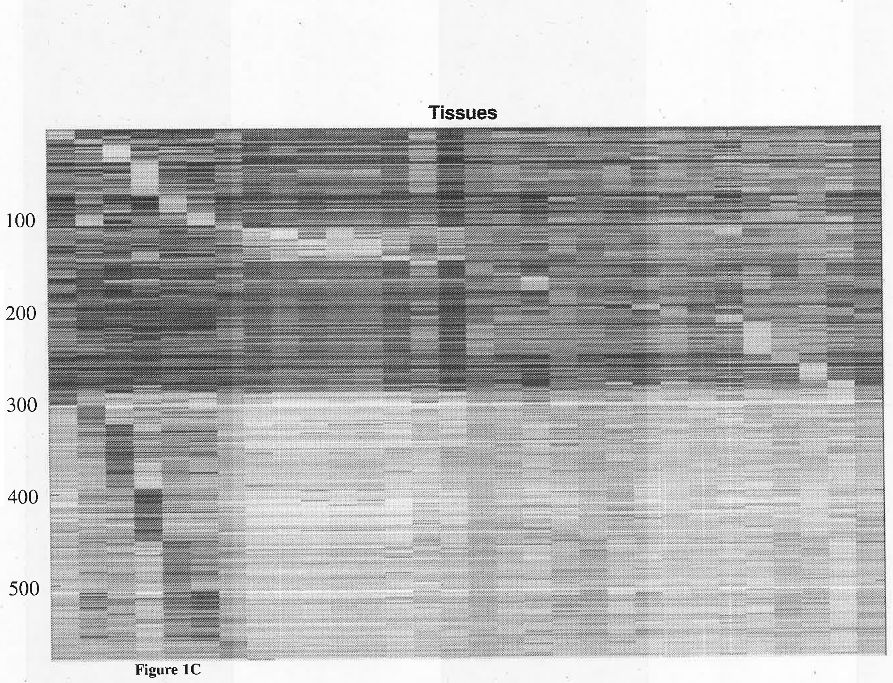

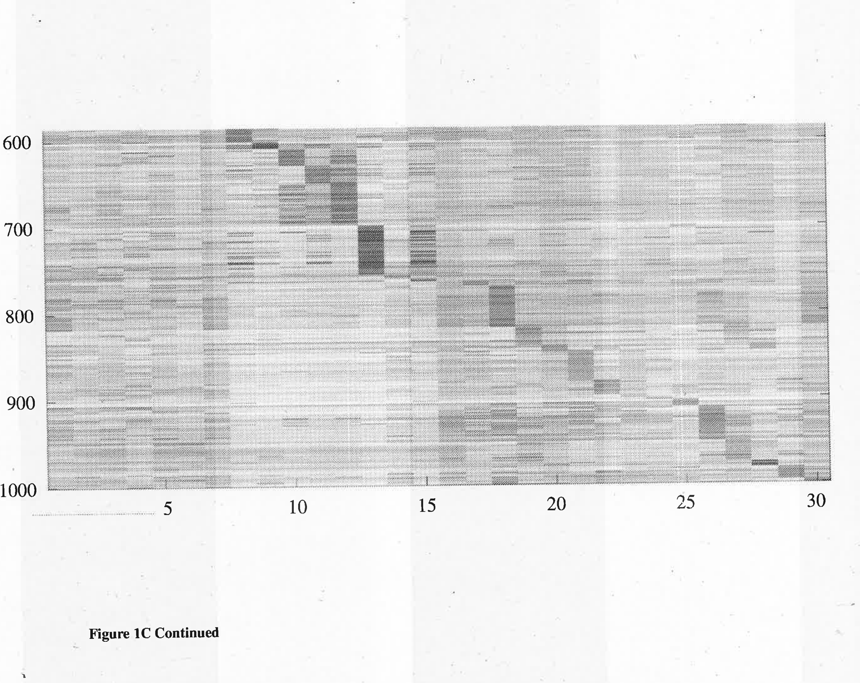

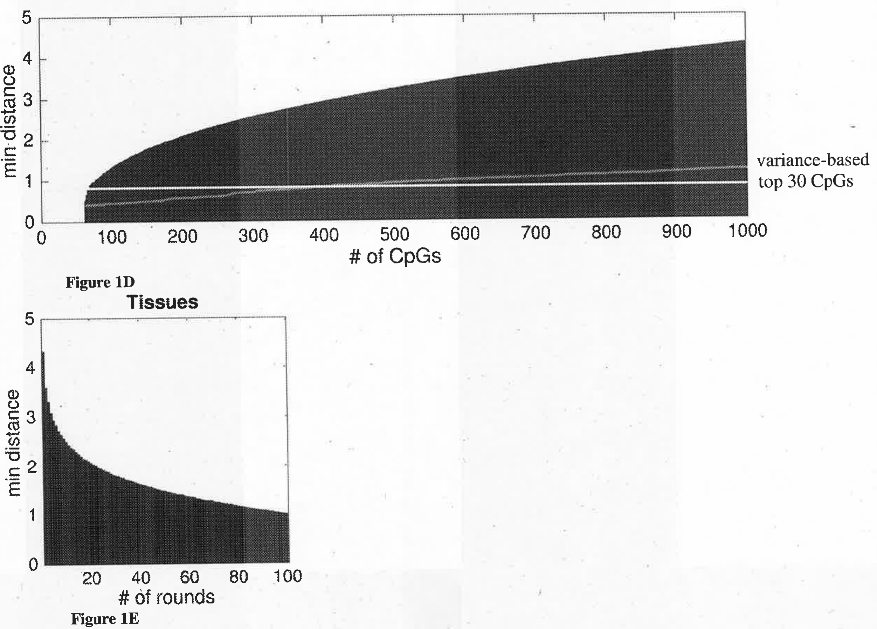

A representative example of CpG selection using the Compressed Sensing algorithm can be found in **Figure 1.** CpGs can be selected using one round of the algorithm **(Fig. 1A),** or by repeated rounds **(Fig. 1B).** After that the error correcting code can be applied for as many iterations as are required **(Fig. 1C).** This method greatly improves the difference between similar tissues **(Fig. 1D),** as there are a limited number of informative CpGs sites that can be added to the atlas. **(Fig. 1E).** For example, a two-step feature selection algorithm may be used for identifying informative CpGs in a DNA methylation atlas. This algorithm may be extremely fast, scanning > 450K CpGs in less than a minute, to identify a subset of CpGs and to compute a matrix W whose multiplication by any (mixed) plasma sample will result in an accurate estimation of the admixture parameters.

This approach may be scalable and applied to much larger datasets, covering hundreds or thousands of tissue types across all 30M CpGs. Thus, it may be suitable for analyzing whole-genome bisulfite-seq data, e.g. for cell-of-origin identification. In addition, it may be applied to identify the most informative regions (or CpG blocks) along the genome, thus used for designing efficient targeted applications (e.g. capture-based). The technique allows to explicitly focus on pairs of similar cell types that are prone to be confused by other methods, thus identifying a set of key CpGs for accurate and robust analysis of cell-free DNA methylation data.

In some embodiments, the site selection is performed using a latent probabilistic model applied to cell free DNA methylation bisulfite sequencing data.

For example, a latent probabilistic model is used for the analysis of bisulfite sequencing DNA methylation data to infer the cell type and tissue type composition of cell free DNA (tissue of origin) and to quantitatively detect circulating tumor DNA in peripheral blood samples, while incorporating prior medical knowledge.

For example, circulating cell free DNA fragments in the peripheral blood may be analyzed to infer the quantitative admixture of tissues and specific cell types from which the cell fragments originated, and to detect small fractions of circulating tumor DNA fragments using CpGs methylation patterns along the genome. Disclosed herein is a computational model to analyze data from a whole genome, a reduced representation, a capture-based method, or the like, followed by bisulfite sequence (BS-seq) determination on DNA fragments originating from the plasma of peripheral blood samples of human patients.

The probabilistic model infers the relative amounts (in genomes/ml units) of tissue-specific and tumor-specific DNA fragments found in plasma samples. A unified probabilistic model, whose (latent) parameters are composed of (a) the admixture coefficient $\theta$, and (b) the tissue-specific and tumor-specific statistical models of CpG methylation patterns. A Bayesian or Maximum Likelihood estimations are applied to infer the latent parameters thus quantitatively identifying the admixture contributions and/or to estimate statistical confidence intervals for each admixture coefficient *0* and/or to infer the estimated probability of the *0*>0 for each healthy or pathological cell type.

For example, consider bisulfite sequencing data D, composed of multiple sequenced reads (or fragments) f. The likelihood of the data D given the model parameters $\theta$ can be written as:

$$\mathcal{L}(\theta : D) = \prod_{f \in D} p(f)$$

where the likelihood of observing a read *f* is written as *p(f)* and assuming independence between sequenced reads.

The reads may be sampled from (an unobserved) composition of multiple types *t=1..T* of methylation patterns, each from a different cell type, tumor-type, or some cellular condition. Using the law of total probability, we can decompose p(f) as a weighted sum of conditional probabilities $p_t$ *(f)* for each cell type t:

$$\mathcal{L}(\theta : D) = \prod_{f \in D} \sum_{t \in T} p(t)p(f|t) = \prod_{f \in D} \sum_{t \in T} \theta_t p_t(f)$$

where p(t)= $\theta_t$ represents the relative amount of cell-free DNA fragments in the plasma from cell-type or tumor-type t and p(f|f)=$p_t$ (f) denotes the conditional probability of observing the sequenced read, *f*, in cell-free DNA that has originated from cell type *t*.

The parameters of this model, including the admixture parameters $\theta_t$ and the cell type specific likelihood model $p_t$ (f) are unobserved. Following is described how the likelihood is optimized with regard to *0*.

An unknown distribution $p_t(f)$ of observing a read *f* from the plasma portion contributed by cell type t. One can approximate $p_t(f)$ using a methylation atlas, containing bisulfite sequencing data from purified cell types and tumor biopsies. Such data, from either whole genome bisulfite sequencing or from reduced representation bisulfite sequencing, may be rapidly accumulated and available for multiple cell types and tissues (healthy or pathological). In addition, some distributions $p_t(f)$ may be estimated from other types of CpG methylation data, such as Illumina Infinium 450K or EPIC BeadChip platforms, which may be available for multiple cell types. Statistical correlations between adjacent CpGs in the same CpG haplotype blocks may be used for approximating the joint probability of CpG methylation in a genome wide manner.

The probability of multiple adjacent CpGs may be approximated using probabilistic graphical models that decompose the joint probability of multiple CpGs into compact models with few parameters by assuming conditional independencies (e.g. Markovian mathematical properties). For example, each CpG haplotype block i that contains up to hundred CpGs can be modeled in each cell type t using at least two parameters: $\beta_{ti}$ that denotes the average methylation of CpGs within this block, and $\tau_{ti}$ that denotes the probability of two adjacent CpG being correlated (i.e., similarly methylated in a DNA molecule). This model may allow decomposing the likelihood of a given observation. For example, the likelihood of a read from the *i*'th block in the *t*'th cell type, with a CpG methylation pattern of TCCCTTT (C=methylated CpG, T=unmethylated CpG, with other nucleotides ignored) may be determined with the equation:

$$P(\text{TCCCTTT}|\theta_{t_i}) = \beta_{t_i}^3 (1 - \beta_{t_i})^4 \tau_{t_i}^4 (1 - \tau_{t_i})^2$$

where the read contains three methylated CpGs (each with probability $\beta_{ti}$), four unmethylated . CpG (each with probability *1-$\beta_{ti}$)*, four consecutive pairs of equally methylated CpGs (each, at probability $\tau_{ti}$) and two consecutive pairs with alternating methylation (each with probability 1 - $\tau_{ti}$).

Optionally, given bisulfite sequencing data from cell type t, Maximum Likelihood models are used to infer the values $\beta_{ti}$ and $\tau_{ti}$ for each cell type *t* and haplotype block i using:

$$\hat{\beta}_{t_i} = \frac{\sum_f \sum_{j \in i} 1\{f_j \text{ is methylated in } t\}}{\sum_f \sum_{i \in i} 1\{\}}$$

and

$$\hat{\tau}_{t_i} = \frac{\sum_f \sum_{j,j+1 \in i} 1\{f_j = f_{j+1}\}}{\sum_f \sum_{j,j+1 \in i} 1\{\}}$$

where 1{ } denotes the indicator function, and $f_j$ denotes the *j*'th CpG sequenced in the read f.

Using Bayes' theorem, one can infer for each read *f*, the posterior probability of it originating from the cell type t:

$$P(t|\mathrm{f}) = \frac{P(\mathrm{f}|\theta_{t_i})P(t_i)}{\sum_k P(\mathrm{f}|\theta_{k_i})P(k_i)} \quad .$$

Here, $P(t_i)$ denotes modeling the a-priori probability of observing a read from the i'th block, originating from the t'th cell type. This may allow one to model a non-uniform coverage of reads circulating in the plasma, in a tissue-specific manner, and more importantly, allows for the integration of prior patient-specific medical data into the computational model.

These inferred posterior probabilities of the cell-of-origin of each observed read, may play a role in detecting small amounts of circulating tumor cell free DNA fragments (ctDNA). Similarly, they may be integrated for the identification and quantification of circulating DNA from various sources (e.g. pathological or normal), including the estimations of confidence intervals.

More generally, a similar approach may infer the admixture coefficient p(t) of the entire circulating DNA in the plasma - finding a Maximum Likelihood solution to the deconvolution problem. Optionally this may be done by maximizing the likelihood of the data D, with subject to the *p(t)* using an Expectation Maximization algorithm, which iteratively calculates the expected probability of assigning each read to each originating tissue (E-step) and then computes the Maximum Likelihood estimation (or Bayesian estimation, given some medical prior knowledge) for each cell type that contributed DNA to the plasma.

This model may not be limited to whole genome bisulfite sequencing data and may be applied to reduced representation bisulfite sequencing data, or to capture-based bisulfite sequencing data. Moreover, for scalability and speed up some feature selection procedures may be applied prior to applying this model, thus focusing the model on the informative portions of the data and possibly ignoring sequenced reads that originate from other regions of the genome.

In some embodiments, the 100 most uniquely methylated sites are selected from Table 1. In some embodiments, the 100 most uniquely unmethylated sites are selected from Table 2. In some embodiments, the CpG sites within at least 150 base pairs upstream and downstream of the most uniquely methylated sites are selected from Table 1. In some embodiments, the CpG sites within at least 150 base pairs upstream and downstream of the most uniquely unmethylated sites are selected from Table 2.

In some embodiments, the methylome atlas further comprises at least one of the 500 CpG sites that best differentiate between the most similar pairs of tissues and cell types. In some embodiments, the analysis of which are the 500 best CpGs is performed iteratively, such that a new decision of which pair of tissues or cell types is most similar is made after each new CpG is added. In some embodiments, at least 1, 5, 10, 50, 100, 150, 200, 250, 300, 350, 400, 450, or 500 CpG sites that best differentiate are added to the atlas. One skilled in the art will understand that with greater numbers of tissues/cell types in the atlas, and with more similar tissues/cell types most of these informative CpGs may be added.

Iterative selection of CpGs that differentiate between the most similar pairs of tissues/cell types can be performed as follows in order to correct for very similar methylomes. Starting with a set S of CpGs (as previously described), the Euclidian distance over S was calculated for every pair of cell types <j,k> as $\sqrt[2]{\sum_{i \in S}(X_{i,j} - X_{i,k})^2}$, and the most similar pair <j',k'> was identified. Then, the row with the highest difference in values in columns j' and k' can be identified, and added into the set S. In some embodiments, the 500 CpG sites that best differentiate between the most similar pairs of tissues and/or cell types are selected from Table 3. More details of algorithms that may be used for this correction are found herein.

In some embodiments the methods of the invention can be used to determine the origin of cfDNA even when the cfDNA from one tissue/cell types is a very small percentage of the whole cfDNA. In some embodiments, the cfDNA of a tissue and/or cell type comprises as little 0.5%, 1%, 1.5%, 2%, 3%, 4%, 5%, 6%, 7%, 8%, 9% or 10% of all of the cfDNA. Each possibility represents a separate embodiment of the invention. In some embodiments, the cfDNA of a tissue and/or cell type comprises more than 0.5%, 1%, 1.5%, 2%, 3%, 4%, 5%, 6%, 7%, 8%, 9% or 10% of all of the cfDNA. Each possibility represents a separate embodiment of the invention. In some embodiments, the cfDNA of a tissue and/or cell type comprises less than 0.5%, 1%, 1.5%, 2%, 3%, 4%, 5%, 6%, 7%, 8%, 9% or 10% of all of the cfDNA. Each possibility represents a separate embodiment of the invention. In some embodiments, the cfDNA of a tissue and/or cell type comprises between 0.5%-10%, 1%-10%, 1.5%-10%, 2%-10%, 0.5%-9%, 1%-9%, 1.5%-9%, 2%-9%, 0.5%-8%, 1%-8%, 1.5%-8%, 2%-8%, 0.5%-7%, 1%-7%, 1.5%-7%, 2%-7%, 0.5%-6%, 1%-6%, 1.5%-6%, 2%-6%, 0.5%-5%, 1%-5%, 1.5%-5%, 2%-5%, 0.5%-4%, 1%-4%, 1.5%-4%, 2%-4%, 0.5%-3%, 1%-3%, 1.5%-3%, 2%-3%, 0.5%-2%, 1%-2%, 1.5%-2%, or 0.5%-1.5% of all of the cfDNA. Each possibility represents a separate embodiment of the invention.

In some embodiments, the methods of the invention determine cfDNA is from a tissue. In some embodiments, the methods of the invention determine cfDNA is from a cell type. In some embodiments, the methods of the invention determine cfDNA is from a tissue and/or a cell type. In some embodiments, cfDNA may be determined to come from more than one cell type of a tissue. In some embodiments, the presence of cfDNA from a cell type or cell types may be used to determine the cfDNA is from the tissue from which the cell type is derived. It will be understood by one skilled in the art that the specificity of the methylation marks defines the specificity of the result. If a beta-cell mark is elevated, it means beta-cells died. It says nothing about other cell types inside or outside the pancreas. If markers from the whole pancreas are elevated, or markers from multiple pancreatic cell types are elected, it means pan-pancreatic damage. Thus, if only one cell type is elevated it means selective damage to that cell type.

In some embodiments, the methods of the invention are for use in detecting a disease state or condition in a subject in need thereof and wherein the cfDNA is from the subject. In some embodiments, the methods of the invention are for diagnosing a disease, and/or condition in a subject in need thereof and wherein the cfDNA is from the subject. In some embodiments, the methods of the invention are for diagnosing an increased risk of a disease or condition. A skilled artisan will recognize that many if not all disease states induce cell death in the tissue or cells in which the disease is manifest. As such knowledge of the origin of cell death be a surrogate for the disease. In some embodiments, the disease state or condition is selected from organ transplantation, sepsis, and cancer. In some embodiments, the disease state or condition is selected from organ transplantation, sepsis, cancer, neurodegenerative disease, degenerative disease, infection, inflammatory disease, toxicity, trauma, hypoxia, vascular disease and metabolic stress. In some embodiments, the disease is cancer and the methods of the invention determine the cell or tissue of origin of the cancer.

By another aspect, there is provided a computer program product for determining the cell or tissue of origin of cell free DNA (cfDNA), comprising a non-transitory computer-readable storage medium having program code embodied thereon, the program code executable by at least one hardware processor to:

a. measure or access measurements of DNA methylation of cfDNA;
b. assign a cfDNA molecule from said cfDNA to a cell type or tissue of origin by comparing the methylation of said molecule to a methylome atlas; and
c. provide an output regarding the cell or tissue of origin of cfDNA.

By another aspect, there is provided a computer program product for determining the cell or tissue of origin of cell free DNA (cfDNA), comprising a non-transitory computer-readable storage medium having program code embodied thereon. The program code is executable by at least one hardware processor to:

a. receive a methylation sequencing data acquired from a cfDNA sample and a methylome atlas. The methylome atlas comprises (i) a first set of uniquely methylated sites, (ii) a plurality of neighboring methylated sites, and (iii) a second set of uniquely methylated sites. At least some of the neighboring methylated sites may be within 500 base units to each element of the first set of uniquely methylated sites. The second set of uniquely methylated sites comprises more than double the number of sites of the first set and comprising less than half the number of tissue types in each comparison; In this manner the second set refines the highest ranking or highest probability tissue types, to narrow down the search to one or more final candidates.

b. compare the methylation sequencing data with a methylome atlas to determine a set of candidate tissues. The comparing comprises identifying at least some elements of the first set of uniquely methylated sites in both the methylation sequencing data and the methylome atlas.

c. compare the plurality of neighboring methylated sites of the methylation . sequencing data with the atlas entries of the set of candidate tissues to determine a first subset of candidate tissues.

d. compare the second set of uniquely methylated sites of the methylome atlas within the methylation sequencing data to determine a probability ranking of candidate tissues.

e. assign at least one of the highest-ranking tissues of the probability ranking of candidate tissues to the cfDNA sample.

For example, an atlas of uniquely methylated sites has different scales of differentiation. A first scale may be each tissue type against all other tissue types. A second scale may be one tissue type compared to a few tissue types, such as between 1 and 10 other tissue types. An associated set of neighboring sites may be used on the first or second sets along to better determine similar tissues. For example, there may be k-levels of scales for differentiating between tissue types, such as differentiating between different genetic families or the like. For example, each comparison scale atlas data may be considered a subset of the full comparison atlas with N to N comparisons. For example, a series of rules and comparison subsets may allow differentiating tissues with greater than 95% accuracy, greater than 97.5% accuracy, greater than 98.5% accuracy, greater than 99.5% accuracy, and/or the like.

By another aspect, there is provided a computerized system for determining the cell or tissue of origin of cfDNA, comprising:

a. one or more test devices for measuring DNA methylation of cfDNA;

b. a processor; and

c. storage medium comprising a computer application that, when executed by the processor, is configured to: .

i. measure or access measurements of DNA methylation of cfDNA;

ii. assign a cfDNA molecule from said cfDNA to a cell type or tissue of origin by comparing the methylation of said molecule to a methylome atlas; and

iii. output from the processor the cell or tissue of origin of cfDNA.

By another aspect, there is provided a computerized system for determining the cell or tissue of origin of cfDNA, comprising: (i) at least one hardware processor; and (ii) a non-transitory computer-readable storage medium having program code embodied thereon. The program code executable by the at least one hardware processor to:

a. receive a methylation sequencing data acquired from a cfDNA sample and a methylome atlas, wherein the methylome atlas comprises (i) a first set of uniquely methylated sites, (ii) a plurality of neighboring methylated sites, and (iii) a second set of uniquely methylated sites comprising more than double the number of sites of the first set. The second set of uniquely methylated sites comprises less than half the number of tissue types in each comparison as the first set. At least some of the neighboring methylated sites are within 500 base units to each element of the first set of uniquely methylated sites.

b. compare the methylation sequencing data with a methylome atlas to determine a set of candidate tissues, wherein the comparing comprises identifying at least some elements of the first set of uniquely methylated sites in both the methylation sequencing data and the methylome atlas.

c. compare the plurality of neighboring methylated sites of the methylation sequencing data with the atlas entries of the set of candidate tissues to determine a first subset of candidate tissues.

d. compare the second set of uniquely methylated sites of the methylome atlas within the methylation sequencing data to determine a probability ranking of candidate tissues.

e. assign at least one of the highest-ranking tissues of the probability ranking of candidate tissues to the cfDNA sample.

In some embodiments, the first set comprises between 25 and 100 most uniquely methylated sites. For example, the first subset is a wide scale search for similar tissue types, but may not differentiate to specific tissue types.
In some embodiments, the first set comprises a plurality of most uniquely methylated sites and wherein the plurality of neighboring methylated sites comprises any CpG sites within between 150 and 500 base pairs upstream and downstream of said most uniquely methylated sites in each tissue or cell type. For example, the neighboring methylated sites are the patterns of methylated sites surrounding the uniquely methylated sites, such as within a window difference from each uniquely methylated site, such as a fixed base unit distances, and/or the like.
In some embodiments, the second set comprises between 100 and 500 most uniquely methylated sites. For example, the second subset is a limited list of comparisons (i.e. pairs, triplets, quadruples, etc.) that would fully differentiate a tissue sample data from similar tissue types (i.e. similar uniquely methylated sites).
In some embodiments, the second set of uniquely methylated sites compares a plurality of specific pairs or triplets of tissue types, such as similar tissue types, or the like.
In some embodiments, the first set of uniquely methylated sites are uniquely methylated as compared to all cell types and tissues of the atlas, and wherein the second set of uniquely methylated sites are uniquely methylated in one cell type or tissue as compared to a second most similar cell type or tissue. This example of multiscale genetic searching may allow quick determination of a sample origin and possible pathologies from a minimal sized atlas. The benefits of a small atlas are easier updates, distribution, and/or the like.
In some embodiments, the comparing comprises using a latent probabilistic model. For example, multiple models may be used to determine the highest probability tissue types.

In some embodiments, the methylome atlas is of at least 5 cell types or tissues, wherein said atlas comprises at least 25 of the 100 most uniquely methylated sites and at least 25 of the 100 most uniquely unmethylated sites in each of said 5 cell types or tissues.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Java, Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

These computer readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

Embodiments may comprise a computer program that embodies the functions described and illustrated herein, wherein the computer program is implemented in a computer system that comprises instructions stored in a machine-readable medium and a processor that executes the instructions. However, it should be apparent that there could be many different ways of implementing embodiments in computer programming, and the embodiments should not be construed as limited to any one set of computer program instructions. Further, a skilled programmer would be able to write such a computer program to implement one or more of the disclosed embodiments described herein. Therefore, disclosure of a particular set of program code instructions is not considered necessary for an adequate understanding of how to make and use embodiments. Further, those skilled in the art will appreciate that one or more aspects of embodiments described herein may be performed by hardware, software, or a combination thereof, as may be embodied in one or more computing systems. Moreover, any reference to an act being performed by a computer should not be construed as being performed by a single computer as more than one computer may perform the act.

By testing device is meant a combination of components that allows the methylation of a piece of DNA to be determined. In some embodiments, the testing device allows for the high-throughput determinization of DNA methylation. The components may include any of those described above with respect to the methods for determining DNA methylation.

For example, the components may be bisulfite conversion kits, or Illumina methylation arrays, and so on.

In certain embodiments the system or test kit further comprises a display for the output from the processor.

By another aspect, there is provided a method of constructing a methylome atlas, comprising:

a. providing DNA methylation data from at least 5 tissues and/or cell types;
b. selecting at least the top 25 uniquely hyper-methylated and at least the top 25 uniquely hypomethylated CpG sites per a tissue and/or cell type as compared to the other tissues and/or cell types; and
c. combining the uniquely hyper-methylated and uniquely hypo-methylated sites for all tissues and/or cell types;

thereby constructing a methylome atlas.

In some embodiments, the methods of the invention further comprise:

d. selecting all neighboring CpG sites within at least 150 base pairs upstream and/or downstream of each uniquely hyper-methylated and uniquely hypomethylated CpG site; . and

e. combining the neighboring CpG sites with the uniquely hyper-methylated and uniquely hypomethylated CpG sites.

In some embodiments, the methods of the invention further comprise:

f. selecting at least the top 25 CpG sites that best differentiate between the most similar pair of tissues and/or cell types; and

g. combining the CpG sites that best differentiate with the uniquely hyper-methylated and uniquely hypomethylated CpG sites.

In some embodiments, the atlas is constructed only with data from whole tissues. In some embodiments, the atlas is constructed only with data from purified cell populations. In some embodiments, the atlas is constructed from data from both tissues and cell populations. As used here, the term "cell type" refers to a unique cell population. Cell types are generally defined by marker that identified the population. This marker can be a genetic marker, or protein expression or morphological to give a few non-limiting examples. Separating cell populations is well known in the art, and can be performed, for example, with magnetic beads, by gradient separation, or by FACS sorting.

In some embodiments, the atlas is constructed with data from cell types from tissue. In some embodiments, the cell types are purified populations from a tissue or organ. In some embodiments, the atlas comprises at least 2 purified populations from the same tissue. In some embodiments, the atlas is constructed with data from purified populations of blood derived cells and tissue derived cells. In some embodiments, the atlas is constructed only from blood derived cells or only tissue derived cells.

In some embodiments, the DNA methylation data is genome wide data. In some embodiments, the DNA methylation data is from a part of the genome. In some embodiments, the DNA methylation data is at least 10%, 20%, 30%, 40%, 50%, 60%, 70%, 80% or 90% of all CpG sites in the genome. Each possibility represents a separate embodiment of the invention. In some embodiments, the DNA methylation data is from a DNA methylation chip or array. Methylation chips, such as for example the Illumina Infinium Human Methylation 450K Beadchip array and the Infinium Human Methylation EPIC Beadchip array, are well known in the art and may be used to provide DNA methylation data for the methods of the invention. In some embodiments, the DNA methylation data is from at least 100000, 150000, 200000, 250000, 30000, 350000, 40000, 450000, or 500000 genomic loci. Each possibility represents a separate embodiment of the invention.

In some embodiments, the method comprises selecting at least the top 10, 20, 25, 30, 40, 50, 60, 70, 75, 80, 90, 100, 200, 300, 400, 500, 1000, 2000, 3000, 4000 or 5000 most uniquely methylated sites in a tissue or in each tissue. Each possibility represents a separate embodiment of the invention. In some embodiments, the method comprises selecting at least 10, 20, 25, 30, 40, 50, 60, 70, 75, 80, 90, or 100 of the top 100-most uniquely methylated sites in a tissue or in each tissue. Each possibility represents a separate embodiment of the invention. In some embodiments, the method comprises selecting at least the top 100 most uniquely methylated sites in a tissue. In some embodiments, the method further comprises selecting any CpG sites within at least 50, 100, 150, 200, 250, 300, 400, 500, 1000, 1500, or 2000 base pairs upstream and/or downstream of the most uniquely methylated sites in a tissue or in each tissue. Each possibility represents a separate embodiment of the invention.

In some embodiments, the method comprises selecting at least the top 10, 20, 25, 30, 40, 50, 60, 70, 75, 80, 90, 100, 200, 300, 400, 500, 1000, 2000, 3000, 4000 or 5000 most uniquely unmethylated sites in a tissue or in each tissue. Each possibility represents a separate embodiment of the invention. In some embodiments, the method comprises selecting at least 10, 20, 25, 30, 40, 50, 60, 70, 75, 80, 90, or 100 of the top 100 most uniquely unmethylated sites in a tissue or in each tissue. Each possibility represents a separate embodiment of the invention. In some embodiments, the method comprises selecting at least the 100 most uniquely unmethylated sites in a tissue. In some embodiments, the method further comprises selecting any CpG sites within at least 50, 100, 150, 200, 250, 300, 400, 500, 1000, 1500, or 2000 base pairs upstream and/or downstream of the most uniquely unmethylated sites in a tissue or in each tissue. Each possibility represents a separate embodiment of the invention.

In some embodiments, the method further comprises selecting at least.the top 5, 10, 20, 30, 40, 50, 75, 100, 150, 200, 250, 300, 350, 400, 450, or 500 CpG sites that best differentiate between the most similar pairs of tissues and/or cell types. Each possibility represents a separate embodiment of the invention. In some embodiments, the analysis of which are the best CpGs is performed iteratively, such that a new decision of which pair of tissues or cell types is most similar is made after each new CpG is added. One skilled in the art will understand that with greater numbers of tissues/cell types in the atlas, and with more similar tissues/cell types most of these informative CpGs may be added.

In some embodiments, the DNA methylation data has been preprocessed to remove unreliable CpG sites. In some embodiments, an unreliable site is a site with less than 3 beads. In some embodiments, an unreliable site has a P-value representing the total fluorescence of the relevant probes that is below 0.01 or 0.05. In some embodiments, an unreliable site has a median absolute error of below 0.05.

As used herein, the term "about" when combined with a value refers to plus and minus 10% of the reference value. For example, a length of about 1000 nanometers (nm) refers to a length of 1000 nm+- 100 nm.

It is noted that as used herein and in the appended claims, the singular forms "a," "an," and "the" include plural referents unless the context clearly dictates otherwise. Thus, for example, reference to "a polynucleotide" includes a plurality of such polynucleotides and reference to "the polypeptide" includes reference to one or more polypeptides and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

In those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

Various embodiments and aspects of the present invention as delineated hereinabove and as claimed in the claims section below find experimental support in the following examples.

## EXAMPLES

Generally, the nomenclature used herein, and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Strategies for Protein Purification and Characterization - A Laboratory Course Manual" CSHL. Press (1996); all of which are incorporated by reference. Other general references are provided throughout this document.

**Materials and Methods**

**Reference matrix**

All DNA methylation profiles were profiled either on the Illumina Infinium Human Methylation 450K Beadchip array or the Infinium Human Methylation EPIC Beadchip array. DNA methylation data for white blood cells (neutrophils, monocytes, eosinophils, B-cells, CD4+ T-cells, CD8+ T-cells, NK-cells, n=6 each) were downloaded from GSE35069 (450K). Data for erythrocyte progenitors (n=5) were downloaded from GSE63409 (450K), and data for left atrium (n=4) were downloaded from GSE62727 (450K). Data for bladder (n=19), breast (n=98), cervix (n=3), colon (n=38), esophagus (n=16), head and neck (n=34), kidney (n=160), prostate (n=50), rectum (n=7), stomach (n=2), thyroid (n=56) and uterus (n=34) were downloaded from TCGA (450K). DNA methylation data for adipocytes (n=3, 450K), hepatocytes (n=2, 450K and EPIC), alveolar lung cells (n=2, EPIC), neurons (n=1, 450K), vascular endothelial cells (n=2, EPIC) and pancreatic acinar cells (n=3, 450K (n=1) and EPIC (n=2)), duct cells (n=3, 450K (n=1) and EPIC (n=2)), beta cells (n=5, 450K (n=3) and EPIC (n=2)) were generated from samples. After construction of the atlas used in the experiment described hereinbelow, data was also compiled for lung bronchial cells, cholangiocytes, muscle, oligodendrocytes and ovary samples.

**Cell isolation**

Cancer-free primary human tissue was obtained from consenting donors, dissociated to single cells, sorted using cell type-specific antibodies, and lysed to obtain genomic DNA, from which 250ng were applied to an Illumina EPIC methylation array. Adipocytes, cortical neurons, hepatocytes, pancreatic acinar cells, pancreatic beta cells and duct cells were obtained from cadaveric donors, as was distal lung tissue. Alveolar epithelial cells were isolated from the lung by FACS using an antibody for EpCAM. Vascular endothelial cells were isolated using anti-CD31 magnetic beads from the saphenous vein, following surgically excision.

**Blood samples**

Donors were consented and whole blood (usually 20 ml) was drawn, collected into an EDTA tube, and spun quickly to separate plasma, which was stored at -20c until isolation of cfDNA.

**DNA extraction**

250 ng was collected from each sample, except where otherwise specified. DNA concentration was measured with Qubit. cfDNA extraction from plasma was performed with the QIAsymphony liquid handling robot.

**Data processing**

Methylation array data were processed with the minfi package in R. For each sample analyzed on the Illumina Methylation array, CpG sites were filtered out if they were represented by less than 3 beads on the array, if the detection P-value, representing total fluorescence of the relevant probes, was lower than 0.01 or if they mapped to a sex chromosome. Background correction and normalization were performed with the preprocess Illumina function, which removes background calculated based on internal control probes and normalizes all samples to a predetermined control sample.

**CpG selection**

As the reference database included samples analyzed with two highly similar yet not identical platforms, the Illumina 450K array and the Illumina EPIC array, sites with low reproducibility between the platforms were identified and removed. To this end, data from samples analyzed on both platforms were collected: 15 samples from GSE86833, 12 samples from GSE92580, 1 sample from our generated dataset (hepatocytes). For each overlapping CpG, the median absolute error (MAE) between the 450K samples and the corresponding EPIC samples was calculated, and 37747 CpGs with a MAE<0.05 were removed.

For finding cell-type specific CpGs, after obtaining a matrix X of $N$ cpgs by $K$ cell types, where each column represents the average methylation of all samples of that cell type at each CpGs, there was then created a matrix $X'$ where

each row $X_i'$ was calculated as $X_i' = X_i \times \frac{1}{\sum_k^K X_{i,k}}$. For each cell type k, the 5000 rows with the highest value in column $k$ as uniquely hypermethylated CpGs were identified. To identify uniquely hypomethylated CpGs, a similar process was performed: a matrix $X'' = 1 - X$ was defined, then a matrix $X_i''' = X_i'' \times \frac{1}{\sum_k^K X''_{i,k}}$ was defined and again the 5000 rows with the highest value in column k as uniquely hypomethylated CpGs were identified. For each cell type, the top 5000 hypomethylated CpGs and unmethylated CpGs were included in the reference matrix.

**Deconvolution**

To calculate the relative contribution of each cell type to a given sample, non-negative least squares were performed, as implemented in the nnls package in R. Given a matrix X of reference methylation values with $N$ CpGs and $K$ cell types, and a vector $y$ of methylation values of length $N,$ non-negative coefficients $\beta$' were identified by solving $argmin_\beta \|X\beta - y\|_2$, subject to $\beta \geq 0$. The resulting $\beta$ was adjusted to have a sum of 1, where for each $\beta_k$ it was defined $\beta_k' = \frac{\beta_k}{\sum_k^K \beta_k}$. To obtain absolute levels of cfDNA (genome equivalent/ml) per cell type, the resulting $\beta_k'$ was multiplied by the total concentration of cfDNA present in the sample, as measured by Qubit.

**Simulations**

4 leukocyte samples were profiled with Illumina methylation arrays (3 with 450K, l with EPIC). For each cell type, each available sample was mixed with each leukocyte sample in ratios 0%:100%, 0.1%:99.9%, 0.2%:99.8%, ..., 100%:0%. Then for each cell type, at each ratio, the average predicted percentage was calculated as well as the 90% confidence interval.

**Sample pooling**

Pooled DNA samples were obtained by mixing DNA from several individuals. DNA was extracted from 8 ml of plasma, and samples were added until 250 ng reached (5-10 samples per pool). No individual contributed more than 2 times as much DNA to a pool than another individual.

**Results**

Herein the ability to identify the cellular contributors to cfDNA in healthy and pathological conditions is evaluated in an unbiased manner, using an extensive reference atlas of human cell- and tissue-specific methylomes. The Illumina Infinium Methylation Array was used, which allows for the simultaneous analysis of the methylation status of >450,000 CpG sites throughout the human genome. Illumina methylation arrays have already been used in the deconvolution of whole blood methylation profiles to determine the relative proportions of white blood cells in a sample, a crucial step in Epigenome-Wide Association Studies (EWAS). However, to date, this deconvolution approach has been applied only to whole blood samples, where all contributing cells are well known types of white blood cells. By contrast, the plasma methylome presents major challenges. First, the Illumina arrays require 250-500ng DNA, which in healthy individuals can be obtained from 100-200ml blood, much above the standard in blood tests. Second, both the cellular sources and their relative contributions to cfDNA are not known, complicating the computational problem of accurate deconvolution. It was hypothesized that by generating a comprehensive database of methylation profiles of human tissues and cell types, it would be possible to deconvolute the methylation profiles of plasma-derived cfDNA, and hence to infer the cellular contributions to cfDNA from a wide range of cell types. The major tissues contributing to cfDNA of healthy individuals was determined, as well as in several pathologies known to involve an increase in circulating cfDNA: Organ transplantation, sepsis and cancer.

**Example 1: Development of a DNA methylation atlas**

To obtain a comprehensive DNA methylation database of human cell types, datasets which were previously published, either as part of The Cancer Genome Atlas (TCGA) (Weisenberger, 2014) or by individual groups that deposited data in the Gene Expression Omnibus (GEO) were employed. In selecting datasets to be included in the database, the following criteria were used: 1) use primary tissue sources, which have not been passaged in culture (reasoning that culture may change methylation patterns or alter the cellular composition of a mixed tissue, e.g. enrich for fibroblasts); 2) use the methylomes of healthy human tissues, which are expected to be universally conserved; and 3) whenever possible use methylomes of cell types, rather than whole tissues. It was reasoned that since all tissues are a composite of multiple cell types (e.g. different types of epithelial cells, blood, vasculature and fibroblasts), methylation signatures of minority populations might be difficult to identify, and unique signatures of the tissue might be masked by the methylome of stroma.

Unfortunately, other than isolated blood cell types, the vast majority of publicly available methylomes come from bulk tissue. Therefore, methylation profiles of key human cell types which had not been previously published were generated, and these were included in the database. Primary human adipocytes, cortical neurons, hepatocytes, lung alveolar cells, pancreatic beta cells, pancreatic acinar cells, pancreatic duct cells, and vascular endothelial cells were isolated. As detailed in the hereinabove Material and Methods, surgical samples from each tissue were enzymatically dissociated according to published protocols, stained with antibodies against the cell type of interest, and sorted using either flow cytometry (FACS) or magnetic beads (MACS). DNA from sorted cells was then prepared, and the methylome obtained using the Illumina 450k or EPIC array platforms. The result of this effort was a human methylome reference atlas, composed of 29 tissues or cell types **(Fig. 2A).** Five more cell types were then added to the atlas at a later date, though the same methodology was used.

**Example 2: Deconvoluting the methylome of a mixture to determine cell type composition**

To deconvolute cfDNA methylation profiles, a subset of CpG sites in the genome which are differentially methylated among the cell types and tissues in the atlas were selected. Only a subset of the methylome was selected to be used for deconvolution based on several considerations. Most CpG sites represented in the Illumina arrays have a similar methylation patters across all tissues; as a result, the methylation score of such sites is not informative, but may add noise to the deconvolution algorithm.

For each tissue 5000 CpG sites were selected that had the highest methylation score in that tissue compared with all other tissues, and the 5000 CpG sites that were most hypomethylated in that tissue were also selected (see Materials and Methods). From those possible 10,000 the 100 most hyper-methylated and the 100 most hypomethylated for each tissue (Table 1) were selected for deconvolution analysis. To these 200 sites from each tissue, any CpG within 150 base-pairs upstream or downstream of a selected CpG was added (Table 2). As this was done on a per CpG bases the number of sites in each tissue varied slightly. Lastly, 500 additional CpGs (Table 3) were added. These CpGs were iteratively selected as the most differentially methylated CpG site between the most similar pair of cell types, at each iteration.

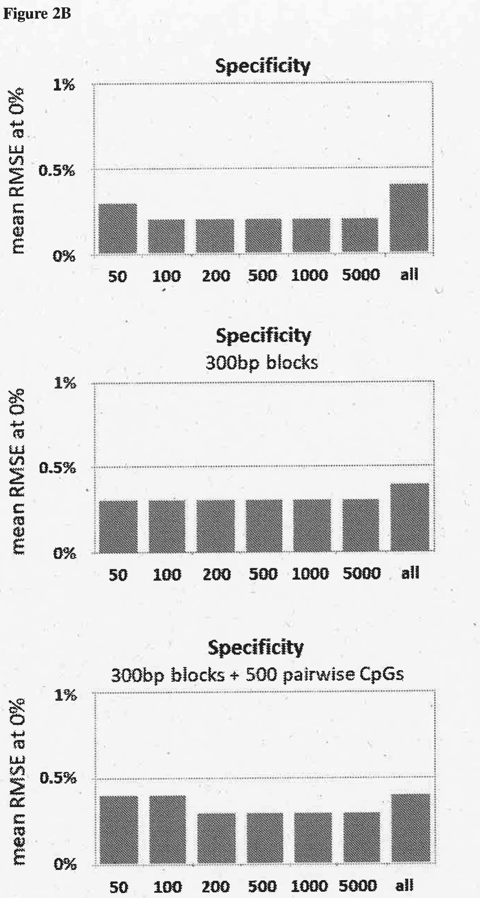

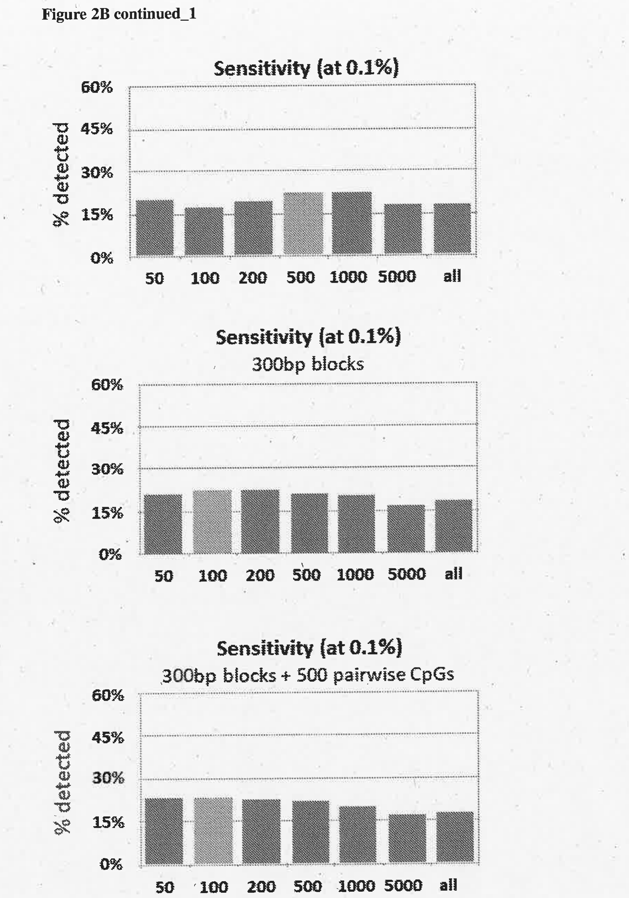

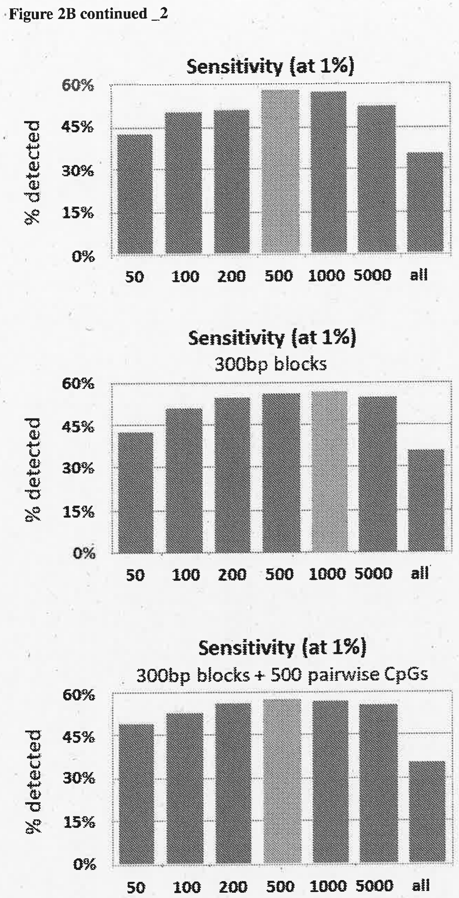

Notably, other feature selection approaches are also possible; for example, it has been reported that others have relied mostly on a subset of methylation sites that are most variable among tissues; however, it was found that tissue-specific sites are superior to tissue-variable sites in deconvolution performance **(Fig. 2B).** Specificity values were estimated as the mean error (RMSE) for simulations with 0% mixing (namely, pure leukocytes), for convolution with different numbers of selected CpGs per cell types. These include the top K differentially hypermethylated CpGs and top K hypomethylated CpGs per cell types, with K varying from 50 CpGs (50x2x29=2900 total), through 100, 200, 500, 1000, 5000, or all methylation array CpGs **(Fig. 2B,** right-most column). Intriguingly, deconvolution with all CpGs is less accurate (and less efficient) compared to models with fewer, selected, CpGs. Sensitivity values, were estimated as percent of simulated mixes correctly detected at 0.1% and 1% mixing in. The optimal selection was found to be 500 hyper + 500 hypomethylated CpGs per cell type, for total of 29,000 CpGs **(Fig. 2B,** orange bars). Deconvolution was also performed with the inclusion of all neighboring CpGs (up to 150bp away) from previously selected ones. The addition of neighboring CpGs allows for accurate deconvolution with fewer CpGs, e.g. 2x100 CpG blocks per cell type (total of 8048 CpGs in 3,860 CpG "haplotype blocks") **(Fig. 2B,** middle row). The inclusion of 500 CpGs with additional pairwise-specificity that are specifically selected to distinguish between similar cell types (e.g. different T cells, uterus vs. cervix, etc.), allowed for a further improvement in sensitivity and specificity with fewer CpGs **(Fig. 2B,** bottom row).

The Tissues/Cell Types are listed as follows: 1=Monocytes, 2=B-cells, 3=CD4+ T-cells, 4=NK-cells, 5=CD8+ T-cells, 6=Eosinophils, 7=Neutrophils, 8=Erythrocyte Progenitors, 9=Adipocytes, 10=Neurons, 11=Hepatocytes, 12=Lung alveolar cells, 13=Pancreatic Beta cells, 14=Pancreatic acinar cells, 15=Pancreatic duct cells, 16=vascular endothelial cells, 17=Left atrium, 18=bladder, 19=Breast, 20=Cervix, 21=Colon, 22=Esophagus, 23=Oral cavity, 24=Kidney, 25=Prostate, 26=Rectum, 27=Stomach , 28=Thyroid, 29=Uterus, 30=Lung bronchial cells, 31=Cholangiocytes, 32=Muscle, 33=Oligodendrocytes, and 34=Ovary.

MU=Most uniquely hyper/hypo-methylated, N=Neighboring

**Table 1: Top 100 hyper-methylated sites per tissue/cell type**

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg04221877 | chr1 | 25257515 | 1 | N | cg05167251 | chr2 | 176987465 | 18 | MU |
| cg15014975 | chr1 | 25257547 | 1 | MU | cg22674699 | chr2 | 176987918 | 18 | MU |
| cg24019564 | chr1 | 25257566 | 1 | N | cg225417 | 35 | chr2 | 176988203 18 | MU |
| cg10993442 | chr1 | 25257587 | 1 | N | cg14236662 | chr2 | 176988284 | 18 | N |
| cg24842859 | chr1 | 25257599 | 1 | N | cg15991405 | chr2 | 176988480 | 18 | N |
| cg20695936 | chr1 | 25257624 | 1 | N | cg12969193 | chr2 | 176988505 | 18 | N |
| cg13106389 | chr1 | 25257626 | 1 | N | cg13158481 | chr2 | 176988580 | 18 | MU |
| cg18087266 | chr1 | 25257629 | 1 | N | cg13544006 | chr20 | 34189411 | 18 | MU |
| cg16306706 | chr1 | 42643274 | 1 | MU | cg20361154 | chr22 | 22862884 | 18 | MU |
| cg07865091 | chr1 | 43814306 | 1 | N | cg22973319 | chr3 | 50310839 | 18 | MU |
| cg18856478 | chr1 | 43814358 | 1 | MU | cg15057061 | chr3 | 181437299 | 18 | MU |
| cg06723492 | chr1 | 161135296 | 1 | MU | cg06613480 | chr3 | 181437383 | 18 | N |
| cg22109530 | chr1 | 178310534 | 1 | MU | cg09540111 | chr3 | 186649601 | 18 | MU |
| cg14515791 | chr1 | 178310545 | 1 | N | cg06374962 | chr4 | 111531926 | 18 | MU |
| cg03254916 | chr1 | 201978949 | 1 | MU | cg18064927 | chr4 | 111531975 | 18 | MU |
| cg21160472 | chr1 | 212782112 | 1 | MU | cg22006640 | chr4 | 111532035 | 18 | N |
| cg24686918 | chr1 | 221613658 | 1 | MU | cg11416290 | chr4 | 111532410 | 18 | MU |
| cg13677741 | chr1 | 236031096 | 1 | MU | cg23806894 | chr4 | 111535528 | 18 | MU |
| cg05396897 | chr1 | 247611448 | 1 | N | cg09780241 | chr4 | 111535597 | 18 | MU |
| cg09226051 | chr1 | 247611502 | 1 | MU | cg02725370 | chr4 | 111551829 | 18 | MU |
| cg27388962 | chr10 | 4705434 | 1 | MU | cg01278596 | chr5 | 139228062 | 18 | MU |
| cg18675610 | chr10 | 32216311 | 1 | MU | cg15992535 | chr5 | 139228150 | 18 | N |
| cg22706007 | chr10 | 32216359 | 1 | N | cg02009088 | chr5 | 139228153. | 18 | N |
| cg14034325 | chr10 | 43891459 | 1 | MU | cg06564388 | chr5 | 153855257 | 18 | N |
| cg14789659 | chr10 | 63809073 | 1 | MU | cg26202847 | chr5 | 153855353 | 18 | MU |
| cg00928816 | chr10 | 63809098 | 1 | N | cg03158581 | chr5 | 153858816 | 18 | N |
| cg20746552 | chr10 | 63809108 | 1 | N | cg15376615 | chr5 | 153858822 | 18 | MU |
| cg16389209 | chr10 | 63809121 | 1 | N | cg26477488 | chr5 | 153858936 | 18 | N |
| cg07520810 | chr10 | 63809149 | 1 | N | cg01834210 | chr5 | 153858967 | 18 | N |
| cg16401465 | chr10 | 63809170 | 1 | N | cg22108469 | chr5 | 174158984 | 18 | N |
| cg25103895 | chr10 | 64565642 | 1 | N | cg03807298 | chr5 | 174159039 | 18 | MU |
| cg20684110 | chr10 | 64565750 | 1 | MU | cg13491462 | chr6 | 1384491 | 18 | N |
| cg01005506 | chr10 | 64565768 | 1 | N | cg14258623 | chr6 | 1384591 | 18 | MU |
| cg12474798 | chr10 | 64565772 | 1 | MU | cg16424078 | chr6 | 1384705 | 18 | N |

(continued)

| Illumina Probe Name | Chr. | Position | Tissue e/Cell Type | MU/N |
|---|---|---|---|---|
| cg27200446 | chr6 | 41606439 | 18 | MU |
| cg23413349 | chr6 | 106958388 | 18 | MU |
| cg09184502 | chr6 | 106958474 | 18 | MU |
| cg04681554 | chr7 | 27283409 | 18 | N |
| cg16355271 | chr7 | 27283528 | 18 | N |
| cg01790002 | chr7 | 27283545 | 18 | MU |
| cg08483376 | chr7 | 27285136 | 18 | MU |
| cg02194223 | chr7 | 27285433 | 18 | N |
| cg01357429 | chr7 | 27285563 | 18 | MU |
| cg19480724 | chr7 | 27285831 | 18 | MU |
| cg22964918 | chr7 | 27286075 | 18 | MU |
| cg22158650 | chr7 | 27291388 | 18 | MU |
| cg01688536 | chr7 | 35296968 | 18 | MU |
| cg13537061 | chr7 | 35298720 | 18 | MU |
| cg13421062 | chr7 | 35301069 | 18 | N |
| cg05809668 | chr7 | 35301188 | 18 | MU |
| cg11878331 | chr8 | 72469507 | 18 | MU |
| cg18855674 | chr8 | 72469553 | 18 | N |
| cg25832771 | chr8 | 72756058 | 18 | N |
| cg09734791 | chr8 | 72756155 | 18 | MU |
| cg00116234 | chr9 | 18474243 | 18 | MU |
| cg08641866 | chr1 | 1141790 | 19 | MU |
| cg14886269 | chr1 | 1141927 | 19 | N |
| cg15706223 | chr1 | 1141934 | 19 | N |
| cg06097659 | chr1 | 1141991 | 19 | MU |
| cg04343794 | chr1 | 1142132 | 19 | MU |
| cg25369015 | chr1 | 9599256 | 19 | N |
| cg19896639 | chr1 | 9599276 | 19 | MU |
| cg03901886 | chr1 | 9600115 | 19 | MU |
| cg13631913 | chr1 | 9600345 | 19 | MU |
| cg13688474 | chr1 | 9600443 | 19 | N |
| cg01566260 | chr1 | 32180356 | 19 | MU |
| cg07288476 | chr1 | 32180486 | 19 | N |
| cg26228266 | chr1 | 35351340 | 19 | MU |

| Illumina Probe Name | Chr. | Position | Tissue/Cell | MU/N |
|---|---|---|---|---|
| cg26033520 | chr10 | 74004071 | 1 | MU |
| cg04858631 | chr10 | 74035570 | 1 | MU |
| cg23231163 | chr10 | 75533350 | 1 | N |
| cg19442545 | chr10 | 75533431 | 1 | MU |
| cg16988611 | chr10 | 82224946 | 1 | MU |
| cg15149596 | chr11 | 19138954 | 1 | MU |
| cg07438103 | chr11 | 47175143 | 1 | MU |
| cg03711944 | chr11 | 47377212 | 1 | MU |
| cg16127573 | chr11 | 57532299 | 1 | MU |
| cg10879244 | chr11 | 64577190 | 1 | N |
| cg22897141 | chr11 | 64577338 | 1 | MU |
| cg25170017 | chr11 | 64644487 | 1 | MU |
| cg06889535 | chr11 | 126455712 | 1 | MU |
| cg17572255 | chr12 | 50676705 | 1 | MU |
| cg20260663 | chr12 | 56211576 | 1 | MU |
| cg08425810 | chr12 | 58132558 | 1 | MU |
| cg08832851 | chr12 | 92535822 | 1 | MU |
| cg08857351 | chr12 | 117627450 | 1 | MU |
| cg21737773 | chr12 | 117627464 | 1 | N |
| cg02486646 | chr12 | 117627478 | 1 | N |
| cg23247274 | chr13 | 21750649 | 1 | MU |
| cg06340704 | chr13 | 97877417 | 1 | MU |
| cg19300307 | chrl3 | 99136580 | 1 | MU |
| cg01424562 | chr14 | 69256677 | 1 | MU |
| cg12827637 | chrl4 | 69256791 | 1 | MU |
| cg08169020 | chr14 | 69256888 | 1 | MU |
| cg19692929 | chr14 | 92575120 | 1 | MU |
| cg16062483 | chr14 | 98444417 | 1 | N |
| cg16278496 | chr14 | 98444476 | 1 | MU |
| cg11798182 | chr14 | 98444513 | 1 | N |
| cg00034769 | chr14 | 98444533 | 1 | N |
| cg25913761 | chr15 | 90727560 | 1 | MU |
| cg26955540 | chr15 | 90727570 | 1 | MU |
| cg06008511 | chr15 | 90727886 | 1 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg18569141 | chr15 | 90727995 | 1 | N | cg04158367 | chr1 | 92952440 | 19 | N |
| cg16439823 | chr15 | 90728020 | 1 | N | cg26027669 | chr1 | 92952467 | 19 | N |
| cg19331221 | chr15 | 90728027 | 1 | MU | cg16297569 | chr1 | 92952517 | 19 | N |
| cg09465703 | chr16 | 733842 | 1 | MU | cg17858328 | chr1 | 92952521 | 19 | MU |
| cg05780543 | chr16 | 46963764 | 1 | MU | cg20125091 | chr1 | 92952641 | 19 | N |
| cg09267773 | chr16 | 79802467 | 1 | MU | cg23624705 | chr1 | 92952655 | 19 | N |
| cg09642825 | chr16 | 87416230 | 1 | MU | cg18943693 | chr1 | 155043561 | 19 | MU |
| cg03646327 | chr16 | 88509768 | 1 | N | cg26022183 | chr1 | 156215655 | 19 | MU |
| cg05507654 | chr16 | 88509857 | 1 | MU | cg10544251 | chr1 | 156215727 | 19 | N |
| cg27189395 | chr16 | 89725071 | 1 | MU | cg20489026 | chr1 | 160053931 | 19 | MU |
| cg04043919 | chr16 | 89725091 | 1 | N | cg15580417 | chr1 | 160053947 | 19 | N |
| cg21692159 | chr17 | 36898937 | 1 | MU | cg04148762 | chr1 | 203598573 | 19 | N |
| cg10361922 | chr17 | 40925790 | 1 | MU | cg17721249 | chr1 | 203598594 | 19 | N |
| cg02962602 | chr17 | 80171413 | 1 | MU | cg26253438 | chr1 | 203598623 | 19 | MU |
| cg02413187 | chr19 | 1356269 | 1 | N | cg15732851 | chr1 | 203598761 | 19 | MU |
| cg23674169 | chr19 | 1356278 | 1 | N | cg09352372 | chr1 | 212838765 | 19 | MU |
| cg13646005 | chr19 | 1356304 | 1 | N | cg03599978 | chr1 | 219834537 | 19 | MU |
| cg23057220 | chr19 | 1356315 | 1 | MU | cg26682664 | chr1 | 219834581 | 19 | N |
| cg26282762 | chr19 | 1356444 | 1 | N | cg08978597 | chr10 | 8087295 | 19 | MU |
| cg08846870 | chr19 | 15568360 | 1 | MU | cg01364137 | chr10 | 8087426 | 19 | N |
| cg05258935 | chr19 | 39086923 | 1 | MU | cg23768829 | chr10 | 8089653 | 19 | N |
| cg22006825 | chr19 | 41771771 | 1 | N | cg20281962 | chr10 | 8089733 | 19 | MU |
| cg12946660 | chr19 | 41771873 | 1 | MU | cg18794404 | chr10 | 22542024 | 19 | MU |
| cg01771876 | chr2 | 11886613 | 1 | MU | cg09614653 | chr10 | 22542145 | 19 | N |
| cg11845111 | chr2 | 191398756 | 1 | N | cg14159304 | chr10 | 105212236 | 19 | MU |
| cg27211696 | chr2 | 191398769 | 1 | N | cg04617948 | chr10 | 129535138 | 19 | N |
| cg10560079 | chr2 | 191398806 | 1 | MU | cg25539045 | chr10 | 129535186 | 19 | MU |
| cg00982136 | chr2 | 201726139 | 1 | MU | cg16642284 | chr10 | 129535378 | 19 | MU |
| cg08474651 | chr2 | 201729683 | 1 | MU | cg25576011 | chr10 | 129535458 | 19 | N |
| cg26047334 | chr2 | 218785909 | 1 | MU | cg09614415 | chr10 | 129535509 | 19 | N |
| cg08063160 | chr2 | 238578376 | 1 | MU | cg26365854 | chr11 | 44330903 | 19 | MU |
| cg03504039 | chr2 | 238578435 | 1 | N | cg08408994 | chr 11 | 44330958 | 19 | N |
| cg22734058 | chr2 | 238578491 | 1 | N | cg19520927 | chr11 | 44341646 | 19 | MU |
| cg20074395 | chr20 | 1246715 | 1 | MU | cg04017555 | chr11 | 61062704 | 19 | N |

(continued)

| Illumina Probe Name | Chr. | Position | Tissu e/Cell Type | MU/N |
|---|---|---|---|---|
| cg27175288 | chr11 | 61062837 | 19 | MU |
| cg05190577 | chr11 | 61062843 | 19 | N |
| cg07402729 | chr11 | 61062902 | 19 | MU |
| cg06282270 | chr11 | 61062910 | 19 | MU |
| cg09676684 | chr11 | 61062962 | 19 | N |
| cg01698298 | chr11 | 61062964 | 19 | N |
| cg23619769 | chr11 | 71952356 | 19 | MU |
| cg26987660 | chr11 | 71952431 | 19 | N |
| cg16600991 | chr12 | 54389477 | 19 | MU |
| cg21837192 | chr12 | 54398765 | 19 | N |
| cg20643952 | chr12 | 54398809 | 19 | MU |
| cg12590051 | chr12 | 54398891 | 19 | N |
| cg18540674 | chr12 | 54405379 | 19 | MU |
| cg18780217 | chr12 | 112204756 | 19 | N |
| cg21470387 | chr12 | 112204761 | 19 | N |
| cg20997247 | chr13 | 46425379 | 19 | MU |
| cg19352193 | chr13 | 46425508 | 19 | MU |
| cg00616243 | chr13 | 95202420 | 19 | MU |
| cg07035145 | chr14 | 51410689 | 19 | MU |
| cg23324048 | chr14 | 60794590 | 19 | MU |
| cg06481122 | chr15 | 37403088 | 19 | MU |
| cg27031435 | chr15 | 37403184 | 19 | N |
| cg11926610 | chr15 | 37403211 | 19 | N |
| cg16656979 | chr15 | 37403242 | 19 | N |
| cg12418190 | chr15 | 37403304 | 19 | MU |
| cg03148231 | chr15 | 89954845 | 19 | N |
| cg10308906 | chr15 | 89954991 | 19 | N |
| cg21340230 | chr15 | 89955105 | 19 | MU |
| cg26029554 | chr16 | 2765620 | 19 | N |
| cg04784471 | chr16 | 2765637 | 19 | N |
| cg02863489 | chr16 | 2765710 | 19 | MU |
| cg16176379 | chr16 | 55543049 | 19 | N |
| cg09793988 | chr16 | 55543053 | 19 | N |
| cg01751134 | chr16 | 55543166 | 19 | MU |

| Illumina Probe Name | Chr. | Position | Tissue/Cell | MU/N |
|---|---|---|---|---|
| cg04053242 | chr20 | 1246772 | 1 | N |
| cg08257293 | chr20 | 30308440 | 1 | MU |
| cg01377358 | chr20 | 30948211 | 1 | MU |
| cg05056497 | chr21 | 35899448 | 1 | MU |
| cg14785464 | chr21 | 38362725 | 1 | MU |
| cg06167719 | chr21 | 38362727 | 1 | MU |
| cg03419014 | chr21 | 38362742 | 1 | N |
| cg09037712 | chr21 | 38362754 | 1 | N |
| cg25703541 | chr22 | 24373054 | 1 | N |
| cg02953382 | chr22 | 24373134 | 1 | MU |
| cg26460530 | chr22 | 39919067 | 1 | MU |
| cg15376996 | chr22 | 50912555 | 1 | MU |
| cg25381017 | chr3 | 3151795 | 1 | MU |
| cg08404225 | chr3 | 3151899 | 1 | N |
| cg13512830 | chr3 | 149530294 | 1 | N |
| cg12461469 | chr3 | 149530341 | 1 | MU |
| cg06398251 | chr3 | 184033620 | 1 | MU |
| cg14855367 | chr3 | 191048308 | 1 | MU |
| cg27312961 | chr3 | 191048361 | 1 | N |
| cg13506158 | chr3 | 191048377 | 1 | N |
| cg27269488 | chr3 | 191048411 | 1 | N |
| cg00591949 | chr3 | 191048439 | 1 | MU |
| cg22879098 | chr5 | 672845 | 1 | MU |
| cg24082121 | chr5 | 672872 | 1 | MU |
| cg11895835 | chr5 | 111078476 | 1 | N |
| cg23256675 | chr5 | 111078579 | 1 | MU |
| cg17533455 | chr5 | 130599827 | 1 | MU |
| cg22017733 | chr5 | 162936453 | 1 | MU |
| cg23012579 | chr5 | 172359545 | 1 | N |
| cg18034299 | chr5 | 172359680 | 1 | MU |
| cg16416715 | chr6 | 32942358 | 1 | N |
| cg26264318 | chr6 | 32942400 | 1 | N |
| cg25158622 | chr6 | 32942429 | 1 | N |
| cg26953232 | chr6 | 32942494 | 1 | MU |

(continued)

| Illumina Probe Name | Chr. | Position | Tissue/Cell | MU/N |
|---|---|---|---|---|
| cg24275356 | chr6 | 32942541 | 1 | MU |
| cg27395200 | chr6 | 32942710 | 1 | MU |
| cg07275218 | chr6 | 32942714 | 1 | N |
| cg17451586 | chr6 | 32942808 | 1 | N |
| cg03780271 | chr6 | 33280052 | 1 | N |
| cg12589538 | chr6 | 33280149 | 1 | MU |
| cg11796996 | chr6 | 33280159 | 1 | MU |
| cg02863594 | chr6 | 33280199 | 1 | N |
| cg01253676 | chr6 | 33280212 | 1 | N |
| cg20998791 | chr6 | 33280228 | 1 | N |
| cg18353226 | chr6 | 33280390 | 1 | N |
| cg14419102 | chr6 | 33280400 | 1 | MU |
| cg06375761 | chr6 | 33280408 | 1 | N |
| cg26083458 | chr6 | 33280424 | 1 | MU |
| cg13638257 | chr6 | 33280436 | 1 | MU |
| cg06493154 | chr6 | 42859023 | 1 | MU |
| cg20227056 | chr6 | 130096463 | 1 | N |
| cg25399239 | chr6 | 139013092 | 1 | MU |
| cg18136963 | chr6 | 139013146 | 1 | MU |
| cg23422583 | chr7 | 6388872 | 1 | MU |
| cg11532431 | chr7 | 27169674 | 1 | MU |
| cg00562553 | chr7 | 27169740 | 1 | N |
| cg04317399 | chr7 | 27170313 | 1 | N |
| cg07317062 | chr7 | 27170388 | 1 | N |
| cg19142026 | chr7 | 27170394 | 1 | MU |
| cg11410718 | chr7 | 27170412 | 1 | N |
| cg23220823 | chr7 | 45041140 | 1 | MU |
| cg05875700 | chr8 | 638208 | 1 | MU |
| cg12641240 | chr8 | 638330 | 1 | N |
| cg00936790 | chr8 | 29027844 | 1 | MU |
| cg04962480 | chr8 | 101962118 | 1 | MU |
| cg23006204 | chr9 | 130700866 | 1 | N |
| cg14395885 | chr9 | 130700923 | 1 | N |
| cg21181453 | chr9 | 130700954 | 1 | MU |

| Illumina Probe Name | Chr. | Position | Tissue e/Cell Type | MU/N |
|---|---|---|---|---|
| cg07215504 | chr16 | 88497007 | 19 | N |
| cg03035213 | chr16 | 88497077 | 19 | MU |
| cg18331855 | chr17 | 1131781 | 19 | N |
| cg11424456 | chr17 | 1131878 | 19 | MU |
| cg25432975 | chr17 | 1959066 | 19 | N |
| cg25520679 | chr17 | 1959121 | 19 | N |
| cg17029019 | chr17 | 1959124 | 19 | MU |
| cg23882658 | chr17 | 1959132 | 19 | N |
| cg25660390 | chr17 | 73073700 | 19 | MU |
| cg24213437 | chr19 | 11529741 | 19 | N |
| cg02433671 | chr19 | 11529856 | 19 | MU |
| cg08198862 | chr19 | 11529947 | 19 | N |
| cg08068820 | chr19 | 11530021 | 19 | MU |
| cg08000684 | chr19 | 11530025 | 19 | MU |
| cg14913644 | chr19 | 11530065 | 19 | MU |
| cg27493151 | chr19 | 38701994 | 19 | MU |
| cg22893791 | chr2 | 26407628 | 19 | N |
| cg10927841 | chr2 | 26407743 | 19 | MU |
| cg18958844 | chr2 | 55509779 | 19 | MU |
| cg08138685 | chr2 | 119610526 | 19 | MU |
| cg17342925 | chr2 | 119610605 | 19 | N |
| cg07716249 | chr2 | 133428422 | 19 | N |
| cg20265661 | chr2 | 133428502 | 19 | MU |
| cg01850449 | chr2 | 133428510 | 19 | N |
| cg09622982 | chr2 | 133428523 | 19 | N |
| cg15665400 | chr2 | 133428652 | 19 | MU |
| cg06862374 | chr2 | 219736549 | 19 | N |
| cg15415787 | chr2 | 220349117 | 19 | MU |
| cg02869459 | chr2 | 220349208 | 19 | MU |
| cg10515731 | chr2 | 220378996 | 19 | MU |
| cg18844301 | chr2 | 220379000 | 19 | MU |
| cg17610929 | chr2 | 220379044 | 19 | MU |
| cg24743778 | chr2 | 220379058 | 19 | MU |
| cg19210770 | chr2 | 220379091 | 19 | N |

29

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg00921973 | chr9 | 130701076 | 1 | N | cg12046414 | chr2 | 220379199 | 19 | MU |
| cg07558455 | chr1 | 62784762 | 2 | N | cg21767952 | chr2 | 220379260 | 19 | N |
| cg16028201 | chr1 | 62784802 | 2 | MU | cg07906520 | chr20 | 37435716 | 19 | MU |
| cg13673164 | chr1 | 110009509 | 2 | MU | cg23085343 | chr20 | 50159160 | 19 | MU |
| cg04997273 | chr1 | 117452884 | 2 | MU | cg17648210 | chr21 | 46352817 | 19 | MU |
| cg11221228 | chr1 | 117452905 | 2 | N | cg11417653 | chr22 | 24181212 | 19 | MU |
| cg24862510 | chr1 | 164290545 | 2 | MU | cg25940946 | chr22 | 24181263 | 19 | N |
| cg18222582 | chr1 | 232765331 | 2 | N | cg25037461 | chr22 | 24181268 | 19 | N |
| cg16026114 | chr1 | 232765417 | 2 | MU | cg14774086 | chr22 | 24181270 | 19 | N |
| cg11158057 | chr1 | 239549798 | 2 | MU | cg23060646 | chr3 | 9904411 | 19 | MU |
| cg08542090 | chr1 | 239549830 | 2 | N | cg00498591 | chr3 | 9904557 | 19 | MU |
| cg23684246 | chr1 | 239549853 | 2 | N | cg09469667 | chr3 | 9904687 | 19 | N |
| cg22017581 | chr1 | 239550177 | 2 | N | cg23350106 | chr3 | 9957221 | 19 | MU |
| cg13985485 | chr1 | 239550283 | 2 | N | cg04066019 | chr3 | 16554466 | 19 | MU |
| cg13570831 | chr1 | 239550303 | 2 | MU | cg06085877 | chr3 | 42304966 | 19 | MU |
| cg17426273 | chr10 | 21462441 | 2 | MU | cg21327694 | chr3 | 42305063 | 19 | MU |
| cg08564661 | chr10 | 124133785 | 2 | MU | cg19612450 | chr3 | 42305186 | 19 | N |
| cg03205140 | chr10 | 124133802 | 2 | N | cg21148362 | chr3 | 62353708 | 19 | MU |
| cg10576280 | chr10 | 124133822 | 2 | N | cg20276377 | chr3 | 99595026 | 19 | MU |
| cg05858607 | chr10 | 128593624 | 2 | N | cg22092811 | chr3 | 99595145 | 19 | MU |
| cg00622654 | chr10 | 128593718 | 2 | MU | cg18337222 | chr3 | 99595254 | 19 | N |
| cg06128198 | chr10 | 128593922 | 2 | MU | cg23816347 | chr3 | 126113640 | 19 | MU |
| cg22665692 | chr10 | 128593943 | 2 | MU | cg09686443 | chr3 | 126113658 | 19 | N |
| cg23665778 | chr10 | 128593946 | 2 | N | cg01815671 | chr3 | 126113663 | 19 | N |
| cg18932726 | chr10 | 128594141 | 2 | N | cg11229862 | chr3 | 126113682 | 19 | N |
| cg05608777 | chr10 | 128594144 | 2 | MU | cg20312228 | chr3 | 126113707 | 19 | N |
| cg02300377 | chr11 | 12399056 | 2 | N | cg21109744 | chr3 | 126113743 | 19 | N |
| cg26320696 | chr11 | 12399109 | 2 | N | cg00891278 | chr3 | 126113784 | 19 | N |
| cg18778433 | chr11 | 12399176 | 2 | MU | cg00355389 | chr3 | 139653431 | 19 | N |
| cg18270629 | chr11 | 31831974 | 2 | MU | cg01755562 | chr3 | 139653545 | 19 | MU |
| cg16802508 | chr11 | 67070738 | 2 | N | cg03458172 | chr3 | 157815672 | 19 | N |
| cg25915131 | chr11 | 67070756 | 2 | N | cg05401764 | chr3 | 157815808 | 19 | MU |
| cg19176391 | chr11 | 67070760 | 2 | MU | cg00859352 | chr3 | 171176909 | 19 | N |
| cg05289892 | chr11 | 67070816 | 2 | MU | cg23533254 | chr3 | 171176940 | 19 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg25753024 | chr11 | 67070913 | 2 | MU | cg20817902 | chr4 | 81124121 | 19 | MU |
| cg08662757 | chr11 | 67070967 | 2 | MU | cg25378916 | chr5 | 1875013 | 19 | MU |
| cg26991761 | chr11 | 67071064 | 2 | N | cg06654549 | chr5 | 1875058 | 19 | MU |
| cg03565189 | chr11 | 69924724 | 2 | MU | cg09155145 | chr5 | 1875188 | 19 | N |
| cg02788102 | chr11 | 115374969 | 2 | N | cg17629929 | chr5 | 1875548 | 19 | MU |
| cg19697530 | chr11 | 115375071 | 2 | MU | cg14024788 | chr5 | 1875611 | 19 | MU |
| cg14944514 | chr11 | 115375192 | 2 | N | cg18856388 | chr5 | 1875886 | 19 | MU |
| cg02083559 | chr12 | 24103358 | 2 | MU | cg21982950 | chr5 | 1876200 | 19 | N |
| cg27523779 | chr12 | 24103374 | 2 | N | cg07961015 | chr5 | 1876215 | 19 | N |
| cg22249612 | chr12 | 56121485 | 2 | MU | cg14564616 | chr5 | 1876337 | 19 | MU |
| cg24642065 | chr12 | 130527030 | 2 | N | cg14451382 | chr5 | 1876397 | 19 | N |
| cg13576883 | chr12 | 130527038 | 2 | N | cg18349911 | chr5 | 1877135 | 19 | MU |
| cg15584445 | chr12 | 130527046 | 2. | N | cg00370293 | chr5 | 43397113 | 19 | N |
| cg10233825 | chr12 | 130527104 | 2 | MU | cg16324633 | chr5 | 43397167 | 19 | N |
| cg02681173 | chr12 | 130527106 | 2 | N | cg12634932 | chr5 | 43397230 | 19 | MU |
| cg25591794 | chr13 | 28554842 | 2 | MU | cg10864145 | chr5 | 43397232 | 19 | MU |
| cg06621094 | chr13 | 75149963 | 2 | MU | cg18199554 | chr5 | 43397288 | 19 | N |
| cg18656829 | chr13 | 100632250 | 2 | MU | cg08763705 | chr5 | 139525657 | 19 | MU |
| cg20327444 | chr13 | 110959647 | 2 | MU | cg03490331 | chr5 | 172672817 | 19 | N |
| cg17541528 | chr13 | 110959650 | 2 | N | cg17543488 | chr5 | 172672959 | 19 | MU |
| cg01956420 | chr13 | 110959668 | 2 | N | cg08506654 | chr5 | 172673098 | 19 | N |
| cg18641486 | chr14 | 37641660 | 2 | N | cg27615363 | chr5 | 174120078 | 19 | MU |
| cg23291305 | chr14 | 37641726 | 2 | MU | cg17339488 | chr5 | 174120120 | 19 | N |
| cg18909638 | chr14 | 37666810 | 2 | N | cg13722431 | chr6 | 2903533 | 19 | MU |
| cg01841758 | chr14 | 37666893 | 2 | N | cg05224741 | chr6 | 10422322 | 19 | MU |
| cg06802658 | chr14 | 37666920 | 2 | N | cg24865270 | chr6 | 10422447 | 19 | N , |
| cg18655441 | chr14 | 37666931 | 2 | MU | cg05873285 | chr6 | 106960491 | 19 | MU |
| cg10644361 | chr14 | 37667230 | 2 | N | cg00564163 | chr7 | 87935979 | 19 | N |
| cg23919479 | chr14 | 37667261 | 2 | MU | cg21227610 | chr7 | 87936078 | 19 | MU |
| cg04925858 | chr14 | 77499547 | 2 | MU | cg12833207 | chr7 | 87936190 | 19 | N |
| cg02971654 | chr14 | 79745343 | 2 | N | cg19430577 | chr7 | 87936225 | 19 | N |
| cg07231787 | chr14 | 79745403 | 2 | N | cg20180843 | chr7 | 100091181 | 19 | N |
| cg21918126 | chr14 | 79745471 | 2 | MU | cg24525457 | chr7 | 100091255 | 19 | MU |
| cg01520867 | chr14 | 104583632 | 2 | MU | cg03113878 | chr7 | 100091364 | 19 | MU |

EP 4 741 515 A2

(continued)

| Illumina Probe Name | Chr. | Position | Tissue/Cell Type | MU/N |
|---|---|---|---|---|
| cg06201680 | chr7 | 151078646 | 19 | MU |
| cg12775656 | chr7 | 151078750 | 19 | N |
| cg02512352 | chr7 | 151137048 | 19 | N |
| cg23273694 | chr7 | 151137162 | 19 | MU |
| cg05551889 | chr7 | 151137192 | 19 | N |
| cg19305111 | chr8 | 142428139 | 19 | MU |
| cg00467244 | chr8 | 142428240 | 19 | MU |
| cg26425321 | chr8 | 142428317 | 19 | N |
| cg09968723 | chr8 | 143545789 | 19 | MU |
| cg14100888 | chr9 | 129445599 | 19 | MU |
| cg05927190 | chr1 | 54203548 | 20 | MU |
| cg17786642 | chr1 | 54203678 | 20 | N |
| cg18533397 | chr1 | 110186005 | 20 | MU |
| cg00924017 | chr1 | 110186027 | 20 | N |
| cg22221025 | chr1 | 110186044 | 20 | N |
| cg25720793 | chr1 | 160983767 | 20 | MU |
| cg08313638 | chr10 | 12110577 | 20 | MU |
| cg00579859 | chr10 | 60026291 | 20 | MU |
| cg10199606 | chr10 | 119494317 | 20 | MU |
| cg19358442 | chr11 | 44325687 | 20 | N |
| cg04549333 | chr11 | 44325823 | 20 | MU |
| cg14144305 | chr11 | 44325960 | 20 | N |
| cg11260848 | chr11 | 44326260 | 20 | N |
| cg07696033 | chr11 | 44326410 | 20 | MU |
| cg25363445 | chr11 | 44326516 | 20 | N |
| cg19520927 | chr11 | 44341646 | 20 | MU |
| cg11656177 | chr11 | 45392729 | 20 | N |
| cg24530812 | chr11 | 45392773 | 20 | MU |
| cg05068461 | chr11 | 45392805 | 20 | N |
| cg26374686 | chr11 | 78357700 | 20 | MU |
| cg21127537 | chr11 | 113184936 | 20 | N |
| cg23461714 | chr11 | 113184990 | 20 | N |
| cg12177743 | chr11 | 113185079 | 20 | MU |
| cg06008480 | chr11 | 113185089 | 20 | N |

| Illumina Probe Name | Chr. | Position | Tissue/Cell | MU/N |
|---|---|---|---|---|
| cg06218726 | chr14 | 104583740 | 2 | N |
| cg27021357 | chr15 | 84115969 | 2 | N |
| cg22946150 | chr15 | 84116107 | 2 | N |
| cg21145136 | chr15 | 84116115 | 2 | MU |
| cg09201151 | chr15 | 84116151 | 2 | N |
| cg21318213 | chr15 | 96875591 | 2 | N |
| cg04330371 | chr15 | 96875656 | 2 | N |
| cg06926934 | chr15 | 96875675 | 2 | MU |
| cg10040836 | chr15 | 96875689 | 2 | N |
| cg00367497 | chr15 | 96875716 | 2 | N |
| cg23042706 | chr15 | 96875781 | 2 | N |
| cg03437748 | chr15 | 99193247 | 2 | MU |
| cg02056682 | chrl6 | 54965084 | 2 | MU |
| cg05870072 | chrI6 | 54965087 | 2 | N |
| cg05362516 | chr16 | 54965089 | 2 | N |
| cg06191898 | chr16 | 54965100 | 2 | N |
| cg09641689 | chr16 | 54965142 | 2 | N |
| cg08974450 | chr16 | 84402160 | 2 | MU |
| cg10282162 | chr17 | 11144311 | 2 | MU |
| cg23953396 | chr17 | 72427941 | 2 | MU |
| cg07910813 | chr18 | 65183745 | 2 | MU |
| cg12975230 | chr18 | 73167671 | 2 | MU |
| cg24218925 | chr19 | 2578938 | 2 | N |
| cg13650938 | chr19 | 2579075 | 2 | MU |
| cg22746789 | chr19 | 2579221 | 2 | N |
| cg15208375 | chr19 | 31841555 | 2 | N |
| cg11199770 | chr19 | 31841663 | 2 | MU |
| cg03287340 | chr19 | 50651195 | 2 | MU |
| cg19145730 | chr2 | 46043 | 2 | MU |
| cg14255256 | chr2 | 46465 | 2 | MU |
| cg01880096 | chr2 | 46558 | 2 | N |
| cg13021841 | chr2 | 14774902 | 2 | N |
| cg05602862 | chr2 | 14775046 | 2 | MU |
| cg11533098 | chr2 | 14775132 | 2 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg03858756 | chr2 | 176994637 | 2 | N | cg27068490 | chr11 | 113185166 | 20 | MU |
| cg15808943 | chr2 | 176994665 | 2 | N | cg21770330 | chr11 | 124616459 | 20 | MU |
| cg24416513 | chr2 | 176994764 | 2 | MU | cg17842066 | chr12 | 7125201 | 20 | MU |
| cg11041835 | chr2 | 189156425 | 2 | MU | cg16600991 | chr12 | 54389477 | 20 | MU |
| cg17931661 | chr2 | 189156549 | 2 | N | cg07541701 | chr12 | 54389934 | 20 | N |
| cg13895867 | chr2 | 237076164 | 2 | N | cg14639847 | chr12 | 54389982 | 20 | MU |
| cg19052355 | chr2 | 237076306 | 2 | MU | cg21837192 | chr12 | 54398765 | 20 | N |
| cg22654039 | chr20 | 11871282 | 2 | N | cg20643952 | chr12 | 54398809 | 20 | MU |
| cg07436991 | chr20 | 11871311 | 2 | N | cg12590051 | chr12 | 54398891 | 20 | N |
| cg14603345 | chr20 | 11871375 | 2 | N | cg05855588 | chr13 | 33590273 | 20 | MU |
| cg23944804 | chr20 | 11871384 | 2 | MU | cg14789645 | chr13 | 98794353 | 20 | N |
| cg12894649 | chr20 | 11871396 | 2 | N | cg10805850 | chr13 | 98794470 | 20 | MU |
| cg04624110 | chr20 | 13976093 | 2 | N | cg00221494 | chr13 | 98794593 | 20 | N |
| cg01552272 | chr20 | 13976096 | 2 | N | cg18912855 | chr14 | 105830859 | 20 | MU |
| cg23169957 | chr20 | 13976106 | 2 | N | cg23855392 | chr15 | 80189782 | 20 | MU |
| cg25557432 | chr20 | 13976117 | 2 | MU | cg19827023 | chr15 | 96957617 | 20 | MU |
| cg04716990 | chr20 | 13976126 | 2 | MU | cg26920902 | chr15 | 96960987 | 20 | MU |
| cg06571075 | chr20 | 13976143 | 2 | N | cg03298437 | chr15 | 96961054 | 20 | N |
| cg26059153 | chr20 | 13976190 | 2 | MU | cg09499248 | chr16 | 103531 | 20 | MU |
| cg23687000 | chr21 | 38071673 | 2 | MU | cg00720747 | chr16 | 4363832 | 20 | MU |
| cg14769207 | chr21 | 38071807 | 2 | N | cg04586579 | chr16 | 82044957 | 20 | N |
| cg16155122 | chr22 | 25201958 | 2 | N | cg21649442 | chr16 | 82044960 | 20 | N |
| cg09097345 | chr22 | 25202086 | 2 | MU | cg10147797 | chr16 | 82045078 | 20 | MU |
| cg14179908 | chr22 | 44319666 | 2 | N | cg02703870 | chr16 | 82045140 | 20 | N |
| cg13184872 | chr22 | 44319719 | 2 | MU | cg03629151 | chr16 | 82045183 | 20 | N |
| cg24333845 | chr3 | 152016806 | 2 | MU | cg04821708 | chr16 | 82045203 | 20 | N |
| cg22079161 | chr4 | 7940579 | 2 | MU | cg01645998 | chrl6 | 82045222 | 20 | N |
| cg04859706 | chr4 | 30721997 | 2 | MU | cg08076158 | chr16 | 86539022 | 20 | MU |
| cg13624385 | chr4 | 30722004 | 2 | N | cg05173913 | chr17 | 34091901 | 20 | MU |
| cg25894334 | chr4 | 77172836 | 2 | MU | cg20245757 | chr17 | 46663759 | 20 | MU |
| cg08073591 | chr4 | 77172841 | 2 | MU | cg20490001 | chr17 | 46663873 | 20 | N |
| cg17559786 | chr4 | 77172861 | 2 | N | cg12846139 | chr17 | 79372242 | 20 | N |
| cg21599230 | chr4 | 118006619 | 2 | MU | cg22230229 | chr17 | 79372254 | 20 | MU |
| cg24183909 | chr4 | 118006747 | 2 | N | cg23291280 | chr19 | 31847970 | 20 | N |

| Illumina Probe Name | Chr. | Position | Tissue e/Cell Type | MU/N |
|---|---|---|---|---|
| cg02128244 | chr19 | 31848008 | 20 | N |
| cg21533331 | chr19 | 31848049 | 20 | MU |
| cg24436715 | chr19 | 31848130 | 20 | N |
| cg19612309 | chr19 | 41112379 | 20 | MU |
| cg17519645 | chr19 | 52206667 | 20 | N |
| cg16854917 | chr19 | 52206691 | 20 | MU |
| cg24879782 | chr2 | 19561482 | 20 | N |
| cg06530338 | chr2 | 19561612 | 20 | MU |
| cg01196788 | chr2 | 19561709 | 20 | N |
| cg23719318 | chr2 | 20865651 | 20 | MU |
| cg19500479 | chr2 | 20865674 | 20 | N |
| cg24539668. | chr2 | 47499521 | 20 | MU |
| cg07669260 | chr2 | 88316847 | 20 | N |
| cg16842767 | chr2 | 88316858 | 20 | N |
| cg15680720 | chr2 | 171568412 | 20 | MU |
| cg20389472 | chr2 | 171568526 | 20 | MU |
| cg27583307 | chr2 | 200320750 | 20 | MU |
| cg07855083 | chr2 | 200320871 | 20 | N |
| cg22973789 | chr2 | 200321011 | 20 | MU |
| cg17204275 | chr2 | 200321136 | 20 | N |
| cg10515731 | chr2 | 220378996 | 20 | MU |
| cg18844301 | chr2 | 220379000 | 20 | N |
| cg17610929 | chr2 | 220379044 | 20 | MU |
| cg24743778 | chr2 | 220379058 | 20 | N |
| cg19210770 | chr2 | 220379091 | 20 | N |
| cg03387930 | chr20 | 56725833 | 20 | N |
| cg22991320 | chr20 | 56725871 | 20 | N |
| cg19155932 | chr20 | 56725873 | 20 | N |
| cg09663111 | chr20 | 56725876 | 20 | N |
| cg25906442 | chr20 | 56725887 | 20 | MU |
| cg07655948 | chr20 | 56725899 | 20 | N |
| cg18562935 | chr20 | 56726000 | 20 | N |
| cg24500900 | chr20 | 61051423 | 20 | N |
| cg08568720 | chr20 | 61051432 | 20 | N |

(continued)

| Illumina Probe Name | Chr. | Position | Tissue/Cell | MU/N |
|---|---|---|---|---|
| cg07837556 | chr4 | 118006750 | 2 | N |
| cg23001131 | chr4 | 152246547 | 2 | MU |
| cg24634420 | chr4 | 157892748 | 2 | MU |
| cg20937139 | chr4 | 157892758 | 2 | N |
| cg22451233 | chr4 | 157892765 | 2 | N |
| cg08712866 | chr4 | 157997526 | 2 | MU |
| cg11163620 | chr4 | 157997554 | 2 | N |
| cg25107254 | chr4 | 187476455 | 2 | N |
| cg08915603 | chr4 | 187476569 | 2 | MU |
| cg09473510 | chr4 | 187476573 | 2 | MU |
| cg06293787 | chr4 | 187476608 | 2 | MU |
| cg12363722 | chr4 | 187476703 | 2 | N |
| cg16248329 | chr4 | 187644739 | 2 | MU |
| cg13658542 | chr4 | 187645059 | 2 | MU |
| cg01244944 | chr4 | 187645118 | 2 | N |
| cg27475132 | chr4 | 187645120 | 2 | MU |
| cg24410535 | chr4 | 187645144 | 2 | N |
| cg24675098 | chr5 | 35617879 | 2 | N |
| cg27003682 | chr5 | 35617945 | 2 | MU |
| cg01581237 | chr5 | 35617969 | 2 | N |
| cg00579717 | chr5 | 170289638 | 2 | MU |
| cg17156565 | chr5 | 174151492 | 2 | N |
| cg25998402 | chr5 | 174151498 | 2 | MU |
| cg12387713 | chr5 | 174151567 | 2 | N |
| cg15123984 | chr5 | 174151634 | 2 | N |
| cg24996482 | chr5 | 174178256 | 2 | MU |
| cg01096398 | chr6. | 1312475 | 2 | MU |
| cg03730958 | chr6 | 1312519 | 2 | N |
| cg27336026 | chr6 | 1312532 | 2 | N |
| cg05685587 | chr6 | 1312577 | 2 | N |
| cg26422161 | chr6 | 1313211 | 2 | MU |
| cg24636336 | chr6 | 19805167 | 2 | MU |
| cg06526693 | chr6 | 19805277 | 2 | N |
| cg17833071 | chr6 | 33084770 | 2 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg23075555 | chr6 | 33084789 | 2 | N | cg18278265 | chr20 | 61051438 | 20 | N |
| cg13524302 | chr6 | 33084798 | 2 | MU | cg02146001 | chr20 | 61051548 | 20 | N |
| cg02662362 | chr6 | 33084814 | 2 | MU | cg23770904 | chr20 | 61051561 | 20 | MU |
| cg24465429 | chr6 | 33084825 | 2 | MU | cg09339194 | chr20 | 61051585 | 20 | N |
| cg24266485 | chr6 | 33084840 | 2 | N | cg00677866 | chr20 | 61051685 | 20 | N |
| cg24185832 | chr6 | 33084888 | 2 | N | cg18872321 | chr20 | 61051751 | 20 | MU |
| cg05337177 | chr6 | 33084928 | 2 | N | cg25667841 | chr20 | 61051762 | 20 | N |
| cg23640002 | chr6 | 33084933 | 2 | N | cg14388488 | chr20 | 61051777 | 20 | N |
| cg22466350 | chr6 | 128841137 | 2 | MU | cg07914822 | chr20 | 61051795 | 20 | N |
| cg17364114 | chr6 | 160769359 | 2 | MU | cg03777459 | chr20 | 61051802 | 20 | N |
| cg07237939 | chr6 | 160769754 | 2 | MU | cg27526665 | chr3 | 24537050 | 20 | N |
| cg16053716 | chr7 | 193438 | 2 | N | cg27622506 | chr3 | 24537160 | 20 | MU |
| cg07615067 | chr7 | 193580 | 2 | MU | cg21303011 | chr3 | 24537177 | 20 | N |
| cg19815904 | chr7 | 80548307 | 2 | N | cg00323305 | chr3 | 24537182 | 20 | MU |
| cg03823904 | chr7 | 80548456 | 2 | MU | cg27388911 | chr3 | 24537277 | 20 | N |
| cg12456681 | chr7 | 89748633 | 2 | N | cg16579438 | chr3 | 24537301 | 20 | N |
| cg24617568 | chr7 | 89748647 | 2 | N | cg25336332 | chr3 | 24537309 | 20 | MU |
| cg05884394 | chr7 | 89748671 | 2 | N | cg12258785 | chr3 | 24537407 | 20 | MU |
| cg03098837 | chr7 | 89748686 | 2 | MU | cg07891533 | chr3 | 45209039 | 20 | N |
| cg04148584 | chr7 | 107643946 | 2 | MU | cg19092164 | chr3 | 45209187 | 20 | MU |
| cg26087411 | chr7 | 107643948 | 2 | MU | cg13153394 | chr3 | 45209196 | 20 | MU |
| cg17657268 | chr7 | 107643956 | 2 | MU | cg15359249 | chr3 | 45209252 | 20 | N |
| cg09284719 | chr7 | 107644024 | 2 | N | cg02704158 | chr3 | 138656042 | 20 | MU |
| cg19101326 | chr8 | 106331160 | 2 | MU | cg24284497 | chr3 | 138656356 | 20 | N |
| cg17154315 | chr8 | 106331166 | 2 | N | cg08031151 | chr3 | 138656471 | 20 | MU |
| cg11585820 | chr8 | 145925948 | 2 | N | cg01711746 | chr3 | 138657291 | 20 | MU |
| cg09979478 | chr8 | 145925997 | 2 | MU | cg07287724 | chr3 | 138657429 | 20 | N |
| cg03134157 | chr8 | 145926089 | 2 | N | cg11023900 | chr3 | 138657666 | 20 | MU |
| cg06671450 | chr9 | 36985987 | 2 | N | cg21530266 | chr3 | 138657774 | 20 | MU |
| cg14317609 | chr9 | 36986006 . | 2 | MU | cg02019774 | chr3 | 138658470 | 20 | N |
| cg14344261 | chr9 | 36986380 | 2 | MU | cg24625136 | chr3 | 138658554 | 20 | MU |
| cg13910860 | chr9 | 36986407 | 2 | MU | cg21575509 | chr3 | 138658704 | 20 | MU |
| cg13685727 | chr9 | 90589530 | 2 | MU | cg23146833 | chr3 | 171175866 | 20 | N |
| cg16357921 | chr9 | 90589634 | 2 | N | cg22877570 | chr3 | 171175950 | 20 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg13920435 | chr9 | 90589640 | 2 | MU | cg26065488 | chr3 | 171176016 | 20 | N |
| cg24163210 | chr1 | 1713944 | 3 | N | cg00859352 | chr3 | 171176909 | 20 | N |
| cg00558749 | chr1 | 1713950 | 3 | N | cg23533254 | chr3 | 171176940 | 20 | MU |
| cg13461447 | chr1 | 1714001 | 3 | MU | cg19631064 | chr3 | 197807355 | 20 | MU |
| cg10207218 | chr1 | 1714140 | 3 | N | cg03241282 | chr3 | 197807491 | 20 | N |
| cg19276111 | chr1 | 24229232 | 3 | MU | cg00677217 | chr4 | 3747401 | 20 | MU |
| cg24452347 | chr1 | 24229300 | 3 | MU | cg05554885 | chr4 | 3747424 | 20 | N |
| cg26404511 | chr1 | 24229575 | 3 | MU | cg01338955 | chr4 | 3747677 | 20 | MU |
| cg27424995 | chr1 | 27902555 | 3 | MU | cg17807131 | chr4 | 4854690 | 20 | MU |
| cg27543538 | chr1 | 27902687 | 3 | N | cg13752974 | chr4 | 4854786 | 20 | N |
| cg06086731 | chr1 | 38942075 | 3 | N | cg05983231 | chr4 | 4856380 | 20 | N |
| cg02983950 | chr1 | 38942220 | 3 | MU | cg15494597 | chr4 | 4856510 | 20 | MU |
| cg04406436 | chr1 | 38942313 | 3 | N | cg06688323 | chr4 | 4858340 | 20 | N |
| cg05388840 | chr1 | 41981911 | 3 | N | cg13332538 | chr4 | 4858482 . | 20 | MU |
| cg12034678 | chr1 | 41982027 | 3 | MU | cg05600350 | chr4 | 4858598 | 20 | MU |
| cg11177980 | chr1 | 41982115 | 3 | N | cg12378888 | chr4 | 4867102 | 20 | MU |
| cg08894594 | chr1 | 62208631 | 3 | MU | cg14872952 | chr4 | 4867164 | 20 | N |
| cg10316635 | chr1 | 62208645 | 3 | MU | cg02680341 | chr4 | 4871525 | 20 | MU |
| cg21279955 | chr1 | 153747551 | 3 | N | cg19403541 | chr4 | 174438312 | 20 | MU |
| cg19773045 | chr1 | 153747600 | 3 | N | cg18644703 | chr5 | 5887068 | 20 | MU |
| cg06239526 | chr1 | 153747609 | 3 | N | cg23094728 | chr5 | 56110263 | 20 | MU |
| cg24408896 | chr1 | 153747658 | 3 | N | cg22736807 | chr5 | 92938149 | 20 | N |
| cg16247817 | chr1 | 153747686 | 3 | MU | cg06820621 | chr5 | 92938232 | 20 | MU |
| cg08623548 | chr1 | 153936729 | 3 | MU | cg14477868 | chr5 | 92938260 | 20 | N |
| cg26008365 | chr1 | 153958797 | 3 | MU | cg25194822 | chr5 | 113699604 | 20 | MU |
| cg06121450 | chr1 | 165798382 | 3 | N | cg11712506 | chr5 | 113699683 | 20 | N |
| cg15473346 | chr1 | 165798420 | 3 | MU | cg25108022 | chr5 | 172199313 | 20 | N |
| cg21161394 | chr1 | 167408709 | 3 | N | cg23002268 | chr5 | 172199318 | 20 | N |
| cg02648847 | chr1 | 167408735 | 3 | N | cg09799633 | chr5 | 172199460 | 20 | MU |
| cg12446199 | chr1 | 167408841 | 3 | MU | cg09493150 | chr5 | 172199605 | 20 | N |
| cg01565130 | chr1 | 167690726 | 3 | MU | cg21570988 | chr6 | 32116841 | 20 | N |
| cg06912814 | chr1 | 201139740 | 3 | N | cg19192280 | chr6 | 32116893 | 20 | N |
| cg27557428 | chr1 | 201139878 | 3 | MU | cg02925367 | chr6 | 32116905 | 20 | MU |
| cg10040748 | chr1 | 211431567 | 3 | MU | cg17626960 | chr6 | 32116918 | 20 | N |

(continued)

| Illumina Probe Name | Chr. | Position | Tissue/Cell | MU/N |
|---|---|---|---|---|
| cg07363543 | chr1 | 224691275 | 3 | MU |
| cg05396897 | chr1 | 247611448 | 3 | N |
| cg09226051 | chr1 | 247611502 | 3 | MU |
| cg03680517 | chr10 | 43632967 | 3 | N |
| cg07178550 | chr10 | 43633030 | 3 | MU |
| cg08388111 | chr10 | 74032820 | 3 | MU |
| cg21344746 | chr10 | 80831230 | 3 | MU |
| cg13954667 | chr10 | 88295472 | 3 | N |
| cg13331196 | chr10 | 88295591 | 3 | MU |
| cg08496387 | chr11 | 11862730 | 3 | N |
| cg12492653 | chr11 | 11862867 | 3 | N |
| cg02501827 | chr11 | 11862879 | 3 | MU |
| cg15846482 | chr11 | 18610557 | 3 | MU |
| cg21272996 | chr11 | 57529255 | 3 | MU |
| cg18213545 | chr11 | 57529400 | 3 | N |
| cg10831212 | chr11 | 57529419 | 3 | MU |
| cg19210276 | chr11 | 57529465 | 3 | MU |
| cg09878888 | chr11 | 57529614 | 3 | N |
| cg15995296 | chr11 | 67210812 | 3 | MU |
| cg26858704 | chr12 | 4918848 | 3 | MU |
| cg24621362 | chr12 | 6492890 | 3 | N |
| cg19476647 | chr12 | 6492948 | 3 | N |
| cg23079808 | chr12 | 6493003 | 3 | MU |
| cg01607849 | chr12 | 6976129 | 3 | MU |
| cg05795313 | chr12 | 48745136 | 3 | MU |
| cg04545296 | chr12 | 48745243 | 3 | N |
| cg10183001 | chrl2 | 51318430 | 3 | N |
| cg13437525 | chr12 | 51318487 | 3 | MU |
| cg22249612 | chr12 | 56121485 | 3 | MU |
| cg02003159 | chr12 | 107711614 | 3 | N |
| cg24124443 | chr12 | 107711648 | 3 | MU |
| cg10421247 | chr12 | 120524653 | 3 | MU |
| cg26406891 | chr12 | 125002940 | 3 | N |
| cg15085899 | chr12 | 125003064 | 3 | MU |

| Illumina Probe Name | Chr. | Position | Tissue e/Cell Type | MU/N |
|---|---|---|---|---|
| cg11945824 | chr6 | 32116963 | 20 | N |
| cg04630292 | chr6 | 32116994 | 20 | N |
| cg24125828 | chr6 | 32117049 | 20 | N |
| cg18111114 | chr6 | 32498493 | 20 | MU |
| cg00587009 | chr6 | 106429129 | 20 | MU |
| cg19509311 | chr6 | 106429191 | 20 | N |
| cg14765959 | chr6 | 106429316 | 20 | MU |
| cg09570682 | chr6 | 106429443 | 20 | MU |
| cg06774797 | chr6 | 132271971 | 20 | MU |
| cg05848863 | chr7 | 16794078 | 20 | MU |
| cg13534901 | chr7 | 17339481 | 20 | MU |
| cg25228422 | chr7 | 19152335 | 20 | MU |
| cg26279021 | chr7 | 19158349 | 20 | MU |
| cg27334919 | chr7 | 19158378 | 20 | N |
| cg16168668 | chr7 | 19158566 | 20 | MU |
| cg14391419 | chr7 | 19158647 | 20 | N |
| cg10126205 | chr7 | 19158664 | 20 | N |
| cg21424940 | chr7 | 19158666 | 20 | MU |
| cg04904385 | chr7 | 19158692 | 20 | MU |
| cg10624122 | chr7 | 19158747 | 20 | MU |
| cg23979631 | chr7 | 27142427 | 20 | N |
| cg24058604 | chr7 | 27142437 | 20 | N |
| cg05921905 | chr7 | 27142527 | 20 | MU |
| cg06769202 | chr7 | 27142535 | 20 | N |
| cg02979457 | chr7 | 27142547 | 20 | N |
| cg22943986 | chr7 | 27142700 | 20 | N |
| cg04737131 | chr7 | 27142708 | 20 | MU |
| cg20087093 | chr7 | 27142799 | 20 | N |
| cg03529432 | chr7 | 27187502 | 20 | MU |
| cg22469274 | chr7 | 27187553 | 20 | N |
| cg14044640 | chr7 | 27187560 | 20 | N |
| cg09936824 | chr7 | 27187670 | 20 | MU |
| cg12110087 | chr7 | 27187691 | 20 | N |
| cg02384857 | chr7 | 27197614 | 20 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg25365958 | chr13 | 42615991 | 3 | MU | cg02000318 | chr7 | 27197713 | 20 | N |
| cg26685941 | chr13 | 95952902 | 3 | MU | cg21035142 | chr7 | 151136940 | 20 | N |
| cg05412028 | chr13 | 95952937 | 3 | MU | cg06288696 | chr7 | 151137008 | 20 | N |
| cg03103888 | chr13 | 114147763 | 3 | MU | cg02512352 | chr7 | 151137048 | 20 | MU |
| cg04542489 | chr14 | 23775746 | 3 | N | cg23273694 | chr7 | 151137162 | 20 | N |
| cg25149155 | chr14 | 23775777 | 3 | N | cg05551889 | chr7 | 151137192 | 20 | N |
| cg12917475 | chr14 | 23775787 | 3 | N | cg11380909 | chr8 | 6658332 | 20 | N |
| cg05877109 | chr14 | 23775794 | 3 | MU | cg26356462 | chr8 | 6658407 | 20 | MU |
| cg18976418 | chr14 | 39735496 | 3 | MU | cg01917069 | chr8 | 6658537 | 20 | N |
| cg14873488 | chr14 | 39735625 | 3 | MU | cg25354466 | chr8 | 65281397 | 20 | N |
| cg23051349 | chr14 | 104094779 | 3 | MU | cg18249580 | chr8 | 65281496 | 20 | MU |
| cg05321721 | chr14 | 104094918 | 3 | N | cg11829072 | chr8 | 125463024 | 20 | MU |
| cg20900050 | chr15 | 31689509 | 3 | N | cg25375711 | chr8 | 125463057 | 20 | N |
| cg12426092 | chr15 | 31689570 | 3 | MU | cg26547947 | chr8 | 125463066 | 20 | N |
| cg20629587 | chr15 | 31689706 | 3 | N | cg13843266 | chr9 | 38069959 | 20 | MU |
| cg02365220 | chr15 | 50474069 | 3 | N | cg06974755 | chr9 | 135996931 | 20 | MU |
| cg25417405 | chr15 | 50474129 | 3 | N | cg10634619 | chr1 | 3663435 | 21 | N |
| cg25150243 | chr15 | 50474159 | 3 | MU | cg15472784 | chr1 | 3663531 | 21 | MU |
| cg25729350 | chrl5 | 58624534 | 3 | MU | cg25467973 | chr1 | 3663705 | 21 | MU |
| cg23600866 | chr15 | 99791109 | 3 | MU | cg05491852 | chr1 | 3663832 | 21 | N |
| cg08640252 | chr16 | 1820949 | 3 | MU | cg16007456 | chr1 | 101004638 | 21 | MU |
| cg06074992 | chr16 | 71598553 | 3 | MU | cg20302133 | chr1 | 111217194 | 21 | MU |
| cg06126721 | chr17 | 1478065 | 3 | MU | cg26013553 | chr1 | 111217406 | 21 | N |
| cg23837623 | chr17 | 1478208 | 3 | N | cg11595545 | chr1 | 111217497 | 21 | MU |
| cg03666441 | chr17 | 7254671 | 3 | MU | cg01423964 | chr1 | 111217575 | 21 | N |
| cg05164926 | chr17 | 7255624 | 3 | MU | cg10055771 | chr1 | 150254869 | 21 | MU |
| cg10479431 | chr17 | 7461421 | 3 | N | cg08293086 | chr1 | 150266477 | 21 | N |
| cg05514680 | chr17 | 7461556 | 3 | MU | cg23819679 | chr1 | 150266488 | 21 | N |
| cg23161218 | chr17 | 7461638 | 3 | N | cg02460371 | chr1 | 150266604 | 21 | MU |
| cg26695387 | chr17 | 21189768 | 3 | N | cg15934674 | chr1 | 150266645 | 21 | N |
| cg15416179 | chr17 | 21189859 | 3 | MU | cg05888917 | chr1 | 156626749 | 21 | MU |
| cg22935450 | chr17 | 38465393 | 3 | N | cg00765922 | chr1 | 156626839 | 21 | N |
| cg03053374 | chr17 | 38465422 | 3 | N | cg17154724 | chr1 | 171810322 | 21 | MU |
| cg00236832 | chr17 | 38465489 | 3 | N | cg04399751 | chr1 | 171810376 | 21 | N |

(continued)

| Illumina Probe Name | Chr. | Position | Tissue/Cell | MU/N | Illumina Probe Name | Chr. | Position | Tissue e/Cell Type | MU/N |
|---|---|---|---|---|---|---|---|---|---|
| cg26654286 | chr17 | 38465510 | 3 | MU | cg04986015 | chr1 | 171810433 | 21 | N |
| cg10409248 | chr17 | 77775073 | 3 | MU | cg02011074 | chr1 | 171810468 | 21 | N |
| cg04173586 | chr19 | 2167496 | 3 | MU | cg09557387 | chr1 | 207818395 | 21 | MU |
| cg27073066 | chr19 | 2169160 | 3 | MU | cg11752769 | chr1 | 207818423 | 21 | N |
| cg17100943 | chr19 | 6459300 | 3 | MU | cg00115980 | chr1 | 207818434 | 21 | N |
| cg13621184 | chr19 | 7459697 | 3 | MU | cg26750487 | chr1 | 207818449 | 21 | MU |
| cg25737313 | chr19 | 12899557 | 3 | MU | cg20744625 | chr10 | 64578469 | 21 | MU |
| cg21869046 | chnl9 | 41225005 | 3 | MU | cg19704726 | chr10 | 94450056 | 21 | MU |
| cg25784219 | chr2 | 28618328 | 3 | MU | cg23727983 | chr11 | 125774082 | 21 | N |
| cg04011671 | chr2 | 43202474 | 3 | MU | cg04609163 | chr11 | 125774090 | 21 | N |
| cg03717364 | chr2 | 43202481 | 3 | MU | cg01736784 | chr11 | 125774092 | 21 | N |
| cg00168785 | chr2 | 160142643 | 3 | MU | cg20559217 | chr11 | 126173516 | 21 | N |
| cg20862669 | chr2 | 160142734 | 3 | N | cg22911867 | chr11 | 126173548 | 21 | N |
| cg03997390 | chr2 | 201170790, | 3 | N | cg13877106 | chr11 | 126173568 | 21 | N |
| cg18609578 | chr2 | 201170806 | 3 | MU | cg01128480 | chr11 | 126173582 | 21 | MU |
| cg15650509 | chr2 | 237476609 | 3 | MU | cg17494733 | chr11 | 126173585 | 21 | N |
| cg14897238 | chr21 | 43198283 | 3 | N | cg17462074 | chr12 | 14133168 | 21 | MU |
| cg21863949 | chr21 | 43198431 | 3 | MU | cg17094171 | chr12 | 14133281 | 21 | N |
| cg27653384 | chr22 | 22293118 | 3 | MU | cg24226973 | chr12 | 14133336 | 21 | N |
| cg25703541 | chr22 | 24373054 | 3 | N | cg13028930 | chr12 | 14133404 | 21 | N |
| cg02953382 | chr22 | 24373134 | 3 | MU | cg17203063 | chr12 | 75601711 | 21 | N |
| cg23595222 | chr22 | 46449430 | 3 | N | cg25820699 | chr12 | 75601803 | 21 | MU |
| cg26394940 | chr22 | 46449461 | 3 | N | cg10148473 | chr12 | 75601824 | 21 | N |
| cg11153400 | chr22 | 46449498 | 3 | MU | cg16370491 | chr12 | 75601928 | 21 | N |
| cg01080924 | chr22 | 46449550 | 3 | N | cg02818811 | chr12 | 75728458 | 21 | MU |
| cg01564818 | chr3 | 9464436 | 3 | MU | cg21087137 | chr12 | 75728469 | 21 | MU |
| cg01870865 | chr3 | 48507087 | 3 | MU | cg24234651 | chr12 | 75728471 . | 21 | MU |
| cg14870461 | chr3 | 69062103 | 3 | MU | cg02478448 | chr13 | 37006063 | 21 | N |
| cg03399783 | chr3 | 69062163 | 3 | N | cg18348647 | chr13 | 37006107 | 21 | MU |
| cg14612335 | chr3 | 170074131 | 3 | MU | cg18948722 | chr13 | 37006116 | 21 | N |
| cg13607248 | chr3 | 183874556 | 3 | MU | cg05137358 | chr13 | 37006127 | 21 | N |
| cg10683341 | chr4 | 39639289 | 3 | MU. | cg23316360 | chr13 | 78492916 | 21 | N |
| cg23651614 | chr4 | 39639327 | 3 | N | cg16571983 | chr13 | 78492922 | 21 | N |
| cg19933595 | chr4 | 146857013 | 3 | MU | cg06179060 | chr13 | 78493012 | 21 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg01894064 | chr4 | 154073371 | 3 | N | cg04390523 | chr13 | 78493100 | 21 | N |
| cg01022974 | chr4 | 154073480 | 3 | MU | cg13818654 | chr13 | 78493126 | 21 | N |
| cg00349895 | chr4 | 154073501 | 3 | MU | cg15836660 | chr13 | 78493128 | 21 | N |
| cg00381131 | chr4 | 154073616 | 3 | N | cg01910869 | chr13 | 78493182 | 21 | N |
| cg15784646 | chr4 | 159707693 | 3 | MU | cg19916212 | chr13 | 78493203 | 21 | N |
| cg19493077 | chr5 | 32613266 | 3 | MU | cg23702615 | chr13 | 78493215 | 21 | N |
| cg19725489 | chr5 | 72252795 | 3 | MU | cg18032190 | chr13 | 78493229 | 21 | MU |
| cg24531022 | chr5 | 102201825 | 3 | MU | cg22310279 | chr13 | 78493232 | 21 | N |
| cg10640944 | chr5 | 102201890 | 3 | N | cg24236409 | chr13 | 78493282 | 21 | MU |
| cg11006267 | chr5 | 139017424 | 3 | MU | cg12602112 | chr13 | 78493294 | 21 | N |
| cg25606201 | chr5 | 180614858 | 3 | MU | cg19650157 | chr13 | 78493297 | 21 | N |
| cg25728685 | chr6 | 28351148 | 3 | MU | cg13434989 | chr13 | 78493305 | 21 | N |
| cg19930737 | chr6 | 44044325 | 3 | MU | cg10016380 | chr13 | 78493313 | 21 | N |
| cg16088539 | chr6 | 44044443 | 3 | N | cg12847373 | chr13 | 78493349 | 21 | N |
| cg20865323 | chr6 | 143999666 | 3 | MU | cg19742055 | chr13 | 78493365 | 21 | N |
| cg22707529 | chr6 | 143999715 | 3 | MU | cg06057566 | chr13 | 78493561 | 21 | N |
| cg15232539 | chr8 | 19459672 | 3 | MU | cg21675115 | chr13 | 78493564 | 21 | N |
| cg15257755 | chr8 | 22225668 | 3 | MU | cg26022015 | chr13 | 78493583 | 21 | N |
| cg00672507 | chr8 | 42997105 | 3 | MU | cg12935136 | chr13 | 78493590 | 21 | N |
| cg14222879 | chr8 | 71316696 | 3 | MU | cg25717994 | chr13 | 78493631 | 21 | N |
| cg11911122 | chr8 | 71316769 | 3 | MU | cg09152886 | chr13 | 78493651 | 21 | MU |
| cg05868799 | chr8 | 71316869 | 3 | N | cg02147695 | chr13 | 78493657 | 21 | MU |
| cg13469590 | chr9 | 19229767 | 3 | MU | cg23494140 | chr13 | 78493671 | 21 | MU |
| cg01447281 | chr1 | 8482689 | 4 | MU | cg07035515 | chr13 | 78493704 | 21 | N |
| cg18660064 | chr1 | 23504632 | 4 | MU | cg09357740 | chr14 | 21131543 | 21 | N |
| cg24442740 | chr1 | 27902069 | 4 | MU | cg22895601 | chr14 | 21131621 | 21 | MU |
| cg27424995 | chr1 | 27902555 | 4 | MU | cg21915647 | chr14 | 21439301 | 21 | MU |
| cg27543538 | chr1 | 27902687 | 4 | N | cg09790137 | chr14 | 21439440 | 21 | MU |
| cg01018002 | chr1 | 28844479 | 4 | MU | cg10924691 | chr14 | 21439548 | 21 | MU |
| cg02447380 | chr1 | 28919300 | 4 | MU | cg19818764 | chr14 | 21439700 | 21 | MU |
| cg02525756 | chr1 | 28919362 | 4 | N | cg10960632 | chr14 | 21439883 | 21 | MU |
| cg24118052 | chr1 | 28919387 | 4 | N | cg12606485 | chr14 | 21439907 | 21 | N |
| cg22174844 | chr1 | 39570747 | 4 | MU | cg03419058 | chr15 | 26108391 | 21 | MU |
| cg05388840 | chr1 | 41981911 | 4 | N | cg16389285 | chr15 | 26108399 | 21 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg12034678 | chr1 | 41982027 | 4 | MU | cg20124450 | chr15 | 26108401 | 21 | N |
| cg11177980 | chr1 | 41982115 | 4 | N | cg22113930 | chr15 | 26108410 | 21 | N |
| cg24312719 | chr1 | 93251184 | 4 | MU | cg26230285 | chr15 | 26108412 | 21 | MU |
| cg17140307 | chr1 | 93251203 | 4 | N | cg21711132 | chr15 | 27215952 | 21 | N |
| cg11226183 | chr1 | 93251218 | 4 | N | cg02069715 | chr15 | 27215962 | 21 | N |
| cg08978953 | chr1 | 120190520 | 4 | N | cg09257796 | chr15 | 27215971 | 21 | N |
| cg09266149 | chr1 | 120190550 | 4 | MU | cg03024760 | chr15 | 27215997 | 21 | N |
| cg27431596 | chr1 | 120190588 | 4 | N | cg02281208 | chr15 | 27216008 | 21 | N |
| cg08623548 | chr1 | 153936729 | 4 | MU | cg08182446 | chr15 | 27216012 | 21 | N |
| cg06121450 | chr1 | 165798382 | 4 | N | cg27553667 | chr15 | 27216083 | 21 | N |
| cg15473346 | chr1 | 165798420 | 4 | MU | cg12919520 | chr15 | 27216088 | 21 | MU |
| cg21161394 | chr1 | 167408709 | 4 | N | cg17741501 | chrl5 | 29077493 | 21 | MU |
| cg02648847 | chr1 | 167408735 | 4 | N | cg14910265 | chr15 | 45670406 | 21 | N |
| cg12446199 | chr1 | 167408841 | 4 | MU | cg11431346 | chr15 | 45670478 | 21 | N |
| cg06912814 | chr1 | 201139740 | 4 | N | cg01145430 | chr15 | 45670526 | 21 | MU |
| cg27557428 | chr1 | 201139878 | 4 | MU | cg25908973 | chr15 | 45670865 | 21 | MU |
| cg06528575 | chr1 | 207082828 | 4 | N | cg10088041 | chr15 | 45670872 | 21 | MU |
| cg00008647 | chr1 | 207082900 | 4 | MU | cg00645339 | chr15 | 45670875 | 21 | MU |
| cg08360820 | chr10 | 11652640 | 4 | MU | cg26796135 | chr15 | 45671001 | 21 | N |
| cg14419424 | chr10 | 65388604 | 4 | MU | cg24328539 | chr15 | 45671016 | 21 | N |
| cg22620363 | chr10 | 80826794 | 4 | MU | cg05896902 | chr15 | 45671018 | 21 | N |
| cg21344746 | chr10 | 80831230 | 4 | MU | cg20973720 | chr15 | 79383167 | 21 | MU |
| cg25011475 | chr10 | 88517608 | 4 | MU | cg17360233 | chr16 | 10479841 | 21 | N |
| cg22074576 | chr11 | 3181726 | 4 | MU | cg06754445 | chr16 | 10479875 | 21 | N |
| cg11678461 | chr11 | 3181822 | 4 | N | cg05064297 | chr16 | 10479966 | 21 | MU |
| cg23357130 | chr11 | 3181928 | 4 | MU | cg03131298 | chr16 | 31580889 | 21 | MU |
| cg04717837 | chr11 | 3225004 | 4 | N | cg04764012 | chr16 | 31580964 | 21 | N |
| cg03347559 | chr11 | 3225059 | 4 | MU | cg26946978 | chr16 | 81779881 | 21 | MU |
| cg11496778 | chr11 | 3225076 | 4 | N | cg27595997 | chr16 | 81779999 | 21 | MU |
| cg08635931 | chr11 | 10880635 | 4 | MU | cg12855166 | chr17 | 30846586 | 21 | MU |
| cg10879244 | chr11 | 64577190 | 4 | N | cg20668644 | chr17 | 38347603 | 21 | MU |
| cg22897141 | chr11 | 64577338 | 4 | MU | cg19827780 | chr17 | 38347659 | 21 | N |
| cg08092930 | chr11 | 70117714 | 4 | MU | cg00129651 | chrl7 | 38347710 | 21 | N |
| cg04983516 | chr11 | 79151719 | 4 | MU | cg11155697 | chr17 | 46671234 | 21 | N |

(continued)

| Illumina Probe Name | Chr. | Position | Tissue/Cell | MU/N |
|---|---|---|---|---|
| cg25965355 | chr11 | 79151966 | 4 | N |
| cg12246510 | chr11 | 79152102 | 4 | MU |
| cg02409108 | chr11 | 79152112 | 4 | N |
| cg20275558 | chr11 | 85338473 | 4 | MU |
| cg11758932 | chr11 | 109964976 | 4 | MU |
| cg26426447 | chr11 | 118134959 | 4 | N |
| cg09033131 | chr11 | 118135094 | 4 | MU |
| cg16594165 | chr11 | 118135105 | 4 | N |
| cg07315693 | chr11 | 118135203 | 4 | N |
| cg03963011 | chr11 | 128418551 | 4 | N |
| cg25244476 | chr11 | 128418692 | 4 | MU |
| cg01084404 | chr12 | 48214523 | 4 | MU |
| cg22249612 | chr12 | 561121485 | 4 | MU |
| cg24235581 | chr12 | 58290452 | 4 | MU |
| cg15109018 | chr12 | 85862615 | 4 | MU |
| cg08273772 | chr12 | 112035212 | 4 | N |
| cg06923988 | chr12 | 112035285 | 4 | MU |
| cg22529546 | chr12 | 112819040 | 4 | N |
| cg11287899 | chr12 | 112819167 | 4 | MU |
| cg15023427 | chr12 | 112819249 | 4 | N |
| cg26406891 | chr12 | 125002940 | 4 | N |
| cg15085899 | chr12 | 125003064 | 4 | MU |
| cg03268893 | chr13 | 27557503 | 4 | MU |
| cg11829872 | chr14 | 31494210 | 4 | MU |
| cg05081614 | chr14 | 31494237 | 4 | N |
| cg26066349 | chr14 | 31675363 | 4 | MU |
| cg00841968 | chr14 | 69446664 | 4 | MU |
| cg16256390 | chr14 | 99712336 | 4 | N |
| cg27043010 | chr14 | 99712380 | 4 | MU |
| cg02256631 | chr16 | 313342952 | 4 | MU |
| cg15817542 | chr16 | 31343056 | 4 | N |
| cg01972875 | chr16 | 67563389 | 4 | MU |
| cg02225847 | chr16 | 67563418 | 4 | N |
| cg26604214 | chr17 | 2907678 | 4 | MU |

| Illumina Probe Name | Chr. | Position | Tissue e/Cell Type | MU/N |
|---|---|---|---|---|
| cg02712075 | chr17 | 46671244 | 21 | N |
| cg10153335 | chr17 | 46671293 | 21 | N |
| cg02293936 | chr17 | 46671298 | 21 | N |
| cg24626312 | chr17 | 46671332 | 21 | MU |
| cg13216267 | chr18 | 77793342 | 21' | MU |
| cg02401399 | chr19 | 36523584 | 21 | MU |
| cg24027100 | chr19 | 36822549 | 21 | N |
| cg21808240 | chr19 | 36822564 | 21 | N |
| cg02810156 | chr19 | 36822672 | 21 | MU |
| cg22456785 | chr19 | 36822770 | 21 | N |
| cg02587316 | chr19 | 37096321 | 21 | N |
| cg05020604 | chr19 | 37096329 | 21 | MU |
| cg07602980 | chr19 | 50815719 | 21 | MU |
| cg08063125 | chr19 | 56989543 | 21 | MU |
| cg03252986 | chr19 | 57751591 | 21 | N |
| cg02446106 | chr19 | 57751691 | 21 | MU |
| cg02653557 | chr19 | 58095424 | 21 | N |
| cg18435449 | chr19 | 58095445 | 21 | MU |
| cg04342092 | chr19 | 58095468 | 21 | N |
| cg00800512 | chr19 | 58095518 | 21 | N |
| cg12060744 | chr19 | 58095581 | 21 | MU |
| cg01046104 | chr19 | 58095588 | 21 | MU |
| cg18579862 | chr19 | 58095595 | 21 | MU |
| cg26246807 | chr19 | 58095659 | 21 | N |
| cg05661282 | chr19 | 58220370 | 21 | MU |
| cg21790626 | chr19 | 58220494 | 21 | MU |
| cg27049766 | chr19 | 58220516 | 21 | N |
| cg12074025 | chr19 | 58238850 | 21 | MU |
| cg19246110 | chr19 | 58238928 | 21 | N |
| cg11977686 | chr19 | 58238987 | 21 | MU |
| cg07474494 | chr19 | 58446600 | 21 | N |
| cg11998703 | chr19 | 58446669 | 21 | MU |
| cg13668618 | chr19 | 58446745 | 21 | N |
| cg12961842 | chr19 | 58446758 | 21 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg03666441 | chr17 | 7254671 | 4 | MU | cg11788523 | chr19 | 58446770 | 21 | N |
| cg04267691 | chr17 | 9862752 | 4 | N | cg15060012 | chr19 | 58446783 | 21 | N |
| cg12697325 | chr17 | 9862763 | 4 | N | cg21444693 | chr19 | 58446787 | 21 | N |
| cg26996616 | chr17 | 9862873 | 4 | N | cg18673377 | chr19 | 58446898 | 21 | N |
| cg12833931 | chr17 | 9862893 | 4 | N | cg10659886 | chr19 | 58629719 | 21 | N |
| cg17192115 | chr17 | 9862900 | 4 | MU | cg08873805 | chr19 | 58629837 | 21 | MU |
| cg06377635 | chr17 | 9862924 | 4 | N | cg14231297 | chr19 | 58629901 | 21 | N |
| cg11270042 | chr17 | 26219919 | 4 | MU | cg17408527 | chr19 | 58629967 | 21 | N |
| cg26785617 | chr17 | 26219967 | 4 | N | cg18693673 | chr19 | 58629975 | 21 | N |
| cg05041073 | chr17 | 37774893 | 4 | MU | cg01354088 | chr2 | 10183140 | 21 | N |
| cg21936280 | chr17 | 37774934 | 4 | N | cg23458613 | chr2 | 10183227 | 21 | N |
| cg11183072 | chr17 | 37894397 | 4 | MU | cg12005760 | chr2 | 10183271 | 21 | MU |
| cg13761998 | chr17 | 37894403 | 4 | N | cg07285673 | chr2 | 29337984 | 21 | N |
| cg17740645 | chr17 | 37894413 | 4 | N | cg09695033 | chr2 | 29337988 | 21 | N |
| cg13784792 | chr17 | 39942930 | 4 | N | cg06333058 | chr2 | 29338077 | 21 | N |
| cg18733255 | chr17 | 39942970 | 4 | MU | cg25737323 | chr2 | 29338100 | 21 | MU |
| cg05768702 | chr17 | 47271133 | 4 | N | cg13257636 | chr2 | 29338109 | 21 | N |
| cg20243351 | chr17 | 47271156 | 4 | MU | cg23428985 | chr2 | 29338113 | 21 | MU |
| cg03384992 | chr18 | 21852325 | 4 | MU | cg23255835 | chr2 | 29338121 | 21 | N |
| cg26398656 | chr18 | 21852327 | 4 | N | cg03135351 | chr2 | 29338258 | 21 | MU |
| cg06593258 | chr18 | 21852333 | 4 | N | cg08808128 | chr2 | 29338432 | 21 | MU |
| cg21840153 | chr18 | 21852436 | 4 | N | cg01648237 | chr2 | 61372066 | 21 | N |
| cg08894153 | chr19 | 12606729 | 4 | MU | cg05037927 | chr2 | 61372117 | 21 | N |
| cg05022378 | chr19 | 12606767 | 4 | N | cg16328106 | chr2 | 61372138 | 21 | MU |
| cg12150039 | chr19 | 35490532 | 4 | MU | cg19790321 | chr2 | 61372165 | 21 | N |
| cg22045942 | chr19 | 35490561 | 4 | N | cg24757310 | chr2 | 61372226 | 21 | MU |
| cg12412075 | chr19 | 49618425 | 4 | MU | cg18158151 | chr2 | 61372256 | 21 | MU |
| cg03630273 | chr19 | 49618499 | 4 | N | cg21185289 | chr2 | 74743437 | 21 | MU |
| cg11284196 | chr19 | 51190047 | 4 | N | cg04380340 | chr2 | 201450506 | 21 | N |
| cg09284949 | chr19 | 51190179 | 4 | MU | cg13000082 | chr2 | 201450527 | 21 | N |
| cg19358877 | chr19 | 57019279 | 4 | MU | cg08266417 | chr2 | 201450575 | 21 | MU |
| cg24713204 | chr19 | 57019373 | 4 | N | cg09729613 | chr2 | 201450601 | 21 | MU |
| cg24152605 | chr19 | 57050359 | 4 | MU | cg08952506 | chr2 | 201450610 | 21 | N |
| cg22054362 | chr19 | 57050367 | 4 | MU | cg02144933 | chr2 | 201450690 | 21 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg21970554 | chr19 | 57050370 | 4 | MU | cg12627583 | chr2 | 201450731 | 21 | N |
| cg06243556 | chr19 | 58609473 | 4 | N | cg13875120 | chr2 | 201450743 | 21 | N |
| cg25784220 | chr19 | 58609602 | 4 | MU | cg14373923 | chr20 | 61638433 | 21 | N |
| cg22721334 | chr19 | 58609618 | 4 | N | cg01699217 | chr20 | 61638488 | 21 | N |
| cg22031998 | chr19 | 58609730 | 4 | N | cg23806621 | chr20 | 61638501 | 21 | N |
| cg18428688 | chr19 | 58609744 | 4 | N | cg14060496 | chr20 | 61638518 | 21 | N |
| cg19391498 | chr2 | 38892846 | 4 | N | cg26492446 | chr20 | 61638574 | 21 | MU |
| cg05275752 | chr2 | 38892887 | 4 | N | cg27501878 | chr20 | 61638588 | 21 | N |
| cg01546378 | chr2 | 38892901 | 4 | N | cg23867997 | chr20 | 61810624 | 21 | MU |
| cg12043428 | chr2 | 38892958 | 4 | MU | cg17353354 | chr21 | 46707012 | 21 | MU |
| cg08308459 | chr2 | 38893161 | 4 | N | cg25562664 | chr3 | 119529148 | 21 | N |
| cg05604112 | chr2 | 38893237 | 4 | N | cg24129382 | chr3 | 119529158 | 21. | MU |
| cg23643435 | chr2 | 38893251 | 4 | MU | cg24846416 | chr3 | 119529290 | 21 | N |
| cg19286686 | chr2 | 158731699 | 4 | N | cg18780412 | chr3 | 179755086 | 21 | MU |
| cg23967461 | chr2 | 158731736 | 4 | MU | cg21176048 | chr3 | 179755235 | 21 | N |
| cg01943873 | chr2 | 158731796 | 4 | MU | cg03405515 | chr4 | 156297854 | 21 | MU |
| cg22654039 | chr20 | 11871282 | 4 | N | cg03616357 | chr4 | 156297942 | 21 | N |
| cg07436991 | chr20 | 11871311 | 4 | N | cg20334243 | chr4 | 156298002 | 21 | N |
| cg14603345 | chr20 | 11871375 | 4 | N | cg18323912 | chr5 | 42994709 | 21 | N |
| cg23944804 | chr20 | 11871384 | 4 | MU | cg04223424 | chr5 | 42994776 | 21 | MU |
| cg12894649 | chr20 | 11871396 | 4 | N | cg18110165 | chr5 | 49736988 | 21 | MU |
| cg26308477 | chr20 | 44065479 | 4 | MU | cg03179043 | chr5 | 49737178 | 21 | N |
| cg10546252 | chr22 | 24384294 | 4 | N | cg08722065 | chr5 | 49737182 | 21 | MU |
| cg24238852 | chr22 | 24384340 | 4 | N | cg02344943 | chr5 | 49737236 | 21 | MU |
| cg11478607 | chr22 | 24384400 | 4 | MU | cg18617005 | chr5 | 140787504 | 21 | N |
| cg09805010 | chr3 | 24536077 | 4 | MU | cg18507379 | chr5 | 140787507 | 21 | MU |
| cg02333852 | chr3 | 24536252 | 4 | MU | cg09465698 | chr5 | 140787623 | 21 | N |
| cg13279811 | chr3 | 24725365 | 4 | MU | cg13933262 | chr5 | 140797172 | 21 | MU |
| cg22306344 | chr3 | 49966773 | 4 | MU | cg14011639 | chr5 | 140797234 | 21 | N |
| cg12349858 | chr3 | 160822545 | 4 | MU | cg10435816 | chr5 | 140797280 | 21 | N |
| cg13607248 | chr3 | 183874556 | 4 | MU | cg23563234 | chr5 | 140797286 | 21 | N |
| cg08376864 | chr3 | 192958726 | 4 | N | cg10723962 | chr6 | 26240782 | 21 | MU |
| cg24710320 | chr3 | 192958832 | 4 | MU | cg27131891 | chr6 | 27513414 | 21 | N |
| cg22913249 | chr4 | 41215979 | 4 | MU | cg05310764 | chr6 | 27513479 | 21 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg18587806 | chr4 | 41362843 | 4 | MU | cg11623922 | chr6 | 27759386 | 21 | MU |
| cg26248284 | chr4 | 41362853 | 4 | N | cg17438737 | chr6 | 27759399 | 21 | N |
| cg17187287 | chr4 | 115519593 | 4 | MU | cg24799187 | chr6 | 27759410 | 21 | N |
| cg09802389 | chr4 | 122617307 | 4 | MU | cg17512365 | chr6 | 29760090 | 21 | N |
| cg01803059 | chr4 | 146402404 | 4 | N | cg13688808 | chr6 | 29760158 | 21 | N |
| cg02971575 | chr4 | 146402540 | 4 | MU | cg17394649 | chr6 | 29760164 | 21 | MU |
| cg18485193 | chr5 | 16936369 | 4 | MU | cg18055610 | chr6 | 30095419 | 21 | N |
| cg17942573 | chr5 | 16936391 | 4 | N | cg19059495 | chr6 | 30095495 | 21 | N |
| cg07705810 | chr5 | 16936470 | 4 | N | cg01964152 | chr6 | 30095546 | 21 | N |
| cg19725489 | chr5 | 72252795 | 4 | MU | cg08548396 | chr6 | 30095549 | 21 | MU |
| cg13904970 | chr5 | 123987667 | 4 | MU | cg12534216 | chr6 | 30095570 | 21 | N |
| cg25728685 | chr6 | 28351148 | 4 | MU | cg20383155 | chr6 | 31831428 | 21 | N |
| cg23197007 | chr6 | 30655617 | 4 | N | cg18363192 | chr6 | 31831434 | 21 | N |
| cg13357602 | chr6 | 30655720 | 4 | MU | cg27347290 | chr6 | 31831439 | 21 | N |
| cg22501342 | chr6 | 37322729 | 4 | N | cg22753340 | chr6 | 31831489 | 21 | N |
| cg24691069 | chr6 | 37322773 | 4 | MU | cg23174932 | chr6 | 31831502 | 21 | MU |
| cg19478500 | chr6 | 125284655 | 4 | MU | cg00397479 | chr6 | 31831510 | 21 | N |
| cg16203203 | chr6 | 125284659 | 4 | N | cg17353698 | chr6 | 31831599 | 21 | N |
| cg19033466 | chr6 | 144608035 | 4 | N | cg18857062 | chr6 | 43276478 | 21 | N |
| cg17772171 | chr6 | 144608178 | 4 | MU | cg11982736 | chr6 | 43276570 | 21 | N |
| cg23833452 | chr6 | 146864752 | 4 | N | cg27291698 | chr6 | 43276578 | 21 | MU |
| cg10766475 | chr6 | 146864781 | 4 | N | cg21109265 | chr6 | 43276647 | 21 | N |
| cg26252281 | chr6 | 146864885 | 4 | MU | cg13097407 | chr6 | 43276658 | 21 | N |
| cg10028217 | chr7 | 122526824 | 4 | N | cg07544038 | chr6 | 71122681 | 21 | MU |
| cg26316023 | chr7 | 122526826 | 4 | N | cg25599475 | chr7 | 853038 | 21 | MU |
| cg06159603 | chr7 | 122526836 | 4 | N | cg17314888 | chr7 | 73442282 | 21 | N |
| cg05293994 | chr7 | 122526843 | 4 | MU | cg03897712 | chr7 | 73442370 | 21 | MU |
| cg19809988 | chr7 | 149569883 | 4 | N | cg23686014 | chr7 | 93519924 | 21 | N |
| cg12691229 | chr7 | 149569946 | 4 | MU | cg17338208 | chr7 | 93520024 | 21 | N |
| cg00753630 | chr7 | 149569987 | 4 | N | cg16934178 | chr7 | 93520074 | 21 | MU |
| cg23903252 | chr7 | 149569998 | 4 | N | cg16762778 | chr8 | 27449867 | 21 | MU |
| cg06961439 | chr7 | 149570036 | 4 | N | cg08970347 | chr8 | 27449907 | 21 | MU |
| cg27527018 | chr7 | 149570040 | 4 | N | cg24592790 | chr8 | 27450047 | 21 | N |
| cg21602557 | chr8 | 119122878 | 4 | MU | cg14907769 | chr8 | 49468941 | 21 | N |

(continued)

| Illumina Probe Name | Chr. | Position | Tissue/Cell | MU/N |
|---|---|---|---|---|
| cg14221550 | chr8 | 120220050 | 4 | N |
| cg09273054 | chr8 | 120220131 | 4 | MU |
| cg11497957 | chr8 | 125985846 | 4 | MU |
| cg12738197 | chr8 | 125985883 | 4 | N |
| cg05022789 | chr8 | 135469681 | 4 | N |
| cg01919374 | chr8 | 135469790 | 4 | MU |
| cg13469590 | chr9 | 19229767 | 4 | MU |
| cg14247332 | chr9 | 132805979 | 4 | MU |
| cg24442740 | chr1 | 27902069 | 5 | MU |
| cg27424995 | chr1 | 27902555 | 5 | MU |
| cg27543538 | chr1 | 27902687 | 5 | N |
| cg02447380 | chr1 | 28919300 | 5 | MU |
| cg02525756 | chr1 | 28919362 | 5 | N |
| cg24118052 | chr1 | 28919387 | 5 | N |
| cg05388840 | chr1 | 41981911 | 5 | N |
| cg12034678 | chr1 | 41982027 | 5 | MU |
| cg11177980 | chr1 | 41982115 | 5 | N |
| cg24312719 | chr1 | 93251184 | 5 | MU |
| cg17140307 | chr1 | 93251203 | 5 | N |
| cg11226183 | chr1 | 93251218 | 5 | N |
| cg08978953 | chr1 | 120190520 | 5 | MU |
| cg09266149 | chr1 | 120190550 | 5 | N |
| cg27431596 | chr1 | 120190588 | 5 | MU |
| cg18086761 | chr1 | 151763997 | 5 | N |
| cg20686828 | chr1 | 151764147 | 5 | MU |
| cg11411904 | chr1 | 153935719 | 5 | MU |
| cg08623548 | chr1 | 153936729 | 5 | N |
| cg06121450 | chr1 | 165798382 | 5 | MU |
| cg15473346 | chr1 | 165798420 | 5 | N |
| cg21161394 | chr1 | 167408709 | 5 | N |
| cg02648847 | chr1 | 167408735 | 5 | MU |
| cg12446199 | chr1 | 167408841 | 5 | MU |
| cg00909514 | chr1 | 200860534 | 5 | MU |
| cg08360820 | chr10 | 11652640 | 5 | MU |

| Illumina Probe Name | Chr. | Position | Tissue e/Cell Type | MU/N |
|---|---|---|---|---|
| cg10826197 | chr8 | 49468988 | 21 | MU |
| cg15467646 | chr1 | 22141014 | 22 | MU |
| cg27510832 | chr1 | 76080684 | 22 | N |
| cg07923233 | chr1 | 76080727 | 22 | MU |
| cg27547954 | chr1 | 76081962 | 22 | MU |
| cg16966815 | chr1 | 77333122 | 22 | N |
| cg13823136 | chr1 | 77333138 | 22 | N |
| cg13463054 | chr1 | 77333159 | 22 | MU |
| cg06201642 | chr1 | 77333198 | 22 | N |
| cg14421860 | chr1 | 101004934 | 22 | MU |
| cg27297088 | chr1 | 179712158 | 22 | N |
| cg09683824 | chr1 | 179712191 | 22 | N |
| cg00181968 | chr1 | 179712204 | 22 | MU |
| cg00962913 | chr1 | 179712281 | 22 | N |
| cg13449778 | chr1 | 179712298 | 22 | MU |
| cg00662647 | chr1 | 234350051 | 22 | N |
| cg27041794 | chr10 | 11059708 | 22 | N |
| cg17290701 | chr10 | 11059714 | 22 | MU |
| cg26328510 | chr10 | 11059718 | 22 | MU |
| cg03813164 | chr10 | 11059725 | 22 | N |
| cg12356890 | chr10 | 11059727 | 22 | MU |
| cg11472279 | chr10 | 11059733 | 22 | MU |
| cg14649650 | chr10 | 13933996 | 22 | MU |
| cg16397794 | chr10 | 28032282 | 22 | MU |
| cg26664871 | chr10 | 28032370 | 22 | MU |
| cg17339505 | chr10 | 43572621 | 22 | MU |
| cg06048524 | chr10 | 44880542 | 22 | N |
| cg17267805 | chr10 | 44880545 | 22 | N |
| cg26267854 | chr10 | 44880562 | 22 | MU |
| cg19235339 | chr10 | 90342899 | 22 | N |
| cg05339066 | chr10 | 90343166 | 22 | N |
| cg10131095 | chr10 | 90343174 | 22 | MU |
| cg06118999 | chr10 | 90343192. | 22 | N |
| cg18192294 | chr10 | 90343204 | 22 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg22620363 | chr10 | 80826794 | 5 | MU | cg06981182 | chr10 | 90343208 | 22 | N |
| cg21344746 | chr10 | 80831230 | 5 | MU | cg11861709 | chr10 | 90343220 | 22 | N |
| cg25011475 | chr10 | 88517608 | 5 | MU | cg06079710 | chr10 | 90343285 | 22 | N |
| cg22074576 | chr11 | 3181726 | 5 | MU | cg24680586 | chr10 | 105036727 | 22 | N |
| cg11678461 | chr11 | 3181822 | 5 | N | cg23003534 | chr10 | 105036747. | 22 | N |
| cg23357130 | chr11 | 3181928 | 5 | MU | cg17567560 | chr10 | 105036863 | 22 | MU |
| cg04717837 | chr11 | 3225004 | 5 | N | cg08521987 | chr10 | 119000927 | 22 | N |
| cg03347559 | chr11 | 3225059 | 5 | MU | cg00512279 | chr10 | 119000991 | 22 | N |
| cg11496778 | chr11 | 3225076 | 5 | N | cg19721867 | chr10 | 119001066 | 22 | MU |
| cg18213545 | chr11 | 57529400 | 5 | N | cg03908015 | chr10 | 124895441 | 22 | N |
| cg10831212 | chr11 | 57529419 | 5 | MU | cg18685408 | chr10 | 124895448 | 22 | MU |
| cg19210276 | chr11 | 57529465 | 5 | N | cg14663510 | chr10 | 124895461 | 22 | N |
| cg03101664 | chr11 | 64794610 | 5 | N | cg00560482 | chr10 | 124895474 | 22 | N |
| cg24585346 | chr11 | 64794741 | 5 | N | cg06897927 | chr10 | 124895500 | 22 | N |
| cg19146924 | chr11 | 64794755 | 5 | MU | cg20403498 | chr10 | 124895693 | 22 | MU |
| cg06065225 | chr11 | 65640137 | 5 | MU | cg19564098 | chr10 | 124895697 | 22 | MU |
| cg08092930 | chr11 | 70117714 | 5 | MU | cg21965997 | chr10 | 135150367 | 22 | N |
| cg04983516 | chr11 | 79151719 | 5 | MU | cg14614314 | chr10 | 135150428 | 22 | MU |
| cg26426447 | chr11 | 118134959 | 5 | N | cg13334650 | chr11 | 6440065 | 22 | MU |
| cg09033131 | chr11 | 118135094 | 5 | MU | cg12740527 | chr11 | 8289974 | 22 | MU |
| cg16594165 | chr11 | 118135105 | 5 | N | cg04261192 | chr11 | 20691341 | 22 | N |
| cg07315693 | chr11 | 118135203 | 5 | N | cg01563031 | chr11 | 20691429 | 22 | MU |
| cg26202340 | chr11 | 126152357 | 5 | N | cg16624692 | chr11 | 65601301 | 22 | MU |
| cg02305850 | chr11 | 126152462 | 5 | N | cg19018097 | chr11 | 65601328 | 22 | N |
| cg02973469 | chr11 | 126152481 | 5 | MU | cg11198128 | chr11 | 65601332 | 22 | MU |
| cg03963011 | chr11 | 128418551 | 5 | N | cg04628008 | chr11 | 113931000 | 22 | MU |
| cg25244476 | chr11 | 128418692 | 5 | MU | cg21521683 | chr12 | 22487682 | 22 | MU |
| cg20645074 | chr12 | 49182479 | 5 | MU | cg07017374 | chr13 | 28674451 | 22 | MU |
| cg24235581 | chr12 | 58290452 | 5 | MU | cg27398263 | chr13 | 79177700 | 22 | MU |
| cg06003296 | chr12 | 71003681 | 5 | MU | cg09010671 | chr13 | 79177763 | 22 | N |
| cg02003159 | chr12 | 107711614 | 5 | N | cg26110710 | chr13 | 88323607 | 22 | MU |
| cg24124443 | chr12 | 107711648 | 5 | MU | cg17194154 | chr13 | 95359974 | 22 | MU |
| cg08273772 | chr12 | 11203522 | 5 | N | cg00756058 | chr13 | 96296844 | 22 | MU |
| cg06923988 | chr12 | 112035285 | 5 | MU | cg26886381 | chr13 | 96296979 | 22 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg22529546 | chr12 | 112819040 | 5 | N | cg02788400 | chr13 | 96296983 | 22 | N |
| cg11287899 | chr12 | 112819167 | 5 | MU | cg25277187 | chr14 | 52535028 | 22 | N |
| cg15023427 | chr12 | 112819249 | 5 | N | cg14897833 | chr14 | 52535178 | 22 | MU |
| cg10421247 | chr12 | 120524653 | 5 | MU | cg13592399 | chr14 | 52535758 | 22 | MU |
| cg26406891 | chr12 | 125002940 | 5 | N | cg12686317 | chr15 | 55880894 | 22 | N |
| cg15085899 | chr12 | 125003064 | 5 | MU | cg19850348 | chr15 | 55880997 | 22 | MU |
| cg27485716 | chr12 | 133286734 | 5 | MU | cg25902187 | chr15 | 55881044 | 22 | N |
| cg25365958 | chr13 | 42615991 | 5 | MU | cg11480800 | chr15 | 55881075 | 22 | N |
| cg26685941 | chr13 | 95952902 | 5 | N | cg23030278 | chr15 | 55881081 | 22 | N |
| cg05412028 | chr13 | 95952937 | 5 | MU | cg09275667 | chr15 | 55881083 | 22 | N |
| cg18641486 | chr14 | 37641660 | 5 | N | cg17841803 | chr15 | 55881089 | 22 | N |
| cg23291305 | chr14 | 37641726 | 5 | MU | cg08769966 | chr15 | 92937735 | 22 | MU |
| cg18976418 | chr14 | 39735496 | 5 | N | cg03217795 | chr16 | 23847556 | 22 | MU |
| cg14873488 | chr14 | 39735625 | 5 | MU | cg05436658 | chr16 | 23847568 | 22 | N |
| cg00841968 | chr14 | 69446664 | 5 | MU | cg03156893 | chr16 | 23847675 | 22 | N |
| cg01545587 | chr14 | 105993579 | 5 | MU | cg03394150 | chr16 | 28074384 | 22 | MU |
| cg15096085 | chr15 | 90455635 | 5 | N | cg09528825 | chr16 | 28074388 | 22 | MU |
| cg13556511 | chr15 | 90455728 | 5 | MU | cg10471437 | chr16 | 28074462 | 22 | MU |
| cg09234378 | chr15 | 90455792 | 5 | N | cg08806408 | chr16 | 51185001 | 22 | N |
| cg14292280 | chr15 | 90455799 | 5 | N | cg00124695 | chr16 | 51185039 | 22 | MU |
| cg08640252 | chr16 | 1820949 | 5 | MU | cg08439930 | chr16 | 51185060 | 22 | N |
| cg03100639 | chr16 | 31483137 | 5 | N | cg06653699 | chr16 | 51185082 | 22 | N |
| cg10206344 | chr16 | 31483277 | 5 | N | cg09016242 | chr16 | 51185110 | 22 | N |
| cg09983897 | chr16 | 31483285 | 5 | MU | cg06232807 | chr16 | 51185132 | 22 | N |
| cg03666441 | chr17 | 7254671 | 5 | MU | cg01146232 | chr16 | 51185147 | 22 | N |
| cg26695387 | chr17 | 21189768 | 5 | N | cg19047292 | chr16 | 56228442 | 22 | N |
| cg15416179 | chr17 | 21189859 | 5 | MU | cg07700514 | chr16 | 56228467 | 22 | MU |
| cg11270042 | chr17 | 26219919 | 5 | MU | cg16158681 | chr16 | 56623109 | 22 | N |
| cg26785617 | chr17 | 26219967 | 5 | N | cg03015433 | chr16 | 56623111 | 22 | N |
| cg18868622 | chr17 | 36906264 | 5 | MU | cg06213463 | chr16 | 56623215 | 22 | MU |
| cg05041073 | chr17 | 37774893 | 5 | MU | cg16393726 | chr16 | 56623312 | 22 | N |
| cg21936280 | chr17 | 37774934 | 5 | N | cg19021197 | chr17 | 59476212 | 22 | N |
| cg13784792 | chr17 | 39942930 | 5 | N | cg01765249 | chr17 | 59476334 | 22 | MU |
| cg18733255 | chr17 | 39942970 | 5 | MU | cg14485004 | chr17 | 75524913 | 22 | N |

(continued)

| Illumina Probe Name | Chr. | Position | Tissue/Cell | MU/N |
|---|---|---|---|---|
| cg06795963 | chr17 | 40932359 | 5 | N |
| cg24104567 | chr17 | 40932508 | 5 | MU |
| cg02763536 | chr17 | 40932515 | 5 | N |
| cg16208357 | chr17 | 40932520 | 5 | N |
| cg05768702 | chr17 | 47271133 | 5 | N |
| cg20243351 | chr17 | 47271156 | 5 | MU |
| cg03384992 | chr18 | 21852325 | 5 | MU |
| cg26398656 | chr18 | 21852327 | 5 | N |
| cg06593258 | chr18 | 21852333 | 5 | N |
| cg21840153 | chr18 | 21852436 | 5 | N |
| cg08894153 | chr19 | 12606729 | 5 | MU |
| cg05022378 | chr19 | 12606767 | 5 | N |
| cg25737313 | chr19 | 12899557 | 5 | MU |
| cg03287340 | chr19 | 50651195 | 5 | MU |
| cg24152605 | chr19 | 57050359 | 5 | N |
| cg22054362 | chr19 | 57050367 | 5 | MU |
| cg21970554 | chr19 | 57050370 | 5 | N |
| cg04011671 | chr2 | 43202474 | 5 | MU |
| cg03717364 | chr2 | 43202481 | 5 | N |
| cg19286686 | chr2 | 158731699 | 5 | N |
| cg23967461 | chr2 | 158731736 | 5 | MU |
| cg01943873 | chr2 | 158731796 | 5 | N |
| cg10903451 | chr2 | 164592851 | 5 | MU |
| cg00490655 | chr2 | 164592854 | 5 | N |
| cg15650509 | chr2 | 237476609 | 5 | MU |
| cg22654039 | chr20 | 11871282 | 5 | N |
| cg07436991 | chr20 | 11871311 | 5 | N |
| cg14603345 | chr20 | 11871375 | 5 | N |
| cg23944804 | chr20 | 11871384 | 5 | MU |
| cg12894649 | chr20 | 11871396 | 5 | N |
| cg10546252 | chr22 | 24384294 | 5 | N |
| cg24238852 | chr22 | 24384340 | 5 | MU |
| cg11478607 | chr22 | 24384400 | 5 | MU |
| cg09805010 | chr3 | 24536077 | 5 | MU |

| Illumina Probe Name | Chr. | Position | Tissue e/Cell Type | MU/N |
|---|---|---|---|---|
| cg02565715 | chr17 | 75525001 | 22 | N |
| cg27591450 | chr17 | 75525004 | 22 | MU |
| cg06263341 | chr17 | 75525151 | 22 | N |
| cg10362542 | chr17 | 77179709 | 22 | MU |
| cg04093932 | chr17 | 77179778 | 22 | N |
| cg12967001 | chr18 | 5544089 | 22 | N |
| cg16622495 | chr18 | 5544099 | 22 | N |
| cg23564700 | chr18 | 5544169 | 22 | N |
| cg06550462 | chr18 | 5544208 | 22 | MU |
| cg16924702 | chr18 | 5544231 | 22 | MU |
| cg07126263 | chr18 | 57364862 | 22 | N |
| cg01281285 | chr18 | 57364872 | 22 | MU |
| cg24724633 | chr19 | 36909413 | 22 | N |
| cg25886284 | chr19 | 36909418 | 22 | N |
| cg11265561 | chr19 | 53696569 | 22 | N |
| cg12360029 | chr19 | 53696623 | 22 | MU |
| cg07686635 | chr19 | 53696642 | 22 | N |
| cg01620580 | chr19 | 53696649 | 22 | MU |
| cg09568464 | chr19 | 56904901 | 22 | N |
| cg02763101 | chr19 | 56904945 | 22 | N |
| cg22647407 | chr19 | 56904965 | 22 | MU |
| cg08464824 | chr19 | 56904977 | 22 | N |
| cg13916740 | chr19 | 56904997 | 22 | MU |
| cg20984085 | chr19 | 56905032 | 22 | N |
| cg09265529 | chr19 | 57078765 | 22 | MU |
| cg27544219 | chr19 | 57078794 | 22 | N |
| cg01519714 | chr2 | 27070750 | 22 | N |
| cg26491213 | chr2 | 45168819 | 22 | MU |
| cg05740609 | chr2 | 45168921 | 22 | N |
| cg22437074 | chr2 | 45168924 | 22 | N |
| cg13451280 | chr2 | 68870812 | 22 | MU |
| cg01759126 | chr2 | 68870892 | 22 | MU |
| cg00778995 | chr2 | 105470558 | 22 | MU |
| cg13899678 | chr2 | 105478933 | 22 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg02333852 | chr3 | 24536252 | 5 | MU | cg11014373 | chr2 | 105479054 | 22 | MU |
| cg20985755 | chr3 | 24536474 | 5 | MU | cg12518410 | chr2 | 109745812 | 22 | MU |
| cg13790603 | chr3 | 24536478 | 5 | N | cg09392940 | chr2 | 109745828 | 22 | N |
| cg22306344 | chr3 | 49966773 | 5 | MU | cg23046629 | chr2 | 109745841 | 22 | N |
| cg23320649 | chr3 | 50604613 | 5 | N | cg22830113 | chr2 | 127783168 | 22. | MU |
| cg10779423 | chr3 | 50604763 | 5 | MU | cg11113760 | chr2 | 180726247 | 22 | N |
| cg02321062 | chr3 | 107244746 | 5 | MU | cg09279240 | chr2 | 180726252 | 22 | N |
| cg01655150 | chr3 | 126195255 | 5 | N | cg06695611 | chr2 | 180726328 | 22 | MU |
| cg16898124 | chr3 | 126195383 | 5 | MU | cg16400999 | chr2 | 180726349 | 22 | N |
| cg12349858 | chr3 | 160822545 | 5 | MU | cg14670435 | chr2 | 180726464 | 22 | N |
| cg14612335 | chr3 | 170074131 | 5 | MU | cg24613479 | chr2 | 197458046 | 22 | N |
| cg13607248 | chr3 | 183874556 | 5 | MU | cg01381678 | chr2 | 197458093 | 22 | MU |
| cg11836119 | chr4 | 206874 | 5 | MU | cg12644696 | chr2 | 197458104 | 22 | MU |
| cg22913249 | chr4 | 41215979 | 5 | MU | cg17859110 | chr20 | 41818770 | 22 | MU |
| cg18587806 | chr4 | 41362843 | 5 | MU | cg07167168 | chr20 | 41818788 | 22 | N |
| cg26248284 | chr4 | 41362853 | 5 | N | cg21672843 | chr20 | 41818905 | 22 | N |
| cg17187287 | chr4 | 115519593 | 5 | MU | cg06685464 | chr22 | 20790837 | 22 | N |
| cg01803059 | chr4 | 146402404 | 5 | N | cg20401551 | chr22 | 20790985 | 22 | MU |
| cg02971575 | chr4 | 146402540 | 5 | MU | cg19895164 | chr22 | 20791024 | 22 | N |
| cg18485193 | chr5 | 16936369 | 5 | MU | cg22935149 | chr22 | 38851884 | 22 | MU |
| cg17942573 | chr5 | 16936391 | 5 | N | cg20525183 | chr22 | 45405768 | 22 | N |
| cg07705810 | chr5 | 16936470 | 5 | N | cg01995480 | chr22 | 45405827 | 22 | N |
| cg19493077 | chr5 | 32613266 | 5 | MU | cg23080354 | chr22 | 45405880 | 22 | N |
| cg19725489 | chr5 | 72252795 | 5 | MU | cg25561581 | chr22 | 45405899 | 22 | MU |
| cg13904970 | chr5 | 123987667 | 5 | MU | cg10080155 | chr22 | 45405904 | 22 | N |
| cg25606201 | chr5 | 180614858 | 5 | MU | cg18310412 | chr3 | 32858280 | 22 | MU |
| cg25728685 | chr6 | 28351148 | 5 | MU | cg20751361 | chr3 | 37493945 | 22 | N |
| cg23197007 | chr6 | 30655617 | 5 | N | cg25870420 | chr3 | 37493949 | 22 | MU |
| cg13357602 | chr6 | 30655720 | 5 | MU | cg03340649 | chr3 | 44626453 | 22 | N |
| cg09424348 | chr6 | 30881549 | 5 | N | cg22598028 | chr3 | 44626492 | 22 | N |
| cg15080939 | chr6 | 30881560 | 5 | N | cg01139508 | chr3 | 44626538 | 22 | MU |
| cg05267955 | chr6 | 30881562 | 5 | N | cg10172415 | chr3 | 71803558 | 22 | MU |
| cg18261909 | chr6 | 30881571 | 5 | N | cg13384396 | chr3 | 123167677 | 22 | N |
| cg27650171 | chr6 | 30881579 | 5 | N | cg02978184 | chr3 | 123167770 | 22 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg16555181 | chr6 | 30881643 | 5 | N | cg18403551 | chr3 | 140770306 | 22 | MU |
| cg02186769 | chr6 | 30881645 | 5 | N | cg23332582 | chr3 | 140770308 | 22 | N |
| cg26860970 | chr6 | 30881658 | 5 | N | cg22036988 | chr3 | 140770345 | 22 | N |
| cg05103231 | chr6 | 30881664 | 5 | MU | cg23209255 | chr3 | 154797917 | 22 | MU |
| cg00649377 | chr6 | 30881728 | 5 | N | cg16701456 | chr4 | 13546111 | 22 | N |
| cg04895387 | chr6 | 33180930 | 5 | N | cg06093712 | chr4 | 13546145 | 22 | N |
| cg07090273 | chr6 | 33181011 | 5 | N | cg27438798 | chr4 | 13546151 | 22 | N |
| cg27112525 | chr6 | 33181014 | 5 | N | cg23584743 | chr4 | 13546157 | 22 | N |
| cg10041635 | chr6 | 33181021 | 5 | N | cg20393324 | chr4 | 13546169 | 22 | N |
| cg02775469 | chr6 | 33181031 | 5 | MU | cg04808029 | chr4 | 13546210 | 22 | MU |
| cg22501342 | chr6 | 37322729 | 5 | N | cgl8894815 | chr4 | 13546292 | 22 | N |
| cg24691069 | chr6 | 37322773 | 5 | MU | cg22777326 | chr4 | 13546316 | 22 | N |
| cg19930737 | chr6 | 44044325 | 5 | MU | cg14866200 | chr4 | 42399843 | 22 | MU |
| cg16088539 | chr6 | 44044443 | 5 | N | cg23684249 | chr4 | 42399858 | 22 | N |
| cg24311382 | chr6 | 132272408 | 5 | N | cg20276585 | chr4 | 81952330 | 22 | MU |
| cg18849840 | chr6 | 132272545 | 5 | MU | cg17714276 | chr4 | 156588619 | 22 | MU |
| cg15897310 | chr6 | 132272560 | 5 | N | cg27662483 | chr4 | 156588683 | 22 | N |
| cg01813033 | chr6 | 132272609 | 5 | N | cg22643435 | chr4 | 156588740 | 22 | N |
| cg10028217 | chr7 | 122526824 | 5 | N | cg24642320 | chr4 | 186049687 | 22 | MU |
| cg26316023 | chr7 | 122526826 | 5 | N | cg15131808 | chr5 | 528580 | 22 | MU |
| cg06159603 | chr7 | 122526836 | 5 | N | cg14732324 | chr5 | 528621 | 22 | N |
| cg05293994 | chr7 | 122526843 | 5 | MU | cg25302419 | chr5 | 11904015 | 22 | N |
| cg14222879 | chr8 | 71316696 | 5 | MU | cg14081218 | chr5 | 11904022 | 22 | N |
| cg11911122 | chr8 | 71316769 | 5 | N | cg00908526 | chr5 | 11904084 | 22 | N |
| cg21602557 | chr8 | 119122878 | 5 | MU | cg04996219 | chr5 | 11904110 | 22 | MU |
| cg11497957 | chr8 | 125985846 | 5 | MU | cg07195011 | chr5 | 11904114 | 22 | N |
| cg12738197 | chr8 | 125985883 | 5 | N | cg13368756 | chr5 | 11904127 | 22 | N |
| cg05022789 | chr8 | 135469681 | 5 | N | cg00577935 | chr5 | 112073348 | 22 | N |
| cg01919374 | chr8 | 135469790 | 5 | MU | cg08571859 | chr5 | 112073350 | 22 | N |
| cg13469590 | chr9 | 19229767 | 5 | MU | cg14511739 | chr5 | 112073373 | 22 | N |
| cg14281646 | chr9 | 80645556 | 5 | MU | cg22035501 | chr5 | 112073398 | 22 | MU |
| cg03031959 | chr9 | 85677921 | 5 | MU | cg11613015 | chr5 | 112073406 | 22 | MU |
| cg14247332 | chr9 | 132805979 | 5 | MU | cg14479889 | chr5 | 112073426 | 22 | N |
| cg16829306 | chr1 | 21671508 | 6 | MU | cg16970232 | chr5 | 112073433 | 22 | N |

EP 4 741 515 A2

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg16306706 | chr1 | 42643274 | 6 | MU | cg03667968 | chr5 | 112073438 | 22 | MU |
| cg07865091 | chr1 | 43814306 | 6 | N | cg20311501 | chr5 | 112073502 | 22 | N |
| cg18856478 | chr1 | 43814358 | 6 | MU | cg23938220 | chr5 | 112073538 | 22 | N |
| cg24591770 | chr1 | 45082704 | 6 | MU | cg02511809 | chr5 | 112073544 | 22 | N |
| cg20820767 | chr1 | 45082840 | 6 | N | cg11897314 | chr5 | 119799832 | 22 | N |
| cg26315985 | chr1 | 65363870 | 6 | MU | cg27107171 | chr5 | 119799864 | 22 | MU |
| cg02297709 | chr1 | 160159670 | 6 | MU | cg18525611 | chr5 | 132948185 | 22 | N |
| cg15926420 | chr1 | 160159672 | 6 | N | cg04504441 | chr5 | 132948217 | 22 | N |
| cg12420283 | chr1 | 173990399 | 6 | MU | cg00586365 | chr5 | 132948251 | 22 | MU |
| cg22109530 | chr1 | 178310534 | 6 | MU | cg12684209 | chr6 | 393110 | 22 | N |
| cg14515791 | chr1 | 178310545 | 6 | N | cg21277995 | chr6 | 393239 | 22 | MU |
| cg24686918 | chr1 | 221613658 | 6 | MU | cg27200446 | chr6 | 41606439 | 22 | MU |
| cg13677741 | chr1 | 236031096 | 6 | MU | cg21195414 | chr6 | 53213871 | 22 | MU |
| cg27388962 | chr10 | 4705434 | 6 | MU | cg00024396 | chr6 | 53214008 | 22 | N |
| cg18675610 | chr10 | 32216311 | 6 | MU | cg24524396 | chr6 | 53214019 | 22 | N |
| cg22706007 | chr10 | 32216359 | 6 | N | cg16556906 | chr6 | 71666682 | 22 | MU |
| cg14789659 | chr10 | 63809073 | 6 | MU | cg24348705 | chr6 | 71666809 | 22 | N |
| cg00928816 | chr10 | 63809098 | 6 | N | cg12306213 | chr6 | 71666824 | 22 | N |
| cg20746552 | chr10 | 63809108 | 6 | N | cg16202970 | chr6 | 99283201 | 22 | MU |
| cg16389209 | chr10 | 63809121 | 6 | MU | cg17398252 | chr6 | 105585179 | 22 | MU |
| cg07520810 | chr10 | 63809149 | 6 | MU | cg05655837 | chr6 | 166582188 | 22 | N |
| cg16401465 | chr10 | 63809170 | 6 | N | cg17188046 | chr6 | 166582197 | 22 | N |
| cg25103895 | chr10 | 64565642 | 6 | N | cg14638883 | chr6 | 166582201 | 22 | N |
| cg20684110 | chr10 | 64565750 | 6 | MU | cg19675288 | chr6 | 166582206 | 22 | N |
| cg01005506 | chr10 | 64565768 | 6 | N | cg06073449 | chr6 | 166582310 | 22 | MU |
| cg12474798 | chr10 | 64565772 | 6 | MU | cg06463958 | chr6 | 166582393 | 22 | N |
| cg26033520 | chr10 | 74004071 | 6 | MU | cg07778029 | chr7 | 27205114 | 22 | N |
| cg04858631 | chr10 | 74035570 | 6 | MU | cg20741169 | chr7 | 27205159 | 22 | N |
| cg23231163 | chr10 | 75533350 | 6 | N | cg05065989 | chr7 | 27205200 | 22 | N |
| cg19442545 | chr10 | 75533431 | 6 | MU | cg16104915 | chr7 | 27205217 | 22 | N |
| cg07438103 | chr11 | 47175143 | 6 | MU | cg18447772 | chr7 | 27205224 | 22 | MU |
| cg03711944 | chr11 | 47377212 | 6 | MU | cg12600174 | chr7 | 27205230 | 22 | N |
| cg13771313 | chr11 | 72533295 | 6 | MU | cg21001184 | chr7 | 27205262 | 22 | N |
| cg26077811 | chr11 | 119232263 | 6 | MU | cg15083933 | chr7 | 33944635 | 22 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg14239983 | chr11 | 121337384 | 6 | N | cg11813976 | chr8 | 25901335 | 22 | N |
| cg22964469 | chr11 | 121337508 | 6 | MU | cg16749235 | chr8 | 25901422 | 22 | MU |
| cg06889535 | chr11 | 126455712 | 6 | MU | cg01588438 | chr8 | 67344553 | 22 | N |
| cg20260663 | chr12 | 56211576 | 6 | MU | cg09383816 | chr8 | 67344556 | 22 | N |
| cg14845962 | chr12 | 58120237 | 6 | MU | cg18065361 | chr8 | 67344588 | 22 | MU |
| cg16402452 | chr12 | 58129855 | 6 | MU | cg19283840 | chr8 | 67344642 | 22 | N |
| cg18215449 | chr12 | 66089473 | 6 | MU | cg20295442 | chr8 | 67344665 | 22 | N |
| cg08857351 | chr12 | 117627450 | 6 | MU | cg20912169 | chr8 | 67344720 | 22 | N |
| cg21737773 | chr12 | 117627464 | 6 | N | cg25832771 | chr8 | 72756058 | 22 | N |
| cg02486646 | chr12 | 117627478 | 6 | N | cg09734791 | chr8 | 72756155 | 22 | MU |
| cg10807894 | chr12 | 125169804 | 6 | MU | cg16896847 | chr8 | 144512042 | 22 | MU |
| cg23247274 | chr13 | 21750649 | 6 | MU | cg01403246 | chr8 | 144512192 | 22 | N |
| cg18842353 | chr13 | 97794052 | 6 | MU | cg14048780 | chr9 | 91793365 | 22 | MU |
| cg18973863 | chr13 | 97794113 | 6 | N | cg26643377 | chr1 | 11710460 | 23 | N |
| cg06340704 | chr13 | 97877417 | 6 | MU | cg13875012 | chr1 | 11710610 | 23 | MU |
| cg15185986 | chr13 | 98919378 | 6 | MU | cg17949727 | chr1 | 38412684 | 23 | MU |
| cg19300307 | chr13 | 99136580 | 6 | MU | cg10784030 | chr1 | 38412688 | 23 | MU |
| cg01424562 | chr14 | 69256677 | 6 | MU | cg05749969 | chr1 | 38412711 | 23 | MU |
| cg12827637 | chr14 | 69256791 | 6 | MU | cg02309273 | chr1 | 38412744 | 23 | MU |
| cg08169020 | chr14 | 69256888 | 6 | MU | cg22812770 | chr1 | 52195547 | 23 | MU |
| cg19692929 | chr14 | 92575120 | 6 | MU | cg06327150 | chr1 | 52195686 | 23 | N |
| cg16062483 | chr14 | 98444417 | 6 | N | cg17318529 | chr1 | 228196198 | 23 | N |
| cg16278496 | chr14 | 98444476 | 6 | MU | cg26332740 | chr1 | 228196227 | 23 | MU |
| cg11798182 | chr14 | 98444513 | 6 | N | cg10019916 | chr1 | 228196376 | 23 | N |
| cg00034769 | chr14 | 98444533 | 6 | N | cg09850632 | chr1 | 229406661 | 23 | N |
| cg25913761 | chr15 | 90727560 | 6 | MU | cg03301331 | chr1 | 229406681 | 23 | N |
| cg26955540 | chr15 | 90727570 | 6 | MU | cg16909962 | chr1 | 229406711 | 23 | MU |
| cg06008511 | chr15 | 90727886 | 6 | N | cg14383658 | chr10 | 102899262 | 23 | N |
| cg18569141 | chrl5 | 90727995 | 6 | MU | cg05482942 | chr10 | 102899285 | 23 | MU |
| cg16439823 | chr15 | 90728020 | 6 | N | cg05627029 | chr10 | 122708532 | 23 | MU |
| cg19331221 | chr15 | 90728027 | 6 | MU | cg24681845 | chr10 | 126069759 | 23 | MU |
| cg08438529 | chr16 | 1052939 | 6 | MU | cg08734600 | chr10 | 126136228 | 23 | MU |
| cg09267773 | chrl6 | 79802467 | 6 | MU | cg08932440 | chr10 | 126136607 | 23 | MU |
| cg06601071 | chr16 | 85525842 | 6 | MU | cg06299833 | chr10 | 126136709 | 23 | N |

EP 4 741 515 A2

53

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg09642825 | chr16 | 87416230 | 6 | MU | cg18494399 | chr11 | 35441558 | 23 | MU |
| cg03646327 | chr16 | 88509768 | 6 | N | cg21163960 | chr11 | 35441777 | 23 | MU |
| cg05507654 | chr16 | 88509857 | 6 | MU | cg10159951 | chr11 | 35441881 | 23 | MU |
| cg21692159 | chr17 | 36898937 | 6 | MU | cg08258650 | chr11 | 35441900 | 23 | MU |
| cg16936953 | chr17 | 57915665 | 6 | MU | cg12737152 | chr11 | 129565909 | 23 | MU |
| cg12054453 | chr17 | 57915717 | 6 | N | cg26843324 | chr12 | 113905115 | 23 | MU |
| cg01409343 | chr17 | 57915740 | 6 | N | cg11222127 | chr12 | 113905210 | 23 | N |
| cg02782634 | chr17 | 57916643 | 6 | MU | cg04912712 | chr14 | 89817982 | 23 | MU |
| cg27023597, | chr17 | 57918262 | 6 | MU | cg13940834 | chr15 | 78236707 | 23 | MU |
| cg02962602 | chr17 | 80171413 | 6 | MU | cg09499248 | chr16 | 103531 | 23 | MU |
| cg14575053 | chr18 | 61616390 | 6 | N | cg05033333 | chr16 | 3222180 | 23 | MU |
| cg23782616 | chr18 | 61616526 | 6 | MU | cg07582157 | chr16 | 55514269 | 23 | N |
| cg08846870 | chr19 | 15568360 | 6 | MU | cg09350341 | chr16 | 55514378 | 23 | MU |
| cg05258935 | chr19 | 39086923 | 6 | MU | cg02458945 | chr16 | 55514470 | 23 | MU |
| cg03974193 | chr2 | 102316442 | 6 | MU | cg02512505 | chr16 | 56659552 | 23 | MU |
| cg13522882 | chr2 | 102316496 | 6 | MU | cg05743734 | chr16 | 56659659 | 23 | N |
| cg02646515 | chr2 | 128168987 | 6 | MU | cg00748373 | chr16 | 68676741 | 23 | MU |
| cg11845111 | chr2 | 191398756 | 6 | N | cg06575065 | chr16 | 68676743 | 23 | N |
| cg27211696 | chr2 | 191398769 | 6 | N | cg27417609 | chr16 | 68676806 | 23 | N |
| cg10560079 | chr2 | 191398806 | 6 | MU | cg26970841 | chr16 | 85932666 | 23 | MU |
| cg00982136 | chr2 | 201726139 | 6 | MU | cg19619387 | chr17 | 1881036 | 23 | N |
| cg08063160 | chr2 | 238578376 | 6 | MU | cg04743758 | chr17 | 1881161 | 23 | MU |
| cg03504039 | chr2 | 238578435 | 6 | N | cg21342910 | chr17 | 48042779 | 23 | MU |
| cg22734058 | chr2 | 238578491 | 6 | N | cg08278108 | chr17 | 48042917 | 23 | N |
| cg27051683 | chr2 | 242802069 | 6 | N | cg07477034 | chr17 | 53341969 | 23 | N |
| cg03889044 | chr2 | 242802099 | 6 | N | cg18943957 | chr17 | 53342029 | 23 | MU |
| cg17322655 | chr2 | 242802127 | 6 | N | cg12160600 | chr17 | 73029893 | 23 | MU |
| cg20805133 | chr2 | 242802192 | 6 | MU | cg19472078 | chr17 | 77020166 | 23 | N |
| cg20074395 | chr20 | 1246715 | 6 | MU | cg18996910 | chr17 | 77020220 | 23 | MU |
| cg04053242 | chr20 | 1246772 | 6 | N | cg08349573 | chr17 | 77020243 | 23 | N |
| cg12477880 | chr21 | 36259241 | 6 | N | cg24844534 | chr17 | 77020267 | 23 | N |
| cg00994804 | chr21 | 36259383 | 6' | MU | cg26312191 | chr17 | 80279310 | 23 | N |
| cg06758350 | chr21 | 36259460 | 6 | N | cg11740099 | chr17 | 80279430 | 23 | MU |
| cg25703541 | chr22 | 24373054 | 6 | N | cg03325767 | chr17 | 80279477 | 23 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg02953382 | chr22 | 24373134 | 6 | MU | cg19930417 | chr17 | 80279562 | 23 | N |
| cg12043019 | chr22 | 50356277 | 6 | MU | cg08198862 | chr19 | 11529947 | 23 | N |
| cg15376996 | chr22 | 50912555 | 6 | MU | cg08068820 | chr19 | 11530021 | 23 | MU |
| cg13512830 | chr3 | 149530294 | 6 | N | cg08000684 | chr19 | 11530025 | 23 | MU |
| cg12461469 | chr3 | 149530341 | 6 | MU | cg14913644 | chr19 | 11530065 | 23 | N |
| cg02524983 | chr3 | 188012918 | 6 | MU | cg25383503 | chr19 | 18714552 | 23 | MU |
| cg14855367 | chr3 | 191048308 | 6 | MU | cg02085210 | chr2 | 10589054 | 23 | MU |
| cg27312961 | chr3 | 191048361 | 6 | MU | cg18958844 | chr2 | 55509779 | 23 | MU |
| cg13506158 | chr3 | 191048377 | 6 | N | cg27364741 | chr2 | 63281069 | 23 | N |
| cg27269488 | chr3 | 191048411 | 6 | N | cg25622366 | chr2 | 63281139 | 23 | MU |
| cg00591949 | chr3 | 191048439 | 6 | N | cg07938743 | chr2 | 63283939 | 23 | N |
| cg25618513 | chr4 | 153456716 | 6 | MU | cg21472506 | chr2 | 63283967 | 23 | MU |
| cg22879098 | chr5 | 672845 | 6 | MU | cg23229261 | chr2 | 63284066 | 23 | MU |
| cg24082121 | chr5 | 672872 | 6 | MU | cg05092308 | chr2 | 63285365 | 23 | N |
| cg11895835 | chr5 | 111078476 | 6 | MU | cg19351026 | chr2 | 63285425 | 23 | MU |
| cg23256675 | chr5 | 111078579 | 6 | N | cg13768290 | chr2 | 63285491 | 23 | N |
| cg27016579 | chr5 | 168778028 | 6 | MU | cg27315333 | chr2 | 63285950 | 23 | N |
| cg20510285 | chr6 | 11263797 | 6 | MU | cg11536474 | chr2 | 63286049 | 23 | MU |
| cg25607383 | chr6 | 30853569 | 6 | N | cg22061831 | chr2 | 131721692 | 23 | MU |
| cg11975790 | chr6 | 30853608 | 6 | MU | cg27406975 | chr2 | 172955512 | 23 | MU |
| cg03780271 | chr6 | 33280052 | 6 | N | cg24813176 | chr2 | 219736167 | 23 | N |
| cg12589538 | chr6 | 33280149 | 6 | N | cg22344703 | chr2 | 219736312 | 23 | MU |
| cg11796996 | chr6 | 33280159 | 6 | MU | cg06862374 | chr2 | 219736549 | 23 | MU |
| cg02863594 | chr6 | 33280199 | 6 | N | cg22587479 | chr2 | 219738226 | 23 | MU |
| cg01253676 | chr6 | 33280212 | 6 | N | cg00011225 | chr2 | 219738314 | 23 | N |
| cg20998791 | chr6 | 33280228 | 6 | N | cg07211212 | chr20 | 55200146 | 23 | MU |
| cg18353226 | chr6 | 33280390 | 6 | N | cg15316660 | chr21 | 38119920 | 23 | MU |
| cg14419102 | chr6 | 33280400 | 6 | N | cg09556952 | chr21 | 38119946 | 23 | N |
| cg06375761 | chr6 | 33280408 | 6 | N | cg08886651 | chr22 | 19743029 | 23 | MU |
| cg26083458 | chr6 | 33280424 | 6 | N | cg20295671 | chr22 | 22090486 | 23 | MU |
| cg13638257 | chr6 | 33280436 | 6 | MU | cg20361154 | chr22 | 22862884 | 23 | MU |
| cg26401541 | chr6 | 91078974 | 6 | MU | cg11417653 | chr22 | 24181212 | 23 | MU |
| cg20227056 | chr6 | 130096463 | 6 | MU | cg25940946 | chr22 | 24181263 | 23 | N |
| cg07185983 | chr6 | 139012860 | 6 | N | cg25037461 | chr22 | 24181268 | 23 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg06864789 | chr6 | 139012992 | 6 | MU | cg14774086 | chr22 | 24181270 | 23 | N |
| cg25399239 | chr6 | 139013092 | 6 | N | cg03970350 | chr22 | 31003010 | 23 | N |
| cg18136963 | chr6 | 139013146 | 6 | MU | cg26532826 | chr22 | 31003127 | 23 | N |
| cg04368789 | chr7 | 2904671 | 6 | MU | cg11875119 | chr22 | 31003147 | 23 | MU |
| cg11532431 | chr7 | 27169674 | 6 | MU | cg02111168 | chr22 | 31003283 | 23 | MU |
| cg00562553 | chr7 | 27169740 | 6 | N | cg20276377 | chr3 | 99595026 | 23 | MU |
| cg04317399 | chr7 | 27170313 | 6 | N | cg22092811 | chr3 | 99595145 | 23 | MU |
| cg07317062 | chr7 | 27170388 | 6 | N | cg18337222 | chr3 | 99595254 | 23 | N |
| cg19142026 | chr7 | 27170394 | 6 | MU | cg20691722 | chr3 | 181421427 | 23 | MU |
| cg11410718 | chr7 | 27170412 | 6 | N | cg02213103 | chr3 | 181421548 | 23 | N |
| cg05098343 | chr7 | 37071686 | 6 | MU | cg27290624 | chr3 | 194208441 | 23 | MU |
| cg23220823 | chr7 | 45041140 | 6 | MU | cg11862731 | chr3 | 194408516 | 23 | MU |
| cg08486903 | chr7 | 98739496 | 6 | N | cg16578537 | chr3 | 194408556 | 23 | MU |
| cg18887230 | chr7 | 98739627 | 6 | MU | cg09652652 | chr3 | 194408845 | 23 | MU |
| cg08571362 | chr7 | 100224015 | 6 | N | cg10623840 | chr3 | 194408965 | 23 | N |
| cg11432630 | chr7 | 100224149 | 6 | MU | cg08377398 | chr4 | 6202553 | 23 | MU |
| cg23896514 | chr7 | 150103902 | 6 | MU | cg04865110 | chr4 | 6202558 | 23 | N |
| cg05875700 | chr8 | 638208 | 6 | MU | cg00731785 | chr4 | 15704680 | 23 | MU |
| cg12641240 | chr8 | 638330 | 6 | N | cg13528854 | chr4 | 15704795 | 23 | N |
| cg00936790 | chr8 | 29027844 | 6 | MU | cg11416290 | chr4 | 111532410 | 23 | MU |
| cg17084151 | chr8 | 102803237 | 6 | MU | cg18349911 | chr5 | 1877135 | 23 | MU |
| cg23006204 | chr9 | 130700866 | 6 | N | cg22156700 | chr5 | 72732486 | 23 | MU |
| cg14395885 | chr9 | 130700923 | 6 | N | cg19092317 | chr5 | 72739768 | 23 | MU |
| cg21181453 | chr9 | 130700954 | 6 | MU | cg01889143 | chr5 | 134825791 | 23 | MU |
| cg00921973 | chr9 | 130701076 | 6 | N | cg24792289 | chr5 | 134825895 | 23 | N |
| cg07865091 | chr1 | 43814306 | 7 | N | cg13707560 | chr5 | 137474700 | 23 | N |
| cg18856478 | chr1 | 43814358 | 7 | MU | cg16354091. | chr5 | 137474830 | 23 | MU |
| cg24591770 | chr1 | 45082704 | 7 | MU | cg09047573 | chr5 | 137474905 | 23 | N |
| cg20820767 | chr1 | 45082840 | 7 | N | cg04233770 | chr5 | 137475002 | 23 | MU |
| cg26315985 | chr1 | 65363870 | 7 | MU | cg22656956 | chr5 | 137475177 | 23 | MU |
| cg02297709 | chr1 | 160159670 | 7 | MU | cg25507001 | chr5 | 137475288 | 23 | N |
| cg15926420 | chr1 | 160159672 | 7 | N | cg20452230 | chr5 | 137475379 | 23 | MU |
| cg12420283 | chr1 | 173990399 | 7 | MU | cg09602288 | chr6 | 10382800 | 23 | N |
| cg22109530 | chr1 | 178310534 | 7 | MU | cg20168806 | chr6 | 10382828 | 23 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg14515791 | chr1 | 178310545 | 7 | N | cg24336989 | chr6 | 10385903 | 23 | MU |
| cg11480873 | chr1 | 205744417 | 7 | MU | cg11811610 | chr6 | 10390811 | 23 | N |
| cg24686918 | chr1 | 221613658 | 7 | MU | cg24452128 | chr6 | 10390919 | 23 | MU |
| cg13677741 | chr1 | 236031096 | 7 | MU | cg19607165 | chr6 | 10390961 | 23 | N |
| cg27388962 | chr10 | 4705434 | 7 | MU | cg17754510 | chr6 | 10391412 | 23 | MU |
| cg07450214 | chr10 | 27389879 | 7 | MU | cg05639246 | chr6 | 10393778 | 23 | MU |
| cg08856118 | chr10 | 27389908 | 7 | N | cg13486532 | chr6 | 10421460 | 23 | MU |
| cg18675610 | chr10 | 32216311 | 7 | MU | cg10293403 | chr6 | 10421670 | 23 | MU |
| cg22706007 | chr10 | 32216359 | 7 | N | cg05224741 | chr6 | 10422322 | 23 | MU |
| cg14789659 | chr10 | 63809073 | 7 | MU | cg24865270 | chr6 | 10422447 | 23 | N |
| cg00928816 | chr10 | 63809098 | 7 | N | cg17217665 | chr6 | 44186914 | 23 | N |
| cg20746552 | chr10 | 63809108 | 7 | N | cg04742345 | chr6 | 44186969 | 23 | N |
| cg16389209 | chr10 | 63809121 | 7 | MU | cg11452354 | chr6 | 44187052 | 23 | MU |
| cg07520810 | chr10 | 63809149 | 7 | MU | cg10519140 | chr6 | 44187076 | 23 | N |
| cg16401465 | chr10 | 63809170 | 7 | N | cg13793145 | chr6 | 44187109 | 23 | MU |
| cg25103895 | chr10 | 64565642 | 7 | N | cg07053014 | chr6 | 44187132 | 23 | N |
| cg20684110 | chr10 | 64565750 | 7 | MU | cg04175292 | chr6 | 44187226 | 23 | N |
| cg01005506 | chr10 | 64565768 | 7 | N | cg17507887 | chr6 | 50786511 | 23 | MU |
| cg12474798 | chr10 | 64565772 | 7 | MU | cg09363735 | chr6 | 50808699 | 23 | N |
| cg26033520 | chr10 | 74004071 | 7 | MU | cg03159396 | chr6 | 50808813 | 23 | MU |
| cg04858631 | chr10 | 74035570 | 7 | MU | cg09107912 | chr6 | 159590059 | 23 | MU |
| cg23231163 | chr10 | 75533350 | 7 | N | cg00157796 | chr6 | 159590231 | 23 | MU |
| cg19442545 | chr10 | 75533431 | 7 | MU | cg13990351 | chr7 | 64349133 | 23 | MU |
| cg25225070 | chr11 | 9587743 | 7 | MU | cg01129004 | chr7 | 64349155 | 23 | MU |
| cg03711944 | chr11 | 47377212 | 7 | MU | cg13100965 | chr7 | 64349450 | 23 | MU |
| cg09367425 | chr11 | 61733997 | 7 | MU | cg19082708 | chr7 | 64349557 | 23 | MU |
| cg25170017 | chr11 | 64644487 | 7 | MU | cg16814808 | chr7 | 64349706 | 23 | N |
| cg06889535 | chr11 | 126455712 | 7 | MU | cg26792755 | chr7 | 140714919 | 23 | MU |
| cg14845962 | chr12 | 58120237 | 7 | MU | cg10191684 | chr8 | 22423014 | 23 | MU |
| cg08425810 | chr12 | 58132558 | 7 | MU | cg07924703 | chr8 | 22423020 | 23 | N |
| cg18215449 | chr12 | 66089473 | 7 | MU | cg18406492 | chr8 | 38008491 | 23 | N |
| cg13442969 | chr12 | 68044208 | 7 | MU | cg09630404 | chr8 | 38008543 | 23 | MU |
| cg08832851 | chr12 | 92535822 | 7 | MU | cg07852402 | chr9 | 96713643 | 23 | MU |
| cg08857351 | chr12 | 117627450 | 7 | MU | cg14374754 | chr9 | 96714295 | 23 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg21737773 | chr12 | 117627464 | 7 | N | cg22902266 | chr9 | 96714313 | 23 | N |
| cg02486646 | chr12 | 117627478 | 7 | N | cg10454937 | chr9 | 96715047 | 23 | MU |
| cg10807894 | chr12 | 125169804 | 7 | MU | cg13398482 | chr9 | 96715256 | 23 | MU |
| cg23247274 | chr13 | 21750649 | 7 | MU | cg01150641 | chr1 | 976168 | 24 | N |
| cg18842353 | chr13 | 97794052 | 7 | MU | cg23625715 | chr1 | 976172 | 24 | MU |
| cg18973863 | chr13 | 97794113 | 7 | N | cg26222311 | chr1 | 976227 | 24 | N |
| cg06340704 | chr13 | 97877417 | 7 | MU | cg22433276 | chr1 | 1072642 | 24 | MU |
| cg19300307 | chr13 | 99136580 | 7 | MU | cg15550572 | chr1 | 2064441 | 24 | MU |
| cg01424562 | chr14 | 69256677 | 7 | MU | cg25466090 | chr1 | 2064691 | 24 | MU |
| cg12827637 | chr14 | 69256791 | 7 | MU | cg23844174 | chr1 | 2064819 | 24 | MU |
| cg08169020 | chr14 | 69256888 | 7 | MU | cg20248421 | chr1 | 6507979 | 24 | MU |
| cg19692929 | chr14 | 92575120 | 7 | MU | cg19809453 | chr1 | 6508028 | 24 | MU |
| cg16231923 | chr15 | 73923634 | 7 | MU | cg14531560 | chr1 | 16085371 | 24 | MU |
| cg25913761 | chr15 | 90727560 | 7 | MU | cg17216478 | chr1 | 21616619 | 24 | MU |
| cg26955540 | chr15 | 90727570 | 7 | MU | cg06819200 | chr1 | 21616641 | 24 | N |
| cg06008511 | chr15 | 90727886 | 7 | N | cg21816308 | chr1 | 39044140 | 24 | N |
| cg18569141 | chr15 | 90727995 | 7 | N | cg23048481 | chr1 | 39044196 | 24 | MU |
| cg16439823 | chr15 | 90728020 | 7 | N | cg15489005 | chr1 | 39044328 | 24 | N |
| cg19331221 | chr15 | 90728027 | 7 | MU | cg06531379 | chr1 | 47911449 | 24 | N |
| cg26813301 | chr16 | 2653222 | 7 | N | cg26864230 | chr1 | 47911508 | 24 | MU |
| cg03314158 | chr16 | 2653280 | 7 | MU | cg03119043 | chr1 | 47915553 | 24 | N |
| cg06035616 | chr16 | 2653306 | 7 | N | cg18017482 | chr1 | 47915599 | 24 | N |
| cg09267773 | chr16 | 79802467 | 7 | MU | cg00256155 | chr1 | 47915647 | 24 | MU |
| cg06601071 | chr16 | 85525842 | 7 | MU | cg22606303 | chr1 | 47915774 | 24 | N |
| cg09642825 | chr16 | 87416230 | 7 | MU | cg07463059 | chr1 | 158979810 | 24 | MU |
| cg03646327 | chr16 | 88509768 | 7 | N | cg11880855 | chr10 | 16562917 | 24 | N |
| cg05507654 | chr16 | 88509857 | 7 | MU | cg25104555 | chr10 | 16562998 | 24 | MU |
| cg21692159 | chr17 | 36898937 | 7 | MU | cg18279326 | chr10 | 102475324 | 24 | N |
| cg10361922 | chr17 | 40925790 | 7 | MU | cg20100910 | chr10 | 102475429 | 24 | MU |
| cg16936953 | chr17 | 57915665 | 7 | MU | cg22633840 | chr10 | 102502084 | 24 | N |
| cg12054453 | chr17 | 57915717 | 7 | N | cg24127310 | chr10 | 102502104 | 24 | MU |
| cg01409343 | chr17 | 57915740 | 7 | N | cg26156687 | chr10 | 102586168 | 24 | MU |
| cg02782634 | chr17 | 57916643 | 7 | MU | cg07046369 | chr10 | 102586254 | 24 | N |
| cg27023597 | chr17 | 57918262 | 7 | MU | cg25531836 | chr10 | 102586999 | 24 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg07166266 | chr17 | 74067713 | 7 | MU | cg26439963 | chr10 | 102587110 | 24 | MU |
| cg26394308 | chr17 | 74067861 | 7 | N | cg16574134 | chr10 | 102588315 | 24 | MU |
| cg20772590 | chr17 | 75446431 | 7 | N | cg20095124 | chr10 | 102588691 | 24 | MU |
| cg14885762 | chr17 | 75446450 | 7 | N | cg17480467 | chr10 | 102588959 | 24 | MU |
| cg00324097 | chr17 | 75446549 | 7 | MU | cg14778235 | chr10 | 102589102 | 24 | MU |
| cg21204860 | chr17 | 75446565 | 7 | N | cg08217716 | chr10 | 102589250 | 24 | MU |
| cg19654743 | chr17 | 75446592 | 7 | N | cg06921011 | chr10 | 102589467 | 24 | N |
| cg06513247 | chr17 | 75446661 | 7 | N | cg18494190 | chr10 | 102589532 | 24 | N |
| cg08846870 | chr19 | 15568360 | 7 | MU | cg04436818 | chr10 | 102589545 | 24 | MU |
| cg05258935 | chr19 | 39086923 | 7 | MU | cg12508624 | chr11 | 119293780 | 24 | N |
| cg17847861 | chr19 | 40972683 | 7 | MU | cg10601287 | chr11 | 119293861 | 24 | N |
| cg02646515 | chr2 | 128168987 | 7 | MU | cg17236709 | chr11 | 119293863 | 24 | N |
| cg11845111 | chr2 | 191398756 | 7 | N | cg16566400 | chr11 | 119293869 | 24 | MU |
| cg27211696 | chr2 | 191398769 | 7 | N | cg05451852 | chr11 | 123300839 | 24 | N |
| cg10560079 | chr2 | 191398806 | 7 | MU | cg14917395 | chr11 | 123300849 | 24 | MU |
| cg00982136 | chr2 | 201726139 | 7 | MU | cg19419279 | chr11 | 123300867 | 24 | MU |
| cg08474651 | chr2 | 201729683 | 7 | MU | cg03401096 | chr11 | 123301171 | 24 | MU |
| cg08063160 | chr2 | 238578376 | 7 | MU | cg05289253 | chr11 | 123301674 | 24 | MU |
| cg03504039 | chr2 | 238578435 | 7 | N | cg27355456 | chr11 | 123301748 | 24 | MU |
| cg22734058 | chr2 | 238578491 | 7 | N | cg22829917 | chr12 | 54357408 | 24 | N |
| cg20074395 | chr20 | 1246715 | 7 | MU | cg18040901 | chr12 | 54357530 | 24 | MU |
| cg04053242 | chr20 | 1246772 | 7 | N | cg15731655 | chr12 | 54357604 | 24 | N |
| cg08257293 | chr20 | 30308440 | 7 | MU | cg12232783 | chr12 | 54422127 | 24 | N |
| cg12477880 | chr21 | 36259241 | 7 | N | cg04576672 | chr12 | 54422239 | 24 | MU |
| cg00994804 | chr21 | 36259383 | 7 | MU | cg27441225 | chr12 | 54422350 | 24 | MU |
| cg06758350 | chr21 | 36259460 | 7 | N | cg11941633 | chr12 | 54422447 | 24 | N |
| cg14785464 | chr21 | 38362725 | 7 | MU | cg22378817 | chr12 | 54422488 | 24 | N |
| cg06167719 | chr21 | 38362727 | 7 | MU | cg05982757 | chr12 | 54427528 | 24 | N |
| cg03419014 | chr21 | 38362742 | 7 | N | cg16983211 | chr12 | 54427636 | 24 | MU |
| cg09037712 | chr21 | 38362754 | 7 | N | cg15700739 | chr12 | 54427700 | 24 | N |
| cg26460530 | chr22 | 39919067 | 7 | MU | cg18922524 | chr12 | 54447136 | 24 | N |
| cg15376996 | chr22 | 50912555 | 7 | MU | cg03923561 | chr12 | 54447220 | 24 | N |
| cg13512830 | chr3 | 149530294 | 7 | N | cg02264990 | chr12 | 54447243 | 24 | MU |
| cg12461469 | chr3 | 149530341 | 7 | MU | cg09720701 | chr12 | 54447283 | 24 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg26149032 | chr3 | 158519514 | 7 | N | cg01579535 | chr12 | 54447292 | 24 | N |
| cg07765813 | chr3 | 158519566 | 7 | MU | cg01524853 | chr12 | 54447807 | 24 | N |
| cg14855367 | chr3 | 191048308 | 7 | MU | cg22747076 | chr12 | 54447873 | 24 | MU |
| cg27312961 | chr3 | 191048361 | 7 | MU | cg03800721 | chr12 | 120525338 | 24 | MU |
| cg13506158 | chr3 | 191048377 | 7 | N | cg27075104 | chr12 | 122277214 | 24 | N |
| cg27269488 | chr3 | 191048411 | 7 | N | cg26386472 | chr12 | 122277360 | 24 | MU |
| cg00591949 | chr3 | 191048439 | 7 | N | cg06601685 | chr12 | 122277439 | 24 | N |
| cg03956042 | chr3 | 194206497 | 7 | MU | cg16232183 | chr13 | 96204854 | 24 | N |
| cg25618513 | chr4 | 153456716 | 7 | MU | cg06428163 | chr13 | 96204856 | 24 | N |
| cg22879098 | chr5 | 672845 | 7 | MU | cg04603730 | chr13 | 96204870 | 24 | MU |
| cg24082121 | chr5 | 672872 | 7 | MU | cg10305311 | chr13 | 96204873 | 24 | MU |
| cg11895835 | chr5 | 111078476 | 7 | MU | cg09469554 | chr13 | 96204917 | 24 | N |
| cg23256675 | chr5 | 111078579 | 7 | N | cg25032595 | chr13 | 96204978 | 24 | N |
| cg27016579 | chr5 | 168778028 | 7 | MU | cg16556145 | chr13 | 96205184 | 24 | MU |
| cg25607383 | chr6 | 30853569 | 7 | N | cg18393747 | chr13 | 96205295 | 24 | N |
| cg11975790 | chr6 | 30853608 | 7 | MU | cg02223067 | chr13 | 113623233 | 24 | MU |
| cg18353226 | chr6 | 33280390 | 7 | N | cg09678836 | chr13 | 113623844 | 24 | MU |
| cg14419102 | chr6 | 33280400 | 7 | N | cg20070026 | chr14 | 102248073 | 24 | MU |
| cg06375761 | chr6 | 33280408 | 7 | MU | cg22796749 | chr14 | 105512071 | 24 | MU |
| cg26083458 | chr6 | 33280424 | 7 | N | cg10177032 | chr16 | 4103492 | 24 | MU |
| cg13638257 | chr6 | 33280436 | 7 | MU | cg01352090 | chr16 | 4103533 | 24 | N |
| cg06493154 | chr6 | 42859023 | 7 | MU | cg00355909 | chr1 6 | 66638320 | 24 | MU |
| cg09757525 | chr6 | 90928574 | 7 | MU | cg08601917 | chr16 | 66638396 | 24 | N |
| cg26401541 | chr6 | 91078974 | 7 | MU | cg06445928 | chr16 | 66638407 | 24 | N |
| cg20227056 | chr6 | 130096463 | 7 | MU | cg09233013 | chr16 | 66638412 | 24 | MU |
| cg07185983 | chr6 | 139012860 | 7 | N | cg26560414 | chr16 | 66638433 | 24 | MU |
| cg06864789 | chr6 | 139012992 | 7 | MU | cg08682544 | chr16 | 66638438 | 24 | MU |
| cg25399239 | chr6 | 139013092 | 7 | N | cg02070685 | chr16 | 84002370 | 24 | MU |
| cg18136963 | chr6 | 139013146 | 7 | MU | cg06367117 | chr17 | 26903928 | 24 | N |
| cg04368789 | chr7 | 2904671 | 7 | MU | cg14668747 | chr17 | 26904053 | 24 | MU |
| cg11532431 | chr7 | 27169674 | 7 | MU | cg14501061 | chr17 | 26904058 | 24 | N |
| cg00562553 | chr7 | 27169740 | 7 | N | cg27183188 | chr17 | 26904121 | 24 | MU |
| cg05098343 | chr7 | 37071686 | 7 | MU | cg13990559 | chr17 | 26904150 | 24 | N |
| cg23220823 | chr7 | 45041140 | 7 | MU | cg01626899 | chr17 | 26925852 | 24 | MU |

EP 4 741 515 A2

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg23896514 | chr7 | 150103902 | 7 | MU | cg08062469 | chr17 | 26926627 | 24 | MU |
| cg05875700 | chr8 | 638208 | 7 | MU | cg06803850 | chr17 | 26926738 | 24 | N |
| cg12641240 | chr8 | 638330 | 7 | N | cg04636269 | chr17 | 35300868 | 24 | N |
| cg00936790 | chr8 | 29027844 | 7 | MU | cg04588165 | chr17 | 35300874 | 24 | MU |
| cg04962480 | chr8 | 101962118 | 7 | MU | cg04264908 | chr17 | 35303330 | 24 | MU |
| cg17084151 | chr8 | 102803237 | 7 | MU | cg22097768 | chr17 | 61615913 | 24 | MU |
| cg23006204 | chr9 | 130700866 | 7 | N | cg07960944 | chr19 | 11199851 | 24 | N |
| cg14395885 | chr9 | 130700923 | 7 | N | cg05249393 | chr19 | 11199883 | 24 | N |
| cg21181453 | chr9 | 130700954 | 7 | MU | cg22381454 | chr19 | 11199903 | 24 | MU |
| cg00921973 | chr9 | 130701076 | 7 | N | cg18596381 | chr19 | 11199917 | 24 | N |
| cg22437405 | chr1 | 38000170 | 8 | MU | cg19751789 | chr19 | 11199944 | 24 | N |
| cg26315985 | chr1 | 65363870 | 8 | MU | cg12013591 | chr19 | 11199965 | 24 | N |
| cg08367326 | chr1 | 110052010 | 8 | MU | cg19221841 | chr19 | 41197049 | 24 | MU |
| cg12420283 | chr1 | 173990399 | 8 | MU | cg19643921 | chr19 | 41197106 | 24 | N |
| cg02635260 | chr1 | 222988117 | 8 | MU | cg04312358 | chr19 | 41197166 | 24 | N |
| cg13677741 | chr1 | 236031096 | 8 | MU | cg11872300 | chr19 | 42785225 | 24 | MU |
| cg08614871 | chr10 | 3824555 | 8 | N | cg10136354 | chr2 | 73151457 | 24 | N |
| cg24287110 | chr10 | 3824687 | 8 | MU | cg16781647 | chr2 | 73151595 | 24 | MU |
| cg27388962 | chr10 | 4705434 | 8 | MU | cg03133266 | chr22 | 23908776 | 24 | MU |
| cg14789659 | chr10 | 63809073 | 8 | MU | cg03553786 | chr3 | 13692202 | 24 | N |
| cg00928816 | chr10 | 63809098 | 8 | N | cg23620822 | chr3 | 13692212 | 24 | MU |
| cg20746552 | chr10 | 63809108 | 8 | N | cg08417257 | chr3 | 13692343 | 24 | N |
| cg16389209 | chr10 | 63809121 | 8 | N | cg01511742 | chr3 | 71112437 | 24 | MU |
| cg07520810 | chr10 | 63809149 | 8 | MU | cg23067085 | chr3 | 71180032 | 24 | N |
| cg16401465 | chr10 | 63809170 | 8 | N | cg22076311 | chr3 | 71180052 | 24 | MU |
| cg25103895 | chr10 | 64565642 | 8 | N | cg18166376 | chr3 | 71180064 | 24 | N |
| cg20684110 | chr10 | 64565750 | 8 | N | cg21836903 | chr3 | 71180071 | 24 | MU |
| cg01005506 | chr10 | 64565768 | 8 | N | cg12532398 | chr4 | 103997887 | 24 | MU |
| cg12474798 | chr10 | 64565772 | 8 | MU | cg10261589 | chr4 | 103997969 | 24 | MU |
| cg23231163 | chr10 | 75533350 | 8 | N | cg00939735 | chr4 | 185941265 | 24 | MU |
| cg19442545 | chr10 | 75533431 | 8 | MU | cg04905049 | chr4 | 185941304 | 24 | N |
| cg07397033 | chr10 | 98031201 | 8 | N | cg00473462 | chr5 | 472545 | 24 | MU |
| cg08901339 | chr10 | 98031261 | 8 | N | cg00049323 | chr5 | 472564 | 24 | N |
| cg18448570 | chr10 | 98031337 | 8 | MU | cg17284070 | chr5 | 473007 | 24 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg11613902 | chr10 | 98156120 | 8 | N | cg15084390 | chr5 | 475085 | 24 | MU |
| cg04983289 | chr10 | 98156179 | 8 | MU | cg21743597 | chr5 | 475205 | 24 | N |
| cg00177239 | chr10 | 116581112 | 8 | N | cg17633020 | chr5 | 58335180 | 24 | MU |
| cg07216976 | chr10 | 116581248 | 8 | MU | cg18242030 | chr5 | 58335271 | 24 | MU |
| cg04561237 | chr10 | 126430405 | 8 | MU | cg22078451 | chr5 | 58335681 | 24 | MU |
| cg26429520 | chr11 | 1283875 | 8 | N | cg02632185 | chr5 | 66299786 | 24 | MU |
| cg01274643 | chr11 | 1283946 | 8 | MU | cg17449851 | chr5 | 66300406 | 24 | MU |
| cg11027717 | chr11 | 33713904 | 8 | MU | cg11831286 | chr5 | 66300433 | 24 | MU |
| cg19668951 | chr11 | 47430812 | 8 | MU | cg27116842 | chr5 | 66300503 | 24 | MU |
| cg25170017 | chr11 | 64644487 | 8 | MU | cg22120063 | chr5 | 72596188 | 24 | MU |
| cg14519515 | chr11 | 67033594 | 8 | MU | cg25113089 | chr5 | 72598192 | 24 | MU |
| cg16744438 | chr11 | 118657127 | 8 | MU | cg00766220 | chr5 | 72598865 | 24 | N |
| cg26077811 | chr11 | 119232263 | 8 | MU | cg11795854 | chr5 | 72598965 | 24 | MU |
| cg26894079 | chr11 | 122954435 | 8 | MU | cg12390895 | chr5 | 929463500 | 24 | MU |
| cg19733736 | chr12 | 56115029 | 8 | MU | cg18301423 | chr5 | 131593218 | 24 | N |
| cg22249612 | chr12 | 56121485 | 8 | MU | cg22638593 | chr5 | 131593259 | 24 | MU |
| cg13442969 | chr12 | 68044208 | 8 | MU | cg02033258 | chr5 | 131593261 | 24 | MU |
| cg10502324 | chr12 | 76372038 | 8 | MU | cg21333861 | chr6 | 33244976 | 24 | MU |
| cg08832851 | chr12 | 92535822 | 8 | MU | cg07348922 | chr6 | 33244990 | 24 | N |
| cg09942210 | chr12 | 124965227 | 8 | MU | cg00974935 | chr6 | 106959535 | 24 | N |
| cg10807894 | chrl2 | 125169804 | 8 | MU | cg18405900 | chr6 | 106959549 | 24 | N |
| cg17936564 | chr13 | 33113331 | 8 | N | cg19343088 | chr6 | 106959551 | 24 | N |
| cg11630632 | chr13 | 33113343 | 8 | MU | cg06025938 | chr6 | 106959592 | 24 | MU |
| cg18842353 | chr13 | 97794052 | 8 | MU | cg12448860 | chr6 | 106959601 | 24 | N |
| cg18973863 | chr13 | 97794113 | 8 | N | cg05873285 | chr6 | 106960491 | 24 | MU |
| cg19300307 | chr13 | 99136580 | 8 | MU | cg05158615 | chr7 | 24323559 | 24 | N |
| cg01424562 | chr14 | 69256677 | 8 | MU | cg24242823 | chr7 | 24323675 | 24 | MU |
| cg12827637 | chr14 | 69256791 | 8 | MU | cg24885417 | chr7 | 24323764 | 24 | MU |
| cg08169020 | chr14 | 69256888 | 8 | MU | cg00355281 | chr7 | 24323767 | 24 | N |
| cg04925858 | chr14 | 77499547 | 8 | MU | cg16964348 | chr7 | 24323799 | 24 | MU |
| cg18496447 | chr15 | 45480917 | 8 | MU | cg25884711 | chr7 | 24323840 | 24 | MU |
| cg09499248 | chr16 | 103531 | 8 | MU | cg21097881 | chr7 | 24323939 | 24 | MU |
| cg26813301 | chr16 | 2653222 | 8 | MU | cg06206902 | chr7 | 27191226 | 24 | MU |
| cg03314158 | chrl6 | 2653280 | 8 | MU | cg16771406 | chr7 | 27191352 | 24 | N |

(continued)

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg06035616 | chr16 | 2653306 | 8 | N | cg18232548 | chr7 | 50535776 | 24 | MU |
| cg03680291 | chr16 | 47052148 | 8 | MU | cg16424920 | chr8 | 9760855 | 24 | N |
| cg00448868 | chr16 | 66775493 | 8 | MU | cg10866298 | chr8 | 9760869 | 24 | N |
| cg09642825 | chr16 | 87416230 | 8 | MU | cg21535000 | chr8 | 9760877 | 24 | MU |
| cg03646327 | chr16 | 88509768 | 8 | N | cg12877251 | chr8 | 9760880 | 24 | MU |
| cg05507654 | chr16 | 88509857 | 8 | MU | cg00571033 | chr8 | 9760957 | 24 | N |
| cg08255475 | chr16 | 88871329 | 8 | MU | cg14278808 | chr8 | 9761141 | 24 | N |
| cg27189395 | chr16 | 89725071 | 8 | MU | cg15248835 | chr8 | 9761171 | 24 | MU |
| cg04043919 | chr16 | 89725091 | 8 | N | cg03167763 | chr1 | 20512397 | 25 | N |
| cg23344321 | chr17 | 28707212 | 8 | MU | cg19696441 | chr1 | 20512463 | 25 | MU |
| cg21692159 | chr17 | 36898937 | 8 | MU | cg24479524 | chr1 | 20512796 | 25 | N |
| cg13488013 | chr17 | 47592680 | 8 | MU | cg11651220 | chr1 | 20512944 | 25 | MU |
| cg16936953 | chr17 | 57915665 | 8 | N | cg17949727 | chr1 | 38412684 | 25 | MU |
| cg12054453 | chr17 | 57915717 | 8 | MU | cg10784030 | chr1 | 38412688 | 25 | N |
| cg01409343 | chr17 | 57915740 | 8 | N | cg05749969 | chr1 | 38412711 | 25 | N |
| cg14131285 | chr17 | 71949210 | 8 | MU | cg02309273 | chr1 | 38412744 | 25 | N |
| cg02962602 | chr17 | 80171413 | 8 | MU | cg26246897 | chr1 | 59612710 | 25 | MU |
| cg04727071 | chr19 | 4061359 | 8 | MU | cg17318529 | chr1 | 228196198 | 25 | N |
| cg25594549 | chr19 | 16740308 | 8 | MU | cg26332740 | chr1 | 228196227 | 25 | MU |
| cg19390582 | chr19 | 16740428 | 8 | N | cg10019916 | chr1 | 228196376 | 25 | N |
| cg22006825 | chr19 | 41771771 | 8 | N | cg04273303 | chr1 | 228225533 | 25 | MU |
| cg12946660 | chr19 | 41771873 | 8 | MU | cg09227621 | chr1 | 228225606 | 25 | MU |
| cg07377519 | chr19 | 50269083 | 8 | MU | cg00078968 | chr1 | 228225659 | 25 | N |
| cg01930746 | chr2 | 10950511 | 8 | MU | cg23074048 | chr10 | 8088801 | 25 | MU |
| cg11051158 | chr2 | 75938223 | 8 | N | cg11444332 | chr10 | 8088904 | 25 | N |
| cg20704342 | chr2 | 75938273 | 8 | N | cg23768829 | chr10 | 8089653 | 25 | N |
| cg23051299 | chr2 | 75938289 | 8 | MU | cg20281962 | chr10 | 8089733 | 25 | MU |
| cg01044662 | chr2 | 75938360 | 8 | N | cg27542609 | chr10 | 8090924 | 25 | MU |
| cg23725986 | chr2 | 75938438 | 8 | N | cg14365271 | chrl0 | 21783701 | 25 | MU |
| cg12968518 | chr2 | 148602504 | 8 | MU | cg18794404 | chr10 | 22542024 | 25 | MU |
| cg11845111 | chr2 | 191398756 | 8 | N | cg09614653 | chr10 | 22542145 | 25 | N |
| cg27211696 | chr2 | 191398769 | 8 | N | cg11700824 | chr10 | 88730823 | 25 | N |
| cg10560079 | chr2 | 191398806 | 8 | MU | cg12191938 | chr10 | 88730946 | 25 | MU |
| cg00982136 | chr2 | 201726139 | 8 | MU | cg05627029 | chr10 | 122708532 | 25 | MU |

EP 4 741 515 A2

(continued)

| Illumina Probe Name | Chr. | Position | Tissue/Cell Type | MU/N |
|---|---|---|---|---|
| cg13315450 | chr2 | 220114289 | 8 | MU |
| cg01377358 | chr20 | 30948211 | 8 | MU |
| cg05056497 | chr21 | 35899448 | 8 | MU |
| cg14785464 | chr21 | 38362725 | 8 | N |
| cg06167719 | chr21 | 38362727 | 8 | MU |
| cg03419014 | chr21 | 38362742 | 8 | N |
| cg09037712 | chr21 | 38362754 | 8 | N |
| cg27653384 | chr22 | 22293118 | 8 | MU |
| cg24049841 | chr22 | 23484598 | 8 | MU |
| cg18467551 | chr22 | 23484600 | 8 | N |
| cg01381829 | chr22 | 40573054 | 8 | N |
| cg25276892 | chr22 | 40573076 | 8 | N |
| cg22508831 | chr22 | 40573204 | 8 | MU |
| cg15376996 | chr22 | 50912555 | 8 | MU |
| cg25381017 | chr3 | 3151795 | 8 | MU |
| cg08404225 | chr3 | 3151899 | 8 | N |
| cg26269881 | chr3 | 5023310 | 8 | MU |
| cg01180628 | chr3 | 5023394 | 8 | N |
| cg12637869 | chr3 | 121741209 | 8 | MU |
| cg13512830 | chr3 | 149530294 | 8 | N |
| cg12461469 | chr3 | 149530341 | 8 | MU |
| cg19980260 | chr3 | 171858472 | 8 | MU |
| cg06398251 | chr3 | 184033620 | 8 | MU |
| cg03956042 | chr3 | 194206497 | 8 | MU |
| cg22879098 | chr5 | 672845 | 8 | MU |
| cg24082121 | chr5 | 672872 | 8 | MU |
| cg11895835 | chr5 | 111078476 | 8 | MU |
| cg23256675 | chr5 | 111078579 | 8 | N |
| cg25518868 | chr5 | 140984057 | 8 | MU |
| cg27016579 | chr5 | 168778028 | 8 | MU |
| cg10692140 | chr6 | 30496072 | 8 | MU |
| cg27395200 | chr6 | 32942710 | 8 | MU |
| cg07275218 | chr6 | 32942714 | 8 | N |
| cg17451586 | chr6 | 32942808 | 8 | N |
| cg15603424 | chr11 | 13300592 | 25 | MU |
| cg08300419 | chr11 | 47208959 | 25 | MU |
| cg22879515 | chr11 | 111383515 | 25 | MU |
| cg21881253 | chr11 | 111383576 | 25 | MU |
| cg13767940 | chr11 | 111383603 | 25 | MU |
| cg01192900 | chr11 | 111383668 | 25 | N |
| cg03419885 | chr11 | 125036385 | 25 | MU |
| cg00737840 | chr11 | 125036420 | 25 | N |
| cg04299135, | chr11 | 129244441 | 25 | MU |
| cg17692125 | chr11 | 129244505 | 25 | N |
| cg04094148 | chr11 | 129244523 | 25 | N |
| cg25810484 | chr12 | 54089486 | 25 | MU |
| cg11560280 | chr12 | 54089497 | 25 | MU |
| cg23922739 | chr12 | 54338743 | 25 | N |
| cg08803500 | chr12 | 54338824 | 25 | MU |
| cg11306441 | chr12 | 54520919 | 25 | MU |
| cg19430489 | chr12 | 75728104 | 25 | MU |
| cg26343133 | chr12 | 75728149 | 25 | N |
| cg07684809 | chr12 | 75728212 | 25 | MU |
| cg02818811 | chr12 | 75728458 | 25 | N |
| cg21087137 | chr12 | 75728469 | 25 | MU |
| cg24234651 | chr12 | 75728471 | 25 | MU |
| cg25524962 | chr13 | 20735819 | 25 | MU |
| cg21115608 | chr13 | 20735860 | 25 | N |
| cg18255595 | chr13 | 20735871 | 25 | N |
| cg26717009 | chr13 | 20735967 | 25 | MU |
| cg15462349 | chr14 | 37116296 | 25 | N |
| cg14007757 | chr14 | 37116408 | 25 | MU |
| cg16400017 | chr14 | 371125155 | 25 | N |
| cg01672943 | chr14 | 37125292 | 25 | MU |
| cg01972418 | chr14 | 37125430 | 25 | MU |
| cg07035145 | chr14 | 51410689 | 25 | MU |
| cg14982203 | chr14 | 90084444 | 25 | MU |
| cg27017085 | chr15 | 51385812 | 25 | N |

(continued)

| Illumina Probe Name | Chr. | Position | Tissue/Cell | MU/N |
|---|---|---|---|---|
| cg14284211 | chr6 | 35570224 | 8 | MU |
| cg06493154 | chr6 | 42859023 | 8 | MU |
| cg26401541 | chr6 | 91078974 | 8 | MU |
| cg20227056 | chr6 | 130096463 | 8 | MU |
| cg05098343 | chr7 | 37071686 | 8 | MU |
| cg23220823 | chr7 | 45041140 | 8 | MU |
| cg13108141 | chr7 | 129590607 | 8 | MU |
| cg12858593 | chr8 | 22131899 | 8 | MU |
| cg13152952 | chr8 | 41570554 | 8 | MU |
| cg10601625 | chr8 | 41570622 | 8 | N |
| cg25801976 | chr8 | 48648112 | 8 | MU |
| cg24163242 | chr8 | 48648813 | 8 | MU |
| cg19414630 | chr8 | 48648874 | 8 | MU |
| cg00044505 | chr8 | 48649086 | 8 | MU |
| cg24636969 | chr8 | 81787111 | 8 | MU |
| cg12077319 | chr8 | 81787141 | 8 | N |
| cg13157980 | chr8 | 141599141 | 8 | MU |
| cg11006453 | chr8 | 141599185 | 8 | N |
| cg23731089 | chr8 | 141599208 | 8 | N |
| cg01150641 | chr1 | 976168 | 9 | MU |
| cg23625715 | chr1 | 976172 | 9 | MU |
| cg26222311 | chr1 | 976227 | 9 | MU |
| cg14609025 | chr1 | 119550983 | 9 | MU |
| cg15141467 | chr1 | 155035375 | 9 | MU |
| cg00585901 | chr1 | 155035388 | 9 | MU |
| cg11491247 | chr1 | 155035530 | 9 | N |
| cg03003689 | chr1 | 156674970 | 9 | MU |
| cg00329300 | chr1 | 161008462 | 9 | N |
| cg11731300 | chr1 | 161008542 | 9 | MU |
| cg00446235 | chr1 | 161008644 | 9 | N |
| cg27180868 | chr1 | 161008750 | 9 | MU |
| cg24161057 | chr1 | 161008811 | 9 | MU |
| cg13906823 | chr1 | 161008824 | 9 | MU |
| cg12426196 | chr1 | 161008826 | 9 | MU |

| Illumina Probe Name | Chr. | Position | Tissue/Cell Type | MU/N |
|---|---|---|---|---|
| cg18588323 | chr15 | 51385891 | 25 | N |
| cg05905176 | chr15 | 51385916 | 25 | MU |
| cg00063471 | chr15 | 51385970 | 25 | MU |
| cg13434638 | chr15 | 68723690 | 25 | MU |
| cg05099185 | chr15 | 68723805 | 25 | N |
| cg05196969 | chr15 | 76627594 | 25 | MU |
| cg08307030 | chr15 | 76634380 | 25 | N |
| cg10088491 | chr15 | 76634454 | 25 | N |
| cg14433497 | chrl5 | 76634516 | 25 | MU |
| cg19181244 | chr15 | 78237003 | 25 | MU |
| cg12160600 | chr17 | 73029893 | 25 | MU |
| cg05513069 | chr18 | 43608309 | 25 | N |
| cg24213437 | chr19 | 11529741 | 25 | N |
| cg02433671 | chr19 | 11529856 | 25 | MU |
| cg08198862 | chr19 | 11529947 | 25 | MU |
| cg08068820 | chr19 | 11530021 | 25 | MU |
| cg08000684 | chr19 | 11530025 | 25 | N |
| cg14913644 | chr19 | 11530065 | 25 | MU |
| cg09109450 | chr2 | 21022565 | 25 | MU |
| cg20248458 | chr2 | 21022587 | 25 | MU |
| cg21748207 | chr2 | 26395359 | 25 | N |
| cg24563094 | chr2 | 26395458 | 25 | MU |
| cg02096396 | chr2 | 26395556 | 25 | N |
| cg26117023 | chr2 | 74782096 | 25 | MU |
| cg24478096 | chr2 | 160761085 | 25 | MU |
| cg09323727 | chr2 | 176936436 | 25 | MU |
| cg00459623 | chr2 | 176936581 | 25 | MU |
| cg25180342 | chr2 | 176937957 | 25 | MU |
| cg07694390 | chr2 | 176937971 | 25 | N |
| cg07802350 | chr2 | 176956573 | 25 | MU |
| cg12503267 | chr2 | 176963315 | 25 | MU |
| cg17512364 | chr2 | 176963353 | 25 | MU |
| cg26708100 | chr2 | 176983815 | 25 | MU |
| cg10393811 | chr2 | 176983927 | 25 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg07135032 | chr1 | 165322099 | 9 | N | cg14142007 | chr2 | 176987225 | 25 | MU |
| cg14417498 | chr1 | 165322199 | 9 | MU | cg09968620 | chr2 | 176987383 | 25 | N |
| cg14168733 | chr1 | 201708718 | 9 | N | cg14991487 | chr2 | 176987404 | 25 | N |
| cg16023122 | chr1 | 201708836 | 9 | MU | cg04739647 | chr2 | 176987462 | 25 | MU |
| cg13877974 | chr1 | 201708888 | 9 | N | cg05167251 | chr2 | 176987465 | 25 | MU |
| cg01828733 | chr1 | 201708932 | 9 | N | cg22674699 | chr2 | 176987918 | 25 | MU |
| cg22577136 | chr1 | 206644165 | 9 | MU | cg22541735 | chr2 | 176988203 | 25 | MU |
| cg14673387 | chr10 | 47008185 | 9 | MU | cg14236662 | chr2 | 176988284. | 25 | N |
| cg12043818 | chr10 | 47008202 | 9 | N | cg25984400 | chr2 | 177001883 | 25 | MU |
| cg07990749 | chr10 | 47008316 | 9 | N | cg25903497 | chr2 | 219157103 | 25 | N |
| cg06456154 | chr10 | 119310356 | 9 | N | cg18357696 | chr2 | 219157111 | 25 | N |
| cg15526081 | chr10 | 119310404 | 9 | MU | cg08189615 | chr2 | 219157119 | 25 | MU |
| cg08451832 | chr10 | 119310964 | 9 | MU | cg06862374 | chr2 | 219736549 | 25 | MU |
| cg17160984 | chr10 | 131765778 | 9 | MU | cg11417653 | chr22 | 24181212 | 25 | MU |
| cg04705435 | chr11 | 17411270 | 9 | MU | cg25940946 | chr22 | 24181263 | 25 | N |
| cg19358442 | chr11 | 44325687 | 9 | N | cg25037461 | chr22 | 24181268 | 25 | N |
| cg04549333 | chr11 | 44325823 | 9 | MU | cg14774086 | chr22 | 24181270 | 25 | N |
| cg14144305 | chr11 | 44325960 | 9 | N | cg23350106 | chr3 | 9957221 | 25 | MU |
| cg11260848 | chr11 | 44326260 | 9 | N | cg26712080 | chr3 | 55519588 | 25 | MU |
| cg07696033 | chr11 | 44326410 | 9 | MU | cg00859352 | chr3 | 171176909 | 25 | N |
| cg25363445 | chr11 | 44326516 | 9 | N | cg23533254 | chr3 | 171176940 | 25 | MU |
| cg14045803 | chr11 | 72492461 | 9 | N | cg22546686 | chr4 | 82136823 | 25 | MU |
| cg08749455 | chr11 | 72492538 | 9 | MU | cg13798300 | chr4 | 82136839 | 25 | MU |
| cg21303763 | chr12 | 3309708 | 9 | N | cg15972560 | chr4 | 82136940 | 25 | N |
| cg23065927 | chr12 | 3309816 | 9 | MU | cg06214925 | chr4 | 109092628 | 25 | N |
| cg25021051 | chr12 | 53448178 | 9 | MU | cg12938003 | chr4 | 109092724 | 25 | N |
| cg09494609 | chr12 | 53448180 | 9 | MU | cg03149565 | chr4 | 109092769 | 25 | MU |
| cg15628956 | chr12 | 53448234 | 9 | MU | cg00597801 | chr4 | 109092918 | 25 | N |
| cg18922524 | chr12 | 54447136 | 9 | N | cg21982950 | chr5 | 1876200 | 25 | N |
| cg03923561 | chr12 | 54447220 | 9 | MU | cg07961015 | chr5 | 1876215 | 25 | N |
| cg02264990 | chr12 | 54447243 | 9 | MU | cg14564616 | chr5 | 1876337 | 25 | MU |
| cg09720701 | chr12 | 54447283 | 9 | MU | cg14451382 | chr5 | 1876397 | 25 | N |
| cg01579535 | chr12 | 54447292 | 9 | N | cg18349911 | chr5 | 1877135 | 25 | MU |
| cg01524853 | chr12 | 54447807 | 9 | N | cg15199326 | chr6 | 2842005 | 25 | N |

EP 4 741 515 A2

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg22747076 | chr12 | 54447873 | 9 | MU | cg19349369 | chr6 | 2842146 | 25 | MU |
| cg18563999 | chr12 | 54785003 | 9 | N | cg14652638 | chr6 | 2842215 | 25 | N |
| cg03946762 | chr12 | 54785043 | 9 | N | cg27056119 | chr6 | 2842239 | 25 | MU |
| cg20967139 | chr12 | 54785055 | 9 | MU | cg08542865 | chr6 | 2842248 | 25 | N |
| cg18886436 | chr12 | 54785171 | 9 | MU | cg00031256 | chr6 | 2842255 | 25 | MU |
| cg07000334 | chr12 | 54785286 | 9 | N | cg11393995 | chr6 | 2842359 | 25 | N |
| cg11856711 | chr12 | 54785309 | 9 | N | cg09602288 | chr6 | 10382800 | 25 | N |
| cg17655624 | chr12 | 54785320 | 9 | N | cg20168806 | chr6 | 10382828 | 25 | MU |
| cg08900101 | chr12 | 114833043 | 9 | MU | cg24336989 | chr6 | 10385903 | 25 | MU |
| cg05065572 | chr12 | 114833912 | 9 | N | cg11811610 | chr6 | 10390811 | 25 | N |
| cg12610207 | chr12 | 114833950 | 9 | MU | cg24452128 | chr6 | 10390919 | 25 | MU |
| cg01519253 | chr12 | 114886463 | 9 | MU | cg19607165 | chr6 | 10390961 | 25 | N |
| cg15964309 | chr12 | 114886561 | 9 | N | cg17754510 | chr6 | 10391412 | 25 | MU |
| cg24722950 | chr14 | 62035475 | 9 | N | cg05224741 | chr6 | 10422322 | 25 | MU |
| cg21890551 | chr14 | 62035585 | 9 | MU | cg24865270 | chr6 | 10422447 | 25 | N |
| cg04961582 | chr14 | 95239464 | 9 | N | cg03204678 | chr6 | 26614535 | 25 | MU |
| cg19870698 | chr14 | 95239570 | 9 | MU | cg16519587 | chr6 | 26614649 | 25 | MU |
| cg01796166 | chr14 | 95239586 | 9 | N | cg12831863 | chr6 | 28457210 | 25 | N |
| cg13660279 | chr16 | 54318221 | 9 | N | cg03778669 | chr6 | 28457246 | 25 | MU |
| cg09888562 | chr16 | 54318314 | 9 | MU | cg03089717 | chr6 | 28557411 | 25 | N |
| cg19761211 | chr16 | 70415365 | 9 | N | cg15880846 | chr6 | 28557424 | 25 | N |
| cg01269537 | chr16 | 70415514 | 9 | MU | cg23490638 | chr6 | 28557467 | 25 | N |
| cg03580106 | chr16 | 89283057 | 9 | MU | cg06866163 | chr6 | 28557486 | 25 | MU |
| cg16558432 | chr17 | 5403805 | 9 | MU | cg12683342 | chr6 | 28557518 | 25 | N |
| cg08554115 | chr17 | 5403907 | 9 | N | cg13922451 | chr6 | 28557542 | 25 | N |
| cg22298430 | chr17 | 5404330 | 9 | MU | cg03470207 | chr6 | 28574981 | 25 | N |
| cg27230784 | chr17 | 5404337 | 9 | MU | cg25315731 | chr6 | 28574994 | 25 | MU |
| cg06462347 | chr17 | 5404508 | 9 | N | cg20040488 | chr6 | 28956510 | 25 | N |
| cg18671949 | chr17 | 5404581 | 9 | MU | cg24485957 | chr6 | 28956565 | 25 | N |
| cg02532538 | chr17 | 33787739 | 9 | MU | cg02766845 | chr6 | 28956577 | 25 | MU |
| cg08151857 | chr17 | 46691820 | 9 | MU | cg17545346 | chr6 | 28956609 | 25 | N |
| cg22655521 | chr17 | 48194939 | 9 | MU | cg20691507 | chr6 | 28956683 | 25 | N |
| cg26647303 | chr19 | 2041955 | 9 | N | cg16470716 | chr6 | 28956719 | 25 | N |
| cg00434573 | chr19 | 2041960 | 9 | N | cg14449180 | chr6 | 29894619 | 25 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg18418335 | chr19 | 2042088 | 9 | MU | cg03995122 | chr6 | 29894642 | 25 | MU |
| cg11894504 | chr19 | 2042391 | 9 | MU | cg08231349 | chr6 | 29894644 | 25 | N |
| cg08550839 | chr19 | 35633553 | 9 | N. | cg00409917 | chr6 | 29894679 | 25 | N |
| cg21105227 | chr19 | 35633669 | 9 | MU | cg17217665 | chr6 | 44186914 | 25 | N |
| cg20441502 | chr19 | 35633794 | 9 | MU | cg04742345 | chr6 | 44186969 | 25 | N |
| cg06946452 | chr2 | 119606631 | 9 | N | cg11452354 | chr6 | 44187052 | 25 | MU |
| cg24001710 | chr2 | 119606695 | 9 | N | cg10519140 | chr6 | 44187076 | 25 | N |
| cg26990102 | chr2 | 119606746 | 9 | MU | cg13793145 | chr6 | 44187109 | 25 | N |
| cg09385093 | chr2 | 119607885 | 9 | MU | cg07053014 | chr6 | 44187132 | 25 | N |
| cg08138685 | chr2 | 119610526 | 9 | MU | cg05447008 | chr6 | 73331114 | 25 | MU |
| cg17342925 | chr2 | 119610605 | 9 | N | cg11008243 | chr6 | 73331238 | 25 | N |
| cg26091021 | chr2 | 177021826 | 9 | N | cg13332474 | chr7 | 25935147 | 25 | MU |
| cg10107434 | chr2 | 177021899 | 9 | MU | cg09339462 | chr7 | 25935223 | 25 | MU |
| cg18944451 | chr2 | 177023716 | 9 | N | cg06206902 | chr7 | 27191226 | 25 | MU |
| cg25916282 | chr2 | 177023760 | 9 | MU | cg16771406 | chr7 | 27191352 | 25 | N |
| cg05864326 | chr2 | 177030150 | 9 | N | cg08483376 | chr7 | 27285136 | 25 | MU |
| cg23547017 | chr2 | 177030171 | 9 | N | cg02194223 | chr7 | 27285433 | 25 | N |
| cg14666564 | chr2 | 177030228 | 9 | MU | cg01357429 | chr7 | 27285563 | 25 | MU |
| cg13768137 | chr2 | 177039426 | 9 | N | cg19480724 | chr7 | 27285831 | 25 | MU |
| cg12602409 | chr2 | 177039568 | 9 | MU | cg22964918 | chr7 | 27286075 | 25 | MU |
| cg08940511 | chr2 | 241922778 | 9 | MU | cg01680010 | chr7 | 96647117 | 25 | MU |
| cg15316660 | chr21 | 38119920 | 9 | MU | cg01029638 | chr7 | 120628874 | 25 | MU |
| cg09556952 | chr21 | 38119946 | 9 | N | cg11380909 | chr8 | 6658332 | 25 | N |
| cg01697719 | chr22 | 19754125 | 9 | MU | cg26356462 | chr8 | 6658407 | 25 | MU |
| cg08382235 | chr22 | 19754251 | 9 | MU | cg01917069 | chr8 | 6658537 | 25 | N |
| cg21507095 | chr22 | 19754448 | 9 | MU | cg02409878 | chr8 | 99960498 | 25 | N |
| cg22122410 | chr3 | 128212269 | 9 | MU | cg26570179 | chr8 | 99960500 | 25 | MU |
| cg19638477 | chr3 | 128212273 | 9 | N | cg19305111 | chr8 | 142428139 | 25 | MU |
| cg14781521 | chr3 | 129063090 | 9 | MU | cg00467244 | chr8 | 142428240 | 25 | MU |
| cg03458172 | chr3 | 157815672 | 9 | N | cg26425321 | chr8 | 142428317 | 25 | N |
| cg05401764 | chr3 | 157815808 | 9 | MU | cg10634619 | chr1 | 3663435 | 26 | N |
| cg12378888 | chr4 | 4867102 | 9 | MU | cg15472784 | chr1 | 3663531 | 26 | MU |
| cg14872952 | chr4 | 4867164 | 9 | N | cg25467973 | chr1 | 3663705 | 26 | MU |
| cg05020685 | chr4 | 40858965 | 9 | N | cg05491852 | chr1 | 3663832 | 26 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg23735712 | chr4 | 40858984 | 9 | MU | cg07166679 | chr1 | 12123735 | 26 | MU |
| cg05894734 | chr4 | 40859249 | 9 | MU | cg16007456 | chr1 | 101004638 | 26 | MU |
| cg06485940 | chr4 | 40859293 | 9 | N | cg20302133 | chr1 | 111217194 | 26 | MU |
| cg26244738 | chr4 | 40859344 | 9 | N | cg26013553 | chr1 | 111217406 | 26 | N |
| cg22773661 | chr4 | 48492225 | 9 | MU | cg11595545 | chr1 | 111217497 | 26 | MU |
| cg14249348 | chr4 | 48492304 | 9 | N | cg01423964 | chr1 | 111217575 | 26 | N |
| cg18342279 | chr4 | 48492344 | 9 | MU | cg05888917 | chr1 | 156626749 | 26 | MU |
| cgl3736376 | chr4 | 111539291 | 9 | N | cg00765922 | chr1 | 156626839 | 26 | N |
| cg20291436 | chr4 | 111539352 | 9 | MU | cg08660938 | chr1 | 161510171 | 26 | N |
| cg13193239 | chr4 | 111539479 | 9 | N | cg07706715 | chr1 | 161510194 | 26 | MU |
| cg16184803 | chr4 | 111542444 | 9 | N | cg17154724 | chr1 | 171810322 | 26 | MU |
| cg22717014 | chr4 | 111542473 | 9 | MU | cg04399751 | chr1 | 171810376 | 26 | N |
| cg04392082 | chr4 | 186732926 | 9 | MU | cg04986015 | chr1 | 171810433 | 26 | N |
| cg02790305 | chr4 | 186732932 | 9 | N | cg02011074 | chr1 | 171810468 | 26 | N |
| cg26785346 | chr4 | 186732936 | 9 | N | cg04932544 | chr1 | 210111403 | 26 | MU |
| cg01933073 | chr4 | 186732942 | 9 | N | cg24428042 | chr10 | 11784408 | 26 | MU |
| cg13921444 | chr4 | 186733024 | 9 | N | cg14032732 | chrl0 | 11784473 | 26 | N |
| cg04348265 | chr4 | 186733060 | 9 | N | cg20744625 | chr10 | 64578469 | 26 | MU |
| cg01054755 | chr5 | 138728685 | 9 | MU | cg19704726 | chr10 | 94450056 | 26 | MU |
| cg24761867 | chr5 | 138728704 | 9 | MU | cg26541003 | chr11 | 9594436 | 26 | MU |
| cg01278596 | chr5 | 139228062 | 9 | MU | cg16120811 | chr11 | 9594484 | 26 | N |
| cg15992535 | chr5 | 139228150 | 9 | MU | cg02364610 | chr11 | 46317036 | 26 | N |
| cg02009088 | chr5 | 139228153 | 9 | MU | cg14547067 | chr11 | 46317154 | 26 | MU |
| cg01627750 | chr5 | 139228242 | 9 | N | cg18877285 | chr11 | 124790815 | 26 | N |
| cg00050152 | chr5 | 180479829 | 9 | MU | cg09657963 | chr11 | 124790875 | 26 | MU |
| cg02337825 | chr5 | 180480982 | 9 | MU | cg17462074 | chr12 | 14133168 | 26 | N |
| cg26997880 | chr6 | 32055370 | 9 | N | cg17094171 | chr12 | 14133281 | 26 | MU |
| cg11823230 | chr6 | 32055385 | 9 | N | cg24226973 | chr12 | 14133336 | 26 | N |
| cg05483184 | chr6 | 32055393 | 9 | N | cg13028930 | chr12 | 14133404 | 26 | N |
| cg21460606 | chr6 | 32055402 | 9 | MU | cg21383810 | chr12 | 65515470 | 26 | MU |
| cg19807376 | chr6 | 32055447 | 9 | N | cg02818811 | chr12 | 75728458 | 26 | MU |
| cg15697476 | chr6 | 32055474 | 9 | N | cg21087137 | chr12 | 75728469 | 26 | MU |
| cg09996288 | chr6 | 32055517 | 9 | MU | cg24234651 | chr12 | 75728471 | 26 | MU |
| cg17001689 | chr6 | 32055525 | 9 | N | cg01638592 | chr12 | 111472183 | 26 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg02740950 | chr6 | 32055534 | 9 | N | cg02478448 | chr13 | 37006063 | 26 | N |
| cg18178010 | chr6 | 32055629 | 9 | N | cg18348647 | chr13 | 37006107 | 26 | MU |
| cg00704305 | chr6 | 127664950 | 9 | MU | cg18948722 | chr13 | 37006116 | 26 | N |
| cg01425666 | chr7 | 5468436 | 9 | MU | cg05137358 | chrl3 | 37006127 | 26 | N |
| cg06550177 | chr7 | 5469535 | 9 | MU | cg10158541 | chr13 | 37006265 | 26 | N |
| cg01145709 | chr7 | 10979247 | 9 | MU | cg17495912 | chr13 | 37006340 | 26 | MU |
| cg08929390 | chr7 | 10979341 | 9 | N | cg09357740 | chr14 | 21131543 | 26 | N |
| cg26551975 | chr7 | 27211555 | 9 | MU | cg22895601 | chr14 | 21131621 | 26 | MU |
| cg24988255 | chr7 | 27225396 | 9 | N | cg21915647 | chr14 | 21439301 | 26 | MU |
| cg25901381 | chr7 | 27225485 | 9 | N | cg09790137 | chr14 | 21439440 | 26 | N |
| cg17466857 | chr7 | 27225528 | 9 | MU | cg10924691 | chr14 | 21439548 | 26 | MU |
| cg10657141 | chr7 | 27225543 | 9 | N | cg19818764 | chr14 | 21439700 | 26 | MU |
| cg02087954 | chr7 | 100609534 | 9 | MU | cg11851456 | chr14 | 23938418 | 26 | N |
| cg03185704 | chr7 | 150020125 | 9 | N | cg18876990 | chr14 | 23938456 | 26 | MU |
| cg18221429 | chr7 | 150020136 | 9 | N | cg06765956 | chr14 | 42074886 | 26 | MU |
| cg07753583 | chr7 | 150020206 | 9 | N | cg26245531 | chr14 | 88459216 | 26 | MU |
| cg22747650 | chr7 | 150020218 | 9 | N | cg03419058 | chr15 | 26108391 | 26 | MU |
| cg11026333 | chr7 | 150020269 | 9 | MU | cg16389285 | chr15 | 26108399 | 26 | MU |
| cg01270001 | chr7 | 150020401 | 9 | MU | cg20124450 | chr15 | 26108401 | 26 | MU |
| cg22893248 | chr7 | 150020751 | 9 | N | cg22113930 | chr15 | 26108410 | 26 | MU |
| cg21516287 | chr7 | 150020855 | 9 | N | cg26230285 | chr15 | 26108412 | 26 | MU |
| cg02639123 | chr7 | 150020881 | 9 | MU | cg14910265 | chr15 | 45670406 | 26 | N |
| cg02119840 | chr7 | 150020900 | 9 | N | cg11431346 | chr15 | 45670478 | 26 | N |
| cg11347411 | chr7 | 150020925 | 9 | N | cg01145430 | chr15 | 45670526 | 26 | MU |
| cg11740103 | chr7 | 150020945 | 9 | N | cg25908973 | chr15 | 45670865 | 26 | MU |
| cg04624228 | chr8 | 38411598 | 9 | N | cg10088041 | chr15 | 45670872 | 26 | MU |
| cg04458368 | chr8 | 38411682 | 9 | N | cg00645339 | chr15 | 45670875 | 26 | MU |
| cg24859937 | chr8 | 38411708 | 9 | MU | cg26796135 | chr15 | 45671001 | 26 | N |
| cg15183991 | chr8 | 145047566 | 9 | N | cg24328539 | chr15 | 45671016 | 26 | N |
| cg00886954 | chr8 | 145047713 | 9 | MU | cg05896902 | chr15 | 45671018 | 26 | N |
| cg08455275 | chr8 | 145047979 | 9 | MU | cg25274052 | chr15 | 72565016 | 26 | MU |
| cg11224624 | chr8 | 145049280 | 9 | MU | cg08294267 | chr15 | 72565024 | 26 | N |
| cg08909802 | chr8 | 145049419 | 9 | N | cg20973720 | chr15 | 79383167 | 26 | MU |
| cg21183329 | chr9 | 124976219 | 9 | MU | cg17360233 | chr16 | 10479841 | 26 | N |

EP 4 741 515 A2

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg14061378 | chr9 | 139539092 | 9 | MU | cg06754445 | chr16 | 10479875 | 26 | N |
| cg01868612 | chr1 | 2478046 | 10 | N | cg05064297 | chr16 | 10479966 | 26 | MU |
| cg03529908 | chr1 | 2478089 | 10 | MU | cg03131298 | chr16 | 31580889 | 26 | MU |
| cg02299950 | chr1 | 3087637 | 10 | N | cg04764012 | chr16 | 31580964 | 26 | N |
| cg20154143 | chr1 | 3087709 | 10 | N | cg10343447 | chr16 | 68544200 | 26 | MU |
| cg14217558 | chr1 | 3087734 | 10 | MU | cg26946978 | chr16 | 81779881 | 26 | N |
| cg05347736 | chr1 | 6302164 | 10 | MU | cg27595997 | chr16 | 81779999 | 26 | MU |
| cg22733648 | chr1 | 6302273 | 10 | N | cg08989441 | chr17 | 10633067 | 26 | MU |
| cg15524063 | chr1 | 43832916 | 10 | MU | cg20668644 | chr17 | 38347603 | 26 | MU |
| cg25385412 | chr1 | 43832951 | 10 | MU | cg19827780 | chr17 | 38347659 | 26 | N |
| cg03793131 | chr1 | 43833547 | 10 | N | cg00129651 | chr17 | 38347710 | 26 | N |
| cg22397520 | chr1 | 43833587 | 10 | N | cg06266993 | chr17 | 46671131 | 26 | N |
| cg24729481 | chr1 | 43833600 | 10 | N | cg11155697 | chr17 | 46671234 | 26 | N |
| cg24838029 | chr1 | 43833615 | 10 | MU | cg02712075 | chr17 | 46671244 | 26 | MU |
| cg08936794 | chr1 | 43833873 | 10 | N | cg10153335 | chr17 | 46671293 | 26 | MU |
| cg20301722 | chr1 | 43833891 | 10 | MU | cg02293936 | chr17 | 46671298 | 26 | N |
| cg18540614 | chr1 | 43833945 | 10 | N | cg24626312 | chr17 | 46671332 | 26 | MU |
| cg11798658 | chr1 | 47082039 | 10 | MU | cg14126688 | chr17 | 46671497 | 26 | MU |
| cg23589045 | chr1 | 51983759 | 10 | MU | cg01405107 | chr17 | 46671635 | 26 | N |
| cg07976644 | chr1 | 55266932 | 10 | MU | cg08151857 | chr17 | 46691820 | 26 | MU |
| cg19808825 | chr1 | 55267006 | 10 | N | cg15892664 | chr17 | 53315578 | 26 | MU |
| cg22130834 | chr1 | 55267046 | 10 | N | cg27641522 | chr18 | 53447560 | 26 | N |
| cg01240734 | chr10 | 71892593 | 10 | MU | cg07519816 | chr18 | 53447596 | 26 | MU |
| cg15779600 | chr10 | 71892694 | 10 | N | cg13216267 | chr18 | 77793342 | 26 | MU |
| cg26699283 | chr10 | 71892715 | 10 | N | cg02401399 | chr19 | 36523584 | 26 | MU |
| cg20241658 | chr10 | 71892719 | 10 , | N | cg24027100 | chr19 | 36822549 | 26 | N |
| cg18993457 | chr11 | 441983 | 10 | N | cg21808240 | chr19 | 36822564 | 26 | N |
| cg04021962 | chr11 | 441986 | 10 | MU | cg02810156 | chr19 | 36822672 | 26 | MU |
| cg05904366 | chr11 | 442072 | 10 | N | cg22456785 | chr19 | 36822770 | 26 | N |
| cg24932241 | chr11 | 442077 | 10 | N | cg19406736 | chr19 | 37096010 | 26 | N |
| cg24064264 | chr11 | 615945 | 10 | N | cg25397945 | chr19 | 37096148 | 26 | MU |
| cg07652350 | chr11 | 616009 | 10 | N | cg02587316 | chr19 | 37096321 | 26 | N |
| cg13145591 | chr11 | 616038 | 10 | MU | cg05020604 | chr19 | 37096329 | 26 | MU |
| cg13378284 | chr11 | 616089 | 10 | N | cg07602980 | chr19 | 50815719 | 26 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg18971819 | chr11 | 616112 | 10 | N | cg05661282 | chr19 | 58220370 | 26 | N |
| cg05122965 | chr11 | 5706362 | 10 | MU | cg21790626 | chr19 | 58220494 | 26 | MU |
| cg01799588 | chr11 | 5706381 | 10 | N | cg27049766 | chr19 | 58220516 | 26 | N |
| cg12471950 | chr11 | 5706401 | 10 | N | cg10659886 | chr19 | 58629719 | 26 | N |
| cg24098643 | chr11 | 5706435 | 10 | N | cg08873805 | chr19 | 58629837 | 26 | MU |
| cg09347923 | chr11 | 47736425 | 10 | N | cg14231297 | chr19 | 58629901 | 26 | N |
| cg19112957 | chr11 | 47736463 | 10 | MU | cg17408527 | chr19 | 58629967 | 26 | N |
| cg25168700 | chr11 | 64067685 | 10 | N | cg18693673 | chr19 | 58629975 | 26 | N |
| cg24027931 | chr11 | 64067687 | 10 | N | cg01354088 | chr2 | 10183140 | 26 | N |
| cg04855748 | chr11 | 64067763 | 10 | N | cg23458613 | chr2 | 10183227 | 26 | N |
| cg18383813 | chr11 | 64067793 | 10 | N | cg12005760 | chr2 | 10183271 | 26 | MU |
| cg09675196 | chr11 | 64067795 | 10 | MU | cg01777397 | chr2 | 29337946 | 26 | N |
| cg05098417 | chr11 | 64067858 | 10 | N | cg07285673 | chr2 | 29337984 | 26 | N |
| cg23069847 | chr11 | 64067868 | 10 | N | cg09695033 | chr2 | 29337988 | 26 | N |
| cg15217978 | chr11 | 65343330 | 10 | N | cg06333058 | chr2 | 29338077 | 26 | MU |
| cg02532488 | chr11 | 65343341 | 10 | N | cg25737323 | chr2 | 29338100 | 26 | MU |
| cg18765439 | chr11 | 65343343 | 10 | MU | cg13257636 | chr2 | 29338109 | 26 | N |
| cg10725932 | chr11 | 71934276 | 10 | MU | cg23428985 | chr2 | 29338113 | 26 | MU |
| cg22884792 | chr12 | 49371987 | 10 | MU | cg23255835 | chr2 | 29338121 | 26 | N |
| cg09158990 | chr12 | 49372112 | 10 | N | cg03135351 | chr2 | 29338258 | 26 | MU |
| cg02067983 | chr12 | 49372131 | 10 | N | cg08808128 | chr2 | 29338432 | 26 | MU |
| cg12693595 | chr12 | 57504318 | 10 | N | cg01648237 | chr2 | 61372066 | 26 | N |
| cg00500522 | chr12 | 57504387 | 10 | MU | cg05037927 | chr2 | 61372117 | 26 | N |
| cg22905097 | chr13 | 43566401 | 10 | N | cg16328106 | chr2 | 61372138 | 26 | MU |
| cg19915711 | chr13 | 43566421 | 10 | N | cg19790321 | chr2 | 61372165 | 26 | N |
| cg22125968 | chr13 | 43566472 | 10 | MU | cg24757310 | chr2 | 61372226 | 26 | MU |
| cg01536987 | chr13 | 43566492 | 10 | MU | cg18158151 | chr2 | 61372256 | 26 | N |
| cg03478249 | chr13 | 43566547 | 10 | N | cg21185289 | chr2 | 74743437 | 26 | MU |
| cg12138678 | chr13 | 43566618 | 10 | N | cg05545132 | chr2 | 144695078 | 26 | MU |
| cg16327891 | chr13 | 43566633 | 10 | N | cg23867997 | chr20 | 61810624 | 26 | MU |
| cg26714230 | chr13 | 43566642 | 10 | N | cg13604020 | chr22 | 38902925 | 26 | MU |
| cg18732064 | chr14 | 23356059 | 10 | MU | cg00836482 | chr3 | 119528956 | 26 | MU |
| cg03577460 | chr14 | 24836084 | 10 | N | cg08988364 | chr3 | 143566850 | 26 | MU |
| cg05330668 | chr14 | 24836090 | 10 | MU | cg03290752 | chr3 | 176915082 | 26 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg25076767 | chr14 | 24836148 | 10 | MU | cg18780412 | chr3 | 179755086 | 26 | MU |
| cg15346168 | chr14 | 24836194 | 10 | N | cg21176048 | chr3 | 179755235 | 26 | N |
| cg26705574 | chr14 | 24836219 | 10 | N | cg02728595 | chr3 | 196255632 | 26 | MU |
| cg04134240 | chr14 | 100680625 | 10 | MU | cg14660125 | chr4 | 89378460 | 26 | MU |
| cg24498031 | chr15 | 40545050 | 10 | N | cg26590411 | chr4 | 119199646 | 26 | MU |
| cg13892980 | chr15 | 40545143 | 10 | MU | cg03405515 | chr4 | 156297854 | 26 | MU |
| cg18097281 | chr15 | 40545145 | 10 | MU | cg03616357 | chr4 | 156297942 | 26 | N |
| cg12635019 | chr15 | 40545175 | 10 | MU | cg20334243 | chr4 | 156298002 | 26 | MU |
| cg04305879 | chr15 | 40545215 | 10 | N | cg08015755 | chr4 | 156298050 | 26 | N |
| cg11939465 | chr15 | 40545224 | 10 | N | cg13933262 | chr5 | 140797172 | 26 | MU |
| cg05068428 | chr15 | 40545296 | 10 | MU | cg14011639 | chr5 | 140797234 | 26 | N |
| cg01036164 | chr15 | 40545324 | 10 | N | cg10435816 | chr5 | 140797280 | 26 | N |
| cg04812615 | chr15 | 40545326 | 10 | N | cg23563234 | chr5 | 140797286 | 26 | N |
| cg05426702 | chr15 | 40574544 | 10 | MU | cg02527199 | chr5 | 175085245 | 26 | MU |
| cg22409276 | chr15 | 40763763 | 10 | MU | cg07524827 | chr5 | 175085316 | 26 | N |
| cg24740818 | chr15 | 76440405 | 10 | N | cg12684209 | chr6 | 393110 | 26 | N |
| cg08557928 | chr15 | 76440443 | 10 | MU | cg21277995 | chr6 | 393239 | 26 | MU |
| cg18021161 | chr15 | 76440448 | 10 | MU | cg27131891 | chr6 | 27513414 | 26 | N |
| cg14912033 | chr15 | 79043428 | 10 | N | cg05310764 | chr6 | 27513479 | 26 | MU |
| cg07120314 | chr15 | 79043507 | 10 | MU | cg11623922 | chr6 | 27759386 | 26 | MU |
| cg03148231 | chr15 | 89954845 | 10 | MU | cg17438737 | chr6 | 27759399 | 26 | N |
| cg10308906 | chr15 | 89954991 | 10 | N | cg24799187 | chr6 | 27759410 | 26 | N |
| cg12258919 | chr16 | 2521473 | 10 | MU | cg18055610 | chr6 | 30095419 | 26 | N |
| cg10504751 | chr16 | 56390830 | 10 | MU | cg19059495 | chr6 | 30095495 | 26 | N |
| cg03022368 | chr16 | 83986702 | 10 | N | cg01964152 | chr6 | 30095546 | 26 | N |
| cg08589960 | chr16 | 83986755 | 10 | MU | cg08548396 | chr6 | 30095549 | 26 | MU |
| cg03891598 | chr16 | 83986782 | 10 | N | cg12534216 | chr6 | 30095570 | 26 | N |
| cg00187635 | chr16 | 83986795 | 10 | N | cg20383155 | chr6 | 31831428 | 26 | N |
| cg04313875 | chr17 | 1553202 | 10 | MU | cg18363192 | chr6 | 31831434 | 26 | N |
| cg15380836 | chr17 | 1553341 | 10 | N | cg27347290 | chr6 | 31831439 | 26 | N |
| cg08967106 | chr17 | 35014412 | 10 | MU | cg22753340 | chr6 | 31831489 | 26 | N |
| cg00371418 | chr17 | 35014438 | 10 | N | cg23174932 | chr6 | 31831502 | 26 | MU |
| cg08377924 | chr17 | 41363628 | 10 | N | cg00397479 | chr6 | 31831510 | 26 | N |
| cg19548479 | chr17 | 41363737 | 10 | N | cg17353698 | chr6 | 31831599 | 26 | N |

(continued)

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg24940138 | chr17 | 41363741 | 10 | MU | cg18857062 | chr6 | 43276478 | 26 | N |
| cg21211480 | chr17 | 41363774 | 10 | MU | cg11982736 | chr6 | 43276570 | 26 | N |
| cg15026277 | chr17 | 41363789 | 10 | MU | cg27291698 | chr6 | 43276578 | 26 | MU |
| cg03049782 | chr17 | 41363891 | 10 | N | cg21109265 | chr6 | 43276647 | 26 | N |
| cg24008544 | chr17 | 41363899 | 10 | N | cg13097407 | chr6 | 43276658 | 26 | N |
| cg18222083 | chr17 | 41364007 | 10 | MU | cg07544038 | chr6 | 71122681 | 26 | MU |
| cg04482110 | chr17 | 41364121 | 10 | N | cg27331241 | chr7 | 751830 | 26 | MU |
| cg10842515 | chr17 | 43325167 | 10 | N | cg00356183 | chr7 | 751833 | 26 | N |
| cg19411214 | chr17 | 43325316 | 10 | MU | cg25599475 | chr7 | 853038 | 26 | MU |
| cg25533943 | chr17 | 43325323 | 10 | N | cg02300154 | chr7 | 70597058 | 26 | MU |
| cg21185826 | chr17 | 47979513 | 10 | MU | cg01366419 | chr7 | 70597091 | 26 | N |
| cg03877957 | chr17 | 47979595 | 10 | N | cg17314888 | chr7 | 73442282 | 26 | N |
| cg00023001 | chr17 | 55938748 | 10 | N | cg03897712 | chr7 | 73442370 | 26 | MU |
| cg22451358 | chr17 | 55938871 | 10 | MU | cg16762778 | chr8 | 27449867 | 26 | MU |
| cg06452419 | chr17 | 55938940 | 10 | N | cg08970347 | chr8 | 27449907 | 26 | MU |
| cg14511665 | chr17 | 62773096 | 10 | MU | cg24592790 | chr8 | 27450047 | 26 | N |
| cg06792847 | chr17 | 62773246 | 10 | N | cg22230538 | chr8 | 28258841 | 26 | MU |
| cg19598544 | chr17 | 72209156 | 10 | N | cg13744917 | chr1 | 2986538 | 27 | N |
| cg14506834 | chr17 | 72209198 | 10 | MU | cg12267948 | chr1 | 2986566 | 27 | MU |
| cg11887884 | chr17 | 79486500 | 10 | MU | cg08640609 | chr1 | 7764642 | 27 | MU |
| cg18395558 | chr19 | 12939446 | 10 | N | cg18086187 | chr1 | 10057158 | 27 | N |
| cg09463900 | chr19 | 12939587 | 10 | MU | cg03994053 | chr1 | 10057168 | 27 | N |
| cg16005540 | chr19 | 13983813 | 10 | MU | cg23107691 | chr1 | 10057303 | 27 | MU |
| cg27346356 | chr19 | 19648914 | 10 | N | cg27561954 | chr1 | 10057312 | 27 | N |
| cg23479742 | chr19 | 19648939 | 10 | N | cg09820321 | chr1 | 15251269 | 27 | MU |
| cg01487433 | chr19 | 19648987 | 10 | N | cg11141156 | chr1 | 15251288 | 27 | N |
| cg21073520 | chr19 | 19648991 | 10 | N | cg27303880 | chr1 | 64240188 | 27 | N |
| cg13674885 | chr19 | 19649041 | 10 | N | cg00504690 | chr1 | 64240202 | 27 | MU |
| cg09386096 | chr19 | 19649054 | 10 | MU | cg06805280 | chr1 | 86046570 | 27 | MU |
| cg10313673 | chr19 | 19649144 | 10 | N | cg04453065 | chr1 | 86046661 | 27 | N |
| cg01854969 | chr19 | 35644903 | 10 | MU | cg04997273 | chr1 | 117452884 | 27 | MU |
| cg09966364 | chr19 | 35644908 | 10 | N | cg11221228 | chr1 | 117452905 | 27 | N |
| cg11840540 | chr19 | 35644996 | 10 | N | cg14343890 | chr1 | 228135758 | 27 | MU |
| cg22618240 | chr19 | 35645556 | 10 | N | cg23020486 | chr1 | 236558874 | 27 | MU |

(continued)

| Illumina Probe Name | Chr. | Position | Tissue e/Cell Type | MU/N |
|---|---|---|---|---|
| cg08461107 | chr10 | 30348294 | 27 | N |
| cg12835845 | chr10 | 30348415 | 27 | MU |
| cg12870705 | chr10 | 33624623 | 27 | MU |
| cg18384214 | chr10 | 112836185 | 27 | N |
| cg16358257 | chr10 | 112836609 | 27 | MU |
| cg07560825 | chr10 | 112836681 | 27 | N |
| cg05238375 | chr10 | 112836780 | 27 | N |
| cg10524152 | chr10 | 112836785 | 27 | MU |
| cg26369667 | chr11 | 101981789 | 27 | MU |
| cg18718634 | chr11 | 118023765 | 27 | N |
| cg21095553 | chr11 | 127753433 | 27 | MU |
| cg01591431 | chr11 | 127753443 | 27 | MU |
| cg05445632 | chr11 | 134282404 | 27 | MU |
| cg03853227 | chr12 | 107487365 | 27 | N |
| cg03742214 | chr12 | 107487383 | 27 | MU |
| cg09652427 | chr12 | 107712120 | 27 | N |
| cg19029509 | chr12 | 107712140 | 27 | N |
| cg08612037 | chr12 | 107712210 | 27 | MU |
| cg13882203 | chr12 | 120806178 | 27 | N |
| cg04101969 | chr13 | 20806477 | 27 | MU |
| cg07148716 | chr13 | 20806539 | 27 | N |
| cg21794428 | chr13 | 21635420 | 27 | MU |
| cg13141192 | chr14 | 21492975 | 27 | MU |
| cg23690444 | chr14 | 81902736 | 27 | MU |
| cg06623560 | chr14 | 81902963 | 27 | N |
| cg22393360 | chr14 | 81902974 | 27 | N |
| cg06903818 | chr14 | 105444575 | 27 | MU |
| cg07828276 | chr14 | 105444724 | 27 | MU |
| cg22696438 | chr15 | 71146679 | 27 | MU |
| cg25959239 | chr15 | 71146682 | 2 7 | N |
| cg26653147 | chr15 | 71146819 | 27 | N |
| cg20433014 | chr15 | 75018583 | 27 | N |
| cg01919118 | chr15 | 75018731 | 27 | N |
| cg27051335 | chr15 | 75018733 | 27 | MU |

| Illumina Probe Name | Chr. | Position | Tissue/Cell | MU/N |
|---|---|---|---|---|
| cg26721700 | chr19 | 35645598 | 10 | N |
| cg09386954 | chr19 | 35645620 | 10 | MU |
| cg11260421 | chr19 | 35645680 | 10 | N |
| cg16860694 | chr19 | 35645689 | 10 | MU |
| cg10227705 | chr19 | 35645712 | 10 | MU |
| cg03096975 | chr19 | 46142574 | 10 | N |
| cg07804711 | chr19 | 46142647 | 10 | N |
| cg12536502 | chr19 | 46142673 | 10 | MU |
| cg07749923 | chr19 | 46142711 | 10 | N |
| cg01544351 | chr19 | 46142757 | 10 | N |
| cg23696132 | chr19 | 46273771 | 10 | MU |
| cg14149007 | chr19 | 47219555 | 10 | MU |
| cg23864632 | chr19 | 47220401 | 10 | MU |
| cg12568362 | chr19 | 47220607 | 10 | N |
| cg08230695 | chr19 | 47220755 | 10 | MU |
| cg13685883 | chr19 | 47220848 | 10 | N |
| cg09829983 | chr2 | 7017698 | 10 | N |
| cg18201077 | chr2 | 7017787 | 10 | MU |
| cg26360792 | chr2 | 43019997 | 10 | N |
| cg27299406 | chr2 | 43020013 | 10 | MU |
| cg09480054 | chr2 | 43020129 | 10 | N |
| cg22194948 | chr2 | 43447139 | 10 | MU |
| cg22198946 | chr2 | 43454609 | 10 | MU |
| cg06907744 | chr2 | 43454761 | 10 | N |
| cg16202615 | chr2 | 43454763 | 10 | N |
| cg00338928 | chr2 | 43454817 | 10 | MU |
| cg23201549 | chr2 | 43454852 | 10 | MU |
| cg03771385 | chr2 | 43454876 | 10 | N |
| cg23038392 | chr2 | 43454950 | 10 | N |
| cg04707074 | chr2 | 43454972 | 10 | N |
| cg15300730 | chr2 | 43454991 | 10 | N |
| cg09456094 | chr2 | 231280878 | 10 | N |
| cg11827097 | chr2 | 231280903 | 10 | N |
| cg11702456 | chr2 | 231280907 | 10 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg26008007 | chr2 | 232545866 | 10 | MU | cg03797501 | chr15 | 75018774 | 27 | N |
| cg09030830 | chr20 | 43280135 | 10 | N | cg20004910 | chr15 | 75018852 | 27 | N |
| cg11091439 | chr20 | 43280257 | 10 | MU | cg02705800 | chr15 | 93199018 | 27 | MU |
| cg04839664 | chr20 | 55964528 | 10 | MU | cg25864024 | chr15 | 93199037 | 27 | N |
| cg24210478 | chr20 | 62733486 | 10 | MU | cg15289316 | chr15 | 93199049 | 27 | MU |
| cg17297071 | chr21 | 26934424 | 10 | N | cg13784264 | chr15 | 93199130 | 27 | N |
| cg23433889 | chr21 | 26934455 | 10 | MU | cg20823977 | chr15 | 93199141 | 27 | N |
| cg12749863 | chr21 | 26934576 | 10 | N | cg07258794 | chr16 | 326049 | 27 | MU |
| cg17506742 | chr21 | 34638971 | 10 | N | cg00609966 | chr16 | 1202819 | 27 | N |
| cg01855674 | chr21 | 34639040 | 10 | N | cg18816877 | chr16 | 1202923 | 27 | MU |
| cg14280424 | chr21 | 34639113 | 10 | MU | cg16393726 | chr16 | 56623312 | 27 | N |
| cg16007279 | chr22 | 38598948 | 10 | MU | cg08270206 | chr16 | 56623390 | 27 | MU |
| cg04169403 | chr22 | 45098800 | 10 | N | cg02562519 | chr16 | 85202632 | 27 | MU |
| cg01608788 | chr22 | 45098907 | 10 | MU | cg19118797 | chr17 | 1613572 | 27 | MU |
| cg18772127 | chr3 | 32443496 | 10 | MU | cg25678929 | chr17 | 1613581 | 27 | MU |
| cg02829783 | chr3 | 38180432 | 10 | N | cg19175649 | chr17 | 15164120 | 27 | N |
| cg27043630 | chr3 | 38180527 | 10 | MU | cg26388450 | chr17 | 15164133 | 27 | N |
| cg11816841 | chr3 | 46734995 | 10 | N | cg18011045 | chr17 | 15164270 | 27 | MU |
| cg05369142 | chr3 | 46735009 | 10 | MU | cg22219136 | chr17 | 15164284 | 27 | N |
| cg19998675 | chr3 | 182970903 | 10 | N | cg16462237 | chr17 | 19770908 | 27 | N |
| cg20585088 | chr3 | 182970927 | 10 | N | cg09795194 | chr17 | 19770910 | 27 | MU |
| cg12930573 | chr3 | 182971009 | 10 | N | cg04611479 | chr17 | 41910366 | 27 | MU |
| cg16938490 | chr3 | 182971025 | 10 | MU | cg01147550 | chr17 | 47210027 | 27 | MU |
| cg02238826 | chr3 | 182971069 | 10 | N | cg01429662 | chr17 | 47210162 | 27 | N |
| cg11102611 | chr3 | 182971663 | 10 | MU | cg22259831 | chr17 | 47210166 | 27 | N |
| cg12927498 | chr5 | 141031122 | 10 | MU | cg18392085 | chr17 | 63556315 | 27 | MU |
| cg09904383 | chr5 | 141031125 | 10 | N | cg18106189 | chr18 | 13218595 | 27 | MU |
| cg15844835 | chr5 | 141031142 | 10 | MU | cg21349637 | chr18 | 13218607 | 27 | MU |
| cg08742288 | chr5 | 141031147 | 10 | MU | cg19496782 | chr18 | 19749376 | 27 | N |
| cg13267747 | chr5 | 141031176 | 10 | N | cg03473387 | chr18 | 19749395 | 27 | MU |
| cg01849970 | chr5 | 141031209 | 10 | N | cg16485682 | chr18 | 19749540 | 27 | N |
| cg07844112 | chr5 | 180230022 | 10 | N | cg03143886 | chr18 | 22006473 | 27 | N |
| cg19605920 | chr5 | 180230113 | 10 | MU | cg24949251 | chr18 | 22006480 | 27 | N |
| cg16332813 | chr5 | 180230115 | 10 | N | cg13865352 | chr18 | 22006621 | 27 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg15177964 | chr5 | 180230130 | 10 | N | cg19083143 | chr18 | 22006626 | 27 | N |
| cg07218275 | chr5 | 180230146 | 10 | N | cg11864882 | chr18 | 43652232 | 27 | N |
| cg09867128 | chr6 | 31795671 | 10 | MU | cg17329670 | chr18 | 43652255 | 27 | MU |
| cg03731673 | chr6 | 33393599 | 10 | N | cg21949781 | chr18 | 43652366 | 27 | N |
| cg04614173 | chr6 | 33393607 | 10 | MU | cg24026435 | chr18 | 43652398 | 27 | N |
| cg06789667 | chr6 | 33393635 | 10 | N | cg03877492 | chr19 | 1567421 | 27 | N |
| cg03650397 | chr6 | 159239229 | 10 | MU | cg13647706 | chr19 | 1567517 | 27 | MU |
| cg17222941 | chr7 | 77648567 | 10 | MU | cg12313593 | chr19 | 15619224 | 27 | N |
| cg10271408 | chr7 | 77648600 | 10 | N | cg24456663 | chr19 | 15619329 | 27 | N |
| cg18078841 | chr7 | 77648624 | 10 | N | cg17716453 | chr19 | 15619355 | 27 | MU |
| cg12025522 | chr7 | 77649330 | 10 | MU | cg03801179 | chr2 | 20212423 | 27 | MU |
| cg04976780 | chr7 | 77649540 | 10 | MU | cg21177096 | chr2 | 20212440 | 27 | N |
| cg01914455 | chr7 | 77649564 | 10 | N | cg12299199 | chr2 | 20212501 | 27 | N |
| cg03574119 | chr7 | 100728591 | 10 | N | cg00462460 | chr2 | 20212509 | 27 | N |
| cg22607212 | chr7 | 100728652 | 10 | N | cg00261781 | chr2 | 20212517 | 27 | N |
| cg06772157 | chr7 | 100728711 | 10 | N | cg18705408 | chr2 | 20212524 | 27 | N |
| cg05401447 | chr7 | 100728731 | 10 | MU | cg13451280 | chr2 | 68870812 | 27 | MU |
| cg23063913 | chr7 | 100728792 | 10 | N | cg01759126 | chr2 | 68870892 | 27 | N |
| cg26385074 | chr7 | 100816740 | 10 | MU | cg00394494 | chr2 | 167232645 | 27 | MU |
| cg15654626 | chr8 | 103613080 | 10 | MU | cg22528044 | chr2 | 167232679 | 27 | N |
| cg11995594 | chr8 | 103613219 | 10 | N | cg18047082 | chr2 | 167232696 | 27 | N |
| cg25548415 | chr8 | 145016475 | 10 | MU | cg06552037 | chr2 | 220435838 | 27 | MU |
| cg25678052 | chr8 | 145016627 | 10 | N | cg00506811 | chr2 | 235860443 | 27 | MU |
| cg19407252 | chr8 | 145016746 | 10 | MU | cg23683528 | chr2 | 235860449 | 27 | MU |
| cg22840464 | chr8 | 145016828 | 10 | N | cg05096436 | chr2 | 236578251 | 27 | MU |
| cg21550172 | chr8 | 145018075 | 10 | N | cg07217075 | chr20 | 17511826 | 27 | MU |
| cg26763284 | chr8 | 145018185 | 10 | MU | cg24580066 | chr20 | 18037540 | 27 | MU |
| cg10319399 | chr8 | 145018285 | 10 | N | cg12358000 | chr20 | 25566178 | 27 | MU |
| cg23715237 | chr8 | 145018300 | 10 | N | cg13727122 | chr20 | 25566180 | 27 | MU |
| cg14154487 | chr9 | 125133314 | 10 | MU | cg01240094 | chr21 | 33784522 | 27 | N |
| cg12927153 | chr9 | 125133318 | 10 | MU | cg07773095 | chr21 | 33784558 | 27 | MU |
| cg15090202 | chr1 | 26687087 | 11 | MU | cg00734483 | chr21 | 42539961 | 27 | MU |
| cg04661629 | chr1 | 26687174 | 11 | N | cg16713274 | chr21 | 46825190 | 27 | MU |
| cg22224989 | chr1 | 27986522 | 11 | MU | cg15243570 | chr22 | 20792217 | 27 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg11224362 | chr1 | 40041493 | 11 | MU | cg03952331 | chr22 | 20792222 | 27 | MU |
| cg00630164 | chr1 | 41250062 | 11 | N | cg19840066 | chr22 | 20792224 | 27 | N |
| cg13438961 | chr1 | 41250071 | 11 | MU | cg03448766 | chr22 | 20792243 | 27 | N |
| cg26520930 | chr1 | 41250117 | 11 | N | cg14998335 | chr22 | 20792255 | 27 | N |
| cg17952661 | chr1 | 145395753 | 11 | MU | cg04608146 | chr22 | 20792323 | 27 | N |
| cg02395363 | chr1 | 145395846 | 11 | N | cg21716444 | chr22 | 37915250 | 27 | N |
| cg09897416 | chr1 | 149859252 | 11 | MU | cg21196132 | chr22 | 37915278 | 27 | N |
| cg04148762 | chr1 | 203598573 | 11 | N | cg00207926 | chr22 | 37915326 | 27 | N |
| cg17721249 | chr1 | 203598594 | 11 | MU | cg27409650 | chr22 | 37915342 | 27 | MU |
| cg26253438 | chr1 | 203598623 | 11 | N | cg27166033 | chr22 | 46933090 | 27 | MU |
| cg21397588 | chr1 | 203598954 | 11 | N | cg06957130 | chr22 | 46933231 | 27 | N |
| cg02457623 | chr1 | 203599089 | 11 | MU | cg03503802 | chr22 | 46933237 | 27 | N |
| cg26271110 | chr1 | 206730630 | 11 | MU | cg15219689 | chr22 | 50242492 | 27 | MU |
| cg11037818 | chr1 | 208041879 | 11 | N | cg02691819 | chr3 | 66024561 | 27 | MU |
| cg16549711 | chr1 | 208041887 | 11 | MU | cg15764058 | chr3 | 66024691 | 27 | N |
| cg18224445 | chr1 | 223900693 | 11 | MU | cg17641857 | chr3 | 126243285 | 27 | MU |
| cg07395969 | chr1 | 223900822 | 11 | N | cg09737845 | chr3 | 126243296 | 27 | MU |
| cg02899039 | chr1 | 249152378 | 11 | N | cg18421529 | chr3 | 138067350 | 27 | N |
| cg11817309 | chr1 | 249152443 | 11 | MU | cg17533884 | chr3 | 138067409 | 27 | MU |
| cg00773413 | chr10 | 77168431 | 11 | MU | cg16701456 | chr4 | 13546111 | 27 | N |
| cg25862644 | chr10 | 77168553 | 11 | N | cg06093712 | chr4 | 13546145 | 27 | N |
| cg11700824 | chr10 | 88730823 | 11 | N | cg27438798 | chr4 | 13546151 | 27 | N |
| cg12191938 | chr10 | 88730946 | 11 | MU | cg23584743 | chr4 | 13546157 | 27 | N |
| cg19837790 | chr10 | 97804137 | 11 | MU | cg20393324 | chr4 | 13546169 | 27 | N |
| cg06207432 | chr10 | 97804280 | 11 | N | cg04808029 | chr4 | 13546210 | 27 | MU |
| cg06154084 | chr10 | 102792249 | 11 | MU | cg18894815 | chr4 | 13546292 | 27 | N |
| cg12137206 | chr10 | 113943397 | 11 | MU | cg22777326 | chr4 | 13546316 | 27 | N |
| cg24677674 | chr10 | 113943468 | 11 | N | cg12177702 | chr4 | 37455280 | 27 | N |
| cg11915659 | chr11 | 2161009 | 11 | N | cg21131031 | chr4 | 37455341 | 27 | MU |
| cg17434309 | chr11 | 2161079 | 11 | N | cg05214404 | chr4 | 37455362 | 27 | N |
| cg05859777 | chr11 | 2161102 | 11 | N | cg16080121 | chr4 | 37455366 | 27 | N |
| cg05452899 | chr11 | 2161133 | 11 | N | cg26306909 | chr4 | 37455485 | 27 | N |
| cg10037494 | chr11 | 2161135 | 11 | N | cg14531530 | chr4 | 41362253 | 27 | N |
| cg26517849 | chr11 | 2161141 | 11 | MU | cg25289973 | chr4 | 41362397 | 27 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg13139203 | chr11 | 63581631 | 11 | MU | cg11597475 | chr4 | 57976431 | 27 | N |
| cg10628098 | chr11 | 65292300 | 11 | N | cg21921384 | chr4 | 57976556 | 27 | N |
| cg13974409 | chr11 | 65292307 | 11 | MU | cg01959562 | chr4 | 57976561 | 27 | MU |
| cg07873178 | chr11 | 65307616 | 11 | MU | cg11725975 | chr4 | 72052874 | 27 | MU |
| cg04089426 | chr11 | 65307791 | 11 | MU | cg25894334 | chr4 | 77172836 | 27 | N |
| cg11802864 | chr11 | 65308245 | 11 | MU | cg08073591 | chr4 | 77172841 | 27 | MU |
| cg08676812 | chr11 | 65308423 | 11 | MU | cg17559786 | chr4. | 77172861 | 27 | N |
| cg00206168 | chr11 | 65308501 | 11 | N | cg16435879 | chr4 | 77227529 | 27 | N |
| cg14737600 | chr11 | 66725820 | 11 | N | cg02131049 | chr4 | 77227621 | 27 | MU |
| cg14653250 | chr11 | 66725860 | 11 | N | cg06659580 | chr4 | 77227628 | 27 | MU |
| cg27113009 | chr11 | 66725892 | 11 | N | cg01200289 | chr4 | 77227751 | 27 | N |
| cg09100988 | chr11 | 66725961 | 11 | MU | cg22741823 | chr4 | 108746275 | 27 | MU |
| cg04920951 | chr11 | 67351271 | 11 | MU | cg16217610 | chr4 | 108746323 | 27 | N |
| cg14144513 | chr11 | 69258234 | 11 | N | cg02823293 | chr4 | 108746407 | 27 | N |
| cg23754431 | chr11 | 69258364 | 11 | MU | cg20710394 | chr4 | 170947395 | 27 | MU |
| cg04729730 | chr11 | 69258477 | 11 | N | cg22734085 | chr4 | 184020261 | 27 | MU |
| cg12269002 | chr11 | 77122929 | 11 | N | cg01806295 | chr5 | 72921594 | 27 | N |
| cg18309286 | chr11 | 77123047 | 11 | MU | cg17792315 | chr5 | 72921653 | 27 | MU |
| cg07987843 | chr12 | 3309495 | 11 | MU | cg03628682 | chr5 | 72921796 | 27 | N |
| cg15273066 | chr12 | 49525133 | 11 | MU | cg02075296 | chr5 | 98104747 | 27 | N |
| cg27075104 | chr12 | 122277214 | 11 | N | cg23416667 | chr5 | 98104874 | 27 | MU |
| cg26386472 | chr12 | 122277360 | 11 | MU | cg05071974 | chr5 | 139927110 | 27 | N |
| cg06601685 | chr12 | 122277439 | 11 | N | cg26911562 | chr5 | 139927164 | 27 | N |
| cg03577460 | chr14 | 24836084 | 11 | N | cg26084484 | chr5 | 139927167 | 27 | MU |
| cg05330668 | chr14 | 24836090 | 11 | N | cg07124173 | chr5 | 139927179 | 27 | MU |
| cg25076767 | chr14 | 24836148 | 11 | N | cg15923025 | chr5 | 139927191 | 27 | MU |
| cg15346168 | chr14 | 24836194 | 11 | MU | cg17416146 | chr5 | 139927242 | 27 | N |
| cg26705574 | chr14 | 24836219 | 11 | N | cg10731022 | chr5 | 139927252 | 27 | N |
| cg21107197 | chr14 | 51027861 | 11 | MU | cg07810961 | chr5 | 139927271 | 27 | MU |
| cg16489586 | chr14 | 51027964 | 11 | N | cg01096398 | chr6 | 1312475 | 27 | MU |
| cg04884842 | chr14 | 75643078 | 11 | MU | cg03730958 | chr6 | 1312519 | 27 | N |
| cg07003632 | chr14 | 100259329 | 11 | MU | cg27336026 | chr6 | 1312532 | 27 | N |
| cg14185025 | chr14 | 100259341 | 11 | N | cg05685587 | chr6 | 1312577 | 27 | N |
| cg23411981 | chr14 | 105940377 | 11 | MU | cg04616797 | chr6 | 7542118 | 27 | N |

(continued)

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg03451296 | chr14 | 105940391 | 11 | MU | cg20448717 | chr6 | 7542126 | 27 | MU |
| cg26437123 | chr15 | 74726423 | 11 | MU | cg17200161 | chr6 | 39197233 | 27 | MU |
| cg08125401 | chr15 | 74726457 | 11 | N | cg13583934 | chr6 | 39197256 | 27 | N |
| cg06263834 | chr15 | 74726469 | 11 | N | cg02196294 | chr6 | 39197260 | 27 | N |
| cg26095405 | chr15 | 74726486 | 11 | N | cg09130162 | chr6 | 47277323 | 27 | MU |
| cg13462576 | chr15 | 74726520 | 11 | N | cg08566247 | chr7 | 12726792 | 27 | N |
| cg20927731 | chr15 | 74726561 | 11 | N | cg07229076 | chr7 | 12726813 | 27 | MU |
| cg26585724 | chr15 | 90208739 | 11 | N | cg16544399 | chr7 | 12726941 | 27 | N |
| cg05009389 | chr15 | 90208810 | 11 | MU | cg14169521 | chr7 | 12726944 | 27 | N |
| cg26703661 | chr15 | 90208915 | 11 | N | cg06386517 | chr7 | 50860866 | 27 | N |
| cg02454890 | chr15 | 90544015 | 11 | MU | cg00350885 | chr7 | 50860872 | 27 | MU |
| cg16809962 | chr16 | 21566263 | 11 | MU | cg04148584 | chr7 | 107643946 | 27 | MU |
| cg10155261 | chr16 | 21566385 | 11 | N | cg26087411 | chr7 | 107643948 | 27 | N |
| cg02066638 | chr16 | 87943038 | 11 | MU | cg17657268 | chr7 | 107643956 | 27 | N |
| cg02672397 | chr16 | 87943158 | 11 | MU | cg09284719 | chr7 | 107644024 | 27 | N |
| cg10110271 | chr16 | 87943182 | 11 | N | cg21242584 | chr8 | 25041785 | 27 | N |
| cg03787864 | chr16 | 88717134 | 11 | MU | cg16826874 | chr8 | 25041796 | 27 | MU |
| cg26537639 | chr16 | 88717374 | 11 | N | cg08949296 | chr8 | 75233153 | 27 | MU |
| cg04879832 | chr16 | 88717456 | 11 | MU | cg10594709 | chr8 | 144489858 | 27 | MU |
| cg08843517 | chr16 | 88717464 | 11 | N | cg03939688 | chr9 | 110251706 | 27 | N |
| cg03125427 | chr16 | 88717482 | 11 | MU | cg07309102 | chr9 | 110251826 | 27 | MU |
| cg08177833 | chr16 | 88717537 | 11 | MU | cg09944147 | chr9 | 116918247 | 27 | MU |
| cg00054525 | chr16 | 88717587 | 11 | MU | cg02902865 | chr1 | 12679132 | 28 | MU |
| cg24046411 | chr16 | 88717646 | 11 | N | cg20300343 | chr1 | 149719461 | 28 | N |
| cg05744675 | chr16 | 88717723 | 11 | N | cg00017221 | chr1 | 149719536 | 28 | MU |
| cg12837552 | chr17 | 17687422 | 11 | MU | cg02367655 | chr1 | 178064623 | 28 | MU |
| cg15760257 | chr17 | 26699169 | 11 | MU | cg04273303 | chr1 | 228225533 | 28 | N |
| cg05854644 | chr17 | 26699187 | 11 | N | cg09227621 | chr1 | 228225606 | 28 | MU |
| cg04880618 | chr17 | 74706567 | 11 | MU | cg00078968 | chr1 | 228225659 | 28 | N |
| cg12296532 | chr17 | 79482181 | 11 | MU | cg15881990 | chr10 | 13628544 | 28 | MU |
| cg22076972 | chr19 | 6740812 | 11 | MU | cg14614563 | chr10 | 32218901 | 28 | MU |
| cg13341720 | chr19 | 6740888 | 11 | MU | cg06353720 | chr10 | 91087558 | 28 | MU |
| cg03705926 | chr19 | 6740952 | 11 | MU | cg07310984 | chr10 | 91087591 | 28 | MU |
| cg07046969 | chr19 | 6741049 | 11 | N | cg04364339 | chr10 | 91087703 | 28 | MU |

| Illumina Probe Name | Chr. | Position | Tissue/Cell | MU/N |
|---|---|---|---|---|
| cg13860006 | chr19 | 6741178 | 11 | MU |
| cg06410591 | chr19 | 6741181 | 11 | N |
| cg05896042 | chr19 | 11450090 | 11 | MU |
| cg02349096 | chr19 | 11450198 | 11 | N |
| cg16206460 | chr19 | 15580700 | 11 | N |
| cg07931411 | chr19 | 15580721 | 11 | MU |
| cg07993547 | chr19 | 18210197 | 11 | MU |
| cg07759570 | chr19 | 35491951 | 11 | MU |
| cg03710719 | chr19 | 36389795 | 11 | MU |
| cg27297851 | chr19 | 36389833 | 11 | MU |
| cg11872300 | chr19 | 42785225 | 11 | MU |
| cg04681368 | chr19 | 42806184 | 11 | N |
| cg10632000 | chr19 | 42806201 | 11 | N |
| cg00524108 | chr19 | 42806203 | 11 | N |
| cg22220467 | chr19 | 42806228 | 11 | MU |
| cg09276315 | chr19 | 47614071 | 11 | MU |
| cg22286978 | chr19 | 58858806 | 11 | MU |
| cg10734734 | chr19 | 58859021 | 11 | MU |
| cg03077836 | chr2 | 9347292 | 11 | N |
| cg18303215 | chr2 | 44059003 | 11 | MU |
| cg04221886 | chr2 | 44059094 | 11 | MU |
| cg20385508 | chr2 | 128180829 | 11 | N |
| cg03422204 | chr2 | 128180959 | 11 | N |
| cg09747827 | chr2 | 128181313 | 11 | MU |
| cg16348316 | chr2 | 128181325 | 11 | MU |
| cg01778114 | chr2 | 216877947 | 11 | MU |
| cg12620499 | chr2 | 216877984 | 11 | MU |
| cg05657618 | chr2 | 233792732 | 11 | MU |
| cg27118059 | chr21 | 32931471 | 11 | MU |
| cg11714334 | chr22 | 39638092 | 11 | MU |
| cg03805475 | chr22 | 46972501 | 11 | MU |
| cg01461718 | chr3 | 49711095 | 11 | N |
| cg19800096 | chr3 | 49711245 | 11 | MU |
| cg16503057 | chr3 | 49711251 | 11 | N |

(continued)

| Illumina Probe Name | Chr. | Position | Tissue e/Cell Type | MU/N |
|---|---|---|---|---|
| cg24681845 | chr10 | 126069759 | 28 | MU |
| cg25995133 | chr11 | 407183 | 28 | MU |
| cg14813417 | chr11 | 13205282 | 28 | MU |
| cg24374161 | chr11 | 46582058 | 28 | MU |
| cg12822074 | chr11 | 57243865 | 28 | MU |
| cg06038201 | chr11 | 65487505 | 28 | MU |
| cg24127639 | chr11 | 69064741 | 28 | MU |
| cg08469834 | chr11 | 104839074 | 28 | MU |
| cg05618647 | chr11 | 104839281 | 28 | MU |
| cg16669455 | chr11 | 104839343 | 28 | MU |
| cg16315582 | chr11 | 104839349 | 28 | N |
| cg13980808 | chr12 | 25101448 | 28 | MU |
| cg08244085 | chr12 | 63025730 | 28 | N |
| cg26386409 | chr12 | 63025771 | 28 | N |
| cg01804193 | chr12 | 63026212 | 28 | MU |
| cg16563660 | chr12 | 96792147 | 28 | MU |
| cg20097247 | chr13 | 46425379 | 28 | N |
| cg19352193 | chr13 | 46425508 | 28 | MU |
| cg04199473 | chr14 | 31464361 | 28 | MU |
| cg14876708 | chr14 | 68038121 | 28 | MU |
| cg18580117 | chr15 | 42787236 | 28 | N |
| cg12281446 | chr15 | 42787302 | 28 | MU |
| cg04522310 | chr16 | 27378982 | 28 | MU |
| cg26505691 | chr16 | 27461333 | 28 | N |
| cg10292154 | chr16 | 27461445 | 28 | MU |
| cg10264687 | chr16 | 51147461 | 28 | N |
| cg08006625 | chr16 | 51147491 | 28 | MU |
| cg05583608 | chr16 | 51147536 | 28 | MU |
| cg08082374 | chr16 | 58059152 | 28 | MU |
| cg06367117 | chr17 | 26903928 | 28 | MU |
| cg14668747 | chr17 | 26904053 | 28 | MU |
| cg14501061 | chr17 | 26904058 | 28 | MU |
| cg27183188 | chr17 | 26904121 | 28 | MU |
| cg13990559 | chr17 | 26904150 | 28 | N |

(continued)

| Illumina Probe Name | Chr. | Position | Tissue/Cell | MU/N |
|---|---|---|---|---|
| cg25562664 | chr3 | 119529148 | 11 | N |
| cg24129382 | chr3 | 119529158 | 11 | MU |
| cg24846416 | chr3 | 119529290 | 11 | N |
| cg19155007 | chr3 | 183543564 | 11 | MU |
| cg09738156 | chr3 | 183543587 | 11 | N |
| cg03705396 | chr3 | 183543680 | 11 | N |
| cg10347032 | chr3 | 183543704 | 11 | N |
| cg02734326 | chr4 | 10020555 | 11 | N |
| cg16147221 | chr4 | 10020634 | 11 | MU |
| cg11186122 | chr4 | 174255494 | 11 | MU |
| cg19091677 | chr4 | 174255518 | 11 | MU |
| cg00321931 | chr4 | 174255552 | 11 | N |
| cg05993306 | chr5 | 137090649 | 11 | MU |
| cg19670286 | chr5 | 137610127 | 11 | MU |
| cg02288341 | chr5 | 137610226 | 11 | N |
| cg23511432 | chr5 | 137610255 | 11 | MU |
| cg18442524 | chr5 | 137610372 | 11 | N |
| cg21225170 | chr5 | 137610391 | 11 | N |
| cg09350274 | chr5 | 137610396 | 11 | N |
| cg20424833 | chr5 | 176829639 | 11 | MU |
| cg23407396 | chr6 | 26197481 | 11 | MU |
| cg22404450 | chr6 | 31649291 | 11 | MU |
| cg08759960 | chr6 | 31649335 | 11 | N |
| cg02683578 | chr6 | 31649432 | 11 | N |
| cg23157971 | chr6 | 33396160 | 11 | MU |
| cg15264710 | chr6 | 33396168 | 11 | N |
| cg24852032 | chr6 | 33396223 | 11 | N |
| cg03505346 | chr6 | 33396225 | 11 | N |
| cg00478874 | chr6 | 33396235 | 11 | MU |
| cg11901544 | chr6 | 33396247 | 11 | MU |
| cg18466911 | chr6 | 33396263 | 11 | N |
| cg06681810 | chr6 | 33396295 | 11 | N |
| cg21663580 | chr6 | 43044771 | 11 | MU |
| cg25033767 | chr6 | 43044876 | 11 | N |

| Illumina Probe Name | Chr. | Position | Tissue/Cell Type | MU/N |
|---|---|---|---|---|
| cg08062469 | chr17 | 26926627 | 28 | N |
| cg06803850 | chr17 | 26926738 | 28 | MU |
| cg22465736 | chr17 | 26926841 | 28 | N |
| cg17960080 | chr17 | 26926868 | 28 | N |
| cg10708793 | chr17 | 40264626 | 28 | N |
| cg27249422 | chr17 | 40264678 | 28 | MU |
| cg22453113 | chr17 | 42287971 | 28 | MU |
| cg22749810 | chr17 | 78237389 | 28 | MU |
| cg14175304 | chr17 | 78237397 | 28 | N |
| cg12221087 | chr17 | 78237432 | 28 | N, |
| cg02090762 | chr17 | 79503859 | 28 | MU |
| cg12252135 | chr17 | 79503877 | 28 | MU |
| cg11748640 | chr19 | 5691196 | 28 | MU |
| cg27377863 | chr19 | 16189693 | 28 | MU |
| cg25163134 | chr19 | 40476319 | 28 | MU |
| cg17757000 | chr19 | 40476423 | 28 | N |
| cg24452181 | chr19 | 48984318 | 28 | MU |
| cg05172956 | chr2 | 9283270 | 28 | MU |
| cg01011898 | chr2 | 9283292 | 28 | N |
| cg19482842 | chr2 | 16790314 | 28 | MU |
| cg27115721 | chr2 | 16790338 | 28 | MU |
| cg14640066 | chr2 | 55237421 | 28 | MU |
| cg19254047 | chr2 | 102315097 | 28 | MU |
| cg18295068 | chr2 | 166060400 | 28 | MU |
| cg21382890 | chr2 | 178104794 | 28 | MU |
| cg04741284 | chr20 | 56324226 | 28 | N |
| cg01582438 | chr20 | 62461159 | 28 | MU |
| cg05269938 | chr20 | 62461294 | 28 | MU |
| cg15028052 | chr20 | 62588587 | 28 | MU |
| cg20593868 | chr20 | 62588672 | 28 | MU |
| cg01284306 | chr21 | 19190629 | 28 | N |
| cg13975303 | chr22 | 36850894 | 28 | MU |
| cg21844316 | chr22 | 36851007 | 28 | N |
| cg10455938 | chr22 | 40058150 | 28 | MU |

(continued)

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg10774155 | chr7 | 65338427 | 11 | MU | cg12511214 | chr22 | 40058293 | 28 | N |
| cg27080725 | chr7 | 100075304 | 11 | MU | cg22982767 | chr22 | 46454012 | 28 | MU |
| cg13975648 | chr7 | 100075346 | 11 | N | cg25572043 | chr3 | 15839627 | 28 | MU |
| cg05175020 | chr7 | 100075402 | 11 | N | cg19444609 | chr3 | 44039204 | 28 | MU |
| cg04739306 | chr7 | 100230781 | 11 | MU | cg19890879 | chr3 | 44039289 | 28 | N |
| cg21658235 | chr8 | 22456391 | 11 | MU | cg07289306 | chr3 | 44039357 | 28 | MU |
| cg14690197 | chr8 | 22456421 | 11 | N | cg03889542 | chr3 | 44039578 | 28 | MU |
| cg07216194 | chr8 | 27182965 | 11 | N | cg17614974 | chr3 | 44039919 | 28 | MU |
| cg23693289 | chr8 | 27183097 | 11 | MU | cg12796028 | chr3 | 44040206 | 28 | MU |
| cg03900851 | chr8 | 67351768 | 11 | MU | cg26365925 | chr3 | 44063315 | 28 | N |
| cg11688275 | chr8 | 128750871 | 11 | MU | cg16589135 | chr3 | 44063404 | 28 | MU |
| cg15801573 | chr8 | 128750924 | 11 | N | cg07040834 | chr3 | 44063541 | 28 | MU |
| cg27305704 | chr1 | 8002635 | 12 | MU | cg23141355 | chr3 | 44063593 | 28 | MU |
| cg00907427 | chr1 | 23668691 | 12 | MU | cg17219326 | chr3 | 44517846 | 28 | MU |
| cg08718097 | chr1 | 23668792 | 12 | MU | cg08477641 | chr3 | 48507508 | 28 | N |
| cg26991199 | chr1 | 24307153 | 12 | MU | cg09692335 | chr3 | 48507596 | 28 | N |
| cg04021497 | chr1 | 24307291 | 12 | N | cg26029997 | chr3 | 48507610 | 28 | MU |
| cg05039004 | chr1 | 29586299 | 12 | MU | cg19091784 | chr3 | 48507618 | 28 | MU |
| cg04933208 | chr1 | 29586414 | 12 | MU | cg01511742 | chr3 | 71112437 | 28 | MU |
| cg26884027 | chr1 | 29586418 | 12 | N | cg20521702 | chr3 | 98452812 | 28 | MU |
| cg21929771 | chr1 | 29586580 | 12 | MU | cg13338207 | chr3 | 98452879 | 28 | N |
| cg00128766 | chr1 | 47185216 | 12 | MU | cg10128511 | chr3 | 98452940 | 28 | MU |
| cg20967889 | chr1 | 47185236 | 12 | MU | cg07390647 | chr3 | 98453086 | 28 | MU |
| cg20678442 | chr1 | 244893503 | 12 | MU | cg26072437 | chr4 | 170581380 | 28 | N |
| cg04249706 | chr10 | 11206772 | 12 | MU | cg16842643 | chr4 | 170581385 | 28 | MU |
| cg25761326 | chr10 | 11206792 | 12 | MU | cg01860063 | chr5 | 100237986 | 28 | MU |
| cg11482422 | chr10 | 11206813 | 12 | MU | cg05998153 | chr5 | 134075279 | 28 | MU |
| cg17775621 | chr10 | 11206838 | 12 | MU | cg07307388 | chr6 | 25138416 | 28 | MU |
| cg26150462 | chr10 | 11206868 | 12 | MU | cg00579036 | chr6 | 25138551 | 28 | N |
| cg12178980 | chr10 | 11206870 | 12 | MU | cg12238593 | chr6 | 26440340 | 28 | MU |
| cg20379239 | chr10 | 11207015 | 12 | N | cg00087417 | chr6 | 35887678 | 28 | N |
| cg05966978 | chr10 | 11207437 | 12 | MU | cg12215478 | chr6 | 35887710 | 28 | MU |
| cg23976499 | chr10 | 11207527 | 12 | MU | cg05917732 | chr6 | 152623304 | 28 | N |
| cg11416243 | chr10 | 11207907 | 12 | MU | cg02682457 | chr6 | 152623387 | 28 | MU |

(continued)

| Illumina Probe Name | Chr. | Position | Tissue e/Cell Type | MU/N | Illumina Probe Name | Chr. | Position | Tissue/Cell | MU/N |
|---|---|---|---|---|---|---|---|---|---|
| cg12023625 | chr6 | 152623479 | 28 | N | cg18892808 | chr10 | 11207969 | 12 | MU |
| cg27624424 | chr6 | 160112604 | 28 | MU | cg19235339 | chr10 | 90342899 | 12 | MU |
| cg13221458 | chr6 | 160112632 | 28 | N | cg05627029 | chr10 | 122708532 | 12 | MU |
| cg04358131 | chr7 | 1572252 | 28 | MU | cg06733794 | chr10 | 123922970 | 12 | N |
| cg05279413 | chr7 | 1572327 | 28 | MU | cg24098326 | chr10 | 123923050 | 12 | MU |
| cg26468430 | chr7 | 1572571 | 28 | MU | cg06966660 | chr10 | 123923066 | 12 | N |
| cg10884288 | chr7 | 4922196 | 28 | MU | cg25307318 | chr10 | 123923081 | 12 | N |
| cg12600030 | chr7 | 5635820 | 28 | MU | cg01655356 | chr11 | 70672841 | 12 | N |
| cg19928423 | chr7 | 104658369 | 28 | MU | cg25455593 | chr11 | 70672858 | 12 | N |
| cg15571405 | chr7 | 130796167 | 28 | MU | cg11480627 | chr11 | 70672876 | 12 | N |
| cg12577010 | chr7 | 134832544 | 28 | N | cg09726240 | chr11 | 70672878 | 12 | N |
| cg09414827 | chr7 | 134832671 | 28 | MU | cg26591930 | chr11 | 70672986 | 12 | MU |
| cg13120814 | chr7 | 134832770 | 28 | MU | cg25896644 | chr11 | 116371487 | 12 | MU |
| cg10014112 | chr7 | 134832844 | 28 | N | cg00765312 | chr12 | 85674694 | 12 | MU |
| cg06456031 | chr7 | 134832850 | 28 | MU | cg05916707 | chr14 | 37058256 | 12 | MU |
| cg07972322 | chr7 | 134832939 | 28 | N | cg27428578 | chr15 | 28983731 | 12 | MU |
| cg06201680 | chr7 | 151078646 | 28 | MU | cg14774557 | chr15 | 42500697 | 12 | MU |
| cg12775656 | chr7 | 151078750 | 28 | N | cg03859732 | chr15 | 42500737 | 12 | N |
| cg24260847 | chr8 | 37595307 | 28 | N | cg09856295 | chr15 | 42500843 | 12 | N |
| cg01467789 | chr8 | 37595333 | 28 | N | cg16424945 | chr15 | 52274433 | 12 | MU |
| cg14036776 | chr8 | 37595363 | 28 | MU | cg01064265 | chr16 | 55363058 | 12 | MU |
| cg07538339 | chr8 | 93107298 | 28 | MU | cg01317772 | chr16 | 55405200 | 12 | MU |
| cg19007731 | chr8 | 93107394 | 28 | N | cg10235355 | chr17 | 6358830 | 12 | N |
| cg15450160 | chr8 | 99130171 | 28 | MU | cg11487526 | chr17 | 6358928 | 12 | MU |
| cg26764250 | chr8 | 104090700 | 28 | MU | cg24932788 | chr17 | 70118912 | 12 | N |
| cg05754853 | chr8 | 116660506 | 28 | MU | cg01524174 | chr17 | 70119015 | 12 | MU |
| cg03277455 | chr8 | 116660586 | 28 | N | cg21126344 | chr17 | 70119120 | 12 | MU |
| cg22291627 | chr8 | 116660697 | 28 | MU | cg20060655 | chr19 | 1748322 | 12 | MU |
| cg06023345 | chr8 | 127568650 | 28 | MU | cg11894951 | chr19 | 14168210 | 12 | MU |
| cg02501714 | chr9 | 89560185 | 28 | MU | cg00642306 | chr19 | 14168215 | 12 | MU |
| cg23394391 | chr1 | 8409065 | 29 | MU | cg11282936 | chr19 | 17007648 | 12 | MU |
| cg16552822 | chr1 | 19278734 | 29 | MU | cg24548833 | chr19 | 17007698 | 12 | MU |
| cg04993605 | chr1 | 28573052 | 29 | MU | cg09874289 | chr19 | 17008193 | 12 | MU |
| cg11585805 | chr1 | 47973566 | 29 | N | cg08154437 | chr19 | 17008650 | 12 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg05606136 | chr19 | 18336056 | 12 | N | cg18036569 | chr1 | 47973710 | 29 | MU |
| cg06907708 | chr19 | 18336077 | 12 | MU | cg18533397 | chr1 | 110186005 | 29 | MU |
| cg25383503 | chr19 | 18714552 | 12 | MU | cg00924017 | chr1 | 110186027 | 29 | N |
| cg18111443 | chr19 | 20349275 | 12 | MU | cg22221025 | chr1 | 110186044 | 29 | N |
| cg23679260 | chr19 | 20349303 | 12 | N | cg25720793 | chr1 | 160983767 | 29 | MU |
| cg05606351 | chr19 | 54599285 | 12 | MU | cg18328206 | chr1 | 206681378 | 29 | MU |
| cg12092201 | chr2 | 43451775 | 12 | N | cg17869355 | chr1 | 207038496 | 29 | MU |
| cg16876647 | chr2 | 43451842 | 12 | MU | cg08479073 | chr1 | 207038584 | 29 | N |
| cg08193650 | chr2 | 43451868 | 12 | MU | cg00579859 | chr10 | 60026291 | 29 | MU |
| cg17215170 | chr2 | 111874943 | 12 | MU | cg25531836 | chr10 | 102586999 | 29 | N |
| cg05826458 | chr2 | 111875031 | 12 | N | cg26439963 | chr10 | 102587110 | 29 | MU |
| cg15680720 | chr2 | 171568412 | 12 | MU | cg20095124 | chr10 | 102588691 | 29 | MU |
| cg20389472 | chr2 | 171568526 | 12 | MU | cg10199606 | chr10 | 119494317 | 29 | MU |
| cg04257984 | chr2 | 171568984 | 12 | N | cg01265531 | chr11 | 1854174 | 29 | MU |
| cg02227919 | chr2 | 171569132 | 12 | MU | cg01694451 | chr11 | 1854224 | 29 | N |
| cg05301383 | chr2 | 171569144 | 12 | N | cg08259810 | chr11 | 10325778 | 29 | MU |
| cg22699676 | chr2 | 171569187 | 12 | N | cg05149386 | chr11 | 10325809 | 29 | N |
| cg07363270 | chr2 | 171569282 | 12 | N | cg04024692 | chr11 | 16760046 | 29 | MU |
| cg13768137 | chr2 | 177039426 | 12 | N | cg25623977 | chr11 | 71163428 | 29 | MU |
| cg12602409 | chr2 | 177039568 | 12 | MU | cg05963690 | chr11 | 71163466 | 29 | N |
| cg14324370 | chr2 | 177042789 | 12 | MU | cg01630362 | chr11 | 71163528 | 29 | N |
| cg03152288 | chr2 | 177042942 | 12 | MU | cg20728496 | chr11 | 71163555 | 29 | N |
| cg09355771 | chr2 | 177043147 | 12 | MU | cg12177743 | chr11 | 113185079 | 29 | N |
| cg13092806 | chr2 | 177043255 | 12 | MU | cg06008480 | chr11 | 113185089 | 29 | N |
| cg02561726 | chr2 | 183943938 | 12 | MU | cg27068490 | chr11 | 113185166 | 29 | MU |
| cg13283691 | chr2 | 197852904 | 12 | MU | cg21770330 | chr11 | 124616459 | 29 | MU |
| cg18669346 | chr20 | 32308018 | 12 | MU | cg17842066 | chr12 | 7125201 | 29 | MU |
| cg18353345 | chr21 | 46961669 | 12 | MU | cg16600991 | chr12 | 54389477 | 29 | MU |
| cg22413388 | chr22 | 46367617 | 12 | MU | cg07541701 | chr12 | 54389934 | 29 | N |
| cg13458651 | chr22 | 46367703 | 12 | N | cg14639847 | chr12 | 54389982 | 29 | MU |
| cg23060646 | chr3 | 9904411 | 12 | MU | cg19794507 | chr12 | 54391285 | 29 | MU |
| cg00498591 | chr3 | 9904557 | 12 | MU | cg09941406 | chr12 | 54391325 | 29 | N |
| cg09469667 | chr3 | 9904687 | 12 | N | cg21837192 | chr12 | 54398765 | 29 | N |
| cg26365925 | chr3 | 44063315 | 12 | N | cg20643952 | chr12 | 54398809 | 29 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg16589135 | chr3 | 44063404 | 12 | MU | cg12590051 | chr12 | 54398891 | 29 | N |
| cg07040834 | chr3 | 44063541 | 12 | MU | cg03078363 | chr12 | 54408664 | 29 | MU |
| cg23141355 | chr3 | 44063593 | 12 | MU | cg16937769 | chr12 | 54409485 | 29 | N |
| cg09384411 | chr3 | 44063756 | 12 | MU | cg04704531 | chr12 | 54409491 | 29 | MU |
| cg17053963 | chr3 | 44063832 | 12 | MU | cg15817960 | chr12 | 54409599 | 29 | N |
| cg07794554 | chr3 | 119041383 | 12 | N | cg03892356 | chr12 | 54420837 | 29 | MU |
| cg11099041 | chr3 | 119041385 | 12 | MU | cg03052128 | chr12 | 85671811 | 29 | N |
| cg07074543 | chr3 | 119041529 | 12 | MU | cg07899997 | chr12 | 85671892 | 29 | MU |
| cg02650401 | chr3 | 181441501 | 12 | N | cg01311151 | chr12 | 85671976 | 29 | N |
| cg13294849 | chr3 | 181441571 | 12 | MU | cg16413687 | chr12 | 85672623 | 29 | MU |
| cg25960893 | chr3 | 181441680 | 12 | N | cg18041884 | chr12 | 109162555 | 29 | N |
| cg02016130 | chr4 | 53578359 | 12 | MU | cg10800464 | chr12 | 109162641 | 29 | MU |
| cg10800833 | chr4 | 57521657 | 12 | MU | cg20236513 | chr12 | 109162748 | 29 | N |
| cg12791492 | chr4 | 57522094 | 12 | N | cg27002516 | chr12 | 111835267 | 29 | MU |
| cg09853371 | chr4 | 57522145 | 12 | MU | cg14738806 | chr12 | 115102713 | 29 | N |
| cg21899596 | chr4 | 57522493 | 12 | N | cg26620530 | chr12 | 115102726 | 29 | MU |
| cg24852548 | chr4 | 57522632 | 12 | MU | cg01765077 | chr12 | 122356316 | 29 | MU |
| cg12453905 | chr4 | 154143694 | 12 | MU | cg21171335 | chr12 | 122356390 | 29 | N |
| cg13899188 | chr4 | 154144286 | 12 | MU | cg22618164 | chr12 | 122356400 | 29 | N |
| cg11857997 | chr5 | 3591992 | 12 | MU | cg09677945 | chr13 | 43597657 | 29 | N |
| cg05593349 | chr5 | 10307744 | 12 | MU | cg15988970 | chr13 | 43597736 | 29 | MU |
| cg16261871 | chr5 | 59189788 | 12 | N | cg14789645 | chrl3 | 98794353 | 29 | N |
| cg11258089 | chr5 | 59189791 | 12 | N | cg10805850 | chr13 | 98794470 | 29 | MU |
| cg07190535 | chr5 | 59189843 | 12 | N | cg00221494 | chr13 | 98794593 | 29 | N |
| cg24628676 | chr5 | 59189846 | 12 | N | cg24643012 | chr15 | 93353380 | 29 | MU |
| cg08554295 | chr5 | 59189934 | 12 | MU | cg17147466 | chr15 | 93353422 | 29 | N |
| cg07034329 | chr5 | 66124395 | 12 | MU | cg26920902 | chr15 | 96960987 | 29 | MU |
| cg09728102 | chr5 | 66124492 | 12 | MU | cg03298437 | chrl5 | 96961054 | 29 | N |
| cg17760391 | chr5 | 66124501 | 12 | MU | cg06916706 | chr16 | 4465613 | 29 | MU |
| cg10629165 | chr5 | 66124563 | 12 | MU | cg19168338 | chr16 | 4465731 | 29 | N |
| cg20396510 | chr5 | 66124588 | 12 | MU | cg05173913 | chr17 | 34091901 | 29 | MU |
| cg13695535 | chr5 | 66124603 | 12 | MU | cg04264908 | chr17 | 35303330 | 29 | MU |
| cg03511806 | chr5 | 66124801 | 12 | MU | cg20866711 | chr17 | 47647861 | 29 | MU |
| cg07189332 | chr5 | 87439075 | 12 | MU | cg05651778 | chr17 | 60729731 | 29 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg10830145 | chr5 | 87440121 | 12 | MU | cg00125306 | chr17 | 77811842 | 29 | MU |
| cg09451457 | chr5 | 87440246 | 12 | N | cg02986494 | chr19 | 1248287 | 29 | MU |
| cg12436377 | chr5 | 87440296 | 12 | MU | cg14715947 | chr19 | 1248357 | 29 | N |
| cg07100000 | chr5 | 170877840 | 12 | N | cg11288801 | chr19 | 1406661 | 29 | N |
| cg21601919 | chr5 | 170877932 | 12 | MU | cg07766847 | chr19 | 1406733 | 29 | MU |
| cg02145102 | chr6 | 31276418 | 12 | N | cg06341721 | chr19 | 1406759 | 29 | N |
| cg18845950 | chr6 | 31276434 | 12 | N | cg23291280 | chr19 | 31847970 | 29 | N |
| cg05419812 | chr6 | 31276437 | 12 | MU | cg02128244 | chr19 | 31848008 | 29 | N |
| cg06414921 | chr6 | 31276504 | 12 | N | cg21533331 | chr19 | 31848049 | 29 | MU |
| cg04718845 | chr6 | 152623094 | 12 | MU | cg24436715 | chr19 | 31848130 | 29 | MU |
| cg10851835 | chr6 | 152623220 | 12 | N | cg02624855 | chr19 | 41111297 | 29 | N |
| cg05917732 | chr6 | 152623304 | 12 | MU | cg07808231 | chr19 | 41111347 | 29 | MU |
| cg02682457 | chr6 | 152623387 | 12 | MU | cg19612309 | chr19 | 41112379 | 29 | MU |
| cg12023625 | chr6 | 152623479 | 12 | MU | cg25233685 | chr19 | 41115759 | 29 | MU |
| cg07778029 | chr7 | 27205114 | 12 | N | cg11284196 | chr19 | 51190047 | 29 | N |
| cg20741169 | chr7 | 27205159 | 12 | N | cg09284949 | chr19 | 51190179 | 29 | MU |
| cg05065989 | chr7 | 27205200 | 12 | MU | cg17519645 | chr19 | 52206667 | 29 | N |
| cg16104915 | chr7 | 27205217 | 12 | N | cg16854917 | chr19 | 52206691 | 29 | MU |
| cg18447772 | chr7 | 27205224 | 12 | N | cg24879782 | chr2 | 19561482 | 29 | N |
| cg12600174 | chr7 | 27205230 | 12 | N | cg06530338 | chr2 | 19561612 | 29 | MU |
| cg21001184 | chr7 | 27205262 | 12 | N | cg01196788 | chr2 | 19561709 | 29 | N |
| cg16911981 | chr7 | 70061135 | 12 | MU | cg07669260 | chr2 | 88316847 | 29 | N |
| cg03012726 | chr7 | 113722758 | 12 | MU | cg16842767 | chr2 | 88316858 | 29 | MU |
| cg19590227 | chr1 | 10755322 | 13 | MU | cg04225368 | chr2 | 121834589 | 29 | MU |
| cg04305764 | chr1 | 10755341 | 13 | N | cg11664251 | chr2 | 149634581 | 29 | MU |
| cg12426802 | chrl | 25175293 | 13 | MU | cg07580038 | chr2 | 149634631 | 29 | N |
| cg27537203 | chr1 | 38513245 | 13 | MU | cg06010459 | chr2 | 149634726 | 29 | N |
| cg00385863" | chr1 | 41847974 | 13 | MU | cg15680720 | chr2 | 171568412 | 29 | MU |
| cg00290994 | chr1 | 41848739 | 13 | MU | cg20389472 | chr2 | 171568526 | 29 | N |
| cg08037935 | chr1 | 61547340 | 13 | MU | cg07466807 | chr2 | 173326947 | 29 | MU |
| cg17017152 | chr1 | 61547389 | 13 | N | cg14940165 | chr2 | 177021702 | 29 | N |
| cg23304296 | chr1 | 116961183 | 13 | N | cg26091021 | chr2 | 177021826 | 29 | MU |
| cg07822305 | chr1 | 116961213 | 13 | N | cg10107434 | chr2 | 177021899 | 29 | N |
| cg25743043 | chr1 | 116961233 | 13 | MU | cg27583307 | chr2 | 200320750 | 29 | N |

(continued)

| Illumina Probe Name | Chr. | Position | Tissue/Cell Type | MU/N |
|---|---|---|---|---|
| cg07855083 | chr2 | 200320871 | 29 | MU |
| cg22973789 | chr2 | 200321011 | 29 | MU |
| cg17204275 | chr2 | 200321136 | 29 | N |
| cg22587479 | chr2 | 219738226 | 29 | MU |
| cg00011225 | chr2 | 219738314 | 29 | N |
| cg03387930 | chr20 | 56725833 | 29 | N |
| cg22991320 | chr20 | 56725871 | 29 | N |
| cg19155932 | chr20 | 56725873 | 29 | N |
| cg09663111 | chr20 | 56725876 | 29 | MU |
| cg25906442 | chr20 | 56725887 | 29 | N |
| cg07655948 | chr20 | 56725899 | 29 | N |
| cg18562935 | chr20 | 56726000 | 29 | MU |
| cg27526665 | chr3 | 24537050 | 29 | N |
| cg27622506 | chr3 | 24537160 | 29 | MU |
| cg21303011 | chr3 | 24537177 | 29 | MU |
| cg00323305 | chr3 | 24537182 | 29 | MU |
| cg27388911 | chr3 | 24537277 | 29 | MU |
| cg16579438 | chr3 | 24537301 | 29 | MU |
| cg25336332 | chr3 | 24537309 | 29 | MU |
| cg12258785 | chr3 | 24537407 | 29 | MU |
| cg19092164 | chr3 | 45209187 | 29 | N |
| cg13153394 | chr3 | 45209196 | 29 | MU |
| cg15359249 | chr3 | 45209252 | 29 | MU |
| cg24284497 | chr3 | 138656356 | 29 | MU |
| cg0803115.1 | chr3 | 138656471 | 29 | MU |
| cg01711746 | chr3 | 138657291 | 29 | N |
| cg07287724 | chr3 | 138657429 | 29 | MU |
| cg02019774 | chr3 | 138658470 | 29 | N |
| cg24625136 | chr3 | 138658554 | 29 | N |
| cg21575509 | chr3 | 138658704 | 29 | N |
| cg27326452 | chr3 | 138668931 | 29 | MU |
| cg13147822 | chr3 | 168864313 | 29 | MU |
| cg23146833 | chr3 | 171175866 | 29 | MU |
| cg22877570 | chr3 | 171175950 | 29 | MU |

| Illumina Probe Name | Chr. | Position | Tissue/Cell | MU/N |
|---|---|---|---|---|
| cg16596102 | chr1 | 166134346 | 13 | MU |
| cg19160520 | chr1 | 226927217 | 13 | N |
| cg13209122 | chr1 | 226927327 | 13 | MU |
| cg10001282 | chr10 | 71333478 | 13 | MU |
| cg09154558 | chr10 | 71333556 | 13 | N |
| cg25084101 | chr10 | 71336562 | 13 | MU |
| cg05583020 | chr10 | 71336947 | 13 | MU |
| cg24640390 | chr10 | 71336969 | 13 | N |
| cg11471062 | chr10 | 94459183 | 13 | N |
| cg00149473 | chr10 | 94459330 | 13 | MU |
| cg12865381 | chr10 | 94459447 | 13 | N |
| cg11166108 | chr11 | 18416767 | 13 | MU |
| cg11667086 | chr11 | 31848554 | 13 | N |
| cg12500879 | chr11 | 31848649 | 13 | MU |
| cg04145264 | chr11 | 65190825 | 13 | MU |
| cg09411730 | chr11 | 65190946 | 13 | MU |
| cg07985890 | chr11 | 65190999 | 13 | MU |
| cg18019132 | chr11 | 65191707 | 13 | MU |
| cg14758218 | chr11 | 65193528 | 13 | MU |
| cg11990562 | chr11 | 67806526 | 13 | MU |
| cg24030630 | chr11 | 67806668 | 13 | N |
| cg19154027 | chr11 | 101983027 | 13 | MU |
| cg01053411 | chr12 | 12876595 | 13 | MU |
| cg10696085 | chr12 | 50444522 | 13 | MU |
| cg12391352 | chr12 | 99139768 | 13 | MU |
| cg21958524 | chr12 | 99139867 | 13 | N |
| cg22882539 | chr12 | 131438523 | 13 | MU |
| cg20712263 | chr12 | 131438527 | 13 | N |
| cg23379751 | chr12 | 131438540 | 13 | N |
| cg21502466 | chr12 | 131438626 | 13 | N |
| cg04579966 | chr13 | 24846221 | 13 | MU |
| cg09337897 | chr13 | 28394437 | 13 | MU |
| cg00346326 | chr13 | 28394537 | 13 | N |
| cg20945465 | chr13 | 28395271 | 13 | N |

(continued)

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg04674803 | chr13 | 28395390 | 13 | MU | cg26065488 | chr3 | 171176016 | 29 | N |
| cg08603188 | chr13 | 28395728 | 13 | MU | cg00859352 | chr3 | 171176909 | 29 | MU |
| cg01660473 | chr13 | 28395757 | 13 | MU | cg23533254 | chr3 | 171176940 | 29 | MU |
| cg25546248 | chr13 | 28395783 | 13 | N | cg19403541 | chr4 | 174438312 | 29 | MU |
| cg20402552 | chr13 | 28397359 | 13 | MU | cg11236429 | chr4 | 174459412 | 29 | MU |
| cg04547260 | chr13 | 32605259 | 13 | N | cg18644703 | chr5 | 5887068 | 29 | MU |
| cg25667712 | chr13 | 32605332 | 13 | N | cg23094728 | chr5 | 56110263 | 29 | MU |
| cg22219254 | chr13 | 32605406 | 13 | MU | cg22736807 | chr5 | 92938149 | 29 | N |
| cg21091247 | chr14 | 69258861 | 13 | MU | cg06820621 | chr5 | 92938232 | 29 | MU |
| cg13612175 | chr14 | 69259241 | 13 | MU | cg14477868 | chr5 | 92938260 | 29 | N |
| cg14397166 | chr14 | 69259292 | 13 | N | cg25194822 | chr5 | 113699604 | 29 | MU |
| cg19716433 | chr14 | 103589780 | 13 | MU | cg11712506 | chr5 | 113699683 | 29 | N |
| cg01237565 | chr14 | 105715025 | 13 | MU | cg10837843 | chr5 | 172199244 | 29 | N |
| cg22488745 | chr15 | 53096763 | 13 | N | cg23162310 | chr5 | 172199255 | 29 | N |
| cg18189994 | chr15 | 53096816 | 13 | N | cg12333707 | chr5 | 172199303 | 29 | N |
| cg12738347 | chr15 | 53096854 | 13 | MU | cg25108022 | chr5 | 172199313 | 29 | MU |
| cg02558623 | chr15 | 53097060 | 13 | MU | cg23002268 | chr5 | 172199318 | 29 | MU |
| cg10227187 | chr16 | 2030509 | 13 | MU | cg09799633 | chr5 | 172199460 | 29 | MU |
| cg01982240 | chr16 | 2030576 | 13 | N | cg09493150 | chr5 | 172199605 | 29 | N |
| cg05569877 | chr16 | 22203612 | 13 | MU | cg25412017 | chr5 | 175560449 | 29 | MU |
| cg04138181 | chr16 | 23690918 | 13 | MU | cg09533168 | chr5 | 175560568 | 29 | N |
| cg08794939 | chr16 | 88521230 | 13 | MU | cg06572420 | chr5 | 175621395 | 29 | MU |
| cg15413177 | chr16 | 88521287 | 13 | N | cg02208657 | chr5 | 175621409 | 29 | N |
| cg23643814 | chr17 | 2627585 | 13 | MU | cg04263163 | chr5 | 177371702 | 29 | MU |
| cg12187586 | chr17 | 2627661 | 13 | MU | cg23172545 | chr6 | 2875602 | 29 | N |
| cg23748923 | chr17 | 2628248 | 13 | MU | cg07096305 | chr6 | 2875723 | 29 | MU |
| cg10188897 | chr17 | 42392436 | 13 | MU | cg21570988 | chr6 | 32116841 | 29 | N |
| cg03061122 | chr17 | 42392559 | 13 | MU | cg19192280 | chr6 | 32116893 | 29 | N |
| cg01122302 | chr17 | 42392668 | 13 | MU | cg02925367 | chr6 | 32116905 | 29 | MU |
| cg11088422 | chr17 | 42392697 | 13 | MU | cg17626960 | chr6 | 32116918 | 29 | N |
| cg03446079 | chr17 | 42633739 | 13 | MU | cg11945824 | chr6 | 32116963 | 29 | N |
| cg22097768 | chr17 | 61615913 | 13 | MU | cg04630292 | chr6 | 32116994 | 29 | N |
| cg25019648 | chr17 | 72353261 | 13 | MU | cg24125828 | chr6 | 32117049 | 29 | N |
| cg06874218 | chr18 | 3447454 | 13 | N | cg13534901 | chr7 | 17339481 | 29 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg21346639 | chr18 | 3447463 | 13 | MU | cg09864050 | chr7 | 19150174 | 29 | MU |
| cg05265512 | chr18 | 3447583 | 13 | N | cg24496841 | chr7 | 19152017 | 29 | MU |
| cg05737526 | chr18 | 3454175 | 13 | MU | cg11973479 | chr7 | 19152073 | 29 | N |
| cg05001478 | chr19 | 1668832 | 13 | MU | cg27621129 | chr7 | 19152121 | 29 | N |
| cg00438215 | chr19 | 7937107 | 13 | MU | cg25228422 | chr7 | 19152335 | 29 | MU |
| cg08305551 | chr19 | 12978611 | 13 | MU | cg26279021 | chr7 | 19158349 | 29 | MU |
| cg02776664 | chr19 | 12978624 | 13 | MU | cg27334919 | chr7 | 19158378 | 29 | N |
| cg06537894 | chr19 | 12978706 | 13 | N | cg16168668 | chr7 | 19158566 | 29 | MU |
| cg05068987 | chr19 | 12978714 | 13 | N | cg14391419 | chr7 | 19158647 | 29 | N |
| cg00523012 | chr19 | 13264324 | 13 | MU | cg10126205 | chr7 | 19158664 | 29 | N |
| cg19027852 | chr19 | 17717301 | 13 | MU | cg21424940 | chr7 | 19158666 | 29 | N |
| cg06303772 | chr19 | 17717530 | 13 | MU | cg04904385 | chr7 | 19158692 | 29 | N |
| cg25237016 | chr19 | 41018757 | 13 | N | cg10624122 | chr7 | 19158747 | 29 | MU |
| cg05764628 | chr19 | 41018759 | 13 | N | cg01665174 | chr8 | 119756156 | 29 | MU |
| cg17768491 | chr19 | 41018876 | 13 | MU | cg13962186 | chr9 | 79633650 | 29 | N |
| cg12248614 | chr19 | 41018880 | 13 | MU | cg14487131 | chr9 | 79633737 | 29 | MU |
| cg19258034 | chr19 | 41055125 | 13 | N | cg06974755 | chr9 | 135996931 | 29 | MU |
| cg08118159 | chr19 | 41055208 | 13 | MU | cg22157239 | chr1 | 18972096 | 30 | MU |
| cg08484671 | chr19 | 41055297 | 13 | N | cg17949727 | chr1 | 38412684 | 30 | MU |
| cg02247591 | chr19 | 43979372 | 13 | N | cg10784030 | chr1 | 38412688 | 30 | N |
| cg11452571 | chr19 | 43979464 | 13 | MU | cg05749969 | chr1 | 38412711 | 30 | N |
| cg08651867 | chr19 | 43979500 | 13 | MU | cg02309273 | chr1 | 38412744 | 30 | N |
| cg22975160 | chr19 | 43979614 | 13 | MU | cg10512745 | chr1 | 50884480 | 30 | MU |
| cg18851960 | chr19 | 43979739 | 13 | N | cg09792881 | chr1 | 50884544 | 30 | MU |
| cg02899346 | chr19 | 45996372 | 13 | MU | cg16732616 | chr1 | 50886782 | 30 | MU |
| cg19031575 | chr19 | 45996439 | 13 | N | cg04455430 | chr1 | 50886920 | 30 | N |
| cg01005968 | chr19 | 45996460 | 13 | N | cg25191628 | chr1 | 50886949 | 30 | MU |
| cg19869610 | chr19 | 45996498 | 13 | N | cg12756396 | chr1 | 50886969 | 30 | N |
| cg16082401 | chr19 | 46181221 | 13 | N | cg10375890 | chr1 | 50892511 | 30 | MU |
| cg20434926 | chr19 | 46181274 | 13 | MU | cg19810598 | chr1 | 50892674 | 30 | MU |
| cg18735402 | chr19 | 46181330 | 13 | N | cg04023996 | chr1 | 50892794 | 30 | N |
| cg23928165 | chr19 | 46996713 | 13 | MU | cg01486262 | chr1 | 50893067 | 30 | N |
| cg07789559 | chr19 | 47730030 | 13 | MU | cg13368519 | chr1 | 50893149 | 30 | MU |
| cg01370122 | chr2 | 1858674 | 13 | N | cg22627293 | chr1 | 50893226 | 30 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg05974816 | chr2 | 1858740 | 13 | MU | cg03441844 | chr1 | 161368947 | 30 | N |
| cg20495404 | chr2 | 8823649 | 13 | MU | cg19247228 | chr1 | 161369009 | 30 | MU |
| cg04525491 | chr2 | 8823683 | 13 | MU | cg20976159 | chr1 | 228193529 | 30 | MU |
| cg01184784 | chr2 | 8823756 | 13 | MU | cg09369927 | chr1 | 228193585 | 30 | N |
| cg08738430 | chr2 | 43452561 | 13 | MU | cg26921638 | chr1 | 228193587 | 30 | N |
| cg22437153 | chr2 | 73110657 | 13 | MU | cg17318529 | chr1 | 228196198 | 30 | N |
| cg11446240 | chr2 | 136633306 | 13 | MU | cg26332740 | chr1 | 228196227 | 30 | MU |
| cg23837867 | chr2 | 219849133 | 13 | N | cg10019916 | chr1 | 228196376 | 30 | N |
| cg15138883 | chr2 | 219849135 | 13 | MU | cg09850632 | chr1 | 229406661 | 30 | N |
| cg01157126 | chr2 | 219849189 | 13 | N | cg03301331 | chr1 | 229406681 | 30 | N |
| cg14262681 | chr20 | 21084420 | 13 | MU | cg16909962 | chr1 | 229406711 | 30 | MU |
| cg09923107 | chr20 | 30193892 | 13 | MU | cg04669815 | chr10 | 101296619 | 30 | MU |
| cg00494337 | chr20 | 30194024 | 13 | N | cg24812837 | chr10 | 102894120 | 30 | MU |
| cg25660386 | chr3 | 13009126 | 13 | N | cg04273871 | chr10 | 102894227 | 30 | N |
| cg05278650 | chr3 | 13009132 | 13 | MU | cg14383658 | chr10 | 102899262 | 30 | N |
| cg05956462 | chr3 | 127175400 | 13 | MU | cg05482942 | chr10 | 102899285 | 30 | MU |
| cg00473416 | chr3 | 127176006 | 13 | MU | cg13332774 | chr10 | 106028628 | 30 | N |
| cg10678427 | chr3 | 127176163 | 13 | MU | cg06776915 | chr10 | 106028703 | 30 | MU |
| cg24690054 | chr3 | 127176257 | 13 | N | cg05627029 | chr10 | 122708532 | 30 | MU |
| cg17323383 | chr3 | 182896670 | 13 | MU | cg03933279 | chr12 | 12849136 | 30 | N |
| cg20724810 | chr3 | 182897196 | 13 | N | cg13697193 | chr12 | 12849138 | 30 | N |
| cg00794178 | chr3 | 182897283 | 13 | MU | cg13417420 | chr12 | 12849159 | 30 | MU |
| cg12024828 | chr4 | 1166528 | 13 | MU | cg20631472 | chr12 | 12849269 | 30 | N |
| cg14652131 | chr4 | 1166531 | 13 | N | cg03781931 | chr12 | 85673200 | 30 | N |
| cg24177604 | chr4 | 80994480 | 13 | N | cg14996220 | chr12 | 85673270 | 30 | MU |
| cg18937330 | chr4 | 80994550 | 13 | MU | cg25800765 | chr12 | 85673347 | 30 | N |
| cg08212230 | chr5 | 54455564 | 13 | MU | cg19937061 | chr13 | 25320133 | 30 | MU |
| cg08508814 | chr5 | 54455701 | 13 | N | cg20381975 | chr13 | 25320750 | 30 | N |
| cg05708550 | chr5 | 137688227 | 13 | N | cg04759767 | chr13 | 25320824 | 30 | MU |
| cg24137987 | chr5 | 137688255 | 13 | MU | cg00629427 | chr13 | 95357638 | 30 | N |
| cg19597031 | chr5 | 162931471 | 13 | MU | cg13128937 | chr13 | 95357743 | 30 | MU |
| cg19105122 | chr7 | 75896889 | 13 | MU | cg06183559 | chr13 | 95357818 | 30 | N |
| cg16061381 | chr7 | 112430531 | 13 | MU | cg02689863 | chr13 | 95357832 | 30 | N |
| cg13874046 | chr7 | 112430565 | 13 | N | cg14956197 | chr14 | 36993831. | 30 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg10822582 | chr7 | 112430590 | 13 | N | cg00928167 | chr14 | 36994016 | 30 | N |
| cg01828650 | chr7 | 112430614 | 13 | N | cg01509464 | chr14 | 36994153 | 30 | MU |
| cg26386449 | chr7 | 112430654 | 13 | N | cg26157386 | chr14 | 36994270 | 30 | MU |
| cg17200920 | chr7 | 127808798 | 13 | MU | cg11089489 | chr14 | 37124313 | 30 | N |
| cg02383785 | chr7 | 127808848 | 13 | N | cg01843946 | chr14 | 37124434 | 30 | MU |
| cg09595202 | chr7 | 156812104 | 13 | MU | cg15425541 | chr14 | 61118751 | 30 | MU |
| cg21182960 | chr7 | 156812171 | 13 | N | cg06620896 | chr15 | 37395115 | 30 | MU |
| cg20979233 | chr8 | 54790841 | 13 | MU | cg01064265 | chr16 | 55363058 | 30 | MU |
| cg00634665 | chr8 | 54790957 | 13 | N | cg18482164 | chr17 | 37764118 | 30 | MU |
| cg12396325 | chr8 | 105235606 | 13 | N | cg03679521 | chr17 | 37764153 | 30 | N |
| cg01779771 | chr8 | 105235715 | 13 | MU | cg11634198 | chr17 | 37764158 | 30 | N |
| cg01949455 | chr9 | 131937559 | 13 | N | cg09393675 | chr17 | 37764250 | 30 | MU |
| cg13775299 | chr9 | 131937583 | 13 | MU | cg01293647 | chr17 | 37764264 | 30 | N |
| cg03114585 | chr1 | 22351389 | 14 | MU | cg07477034 | chr17 | 53341969 | 30 | N |
| cg21789280 | chr1 | 22351425 | 14 | N | cg18943957 | chr17 | 53342029 | 30 | MU |
| cg01573472 | chr1 | 22351524 | 14 | N | cg25470077 | chr19 | 10406364 | 30 | MU |
| cg04290779 | chr1 | 41962067 | 14 | MU | cg24213437 | chr19 | 11529741 | 30 | N |
| cg25110126 | chr1 | 46999211 | 14 | MU | cg02433671 | chr19 | 11529856 | 30 | MU |
| cg04275806 | chr1 | 46999248 | 14 | N | cg08198862 | chr19 | 11529947 | 30 | N |
| cg02958895 | chr1 | 46999348 | 14 | N | cg08068820 | chr19 | 11530021 | 30 | MU |
| cg23045255 | chr1 | 182584068 | 14 | MU | cg08000684 | chr19 | 11530025 | 30 | MU |
| cg24052698 | chr1 | 182584193 | 14 | N | cg14913644 | chr19 | 11530065 | 30 | MU |
| cg00153856 | chr1 | 182584341 | 14 | MU | cg02478247 | chr19 | 23941207 | 30 | MU |
| cg14248553 | chr1 | 201509308 | 14 | N | cg09152619 | chr19 | 23941313 | 30 | N |
| cg04223671 | chr1 | 201509316 | 14 | MU | cg08470875 | chr2 | 26401718 | 30 | MU |
| cg04121938 | chr1 | 201509415 | 14 | N | cg04944784 | chr2 | 26401820 | 30 | N |
| cg00675569 | chr10 | 45914840 | 14 | MU | cg26314531 | chr2 | 26401878 | 30 | MU |
| cg09806262 | chr10 | 45914949 | 14 | N | cg27170947 | chr2 | 26402098 | 30 | MU |
| cg02119927 | chr10 | 71078141 | 14 | MU | cg18940723 | chr2 | 45837923 | 30 | MU |
| cg12799885 | chr10 | 74020976 | 14 | MU | cg27364741 | chr2 | 63281069 | 30 | N |
| cg25104124 | chr10 | 74021022 | 14 | N | cg25622366 | chr2 | 63281139 | 30 | MU |
| cg04861460 | chr10 | 102499989 | 14 | MU | cg07938743 | chr2 | 63283939 | 30 | N |
| cg19665644 | chr11 | 13030474 | 14 | MU | cg21472506 | chr2 | 63283967 | 30 | MU |
| cg20918243 | chr11 | 13030512 | 14 | N | cg23229261 | chr2 | 63284066 | 30 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg09596429 | chr11 | 13030517 | 14 | N | cg05092308 | chr2 | 63285365 | 30 | N |
| cg05817758 | chr11 | 13030567 | 14 | N | cg19351026 | chr2 | 63285425 | 30 | MU |
| cg08876434 | chr11 | 13030637 | 14 | N | cg13768290 | chr2 | 63285491 | 30 | N |
| cg25344734 | chr11 | 13030643 | 14 | N | cg27315333 | chr2 | 63285950 | 30 | N |
| cg26005578 | chr11 | 13030676 | 14 | MU | cg11536474 | chr2 | 63286049 | 30 | MU |
| cg05415131 | chr11 | 58940866 | 14 | MU | cg01579289 | chr2 | 71503740 | 30 | MU |
| cg14329059 | chr11 | 58940964 | 14 | N | cg13686615 | chr2 | 71503742 | 30 | N |
| cg19182353 | chr11 | 118479428 | 14 | MU | cg23045594 | chr2 | 71503883 | 30 | N |
| cg11633126 | chr11 | 123947157 | 14 | MU | cg27553926 | chr2 | 97136508 | 30 | N |
| cg10424454 | chr11 | 123947190 | 14 | N | cg04706655 | chr2 | 97136556 | 30 | MU |
| cg13945107 | chr11 | 123947230 | 14 | MU | cg06862374 | chr2 | 219736549 | 30 | MU |
| cg06807.966 | chr12 | 4274034 | 14 | MU | cg22587479 | chr2 | 219738226 | 30 | MU |
| cg08873912 | chr12 | 4274070 | 14 | N | cg00011225 | chr2 | 219738314 | 30 | MU |
| cg03701001 | chr12 | 51818636 | 14 | MU | cg02178763 | chr20 | 21681966 | 30 | MU |
| cg13127386 | chr12 | 51818768 | 14 | N | cg18130044 | chr20 | 21682362 | 30 | MU |
| cg12060306 | chr12 | 52702064 | 14 | MU | cg08736638 | chr20 | 21682505 | 30 | N |
| cg11832404 | chr12 | 65218258 | 14 | N | cg15556943 | chr20 | 21694617 | 30 | MU |
| cg23684878 | chr12 | 65218260 | 14 | MU | cg07213060 | chr20 | 21694827 | 30 | MU |
| cg14019323 | chr12 | 65218413 | 14 | MU | cg07211212 | chr20 | 55200146 | 30 | MU |
| cg00494665 | chr12 | 65218728 | 14 | N | cg27307781 | chr21 | 37442289 | 30 | MU |
| cg23013309 | chr12 | 65218841 | 14 | N | cg08886651 | chr22 | 19743029 | 30 | MU |
| cg08815398 | chr12 | 65218869 | 14 | MU | cg08798701 | chr3 | 129118382 | 30 | MU |
| cg15992563 | chr13 | 28491540 | 14 | N | cg20691722 | chr3 | 181421427 | 30 | MU |
| cg10034364 | chr13 | 28491590 | 14 | MU | cg02213103 | chr3 | 181421548 | 30 | N |
| cg01193690 | chr13 | 28491615 | 14 | N | cg02509058 | chr5 | 2753746 | 30 | N |
| cg04547260 | chr13 | 32605259 | 14 | N | cg12860686 | chr5 | 2753876 | 30 | MU |
| cg25667712 | chr13 | 32605332 | 14 | N | cg10958362 | chr5 | 2754016 | 30 | N |
| cg22219254 | chr13 | 32605406 | 14 | MU | cg18652721 | chr5 | 2754074 | 30 | MU |
| cg20754145 | chr13 | 32605902 | 14 | MU | cg04509074 | chr5 | 2754274 | 30 | MU |
| cg25481474 | chr13 | 32605952 | 14 | MU | cg27144115 | chr5 | 2754322 | 30 | N |
| cg16739845 | chr14 | 92040772 | 14 | MU | cg21629500 | chr5 | 2754753 | 30 | N |
| cg13166888 | chr14 | 92040875 | 14 | N | cg23164183 | chr5 | 2754894 | 30 | MU |
| cg24165760 | chr14 | 103243389 | 14 | MU | cg21999939 | chr5 | 2755004 | 30 | N |
| cg01237565 | chr14 | 105715025 | 14 | MU | cg01294808 | chr5 | 3599686 | 30 | N |

(continued)

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg18912855 | chr14 | 105830859 | 14 | MU | cg18635552 | chr5 | 3599719 | 30 | MU |
| cg02430385 | chr15 | 45421951 | 14 | N | cg14223293 | chr5 | 3602272 | 30 | N |
| cg19295951 | chr15 | 45421998 | 14 | N | cg06585708 | chr5 | 3602413 | 30 | MU |
| cg08328777 | chr15 | 45422004 | 14 | N | cg04635983 | chr5 | 3602558 | 30 | N |
| cg24505618 | chr15 | 45422054 | 14 | N | cg08466792 | chr5 | 3603227 | 30 | MU |
| cg05804220 | chr15 | 45422062 | 14 | N | cg19082560 | chr5 | 3603280 | 30 | N |
| cg10496082 | chr15 | 45422083 | 14 | MU | cg20406460 | chr5 | 3606489 | 30 | N |
| cg10583297 | chr15 | 45422095 | 14 | N | cg13729210 | chr5 | 3606561 | 30 | MU |
| cg24500441 | chr15 | 66274709 | 14 | MU | cg22156700 | chr5 | 72732486 | 30 | MU |
| cg00557308 | chr15 | 66274821 | 14 | N | cg02251859 | chr5 | 89854293 | 30 | MU |
| cg06100807 | chr15 | 90030016 | 14 | MU | cg11867257 | chr5 | 89854334 | 30 | N |
| cg08641118 | chr15 | 90030046 | 14 | N | cg02792655 | chr5 | 89854364 | 30 | N |
| cg22526139 | chr15 | 90030153 | 14 | N | cg14798760 | chr5 | 89854414 | 30 | N |
| cg01021488 | chr15 | 99395581 | 14 | N | cg08618802 | chr5 | 89854425 | 30 | N |
| cg01224063 | chr15 | 99395633 | 14 | MU | cg13441766 | chr5 | 134376442 | 30 | MU |
| cg16427096 | chr16 | 1202468 | 14 | MU | cg14994060 | chr5 | 134376489 | 30 | N |
| cg06277900 | chr16 | 67198029 | 14 | MU | cg09602288 | chr6 | 10382800 | 30 | N |
| cg11996632 | chr16 | 70284893 | 14 | N | cg20168806 | chr6 | 10382828 | 30 | MU |
| cg02727363 | chr16 | 70284928 | 14 | MU | cg00110654 | chr6 | 10385489 | 30 | MU |
| cg09594067 | chr16 | 70285806 | 14 | MU | cg24336989 | chr6 | 10385903 | 30 | MU |
| cg07006158 | chr16 | 70285910 | 14 | N | cg11811610 | chr6 | 10390811 | 30 | N |
| cg14357669 | chr16 | 70285955 | 14 | N | cg24452128 | chr6 | 10390919 | 30 | MU |
| cg02602699 | chr17 | 235235 | 14 | MU | cg19607165 | chr6 | 10390961 | 30 | |
| cg26053771 | chr17 | 8192194 | 14 | N | cg17754510 | chr6 | 10391412 | 30 | MU |
| cg15754548 | chr17 | 8192281 | 14 | MU | cg05639246 | chr6 | 10393778 | 30 | MU |
| cg20448053 | chr17 | 8192352 | 14 | N | cg17557766 | chr6 | 10420696 | 30 | |
| cg05151395 | chr17 | 8192371 | 14 | N | cg00689580 | chr6 | 10420798 | 30 | MU |
| cg20289346 | chr17 | 37310500 | 14 | MU | cg10129408 | chr6 | 10421001 | 30 | MU |
| cg09920557 | chr17 | 61553938 | 14 | MU | cg13486532 | chr6 | 10421460 | 30 | MU |
| cg25054907 | chr17 | 61553954 | 14 | N | cg10293403 | chr6 | 10421670 | 30 | MU |
| cg02131967 | chr17 | 61554106 | 14 | MU | cg22267770 | chr6 | 10422139 | 30 | MU |
| cg24932788 | chr17 | 70118912 | 14 | N | cg06014958 | chr6 | 10425553 | 30 | N |
| cg01524174 | chr17 | 70119015 | 14 | MU | cg03074925 | chr6 | 10425625 | 30 | N |
| cg21126344 | chr17 | 70119120 | 14 | N | cg22367191 | chr6 | 10425648 | 30 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg19517525 | chr17 | 79480921 | 14 | N | cg08178940 | chr6 | 50808627 | 30 | N |
| cg01881939 | chr17 | 79480981 | 14 | MU | cg09363735 | chr6 | 50808699 | 30 | MU |
| cg05488523 | chr17 | 79481108 | 14 | N | cg03159396 | chr6 | 50808813 | 30 | MU |
| cg15045802 | chr19 | 1857434 | 14 | MU | cg25228422 | chr7 | 19152335 | 30 | MU |
| cg02161471 | chr19 | 1857442 | 14 | N | cg07812819 | chr7 | 89975908 | 30 | MU |
| cg26026416 | chr19 | 3434930 | 14 | N | cg05306225 | chr8 | 4848947 | 30 | MU |
| cg20707679 | chr19 | 3434945 | 14 | MU | cg26205771 | chr8 | 53851156 | 30 | MU |
| cg10811474 | chr19 | 8428787 | 14 | N | cg11878331 | chr8 | 72469507 | 30 | MU |
| cg17793059 | chr19 | 8428850 | 14 | MU | cg18855674 | chr8 | 72469553 | 30 | N |
| cg16745616 | chr19 | 8428856 | 14 | N | cg03186333 | chr8 | 72470352 | 30 | MU |
| cg11758861 | chr19 | 13135514 | 14 | MU | cg15795787 | chr8 | 72470415 | 30 | N |
| cg17976191 | chr19 | 13135527 | 14 | N | cg23954787 | chr8 | 72470700 | 30 | N |
| cg24277817 | chr19 | 13135662 | 14 | N | cg22863920 | chr8 | 72470832 | 30 | MU |
| cg27212234 | chr19 | 36048757 | 14 | MU | cg20386316 | chr8 | 72470982 | 30 | MU |
| cg11945235 | chr19 | 52552025 | 14 | N | cg26818625 | chr8 | 72471153 | 30 | MU |
| cg24021890 | chr19 | 52552120 | 14 | MU | cg02510299 | chr8 | 121457568 | 30 | MU |
| cg14944575 | chr19 | 52552197 | 14 | MU | cg05799038 | chr8 | 144503807 | 30 | MU |
| cg07629094 | chr19 | 52552213 | 14 | N | cg02649849 | chr9 | 969473 | 30 | MU |
| cg13584718 | chr19 | 52552383 | 14 | MU | cg19291576 | chr9 | 969530 | 30 | N |
| cg02729352 | chr19 | 52552443 | 14 | N | cg01150641 | chr1 | 976168 | 31 | N |
| cg11919S25 | chr19 | 52552461 | 14 | N | cg23625715 | chr1 | 976172 | 31 | MU |
| cg06665109 | chr19 | 54369436 | 14 | N | cg26222311 | chr1 | 976227 | 31 | N |
| cg09418984 | chr19 | 54369447 | 14 | N | cg22337605 | chr1 | 12227417 | 31 | MU |
| cg06472476 | chr19 | 54369471 | 14 | N | cg15090202 | chr1 | 26687087 | 31 | MU |
| cg13499300 | chr19 | 54369556 | 14 | N | cg04661629 | chr1 | 26687174 | 31 | N |
| cg05337743 | chr19 | 54369571 | 14 | MU | cg15696662 | chr1 | 46669221 | 31 | MU |
| cg23305567 | chr19 | 54369576 | 14 | N | cg24542714 | chr1 | 46669345 | 31 | N |
| cg03585419 | chr19 | 54369681 | 14 | MU | cg17952661 | chr1 | 145395753 | 31 | MU |
| cg16253840 | chr19 | 58361533 | 14 | MU | cg02395363 | chr1 | 145395846 | 31 | N |
| cg04092762 | chr2 | 30454146 | 14 | MU | cg11278262 | chr1 | 155146720 | 31 | MU |
| cg00852846 | chr2 | 85664713 | 14 | N | cg25651783 | chr1 | 155265033 | 31 | N |
| cg06358970 | chr2 | 85664826 | 14 | MU | cg01971813 | chr1 | 155265180 | 31 | MU |
| cg15256164 | chr2 | 174220625 | 14 | MU | cg10513702 | chr1 | 200003800 | 31 | MU |
| cg20364050 | chr20 | 825240 | 14 | N | cg15062377 | chr1 | 200004205 | 31 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg20353496 | chr20 | 825268 | 14 | MU | cg16046444 | chr1 | 200004265 | 31 | MU |
| cg19424265 | chr20 | 825408 | 14 | N | cg06870728 | chr10 | 8095363 | 31 | N |
| cg14855292 | chr20 | 825415 | 14 | N | cg13543854 | chrl0 | 8095477 | 31 | N |
| cg09923107 | chr20 | 30193892 | 14 | MU | cg06022942 | chr10 | 8095484 | 31 | N |
| cg00494337 | chr20 | 30194024 | 14 | N | cg11018337 | chr10 | 8095495 | 31 | N |
| cg04378107 | chr22 | 22006019 | 14 | MU | cg13814485 | chr10 | 8095500 | 31 | MU |
| cg09745440 | chr22 | 31481318 | 14 | MU | cg04895321 | chr10 | 15210264 | 31 | MU |
| cg03989125 | chr22 | 38214979 | 14 | MU | cg19378330 | chr10 | 101605988 | 31 | MU |
| cg20893579 | chr22 | 38215064 | 14 | N | cg12673726 | chr10 | 101606026 | 31 | MU |
| cg25887069 | chr22 | 38221009 | 14 | N | cg13332774 | chr10 | 106028628 | 31 | N |
| cg01961447 | chr22 | 38221095 | 14 | MU | cg06776915 | chr10 | 106028703 | 31 | MU |
| cg07879739 | chr22 | 38221187 | 14 | N | cg02105002 | chr10 | 106028928 | 31 | MU |
| cg05833158 | chr3 | 9993731 | 14 | N | cg17167468 | chr10 | 113943149 | 31 | MU |
| cg21556309 | chr3 | 9993818 | 14 | MU | cg12137206 | chr10 | 113943397 | 31 | MU |
| cg06181784 | chr3 | 9993934 | 14 | MU | cg24677674 | chr10 | 113943468 | 31 | N |
| cg06663068 | chr3 | 9994061 | 14 | MU | cg24965984 | chr10 | 113943723 | 31 | MU |
| cg21746851 | chr3 | 9994105 | 14 | MU | cg05653400 | chr10 | 113943887 | 31 | MU |
| cg08941824 | chr3 | 9994229 | 14 | N | cg08564661 | chr10 | 124133785 | 31 | N |
| cg12691534 | chr3 | 50275394 | 14 | N | cg03205140 | chr10 | 124133802 | 31 | N |
| cg06508783 | chr3 | 50275446 | 14 | MU | cg10576280 | chr10 | 124133822 | 31 | MU |
| cg12996903 | chr3 | 50275575 | 14 | N | cg09909671 | chr11 | 13689641 | 31 | MU |
| cg20881888 | chr3 | 50383079 | 14 | N | cg23308107 | chr11 | 47429939 | 31 | MU |
| cg11270070 | chr3 | 50383118 | 14 | N | cg19323951 | chr11 | 64993076 | 31 | MU |
| cg23208152 | chr3 | 50383134 | 14 | MU | cg07873178 | chr11 | 65307616 | 31 | MU |
| cg01031032 | chr3 | 112051894 | 14 | N | cg04089426 | chr11 | 65307791 | 31 | MU |
| cg09006015 | chr3 | 112051908 | 14 | N | cg11802864 | chr11 | 65308245 | 31 | MU |
| cg04367486 | chr3 | 112051991 | 14 | MU | cg03522668 | chr11 | 68780866 | 31 | N |
| cg01060026 | chr3 | 129324501 | 14 | MU | cg09102864 | chr11 | 68780892 | 31 | N |
| cg12024828 | chr4 | 1166528 | 14 | MU | cg04658945 | chr11 | 68780897 | 31 | MU |
| cg14652131 | chr4 | 1166531 | 14 | N | cg12641569 | chr11 | 70601781 | 31 | MU |
| cg25214789 | chr5 | 7850070 | 14 | N | cg21007414 | chr11 | 76381687 | 31 | MU |
| cg12539796 | chr5 | 7850203 | 14 | MU | cg05342945 | chr12 | 48394962 | 31 | MU |
| cg06554120 | chr5 | 11385067 | 14 | MU | cg08995424 | chr12 | 54070891 | 31 | N |
| cg23525243 | chr5 | 11385735 | 14 | MU | cg06632207 | chr12 | 54070931 | 31 | MU |

(continued)

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg19438860 | chr5 | 11385821 | 14 | N | cg13691247 | chr12 | 54071090 | 31 | MU |
| cg24120357 | chr5 | 118691415 | 14 | N | cg23278196 | chr12 | 54071111 | 31 | MU |
| cg16645133 | chr5 | 118691425 | 14 | MU | cg09706122 | chr12 | 54071165 | 31 | N |
| cg08076617 | chr5 | 176739172 | 14 | N | cg04418091 | chr12 | 54071168 | 31 | N |
| cg09044296 | chr5 | 176739296 | 14 | N | cg16264705 | chr12 | 54071194 | 31 | N |
| cg16616449 | chr5 | 176739309 | 14 | MU | cg27075104 | chr12 | 122277214 | 31 | N |
| cg10533277 | chr5 | 176739312 | 14 | MU | cg26386472 | chr12 | 122277360 | 31 | MU |
| cg17466946 | chr5 | 176739371 | 14 | MU | cg06601685 | chr12 | 122277439 | 31 | N |
| cg26087598 | chr5 | 176739400 | 14 | N | cg22268137 | chr13 | 28497117 | 31 | MU |
| cg01248080 | chr6 | 711212 | 14 | MU | cg25299895 | chr13 | 28498091 | 31 | N |
| cg21026663 | chr6 | 26189234 | 14 | MU | cg21900495 | chr13 | 28498161. | 31 | MU |
| cg19156220 | chr6 | 33244752 | 14 | N | cg16302790 | chr13 | 28498334 | 31 | MU |
| cg17225129 | chr6 | 33244755 | 14 | MU | cg09975620 | chr13 | 28498384 | 31 | MU |
| cg21387418 | chr6 | 33244775 | 14 | MU | cg24601716 | chr13 | 28498451 | 31 | N |
| cg27373972 | chr6 | 33244788 | 14 | MU | cg20194314 | chr13 | 28503060 | 31 | MU |
| cg26381352 | chr6 | 33244799 | 14 | MU | cg06883496 | chr13 | 28503235 | 31 | N |
| cg21333861 | chr6 | 33244976 | 14 | MU | cg17200768 | chr13 | 28503373 | 31 | MU |
| cg07348922 | chr6 | 33244990 | 14 | N | cg04851742 | chr13 | 28503508 | 31 | N |
| cg27098900 | chr6 | 33245128 | 14 | MU | cg04570827 | chr13 | 46627156 | 31 | MU |
| cg19664267 | chr6 | 33245163 | 14 | MU | cg07376541 | chr13 | 46627165 | 31 | MU |
| cg15543281 | chr6 | 33245181 | 14 | MU | cg17305638 | chr13 | 46627203 | 31 | N |
| cg11772919 | chr6 | 33245328 | 14 | N | cg15902874 | chr14 | 31677716 | 31 | MU |
| cg17100761 | chr7 | 100318080 | 14 | N | cg01085210 | chr14 | 38067219 | 31 | MU |
| cg20893717 | chr7 | 100318190 | 14 | MU | cg19578835 | chr14 | 38067622 | 31 | MU |
| cg10864074 | chr7 | 100318194 | 14 | MU | cg21731375 | chr14 | 38067742 | 31 | N |
| cg09453116 | chr7 | 112430366 | 14 | MU | cg11086982 | chr14 | 73736972 | 31 | MU |
| cg16061381 | chr7 | 112430531 | 14 | MU | cg08234878 | chr14 | 73737095 | 31 | N |
| cg13874046 | chr7 | 112430565 | 14 | N | cg26585724 | chr15 | 90208739 | 31 | N |
| cg10822582 | chr7 | 112430590 | 14 | N | cg05009389 | chr15 | 90208810 | 31 | MU |
| cg01828650 | chr7 | 112430614 | 14 | N | cg26703661 | chr15 | 90208915 | 31 | MU |
| cg26386449 | chr7 | 112430654 | 14 | N | cg16809962 | chr16 | 21566263 | 31 | MU |
| cg03764259 | chr7 | 127176905 | 14 | MU | cg10155261 | chr16 | 21566385 | 31 | N |
| cg26243894 | chr7 | 128049684 | 14 | MU | cg02256631 | chr16 | 31342952 | 31 | MU |
| cg05224190 | chr7 | 139168523 | 14 | N | cg15817542 | chr16 | 31343056 | 31 | N |

EP 4 741 515 A2

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg25181507 | chr7 | 139168659 | 14 | MU | cg07277869 | chr16 | 69760850 | 31 | N |
| cg13578647 | chr7 | 139168804 | 14 | N | cg19194454 | chr16 | 69760878 | 31 | N |
| cg07981013 | chr7 | 156795767 | 14 | N | cg26598152 | chr16 | 69760928 | 31 | N |
| cg18845377 | chr7 | 156795892 | 14 | MU | cg05191006 | chr16 | 69760966 | 31 | MU |
| cg16283362 | chr7 | 156795914 | 14 | N | cg02066638 | chr16 | 87943038 | 31 | MU |
| cg04017769 | chr8 | 38644939 | 14 | MU | cg02672397 | chr16 | 87943158 | 31 | MU |
| cg01150641 | chr1 | 976168 | 15 | N | cg10110271 | chr16 | 87943182 | 31 | N |
| cg23625715 | chr1 | 976172 | 15 | MU | cg27246277 | chr17 | 42084477 | 31 | N |
| cg26222311 | chr1 | 976227 | 15 | N | cg00483503 | chr17 | 42084612 | 31 | MU |
| cg02390319 | chr1 | 3310268 | 15 | MU | cg07975472 | chr19 | 1503610 | 31 | MU |
| cg25369015 | chr1 | 9599256 | 15 | MU | cg22732101 | chr19 | 16394638 | 31 | N |
| cg19896639 | chr1 | 9599276 | 15 | N | cg21865533 | chr19 | 16394774 | 31 | MU |
| cg05039004 | chr1 | 29586299 | 15 | MU | cg10351808 | chr19 | 35632493 | 31 | MU |
| cg04933208 | chr1 | 29586414 | 15 | N | cg05216501 | chr19 | 35632564 | 31 | N |
| cg26884027 | chr1 | 29586418 | 15 | N | cg03710719 | chr19 | 36389795 | 31 | MU |
| cg08877374 | chr1 | 31845904 | 15 | N | cg27297851 | chr19 | 36389833 | 31 | N |
| cg14407437 | chr1 | 31845923 | 15 | N | cg19221841 | chr19 | 41197049 | 31 | MU |
| cg07345934 | chr1 | 31845943 | 15 | N | cg19643921 | chr19 | 41197106 | 31 | N |
| cg18368411 | chr1 | 31845959 | 15 | MU | cg04312358 | chr19 | 41197166 | 31 | N |
| cg15833534 | chr1 | 31845985 | 15 | N | cg15123692 | chr2 | 23886357 | 31 | MU |
| cg19316148 | chr1 | 31846089 | 15 | N | cg18303215 | chr2 | 44059003 | 31 | MU |
| cg19901801 | chr1 | 114697032 | 15 | N | cg04221886 | chr2 | 44059094 | 31 | MU |
| cg12804010 | chr1 | 114697113 | 15 | MU | cg23972738 | chr2 | 66667039 | 31 | N |
| cg23304296 | chr1 | 116961183 | 15 | N | cg15978039 | chr2 | 66667065 | 31 | N |
| cg07822305 | chr1 | 116961213 | 15 | MU | cg17454831 | chr2 | 66667073 | 31 | N |
| cg25743043 | chr1 | 116961233 | 15 | MU | cg16178603 | chr2 | 66667101 | 31 | MU |
| cg1717987S | chr1 | 116961404 | 15 | MU | cg08187418 | chr2 | 74425512 | 31 | N |
| cg17182302 | chr1 | 116961525 | 15 | N | cg13755546 | chr2 | 74425580 | 31 | MU |
| cg21860629 | chr1 | 153234268 | 15 | MU | cg21497607 | chr2 | 74425593 | 31 | N |
| cg11534293 | chr1 | 153234342 | 15 | N | cg20385508 | chr2 | 128180829 | 31 | MU |
| cg10228283 | chr1 | 153234387 | 15 | N | cg03422204 | chr2 | 128180959 | 31 | N |
| cg26944851 | chr10 | 23479645 | 15 | MU | cg09747827 | chr2 | 128181313 | 31 | N |
| cg24799921 | chr10 | 77168180 | 15 | MU | cg16348316 | chr2 | 128181325 | 31 | MU |
| cg11471062 | chr10 | 94459183 | 15 | N | cg13283691 | chr2 | 197852904 | 31 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg00149473 | chr10 | 94459330 | 15 | MU | cg16892443 | chr20 | 24898990 | 31 | MU |
| cg12865381 | chr10 | 94459447 | 15 | MU | cg13662093 | chr20 | 33865505 | 31 | MU |
| cg08151063 | chr10 | 94459528 | 15 | MU | cg07134254 | chr20 | 33865797 | 31 | MU |
| cg08822360 | chr10 | 94459553 | 15 | MU | cg04507495 | chr3 | 47563649 | 31 | MU |
| cg15273468 | chr10 | 94459665 | 15 | MU | cg08477641 | chr3 | 48507508 | 31 | N |
| cg22044002 | chr10 | 94825547 | 15 | N | cg09692335 | chr3 | 48507596 | 31 | N |
| cg00714725 | chr10 | 94825576 | 15 | N | cg26029997 | chr3 | 48507610 | 31 | MU |
| cg14375937 | chr10 | 94825667 | 15 | MU | cg19091784 | chr3 | 48507618 | 31 | MU |
| cg02825977 | chr10 | 94825848 | 15 | MU | cg09595163 | chr3 | 55515541 | 31 | N |
| cg23274680 | chr10 | 94828605 | 15 | MU | cg05781968 | chr3 | 55515607 | 31 | MU |
| cg16570157 | chr11 | 829783 | 15 | MU | cg00836482 | chr3 | 119528956 | 31 | MU |
| cg16459349 | chr11 | 6341717 | 15 | N | cg05670461 | chr3 | 157155088 | 31 | MU |
| cg20938665 | chr11 | 6341842 | 15 | N | cg15565872 | chr3 | 157155202 | 31 | N |
| cg27132391 | chr11 | 6341850 | 15 | N | cg00004082 | chr4 | 10020360 | 31 | MU |
| cg15202102 | chr11 | 6341867 | 15 | MU | cg02734326 | chr4 | 10020555 | 31 | MU |
| cg26678920 | chr11 | 6341888 | 15 | N | cg16147221 | chr4 | 10020634 | 31 | MU |
| cg05628549 | chr11 | 6341895 | 15 | N | cg15297110 | chr4 | 105412093 | 31 | MU |
| cg10064871 | chr11 | 6341908 | 15 | N | cg26817935 | chr5 | 58571800 | 31 | N |
| cg11166108 | chr11 | 18416767 | 15 | MU | cg17563336 | chr5 | 58571903 | 31 | MU |
| cg19323951 | chr11 | 64993076 | 15 | MU | cg20424833 | chr5 | 176829639 | 31 | MU |
| cg16053334 | chr11 | 65343118 | 15 | MU | cg24787470 | chr5 | 176829984 | 31 | MU |
| cg12641569 | chr11 | 70601781 | 15 | MU | cg06105778 | chr5 | 176830667 | 31 | MU |
| cg22049858 | chr11 | 94884121 | 15 | MU | cg03059420 | chr6 | 30923241 | 31 | N |
| cg08294986 | chr11 | 119227020 | 15 | MU | cg00474080 | chr6 | 30923243 | 31 | MU |
| cg10353108 | chr11 | 119227105 | 15 | MU | cg11935153 | chr6 | 30923306 | 31 | MU |
| cg27092752 | chr11 | 119227298 | 15 | MU | cg04464085 | chr6 | 30923327 | 31 | N |
| cg10904972 | chr11 | 119227328 | 15 | MU | cg05913906 | chr6 | 30923370 | 31 | MU |
| cg14359435 | chr11 | 119227592 | 15 | MU | cg03513762 | chr6 | 30923420 | 31 | N |
| cg25538627 | chr11 | 119227631 | 15 | MU | cg00334245 | chr6 | 30923446 | 31 | N |
| cg08533336 | chr11 | 119227717 | 15 | N | cg21776S83 | chr6 | 30923545 | 31 | N |
| cg20059012 | chr12 | 53613154 | 15 | MU | cg03714531 | chr6 | 30923590 | 31 | N |
| cg14415816 | chr12 | 106978920 | 15 | N | cg09657173 | chr6 | 30923593 | 31 | MU |
| cg00450784 | chr12 | 106979062 | 15 | MU | cg02872545 | chr6 | 30923697 | 31 | N |
| cg17851046 | chr12 | 106979078 | 15 | N | cg00119460 | chr6 | 30923743 | 31 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg16201038 | chr13 | 28544760 | 15 | MU | cg27080725 | chr7 | 100075304 | 31 | MU |
| cg01499197 | chr13 | 28544814 | 15 | N | cg13975648 | chr7 | 100075346 | 31 | N |
| cg11283429 | chr13 | 28549574 | 15 | N | cg05175020 | chr7 | 100075402 | 31 | N |
| cg12938068 | chr13 | 28549698 | 15 | MU | cg04739306 | chr7 | 100230781 | 31 | MU |
| cg01899130 | chr14 | 55348681 | 15 | MU | cg01749347 | chr7 | 100230985 | 31 | MU |
| cg05142765 | chr14 | 95234822 | 15 | N | cg04499151 | chr7 | 100231278 | 31 | MU |
| cg23695707 | chr14 | 95234826 | 15 | MU | cg13081600 | chr7 | 155597463 | 31 | N |
| cg18687675 | chr14 | 95234965 | 15 | N | cg20381798 | chr7 | 155597490 | 31 | MU |
| cg19716433 | chr14 | 103589780 | 15 | MU | cg13905586 | chr7 | 155597842 | 31 | MU |
| cg01237565 | chr14 | 105715025 | 15 | MU | cg02900995 | chr7 | 155598018 | 31 | MU |
| cg08867241 | chr15 | 53087487 | 15 | N | cg06815112 | chr8 | 27182871 | 31 | N |
| cg22078710 | chr15 | 53087599 | 15 | MU | cg18234130 | chr8 | 27182889 | 31 | MU |
| cg20334252 | chr15 | 53096248 | 15 | MU | cg21186296 | chr8 | 27182909 | 31 | N |
| cg22488745 | chr15 | 53096763 | 15 | N | cg23736307 | chr8 | 27182930 | 31 | MU |
| cg18189994 | chr15 | 53096816 | 15 | MU | cg07216194 | chr8 | 27182965 | 31 | MU |
| cg12738347 | chr15 | 53096854 | 15 | MU | cg23693289 | chr8 | 27183097 | 31 | MU |
| cg02558623 | chr15 | 53097060 | 15 | MU | cg14221460 | chr8 | 27183342 | 31 | MU |
| cg01663018 | chr15 | 53097777 | 15 | MU | cg15082028 | chr1 | 19043507 | 32 | MU |
| cg00860379 | chr15 | 53097822 | 15 | N | cg00794881 | chr1 | 19043573 | 32 | N |
| cg12352124 | chr15 | 53097840 | 15 | MU | cg08350288 | chr1 | 19043598 | 32 | N |
| cg16424519 | chr16 | 21531031 | 15 | N | cg22945302 | chr1 | 26606601 | 32 | MU |
| cg10025396 | chr16 | 21531113 | 15 | MU | cg01047432 | chr1 | 52195190 | 32 | MU |
| cg08512490 | chr16 | 72821498 | 15 | MU | cg14609025 | chr1 | 119550983 | 32 | MU |
| cg16630989 | chr16 | 72821566 | 15 | MU | cg04981885 | chr1 | 160991156 | 32 | MU |
| cg04814450 | chr16 | 72821665 | 15 | N | cg02838178 | chr1 | 201368613 | 32 | N |
| cg04150136 | chr16 | 88700916 | 15 | MU | cg10095226 | chr1 | 201368738 | 32 | MU |
| cg03026373 | chr17 | 36715787 | 15 | MU | cg07561547 | chr1 | 201368783 | 32 | N |
| cg14862207 | chr17 | 36715798 | 15 | MU | cg21964551 | chr1 | 201368791 | 32 | MU |
| cg04194674 | chr17 | 36715953 | 15 | MU | cg27425612 | chr1 | 201368843 | 32 | MU |
| cg19595402 | chr17 | 36728859 | 15 | MU | cg23291161 | chr1 | 201368914 | 32 | N |
| cg19584803 | chr17 | 37794056 | 15 | N | cg25947945 | chr1 | 201368946 | 32 | N |
| cg24526433 | chr17 | 37794080 | 15 | MU | cg25318301 | chr1 | 228566560 | 32 | MU |
| cg03337181 | chr17 | 39822434 | 15 | MU | cg11416243 | chr10 | 11207907 | 32 | MU |
| cg17928869 | chr17 | 39822542 | 15 | N | cg18892808 | chr10 | 11207969 | 32 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg17222164 | chr17 | 43339476 | 15 | N | cg00353773 | chr10 | 44880708 | 32 | N |
| cg03048083 | chr17 | 43339497 | 15 | MU | cg26718433 | chr10 | 44880730 | 32 | N |
| cg01627847 | chr17 | 43339512 | 15 | MU | cg10927719 | chr10 | 44880819 | 32 | MU |
| cg08124910 | chr17 | 43339515 | 15 | MU | cg24163616 | chr10 | 100027958 | 32 | MU |
| cg01832036 | chr17 | 48050274 | 15 | MU | cg06154084 | chr10 | 102792249 | 32 | MU |
| cg13604794 | chr17 | 48050338 | 15 | N | cg24023187 | chr10 | 102977196 | 32 | N |
| cg19558029 | chr18 | 44526605 | 15 | N | cg12334793 | chr10 | 102977342 | 32 | MU |
| cg12171183 | chr18 | 44526700 | 15 | N | cg10780890 | chr10 | 102978277 | 32 | MU |
| cg06198398 | chr18 | 44526707 | 15 | N | cg05762694 | chr10 | 103990984 | 32 | MU |
| cg20469799 | chr18 | 44526743 | 15 | MU | cg07754999 | chr10 | 103991057 | 32 | MU |
| cg05498007 | chr18 | 44526756 | 15 | N | cg00630724 | chr10 | 103991299 | 32 | MU |
| cg05529506 | chr18 | 44526867 | 15 | N | cg20641465 | chr10 | 103991465 | 32 | MU |
| cg11931731 | chr18 | 44527026 | 15 | MU | cg23994051 | chr10 | 112432175 | 32 | N |
| cg14694342 | chr19 | 5828172 | 15 | MU | cg03520976 | chr10 | 112432321 | 32 | MU |
| cg11575912 | chr19 | 5828299 | 15 | MU | cg24456130 | chr10 | 131768029 | 32 | N |
| cg09911521 | chr19 | 5828301 | 15 | N | cg27513573 | chr10 | 131768098 | 32 | MU |
| cg07759570 | chr19 | 35491951 | 15 | MU | cg08994045 | chr11 | 2883912 | 32 | MU |
| cg11631271 | chr19 | 38700487 | 15 | MU | cg1271SS69 | chr11 | 20154183 | 32 | MU |
| cg19221841 | chr19 | 41197049 | 15 | MU | cg09767822 | chr11 | 20178040 | 32 | N |
| cg19643921 | chr19 | 41197106 | 15 | N | cg08835103 | chr11 | 20178138 | 32 | MU |
| cg04312358 | chr19 | 41197166 | 15 | N | cg05469285 | chr11 | 20178979 | 32 | MU |
| cg23928165 | chr19 | 46996713 | 15 | MU | cg14427437 | chr11 | 20229471 | 32 | MU |
| cg11956396 | chrl9 | 55591707 | 15 | MU | cg16543094 | chr11 | 20229517 | 32 | MU |
| cg01165043 | chr2 | 64994401 | 15 | MU | cg23890008 | chr11 | 20229545 | 32 | N |
| cg18815025 | chr2 | 85980731 | 15 | N | cg13139203 | chr11 | 63581631 | 32 | MU |
| cg12930553 | chr2 | 85980742 | 15 | N | cg19311889 | chr12 | 57483793 | 32 | MU |
| cg03128635 | chr2 | 85980756 | 15 | MU | cg15126179 | chr13 | 79181338 | 32 | N |
| cg09662694 | chr2 | 85980803 | 15 | N | cg08403195 | chr13 | 79181440 | 32 | MU |
| cg15256164 | chr2 | 174220625 | 15 | MU | cg19981568 | chr14 | 60977300 | 32 | MU |
| cg20898829 | chr20 | 3229660 | 15 | MU | cg14282221 | chr14 | 101121018 | 32 | N |
| cg03876823 | chr20 | 32899771 | 15 | N | cg02480144 | chr14 | 101121093 | 32 | N |
| cg09250727 | chr20 | 32899784 | 15 | N | cg14500250 | chr14 | 101121140 | 32 | MU |
| cg12002708 | chr20 | 32899806 | 15 | MU | cg24015081 | chr14 | 105144033 | 32 | MU |
| cg23636941 | chr20 | 32899839 | 15 | N | cg22044901 | chr15 | 68126292 | 32 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg19069346 | chr21 | 41239214 | 15 | MU | cg25903779 | chr15 | 68128025 | 32 | MU |
| cg25174409 | chr21 | 41239323 | 15 | N | cg14210607 | chr15 | 68128175 | 32 | N |
| cg22413388 | chr22 | 46367617 | 15 | MU | cg02454890 | chr15 | 90544015 | 32 | MU |
| cg13458651 | chr22 | 46367703 | 15 | N | cg00133595 | chr16 | 31228059 | 32 | MU |
| cg03553786 | chr3 | 13692202 | 15 | N | cg10038009 | chr16 | 31228202 | 32 | MU |
| cg23620822 | chr3 | 13692212 | 15 | MU | cg13352836 | chr16 | 31228263 | 32 | N |
| cg08417257 | chr3 | 13692343 | 15 | N | cg08346922 | chr16 | 31228299 | 32 | N |
| cg13707894 | chr3 | 27674461 | 15 | MU | cg27656614 | chr16 | 81479309 | 32 | MU |
| cg23439966 | chr3 | 27674472 | 15 | N | cg06251016 | chrl6 | 89258571 | 32 | MU |
| cg22973319 | chr3 | 50310839 | 15 | MU | cg01465169 | chr17 | 27944849 | 32 | MU |
| cg22037648 | chr3 | 50311009 | 15 | MU | cg08377924 | chr17 | 41363628 | 32 | N |
| cg13385156 | chr4 | 1750374 | 15 | N | cg19548479 | chr17 | 41363737 | 32 | N |
| cg12293340 | chr4 | 1750442 | 15 | MU | cg24940138 | chr17 | 41363741 | 32 | MU |
| cg16720619 | chr4 | 85423302 | 15 | MU | cg21211480 | chr17 | 41363774 | 32 | N |
| cg18097224 | chr5 | 133451237 | 15 | MU | cg15026277 | chr17 | 41363789 | 32 | N |
| cg11485451 | chr5 | 133451240 | 15 | MU | cg03049782 | chr17 | 41363891 | 32 | N |
| cg13046271 | chr5 | 133451243 | 15 | N | cg18878992 | chr17 | 43974344 | 32 | MU |
| cg17487170 | chr5 | 133451259 | 15 | N | cg19156875 | chr17 | 43974446 | 32 | N |
| cg19844653 | chr5 | 133451294 | 15 | N | cg11849086 | chr17 | 62776530 | 32 | MU |
| cg25947408 | chr5 | 133451383 | 15 | N | cg19472078 | chr17 | 77020166 | 32 | MU |
| cg04356381 | chr6 | 25050788 | 15 | MU | cg18996910 | chr17 | 77020220 | 32 | MU |
| cg11282676 | chr6 | 26018003 | 15 | N | cg08349573 | chr17 | 77020243 | 32 | MU |
| cg14703002 | chr6 | 26018058 | 15 | N | cg24844534 | chr17 | 77020267 | 32 | N |
| cg23829024 | chr6 | 26018074 | 15 | MU | cg18009376 | chr17 | 80056900 | 32 | MU |
| cg05816193 | chr6 | 26018127 | 15 | N | cg05896042 | chr19 | 11450090 | 32 | MU |
| cg14652095 | chr6 | 26018185 | 15 | N | cg02349096 | chr19 | 11450198 | 32 | N |
| cg00903676 | chr6 | 26172091 | 15 | N | cg00523012 | chr19 | 13264324 | 32 | MU |
| cg01272707 | chr6 | 26172185 | 15 | MU | cg24125901 | chr19 | 18716439 | 32 | MU |
| cg27209395 | chr6 | 26172397 | 15 | MU | cg04072597 | chr19 | 18716499 | 32 | N |
| cg27579711 | chr6 | 26172931 | 15 | MU | cg10617994 | chr19 | 18716527 | 32 | N |
| cg11652360 | chr6 | 33160120 | 15 | N | cg23635599 | chr19 | 45281006 | 32 | N |
| cg17161718 | chr6 | 33160156 | 15 | N | cg01057656 | chr19 | 45281140 | 32 | MU |
| cg16458153 | chr6 | 33160192 | 15 | N | cg18612786 | chr19 | 45281236 | 32 | N |
| cg11384924 | chr6 | 33160224 | 15 | N | cg16529268 | chr19 | 45281284 | 32 | N |

(continued)

| Illumina Probe Name | Chr. | Position | Tissue/Cell | MU/N |
|---|---|---|---|---|
| cg01635061 | chr6 | 33160234 | 15 | N |
| cg27181765 | chr6 | 33160250 | 15 | N |
| cg13586420 | chr6 | 33160262 | 15 | MU |
| cg01243297 | chr6 | 33160292 | 15 | N |
| cg04770813 | chr6 | 33160297 | 15 | N |
| cg04138361 | chr6 | 33160304 | 15 | MU |
| cg02452416 | chr6 | 33160450 | 15 | N |
| cg03321503 | chr6 | 91320082 | 15 | N |
| cg23697202 | chr6 | 91320190 | 15 | MU |
| cg00538726 | chr6 | 91320336 | 15 | N |
| cg12600030 | chr7 | 5635820 | 15 | MU |
| cg10108372 | chr7 | 156811362 | 15 | N |
| cg03413097 | chr7 | 156811422 | 15 | MU |
| cg02379082 | chr7 | 156811484 | 15 | N |
| cg21617762 | chr7 | 156811517 | 15 | MU |
| cg09940032 | chr8 | 142215736 | 15 | MU |
| cg06768944 | chr8 | 142216276 | 15 | MU |
| cg13738140 | chr9 | 140189802 | 15 | MU |
| cg01978368 | chr1 | 16563189 | 16 | MU |
| cg14279121 | chr1 | 52194898 | 16 | MU |
| cg15141467 | chr1 | 155035375 | 16 | MU |
| cg00585901 | chr1 | 155035388 | 16 | MU |
| cg11491247 | chr1 | 155035530 | 16 | MU |
| cg27180868 | chr1 | 161008750 | 16 | MU |
| cg24161057 | chr1 | 161008811 | 16 | N |
| cg13906823 | chr1 | 161008824 | 16 | N |
| cg12426196 | chr1 | 161008826 | 16 | MU |
| cg04868078 | chr1 | 197898229 | 16 | MU |
| cg04894705 | chr1 | 212606756 | 16 | MU |
| cg22236626 | chr1 | 212606819 | 16 | MU |
| cg09850632 | chr1 | 229406661 | 16 | N |
| cg03301331 | chr1 | 229406681 | 16 | N |
| cg16909962 | chr1 | 229406711 | 16 | MU |
| cg24842260 | chr10 | 771164147 | 16 | MU |

| Illumina Probe Name | Chr. | Position | Tissu e/Cell Type | MU/N |
|---|---|---|---|---|
| cg04434147 | chr19 | 45281287 | 32 | N |
| cg252S3217 | chr19 | 47776728 | 32 | MU |
| cg24083817 | chr19 | 49217255 | 32 | MU |
| cg20495404 | chr2 | 8823649 | 32 | MU |
| cg04525491 | chr2 | 8823683 | 32 | MU |
| cg01184784 | chr2 | 8823756 | 32 | MU |
| cg06946452 | chr2 | 119606631 | 32 | N |
| cg24001710 | chr2 | 119606695 | 32 | N |
| cg26990102 | chr2 | 119606746 | 32 | MU |
| cg09385093 | chr2 | 119607885 | 32 | MU |
| cg08138685 | chr2 | 119610526 | 32 | MU |
| cg17342925 | chr2 | 119610605 | 32 | N |
| cg01469887 | chr2 | 175205716 | 32 | MU |
| cg09344183 | chr2 | 175205785 | 32 | N |
| cg27078140 | chr2 | 175205821 | 32 | N |
| cg10828660 | chr2 | 175205852 | 32 | MU |
| cg22306928 | chr2 | 219866424 | 32 | MU |
| cg00215432 | chr2 | 219866437 | 32 | MU |
| cg00218620 | chr2 | 219866460 | 32 | N |
| cg00705280 | chr2 | 219866493 | 32 | N |
| cg21615583 | chr2 | 219866512 | 32 | MU |
| cg11912765 | chr2 | 223159838 | 32 | N |
| cg18406033 | chr2 | 223159869 | 32 | MU |
| cg10544031 | chr2 | 223164635 | 32 | N |
| cg13496838 | chr2 | 223164747 | 32 | MU |
| cg14688579 | chr2 | 223168453 | 32 | MU |
| cg07119829 | chr2 | 223168487 | 32 | N |
| cg14559259 | chr2 | 223168575 | 32 | N |
| cg24419324 | chr2 | 223172329 | 32 | N |
| cg02099878 | chr2 | 223176167 | 32 | MU |
| cg11120927 | chr2 | 239072674 | 32 | MU |
| cg26705720 | chr20 | 10652787 | 32 | MU |
| cg20631750 | chr20 | 55839564 | 32 | MU |
| cg27565555 | chr20 | 55839663 | 32 | MU |

(continued)

| Illumina Probe Name | Chr. | Position | Tissue/Cell | MU/N | Illumina Probe Name | Chr. | Position | Tissue/Cell Type | MU/N |
|---|---|---|---|---|---|---|---|---|---|
| cg03958038 | chr10 | 77164227 | 16 | N | cg17599586 | chr22 | 32026779 | 32 | N |
| cg06706894 | chr10 | 80167002 | 16 | MU | cg22884130 | chr22 | 32026873 | 32 | MU |
| cg15550435 | chr10 | 92979939 | 16 | MU | cg10537193 | chr22 | 32026975 | 32 | N |
| cg07424295 | chr10 | 92980030 | 16 | N | cg00210994 | chr3 | 99594893 | 32 | N |
| cg17001035 | chr10 | 92980059 | 16 | N | cg15963552 | chr3 | 99594931 | 32 | MU |
| cg09176256 | chr10 | 119293967 | 16 | MU | cg20276377 | chr3 | 99595026 | 32 | MU |
| cg23087306 | chr10 | 119294055 | 16 | N | cg22092811 | chr3 | 99595145 | 32 | MU |
| cg26956371 | chr10 | 124713989 | 16 | MU | cg18337222 | chr3 | 99595254 | 32 | N |
| cg18565342 | chr10 | 124714098 | 16 | N | cg12180270 | chr4 | 26030463 | 32 | MU |
| cg10072309 | chr11 | 537558 | 16 | MU | cg23806894 | chr4 | 111535528 | 32 | N |
| cg27158867 | chr11 | 537638 | 16 | N | cg09780241 | chr4 | 111535597 | 32 | MU |
| cg13761440 | chr11 | 30608165 | 16 | N | cg20287640 | chr5 | 131825644 | 32 | MU |
| cg17518931 | chr11 | 30608198 | 16 | MU | cg02750098 | chr5 | 172656021 | 32 | N |
| cg00356723 | chr11 | 63705697 | 16 | N | cg26305817 | chr5 | 172656055 | 32 | N |
| cg24554061 | chr11 | 63705760 | 16 | MU | cg22009488 | chr5 | 172656166 | 32 | MU |
| cg04592747 | chr11 | 66035942 | 16 | MU | cg03490331 | chr5 | 172672817 | 32 | N |
| cg09284707 | chr11 | 107799080 | 16 | N | cg17543488 | chr5 | 172672959 | 32 | MU |
| cg08482682 | chr11 | 107799197 | 16 | MU | cg08506654 | chr5 | 172673098 | 32 | N |
| cg23156348 | chr11 | 124981869 | 16 | MU | cg01472299 | chr6 | 4775063 | 32 | MU |
| cg06199921 | chr11 | 124981942 | 16 | N | cg18559739 | chr6 | 4775132 | 32 | N |
| cg04244494 | chr12 | 510476 | 16 | N | cg24816460 | chr6 | 4775203 | 32 | N |
| cg10102418 | chr12 | 510594 | 16 | MU | cg08249424 | chr6 | 4775222 | 32 | MU |
| cg27509306 | chr12 | 510604 | 16 | N | cg26997880 | chr6 | 32055370 | 32 | N |
| cg03239552 | chr12 | 54339653 | 16 | MU | cg11823230 | chr6 | 32055385 | 32 | MU |
| cg16479539 | chr12 | 54345212 | 16 | MU | cg05483184 | chr6 | 32055393 | 32 | MU |
| cg02387326 | chr12 | 54345679 | 16 | MU | cg21460606 | chr6 | 32055402 | 32 | MU |
| cg16650901 | chr12 | 54345791 | 16 | N | cg19807376 | chr6 | 32055447 | 32 | MU |
| cg21707187 | chr12 | 54345862 | 16 | MU | cg15697476 | chr6 | 32055474 | 32 | N |
| cg09973284 | chr12 | 54346155 | 16 | MU | cg09996288 | chr6 | 32055517 | 32 | MU |
| cg26953453 | chr12 | 54346784 | 16 | N | cg17001689 | chr6 | 32055525 | 32 | N |
| cg02618300 | chr12 | 54346859 | 16 | MU | cg02740950 | chr6 | 32055534 | 32 | MU |
| cg14212206 | chr12 | 54350153 | 16 | N | cg18178010 | chr6 | 32055629 | 32 | N |
| cg13233461 | chr12 | 54350209 | 16 | MU | cg21404045 | chr7 | 27232412 | 32 | MU |
| cg06729806 | chr12 | 54350294 | 16 | N | cg16761035 | chr7 | 94285397 | 32 | N |

(continued)

| Illumina Probe Name | Chr. | Position | Tissu e/Cell Type | MU/N |
|---|---|---|---|---|
| cg04366249 | chr7 | 94285501 | 32 | MU |
| cg14388858 | chr7 | 94285520 | 32 | N |
| cg19107595 | chr7 | 94285642 | 32 | N |
| cg27452341 | chr7 | 143042635 | 32 | MU |
| cg27085741 | chr7 | 143042741 | 32 | MU |
| cg03002479 | chr7 | 143106004 | 32 | N |
| cg06161630 | chr7 | 143106114 | 32 | N |
| cg27426220 | chr7 | 143106126 | 32 | N |
| cg02376703 | chr7 | 143106130 | 32 | MU |
| cg03185704 | chr7 | 150020125 | 32 | N |
| cg18221429 | chr7 | 150020136 | 32 | N |
| cg07753583 | chr7 | 150020206 | 32 | N |
| cg22747650 | chr7 | 150020218 | 32 | MU |
| cg11026333 | chr7 | 150020269 | 32 | N |
| cg01270001 | chr7 | 150020401 | 32 | N |
| cg10191684 | chr8 | 22423014 | 32 | MU |
| cg07924703 | chr8 | 22423020 | 32 | MU |
| cg07469555 | chr8 | 22423055 | 32 | MU |
| cg21803959 | chr8 | 144503255 | 32 | MU |
| cg05799038 | chr8 | 144503807 | 32 | MU |
| cg07981910 | chr9 | 124461412 | 32 | MU |
| cg00070042 | chr9 | 133308594 | 32 | N |
| cg14362630 | chr9 | 133308669 | 32 | MU |
| cg13496979 | chr9 | 133308888 | 32 | MU |
| cg13943217 | chr9 | 133542125 | 32 | MU |
| cg11405655 | chr1 | 999599 | 33 | N |
| cg25523405 | chr1 | 999679 | 33 | MU |
| cg02105666 | chr1 | 999724 | 33 | MU |
| cg21139076 | chr1 | 999826 | 33 | N |
| cg01868612 | chr1 | 2478046 | 33 | MU |
| cg03529908 | chr1 | 2478089 | 33 | MU |
| cg20489026 | chr1 | 160053931 | 33 | MU |
| cg15580417 | chr1 | 160053947 | 33 | N |
| cg26612165 | chr1 | 181059499 | 33 | N |

| Illumina Probe Name | Chr. | Position | Tissu e/Cell | MU/N |
|---|---|---|---|---|
| cg22829917 | chr12 | 54357408 | 16 | N |
| cg18040901 | chr12 | 54357530 | 16 | MU |
| cg15731655 | chr12 | 54357604 | 16 | N |
| cg01524853 | chr12 | 54447807 | 16 | N |
| cg22747076 | chr12 | 54447873 | 16 | MU |
| cg14836636 | chr12 | 120106292 | 16 | MU |
| cg04579966 | chr13 | 24846221 | 16 | MU |
| cg05855588 | chr13 | 33590273 | 16 | MU |
| cg02388378 | chr16 | 54968727 | 16 | MU |
| cg01961105 | chr16 | 54968783 | 16 | N |
| cg10383447 | chr16 | 58535093 | 16 | MU |
| cg08092105 | chr16 | 58535384 | 16 | MU |
| cg11640773 | chr16 | 58535416 | 16 | N |
| cg27102864 | chr16 | 58535418 | 16 | N |
| cg00584450 | chr16 | 86612312 | 16 | N |
| cg08645670 | chr16 | 86612451 | 16 | MU |
| cg06995715 | chr16 | 86612590 | 16 | N |
| cg26958524 | chr16 | 86613067 | 16 | MU |
| cg11165158 | chr16 | 86613094 | 16 | N |
| cg07215504 | chr16 | 88497007 | 16 | MU |
| cg03035213 | chr16 | 88497077 | 16 | MU |
| cg20161976 | chr17 | 7976456 | 16 | MU |
| cg25711358 | chr17 | 7976532 | 16 | N |
| cg04368796 | chr17 | 40171968 | 16 | MU |
| cg18569335 | chr17 | 40171970 | 16 | N |
| cg03062002 | chr17 | 40832009 | 16 | MU |
| cg02048645 | chr17 | 49199316 | 16 | N |
| cg05207834 | chr17 | 49199381 | 16 | MU |
| cg14131285 | chr17 | 71949210 | 16 | MU |
| cg16833452 | chr17 | 74519436 | 16 | MU |
| cg00740088 | chr18 | 19745316 | 16 | N |
| cg17959327 | chr18 | 19745458 | 16 | MU |
| cg19612309 | chr19 | 41112379 | 16 | MU |
| cg19818998 | chr19 | 52207792 | 16 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg07644196 | chr2 | 105473969 | 16 | MU | cg03897866 | chr1 | 181059633 | 33 | MU |
| cg09601912 | chr2 | 119591144 | 16 | MU | cg04055819 | chr10 | 13749602 | 33 | MU |
| cg06946452 | chr2 | 119606631 | 16 | N | cg20517614 | chr10 | 13749625 | 33 | N |
| cg24001710 | chr2 | 119606695 | 16 | N | cg12565250 | chr10 | 72165370 | 33 | MU |
| cg26990102 | chr2 | 119606746 | 16 | MU | cg01657744 | chr10 | 88471748 | 33 | MU |
| cg09385093 | chr2 | 119607885 | 16 | MU | cg03303359 | chr10 | 88471773 | 33 | N |
| cg24430189 | chr2 | 128173685 | 16 | N | cg14794936 | chr10 | 88471856 | 33 | MU |
| cg02473600 | chr2 | 128173750 | 16 | MU | cg25365990 | chr10 | 88471978 | 33 | N |
| cg18110444 | chr2 | 136743460 | 16 | MU | cg09224689 | chr11 | 31825166 | 33 | MU |
| cg11009335 | chr2 | 166651299 | 16 | MU | cg04938549 | chr11 | 31825214 | 33 | MU |
| cg25492645 | chr2 | 166651320 | 16 | N | cg27011060 | chr11 | 31825226 | 33 | N |
| cg02764346 | chr2 | 166651375 | 16 | N | cg01579950 | chr11 | 64481018 | 33 | MU |
| cg07466807 | chr2 | 173326947 | 16 | MU | cg15676015 | chr11 | 67272636 | 33 | MU |
| cg18944451 | chr2 | 177023716 | 16 | N | cg16948827 | chr11 | 126455885 | 33 | MU |
| cg25916282 | chr2 | 177023760 | 16 | MU | cg10839291 | chr11 | 126456008 | 33 | N |
| cg10109215 | chr20 | 30605810 | 16 | N | cg10695848 | chr12 | 48206783 | 33 | MU |
| cg14162940 | chr20 | 30605868 | 16 | N | cg02408775 | chr12 | 48206848 | 33 | MU |
| cg23929284 | chr20 | 30605916 | 16 | MU | cg23588713 | chr12 | 56523270 | 33 | MU |
| cg23085343 | chr20 | 50159160 | 16 | MU | cg21334598 | chr12. | 117316490 | 33 | MU |
| cg11086066 | chr20 | 50159508 | 16 | N | cg02823783 | chr12 | 124246919 | 33 | MU |
| cg00401091 | chr20 | 50159536 | 16 | MU | cg01240444 | chr12 | 124247047 | 33 | N |
| cg13844474 | chr20 | 50159653 | 16 | N | cg11026555 | chr12 | 124247223 | 33 | MU |
| cg27307781 | chr21 | 37442289 | 16 | MU | cg27234864 | chr13 | 21295941 | 33 | MU |
| cg01697719 | chr22 | 19754125 | 16 | MU | cg04370314 | chr13 | 100643312 | 33 | MU |
| cg08382235 | chr22 | 19754251 | 16 | MU | cg22489321 | chr13 | 100643388 | 33 | N |
| cg07644571 | chr22 | 22307395 | 16 | MU | cg18477204 | chr14 | 70038884 | 33 | MU |
| cg20521702 | chr3 | 98452812 | 16 | N | cg02380334 | chr14 | 70038925 | 33 | N |
| cg13338207 | chr3 | 98452879 | 16 | N | cg14539393 | chr14 | 70038949 | 33 | N |
| cg10128511 | chr3 | 98452940 | 16 | MU | cg19567295 | chr14 | 89884237 | 33 | N |
| cg07390647 | chr3 | 98453086 | 16 | N | cg12790592 | chr14 | 89884273 | 33 | MU |
| cg03458172 | chr3 | 157815672 | 16 | N | cg23411981 | chr14 | 105940377 | 33 | MU |
| cg05401764 | chr3 | 157815808 | 16 | MU | cg03451296 | chr14 | 105940391 | 33 | MU |
| cg06046430 | chr4 | 77819534 | 16 | MU | cg06100807 | chr15 | 90030016 | 33 | MU |
| cg03477792 | chr4 | 77819574 | 16 | MU | cg08641118 | chr15 | 90030046 | 33 | N |

EP 4 741 515 A2

(continued)

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg18351406 | chr4 | 77819688 | 16 | N | cg22526139 | chr15 | 90030153 | 33 | N |
| cg26590411 | chr4 | 119199646 | 16 | MU | cg01003448 | chr16 | 745685 | 33 | N |
| cg07954414 | chr4 | 154389148 | 16 | MU | cg07482202 | chr16 | 745687 | 33 | MU |
| cg09508531 | chr5 | 122424059 | 16 | MU | cg01195246 | chr16 | 745765 | 33 | N |
| cg10480584 | chr5 | 122424091 | 16 | N | cg10504751 | chr16 | 56390830 | 33 | MU |
| cg03490331 | chr5 | 172672817 | 16 | N | cg04313875 | chr17 | 1553202 | 33 | MU |
| cg17543488 | chr5 | 172672959 | 16 | MU | cg15380836 | chr17 | 1553341 | 33 | N |
| cg08506654 | chr5 | 172673098 | 16 | N | cg12608565 | chr17 | 4802692 | 33 | N |
| cg17796323 | chr5 | 177547973 | 16 | N | cg05529816 | chr17 | 4802735 | 33 | N |
| cg12526807 | chr5 | 177548050 | 16 | MU | cg14275842 | chr17 | 4802813 | 33 | MU |
| cg26997880 | chr6 | 32055370 | 16 | N | cg07834574 | chr17 | 4802847 | 33 | MU |
| cg11823230 | chr6 | 32055385 | 16 | N | cg14482741 | chr17 | 4802906 | 33 | MU |
| cg05483184 | chr6 | 32055393 | 16 | N | cg20814095 | chr17 | 4802981 | 33 | MU |
| cg21460606 | chr6 | 32055402 | 16 | MU | cg21071097 | chr17 | 4803053 | 33 | N |
| cg19807376 | chr6 | 32055447 | 16 | N | cg26695387 | chr17 | 21189768 | 33 | N |
| cg15697476 | chr6 | 32055474 | 16 | N | cg15416179 | chr17 | 21189859 | 33 | MU |
| cg09996288 | chr6 | 32055517 | 16 | MU | cg14862207 | chr17 | 36715798 | 33 | MU |
| cg17001689 | chr6 | 32055525 | 16 | N | cg10188897 | chr17 | 42392436 | 33 | MU |
| cg02740950 | chr6 | 32055534 | 16 | N | cg03061122 | chr17 | 42392559 | 33 | MU |
| cg18178010 | chr6 | 32055629 | 16 | MU | cg01122302 | chr17 | 42392668 | 33 | N |
| cg04911280 | chr6 | 44281184 | 16 | MU | cg11088422 | chr17 | 42392697 | 33 | MU |
| cg19291355 | chr6 | 44281188 | 16 | MU | cg17083506 | chr17 | 48226252 | 33 | MU |
| cg05917988 | chr6 | 44281197 | 16 | N | cg05735639 | chr17 | 49021875 | 33 | N |
| cg01122755 | chr6 | 44281249 | 16 | N | cg17129986 | chr17 | 49022021 | 33 | MU |
| cg22387890 | chr7 | 6145595 | 16 | MU | cg00023001 | chr17 | 55938748 | 33 | N |
| cg05848863 | chr7 | 16794078 | 16 | MU | cg22451358 | chr17 | 55938871 | 33 | MU |
| cg26551975 | chr7 | 27211555 | 16 | MU | cg06452419 | chr17 | 55938940 | 33 | N |
| cg12997720 | chr7 | 27225058 | 16 | N | cg12605662 | chr18 | 56935199 | 33 | MU |
| cg05977669 | chr7 | 27225070 | 16 | N | cg04583149 | chr19 | 1207124 | 33 | MU |
| cg24446586 | chr7 | 27225078 | 16 | N | cg10094078 | chr19 | 1467925 | 33 | N |
| cg13352750 | chr7 | 27225123 | 16 | MU | cg03306486 | chr19 | 1467952 | 33 | MU |
| cg15916646 | chr7 | 27225127 | 16 | N | cg19333963 | chr19 | 1467979 | 33 | MU |
| cg10767141 | chr7 | 27225143 | 16 | N | cg04314308 | chr19 | 19336150 | 33 | N |
| cg15760840 | chr7 | 27225222 | 16 | N | cg25814383 | chr19 | 19336240 | 33 | MU |

(continued)

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg10979436 | chr7 | 27242005 | 16 | N | cg03096975 | chr19 | 46142574 | 33 | N |
| cg04521510 | chr7 | 27242044 | 16 | MU | cg07804711 | chr19 | 46142647 | 33 | MU |
| cg20672981 | chr7 | 27242161 | 16 | N | cg12536502 | chr19 | 46142673 | 33 | MU |
| cg05852040 | chr7 | 77044873 | 16 | MU | cg07749923 | chr19 | 46142711 | 33 | N |
| cg10548492 | chr7 | 96746798 | 16 | MU | cg01544351 | chr19 | 46142757 | 33 | MU |
| cg26792755 | chr7 | 140714919 | 16 | MU | cg22893791 | chr2 | 26407628 | 33 | N |
| cg03002479 | chr7 | 143106004 | 16 | N | cg10927841 | chr2 | 26407743 | 33 | MU |
| cg06161630 | chr7 | 143106114 | 16 | N | cg20289949 | chr2 | 43019614 | 33 | N |
| cg27426220 | chr7 | 143106126 | 16 | N | cg20857709 | chr2 | 43019727 | 33 | MU |
| cg02376703 | chr7 | 143106130 | 16 | MU | cg26360792 | chr2 | 43019997 | 33 | MU |
| cg03185704 | chr7 | 150020125 | 16 | N | cg27299406 | chr2 | 43020013 | 33 | N |
| cg18221429 | chr7 | 150020136 | 16 | N | cg09480054 | chr2 | 43020129 | 33 | MU |
| cg07753583 | chr7 | 150020206 | 16 | N | cg08738430 | chr2 | 43452561 | 33 | MU |
| cg22747650 | chr7 | 150020218 | 16 | MU | cg25954914 | chr2 | 54785183 | 33 | MU |
| cg11026333 | chr7 | 150020269 | 16 | N | cg15543284 | chr2 | 54785307 | 33 | N |
| cg01270001 | chr7 | 150020401 | 16 | MU | cg07850967 | chr2 | 54785550 | 33 | MU |
| cg05472468 | chr8 | 72917350 | 16 | N | cg02271560 | chr2 | 55450737 | 33 | N |
| cg01490094 | chr8 | 72917363 | 16 | N | cg11652636 | chr2 | 55450823 | N | N |
| cg02843349 | chr8 | 72917432 | 16 | MU | cg20042692 | chr2 | 55450874 | 33 | N |
| cg16150678 | chr8 | 101170369 | 16 | MU | cg18462521 | chr2 | 64879911 | 33 | MU |
| cg14093715 | chr9 | 126776180 | 16 | MU | cg11009335 | chr2 | 166651299 | 33 | MU |
| cg13492692 | chr9 | 126777673 | 16 | MU | cg25492645 | chr2 | 166651320 | 33 | MU |
| cg14425564 | chr9 | 126794772 | 16 | N | cg02764346 | chr2 | 166651375 | 33 | N |
| cg14071579 | chr9 | 126794830 | 16 | MU | cg10413944 | chr2 | 224702734 | 33 | MU |
| cg14204784 | chr9 | 129387231 | 16 | MU | cg09456094 | chr2 | 231280878 | 33 | MU |
| cg12777520 | chr9 | 129387303 | 16 | N | cg11827097 | chr2 | 231280903 | 33 | N |
| cg14063191 | chr9 | 131486742 | 16 | MU | cg11702456 | chr2 | 231280907 | 33 | MU |
| cg00070042 | chr9 | 133308594 | 16 | N | cg20067688 | chr20 | 30449097 | 33 | MU |
| cg14362630 | chr9 | 133308669 | 16 | MU | cg14745622 | chr20 | 61447686 | 33 | N |
| cg13496979 | chr9 | 133308888 | 16 | MU | cg20331689 | chr20 | 61447729 | 33 | N |
| cg06974755 | chr9 | 135996931 | 16 | MU | cg15266508 | chr20 | 61447742 | 33 | N |
| cg17170948 | chr1 | 59281383 | 17 | MU | cg16653692 | chr20 | 61447749 | 33 | N |
| cg23075983 | chr1 | 59281540 | 17 | MU | cg16847645 | chr20 | 61447790 | 33 | N |
| cg04282788 | chr1 | 210407473 | 17 | N | cg22489878 | chr20 | 61447803 | 33 | N |

(continued)

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg25876656 | chr1 | 210407480 | 17 | N | cg27206421 | chr20 | 61447818 | 33 | N |
| cg20270863 | chr1 | 210407484 | 17 | MU | cg16132351 | chr20 | 61447823 | 33 | MU |
| cg08900371 | chr1 | 210407556 | 17 | MU | cg18291290 | chr20 | 61447879 | 33 | N |
| cg18265162 | chr1 | 210407588 | 17 | N | cg07845648 | chr20 | 61447915 | 33 | N |
| cg05723989 | chr1 | 210407677 | 17 | N | cg18414624 | chr20 | 61447929 | 33 | N |
| cg11401257 | chr1 | 226926945 | 17 | MU | cg00612595 | chr21 | 47717864 | 33 | MU |
| eg11222527 | chr1 | 226926964 | 17 | N | cg00815214 | chr21 | 47717953 | 33 | N |
| cg05869122 | chr1 | 226926968 | 17 | N | cg05611779 | chr22 | 24552379 | 33 | MU |
| cg19160520 | chr1 | 226927217 | 17 | N | cg25247183 | chr22 | 38073049 | 33 | N |
| cg13209122 | chr1 | 226927327 | 17 | MU | cg06822952 | chr22 | 38073178 | 33 | MU |
| cg03669282 | chr1 | 228566718 | 17 | MU | cg04689080 | chr22 | 46263207 | 33 | MU |
| cg08313638 | chr10 | 12110577 | 17 | MU | cg17200465 | chr3 | 40428508 | 33 | N |
| cg07262635 | chr10 | 18689378 | 17 | N | cg00656387 | chr3 | 40428638 | 33 | MU |
| cg25327888 | chr10 | 18689440 | 17 | MU | cg13409248 | chr3 | 40428643 | 33 | N |
| cg12289251 | chr10 | 18689471 | 17 | N | cg10755058 | chr3 | 40428713 | 33 | MU |
| cg12868173 | chr10 | 44185097 | 17 | MU | cg18306943 | chr3 | 40428807 | 33 | N |
| cg17276002 | chr10 | 75172806 | 17 | MU | cg03355998 | chr3 | 147124363 | 33 | MU |
| cg06353720 | chr10 | 91087558 | 17 | N | cg21127068 | chr3 | 147124417 | 33 | N |
| cg07310984 | chr10 | 91087591 | 17 | N | cg16209664 | chr3 | 147131146 | 33 | N |
| cg04364339 | chr10 | 91087703 | 17 | MU | cg12204258 | chr3 | 147131178 | 33 | MU |
| cg24681845 | chr10 | 126069759 | 17 | MU | cg08160350 | chr4 | 996052 | 33 | MU |
| cg08507975 | chr10 | 130339186 | 17 | N | cg05327835 | chr4 | 996175 | 33 | MU |
| cg20319211 | chr10 | 130339292 | 17 | N | cg04508482 | chr4 | 996280 | 33 | N |
| cg12093371 | chr10 | 130339316 | 17 | MU | cg08844887 | chr4 | 1006120 | 33 | N |
| cg22806341 | chr10 | 130339363 | 17 | N | cg17105609 | chr4 | 1006237 | 33 | MU |
| cg01187997 | chr11 | 1594830 | 17 | N | cg08774969 | chr4 | 1006240 | 33 | MU |
| cg22831269 | chr11 | 1594867 | 17 | N | cg20564913 | chr4 | 1006270 | 33 | MU |
| cg17061505 | chr11 | 1594896 | 17 | MU | cg05866411 | chr4 | 1006278 | 33 | MU |
| cg25247290 | chr11 | 32461212 | 17 | MU | cg17807131 | chr4 | 4854690 | 33 | MU |
| cg26993251 | chr11 | 63934014 | 17 | MU | cg13752974 | chr4 | 4854786 | 33 | MU |
| cg25168700 | chr11 | 64067685 | 17 | N | cg13084458 | chr4 | 128553917 | 33 | N |
| cg24027931 | chr11 | 64067687 | 17 | N | cg11779273 | chr4 | 128553930 | 33 | MU |
| cg04855748 | chr11 | 64067763 | 17 | N | cg15437651 | chr4 | 128554035 | 33 | N |
| cg18383813 | chr11 | 64067793 | 17 | N | cg07174665 | chr4 | 129474594 | 33 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg09675196 | chr11 | 64067795 | 17 | MU | cg17521583 | chr4 | 129474618 | 33 | MU |
| cg05098417 | chr11 | 64067858 | 17 | N | cg15225701 | chr5 | 108083455 | 33 | MU |
| cg23069847 | chr11 | 64067868 | 17 | N | cg09565404 | chr5 | 108083489 | 33 | N |
| cg04255276 | chr11 | 65314021 | 17 | N | cg04337096 | chr5 | 146257347 | 33 | N |
| cg13044675 | chr11 | 65314161 | 17 | MU | cg20560075 | chr5 | 146257484 | 33 | MU |
| cg00496004 | chr11 | 130029481 | 17 | N | cg12232308 | chr6 | 30711580 | 33 | N |
| cg21029769 | chr11 | 130029515 | 17 | MU | cg13337949 | chr6 | 30711586 | 33 | N |
| cg26021246 | chr11 | 130029615 | 17 | MU | cg17389738 | chr6 | 30711642 | 33 | N |
| cg18373354 | chr11 | 130029684 | 17 | MU | cg20178172 | chr6 | 30711655 | 33 | MU |
| cg20749769 | chr11 | 130029961 | 17 | MU | cg08384314 | chr6 | 30711680 | 33 | MU |
| cg10089145 | chr11 | 130030103 | 17 | N | cg13804182 | chr6 | 30711796 | 33 | N |
| cg08161947 | chr12 | 19592900 | 17 | MU | cg16493531 | chr6 | 30711802 | 33 | MU |
| cg07145683 | chr12 | 53297443 | 17 | N | cg10916998 | chr6 | 30711832 | 33 | N |
| cg10624054 | chr12 | 53297547 | 17 | MU | cg00011994 | chr6 | 30711904 | 33 | N |
| cg10046767 | chr12 | 53297693 | 17 | N | cg18353226 | chr6 | 33280390 | 33 | N |
| cg03946762 | chr12 | 54785043 | 17 | N | cg14419102 | chr6 | 33280400 | 33 | N |
| cg20967139 | chr12 | 54785055 | 17 | N | cg06375761 | chr6 | 33280408 | 33 | N |
| cg18886436 | chr12 | 54785171 | 17 | MU | cg26083458 | chr6 | 33280424 | 33 | N |
| cg07000334 | chr12 | 54785286 | 17 | N | cg13638257 | chr6 | 33280436 | 33 | N |
| cg11856711 | chr12 | 54785309 | 17 | N | cg05700142 | chr6 | 33280454 | 33 | N |
| cg17655624 | chr12 | 54785320 | 17 | N | cg01654446 | chr6 | 33280476 | 33 | N |
| cg03853227 | chr12 | 107487365 | 17 | N | cg06230948 | chr6 | 33280518 | 33 | MU |
| cg03742214 | chr12 | 107487383 | 17 | MU | cg03591496 | chr6 | 33280653 | 33 | N |
| cg08900101 | chr12 | 114833043 | 17 | MU | cg12215478 | chr6 | 35887710 | 33 | N |
| cg07545317 | chr12 | 115174598 | 17 | MU | cg17639265 | chr6 | 35887853 | 33 | MU |
| cg25930673 | chr12 | 123319894 | 17 | MU | cg23514619 | chr6 | 43237330 | 33 | MU |
| cg09495418 | chr15 | 51634039 | 17 | MU | cg04537567 | chr6 | 99296068 | 33 | MU |
| cg05626259 | chr15 | 68851385 | 17 | N | cg14712531 | chr6 | 108485043 | 33 | MU |
| cg07580591 | chr15 | 68851466 | 17 | MU | cg24472375 | chr6 | 108485102 | 33 | N |
| cg10504751 | chr16 | 56390830 | 17 | MU | cg09236658 | chr6 | 108485152 | 33 | N |
| cg10383447 | chr16 | 58535093 | 17 | MU | cg06103642 | chr6 | 108496716 | 33 | MU |
| cg08092105 | chr16 | 58535384 | 17 | MU | cg11086764 | chr6 | 108496811 | 33 | N |
| cg11640773 | chr16 | 58535416 | 17 | N | cg21762523 | chr6 | 108497717 | 33 | MU |
| cg27102864 | chr16 | 58535418 | 17 | N | cg03673965 | chr6 | 108497734 | 33 | N |

EP 4 741 515 A2

(continued)

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg02506867 | chr16 | 68121045 | 17 | MU | cg19672993 | chr7 | 70160734 | 33 | MU |
| cg19761211 | chr16 | 70415365 | 17 | N | cg05650680 | chr8 | 1765217 | 33 | N |
| cg01269537 | chr16 | 70415514 | 17 | MU | cg25673948 | chr8 | 1765296 | 33 | N |
| cg23669611 | chr17 | 1901684 | 17 | MU | cg11998141 | chr8 | 1765309 | 33 | N |
| cg22298430 | chr17 | 5404330 | 17 | MU | cg09177567 | chr8 | 1765312 | 33 | N |
| cg27230784 | chr17 | 5404337 | 17 | MU | cg09899173 | chr8 | 1765336 | 33 | MU |
| cg06462347 | chr17 | 5404508 | 17 | N | cg15821939 | chr8 | 1765387 | 33 | N |
| cg18671949 | chr17 | 5404581 | 17 | MU | cg16481961 | chr8 | 1765421 | 33 | N |
| cg07990843 | chr17 | 25676201 | 17 | MU | cg00790020 | chr8 | 1765477 | 33 | N |
| cg27413732 | chr17 | 27942773 | 17 | MU | cg10191684 | chr8 | 22423014 | 33 | MU |
| cg13757826 | chr17 | 27943097 | 17 | MU | cg07924703 | chr8 | 22423020 | 33 | MU |
| cg13118906 | chr17 | 61524292 | 17 | MU | cg07469555 | chr8 | 22423055 | 33 | MU |
| cg14204735 | chr17 | 61524304 | 17 | N | cg10698928 | chr8 | 65290320 | 33 | MU |
| cg09007236 | chr17 | 78228820 | 17 | MU | cg14156441 | chr8 | 126447096 | 33 | MU |
| cg26663796 | chr17 | 80255734 | 17 | MU | cg14425564 | chr9 | 126794772 | 33 | N |
| cg08076807 | chr17 | 80255910 | 17 | MU | cg14071579 | chr9 | 126794830 | 33 | MU |
| cg01653417 | chr17 | 80256028 | 17 | N | cg11453712 | chr9 | 139741803 | 33 | N |
| cg02759002 | chr17 | 80256046 | 17 | N | cg14245947 | chr9 | 139741866 | 33 | MU |
| cg26647303 | chr19 | 2041955 | 17 | N | cg09513309 | chr1 | 6614775 | 34 | MU |
| cg00434573 | chr19 | 2041960 | 17 | N | cg23394391 | chr1 | 8409065 | 34 | MU |
| cg18418335 | chr19 | 2042088 | 17 | MU | cg21449655 | chr1 | 15851444 | 34 | MU |
| cg11894504 | chr19 | 2042391 | 17 | MU | cg06703079 | chr1 | 46597625 | 34 | MU |
| cg06908136 | chr19 | 2478866 | 17 | N | cg23660154 | chr1 | 110626540 | 34 | MU |
| cg12120729 | chr19 | 2479002 | 17 | MU | cg14313310 | chr1 | 184006816 | 34 | N |
| cg02624855 | chr19 | 41111297 | 17 | MU | cg00044463 | chr1 | 184006933 | 34 | MU |
| cg07808231 | chr19 | 41111347 | 17 | MU | cg06371502 | chr1 | 197879766 | 34 | MU |
| cg19657814 | chr19 | 47137444 | 17 | MU | cg04868078 | chr1 | 197898229 | 34 | MU |
| cg26629969 | chr19 | 51069496 | 17 | MU | cg17869355 | chr1 | 207038496 | 34 | MU |
| cg17519645 | chr19 | 52206667 | 17 | N | cg08479073 | chr1 | 207038584 | 34 | N |
| cg16854917 | chr19 | 52206691 | 17 | MU | cg08313638 | chr10 | 12110577 | 34 | MU |
| cg09776910 | chr19 | 52674958 | 17 | MU | cg07262635 | chr10 | 18689378 | 34 | N |
| cg08203569 | chr19 | 52675077 | 17 | N | cg25327888 | chr10 | 18689440 | 34 | MU |
| cg16851771 | chr2 | 42360170 | 17 | MU | cg12289251 | chr10 | 18689471 | 34 | N |
| cg09588360 | chr2 | 54785795 | 17 | MU | cg00579859 | chr10 | 60026291 | 34 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg15747933 | chr2 | 152146478 | 17 | MU | cg02616947 | chr10 | 102822508 | 34 | MU |
| cg25748441 | chr2 | 202122587 | 17 | N | cg05855489 | chr10 | 104503620 | 34 | N |
| cg23882545 | chr2 | 202122653 | 17 | N | cg12846190 | chr10 | 104503666 | 34 | MU |
| cg14962032 | chr2 | 202122669 | 17 | MU | cg14838188 | chr10 | 104503687 | 34 | MU |
| cg12604794 | chr2 | 202122753 | 17 | N | cg02230593 | chr10 | 119293083 | 34 | N |
| cg00978584 | chr2 | 202122832 | 17 | MU | cg26288991 | chr10 | 119293207 | 34 | MU |
| cg21840041 | chr2 | 232478360 | 17 | N | cg04421971 | chr10 | 119293341 | 34 | N |
| cg22331108 | chr2 | 232478452 | 17 | MU | cg09176256 | chr10 | 119293967 | 34 | MU |
| cg18166947 | chr22 | 18189443 | 17 | MU | cg23087306 | chr10 | 119294055 | 34 | N |
| cg03987506 | chr3 | 8810549 | 17 | MU | cg07833262 | chr10 | 119296182 | 34 | N |
| cg00078085 | chr3 | 8810592 | 17 | N | cg20792376 | chr10 | 119296297 | 34 | MU |
| cg17573813 | chr3 | 61237223 | 17 | MU | cg06141846 | chr10 | 119296305 | 34 | N |
| cg03416645 | chr3 | 105087206 | 17 | N | cg06456154 | chr10 | 119310356 | 34 | N |
| cg05645404 | chr3 | 105087242 | 17 | MU | cg15526081 | chr10 | 119310404 | 34 | MU |
| cg01031032 | chr3 | 112051894 | 17 | N | cg08451832 | chr10 | 119310964 | 34 | MU |
| cg09006015 | chr3 | 112051908 | 17 | N | cg09334277 | chr10 | 119312077 | 34 | MU |
| cg04367486 | chr3 | 112051991 | 17 | MU | cg03667544 | chr10 | 135049598 | 34 | MU |
| cg12637869 | chr3 | 121741209 | 17 | MU | cg11190277 | chr11 | 69455213 | 34 | N |
| cg02436004 | chr3 | 128211540 | 17 | N | cg26399164 | chr11 | 69455217 | 34 | N |
| cg16674492 | chr3 | 128211686 | 17 | MU | cg00858899 | chr11 | 69455277 | 34 | N |
| cg22122410 | chr3 | 128212269 | 17 | MU | cg07781399 | chr11 | 69455287 | 34 | N |
| cg19638477 | chr3 | 128212273 | 17 | N | cg03735308 | chr11 | 69455331 | 34 | MU |
| cg03458172 | chr3 | 157815672 | 17 | MU | cg12825880 | chr11 | 69455671 | 34 | MU |
| cg05401764 | chr3 | 157815808 | 17 | MU | cg12162138 | chr11 | 78673073 | 34 | MU |
| cg17251433 | chr3 | 185216870 | 17 | MU | cg17542408 | chr11 | 78673100 | 34 | N |
| cg26824216 | chr3 | 187086147 | 17 | N | cg16235582 | chr11 | 78673212 | 34 | N |
| cg15701237 | chr3 | 187086154 | 17 | N | cg03910065 | chr11 | 115372874 | 34 | MU |
| cg19383430 | chr3 | 187086187 | 17 | MU | cg10553088 | chr11 | 115372912 | 34 | N |
| cg16209795 | chr3 | 192126825 | 17 | N | cg14030346 | chr11 | 115372975 | 34 | N |
| cg16366473 | chr3 | 192126849 | 17 | MU | cg25364859 | chr12 | 50222202 | 34 | MU |
| cg15048660 | chr3 | 192126935 | 17 | MU | cg07541701 | chr12 | 54389934 | 34 | N |
| cg23207710 | chr3 | 192126960 | 17 | MU | cg14639847 | chr12 | 54389982 | 34 | MU |
| cg01257828 | chr3 | 192126996 | 17 | N | cg21837192 | chr12 | 54398765 | 34 | N |
| cg22834653 | chr3 | 192232077 | 17 | MU | cg20643952 | chr12 | 54398809 | 34 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg12422844 | chr4 | 8201125 | 17 | N | cg12590051 | chr12 | 54398891 | 34 | N |
| cg17352995 | chr4 | 8201128 | 17 | MU | cg18540674 | chr12 | 54405379 | 34 | MU |
| cg00361146 | chr4 | 139163780 | 17 | MU | cg03078363 | chr12 | 54408664 | 34 | MU |
| cg21985631 | chr4 | 175138377 | 17 | MU | cg16937769 | chr12 | 54.409485 | 34 | N |
| cg20954179 | chr4 | 175138488 | 17 | N | cg04704531 | chr12 | 54409491 | 34 | MU |
| cg04833050 | chr5 | 77806051 | 17 | N | cg15817960 | chr12 | 54409599 | 34 | N |
| cg01283201 | chr5 | 77806169 | 17 | MU | cg04576672 | chr12 | 54422239 | 34 | N |
| cg03729337 | chr5 | 122430019 | 17 | N | cg27441225 | chr12 | 54422350 | 34 | MU |
| cg27087956 | chr5 | 122430153 | 17 | MU | cg11941633 | chr12 | 54422447 . | 34 | MU |
| cg02564291 | chr5 | 122430235 | 17 | N | cg22378817 | chr12 | 54422488 | 34 | N |
| cg19328475 | chr5 | 122430277 | 17 | N | cg18041884 | chr12 | 109162555 | 34 | N |
| cg01054755 | chr5 | 138728685 | 17 | MU | cg10800464 | chr12 | 109162641 | 34 | MU |
| cg24761867 | chr5 | 138728704 | 17 | N | cg20236513 | chr12 | 109162748 | 34 | N |
| cg18398056 | chr6 | 1607922 | 17 | N | cg01765077 | chr12 | 122356316 | 34 | MU |
| cg04661888 | chr6 | 1608060 | 17 | MU | cg21171335 | chr12 | 122356390 | 34 | N |
| cg23989119 | chr6 | 1608107 | 17 | N | cg22618164 | chr12 | 122356400 | 34 | N |
| cg12238593 | chr6 | 26440340 | 17 | MU | cg25930673 | chr12 | 123319894 | 34 | N |
| cg00087417 | chr6 | 35887678 | 17 | MU | cg22842854 | chr12 | 123319900 | 34 | MU |
| cg12215478 | chr6 | 35887710 | 17 | MU | cg05034702 | chr13 | 61989701 | 34 | MU |
| cg24087211 | chr6 | 139309874 | 17 | MU | cg15839219 | chr13 | 61989724 | 34 | N |
| cg01425666 | chr7 | 5468436 | 17 | MU | cg17903776 | chr13 | 61989726 | 34 | N |
| cg06550177 | chr7 | 5469535 | 17 | MU | cg15182360 | chr13 | 61989737 | 34 | N |
| cg13421062 | chr7 | 35301069 | 17 | N | cg12721730 | chr13 | 61989817 | 34 | N |
| cg05809668 | chr7 | 35301188 | 17 | MU | cg09296044 | chr15 | 43477606 | 34 | MU |
| cg16911981 | chr7 | 70061135 | 17 | MU | cg04864199 | chr15 | 45493327 | 34 | MU |
| cg07039423 | chr7 | 128531106 | 17 | MU | cg17911021 | chr15 | 45493416 | 34 | N |
| cg22939733 | chr7 | 128531165 | 17 | N | cg23855392 | chr15 | 80189782 | 34 | MU |
| cg26572488 | chr7 | 132299669 | 17 | MU | cg16718263 | chr15 | 96596349 | 34 | MU |
| cg04624228 | chr8 | 38411598 | 17 | N | cg13920934 | chr15 | 96596478 | 34 | N |
| cg04458368 | chr8 | 38411682 | 17 | N | cg00167654 | chr17 | 5973536 | 34 | MU |
| cg24859937 | chr8 | 38411708 | 17 | MU | cg 13484608 | chr17 | 21191452 | 34 | MU |
| cg15183991 | chr8 | 145047566 | 17 | N | cg06862673 | chr17 | 37617707 | 34 | MU |
| cg00886954 | chr8 | 145047713 | 17 | MU | cg14131285 | chr17 | 71949210 | 34 | MU |
| cg14046477 | chr9 | 127531952 | 17 | MU | cg08661469 | chr17 | 72732823 | 34 | N |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg13409170 | chr9 | 127532069 | 17 | MU | cg01944585 | chr17 | 72732912 | 34 | N |
| cg01150641 | chr1 | 976168 | 18 | N | cg23838375 | chr17 | 72732946 | 34 | N |
| cg23625715 | chr1 | 976172 | 18 | MU | cg09852601 | chr17 | 72732955 | 34 | N |
| cg26222311 | chr1 | 976227 | 18 | N | cg10817887 | chr17 | 72732964 | 34 | MU |
| cg22497741 | chr1 | 1475644 | 18 | MU | cg24166450 | chr17 | 74248466 | 34 | MU |
| cg16306898 | chr1 | 1475675 | 18 | MU | cg16472334 | chr17 | 77721757 | 34 | N |
| cg15487867 | chr1 | 1475742 | 18 | N | cg18933840 | chr17 | 77721824 | 34 | MU |
| cg25730685 | chr1 | 2375010 | 18 | MU | cg26141063 | chr18 | 32870200 | 34 | MU |
| cg00532449 | chr1 | 2375420 | 18 | MU | cg27662412 | chr18 | 39535095 | 34 | MU |
| cg05039004 | chr1 | 29586299 | 18 | MU | cg04622103 | chr19 | 1812664 | 34 | N |
| cg04933208 | chr1 | 29586414 | 18 | MU | cg11251319 | chr19 | 1812732 | 34 | MU |
| cg26884027 | chr1 | 29586418 | 18 | MU | cg005230.12 | chr19 | 13264324 | 34 | MU |
| cg21929771 | chr1 | 29586580 | 18 | MU | cg23291280 | chr19 | 31847970 | 34 | N |
| cg04194494 | chr10 | 45914450 | 18 | MU | cg02128244 | chr19 | 31848008 | 34 | N |
| cg21981270 | chr10 | 45914525 | 18 | N | cg21533331 | chr19 | 31848049 | 34 | MU |
| cg16831085 | chr10 | 45914645 | 18 | MU | cg24436715 | chr19 | 31848130 | 34 | N |
| cg10069493 | chr10 | 45914688 | 18 | MU | cg09961571 | chr19 | 39421871 | 34 | MU |
| cg00675569 | chr10 | 45914840 | 18 | MU | cg15779837 | chr19 | 48918116 | 34 | MU |
| cg09806262 | chr10 | 45914949 | 18 | N | cg23444265 | chr19 | 48918205 | 34 | N |
| cg26329178 | chr10 | 100227782 | 18 | MU | cg02184280 | chr19 | 49954649 | 34 | MU |
| cg08994045 | chr11 | 2883912 | 18 | MU | cg24879782 | chr2 | 19561482 | 34 | N |
| cg26985666 | chr11 | 35441088 | 18 | MU | cg06530338 | chr2 | 19561612 | 34 | MU |
| cg08300419 | chr11 | 47208959 | 18 | MU | cg01196788 | chr2 | 19561709 | 34 | N |
| cg25623977 | chr11 | 71163428 | 18 | MU | cg16851771 | chr2 | 42360170 | 34 | MU |
| cg05963690 | chr11 | 71163466 | 18 | N | cg21550141 | chr2 | 102439950 | 34 | MU |
| cg01630362 | chr11 | 71163528 | 18 | MU | cg15680720 | chr2 | 171568412 | 34 | N |
| cg20728496 | chr11 | 71163555 | 18 | N | cg20389472 | chr2 | 171568526 | 34 | MU |
| cg03078269 | chr12 | 54354974 | 18 | N | cg07466807 | chr2 | 173326947 | 34 | MU |
| cg26809635 | chr12 | 54355087 | 18 | MU | cg26112014 | chr2 | 230135789 | 34 | N |
| cg08900101 | chr12 | 114833043 | 18 | MU | cg14165051 | chr2 | 230135879 | 34 | MU |
| cg05769349 | chr12 | 114847043 | 18 | N | cg07472438 | chr2 | 230135929 | 34 | MU |
| cg06911121 | chr12 | 114847164 | 18 | MU | cg20003983 | chr20 | 56725730 | 34 | N |
| cg20381975 | chr13 | 25320750 | 18 | N | cg03387930 | chr20 | 56725833 | 34 | N |
| cg04.759767 | chr13 | 25320824 | 18 | MU | cg22991320 | chr20 | 56725871 | 34 | MU |

| Illumina Probe Name | Chr. | Position | Tissue/Cell | MU/N | Illumina Probe Name | Chr. | Position | Tissu e/Cell Type | MU/N |
|---|---|---|---|---|---|---|---|---|---|
| cg12177793 | chr14 | 24837892 | 18 | MU | cg19155932 | chr20 | 56725873 | 34 | N |
| cg26009486 | chr14 | 24838008 | 18 | N | cg09663111 | chr20 | 56725876 | 34 | N |
| cg21523564 | chr15 | 75251491 | 18 | MU | cg25906442 | chr20 | 56725887 | 34 | MU |
| cg05627522 | chr15 | 75251581 | 18 | N | cg07655948 | chr20 | 56725899 | 34 | N |
| cg20540209 | chr15 | 76633981 | 18 | MU | cg18562935 | chr20 | 56726000 | 34 | N |
| cg08307030 | chr15 | 76634380 | 18 | N | cg12430776 | chr21 | 19191096 | 34 | MU |
| cg10088491 | chr15 | 76634454 | 18 | N | cg21608237 | chr22 | 45148229 | 34 | MU |
| cg14433497 | chr15 | 76634516 | 18 | MU | cg09954385 | chr22 | 45148285 | 34 | N |
| cg26970841 | chr16 | 85932666 | 18 | MU | cg13996698 | chr22 | 45148306 | 34 | N |
| cg22298430 | chr17 | 5404330 | 18 | MU | cg27526665 | chr3 | 24537050 | 34 | N |
| cg27230784 | chr17 | 5404337 | 18 | MU | cg27622506 | chr3 | 24537160 | 34 | MU |
| cg06462347 | chr17 | 5404508 | 18 | N | cg21303011 | chr3 | 24537177 | 34 | N |
| cg18671949 | chr17 | 5404581 | 18 | MU | cg00323305 | chr3 | 24537182 | 34 | N |
| cg20999084 | chr17 | 8124252 | 18 | MU | cg27388911 | chr3 | 24537277 | 34 | N |
| eg19872299 | chr17 | 17686141 | 18 | MU | cg16579438 | chr3 | 24537301 | 34 | N |
| cg20668644 | chr17 | 38347603 | 18 | MU | cg25336332 | chr3 | 24537309 | 34 | N |
| cg19827780 | chr17 | 38347659 | 18 | N | cg06853609 | chr3 | 37218771 | 34 | MU |
| cg00129651 | chr17 | 38347710 | 18 | MU | cg17573813 | chr3 | 61237223 | 34 | MU |
| cg12861034 | chrl7 | 38347816 | 18 | N | cg18153137 | chr3 | 71114771 | 34 | MU |
| cg06266993 | chr17 | 46671131 | 18 | N | cg19477084 | chr3 | 126006762 | 34 | N |
| cg11155697 | chr17 | 46671234 | 18 | N | cg16342439 | chr3 | 126006835 | 34 | N |
| cg02712075 | chr17 | 46671244 | 18 | MU | cg14310703 | chr3 | 126006903 | 34 | MU |
| cg10153335 | chr17 | 46671293 | 18 | N | cg01709619 | chr3 | 129346694 | 34 | MU |
| cg02293936 | chrl7 | 46671298 | 18 | N | cg02568670 | chr3 | 141173927 | 34 | MU |
| cg24626312 | chr17 | 46671332 | 18 | N | cg04401986 | chr4 | 81189927 | 34 | MU |
| cg14126688 | chr17 | 46671497 | 18 | MU | cg16551732 | chr4 | 169799087 | 34 | MU |
| cg01405107 | chr17 | 46671635 | 18 | N | cg03729337 | chr5 | 122430019 | 34 | N |
| cg12698628 | chr17 | 46802740 | 18 | N | cg27087956 | chr5 | 122430153 | 34 | MU |
| cg19057986 | chrl7 | 46802871 | 18 | MU | cg02564291 | chr5 | 122430235 | 34 | N |
| cg09000583 | chr17 | 46802888 | 18 | MU | cg19328475 | chr5 | 122430277 | 34 | N |
| cg08594218 | chr17 | 46803008 | 18 | MU | cg25412017 | chr5 | 175560449 | 34 | MU |
| cg09130077 | chr17 | 46810702 | 18 | MU | cg09533168 | chr5 | 175560568 | 34 | N |
| cg18479972 | chr17 | 46810705 | 18 | N | cg06572420 | chr5 | 175621395 | 34 | MU |
| cg01388649 | chr17 | 46810768 | 18 | N | cg02208657 | chr5 | 175621409 | 34 | MU |

(continued)

| Illumina Probe Name | Chr. | Position | Tissue/Cell | MU/N |
|---|---|---|---|---|
| cg07925900 | chr17 | 74554786 | 18 | MU |
| cg03167953 | chr17 | 74554844 | 18 | N |
| cg06848185 | chr17 | 75368902 | 18 | MU |
| cg19554255 | chr17 | 75369051 | 18 | N |
| cg16779463 | chr17 | 75369055 | 18 | MU |
| cg17300544 | chr17 | 75369091 | 18 | MU |
| cg03804136 | chr17 | 75369219 | 18 | MU |
| cg15044248 | chr17 | 75369224 | 18 | MU |
| cg20275528 | chr17 | 75369484 | 18 | MU |
| cg12783819 | chr17 | 75369657 | 18 | MU |
| cg14517217 | chr17 | 75447478 | 18 | MU |
| cg18104645 | chr17 | 75447504 | 18 | MU |
| cg11991516 | chr17 | 75447785 | 18 | MU |
| cg02966153 | chr19 | 1401767 | 18 | MU |
| cg04260368 | chr19 | 1401775 | 18 | N |
| cg27317433 | chr19 | 1401781 | 18 | MU |
| cg17299615 | chr19 | 13944571 | 18 | MU |
| cg27067425 | chr19 | 15695299 | 18 | MU |
| cg10791911 | chr19 | 18118800 | 18 | N |
| cg08021780 | chr19 | 18118880 | 18 | MU |
| cg23307203 | chr19 | 18499309 | 18 | MU |
| cg19221841 | chr19 | 41197049 | 18 | MU |
| cg19643921 | chr19 | 41197106 | 18 | N |
| cg04312358 | chr19 | 41197166 | 18 | N |
| cg09323727 | chr2 | 176936436 | 18 | MU |
| cg00459623 | chr2 | 176936581 | 18 | MU |
| cg07802350 | chr2 | 176956573 | 18 | MU |
| cg25626427 | chr2 | 176976174 | 18 | MU |
| cg26708100 | chr2 | 176983815 | 18 | MU |
| cg10393811 | chr2 | 176983927 | 18 | N |
| cg14142007 | chr2 | 176987225 | 18 | MU |
| cg09968620 | chr2 | 176987383 | 18 | N |
| cg14991487 | chr2 | 176987404 | 18 | N |
| cg04739647 | chr2 | 176987462 | 18 | MU |

| Illumina Probe Name | Chr. | Position | Tissu e/Cell Type | MU/N |
|---|---|---|---|---|
| cg04263163 | chr5 | 177371702 | 34 | MU |
| cg23172545 | chr6 | 2875602 | 34 | N |
| cg07096305 | chr6 | 2875723 | 34 | MU |
| cg00799826 | chr6 | 30071200 | 34 | MU |
| cg20797270 | chr6 | 30071288 | 34 | N |
| cg13074835 | chr6 | 31515196 | 34 | MU |
| cg10714284 | chr6 | 32908901 | 34 | MU |
| cg16300030 | chr6 | 32908980 | 34 | N |
| cg20392842 | chr6 | 32909003 | 34 | MU |
| ccg18630040 | chr6 | 46702983 | 34 | N |
| cg26705953 | chr6 | 46703123 | 34 | MU |
| cg26953727 | chr6 | 134176231 | 34 | MU |
| cg10308833 | chr6 | 134176248 | 34 | MU |
| cg25228422 | chr7 | 19152335 | 34 | MU |
| cg16168668 | chr7 | 19158566 | 34 | N |
| cg14391419 | chr7 | 19158647 | 34 | MU |
| cg10126205 | chr7 | 19158664 | 34 | MU |
| cg21424940 | chr7 | 19158666 | 34 | MU |
| cg04904385 | chr7 | 19158692 | 34 | N |
| cg14604568 | chr7 | 32110523 | 34 | MU |
| cg15988350 | chr7 | 32110650 | 34 | MU |
| cg03808172 | chr7 | 96339361 | 34 | MU |
| cg26619624 | chr7 | 96339434 | 34 | N |
| cg26269703 | chr7 | 149119681 | 34 | MU |
| cg11382963 | chr8 | 72754469 | 34 | MU |
| cg16531209 | chr8 | 99951696 | 34 | N |
| cg03020554 | chr8 | 99951730 | 34 | MU |
| cg09825146 | chr8 | 125462982 | 34 | MU |
| cg26141173 | chr8 | 125463007 | 34 | N |
| cg11829072 | chr8 | 125463024 | 34 | MU |
| cg25375711 | chr8 | 125463057 | 34 | MU |
| cg26547947 | chr8 | 125463066 | 34 | N |
| cg26372060 | chr9 | 145691254 | 34 | N |
| cg14614793 | chr9 | 111695728 | 34 | MU |

116

(continued)

| Illumina Probe Name | Chr. | Position | Tissue/Cell Type | MU/N |
| --- | --- | --- | --- | --- |
| cg04043571 | chr9 | 127265989 | 34 | MU |

Table 2: Top 100 hypo-methylated sites per tissue/cell type

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg07033722 | chr1 | 40539032 | 1 | MU | cg25663764 | chr22 | 19965534 | 18 | MU |
| cg17481936 | chr1 | 116371802 | 1 | N | cg22808478 | chr22 | 20073393 | 18 | N |
| cg23952578 | chr1 | 116371871 | 1 | MU | cg08706141 | chr22 | 20073403 | 18 | N |
| cg11939496 | chr1 | 160833560 | 1 | MU | cg07797030 | chr22 | 20073477 | 18 | |
| cg08538581 | chr1 | 162500634 | 1 | MU | cg22786486 | chr22 | 20073528 | 18 | MU |
| cg15288326 | chr1 | 200994098 | 1 | MU | cg06964030 | chr22 | 20073636 | 18 | N |
| cg04851465 | chr1 | 212408609 | 1 | MU | cg05347334 | chr4 | 2439397 | 18 | MU |
| cg24250902 | chr1 | 230415547 | 1 | MU | cg26149658 | chr4 | 2439423 | 18 | N |
| cg03144619 | chr1 | 230415668 | 1 | N | cg15689795 | chr4 | 2439478 | 18 | N |
| cg16660547 | chr10 | 114574152 | 1 | MU | cg17924901 | chr4 | 3475233 | 18 | N |
| cg05923857 | chr10 | 114911615 | 1 | MU | cg05673770 | chr4 | 3475328 | 18 | N |
| cg24788483 | chr10 | 114911652 | 1 | MU | cg21333208 | chr4 | 3475372 | 18 | MU |
| cg16636767 | chr11 | 13694647 | 1 | MU | cg14378364 | chr4 | 123591475 | 18 | MU |
| cg00009088 | chr11 | 60930188 | 1 | MU | cg04311686 | chr5 | 1179328 | 18 | MU |
| cg13980609 | chr11 | 66883117 | 1 | MU | cg03887092 | chr6 | 1080885 | 18 | MU |
| cg05463966 | chr11 | 67803951 | 1 | N | cg00197993 | chr6 | 1201056 | 18 | MU |
| cg12070987 | chr11 | 67804055 | 1 | MU | cg09088836 | chr6 | 2510144 | 18 | N |
| cg04664897 | chr11 | 72925852 | 1 | MU | cg02666566 | chr6 | 2510173 | 18 | MU |
| cg15212210 | chr11 | 118986793 | 1 | N | cg17947992 | chr6 | 2510180 | 18 | MU |
| cg22259797 | chr11 | 118986860 | 1 | MU | cg02115397 | chr6 | 32975850 | 18 | N |
| cg06980932 | chr11 | 121301233 | 1 | MU | cg10620866 | chr6 | 32975875 | 18 | N |
| cg08428868 | chr11 | 123323626 | 1 | MU | cg18477188 | chr6 | 32975885 | 18 | MU |
| cg27366072 | chr12 | 12222913 | 1 | MU | cg16673446 | chr6 | 32975925 | 18 | N |
| cg10456459 | chr12 | 22843015 | 1 | MU | cg24696151 | chr6 | 32975986 | 18 | N |
| cg12866960 | chr12 | 66635398 | 1 | MU | cg14144933 | chr6 | 32975994 | 18 | N |
| cg13500029 | chr12 | 75873039 | 1 | MU | cg07294785 | chr6 | 32976018 | 18 | N |
| cg17183174 | chr13 | 76115526 | 1 | MU | cg18015809 | chr6 | 47202254 | 18 | N |
| cg03313271 | chr13 | 113913094 | 1 | MU | cg21552001 | chr6 | 47202394 | 18 | MU |
| cg19502671 | chr13 | 114129004 | 1 | MU | cg17356964 | chr6 | 106582638 | 18 | N |
| cg23514375 | chr13 | 114129039 | 1 | MU | cg25581448 | chr6 | 106582669 | 18 | MU |
| cg01024962 | chr14 | 31389792 | 1 | MU | cg08112248 | chr6 | 169002003 | 18 | MU |
| cg26298914 | chr14 | 68798365 | 1 | MU | cg14806083 | chr6 | 169002120 | 18 | N |
| eg12655112 | chr15 | 42261154 | 1 | MU | cg02152290 | chr7 | 551601 | 18 | MU |
| cg04354689 | chr16 | 2660830 | 1 | MU | cg15879716 | chr7 | 1932272 | 18 | MU |
| cg03571959 | chr16 | 2660876 | 1 | N | cg03653726 | chr7 | 2769253 | 18 | MU |
| cg00504410 | chr16 | 4732262 | 1 | N | cg06779606 | chr7 | 3041079 | 18 | MU |
| cg03963853 | chr16 | 4732369 | 1 | MU | cg20037968 | chr7 | 3041098 | 18 | N |
| cg07248377 | chr16 | 4732406 | 1 | N | cg02751532 | chr7 | 3041110 | 18 | N |
| cg07737292 | chr16 | 56892460 | 1 | MU | cg15319585 | chr7 | 4307392 | 18 | N |
| cg01735503 | chr16 | 69966815 | 1 | N | cg07005020 | chr7 | 4307433 | 18 | MU |
| cg03549146 | chr16 | 69966902 | 1 | MU | cg09404689 | chr7 | 4307490 | 18 | N |
| cg00158530 | chr16 | 69966973 | 1 | N | cg13621882 | chr7 | 5647885 | 18 | N |
| cg08209934 | chr16 | 69966975 | 1 | N | cg20407483 | chr7 | 5647920 | 18 | MU |
| cg03126713 | chr16 | 89299480 | 1 | MU | cg18753341 | chr7 | 157202405 | 18 | MU |
| cg06988336 | chr16 | 89922397 | 1 | MU | cg06286135 | chr7 | 157511479 | 18 | MU |
| cg01739831 | chr16 | 89922539 | 1 | N | cg14584961 | chr7 | 157533065 | 18 | MU |
| cg08036492 | chr17 | 13976536, | 1 | MU | eg25899922 | chr7 | 158934015 | 18 | N |
| cg13093111 | chr17 | 66308577 | 1 | MU | cg09909417 | chr7 | 158934031 | 18 | MU |
| cg20610950 | chr17 | 75096202 | 1 | MU | cg26431815 | chr8 | 668053 | 18 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg11597902 | chr17 | 75096239 | 1 | N | cg16830075 | chr8 | 142151154 | 18 | MU |
| cg11186858 | chr17 | 75096382 | 1 | MU | cg14056656 | chr9 | 132892163 | 18 | MU |
| cg02675920 | chr17 | 78747934 | 1 | N | cg23887776 | chr1 | 2827798 | 19 | N |
| cg11222173 | chr17 | 78748019 | 1 | N | cg15356461 | chr1 | 2827833 | 19 | MU |
| cg11153071 | chr17 | 78748077 | 1 | MU | cg02328660 | chr1 | 3389744 | 19 | MU |
| cg26783127 | chr17 | 79128918 | 1 | MU | cg03890628 | chr1 | 9666902 | 19 | MU |
| cg16732654 | chr17 | 79129022 | 1 | N | cg24425531 | chr1 | 15372951 | 19 | MU |
| cg23334433 | chr17 | 79129051 | 1 | N | cg10108612 | chr1 | 19203999 | 19 | MU |
| cg20429104 | chr18 | 74114570 | 1 | MU | cg00423597 | chr1 | 33235531 | 19 | MU |
| cg12019801 | chr18 | 74114708 | 1 | N | cg09167705 | chr1 | 33235619 | 19 | N |
| cg14976569 | chr19 | 4667105 | 1 | MU | cg15860924 | chr1 | 33612977 | 19 | N |
| cg26538782 | chr19 | 10308540 | 1 | MU | cg20410537 | chr1 | 33612991 | 19 | MU |
| cg25517015 | chr19 | 17584020 | 1 | MU | cg05266497 | chr1 | 230457961 | 19 | N |
| cg10480329 | chr2 | 27011850 | 1 | MU | cg03347632 | chr1 | 230457979 | 19 | MU |
| cg04781916 | chr2 | 48013473 | 1 | MU | cg26619296 | chr10 | 1585286 | 19 | MU |
| cg06373940 | chr2 | 128052778 | 1 | MU | cg08442149 | chr10 | 1767484 | 19 | MU |
| cg14306819 | chr2 | 128052889 | 1 | N | cg20691436 | chr10 | 5567478 | 19 | MU |
| cg15633603 | chr2 | 190399258 | 1 | MU | cg27292389 | chr10 | 6003524 | 19 | MU |
| cg02796279 | chr2 | 208101334 | 1 | MU | cg25580782 | chr10 | 6003621 | 19 | N |
| cg12884009 | chr2 | 219079038 | 1 | MU | cg17019070 | chr10 | 73466639 | 19 | N |
| cg14012546 | chr2 | 233981788 | 1 | N | cg10505163 | chr10 | 73466728 | 19 | MU |
| cg22666015 | chr2 | 233981885 | 1 | MU | cg26993007 | chr10 | 117884933 | 19 | N |
| cg01112020 | chr2 | 239008986 | 1 | N | cg26338723 | chr10 | 117884983 | 19 | MU |
| cg09039163 | chr2 | 239009036 | 1 | N | cg03885119 | chr10 | 117885028 | 19 | N |
| eg15871206 | chr2 | 239009118 | 1 | MU | cg24919753 | chr10 | 121138105 | 19 | N |
| cg11231069 | chr2 | 240225142 | 1 | MU | cg24811125 | chr10 | 121138124 | 19 | MU |
| eg06978145 | chr20 | 30754792 | 1 | MU | cg14491482 | chrl0 | 121138206 | 19 | N |
| cg04217515 | chr21 | 46325853 | 1 | MU | cg23595571 | chr11 | 1313100 | 19 | MU |
| cg11327657 | chr21 | 46388162 | 1 | MU | eg03784994 | chr11 | 1556050 | 19 | MU |
| cg08425796 | chr22 | 50981121 | 1 | MU | cg14342106 | chr11 | 57069646 | 19 | MU |
| cg02244028 | chr3 | 38992200 | 1 | MU | cg09330078 | chr11 | 57069683 | 19 | N |
| eg06585734 | chr3 | 46037942 | 1 | MU | cg06291428 | chr11 | 57559063 | 19 | MU |
| cg03171795 | chr3 | 70882921 | 1 | MU | cg20969529 | chr11 | 65387182 | 19 | N |
| cg19533955 | chr3 | 99790878 | 1 | MU | cg04932205 | chr11 | 65387290 | 19 | MU |
| cg17086773 | chr3 | 112486072 | 1 | MU | cg11383802 | chr11 | 71145904 | 19 | MU |
| cg18990407 | chr3 | 184297380 | 1 | MU | cg09174502 | chr11 | 130079372 | 19 | MU |
| cg15334250 | chr3 | 184297495 | 1 | N | cg13189671 | chr12 | 123750782. | 19 | N |
| cg27214730 | chr3 | 184297522 | 1 | N | cg14790739 | chr12 | 123750827 | 19 | MU |
| cg13403369 | chr3 | 195868640 | 1 | MU | cg25368917 | chr12 | 124788627 | 19 | MU |
| cg15044270 | chr3 | 196065485 | 1 | N | cg01656620 | chr12 | 124788698 | 19 | N |
| cg13314965 | chr3 | 196065506 | 1 | N | cg18672389 | chr12 | 124789036 | 19 | N |
| cg05445326 | chr3 | 196065569 | 1 | MU | cg14273093 | chr12 | 124789096 | 19 | MU |
| cg00828556 | chr3 | 196351986 | 1 | MU | cg06674275 | chr13 | 113430153 | 19 | N |
| cg25771026 | chr3 | 196352027 | 1 | N | cg12572352 | chr13 | 113430266 | 19 | MU |
| cg14786790 | chr4 | 89766654 | 1 | MU | cg26433554 | chr14 | 23315189 | 19 | N |
| cg20107506 | chr4 | 100874826 | 1 | MU | cg14414203 | chr14 | 23315249 | 19 | MU |
| cg11802666 | chr4 | 185345707 | 1 | MU | cg19678259 | chr14 | 23315264 | 19 | N |
| cg17500228 | chr5 | 448790 | 1 | MU | cg20384325 | chr15 | 49268750 | 19 | MU |
| cg22185977 | chr5 | 1518133 | 1 | MU | cg06332339 | chr15 | 73889528 | 19 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg05573654 | chr5 | 1518179 | 1 | N | cg08435157 | chr15 | 73889561 | 19 | N |
| cg20942286 | chr5 | 79366549 | 1 | MU | cg20238681 | chr15 | 94443233 | 19 | MU |
| cg10967114 | chr5 | 150442668 | 1 | MU | cg07701166 | chr15 | 94443237 | 19 | MU |
| cg05141695 | chr6 | 341180 | 1 | MU | cg06349762 | chr16 | 888938 | 19 | MU |
| cg13719228 | chr6 | 341315 | 1 | N | cg01125617 | chr16 | 889011 | 19 | N |
| cg05612654 | chr6 | 2375895 | 1 | MU | cg08333708 | chr16 | 2535757 | 19 | MU |
| cg01300914 | chr6 | 2763797 | 1 | N | cg01447429 | chr16 | 29222265 | 19 | MU |
| cg20017123 | chr6 | 2763919 | 1 | MU | cg04453552 | chr16 | 57394387 | 19 | MU |
| cg18128312 | chr6 | 25463672 | 1 | MU | cg17248540 | chr16 | 69143500 | 19 | N |
| cg18835472 | chr6 | 26546056 | 1 | MU | cg04320956 | chr16 | 69143512 | 19 | MU |
| eg10718056 | chr6 | 28884599 | 1 | MU | cg02751838 | chr16 | 75148330 | 19 | N |
| eg00259404 | chr6 | 28885568 | 1 | MU | cg00004883 | chr16 | 75148460 | 19 | MU |
| cg06545464 | chr6 | 32299649 | 1 | N | cg21289738 | chr17 | 8383742 | 19 | N |
| cg20966357 | chr6 | 32299676 | 1 | MU | cg21871213 | chr17 | 8383800 | 19 | MU |
| cg01026383 | chr6 | 32905012 | 1 | N | cg02059952 | chr17 | 8384595 | 19 | N |
| cg20978247 | chr6 | 32905085 | 1 | MU | cg06557376 | chr17 | 8384607 | 19 | MU |
| cg19268453 | chr6 | 32905114 | 1 | N | cg10533978 | chr17 | 40842804 | 19 | MU |
| cg01374870 | chr6 | 32905127 | 1 | N | cg24136288 | chr17 | 43183150 | 19 | MU |
| cg04903089 | chr6 | 32905190 | 1 | N | cg02314166 | chr17 | 60767131 | 19 | MU |
| cg07918933 | chr6 | 72051803 | 1 | MU | cg09237133 | chr17 | 80278914 | 19 | MU |
| cg06297318 | chr6 | 127667691 | 1 | MU | cg09340279 | chr17 | 80278947 | 19 | N |
| cg04531756 | chr6 | 135516912 | 1 | N | cg02775417 | chr19 | 643457 | 19 | MU |
| cg02127509 | chr6 | 135516936 | 1 | N | cg12062672 | chr19 | 643555 | 19 | N |
| cg04739200 | chr6 | 135517046 | 1 | MU | cg07962882 | chr19 | 1117289 | 19 | MU |
| cg00235484 | chr6 | 136825415 | 1 | MU | cg09432775 | chr19 | 1117453 | 19 | MU |
| cg12458039 | chr7 | 210075 | 1 | MU | cg05919982 | chr19 | 1117571 | 19 | N |
| cg03663120 | chr7 | 2284600 | 1 | MU | cg22018329 | chr19 | 3116716 | 19 | MU |
| cg05861879 | chr7 | 2284723 | 1 | N | cg18030386 | chr19 | 4944160 | 19 | MU |
| cg15704521 | chr7 | 2773877 | 1 | MU | cg21993464 | chr19 | 14041305 | 19 | MU |
| cg03310874 | chr7 | 4850260 | 1 | MU | cg14697425 | chr19 | 14042840 | 19 | MU |
| cg21932542 | chr7 | 4850345 | 1 | N | cg26008272 | chr19 | 45574917 | 19 | N |
| cg22528270 | chr7 | 151505116 | 1 | MU | cg15732530 | chr19 | 45575023 | 19 | MU |
| cg15310871 | chr8 | 20077936 | 1 | MU | cg24030434 | chr19 | 45575134 | 19 | N |
| cg11699257 | chr8 | 28634455 | 1 | MU | cg23633937 | chr19 | 45575.142 | 19 | N |
| cg07023317 | chr8 | 28961315 | 1 | N | cg08531052 | chr19 | 49633795 | 19 | N |
| cg25596405 | chr8 | 28961333 | 1 | MU | cg11881562 | chr19 | 49633902 | 19 | MU |
| cg09322573 | chr8 | 28961356 | 1 | MU | cg23226510 | chr19 | 56042469 | 19 | N |
| cg08444833 | chr8 | 141574162 | 1 | N | cg00844791 | chr19 | 56042579 | 19 | MU |
| cg07466463 | chr8 | 141574207 | 1 | MU | cg26858540 | chr19 | 56603322 | 19 | MU |
| cg06517984 | chr8 | 143407646 | 1 | MU | cg14385008 | chr2 | 3469154 | 19 | MU |
| cg17117981 | chr8 | 144410972 | 1 | MU | cg06529996 | chr2 | 3469212 | 19 | N |
| cg13931640 | chr9 | 137277819 | 1 | MU | cg20210057 | chr2 | 98351150 | 19 | N |
| cg23087108 | chr1 | 1120617 | 2 | MU | cg21585640 | chr2 | 98351171 | 19 | MU |
| cg24250902 | chr1 | 230415547 | 2 | N | cg25138365 | chr2 | 98351315 | 19 | N |
| cg03144619 | chr1 | 230415668 | 2 | MU | cg04707348 | chr2 | 98351548 | 19 | MU |
| cg07629149 | chr10 | 334731 | 2 | N | cg08042027 | chr2 | 240040089 | 19 | MU |
| cg23844018 | chr10 | 334773 | 2 | MU | cg07215298 | chr2 | 240040219 | 19 | MU |
| cg09848445 | chr10 | 43601862 | 2 | MU | cg25921544 | chr2 | 240040290 | 19 | N |
| cg14725719 | chr10 | 43601916 | 2 | N | cg21478921 | chr2 | 242617935 | 19 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg11199827 | chr10 | 49881056 | 2 | MU | cg03301433 | chr2 | 242617946 | 19 | N |
| cg03969079 | chr10 | 63759909 | 2 | MU | cg03877418 | chr2 | 242757444 | 19 | MU |
| cg25131632 | chr10 | 94549040 | 2 | MU | cg25663764 | chr22 | 19965534 | 19 | MU |
| cg17995557 | chr10 | 126289971 | 2 | MU | cg27447746 | chr22 | 23801478 | 19 | MU |
| cg24517899 | chr10 | 126290093 | 2 | N | cg27620176 | chr22 | 47054067 | 19 | MU |
| cg07652788 | chr10 | 133598589 | 2 | MU | cg26058289 | chr22 | 47054148 | 19 | N |
| cg18961589 | chr10 | 133598669 | 2 | N | cg01908177 | chr3 | 113005203 | 19 | MU |
| cg22697239 | chr11 | 44626708 | 2 | MU | cg20384732 | chr3 | 195601375 | 19 | MU |
| cg01199327 | chr11 | 44626750 | 2 | MU | cg01239666 | chr3 | 195601381 | 19 | MU |
| cg18476690 | chr11 | 61125255 | 2 | N | cg06063209 | chr3 | 195602274. | 19 | MU |
| cg14331899 | chr11 | 61125360 | 2 | MU | cg01425021 | chr4 | 1995036 | 19 | MU |
| cg00464927 | chr11 | 68139121 | 2 | MU | cg26401028 | chr4 | 3516534 | 19 | MU |
| eg13738327 | chr11 | 68139233 | 2 | MU | cg04857395 | chr4 | 3516637 | 19 | N |
| cg08648997 | chr11 | 68139350 | 2 | N | cg031-35535 | chr4 | 141564017, | 19 | MU |
| cg17382048 | chr11 | 118754386 | 2 | N | cg11639612 | chr5 | 139839 | 19 | N |
| cg16235962 | chr11 | 118754507 | 2 | N' | cg20653695 | chr5 | 139859 | 19 | N |
| cg04625873 | chr11 | 118754530 | 2 | N | cg12645891 | chr5 | 139912 | 19 | MU |
| cg25087423 | chr11 | 118754535 | 2 | MU | cg03621279 | chr5 | 140009 | 19 | N |
| cg26164712 | chr11 | 118754565 | 2 | N | cg24923860 | chr5 | 149765871 | 19 | MU |
| cg16280667 | chr11 | 118754593 | 2 | N. | cg16929427 | chr6 | 1578078 | 19 | MU |
| cg05921138 | chr11 | 121440849 | 2 | N | cg27089559 | chr6 | 1578093 | 19 | N |
| cg17232476 | chr11 | 121440880 | 2 | MU | cg00134190 | chr6 | 1578113 | 19 | N |
| cg16931707 | chr12 | 42873202 | 2 | MU | cg16940803 | chr6 | 7441582 | 19 | MU |
| cg25683989 | chr12 | 111124215 | 2 | MU | cg13514037 | chr6 | 31600533 | 19 | N |
| eg17955329 | chr12 | 113528764 | 2 | N | cg03134653 | chr6 | 31600558 | 19 | N |
| cg19631815 | chr12 | 113528791 | 2 | MU | cg00887255 | chr6 | 31600560 | 19 | N |
| cg26426437 | chr12 | 121686502 | 2 | MU | cg19673418 | chr6 | 31600630 | 19 | N |
| cg16085171 | chr12 | 123569118 | 2 | MU | cg18712755 | chr6 | 31600642 | 19 | MU |
| cg04356090 | chr12 | 129280133 | 2 | MU | cg04960128 | chr6 | 31600695 | 19 | N |
| cg05934015 | chr12 | 129280265 | 2 | MU | cg01282751 | chr6 | 31600734 | 19 | N |
| cg16782719 | chr12 | 129280334 | 2 | N | cg21521230 | chr6 | 33288635 | 19 | N |
| cg02742418 | chr12 | 129280388 | 2 | MU | cg04399147 | chr6 | 33288656 | 19 | N |
| cg24102222 | chr12 | 129280449 | 2 | N | cg05147195 | chr6 | 33288734 | 19 | N |
| cg14622549 | chr12 | 132549292 | 2 | MU | cg14499959 | chr6 | 33288773 | 19 | MU |
| eg21703988 | chr12 | 132549404 | 2 | N | cg21350498 | chr6 | 33288785 | 19 | N |
| cg19172447 | chr12 | 132551483 | 2 | MU | cg09864211 | chr6 | 33288800 | 19 | N |
| cg24980032 | chr12 | 132551592 | 2 | N | cg15070677 | chr6 | 33288804 | 19 | MU |
| cg23755933 | chr12 | 133349383 | 2 | MU | cg14523639 | chr6 | 33288839 | 19 | N |
| cg19590591 | chr12 | 133349418 | 2 | MU | cg18704865 | chr6 | 33288851 | 19 | N |
| cg04828493 | chr13 | 111328783 | 2 | MU | cg07098566 | chr6 | 33288891 | 19 | MU |
| cg26053795 | chr14 | 103373907 | 2 | MU | cg03243551 | chr6 | 33288941 | 19 | N |
| cg15861806 | chr14 | 103374045 | 2 | N | cg13059373 | chr6 | 33288944 | 19 | N |
| eg13164814 | chr14 | 103468473 | 2 | N | cg14199833 | chr6 | 33288964 | 19 | MU |
| cg20043258 | chr14 | 103468582 | 2 | MU | cg00117005 | chr6 | 33288995 | 19 | N |
| cg20908276 | chr14 | 106134542 | 2 | MU | cg09883659 | chr6 | 164520746 | 19 | N |
| cg14936008 | chr14 | 107260014 | 2 | MU | cg25862072 | chr6 | 164520845 | 19 | MU |
| cg01666796 | chr15 | 70364327 | 2 | MU | cg10365469 | chr6 | 168417097 | 19 | MU |
| cg12349571 | chr15 | 70364359 | 2 | MU | cg19252565 | chr6 | 168417126 | 19 | N |
| cg00181849 | chr16 | 920856 | 2 | MU | cg11387916 | chr6 | 169273332 | 19 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg27634195 | chr16 | 1440421 | 2 | N | cg05740106 | chr6 | 169273346 | 19 | MU |
| cg09717923 | chr16 | 1440498 | 2 | MU | cg20876525 | chr6 | 169273382 | 19 | N |
| cg04696391 | chr16 | 3021794 | 2 | MU | cg11792186 | chr7 | 2083105 | 19 | N |
| cg06323049 | chr16 | 28943094 | 2 | N | cg18483265 | chr7 | 2083181 | 19 | N |
| cg27565966 | chr16 | 28943198 | 2 | MU | cg15914316 | chr7 | 2083197 | 19 | MU |
| cg05433111 | chr16 | 28943232 | 2 | N | cg02527886 | chr7 | 2968316 | 19 | MU |
| cg01758575 | chr16 | 28943288 | 2 | N | cg10939949 | chr7 | 2968406 | 19 | N |
| cg00709979 | chr16 | 85866348 | 2 | MU | cg24506706 | chr7 | 4169485 | 19 | N |
| cg09201499 | chr16 | 88838113 | 2 | MU | cg08250787 | chr7 | 4169604 | 19 | MU |
| cg09437994 | chr16 | 89035147 | 2 | MU | cg13818127 | chr7 | 4169630 | 19 | MU |
| cg03797660 | chr16 | 89035228 | 2 | N | cg07673838 | chr7 | 416.9752 | 19 | N |
| cg06800849 | chr16 | 89180587 | 2 | MU | cg21080473 | chr7 | 50560524 | 19 | MU |
| cg06255609 | chr17 | 3493563 | 2 | N | cg27394114 | chr7 | 157261038 | 19 | N |
| cg02212339 | chr17 | 3493666 | 2 | MU | cg06518327 | chr7 | 157261163 | 19 | MU |
| cg17441377 | chr17 | 3906640 | 2 | MU | cg16708264 | chr7 | 157336976 | 19 | MU |
| cg01798813 | chr17 | 3906674 | 2 | N | cg13323391 | chr7 | 157579745 | 19 | MU |
| cg01884715 | chr17 | 73316454 | 2 | N | cg18064706 | chr7 | 157646656 | 19 | MU |
| cg06943385 | chr17 | 73316483 | 2 | N | cg14628604 | chr7 | 157646701 | 19 | N |
| cg03717570 | chr17 | 73316496 | 2 | MU | cg17212065 | chr8 | 47057471 | 19 | N |
| cg00900735 | chr17 | 73316505 | 2 | MU | cg15247169 | chr8 | 47057504 | 19 | MU |
| cg07044414 | chr17 | 73316622 | 2 | N | cg22026150 | chr8 | 50653870 | 19 | N |
| cg06091647 | chr17 | 78899392 | 2 | MU | cg04418647 | chr8 | 50653901 | 19 | MU |
| cg14467066 | chr17 | 79419739 | 2 | N | cg01716084 | chr8 | 142367700 | 19 | N |
| cg11699517 | chr17 | 79419796 | 2 | MU | cg22979083 | chr8 | 142367795 | 19 | MU |
| cg21373806 | chr17 | 79419834 | 2 | MU | cg09174681 | chr8 | 144403126 | 19 | MU |
| cg18050634 | chr17 | 79420145 | 2 | MU | cg26185884 | chr8 | 144568432 | 19 | MU |
| cg21284015 | chr17 | 79420246 | 2 | N | cg23170280 | chr8 | 144568483 | 19 | N |
| cg24550880 | chr17 | 79420279 | 2 | N | cg22032812 | chr8 | 144869896 | 19 | N |
| cg23881099 | chr17 | 79924020 | 2 | MU | cg26243623 | chr8 | 144869985 | 19 | MU |
| cg08817507 | chr17 | 79924068 | 2 | MU | cg09541794 | chr8 | 144872852 | 19 | MU |
| cg08732805 | chr17 | 79924176 | 2 | N | cg08214693 | chr8 | 144885540 | 19 | MU |
| cg27579854 | chr17 | 80818992 | 2 | N | cg10827460 | chr1 | 879375 | 20 | N |
| cg11872040 | chr17 | 80819028 | 2 | MU | cg15742605 | chr1 | 879383 | 20 | MU |
| cg08811443 | chr17 | 80819108 | 2 | N | cg09756115 | chr1 | 879415 | 20 | N |
| cg14579184 | chr17 | 80873365 | 2 | MU | cg21299491 | chr1 | 3057366 | 20 | MU |
| cg19755815 | chr17 | 80873572 | 2 | N | cg21913897 | chr1 | 4794072 | 20 | MU |
| cg02092514 | chr17 | 80873656 | 2 | MU | cg27181471 | chr1 | 4794111 | 20 | N |
| cg18092363 | chr18 | 77202678 | 2 | MU | cg04551619 | chr1 | 10692799 | 20 | MU |
| cg26615224 | chr19 | 1621124 | 2 | MU | cg15447913 | chr1 | 23162550 | 20 | MU |
| cg15533809 | chr19 | 2076775 | 2 | MU | cg26144567 | chr1 | 29446085 | 20 | MU |
| cg16743273 | chr19 | 2076833 | 2 | N | cg26827702 | chr1 | 53238265 | 20 | MU |
| cg24299136 | chr19 | 2511707 | 2 | MU | cg09234454 | chr1 | 94560697 | 20 | MU |
| cg00827581 | chr19 | 10434088 | 2 | MU | cg18592195 | chr1 | 227963171 | 20 | MU |
| cg00041047 | chr19 | 49828629 | 2 | MU | cg06768213 | chr1 | 227963296 | 20 | N |
| cg01453814 | chr19 | 49828641 | 2 | MU | cg26251854 | chr1 | 229252074 | 20 | MU |
| cg24188017 | chr19 | 49828658 | 2 | N | cg18537476 | chr1 | 229252112 | 20 | MU |
| cg06237487 | chr19 | 49828667 | 2 | N | cg01117269 | chr10 | 416315 | 20 | MU |
| cg21843262 | chr2 | 44513757 | 2 | MU | cg26873457 | chr10 | 729204 | 20 | MU |
| eg24724513 | chr2 | 44513905 | 2 | N | cg16936421 | chr10 | 729226 | 20 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg10854528 | chr2 | 54855934 | 2 | MU | cg12639192 | chr10 | 1166782 | 20 | N |
| cg19846040 | chr2 | 55015049 | 2 | MU | cg22747480 | chr10 | 1166867 | 20 | MU |
| cg07558704 | chr2 | 73286489 | 2 | MU | cg00779313 | chr10 | 1166994 | 20 | N |
| cg07015784 | chr2 | 219257760 | 2 | MU | cg18944640 | chr10 | 1517083 | 20 | MU |
| cg01591579 | chr2 | 234111145 | 2 | MU | cg08227227 | chr10 | 1517151 | 20 | N |
| cg09799039 | chr2 | 240213875 | 2 | MU | cg05630272 | chr10 | 1517218 | 20 | N |
| cg07835236 | chr20 | 62549301 | 2 | N | cg17575314 | chr10 | 71142526 | 20 | MU |
| eg10842070 | chr20 | 62549359 | 2 | MU | cg15941413 | chr10 | 103420568 | 20 | MU |
| cg05898333 | chr20 | 62549385 | 2 | N | cg07249433 | chr10 | 134420183 | 20 | N |
| cg00791190 | chr22 | 50223573 | 2 | MU | cg11740348 | chr10 | 134420208 | 20 | MU |
| cg07680505 | chr22 | 50223663 | 2 | N | cg27437721 | chr10 | 134420300 | 20 | N |
| cg23683962 | chr3 | 24549503 | 2 | MU | cg12587618 | chr11 | 1979879 | 20 | MU |
| cg15035590 | chr3 | 66437456 | 2 | MU | cg21592241 | chr11 | 2735126 | 20 | MU |
| cg11160673 | chr3 | 107240475 | 2 | MU | cg24769295 | chr11 | 63676485 | 20 | N |
| cg05480110 | chr3 | 195534854 | 2 | MU | cg06982473 | chr11 | 63676611 | 20 | MU |
| cg03653856 | chr3 | 197500859 | 2 | MU | cg09034795 | chr11 | 63676760 | 20 | N |
| cg03732753 | chr4 | 1834583 | 2 | N | cg04815835 | chr11 | 64522197 | 20 | MU |
| cg17574471 | chr4 | 1834678 | 2 | MU | cg12071131 | chr11 | 64522266 | 20 | N |
| cg27024271 | chr4 | 39447968 | 2 | N | cg16516712 | chr11 | 70557445 | 20 | MU |
| cg26800884 | chr4 | 39448008 | 2 | MU | cg23055735 | chr11 | 70557464 | 20 | N |
| cg23730277 | chr4 | 102734752 | 2 | MU | cg01151678 | chr11 | 100473252 | 20 | MU |
| cg00762029 | chr4 | 185317438 | 2 | MU | cg11609668 | chr11 | 117075078 | 20 | MU |
| cg21964798 | chr5 | 1503259 | 2 | MU | cg07825495 | chr12 | 52761098 | 20 | N |
| cg12670963 | chr5 | 1511422 | 2 | N | cg06132342 | chr12 | 52761153 | 20 | N |
| cg11576486 | chr5 | 1511473 | 2 | MU | cg12090001 | chr12 | 52761221 | 20 | MU |
| cg16256592 | chr5 | 175664221 | 2 | N | cg16188537 | chr12 | 109704190 | 20 | MU |
| cg02027263 | chr5 | 175664224 | 2 | MU | cg11724984 | chr12 | 121890864 | 20 | N |
| cg03680932 | chr6 | 16306683 | 2 | MU | cg02827029 | chr12 | 121890907 | 20 | MU |
| cg26088561 | chr6 | 30619080 | 2 | N | cg10627737 | chr13 | 111065189 | 20 | MU |
| cg18048542 | chr6 | 30619113 | 2 | N | cg17444849 | chr13 | 113498069 | 20 | N |
| cg01676996 | chr6 | 30619167 | 2 | N | cg13858127 | chr13 | 113498128 | 20 | MU |
| cg01152616 | chr6 | 30619173 | 2 | N | cg15829092 | chr13 | 113498188 | 20 | N |
| cg13584784 | chr6 | 30619190 | 2 | MU | cg22571393 | chr13 | 113498308 | 20 | N |
| cg05143092 | chr6 | 30619209 | 2 | N | cg11840467 | chr13 | 113498414 | 20 | N |
| cg19849557 | chr6 | 30619242 | 2 | MU | cg01233786 | chr13 | 113498450 | 20 | MU |
| cg18067520 | chr6 | 107114594 | 2 | MU | cg21561701 | chr13 | 114193150 | 20 | N |
| cg16079645 | chr7 | 1039875 | 2 | N | cg03479204 | chr13 | 114193155 | 20 | MU |
| cg21248060 | chr7 | 1039957 | 2 | MU | cg12939777 | chr13 | 114203831 | 20 | MU |
| cg27205928 | chr7 | 1062652 | 2 | MU | cg00822277 | chr13 | 114203903 | 20 | N |
| cg16492851 | chr7 | 1062681 | 2 | N | cg01383440 | chr13 | 114203974 | 20 | N |
| cg04682977 | chr7 | 1062711 | 2 | N | cg01515732 | chr14 | 76012927 | 20 | MU |
| cg25315312 | chr7 | 2020528 | 2 | MU | cg13738571 | chr14 | 76012941 | 20 | N |
| cg20943353 | chr7 | 2020583 | 2 | N | cg20640374 | chr15 | 31355304 | 20 | MU |
| cg11020333 | chr7 | 2861855 | 2 | MU | cg24957518 | chr15 | 31355362 | 20 | N |
| cg16586807 | chr7 | 2948081 | 2 | MU | cg12241333 | chr15 | 31355430 | 20 | N |
| cg17768768 | chr7 | 2948123 | 2 | MU | cg06328338 | chr16 | 1511419 | 20 | MU |
| cg06567596 | chr7 | 97832830 | 2 | MU | cg05258294 | chr16 | 2049878 | 20 | MU |
| cg13659700 | chr7 | 97832841 | 2 | N | cg26923629 | chr16 | 4799203 | 20 | MU |
| cg07006818 | chr7 | 97832945 | 2 | N | cg00535785 | chr16 | 49766785 | 20 | MU |

(continued)

| Illumina Probe Name | Chr. | Position | Tissue /Cell Type | MU/ N | Illumina Probe Name | Chr. | Position | Tissue /Cell Type | MU/N |
|---|---|---|---|---|---|---|---|---|---|
| cg02368706 | chr8 | 1892271 | 2 | MU | cg02227036 | chr16 | 50425329 | 20 | N |
| cg04909834 | chr8 | 1892416 | 2 | N | cg01986767 | chr16 | 50425351 | 20 | MU |
| cg04838847 | chr8 | 110587155 | 2 | MU | cg05922453 | chr16 | 50425414 | 20 | N |
| cg05720871 | chr8 | 110587264 | 2 | N | cg27239168 | chr16 | 58192136 | 20 | N |
| cg27224718 | chr8 | 135614730 | 2 | MU | cg27238852 | chr16 | 58192174 | 20 | MU |
| cg21672855 | chr8 | 135614777 | 2 | N | cg02501155 | chr16 | 79263473 | 20 | MU |
| cg14412639 | chr9 | 34098451 | 2 | MU | cg10221896 | chr16 | 79263595 | 20 | N |
| cg14060402 | chr9 | 37141146 | 2 | MU | cg04510420 | chr16 | 88102758 | 20 | MU |
| cg09276842 | chr1 | 1155026 | 3 | MU | cg27627876 | chr16 | 88102820 | 20 | N |
| cg06532546 | chr1 | 2886654 | 3 | MU | cg06656553 | chr16 | 89960601 | 20 | MU |
| cg22432982 | chr1 | 2886676 | 3 | N | cg25495024 | chr17 | 9189607 | 20 | MU |
| cg01977798 | chr1 | 2886792 | 3 | N | cg27198013 | chr17 | 17714293 | 20 | MU |
| cg06201717 | chr1 | 2949633 | 3 | N | cg09015921 | chr17 | 17714347 | 20 | MU |
| cg24150662 | chr1 | 2949673 | 3 | MU | cg22946219 | chr17 | 17714414 | 20 | N |
| cg11947782 | chr1 | 9775985 | 3 | MU | cg17876831 | chr17 | 18918410 | 20 | MU |
| cg01943221 | chr1 | 9776133 | 3 | N | cg22671072 | chr17 | 18918466 | 20 | N |
| cg07970040 | chr1 | 9789165 | 3 | MU | cg24209115 | chr17 | 18918472 | 20 | N |
| cg04340595 | chr1 | 9789174 | 3 | N | cg15742818 | chr17 | 25913455 | 20 | MU |
| cg01768001 | chr1 | 24831147 | 3 | MU | cg23665668 | chr17 | 38245507 | 20 | MU |
| cg17820878 | chr1 | 27440463 | 3 | MU | cg23526824 | chr17 | 38245542 | 20 | N |
| cg13300580 | chr1 | 27440539 | 3 | N | cg10858898 | chr17 | 38245607 | 20 | N |
| cg12297231 | chr1 | 31531229 | 3 | MU | cg11316510 | chr17 | 38510570 | 20 | MU |
| cg 13909826 | chr1 | 109400731 | 3 | MU | cg12190841 | chr17 | 38510610 | 20 | N |
| cg03016153 | chr1 | 117296954 | 3 | MU | cg12438413 | chr17 | 38510643 | 20 | N |
| cg16374333 | chr1 | 157103641 | 3 | MU | cg00439089 | chr17 | 48263150 | 20 | N |
| cg09032544 | chr1 | 167487295 | 3 | MU | cg16514513 | chr17 | 48263159 | 20 | N |
| cg05799811 | chr1 | 167487396 | 3 | N | cg11615029 | chr17 | 48263189 | 20 | MU |
| cg07786657 | chr1 | 167487633 | 3 | MU | cg11993636 | chr17 | 48263198 | 20 | N |
| cg09554443 | chr1 | 167487762 | 3 | N | cg25735490 | chr17 | 48263204 | 20 | MU |
| cg15009294 | chr1 | 214813712 | 3 | MU | cg01884715 | chr17 | 73316454 | 20 | N |
| cg04640972 | chr10 | 8373522 | 3 | N | cg06943385 | chr17 | 73316483 | 20 | N |
| cg09234252 | chr10 | 8373659 | 3 | MU | cg03717570 | chr17 | 73316496 | 20 | MU |
| cg00929860 | chr10 | 73846448 | 3 | MU | cg00900735 | chr17 | 73316505 | 20 | MU |
| cg17444479 | chr10 | 134429869 | 3 | MU | cg07044414 | chr17 | 73316622 | 20 | N |
| cg20651018 | chr11 | 3035856 | 3 | MU | cg15694704 | chr17 | 78894364 | 20 | N |
| cg24068972 | chr11 | 60833459 | 3 | N | cg06096901 | chr17 | 78894393 | 20 | N |
| cg02661764 | chr11 | 60833531 | 3 | MU | cg21818807 | chr17 | 78894477 | 20 | MU |
| cg01525879 | chr11 | 67207498 | 3 | MU | cg21879029 | chr17 | 78935175 | 20 | N |
| eg01252023 | chr11 | 67207574 | 3 | N. | cg09491897 | chr17 | 78935269 | 20 | N |
| cg02222982 | chr11 | 67207599 | 3 | N | cg07964113 | chr17 | 78935289 | 20 | MU |
| cg24612198 | chr11 | 118175631 | 3 | MU | cg09361653 | chr17 | 78936292 | 20 | N |
| cg06164961 | chr11 | 118175675 | 3 | N | cg17408291 | chr17 | 78936372 | 20 | MU |
| cg07728874 | chr11 | 118213272 | 3 | N | cg02775417 | chr19 | 643457 | 20 | MU |
| cg24841244 | chr11 | 118213330 | 3 | MU | cg12062672 | chr19 | 643555 | 20 | N |
| cg03074244 | chr11 | 118213414 | 3 | N | cg19222405 | chr19 | 1826867 | 20 | MU |
| cg03254928 | chr11 | 118214810 | 3 | N | cg03924776 | chr19 | 1826924 | 20 | N |
| cg13750061 | chr11 | 118214845 | 3 | MU | cg04584009 | chr19 | 1826957 | 20 | N |
| cg07545925 | chr11 | 118214927 | 3 | N | cg00473257 | chr19 | 3964734 | 20 | N |
| cg18070458 | chr11 | 121319927 | 3 | MU | cg06647026 | chr19 | 3964837 | 20 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg12051710 | chr12 | 2989070 | 3 | MU | cg03110167 | chr19 | 3964912 | 20 | N |
| cg01874152 | chr12 | 55375859 | 3 | MU | cg03358468 | chr19 | 5060985 | 20 | N |
| cg08338281 | chr12 | 56322667 | 3 | MU | cg22079102 | chr19 | 5061085 | 20 | MU |
| cg04957307 | chr12 | 92180808 | 3 | MU | cg00513611 | chr19 | 5061205 | 20 | N |
| cg08059719 | chr12 | 122444450 | 3 | N | cg17702388 | chr19 | 16466480 | 20 | N |
| cg14480046 | chr12 | 122444580 | 3 | MU | cg04738220 | chr19 | 16466617 | 20 | MU |
| cg04716447 | chr12 | 122444621 | 3 | N | cg11013793 | chr19 | 16466636 | 20 | N |
| cg09088496 | chr13 | 24825973 . | 3 | MU | cg22182287 | chr2 | 3452347 | 20 | N |
| cg21565496 | chr13 | 40762150 | 3 | MU | cg00548824 | chr2 | 3452407 | 20 | MU |
| cg21845390 | chr13 | 40762260 | 3 | N | cg17738010 | chr2 | 3452437 | 20 | N |
| cg23357533 | chr13 | 42044673 | 3 | MU | cg06360976 | chr2 | 3452543 | 20 | N |
| cg12259024 | chr13 | 114776334 | 3 | N | cg11642891 | chr2 | 3452563 | 20 | MU |
| cg03641640 | chr13 | 114776416 | 3 | MU | cg15807143 | chr2 | 3452624 | 20 | N |
| cg13496119 | chr14 | 98341006 | 3 | MU | cg16707227 | chr2 | 19157847 | 20 | MU |
| cg07015803 | chr14 | 99655593 | 3 | MU | cg26160153 | chr2 | 27373014 | 20 | N |
| cg16452866 | chr14 | 99655676 | 3 | MU | cg24874524 | chr2 | 27373088 | 20 | N |
| cg13619891 | chr14 | 106657452 | 3 | MU | cg14949065 | chr2 | 27373128 | 20 | MU |
| cg24008435 | chr15 | 101137976 | 3 | MU | cg02930665 | chr2 | 27373213 | 20 | N |
| cg01994902 | chr16 | 1576069 | 3 | N | cg17032858 | chr2 | 234164131 | 20 | MU |
| cg27316811 | chr16 | 1576146 | 3 | MU | cg16326123 | chr2 | 240015886 | 20 | MU |
| cg00459119 | chr16 | 12172834 | 3 | N | cg25446604 | chr2 | 242874912 | 20 | MU |
| cg01366941 | chr16 | 12172962 | 3 | MU | cg16016888 | chr2 | 242874987 | 20 | N |
| cg01083549 | chr16 | 89168371 | 3 | MU | cg00364339 | chr3 | 126440043 | 20 | MU |
| cg02098565 | chr16 | 89168508 | 3 | N | cg10175203 | chr3 | 128143634 | 20 | MU |
| cg19001909 | chr17 | 26205940 | 3 | MU | cg00893348 | chr3 | 139353025 | 20 | MU |
| cg08625239 | chr17 | 33572700 | 3 | MU | cg15093769 | chr3 | 139353058 | 20 | MU |
| cg25673720 | chr17 | 74188601 | 3 | MU | cg21896720 | chr4 | 4673378 | 20 | MU |
| cg04945945 | chr17 | 76129402 | 3 | N | cg09757859 | chr4 | 119948505 | 20 | N |
| cg05637296 | chr17 | 76129475 | 3 | N | cg13042918 | chr4 | 119948542 | 20 | MU |
| cg26003388 | chr17 | 76129533 | 3 | MU | cg08000459 | chr4 | 187987206 | 20 | MU |
| cg04162316 | chr17 | 78786355 | 3 | MU | cg12515771 | chr4 | 187987293 | 20 | N |
| cg11949518 | chr17 | 78912765 | 3 | MU | cg07321730 | chr5 | 179302907 | 20 | MU |
| cg02167713 | chr17 | 79630115 | 3 | MU | cg26555052 | chr5 | 180028767 | 20 | N |
| cg03388043 | chr17 | 80084554 | 3 | MU | cg07795168 | chr5 | 180028818 | 20 | MU |
| cg10505658 | chr17 | 80084571 | 3 | N | cg19816983 | chr6 | 31937343 | 20 | N |
| cg20504474 | chr17 | 80261086 | 3 | MU | cg10674805 | chr6 | 31937437 | 20 | MU |
| cg26282887 | chr17 | 80261212 | 3 | N | cg05909390 | chr6 | 31937454 | 20 | N |
| eg10730425 | chr19 | 1035093 | 3 | MU | cg20480259 | chr6 | 31937462 | 20 | N |
| eg02327522 | chr19 | 14633030 | 3 | MU | cg09230350 | chr6 | 31937582 | 20 | N |
| cg11236515 | chr2 | 74213762 | 3 | MU | cg13809627 | chr6 | 33228341 | 20 | MU |
| cg00052964 | chr2 | 85135823 | 3 | MU | cg20528813 | chr6 | 33228355 | 20 | N |
| cg01154505 | chr2 | 112940409 | 3 | MU | cg26738024 | chr6 | 33228461 | 20 | N |
| cg07930752 | chr2 | 204569955 | 3 | N | cg08907436 | chr6 | 152125965 | 20 | N |
| cg13651908 | chr2 | 204570033 | 3 | N | cg07619683 | chr6 | 152126080 | 20 | MU |
| cg10240150 | chr2 | 204570Q67 | 3 | MU | cg07189962 | chr6 | 152126092 | 20 | N |
| cg23366234 | chr21 | 45713704 | 3 | N | cg17267654 | chr6 | 170411557 | 20 | N |
| cg26472802 | chr21 | 45713719 | 3 | MU | cg04452437 | chr6 | 170411595 | 20 | MU |
| cg15722603 | chr22 | 30639887 | 3 | N | cg10224867 | chr6 | 170411613 | 20 | N |
| cg23635663 | chr22 | 30639979 | 3 | MU | cg26814635 | chr7 | 883024 | 20 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg23825480 | chr22 | 31336785 | 3 | MU | cg11356302 | chr7 | 883114 | 20 | N |
| cg05949181 | chr22 | 39154591 | 3 | MU | cg18641463 | chr7 | 2144579 | 20 | MU |
| cg14150023 | chr22 | 40720631 | 3 | MU | cg08625803 | chr7 | 4807504 | 20 | MU |
| cg16093065 | chr22 | 40720633 | 3 | N | cg06947694 | chr7 | 5389271 | 20 | MU |
| cg00791190 | chr22 | 50223573 | 3 | MU | cg21129976 | chr7 | 100421502 | 20 | MU |
| cg07680505 | chr22 | 50223663 | 3 | N | cg22644163 | chr7 | 100421510 | 20 | MU |
| cg05705212 | chr3 | 45984743 | 3 | N | cg15600935 | chr7 | 101556684 | 20 | MU |
| cg08450017 | chr3 | 45984838 | 3 | MU | cg06746318 | chr7 | 101755186 | 20 | MU |
| cg25226014 | chr3 | 45984942 | 3 | MU | cg18541042 | chr7 | 140227195 | 20 | N |
| cg02923224 | chr3 | 60133085 | 3 | MU | cg13675721 | chr7 | 140227273 | 20 | MU |
| cg01040649 | chr3 | 186738320 | 3 | MU | cg03383886 | chr7 | 156336617 | 20 | MU |
| cg25599673 | chr3 | 186738370 | 3 | MU | cg08789101 | chr7 | 156336675 | 20 | N |
| cg07110949 | chr4 | 1309878 | 3 | MU | cg04058237 | chr7 | 156337107 | 20 | N |
| cg02600394 | chr4 | 48136234 | 3 | N | cg21839504 | chr7 | 156337168 | 20 | MU |
| cg20981615 | chr4 | 48136280 | 3 | MU | cg18195333 | chr7 | 156337209 | 20 | N |
| cg17984638 | chr4 | 48136452 | 3 | MU | cg23590389 | chr7 | 157102769 | 20 | MU |
| cg01804183 | chr5 | 35856424 | 3 | MU | cg23558764 | chr8 | 30635400 | 20 | MU |
| cg10837404 | chr5 | 112356289 | 3 | MU | cg21342785 | chr8 | 41707516 | 20 | N |
| cg02387618 | chr5 | 133453480 | 3 | MU | cg20270253 | chr8 | 41707595 | 20 | MU |
| cg04450994 | chr6 | 3318592 | 3 | MU | cg09541794 | chr8 | 144872852 | 20 | MU |
| cg08179431 | chr6 | 26086571 | 3 | MU | cg13768503 | chr9 | 72767353 | 20 | MU |
| cg13015616 | chr6 | 26086585 | 3 | N | cg14528751 | chr9 | 91144074 | 20 | MU |
| cg17615629 | chr6 | 30459867 | 3 | MU | cg13244315 | chr1 | 985393 | 21 | MU |
| cg20105257 | chr6 | 30460244 | 3 | MU | cg19900387 | chr1 | 6533368 | 21 | MU |
| cg02678305 | chr6 | 30460322 | 3 | N | cg09022584 | chr1 | 15541956 | 21 | MU |
| eg14437551 | chr6 | 31539986 | 3 | MU | cg12462916 | chr1 | 220132315 | 21 | MU |
| cg14597739 | chr6 | 31539998 | 3 | MU | cg00532451 | chr1 | 228528913 | 21 | MU |
| cg21999229 | chr6 | 31540014 | 3 | MU | cg13791379 | chr1 | 228528979 | 21 | N |
| cg17169196 | chr6 | 31540026 | 3 | MU | cg26718707 | chr10 | 518370 | 21 | MU |
| cg02402436 | chr6 | 31540051 | 3 | N | cg05394294 | chr10 | 3160926 | 21 | N |
| cg09736959 | chr6 | 31540114 | 3 | N | cg15319824 | chr10 | 3160974 | 21 | N |
| cg24216966 | chr6 | 31540121 | 3 | N | cg02579140 | chr10 | 3160982 | 21 | MU |
| cg11586857 | chr6 | 31540136 | 3 | N | cg14068301 | chr10 | 3161106 | 21 | N |
| cg22277485 | chr6 | 111854566 | 3 | MU | cg20095866 | chr10 | 133951618 | 21 | N |
| cg02098313 | chr6 | 128222378 | 3 | N | cg00851761 | chr10 | 133951761 | 21 | MU |
| cg20267828 | chr6 | 128222390 | 3 | MU | cg17132517 | chr10 | 133951829 | 21 | MU |
| cg21387735 | chr6 | 166908379 | 3 | MU | cg08321048 | chr10 | 133951963 | 21 | MU |
| cg05424831 | chr7 | 105382768 | 3 | MU | cg18049513 | chr11 | 541660 | 21 | MU |
| cg15039797 | chr7 | 139320346 | 3 | MU | cg13614606 | chr11 | 864739 | 21 | N |
| cg19246654 | chr7 | 139320460 | 3 | N | cg17998346 | chr11 | 864819 | 21 | MU |
| cg17995495 | chr7 | 150174507 | 3 | N | cg08373573 | chr11 | 1028291 | 21 | MU |
| cg21377860 | chr7 | 150174540 | 3 | N | cg20625588 | chr11 | 1028356 | 21 | N |
| cg12644845 | chr7 | 150174632 | 3 | MU | cg25259564 | chr11 | 1060659 | 21 | MU |
| cg01877778 | chr7 | 157415538 | 3 | MU | cg13511231 | chr11 | 1060679 | 21 | N |
| cg12419766 | chr7 | 157620445 | 3 | MU | cg16617073 | chr11 | 1060710 | 21 | N |
| cg24885442 | chr8 | 1740059 | 3 | MU | cg05745100 | chr11 | 1478112 | 21 | MU |
| cg07876450 | chr8 | 1740065 | 3 | MU | cg04925032 | chr11 | 1847227 | 21 | N |
| cg16193207 | chr8 | 23107961 | 3 | MU | cg26805905 | chr11 | 1847267 | 21 | N |
| cg11893955 | chr8 | 28918821 | 3 | MU | cg23726178 | chr11 | 1847307 | 21 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg27128883 | chr8 | 37438843 | 3 | MU | cg03529598 | chr11 | 1847357 | 21 | MU |
| cg18835815 | chr8 | 54757048 | 3 | N | cg16718999 | chr11 | 64108790 | 21 | MU |
| cg12047375 | chr8 | 54757132 | 3 | MU | cg08904394 | chr11 | 64108902 | 21 | N |
| cg22689323 | chr8 | 143926325 | 3 | MU | cg08707110 | chr11 | 65620170 | 21 | MU |
| cg11848483 | chr8 | 144543486 | 3 | MU | cg01068102 | chr11 | 65620199 | 21 | MU |
| cg04180114 | chr8 | 144543568 | 3 | N | cg18262591 | chr11 | 68579970 | 21 | MU |
| cg21900799 | chr8 | 145004388 | 3 | MU | cg03855388 | chr11 | 68580018 | 21 | MU |
| cg14522718 | chr9 | 130868874 | 3 | MU | cg20629021 | chr11 | 68580041 | 21 | N |
| cg21191275 | chr9 | 135907982 | 3 | MU | cg11880242 | chr11 | 102282062 | 21 | MU |
| cg15472170 | chr1 | 12012720 | 4 | MU | cg02643834 | chr11 | 130060371 | 21 | N |
| cg11157208 | chr1 | 15392308 | 4 | N | cg11069338 | chr11 | 130060459 | 21 | MU |
| cg03538296 | chr1 | 15392433 | 4 | MU | cg20964024 | chr12 | 111725484 | 21 | N |
| cg21813265 | chr1 | 17742966 | 4 | MU | cg20525229 | chr12 | 111725516 | 21 | MU |
| cg14182690 | chr1 | 25290947 | 4 | N | cg08162124 | chr13 | 113428094 | 21 | MU |
| cg07236781 | chr1 | 25291041 | 4 | MU | cg21897676 | chr13 | 113428161 | 21 | MU |
| cg01416168 | chr1 | 48687359 | 4 | MU | cg01831771 | chr13 | 113428172 | 21 | N |
| cg06183287 | chr1 | 48687392 | 4 | N | cg16100355 | chr13 | 113648607 | 21 | MU |
| cg03421440 | chr1 | 92414520 | 4 | MU | cg12947224 | chr13 | 114189945 | 21 | MU |
| cg09032544 | chr1 | 167487295 | 4 | MU | cg09537621 | chr13 | 114189998 | 21 | N |
| cg05799811 | chr1 | 167487396 | 4 | N | cg02034328 | chr14 | 77968467 | 21 | MU |
| cg15729230 | chr1 | 172628514 | 4 | MU | cg26171489 | chr15 | 92459303 | 21 | MU |
| cg03396826 | chr1 | 180161452 | 4 | MU | cg01149677 | chr16 | 1808230 | 21 | MU |
| cg17893934 | chr10 | 858817 | 4 | MU | cg10108042 | chr16 | 1808309 | 21 | N |
| eg06296597 | chr10 | 858900 | 4 | MU | cg05521198 | chr16 | 4382269 | 21 | MU |
| cg08876481 | chr10 | 858923 | 4 | MU | cg05599155 | chr16 | 29188654 | 21 | MU |
| cg17519479 | chr10 | 1222037 | 4 | MU | cg09602020 | chr16 | 29188796 | 21 | N |
| cg04640972 | chr10 | 8373522 | 4 | N | cg00284420 | chr16 | 87311948 | 21 | MU |
| cg09234252 | chr10 | 8373659 | 4 | MU | cg01579218 | chr16 | 87312047 | 21 | N |
| cg11637084 | chr10 | 52170429 | 4 | MU | cg17633681 | chr16 | 88106987 | 21 | N |
| cg17566718 | chr10 | 72357735 | 4 | MU | cg03395651 | chr16 | 88107091 | 21 | MU |
| cg26971585 | chr10 | 72358574 | 4 | MU | cg10398761 | chr16 | 89169289 | 21 | MU |
| cg02937751 | chr10 | 72358696 | 4 | N | cg10303721 | chr16 | 89674435 | 21 | N |
| cg16183122 | chr10 | 88466389 | 4 | MU | cg03664916 | chr16 | 89674560 | 21 | MU |
| cg05643988 | chr10 | 121575345 | 4 | MU | cg23098912 | chr17 | 5993686 | 21 | MU |
| cg17444479 | chr10 | 134429869 | 4 | MU | cg06421238 | chr17 | 5993785 | 21 | MU |
| cg24431515 | chr11 | 290447 | 4 | MU | cg24096540 | chr17 | 12695725 | 21 | MU |
| cg12332083 | chr11 | 1296469 | 4 | MU | cg24037380 | chr17 | 73727879 | 21 | N |
| cg11944324 | chr11 | 1296536 | 4 | N | cg26314765 | chr17 | 73727957 | 21 | MU |
| cg14907310 | chr11 | 2287734 | 4 | MU | cg20942310 | chr17 | 73728061 | 21 | N |
| cg08570686 | chr11 | 2287843 | 4 | N | cg17838182 | chr17 | 76991208 | 21 | MU |
| cg22885062 | chr11 | 64533556 | 4 | N | cg25052374 | chr17 | 76991253 | 21 | N |
| cg18479798 | chr11 | 64533623 | 4 | MU | cg23911097 | chr17 | 76991378 | . 21 | MU |
| cg27309871 | chr11 | 86224410 | 4 | MU | cg24844295 | chr17 | 78821928 | 21 | MU |
| cg15956469 | chr12 | 10465272 | 4 | MU | cg23660182 | chr19 | 916417 | 21 | MU |
| cg23474890 | chr12 | 122467179 | 4 | MU | cg22483095 | chr19 | 1112679 | 21 | MU |
| cg05957567 | chr12 | 122467228 | 4 | N | cg24361823 | chr19 | 1112732 | 21 | N |
| eg14506192 | chr12 | 122711988 | 4 | N | cg27594157 | chr19 | 1235003 | 21 | MU |
| cg10802680 | chr12 | 122712075 | 4 | N | cg03284113 | chr19 | 1235093 | 21 | N |
| cg14094409 | chr12 | 122712093 | 4 | MU | cg27341866 | chr19 | 2278618 | 21 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg19529732 | chr12 | 122712101 | 4 | N | cg07595134 | chr19 | 2278708 | 21 | MU |
| cg08622923 | chr12 | 122712116 | 4 | N | cg08399733 | chr19 | 2278773 | 21 | N |
| cg11804414 | chr12 | 122712137 | 4 | N | cg17437218 | chr19 | 2278847 | 21 | N |
| cg21805118 | chr12 | 122712153 | 4 | N | cg13465645 | chrl9 | 3578711 | 21 | MU |
| cg09123760 | chr12 | 122712212 | 4 | N | cg05574870 | chr19 | 4054124 | 21 | N |
| cg13587802 | chr12 | 122712381 | 4 | MU | cg27196194 | chr19 | 4054253 | 21 | MU |
| cg13523236 | chr12 | 129277140 | 4 | MU | cg24478966 | chr19 | 42460930 | 21 | MU |
| cg05253716 | chr12 | 129299332 | 4 | N | cg15706424 | chr2 | 469781 | 21 | N |
| cg13617280 | chr12 | 129299462 | 4 | MU | cg08131059 | chr2 | 469790 | 21 | MU |
| cg16316183 | chr12 | 129299465 | 4 | N | cg10900049 | chr2 | 8849962 | 21 | MU |
| cg24410690 | chr12 | 129299481 | 4 | N | cg00240378 | chr2 | 8850020 | 21 | MU |
| cg22040672 | chr12 | 129299605 | 4 | N | cg03448016 | chr2 | 8850066 | 21 | N |
| cg14791799 | chr13 | 24227716 | 4 | MU | cg12793924 | chr2 | 17830624 | 21 | MU |
| cg26573274 | chr13 | 110386152 | 4 | MU | cg10907356 | chr2 | 17830694 | 21 | N |
| cg24430034 | chr13 | 110386176 | 4 | MU | cg27656592 | chr2 | 17830761 | 21 | N |
| cg01561807 | chr13 | 114307602 | 4 | MU | cg12942038 | chr2 | 97427895 | 21 | N |
| cg00054352 | chr13 | 114891672 | 4 | MU | cg11464842 | chr2 | 97427975 | 21 | MU |
| cg20042612 | chr14 | 23027728 | 4 | MU | cg03532037 | chr2 | 106959205 | 21 | N |
| cg18247172 | chr15 | 91370233 | 4 | MU | cg24419520 | chr2 | 106959257 | 21 | MU |
| cg09061454 | chr16 | 31075359 | 4 | MU | cg08093959 | chr2 | 106959853 | 21 | N |
| cg12997627 | chr16 | 85676292 | 4 | N | cg15139063 | chr2 | 106959868 | 21 | MU |
| cg08480774 | chr16 | '85676306 | 4 | N | cg12606455 | chr2 | 106959878 | 21 | MU |
| cg26648465 | chr16 | 85676412 | 4 | MU | cg15314023 | chr2 | 239050026 | 21 | MU |
| cg01582532 | chr16 | 85676526 | 4 | N | cg19430414 | chr2 | 239050088 | 21 | N |
| cg05355684 | chr16 | 88720379 | 4 | MU | cg11523191 | chr20 | 62187126 | 21 | MU |
| cg09115713 | chr16 | 88832476 | 4 | MU | cg07819160 | chr22 | 37420328 | 21 | N |
| cg03831847 | chr16 | 88832485 | 4 | MU | cg12253469 | chr22 | 37420454 | 21 | MU |
| cg08798862 | chr16 | 88832532 | 4 | N | cg13814677 | chr22 | 46809688 | 21 | MU |
| cg05635114 | chr16 | 88832572 | 4 | N | cg19817118 | chr3 | 31494178 | 21 | MU |
| cg04042333 | chr17 | 1104665 | 4 | MU | cg20927547 | chr3 | 31494215 | 21 | MU |
| cg02352716 | chr17 | 1104805 | 4 | N | cg00184032 | chr3 | 31494263 | 21 | N |
| cg15690475 | chr17 | 44101453 | 4 | MU | cg24724109 | chr3 | 31494362 | 21 | N |
| cg15220605 | chr17 | 80393595 | 4 | N | cg13748287 | chr3 | 184290594 | 21 | N |
| cg03106251 | chr17 | 80393598 | 4 | N | cg19161330 | chr3 | 184290643 | 21 | N |
| cg00910503 | chr17 | 80393666 | 4 | MU | cg13735557 | chr3 | 184290720 | 21 | MU |
| cg24042899 | chr17 | 80986552 | 4 | MU | cg10769844 | chr3 | 184290794 | 21 | N |
| cg18698382 | chr19 | 647205 | 4 | N | cg18163092 | chr3 | 197246936 | 21 | MU |
| cg10107671 | chr19 | 647289 | 4 | MU | cg19463199 | chr3 | 197401858 | 21 | N |
| cg10214132 | chr19 | 3814131 | 4 | MU | cg12023911 | chr3 | 197401931 | 21 | N |
| cg22521333 | chr19 | 4652251 | 4 | MU | cg27013765 | chr3 | 197402007 | 21 | MU |
| cg24137511 | chr19 | 18260330 | 4 | MU | cg09559242 | chr3 | 197402153 | 21 | N |
| cg06706159 | chr19 | 18260350 | 4 | MU | cg04045327 | chr4 | 905805 | 21 | MU |
| cg17022038 | chr19 | 18260479 | 4 | N | cg27309282 | chr4 | 1016081 | 21 | MU |
| cg09776718 | chr19 | 30164820 | 4 | MU | cg17095315 | chr4 | 2038352 | 21 | MU |
| cg12103219 | chr19 | 30165308 | 4 | MU | cg05069807 | chr4 | 6945702 | 21 | MU |
| cg12011479 | chr19 | 51873045 | 4 | MU | cg17630294 | chr4 | 7985238 | 21 | MU |
| cg01454237 | chr19 | 55314879 | 4 | N | cg10744200 | chr4 | 24844162 | 21 | MU |
| cg08326410 | chr19 | 55314884 | 4 | MU | cg08100122 | chr4 | 187629232 | 21 | N |
| cg24838349 | chr19 | 55314972 | 4 | N | cg21399040 | chr4 | 187629328 | 21 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg00876196 | chr19 | 55315034 | 4 | N | cg24820270 | chr4 | 187629336 | 21 | MU |
| cg23483108 | chr19 | 55361761 | 4 | N | cg01040779 | chr4 | 187629339 | 21 | N |
| cg01532713 | chr19 | 55361904 | 4 | MU | cg25352342 | chr4 | 187629387 | 21 | MU |
| cg00462849 | chr2 | 12853027 | 4 | MU | cg24833566 | chr4 | 187629400 | 21 | N |
| cg11535839 | chr2 | 28582676 | 4 | MU | cg13454542 | chr4 | 187629427 | 21 | N |
| cg23882186 | chr2 | 99065421 | 4 | MU | cg10010780 | chr4 | 187629520 | 21 | N |
| cg26675329 | chr2 | 103033753 | 4 | N | cg01221209 | chr5 | 554886 | 21 | MU |
| cg11335172 | chr2 | 103033799 | 4 | MU | cg26722544 | chr5 | 179393917 | 21 | MU |
| cg20949306 | chr2 | 135926634 | 4 | MU | cg20465064 | chr5 | 179393940 | 21 | N |
| cg26101890 | chr2 | -228185396 | 4 | MU | cg14815183 | chr6 | 30131613 | 21 | N |
| cg27309098 | chr2 | 236619748 | 4 | MU | cg07421969 | chr6 | 30131634 | 21 | N |
| cg14930335 | chr2 | 236619767 | 4 | MU | cg21988950 | chr6 | 30131691 | 21 | N |
| cg21672829 | chr2 | 236619825 | 4 | N | cg09769113 | chr6 | 30131719 | 21 | N |
| cg19040077 | chr2 | 242702749 | 4 | N | cg05664039 | chr6 | 30131755 | 21 | MU |
| cg13613174 | chr2 | 242702881 | 4 | MU | cg00628477 | chr6 | 30131821 | 21 | N |
| cg24743237 | chr2 | 242702932 | 4 | MU | cg01905142 | chr6 | 30131840 | 21 | N |
| cg24231380 | chr2 | 242813914 | 4 | MU | cg07889936 | chr6 | 30616472 | 21 | MU |
| cg09631939 | chr20 | 60582478 | 4 | MU | cg10669449 | chr6 | 106676237 | 21 | MU |
| cg26757673 | chr22 | 37545423 | 4 | MU | cg11532971 | chr6 | 106676381 | 21 | N |
| cg04392554 | chr22 | 46685472 | 4 | MU | cg17301053 | chr6 | 166891617 | 21 | N |
| cg27274718 | chr3 | 15772890 | 4 | MU | cg04224624 | chr6 | 166891650 | 21 | MU |
| cg22917487 | chr3 | 39322103 | 4 | MU | cg18092351 | chr7 | 960115 | 21 | MU |
| cg19456938 | chr3 | 114012806 | 4 | MU | cg13687274 | chr7 | 960246 | 21 | N |
| cg13669740 | chr3 | 114012833 | 4 | N | cg20449048 | chr7 | 1037343 | 21 | MU |
| cg20832020 | chr3 | 114012912 | 4 | N | cg17334762 | chr7 | 4862349 | 21 | MU |
| cg21333217 | chr3 | 129277321 | 4 | MU | eg25319337 | chr7 | 48008127 | 21 | MU |
| cg00293189 | chr3 | 186856440 | 4 | MU | cg16233515 | chr7 | 73408058 | 21 | MU |
| cg23617947 | chr3 | 195347035 | 4 | MU | cg07243141 | chr7 | 73408060 | 21 | MU |
| cg18973586 | chr4 | 963968 | 4 | MU | cg27099880 | chr7 | 74021938 | 21 | MU |
| cg12226453 | chr4 | 964011 | 4 | MU | cg18771538 | chr7 | 107483786 | 21 | N |
| cg22488278 | chr4 | 2321957 | 4 | N | cg07536914 | chr7 | 107483801 | 21 | MU |
| cg01161042 | chr4 | 2322052 | 4 | N | cg10290200 | chr7 | 128481295 | 21 | MU |
| cg12811871 | chr4 | 2322078 | 4 | MU | cg17677524 | chr7 | 150069026 | 21 | MU |
| cg09494176 | chr4 | 3193868 | 4 | MU | cg24278165 | chr8 | 23083551 | 21 | MU |
| cg22326973 | chr4 | 39771745 | 4 | N | cg23303108 | chr8 | 23083578 | 21 | N |
| cg02407068 | chr4 | 39771851 | 4 | MU | cg03598366 | chr8 | 61789011 | 21 | MU |
| cg18906283 | chr4 | 39771913 | 4 | N | cg13568432 | chr8 | 61789077 | 21 | MU |
| cg06202778 | chr4 | 57514827 | 4 | MU | cg13494954 | chr9 | 97369791 | 21 | MU |
| cg05637113 | chr5 | 121307216 | 4 | MU | cg01219549 | chr9 | 140683649 | 21 | N |
| cg14437551 | chr6 | 31539986 | 4 | MU | cg14050824 | chr9 | 140683797 | 21 | MU |
| cg14597739 | chr6 | 31539998 | 4 | N | cg03935783 | chr1 | 1161069 | 22 | MU |
| cg21999229 | chr6 | 31540014 | 4 | N | cg24002071 | chr1 | 2519920 | 22 | MU |
| cg17169196 | chr6 | 31540026 | 4 | N | cg07563069 | chr1 | 2522330 | 22 | N |
| cg02402436 | chr6 | 31540051 | 4 | N | cg19633487 | chr1 | 2522370 | 22 | MU |
| cg09736959 | chr6 | 31540114 | 4 | N | cg18232828 | chr1 | 10846092 | 22 | MU |
| cg24216966 | chr6 | 31540121 | 4 | N | cg17431922 | chr1 | 19599516 | 22 | MU |
| cg11586857 | chr6 | 31540136 | 4 | N | cg18594482 | chr1 | 27480543 | 22 | N |
| cg10917426 | chr6 | 31867698 | 4 | MU | cg26515162 | chr1 | 27480594 | 22 | MU |
| cg16463880 | chr6 | 31867707 | 4 | MU | cg22481448 | chr1 | 27480668 | 22 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg00459243 | chr6 | 31867726 | 4 | N | cg11803803 | chr1 | 28775373 | 22 | MU |
| cg14562426 | chr6 | 31867757 | 4 | N | cg14092259 | chr1 | 41220011 | 22 | N |
| cg06636203 | chr6 | 31867788 | 4 | N | cg06379876 | chr1 | 41220106 | 22 | MU |
| cg11645762 | chr6 | 31867819 | 4 | N | cg05414613 | chr1 | 53558470 | 22 | N |
| cg09788778 | chr6 | 31867822 | 4 | MU | cg04142017 | chr1 | 53558596 | 22 | MU |
| cg25470384 | chr6 | 31867836 | 4 | MU | cg08256691 | chr1 | 155881846 | 22 | N |
| cg00805874 | chr6 | 31867847 | 4 | MU | cg24337215 | chr1 | 155881944 | 22 | MU |
| cg04603811 | chr6 | 31867873 | 4 | MU | cg01553584 | chr1 | 224400672 | 22 | MU |
| cg25861453 | chr6 | 31867906 | 4 | N | cg05655953 | chr10 | 123810032 | 22 | MU |
| cg25013586 | chr6 | 31867915 | 4 | N | cg06732450 | chr10 | 134572361 | 22 | N |
| cg13127825 | chr6 | 31867919 | 4 | N | cg09768859 | chr10 | 134572388 | 22 | MU |
| cg17243044 | chr6 | 31867945 | 4 | N | cg17994840 | chr10 | 134572417 | 22 | MU |
| cg00058449 | chr6 | 31867956 | 4 | N | cg03246584 | chr10 | 134663467 | 22 | MU |
| cg07249939 | chr6 | 31867995 | 4 | N | cg12818656 | chr11 | 1031856 | 22 | MU |
| cg12484688 | chr6 | 31868021 | 4 | N | cg04539057 | chr11 | 1031985 | 22 | MU |
| cg22755579 | chr6 | 32115211 | 4 | N | cg23270726 | chr11 | 1033157 | 22 | MU |
| cg02287105 | chr6 | 32115284 | 4 | MU | cg09906402 | chr11 | 1033236 | 22 | N |
| cg17720554 | chr6 | 169284285 | 4 | N | cg11056766 | chr11 | 1230072 | 22 | N |
| cg10213762 | chr6 | 169284319 | 4 | MU | cg25840179 | chr11 | 1230144 | 22 | MU |
| cg12550496 | chr6 | 169284344 | 4 | MU | cg24297451 | chr11 | 19453448 | 22 | MU |
| cg27287951 | chr7 | 961322 | 4 | MU | cg14642259 | chr11 | 47359017 | 22 | MU |
| cg06589239 | chr7 | 961457 | 4 | N | cg05649391 | chr11 | 47359115 | 22 | N |
| cg23048001 | chr7 | 2026167 | 4 | MU | eg11179625 | chr11 | 61522741 | 22 | N |
| cg06504636 | chr7 | 2089841 | 4 | N | cg05881566 | chr11 | 61522765 | 22 | MU |
| cg02240030 | chr7 | 2089880 | 4 | MU | cg11060910 | chr11 | 61522775 | 22 | MU |
| cg12803060 | chr7 | 100850157 | 4 | MU | cg07486017 | chr11 | 61522799 | 22 | N |
| cg15039797 | chr7 | 139320346 | 4 | MU | cg06137032 | chr11 | 61522807 | 22 | MU |
| cg19246654 | chr7 | 139320460 | 4 | N | eg16756854 | chr11 | 66116455 | 22 | MU |
| cg19696012 | chr8 | 128745037 | 4 | MU | cg12598767 | chr11 | 72465959 | 22 | MU |
| cg06201717 | chr1 | 2949633 | 5 | N | cg03697509 | chr11 | 72466028 | 22 | N |
| cg24150662 | chr1 | 2949673 | 5 | MU | cg22397625 | chr11 | 119044725 | 22 | N |
| cg01002223 | chr1 | 8987034 | 5 | MU | cg07478335 | chr11 | 119044743 | 22 | MU |
| cg11947782 | chr1 | 9775985 | 5 | MU | cg22335368 | chr11 | 119044774 | 22 | N |
| cg01943221 | chr1 | 9776133 | 5 | N | cg16682206 | chr13 | 111277501 | 22 | N |
| cg07970040 | chr1 | 9789165 | 5 | MU | cg21664614 | chr13 | 111277611 | 22 | MU |
| cg04340595 | chr1 | 9789174 | 5 | N | cg02898500 | chr13 | 113826554 | 22 | MU |
| cg14182690 | chr1 | 25290947 | 5 | N | cg03775163 | chr13 | 114074693 | 22 | MU |
| cg07236781 | chr1 | 25291041 | 5 | MU | cg13980446 | chr13 | 114312327 | 22 | MU |
| cg17820878 | chr1 | 27440463 | 5 | MU | cg14699932 | chr13 | 114748340 | 22 | MU |
| cg13300580 | chr1 | 27440539 | 5 | N | cg10800989 | chr14 | 99786193 | 22 | MU |
| eg11683242 | chr1 | 32716557 | 5 | MU | cg04741516 | chr14 | 99786208 | 22 | N |
| cg09018107 | chr1 | 67798929 | 5 | MU | cg10538275 | chr14 | 99786226 | 22 | N |
| cg25211348 | chr1 | 111769665 | 5 | MU | cg26062476 | chr14 | 103583177 | 22 | MU |
| cg03016153 | chr1 | 117296954 | 5 | MU | cg10533694 | chr14 | 105181370 | 22 | MU |
| cg16374333 | chr1 | 157103641 | 5 | MU | cg11290775 | chr14 | 105181449 | 22 | N |
| cg09032544 | chr1 | 167487295 | 5 | MU | cg27542341 | chr15 | 75248086 | 22 | MU |
| cg05799811 | chr1 | 167487396 | 5 | N | cg07344990 | chr16 | 625906 | 22 | MU |
| cg07786657 | chr1 | 167487633 | 5 | MU | cg03897436 | chr16 | 1425469 | 22 | MU |
| cg09554443 | chr1 | 167487762 | 5 | N | cg00674415 | chr16 | 1859330 | 22 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg15729230 | chr1 | 172628514 | 5 | MU | cg09328356 | chr16 | 29913162 | 22 | MU |
| cg09372617 | chr1 | 198136518 | 5 | MU | cg02488299 | chr16 | 29913223 | 22 | N |
| cg04640972 | chr10 | 8373522 | 5 | N | cg27156649 | chr16 | 30567019 | 22 | MU |
| cg09234252 | chr10 | 8373659 | 5 | MU | cg00167684 | chr16 | 70505196 | 22 | MU |
| cg00929860 | chr10 | 73846448 | 5 | MU | cg07475178 | chr16 | 75149110 | 22 | MU |
| cg17444479 | chr10 | 134429869 | 5 | MU | cg00284420 | chr16 | 87311948 | 22 | MU |
| cg24431515 | chr11 | 290447 | 5 | MU | cg01579218 | chr16 | 87312047 | 22 | N |
| cg22885062 | chr11 | 64533556 | 5 | N | cg16559361 | chr16 | 88543440 | 22 | MU |
| cg18479798 | chr11 | 64533623 | 5 | MU | cg26884787 | chr16 | 88543447 | 22 | N |
| cg24612198 | chr11 | 118175631 | 5 | MU | cg04228827 | chr16 | 88550365 | 22 | N |
| cg06164961 | chr11 | 118175675 | 5 | N | cg02071579 | chr16 | 88550450 | 22 | MU |
| cg07728874 | chr11 | 118213272 | 5 | N | cg00379559 | chr16 | 88564934 | 22 | N |
| cg24841244 | chr11 | 118213330 | 5 | MU | cg10095533 | chr16 | 88565080 | 22 | MU |
| cg03074244 | chr11 | 118213414 | 5 | N | cg04387175 | chr16 | 88568629 | 22 | MU |
| cg10977115 | chr11 | 122709471 | 5 | MU | cg27631965 | chr16 | 88575243 | 22 | N |
| cg22512531 | chr11 | 122709551 | 5 | MU | cg05242494 | chr16 | 88575349 | 22 | MU |
| cg11268190 | chr12 | 6442526 | 5 | N | cg01753252 | chr16 | 88757588 | 22 | MU |
| cg00556515 | chr12 | 6442607 | 5 | MU | cg01183711 | chr16 | 88883084 | 22 | MU |
| cg21330949 | chr12 | 19557320 | 5 | MU | cg19463982 | chr17 | 48614436 | 22 | MU |
| cg20894640 | chr12 | 19557334 | 5 | MU | cg03237431 | chr17 | 65039695 | 22 | MU |
| eg14287022 | chr12 | 19557343 | 5 | N | cg19716073 | chr17 | 65039817 | 22 | N |
| cg08338281 | chr12 | 56322667 | 5 | MU | cg25648211 | chr17 | 76312455 | 22 | MU |
| cg08059719 | chr12 | 122444450 | 5 | N | cg17838182 | chr17 | 76991208 | 22 | MU |
| cg14480046 | chr12 | 122444580 | 5 | MU | cg25052374 | chr17 | 76991253 | 22 | N |
| cg04716447 | chr12 | 122444621 | 5 | N | cg23911097 | chr17 | 76991378 | 22 | MU |
| cg14506192 | chr12 | 122711988 | 5 | MU | cg17129554 | chr17 | 76993272 | 22 | N |
| cg10802680 | chr12 | 122712075 | 5 | N | cg22687077 | chr17 | 76993374 | 22 | N |
| cg14094409 | chr12 | 122712093 | 5 | MU | cg00902147 | chr17 | 76993394 | 22 | MU |
| cg19529732 | chr12 | 122712101 | 5 | MU | cg18186771 | chr17 | 76993506 | 22 | N |
| cg08622923 | chr12 | 122712116 | 5 | N | cg12856760 | chrl7 | 77976092 | 22 | MU |
| cg11804414 | chr12 | 122712137 | 5 | N | cg27177415 | chr17 | 79412681 | 22 | MU |
| cg21805118 | chr12 | 122712153 | 5 | MU | cg06546521 | chr17 | 79412788 | 22 | N |
| cg09123760 | chr12 | 122712212 | 5 | N | cg07049352 | chr17 | 80654335 | 22 | MU |
| cg13587802 | chr12 | 122712381 | 5 | MU | cg17969902 | chr19 | 1274584 | 22 | MU |
| cg24532476 | chr12 | 133424566 | 5 | N | cg11634930 | chr19 | 2046085 | 22 | MU |
| cg09120938 | chr12 | 133424655 | 5 | MU | cg13220129 | chr19 | 2046193 | 22 | N |
| cg09088496 | chr13 | 24825973 | 5 | MU | cg08626004 | chr19 | 2513687 | 22 | MU |
| cg21565496 | chr13 | 40762150 | 5 | MU | cg18415485 | chr19 | 3546364 | 22 | MU |
| cg21845390 | chr13 | 40762260 | 5 | N | cg25793425 | chr19 | 6007842 | 22 | MU |
| cg01561807 | chr13 | 114307602 | 5 | MU | cg26008272 | chr19 | 45574917 | 22 | N |
| cg20042612 | chr14 | 23027728 | 5 | MU | cg15732530 | chr19 | 45575023 | 22 | MU |
| cg07015803 | chr14 | 99655593 | 5 | N | cg24030434 | chr19 | 45575134 | 22 | N |
| cg16452866 | chr14 | 99655676 | 5 | MU | cg23633937 | chr19 | 45575142 | 22 | N |
| cg16039157 | chr15 | 45007336 | 5 | MU | cg15153684 | chr2 | 10686963 | 22 | MU |
| cg25508605 | chr15 | 92706125 | 5 | MU | cg13851334 | chr2 | 10686975 | 22 | MU |
| cg01994902 | chr16 | 1576069 | 5 | N | cg09761224 | chr2 | 10687042 | 22 | MU |
| cg27316811 | chr16 | 1576146 | 5 | MU | cg07622575 | chr2 | 11487955 | 22 | MU |
| cg00459119 | chr16 | 12172834 | 5 | N | cg05372365 | chr2 | 74687608 | 22 | MU |
| cg01366941 | chr16 | 12172962 | 5 | MU | cg03405781 | chr2 | 103095638 | 22 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg26922780 | chr16 | 88769443 | 5 | MU | cg10403518 | chr2 | 103095704 | 22 | N |
| cg07661835 | chr16 | 89163578 | 5 | N | cg10938446 | chr2 | 113875723 | 22 | MU |
| cg03172657 | chr16 | 89163625 | 5 | MU | cg26641275 | chr2 | 239971554 | 22 | MU |
| cg04042333 | chr17 | 1104665 | 5 | MU | cg14646111 | chr20 | 18489660 | 22 | MU |
| cg02352716 | chr17 | 1104805 | 5 | N | cg15457037 | chr20 | 62562848 | 22 | MU . |
| cg23698124 | chr17 | 2154177 | 5 | MU | cg20341238 | chr21 | 44865764 | 22 | MU |
| cg19001909 | chr17 | 26205940 | 5 | MU | cg25310447 | chr21 | 46957444 | 22 | MU |
| cg03930965 | chr17 | 35966055 | 5 | MU | cg10361281 | chr22 | 24811256 | 22 | MU |
| cg24456340 | chr17 | 47286483 | 5 | MU | cg20746101 | chr22 | 50725470 | 22 | N |
| cg15046489 | chr17 | 48857353 | 5 | MU | cg23041250 | chr22 | 50725605 | 22 | N |
| cg04945945 | chr17 | 76129402 | 5 | N | cg12944146 | chr22 | 50725609 | 22 | MU |
| cg05637296 | chr17 | 76129475 | 5 | N | cg11314274 | chr3 | 132004064 | 22 | MU |
| cg26003388 | chr17 | 76129533 | 5 | MU | cg10521403 | chr4 | 1763211 | 22 | MU |
| cg03388043 | chr17 | 80084554 | 5 | MU | cg26605046 | chr4 | 2439731 | 22 | MU |
| cg10505658 | chr17 | 80084571 | 5 | N | cg27491224 | chr4 | 2439740 | 22 | N |
| cg23666829 | chr17 | 80842382 | 5 | N | cg15838301 | chr4 | 7719899 | 22 | MU |
| cg15853299 | chr17 | 80842469 | 5 | MU | cg27175016 | chr4 | 7719984 | 22 | N |
| cg10730425 | chr19 | 1035093 | 5 | MU | cg06018666 | chr4 | 10079364 | 22 | MU |
| cg12103219 | chr19 | 30165308 | 5 | MU | cg07213482 | chr4 | 89660237 | 22 | MU |
| cg11236515 | chr2 | 74213762 | 5 | MU | cg26224791 | chr5 | 1320734 | 22 | MU |
| cg00219921 | chr2 | 87012810 | 5 | MU | cg03887092 | chr6 | 1080885 | 22 | MU |
| cg25939861 | chr2 | 87020937 | 5 | MU | cg00077898 | chr6 | 1081144 | 22 | MU |
| cg23882186 | chr2 | 99065421 | 5 | MU | cg16306083 | chr6 | 170449858 | 22 | N |
| cg04759756 | chr20 | 35273933 | 5 | MU | cg14218838 | chr6 | 170449884 | 22 | MU |
| cg23366234 | chr21 | 45713704 | 5 | MU | cg16645977 | chr7 | 5342805 | 22 | N |
| cg26472802 | chr21 | 45713719 | 5 | MU | cg17230640 | chr7 | 5342893 | 22 | MU |
| cg03062881 | chr21 | 47844012 | 5 | MU | cg27637597 | chr7 | 5342933 | 22 | N |
| cg04314225 | chr21 | 47844134 | 5 | N | cg13802883 | chr7 | 23749533 | 22 | N |
| cg23825480 | chr22 | 31336785 | 5 | MU | cg14898779 | chr7 | 23749607 | 22 | N |
| cg26757673 | chr22 | 37545423 | 5 | MU | cg25612145 | chr7 | 23749639 | 22 | MU |
| cg04392554 | chr22 | 46685472 | 5 | MU | cg13514718 | chr7 | 23749722 | 22 | N |
| cg05705212 | chr3 | 45984743 | 5 | N | cg24090529 | chr7 | 97844910 | 22 | MU |
| cg08450017 | chr3 | 45984838 | 5 | MU | cg23265009 | chr7 | 97844947 | 22 | N |
| cg25226014 | chr3 | 45984942 | 5 | MU | cg23043230 | chr7 | 97935666 | 22 | N |
| cg21333217 | chr3 | 129277321 | 5 | MU | cg15211734 | chr7 | 97935756 | 22 | N |
| cg19010441 | chr3 | 150917888 | 5 | MU | cg10592494 | chr7 | 97935764 | 22 | MU |
| cg18973586 | chr4 | 963968 | 5 | MU | cg18753341 | chr7 | 157202405 | 22 | MU |
| cg12226453 | chr4 | 964011 | 5 | MU | cg17665357 | chr8 | 2024249 | 22 | MU |
| cg07110949 | chr4 | 1309878 | 5 | MU | cg23588952 | chr8 | 2024300 | 22 | N |
| cg22326973 | chr4 | 39771745 | 5 | N | cg13261536 | chr8 | 144343915 | 22 | MU |
| cg02407068 | chr4 | 39771851 | 5 | MU | cg13494954 | chr9 | 97369791 | 22 | MU |
| cg18906283 | chr4 | 39771913 | 5 | N | cg01219549 | chr9 | 140683649 | 22 | N |
| cg17984638 | chr4 | 48136452 | 5 | MU | cg14050824 | chr9 | 140683797 | 22 | MU |
| cg20105257 | chr6 | 30460244 | 5 | MU | cg01922485 | chr1 | 942696 | 23 | MU |
| cg02678305 | chr6 | 30460322 | 5 | N | cg09159050 | chr1 | 1563500 | 23 | MU |
| cg14437551 | chr6 | 31539986 | 5 | MU | cg27235337 | chr1 | 2334785 | 23 | N |
| cg14597739 | chr6 | 31539998 | 5 | MU | cg03960390 | chr1 | 2334914 | 23 | MU |
| cg21999229 | chr6 | 31540014 | 5 | MU | cg15733049 | chr1 | 2334974 | 23 | N |
| cg17169196 | chr6 | 31540026 | 5 | MU | cg02328660 | chr1 | 3389744 | 23 | MU |

(continued)

| Illumina Probe Name | Chr. | Position | Tissue/Cell Type | MU/N | Illumina Probe Name | Chr. | Position | Tissue/Cell Type | MU/N |
|---|---|---|---|---|---|---|---|---|---|
| cg02402436 | chr6 | 31540051 | 5 | N | cg03846767 | chr1 | 3607292 | 23 | N |
| cg09736959 | chr6 | 31540114 | 5 | N | cg24878868 | chr1 | 3607401 | 23 | MU |
| cg24216966 | chr6 | 31540121 | 5 | N | cg26208930 | chr1 | 3607425 | 23 | N |
| cg11586857 | chr6 | 31540136 | 5 | MU | cg17808634 | chr1 | 4275141 | 23 | MU |
| cg10917426 | chr6 | 31867698 | 5 | N | cg20318235 | chr1 | 10707917 | 23 | N |
| cg16463880 | chr6 | 31867707 | 5 | N | cg21787835 | chr1 | 10707934 | 23 | MU |
| cg00459243 | chr6 | 31867726 | 5 | N | cg03962214 | chr1 | 10707953 | 23 | N |
| cg14562426 | chr6 | 31867757 | 5 | N | cg02172466 | chr1 | 10708003 | 23 | N |
| cg06636203 | chr6 | 31867788 | 5 | N | cg17481936 | chr1 | 116371802 | 23 | N |
| cg11645762 | chr6 | 31867819 | 5 | N | cg23952578 | chr1 | 116371871 | 23 | MU |
| cg09788778 | chr6 | 31867822 | 5 | MU | cg21101726 | chr1 | 204124158 | 23 | MU |
| cg25470384 | chr6 | 31867836 | 5 | MU | cg20691436 | chr10 | 5567478 | 23 | MU |
| cg00805874 | chr6 | 31867847 | 5 | MU | cg07748741 | chr10 | 99330056 | 23 | MU |
| cg04603811 | chr6 | 31867873 | 5 | MU | cg02613380 | chr10 | 99330076 | 23 | N |
| cg25861453 | chr6 | 31867906 | 5 | N | cg15232718 | chr10 | 99330203 | 23 | N |
| cg25013586 | chr6 | 31867915 | 5 | N | cg04426802 | chr10 | 126314680 | 23 | MU |
| cg13127825 | chr6 | 31867919 | 5 | N | cg26725552 | chr10 | 126314758 | 23 | N |
| cg17243044 | chr6 | 31867945 | 5 | N | cg07984614 | chr10 | 126314798 | 23 | MU |
| cg00058449 | chr6 | 31867956 | 5 | N | cg21762589 | chr10 | 133796476 | 23 | N |
| cg07249939 | chr6 | 31867995 | 5 | N | cg02835981 | chr10 | 133796494 | 23 | N |
| cg12484688 | chr6 | 31868021 | 5 | N | cg13599131 | chr10 | 133796523 | 23 | N |
| cg02098313 | chr6 | 128222378 | 5 | N | cg27353308 | chr10 | 133796559 | 23 | MU |
| cg20267828 | chr6 | 128222390 | 5 | MU | cg12348511 | chr11 | 3187678 | 23 | N |
| cg12603453 | chr6 | 151694679 | 5 | MU | cg15718663 | chr11 | 3187784 | 23 | N |
| cg21387735 | chr6 | 166908379 | 5 | MU | cg18897335 | chr11 | 3187790 | 23 | N |
| cg17720554 | chr6 | 169284285 | 5 | N | cg05982473 | chr11 | 3187803 | 23 | MU |
| cg10213762 | chr6 | 169284319 | 5 | MU | cg04976753 | chr11 | 3187921 | 23 | N |
| cg12550496 | chr6 | 169284344 | 5 | MU | cg12537619 | chr11 | 3187923 | 23 | N |
| cg17158941 | chr7 | 1073255 | 5 | MU | cg19563452 | chr11 | 3187939 | 23 | N |
| cg15039797 | chr7 | 139320346 | 5 | MU | cg13826564 | chr11 | 65306731 | 23 | MU |
| cg19246654 | chr7 | 139320460 | 5 | N | cg04808027 | chr11 | 66102146 | 23 | N |
| cg01877778 | chr7 | 157415538 | 5 | MU | cg12912663 | chr11 | 66102239 | 23 | MU |
| cg11893955 | chr8 | 28918821 | 5 | MU | cg12068791 | chr11 | 66102352 | 23 | N |
| cg22689323 | chr8 | 143926325 | 5 | MU | cg14139875 | chr11 | 66454595 | 23 | MU |
| cg14392283 | chr8 | 144103587 | 5 | MU | cg22306015 | chr11 | 66454625 | 23 | N |
| cg11848483 | chr8 | 144543486 | 5 | MU | cg04822177 | chr11 | 66454651 | 23 | N |
| cg04180114 | chr8 | 144543568 | 5 | N | cg01176141 | chr11 | 70023032 | 23 | MU |
| cg14522718 | chr9 | 130868874 | 5 | MU | cg13089599 | chr11 | 70023060 | 23 | N |
| cg00826902 | chr1 | 3563954 | 6 | N | cg24832721 | chr11 | 70023142 | 23 | N |
| cg11832534 | chr1 | 3563998 | 6 | MU | cg11436025 | chr11 | 117376238 | 23 | MU |
| cg05399785 | chr1 | 3564031 | 6 | N | cg23217865 | chr11 | 117376295 | 23 | N |
| cg17918090 | chr1 | 17751920 | 6 | MU | cg24288581 | chr11 | 117376331 | 23 | N |
| cg21932513 | chr1 | 17751974 | 6 | N | cg21596290 | chr11 | 119993722 | 23 | N |
| cg07394400 | chr1 | 17752023 | 6 | N | cg14398731 | chr11 | 119993732 | 23 | N |
| cg09475813 | chr1 | 35250346 | 6 | N | cg04745079 | chr11 | 119993751 | 23 | MU |
| cg02030454 | chr1 | 35250489 | 6 | MU | cg04896048 | chr11 | 119993780 | 23 | N |
| cg07033722 | chr1 | 40539032 | 6 | MU | cg10457436 | chr12 | 6745871 | 23 | MU |
| cg15388975 | chr1 | 45120147 | 6 | N | cg13189671 | chr12 | 123750782 | 23 | N |
| cg18315935 | chr1 | 45120295 | 6 | MU | cg14790739 | chr12 | 123750827 | 23 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg06350097 | chr1 | 161474970 | 6 | MU | cg05950157 | chr12 | 130603995 | 23 | MU |
| cg19406975 | chr1 | 249110746 | 6 | MU | cg02300114 | chr12 | 130604047 | 23 | N |
| cg13914486 | chr10 | 6117324 | 6 | MU | cg02026637 | chr12 | 132348618 | 23 | N |
| cg17960717 | chr10 | 76569871 | 6 | MU | cg04455286 | chr12 | 132348639 | 23 | MU |
| cg11771383 | chr10 | 134362126 | 6 | N | cg09750646 | chr13 | 30088615 | 23 | N |
| cg10967866 | chr10 | 134362164 | 6 | MU | cg04769514 | chr13 | 30088640 | 23 | MU |
| cg19137806 | chr10 | 134362170 | 6 | N | cg25493901 | chr13 | 30088729 | 23 | N |
| cg09597767 | chr11 | 325915 | 6 | MU | cg02381064 | chr13 | 30088771 | 23 | N |
| cg03071500 | chr11 | 325964 | 6 | N | cg14213814 | chr13 | 49879669 | 23 | MU |
| cg02195201 | chr11 | 19135951 | 6 | MU | cg00758701 | chr13 | 49879718 | 23 | N |
| cg05300717 | chr11 | 65546210 | 6 | MU | cg01512719 | chr13 | 49879770 | 23 | MU |
| cg24368962 | chr11 | 111570978 | 6 | MU | cg03070534 | chr13 | 114930437 | 23 | MU |
| cg12401653 | chr12 | 1754980 | 6 | N | cg10348368 | chr14 | 105851883 | 23 | MU |
| cg23385838 | chr12 | 1755068 | 6 | MU | cg00479347 | chr14 | 105851951 | 23 | N |
| cg05691934 | chr12 | 1755162 | 6 | N | cg21903221 | chr14 | 105851981 | 23 | N |
| cg13610659 | chr12 | 56622608 | 6 | MU | cg06332339 | chr15 | 73889528 | 23 | MU |
| cg14642045 | chr12 | 109538736 | 6 | MU | cg08435157 | chr15 | 73889561 | 23 | N |
| cg02662180 | chr12 | 125482811 | 6 | MU | cg20268930 | chr15 | 101714939 | 23 | MU |
| eg02170785 | chr14 | 69650830 | 6 | MU | cg16628135 | chr16 | 1397791 | 23 | MU |
| cg13729548 | chr14 | 93405937 | 6 | MU | cg27431500 | chr16 | 1742281 | 23 | MU |
| cg05049312 | chr14 | 93406016 | 6 | N | cg00422714 | chr16 | 1814418 | 23 | MU |
| cg08087268 | chr14 | 93406082 | 6 | N | cg12384802 | chr16 | 1814481 | 23 | N |
| cg21871608 | chr14 | 93409468 | 6 | MU | cg05394010 | chr16 | 2546596 | 23 | MU |
| cg08253808 | chr14 | 102676957 | 6 | MU | cg02225054 | chr16 | 12210834 | 23 | MU |
| cg05398700 | chr14 | 102677141 | 6 | MU | cg27096032 | chr16 | 12210910 | 23 | MU |
| cg21510284 | chr14 | 103854927 | 6 | MU | cg04768745 | chr16 | 12211098 | 23 | MU |
| cg02072813 | chr14 | 105246532 | 6 | N | cg19390357 | chr16 | 12211186 | 23 | MU |
| cg10100767 | chr14 | 105246561 | 6 | MU | cg08842616 | chr16 | 70733772 | 23 | N |
| cg22870386 | chr14 | 105246708 | 6 | N | cg08796240 | chr16 | 70733832 | 23 | MU |
| cg21141030 | chr15 | 65576504 | 6 | MU | cg01033642 | chr16 | 81564192 | 23 | N |
| cg11183227 | chr15 | 91455407 | 6 | MU | cg04897892 | chr16 | 81564314 | 23 | MU |
| cg10342963 | chr15 | 99443213 | 6 | MU | cg01753252 | chr16 | 88757588 | 23 | MU |
| cg23990557 | chr15 | 99443249 | 6 | N | cg09786329 | chr16 | 88821445 | 23 | MU |
| cg26272088 | chr15 | 99443339 | 6 | N | cg09054528 | chr16 | 88821542 | 23 | N |
| cg21499918 | chr15 | 101169888 | 6 | N | cg02089452 | chr16 | 89500152 | 23 | MU |
| cg19619585 | chr15 | 101170019 | 6 | MU | cg02192318 | chr16 | 89500212 | 23 | N |
| cg01304182 | chr16 | 30409908 | 6 | N | cg12914530 | chr16 | 89500225 | 23 | N |
| cg00711896 | chr16 | 30410051 | 6 | MU | cg04833819 | chr17 | 927551 | 23 | N |
| cg12997627 | chr16 | 85676292 | 6 | N | cg21768956 | chr17 | 927652 | 23 | MU |
| cg08480774 | chr16 | 85676306 | 6 | MU | cg13483263 | chr17 | 7493150 | 23 | N |
| cg26648465 | chr16 | 85676412 | 6 | MU | cg13159929 | chr17 | 7493222 | 23 | MU |
| cg01582532 | chr16 | 85676526 | 6 | N | cg03164243 | chr17 | 17717894 | 23 | N |
| cg04567435 | chr16 | 85687652 | 6 | MU | cg04805065 | chr17 | 17717947 | 23 | N |
| cg02724461 | chr16 | 88905520 | 6 | MU | cg09186408 | chr17 | 17717967 | 23 | MU |
| cg09807356 | chr16 | 88905700 | 6 | MU | cg17714010 | chr17 | 78968450 | 23 | MU |
| cg20364025 | chr16 | 88905749 | 6 | N | cg26176014 | chr17 | 80491645 | 23 | N |
| cg16802439 | chr16 | 88907184 | 6 | MU | cg06503715 | chr17 | 80491688 | 23 | MU |
| cg03126713 | chr16 | 89299480 | 6 | MU | cg23515921 | chr17 | 80747189 | 23 | N |
| cg12116137 | chr17 | 1576449 | 6 | MU | cg18602928 | chr17 | 80747240 | 23 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg17441377 | chr17 | 3906640 | 6 | MU | cg19043450 | chr19 | 595705 | 23 | MU |
| cg01798813 | chr17 | 3906674 | 6 | N | cg07962882 | chr19 | 1117289 | 23 | MU |
| cg26624105 | chr17 | 29811956 | 6 | MU | cg09432775 | chr19 | 1117453 | 23 | MU |
| cg06943627 | chr17 | 47590179 | 6 | N | cg05919982 | chr19 | 1117571 | 23 | N |
| cg12561474 | chr17 | 47590237 | 6 | N | cg22672431 | chr19 | 1854549 | 23 | N |
| cg26102082 | chr17 | 47590272 | 6 | MU | cg08287334 | chr19 | 1854633 | 23 | MU |
| cg00590152 | chr17 | 74003831 | 6 | MU | cg01373896 | chr19 | 1854724 | 23 | N |
| cg11597902 | chr17 | 75096239 | 6 | N | cg13220129 | chr19 | 2046193 | 23 | N |
| cg11186858 | chr17 | 75096382 | 6 | MU | cg22811668 | chr19 | 2046303 | 23 | MU |
| cg06725287 | chr17 | 80533762 | 6 | MU | cg00869989 | chr19 | 2774197 | 23 | MU |
| cg22503106 | chr17 | 81040906 | 6 | MU | cg07586345 | chr19 | 2774332' | 23 | N |
| cg23060513 | chr19 | 13041124 | 6 | MU | cg07838427 | chr19 | 10665928 | 23 | MU |
| cg25739938 | chr2 | 9610621 | 6 | MU | cg22824635 | chr19 | 13112283 | 23 | MU |
| cg20460514 | chr2 | 11917490 | 6 | N | cg14697425 | chr19 | 14042840 | 23 | MU |
| cg10142874 | chr2 | 11917623 | 6 | MU | cg26961332 | chr19 | 18420631 | 23 | MU |
| cg01953317 | chr2 | 55642894 | 6 | MU | cg20535805 | chr19 | 18420735 | 23 | N |
| cg18769357 | chr2 | 68691913 | 6 | MU | cg15041373 | chr19 | 39577941 | 23 | MU |
| cg21346154 | chr2 | 70699808 | 6 | MU | cg26008272 | chr19 | 45574917 | 23 | N |
| cg10454864 | chr2 | 112654017 | 6 | MU | cg15732530 | chr19 | 45575023 | 23 | MU |
| cg06746096 | chr2 | 148976109 | 6 | MU | cg24030434 | chr19 | 45575134 | 23 | N |
| cg15609237 | chr2 | 179395885 | 6 | MU | cg23633937 | chr19 | 45575142 | 23 | N |
| cg19906284 | chr2 | 179395912 | 6 | N | cg17774001 | chr2 | 25600752 | 23 | MU |
| cg08691235 | chr2 | 179395935 | 6 | N | cg11960435 | chr20 | 31434982 | 23 | MU |
| cg15724534 | chr2 | 211018363 | 6 | MU | cg25478712 | chr21 | 44840120 | 23 | MU |
| cg00116699 | chr2 | 240205260 | 6 | N | cg20341238 | chr21 | 44865764 | 23 | MU |
| cg17285931 | chr2 | 240205408 | 6 | MU | cg27620176 | chr22 | 47054067 | 23 | MU |
| cg23039807 | chr2 | 240425897 | 6 | MU | cg26058289 | chr22 | 47054148 | 23 | MU |
| cg27637086 | chr2 | 240425955 | 6 | N | cg17213870 | chr22 | 47054273 | 23 | N |
| cg25525535 | chr20 | 32232310 | 6 | N | cg00678709 | chr3 | 14584745 | 23 | MU |
| cg19357305 | chr20 | 32232347 | 6 | MU | cg24508976 | chr3 | 14584778 | 23 | N |
| cg24259291 | chr20 | 47874072 | 6 | N | cg00011754 | chr3 | 14584788 | 23 | N |
| cg21781157 | chr20 | 47874111 | 6 | N | cg11865578 | chr3 | 47043996 | 23 | MU |
| cg01332882 | chr20 | 47874155 | 6 | MU | cg22607164 | chr3 | 184349562 | 23 | MU |
| cg15878616 | chr20 | 52492209 | 6 | MU | cg05485379 | chr3 | 197121410 | 23 | MU |
| cg11597277 | chr20 | 52492248 | 6 | N | cg25611057 | chr3 | 197121419 | 23 | N |
| cg00390694 | chr20 | 62700725 | 6 | MU | cg07349805, | chr4 | 714506 | 23 | N |
| cg25773447 | chr21 | 38574118 | 6 | N | cg04128884 | chr4 | 714516 | 23 | MU |
| cg12100242 | chr21 | 38574170 | 6 | MU | cg12201155 | chr4 | 1290568 | 23 | MU |
| cg18502618 | chr21 | 46902264 | 6 | MU | cg17924901 | chr4 | 3475233 | 23 | N |
| cg18869709 | chr22 | 43342938 | 6 | MU | cg05673770 | chr4 | 3475328 | 23 | N |
| cg18919659 | chr22 | 43343084 | 6 | N | cg21333208 | chr4 | 3475372 | 23 | MU |
| cg08086385 | chr3 | 72430211 | 6 | MU | cg01094351 | chr4 | 153179467 | 23 | MU |
| cg21792134 | chr3 | 121491382 | 6 | MU | cg11856821 | chr4 | 187540609 | 23 | MU |
| cg14516183 | chr3 | 122928186 | 6 | MU | cg18067236 | chr5 | 538582 | 23 | N |
| cg16050349 | chr3 | 138478129 | 6 | MU | cg27046920 | chr5 | 538702 | 23 | MU |
| cg17280398 | chr3 | 138478195 | 6 | N | cg16408279 | chr5 | 2466167 | 23 | MU |
| cg04855678 | chr3 | 195946921 | 6 | N | cg06728135 | chr5 | 2466293 | 23 | N |
| cg17572056 | chr3 | 195947062 | 6 | MU | cg23475112 | chr5 | 131725325 | 23 | MU |
| cg07641926 | chr3 | 195974258 | 6 | N | cg18679635 | chr6 | 6971315 | 23 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg02803925 | chr3 | 195974300 | 6 | MU | cg09146892 | chr6 | 6971335 | 23 | MU |
| cg00828556 | chr3 | 196351986 | 6 | MU | cg19751937 | chr6 | 6971371 | 23 | N |
| cg25771026 | chr3 | 196352027 | 6 | N | cg13887029 | chr6 | 31746795 | 23 | N |
| cg02722672 | chr4 | 2967106 | 6 | MU | cg12053296 | chr6 | 31746808 | 23 | MU |
| cg05736642 | chr4 | 3123132 | 6 | MU | cg12975324 | chr6 | 31746814 | 23 | MU |
| cg20107506 | chr4 | 100874826 | 6 | MU | cg16956876 | chr6 | 31746820 | 23 | N |
| cg20866785 | chr4 | 148733880 | 6 | MU | cg00180930 | chr6 | 31746832 | 23 | N |
| cg04476877 | chr5 | 43600777 | 6 | MU | cg08983186 | chr6 | 31746874 | 23 | N |
| cg17558547 | chr5 | 153836180 | 6 | MU | cg20067575 | chr6 | 150437072 | 23 | MU |
| cg18468726 | chr5 | 154314946 | 6 | MU | cg04076264 | chr6 | 150437118 | 23 | N |
| cg21237481 | chr5 | 172227661 | 6 | MU | cg02152290 | chr7 | 551601 | 23 | MU |
| cg20544852 | chr6 | 28874479 | 6 | MU | cg13381243 | chr7 | 1103063 | 23 | N |
| cg05314350 | chr6 | 28874702 | 6 | N | cg02332902 | chr7 | 1103177 | 23 | MU |
| cg02639080 | chr6 | 28874754 | 6 | MU | cg09526129 | chr7 | 1103262 | 23 | N |
| cg15090899 | chr6 | 167178260 | 6 | MU | cg16601832 | chr7 | 1514361 | 23 | N |
| cg05791862 | chr6 | 167189213 | 6 | N | cg23956190 | chr7 | 1514455 | 23 | MU |
| cg03329755 | chr6 | 167189272 | 6 | MU | cg14576951 | chr7 | 1514568 | 23 | N |
| cg00439981 | chr6 | 170684299 | 6 | MU | cg14108894 | chr7 | 2094628 | 23 | MU |
| cg18650626 | chr7 | 1914073 | 6 | MU | cg22717323 | chr7 | 2094977 | 23 | MU |
| cg03663120 | chr7 | 2284600 | 6 | MU | cg12950665 | chr7 | 4230985 | 23 | N |
| cg05861879 | chr7 | 2284723 | 6 | N | cg03976326 | chr7 | 4231054 | 23 | MU |
| cg25270424 | chr7 | 24965657 | 6 | MU | cg11985321 | chr7 | 158336016 | 23 | MU |
| cg16805291 | chr7 | 36022575 | 6 | MU | cg15654779 | chr8 | 1274678 | 23 | MU |
| cg21762695 | chr7 | 36022695 | 6 | N | cg22152500 | chr8 | 1274686 | 23 | N |
| cg20836212 | chr7 | 65419185 | 6 | N | cg01822827 | chr8 | 99180671 | 23 | N |
| cg25643253 | chr7 | 65419190 | 6 | N | cg03381604 | chr8 | 99180740 | 23 | MU |
| cg23090159 | chr7 | 65419194 | 6 | N | cg27000496 | chr8 | 142231004 | 23 | MU |
| cg12711054 | chr7 | 65419231 | 6 | N | cg20555854 | chr8 | 142231133 | 23 | N |
| cg21104363 | chr7 | 65419248 | 6 | N | cg25834632 | chr8 | 144590015 | 23 | MU |
| cg16760382 | chr7 | 65419261 | 6 | MU | cg09961689 | chr8 | 144590068 | 23 | N |
| cg04290133 | chr7 | 65439512 | 6 | MU | cg16888603 | chr8 | 144696651 | 23 | MU |
| cg07654282 | chr7 | 143700925 | 6 | N | cg10299941 | chr8 | 145009137 | 23 | MU |
| cg26425521 | chr7 | 143700939 | 6 | MU | cg08159271 | chr8 | 145009241 | 23 | N |
| cg05184016 | chr7 | 149543136 | 6 | MU | cg14071947 | chr9 | 136229324 | 23 | MU |
| cg07970948 | chr7 | 149543165 | 6 | N | cg14233878 | chr9 | 139546555 | 23 | MU |
| cg24576940 | chr7 | 150648283 | 6 | MU | cg16990083 | chr1 | 1606557 | 24 | MU |
| cg24836822 | chr7 | 150648840 | 6 | MU | cg24873695 | chr1 | 3123054 | 24 | MU |
| cg22528270 | chr7 | 151505116 | 6 | MU | cg03961030 | chr1 | 6333796 | 24 | N |
| cg23990334 | chr8 | 141046436 | 6 | N | cg06834177 | chr1 | 6333843 | 24 | MU |
| cg09377531 | chr8 | 141046469 | 6 | MU | cg15100947 | chr1 | 6333867 | 24 | N |
| cg02642126 | chr8 | 141046516 | 6 | MU | cg16245035 | chr1 | 10684392 | 24 | MU |
| cg13733787 | chr8 | 141229379 | 6 | N | cg24245726 | chr1 | 15375064 | 24 | N |
| cg16815625 | chr8 | 141229477 | 6 | MU | cg05264881 | chr1 | 15375148 | 24 | MU |
| cg24512544 | chr8 | 141588943 | 6 | MU | cg10761807 | chr1 | 15375232 | 24 | N |
| cg04836151 | chr8 | 144213766 | 6 | MU | cg05803741 | chr1 | 16202759 | 24 | N |
| cg13785123 | chr1 | 8931135 | 7 | MU | cg15135704 | chr1 | 16202851 | 24 | MU |
| cg20700740 | chr1 | 9339683 | 7 | MU | cg23216498 | chr1 | 16202869 | 24 | N |
| cg07033722 | chr1 | 40539032 | 7 | MU | cg10494257 | chr1 | 16342123 | 24 | N |
| cg15388975 | chr1 | 45120147 | 7 | N | cg24140574 | chr1 | 16342155 | 24 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg18315935 | chr1 | 45120295 | 7 | MU | cg06662140 | chr1 | 18809297 | 24 | MU |
| cg14028598 | chr1 | 171714300 | 7 | MU | cg16908901 | chr1 | 94054835 | 24 | MU |
| cg15520845 | chr1 | 205688760 | 7 | MU | cg14095720 | chr1 | 201195051 | 24 | MU |
| cg10826999 | chr1 | 225862288 | 7 | MU | cg09115646 | chr10 | 2978687 | 24 | MU |
| cg23128584 | chr10 | 323649 | 7 | MU | cg05289897 | chr10 | 3904141 | 24 | MU |
| cg11376341 | chr10 | 21796152 | 7 | MU | cg17141577 | chr10 | 12940536 | 24 | MU |
| cg23169883 | chr10 | 72435301 | 7 | MU | cg25894019 | chr10 | 115169101 | 24 | MU |
| cg01040749 | chr10 | 134400440 | 7 | MU | cg22605290 | chr10 | 130757223 | 24 | MU |
| cg09163720 | chr10 | 134400506 | 7 | N | cg16621196 | chr10 | 130757242 | 24 | N |
| cg09597767 | chr11 | 325915 | 7 | MU | cg17560832 | chr10 | 130757336 | 24 | N |
| cg03071500 | chr11 | 325964 | 7 | MU | cg03625662 | chr10 | 132891319 | 24 | MU |
| cg10611016 | chr11 | 6225759 | 7 | MU | cg10589396 | chr10 | 132891341 | 24 | N |
| cg05501357 | chr11 | 33308269 | 7 | N | cg15167871 | chr10 | 132891371 | 24 | N |
| cg25600606 | chr11 | 33308345 | 7 | MU | cg25718735 | chr10 | 132959070 | 24 | MU |
| cg11059944 | chr12 | 10183172 | 7 | N | cg08763309 | chr10 | 132959102 | 24 | N |
| cg17469479 | chr12 | 10183208 | 7 | MU | cg26202872 | chr10 | 132959127 | 24 | N |
| cg26262442 | chr12 | 10183220 | 7 | MU | cg10709708 | chr10 | 133458170 | 24 | MU |
| cg02930518 | chr12 | 10183234 | 7 | N | cg16644803 | chr10 | 133458190 | 24 | MU |
| cg20098659 | chr12 | 10183364 | 7 | N | cg24885631 | chr10 | 133751448 | 24 | N |
| cg10456459 | chr12 | 22843015 | 7 | MU | cg04090697 | chr10 | 133751502 | 24 | N |
| cg19055828 | chr12 | 51139321 | 7 | MU | cg17565051 | chr10 | 133751593 | 24 | MU |
| cg15029631 | chr12 | 52078762 | 7 | MU | cg14398860 | chr10 | 134491205 | 24 | MU |
| cg13610659 | chr12 | 56622608 | 7 | MU | cg15943038 | chr10 | 134491287 | 24 | N |
| cg24131359 | chr12 | 69346994 | 7 | MU | cg25745246 | chr10 | 134491331 | 24 | N |
| cg14642045 | chr12 | 109538736 | 7 | MU | cg04462650 | chr10 | 134610598 | 24 | MU |
| cg04255937 | chr12 | 122269675 | 7 | MU | cg15750941 | chr10 | 135002718 | 24 | MU |
| cg12389043 | chr12 | 124429228 | 7 | N | cg02474600 | chr10 | 135002852 | 24 | N |
| cg17723958 | chr12 | 124429295 | 7 | MU | cg21134090 | chr11 | 455955 | 24 | N |
| cg07276358 | chr13 | 114263342 | 7 | MU | cg16124920 | chr11 | 456063 | 24 | MU |
| cg07804973 | chr13 | 114263406 | 7 | MU | cg06415550 | chr11 | 456082 | 24 | N |
| cg14345154 | chr14 | 89299302 | 7 | MU | cg25909885 | chr11 | 19372012 | 24 | N |
| cg08253808 | chr14 | 102676957 | 7 | MU | cg24137774 | chr11 | 19372014 | 24 | MU |
| cg05398700 | chr14 | 102677141 | 7 | MU | cg13823144 | chr11 | 66475721 | 24 | MU |
| cg18812353 | chr15 | 56385430 | 7 | MU | cg19846609 | chr11 | 124999394 | 24 | MU |
| cg11183227 | chr15 | 91455407 | 7 | MU | cg00243187 | chr11 | 128746214 | 24 | MU |
| cg10342963 | chr15 | 99443213 | 7 | MU | cg18372740 | chr11 | 128746307 | 24 | N |
| cg23990557 | chr15 | 99443249 | 7 | N | cg25859670 | chr11 | 132031417 | 24 | MU |
| cg26272088 | chr15 | 99443339 | 7 | N | cg01140660 | chr11 | 133233200 | 24 | MU |
| cg12031275 | chr15 | 101093834 | 7 | MU | cg24859375 | chr12 | 7121914 | 24 | MU |
| cg21700663 | chr15 | 101093900 | 7 | N | cg14061772 | chr12 | 73266969 | 24 | MU |
| cg21499918 | chr15 | 101169888 | 7 | N | cg25769122 | chr12 | 132939657 | 24 | N |
| cg19619585 | chr15 | 101170019 | 7 | MU | cg10426234 | chr12 | 132939776 | 24 | MU |
| cg09050670 | chr16 | 1521617 | 7 | N | cg06509223 | chr13 | 23422746 | 24 | MU |
| cg05413628 | chr16 | 1521656 | 7 | MU | cg08418978 | chr13 | 96085969 | 24 | N |
| cg08810397 | chr16 | 1521676 | 7 | N | cg22122715 | chr13 | 96086023 | 24 | N |
| cg04354689 | chr16 | 2660830 | 7 | MU | cg20278383 | chr13 | 96086098 | 24 | MU |
| cg03571959 | chr16 | 2660876 | 7 | N | cg27586249 | chr13 | 96086182 | 24 | N |
| cg01735503 | chr16 | 69966815 | 7 | N | cg04157243 | chr13 | 113497537 | 24 | N |
| cg03549146 | chr16 | 69966902 | 7 | MU | cg20806040 | chr13 | 113497607 | 24 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg00158530 | chr16 | 69966973 | 7 | N | cg21148160 | chr14 | 73732130 | 24 | MU |
| cg08209934 | chr16 | 69966975 | 7 | N | cg01666796 | chr15 | 70364327 | 24 | MU |
| cg22381196 | chr16 | 72041376 | 7 | MU | cg12349571 | chr15 | 70364359 | 24 | N |
| cg16802439 | chr16 | 88907184 | 7 | MU | cg08983215 | chr16 | 2140326 | 24 | N |
| cg03126713 | chr16 | 89299480 | 7 | MU | cg10268345 | chr16 | 2140404 | 24 | MU |
| cg06561728 | chr17 | 650113 | 7 | N | cg05598886 | chr16 | 2140431 | 24 | N |
| cg19755813 | chr17 | 650208 | 7 | MU | cg07036112 | chr16 | 2140438 | 24 | N |
| cg12116137 | chr17 | 1576449 | 7 | MU | cg03928410 | chr16 | 15127121 | 24 | N |
| cg23770271 | chr17 | 3704471 | 7 | N | cg03245889 | chr16 | 15127172 | 24 | N |
| cg24738036 | chr17 | 3704476 | 7 | N | cg06978461 | chr16 | 15127193 | 24 | MU |
| cg24087438 | chr17 | 3704482 | 7 | N | cg26953293 | chr16 | 85684108 | 24 | MU |
| cg17074014 | chr17 | 3704494 | 7 | N | cg26967566 | chr16 | 85743863 | 24 | MU |
| cg13984928 | chr17 | 3704574 | 7 | MU | cg15446115 | chr16 | 85743927 | 24 | N |
| cg00463732 | chr17 | 3704621 | 7 | N | cg06593910 | chr16 | 88709927 | 24 | MU |
| cg17331920 | chr17 | 4081325 | 7 | MU | cg27092995 | chr16 | 88826522 | 24 | MU |
| cg13468144 | chr17 | 4081428 | 7 | MU | cg02154436 | chr16 | 88826569 | 24 | MU |
| cg02286380 | chr17 | 4621205 | 7 | MU | cg03273320 | chr17 | 7225152 | 24 | N |
| cg13466002 | chr17 | 4621252 | 7 | MU | cg04574095 | chr17 | 7225294 | 24 | MU |
| cg02675920 | chr17 | 78747934 | 7 | N | cg09632029 | chr17 | 18855506 | 24 | MU |
| cg11222173 | chr17 | 78748019 | 7 | MU | cg19893820 | chr17 | 46871794 | 24 | N |
| cg11153071 | chr17 | 78748077 | 7 | MU | cg00675156 | chr17 | 46871867 | 24 | MU |
| cg14343513 | chr17 | 78753273 | 7 | MU | cg24087796 | chr17 | 46871873 | 24 | N |
| cg16732654 | chr17 | 79129022 | 7 | N | cg08137716 | chr17 | 46871905 | 24 | N |
| cg23334433 | chr17 | 79129051 | 7 | N | cg16145211 | chr17 | 75695378 | 24 | MU |
| cg13618516 | chr17 | 79129078 | 7 | MU | cg08533865 | chr17 | 77084592 | 24 | MU |
| cg12019801 | chr18 | 74114708 | 7 | N | cg23836168 | chr17 | 77084618 | 24 | N |
| cg00471371 | chr18 | 74114728 | 7 | MU | cg12856760 | chr17 | 77976092 | 24 | MU |
| cg27535410 | chr19 | 846354 | 7 | MU | cg10407177 | chr18 | 34916713 | 24 | N |
| cg02715602 | chr19 | 4544446 | 7 | MU | cg20168823 | chr18 | 34916738 | 24 | MU |
| cg23060513 | chr19 | 13041124 | 7 | MU | cg12228319 | chr18 | 34916814 | 24 | N |
| cg04334723 | chr19 | 13054427 | 7 | N | cg00989538 | chr18 | 74832115 | 24 | N |
| cg25605731 | chr19 | 13054434 | 7 | MU | cg21116087 | chr18 | 74832171 | 24 | N |
| cg13466180 | chr19 | 13054470 | 7 | N | cg16990945 | chr18 | 74832261 | 24 | MU |
| cg25739938 | chr2 | 9610621 | 7 | MU | cg25332502 | chr18 | 74832372 | 24 | N |
| cg15609237 | chr2 | 179395885 | 7 | MU | cg20435284 | chr19 | 1166292 | 24 | MU |
| cg19906284 | chr2 | 179395912 | 7 | N | cg25367139 | chr19 | 1961185 | 24 | N |
| cg08691235 | chr2 | 179395935 | 7 | N | cg22278901 | chr19 | 1961290 | 24 | MU |
| cg26742320 | chr2 | 201393628 | 7 | MU | cg12249345 | chr19 | 45316493 | 24 | MU |
| cg15724534 | chr2 | 211018363 | 7 | MU | cg21978694 | chr19 | 45316509 | 24 | MU |
| cg15457217 | chr2 | 224780409 | 7 | MU | cg01743020 | chr2 | 1886086 | 24 | MU |
| cg06867755 | chr2 | 225397387 | 7 | MU | cg09927251 | chr2 | 1886105 | 24 | N |
| cg00116699 | chr2 | 240205260 | 7 | N | cg17162031 | chr2 | 1886127 | 24 | N |
| cg17285931 | chr2 | 240205408 | 7 | MU | cg02381965 | chr2 | 3418480 | 24 | MU |
| cg07901199 | chr2 | 242139830 | 7 | N | cg24929896 | chr2 | 3418530 | 24 | N |
| cg09520857 | chr2 | 242139924 | 7 | MU | cg19361181 | chr2 | 10616043 | 24 | MU |
| cg24259291 | chr20 | 47874072 | 7 | N | cg01286950 | chr2 | 100618399 | 24 | MU |
| cg21781157 | chr20 | 47874111 | 7 | N | cg18003518 | chr2 | 100618438 | 24 | MU |
| cg01332882 | chr20 | 47874155 | 7 | MU | cg03816062 | chr2 | 100618466 | 24 | MU |
| cg15878616 | chr20 | 52492209 | 7 | MU | cg19565305 | chr2 | 178973019 | 24 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg11597277 | chr20 | 52492248 | 7 | MU | cg06382167 | chr2 | 178973065 | 24 | N |
| cg18424841 | chr20 | 61315444 | 7 | MU | cg25207147 | chr2 | 178973079 | 24 | MU |
| cg05085169 | chr20 | 61443751 | 7 | MU | cg21488093 | chr2 | 178973092 | 24 | N |
| cg18502618 | chr21 | 46902264 | 7 | MU | cg26389888 | chr2 | 178973147 | 24 | N |
| cg18869709 | chr22 | 43342938 | 7 | MU | cg07211496 | chr2 | 178973164 | 24 | N |
| cg18919659 | chr22 | 43343084 | 7 | N | cg02800216 | chr2 | 237542624 | 24 | N |
| cg21792134 | chr3 | 121491382 | 7 | MU | cg20722637 | chr2 | 237542662 | 24 | N |
| cg14516183 | chr3 | 122928186 | 7 | MU | cg27104778 | chr2 | 237542706 | 24 | MU |
| cg25752703 | chr3 | 128710390 | 7 | MU | cg16900589 | chr2 | 238617188 | 24 | MU |
| cg04855678 | chr3 | 195946921 | 7 | N | cg06096175 | chr20 | 60701350 | 24 | MU |
| cg17572056 | chr3 | 195947062 | 7 | MU | cg24642039 | chr21 | 46845022 | 24 | N |
| cg00828556 | chr3 | 196351986 | 7 | MU | cg05349039 | chr21 | 46845110 | 24 | N |
| cg25771026 | chr3 | 196352027 | 7 | N | cg07529658 | chr21 | 46845160 | 24 | MU |
| cg18095675 | chr3 | 197272311 | 7 | MU | cg21429725 | chr3 | 196693809 | 24 | MU |
| cg20107506 | chr4 | 100874826 | 7 | MU | cg10152624 | chr4 | 1346939 | 24 | MU |
| cg01400750 | chr4 | 145956168 | 7 | MU | cg05410587 | chr4 | 1347008 | 24 | N |
| cg00992687 | chr5 | 54275155 | 7 | N | cg27630540 | chr4 | 1347166 | 24 | MU |
| cg09452568 | chr5 | 54275198 | 7 | MU | cg02897570 | chr4 | 1623761 | 24 | N |
| cg02368812 | chr6 | 3019900 | 7 | MU | cg01102826 | chr4 | 1623797 | 24 | N |
| cg14066298 | chr6 | 30297565 | 7 | N | cg13929734 | chr4 | 1623883 | 24 | MU |
| cg06230847 | chr6 | 30297594 | 7 | MU | cg01998801 | chr4 | 2396368 | 24 | N |
| cg01383911 | chr6 | 30297627 | 7 | N | cg13664931 | chr4 | 2396482 | 24 | MU |
| cg21426759 | chr6 | 30303126 | 7 | MU | cg08577953 | chr4 | 100574455 | 24 | MU |
| cg07875215 | chr6 | 157912969 | 7 | N | cg12097672 | chr4 | 100574527 | 24 | N |
| cg16679543 | chr6 | 157913024 | 7 | MU | cg09176893 | chr4 | 100574596 | 24 | MU |
| cg14514483 | chr6 | 158068455 | 7 | MU | cg17594047 | chr4 | 100574653 | 24 | N |
| cg21762728 | chr6 | 170165905 | 7 | MU | cg14574910 | chr5 | 2155809 | 24 | MU |
| cg00439981 | chr6 | 170684299 | 7 | MU | cg11835897 | chr5 | 2155934 | 24 | N |
| cg02827175 | chr7 | 1986245 | 7 | N | cg16158575 | chr5 | 2866388 | 24 | N |
| cg16993108 | chr7 | 1986301 | 7 | MU | cg13945749 | chr5 | 2866465 | 24 | MU |
| cg03116466 | chr7 | 2116512 | 7 | N | cg11143131 | chr5 | 131608246 | 24 | MU |
| cg22662844 | chr7 | 2116648 | 7 | MU | cg04794465 | chr6 | 31622326 | 24 | N |
| cg20782252 | chr7 | 2153774 | 7 | MU | cg06495310 | chr6 | 31622363 | 24 | MU |
| cg16688681 | chr7 | 2874522 | 7 | N | cg17634401 | chr6 | 31622439 | 24 | N |
| cg08377331 | chr7 | 2874595 | 7 | MU | cg08623515 | chr6 | 170565117 | 24 | MU |
| cg05031187 | chr7 | 73820613 | 7 | MU | cg21708145 | chr6 | 170565151 | 24 | N |
| cg01666985 | chr7 | 73820652 | 7 | N | cg01105362 | chr7 | 1059627 | 24 | MU |
| cg22528270 | chr7 | 151505116 | 7 | MU | cg15775406 | chr7 | 1333612 | 24 | MU |
| cg17090611 | chr8 | 17017866 | 7 | MU | cg21927314 | chr7 | 9056843 | 24 | MU |
| cg13733787 | chr8 | 141229379 | 7 | N | cg02650121 | chr7 | 36124562 | 24 | MU |
| cg16815625 | chr8 | 141229477 | 7 | MU | cg25203351 | chr7 | 36124652 | 24 | N |
| cg06623899 | chr8 | 142161840 | 7 | MU | cg16668896 | chr7 | 54900801 | 24 | N |
| cg04836151 | chr8 | 144213766 | 7 | MU | cg24317190 | chr7 | 54900936 | 24 | MU |
| cg13633625 | chr9 | 114706971 | 7 | MU | cg23123165 | chr7 | 54901015 | 24 | N |
| cg13876222 | chr9 | 139399348 | 7 | MU | cg14840782 | chr7 | 157280331 | 24 | MU |
| cg12065228 | chr1 | 19652788 | 8 | MU | cg22452170 | chr7 | 157427295 | 24 | MU |
| cg00308936 | chr1 | 85883665 | 8 | MU | cg03071124 | chr7 | 157447178 | 24 | N |
| cg25196715 | chr1 | 94489791 | 8 | MU | cg16796151 | chr7 | 157447244 | 24 | MU |
| cg11787626 | chr1 | 147233738 | 8 | MU | cg06684201 | chr7 | 157447249 | 24 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg21267551 | chr1 | 147233743 | 8 | N | cg10249637 | chr7 | 157655436 | 24 | MU |
| cg12740910 | chr1 | 147233887 | 8 | N | cg09941017 | chr7 | 157688504 | 24 | MU |
| cg24108508 | chr1 | 179016233 | 8 | MU | cg00403616 | chr8 | 591688 | 24 | MU |
| cg02814691 | chr1 | 182760900 | 8 | MU | cg00111335 | chr8 | 593194 | 24 | MU |
| cg06408559 | chr1 | 212963092 | 8 | MU | cg04179953 | chr8 | 593322 | 24 | N |
| cg13483984 | chr1 | 224580455 | 8 | MU | cg26463699 | chr8 | 1624741 | 24 | MU |
| cg23434428 | chr10 | 6557129 | 8 | MU | cg01154272 | chr8 | 1624818 | 24 | N |
| cg23293256 | chr10 | 11631072 | 8 | MU | cg17124844 | chr8 | 1624822 | 24 | N |
| cg19850275 | chr10 | 75654257 | 8 | MU | cg03899721 | chr8 | 8748792 | 24 | MU |
| cg15974642 | chr10 | 91137622 | 8 | MU | cg24998791 | chr8 | 10454100 | 24 | N |
| cg13363640 | chr11 | 2985302 | 8 | MU | cg07797693 | chr8 | 10454129 | 24 | MU |
| cg23580509 | chr11 | 94820250 | 8 | MU | cg08775375 | chr8 | 10454154 | 24 | N |
| cg19232164 | chr11 | 114320724 | 8 | MU | cg02517488 | chr8 | 10470600 | 24 | MU |
| cg07986378 | chr12 | 11898284 | 8 | MU | cg24196612 | chr8 | 21608193 | 24 | N |
| cg04141129 | chr12 | 32530696 | 8 | MU | cg15997512 | chr8 | 21608254 | 24 | N |
| cg03907313 | chr12 | 49087549 | 8 | MU | cg23716449 | chr8 | 21608280 | 24 | MU |
| cg00263619 | chr12 | 94727462 | 8 | MU | cg13294602 | chr8 | 141567779 | 24 | MU |
| cg08806644 | chr12 | 123632825 | 8 | MU | cg14642467 | chr9 | 139935042 | 24 | MU |
| cg10773745 | chr12 | 123633142 | 8 | MU | cg17006413 | chr1 | 942451 | 25 | N |
| cg21414595 | chr12 | 123633306 | 8 | MU | cg21472517 | chr1 | 942471 | 25 | MU |
| cg08221288 | chr14 | 50081280 | 8 | MU | cg18019572 | chr1 | 942489 | 25 | N |
| cg24733475 | chr14 | 64694047 | 8 | MU | cg22673826 | chr1 | 942498 | 25 | N |
| cg13578465 | chr14 | 105167457 | 8 | N | cg19081571 | chr1 | 1190137 | 25 | MU |
| cg27286011 | chr14 | 105167574 | 8 | N | cg13689260 | chr1 | 1190238 | 25 | N |
| cg11730703 | chr14 | 105167607 | 8 | MU | cg01602345 | chr1 | 3277525 | 25 | N |
| cg11031989 | chr15 | 66912788 | 8 | MU | cg21167817 | chr1 | 3277557 | 25 | MU |
| cg12099546 | chr15 | 72763849 | 8 | MU | cg01071314 | chr1 | 3277623 | 25 | MU |
| cg06093201 | chr16 | 1405095 | 8 | MU | cg03050669 | chr1 | 3606777 | 25 | N |
| cg03445653 | chr16 | 1405223 | 8 | MU | cg23163013 | chr1 | 3606892 | 25 | MU |
| cg06876040 | chr16 | 1462119 | 8 | MU | cg03846767 | chr1 | 3607292 | 25 | N |
| cg04858335 | chr16 | 8736327 | 8 | N | cg24878868 | chr1 | 3607401 | 25 | MU |
| cg26821542 | chr16 | 8736372 | 8 | N | cg26208930 | chr1 | 3607425 | 25 | N |
| cg09085220 | chr16 | 8736433 | 8 | MU | cg20628743 | chr1 | 3615715 | 25 | MU |
| cg04012618 | chr16 | 21548169 | 8 | MU | cg03043665 | chr1 | 3615762 | 25 | MU |
| cg27259391 | chr16 | 29314418 | 8 | MU | cg03890628 | chr1 | 9666902 | 25 | MU |
| cg04315923 | chr16 | 29634191 | 8 | MU | cg17244801 | chr1 | 10758237 | 25 | MU |
| cg07173484 | chr16 | 67237089 | 8 | MU | cg00922420 | chr1 | 55529158 | 25 | MU |
| cg08640475 | chr16 | 85551478 | 8 | MU | cg02863087 | chr1 | 180911577 | 25 | MU |
| cg10099827 | chr16 | 85551514 | 8 | MU | cg12462916 | chr1 | 220132315 | 25 | MU |
| cg04983687 | chr16 | 88558223 | 8 | MU | cg18381395 | chr1 | 236924323 | 25 | N |
| cg05958985 | chr16 | 88558237 | 8 | N | cg20282303 | chr1 | 236924392 | 25 | MU |
| cg06857116 | chr17 | 15885326 | 8 | MU | cg17219656 | chr1 | 236924443 | 25 | N |
| cg19135352 | chr17 | 16176891 | 8 | MU | cg24640577 | chr1 | 246939216 | 25 | MU |
| cg07050712 | chr17 | 47372126 | 8 | MU | cg06826710 | chr1 | 246939249 | 25 | N |
| cg21768884 | chr17 | 60770550 | 8 | MU | cg08442149 | chr10 | 1767484 | 25 | MU |
| cg07352016 | chr18 | 55250568 | 8 | MU | cg13728376 | chr10 | 5523245 | 25 | MU |
| cg21614107 | chr19 | 1633073 | 8 | MU | cg20691436 | chr10 | 5567478 | 25 | MU |
| cg26136776 | chr19 | 12998426 | 8 | N | cg09581935 | chr10 | 114591707 | 25 | N |
| cg11896104 | chr19 | 12998435 | 8 | MU | cg02571204 | chr10 | 114591733 | 25 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg01596520 | chr19 | 14225029 | 8 | N | cg11356398 | chr10 | 133783182 | 25 | N . |
| cg19586199 | chr19 | 14225172 | 8 | MU | cg18679487 | chr10 | 133783302 | 25 | MU |
| cg10668363 | chr19 | 52076691 | 8 | MU | cg21762589 | chr10 | 133796476 | 25 | N |
| cg25185536 | chr2 | 37642493 | 8 | MU | cg02835981 | chr10 | 133796494 | 25 | N |
| cg01953317 | chr2 | 55642894 | 8 | MU | cg13599131 | chr10 | 133796523 | 25 | N |
| cg03526652 | chr2 | 72977657 | 8 | MU | cg27353308 | chr10 | 133796559 | 25 | MU |
| cg10443146 | chr2 | 170779769 | 8 | MU | cg14275445 | chr10 | 134560047 | 25 | N |
| cg04498775 | chr21 | 44573062 | 8 | MU | cg08552167 | chr10 | 134560109 | 25 | MU |
| cg26420795 | chr21 | 47451611 | 8 | MU | cg00357546 | chr10 | 135032154 | 25 | MU |
| cg01500431 | chr22 | 24388327 | 8 | MU | cg12448312 | chr10 | 135032219 | 25 | N |
| cg15034267 | chr22 | 39106452 | 8 | MU | cg20285974 | chr11 | 526362 | 25 | MU |
| cg03325598 | chr3 | 12994877 | 8 | MU | cg03690133 | chr11 | 1483833 | 25 | N |
| cg05286196 | chr3 | 12994919 | 8 | MU | cg07335189 | chr11 | 1483973 | 25 | MU |
| cg02474195 | chr3 | 43391449 | 8 | MU | cg25819103 | chr11 | 63906050 | 25 | N |
| cg15856832 | chr3 | 49127364 | 8 | MU | cg04725041 | chr11 | 63906084 | 25 | N |
| cg23812679 | chr3 | 57985453 | 8 | MU | cg05697539 | chr11 | 63906104 | 25 | MU |
| cg09846625 | chr3 | 127305070 | 8 | MU | cg14139875 | chr11 | 66454595 | 25 | MU |
| cg10330847 | chr3 | 141031715 | 8 | MU | cg22306015 | chr11 | 66454625 | 25 | N |
| cg23144852 | chr3 | 193405999 | 8 | MU | cg04822177 | chr11 | 66454651 | 25 | N |
| cg18168751 | chr4 | 974821 | 8 | MU | cg26906629 | chr11 | 68564367 | 25 | MU |
| cg04276669 | chr4 | 26319867 | 8 | MU | cg06042565 | chr11 | 68564477 | 25 | N |
| cg09797433 | chr4 | 87938832 | 8 | MU | cg12263469 | chr11 | 69468863 | 25 | N |
| cg09710521 | chr4 | 103605622 | 8 | MU | cg00953256 | chr11 | 69468875 | 25 | N |
| cg12006794 | chr4 | 156679085 | 8 | MU | cg02723533 | chr11 | 69468991 | 25 | MU |
| cg00916454 | chr4 | 156877765 | 8 | MU | cg00347938 | chr11 | 69469113 | 25 | MU |
| cg17050984 | chr5 | 14397222 | 8 | MU | cg11802013 | chr11 | 69469175 | 25 | N |
| cg05893843 | chr5 | 119846291 | 8 | MU | cg01176141 | chr11 | 70023032 | 25 | MU |
| cg27144766 | chr5 | 150604977 | 8 | MU | cg13089599 | chr11 | 70023060 | 25 | MU |
| cg14522097 | chr5 | 150605036 | 8 | N | cg24832721 | chr11 | 70023142 | 25 | N |
| cg16666745 | chr5 | 153753045 | 8 | MU | cg14498227 | chr11 | 70282774 | 25 | MU |
| cg03967329 | chr6 | 26030329 | 8 | MU | cg13659360 | chr11 | 121023627 | 25 | MU |
| cg26811065 | chr6 | 32825360 | 8 | MU | cg24327383 | chr11 | 121023708 | 25 | N |
| cg08209711 | chr6 | 32825499 | 8 | N | cg13751188 | chr11 | 121023720 | 25 | N |
| cg26888623 | chr6 | 37871320 | 8 | MU | cg07259651 | chr11 | 129802069 | 25 | MU |
| cg02923490 | chr6 | 37872019 | 8 | MU | cg18725348 | chr12 | 131493258 | 25 | MU |
| cg05477517 | chr6 | 164531576 | 8 | MU | cg11113216 | chr12 | 132847641 | 25 | MU |
| cg05279229 | chr7 | 1896384 | 8 | MU | cg04053571 | chr12 | 132847770 | 25 | N |
| cg08858130 | chr7 | 2124459 | 8 | N | cg10130374 | chr13 | 111935412 | 25 | N |
| cg19382157 | chr7 | 2124566 | 8 | MU | cg22795345 | chr13 | 111935508 | 25 | MU |
| cg09768617 | chr7 | 27908936 | 8 | MU | cg14270002 | chr13 | 111935521 | 25 | MU |
| cg23635728 | chr7 | 33765409 | 8 | MU | cg22032283 | chr13 | 111936044 | 25 | N |
| cg20836212 | chr7 | 65419185 | 8 | MU | cg12512173 | chr13 | 111936059 | 25 | N |
| cg25643253 | chr7 | 65419190 | 8 | N | cg21613549 | chr13 | 111936071 | 25 | MU |
| cg23090159 | chr7 | 65419194 | 8 | N | cg03616722 | chr13 | 112602415 | 25 | MU |
| cg12711054 | chr7 | 65419231 | 8 | N | cg03784113 | chr13 | 112602492 | 25 | N |
| cg21104363 | chr7 | 65419248 | 8 | N | cg09473364 | chr13 | 112927190 | 25 | MU |
| cg16760382 | chr7 | 65419261 | 8 | MU | cg07402939 | chr13 | 112927331 | 25 | N |
| cg12349350 | chr7 | 101723836 | 8 | N | cg22612590 | chr13 | 114187973 | 25 | N |
| cg14203426 | chr7 | 101723947 | 8 | MU | cg13241681 | chr13 | 114187990 | 25 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg09761503 | chr7 | 142659349 | 8 | N | cg20539175 | chr13 | 114188051 | 25 | N |
| cg26806603 | chr7 | 142659378 | 8 | N | cg01889306 | chr14 | 91738961 | 25 | N |
| cg17784922 | chr7 | 142659425 | 8 | MU | cg26281789 | chr14 | 91739020 | 25 | MU |
| cg05184016 | chr7 | 149543136 | 8 | MU | cg25965286 | chr14 | 91739097 | 25 | N |
| cg07970948 | chr7 | 149543165 | 8 | MU | cg04043826 | chr14 | 91739105 | 25 | N |
| cg22987457 | chr7 | 157211561 | 8 | N | cg05100432 | chr14 | 104889005 | 25 | N |
| cg05658215 | chr7 | 157211628 | 8 | N | cg00912247 | chr14 | 104889116 | 25 | MU |
| cg07338119 | chr7 | 157211683 | 8 | MU | cg08057475 | chr15 | 45803341 | 25 | MU |
| cg04198125 | chr7 | 157211777 | 8 | MU | cg20268930 | chr15 | 101714939 | 25 | MU |
| cg03184452 | chr8 | 13105155 | 8 | MU | cg03046466 | chr16 | 1005503 | 25 | MU |
| cg26172342 | chr8 | 41654331 | 8 | N | cg13706784 | chr16 | 1014975 | 25 | N |
| cg17052552 | chr8 | 41654404 | 8 | MU | cg02612270 | chr16 | 1015103 | 25 | MU |
| cg08923054 | chr8 | 41654455 | 8 | MU | cg16628135 | chr16 | 1397791 | 25 | MU |
| cg14030674 | chr8 | 41654557 | 8 | N | cg04099705 | chr16 | 1397981 | 25 | MU |
| cg17203290 | chr8 | 99105576 | 8 | MU | cg19494587 | chr16 | 1398004 | 25 | N |
| cg09041614 | chr8 | 121417181 | 8 | MU | cg06698598 | chr16 | 1398226 | 25 | MU |
| cg22353369 | chr8 | 128972450 | 8 | MU | cg04649852 | chr16 | 29229650 | 25 | MU |
| cg01499988 | chr9 | 35755346 | 8 | MU | cg00535785 | chr16 | 49766785 | 25 | MU |
| cg04478751 | chr9 | 114243408 | 8 | MU | cg02501155 | chr16 | 79263473 | 25 | MU |
| cg13545717 | chr9 | 126585875 | 8 | MU | cg10221896 | chr16 | 79263595 | 25 | N |
| cg13928485 | chr9 | 136726359 | 8 | MU | cg01033642 | chr16 | 81564192 | 25 | N |
| eg10317445 | chr1 | 1685553 | 9 | MU | cg04897892 | chr16 | 81564314 | 25 | MU |
| cg22851557 | chr1 | 3038068 | 9 | N | cg03558269 | chr16 | 88217830 | 25 | MU |
| cg01772149 | chr1 | 3038216 | 9 | MU | cg06561728 | chr17 | 650113 | 25 | N |
| cg01203812 | chr1 | 3081078 | 9 | N | cg19755813 | chr17 | 650208 | 25 | MU |
| cg01413848 | chr1 | 3081226 | 9 | MU | cg24060527 | chr17 | 9066156 | 25 | MU |
| cg06723421 | chr1 | 8384440 | 9 | N | cg20705392 | chr17 | 9066278 | 25 | N |
| cg14038482 | chr1 | 8384484 | 9 | MU | cg12601118 | chr17 | 9859123 | 25 | MU |
| cg15575320 | chr1 | 8384513 | 9 | N | cg24543937 | chr17 | 71282102 | 25 | N |
| cg07958878 | chr1 | 8384595 | 9 | N | cg11966886 | chr17 | 71282180 | 25 | MU |
| cg20163085 | chr1 | 10510396 | 9 | MU | cg01449677 | chr17 | 76794888 | 25 | MU |
| cg04256470 | chr1 | 10510465 | 9 | N | cg04117396 | chr17 | 76795028 | 25 | N |
| cg25407372 | chr1 | 10510499 | 9 | N | cg20651080 | chr17 | 80019526 | 25 | MU |
| cg27290274 | chr1 | 15386739 | 9 | MU | cg03571301 | chr17 | 80019566 | 25 | N |
| cg23262325 | chr1 | 15386798 | 9 | N | cg19248659 | chr17 | 80019627 | 25 | N |
| cg00953309 | chr1 | 16460784 | 9 | MU | cg19869698 | chr17 | 80058686 | 25 | N |
| cg12593873 | chr1 | 27111162 | 9 | MU | cg19223190 | chr17 | 80058835 | 25 | MU |
| cg23022656 | chr1 | 42628585 | 9 | MU | cg13939156 | chr17 | 80058883 | 25 | N |
| cg25729585 | chr1 | 203056475 | 9 | MU | cg23515921 | chr17 | 80747189 | 25 | N |
| cg15470344 | chr1 | 203056504 | 9 | N | cg18602928 | chr17 | 80747240 | 25 | MU |
| cg26601431 | chr1 | 235054899 | 9 | MU | cg07114009 | chr17 | 80797948 | 25 | N |
| cg17522332 | chr10 | 1707523 | 9 | MU | cg10828599 | chr17 | 80797983 | 25 | N |
| cg25379549 | chr10 | 1707569 | 9 | MU | cg13616930 | chr17 | 80797991 | 25 | N |
| cg05721751 | chr10 | 1707576 | 9 | MU | cg02384546 | chr17 | 80798013 | 25 | N |
| cg10291105 | chr10 | 3465814 | 9 | MU | cg27285599 | chr17 | 80798023 | 25 | N |
| cg17224613 | chr10 | 30248585 | 9 | N | cg03854913 | chr17 | 80798068 | 25 | MU |
| cg00881448 | chr10 | 30248683 | 9 | MU | cg04934595 | chr17 | 81021419 | 25 | MU |
| cg25418210 | chr10 | 30248738 | 9 | N | cg14515064 | chr17 | 81021472 | 25 | N |
| cg06263395 | chr10 | 46195640 | 9 | MU | cg17969902 | chr19 | 1274584 | 25 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg06518233 | chr10 | 102765350 | 9 | N | cg17760058 | chr19 | 2321626 | 25 | N |
| cg18326783 | chr10 | 102765364 | 9 | N | cg15922228 | chr19 | 2321726 | 25 | N |
| cg05229649 | chr10 | 102765404 | 9 | MU | cg22849149 | chr19 | 2321754 | 25 | MU |
| cg25093234 | chr11 | 3114761 | 9 | MU | cg00869989 | chr19 | 2774197 | 25 | MU |
| cg24759599 | chr11 | 3114884 | 9 | N | cg07586345 | chr19 | 2774332 | 25 | N |
| cg11755933 | chr11 | 67266128 | 9 | N | cg03358468 | chr19 | 5060985 | 25 | N |
| cg10853830 | chr11 | 67266176 | 9 | MU | cg22079102 | chr19 | 5061085 | 25 | MU |
| cg06210783 | chr11 | 68205335 | 9 | MU | cg00513611 | chr19 | 5061205 | 25 | N |
| cg13052101 | chr11 | 68517529 | 9 | MU | cg07838427 | chr19 | 10665928 | 25 | MU |
| cg21917524 | chr11 | 74200334 | 9 | MU | cg14683138 | chr19 | 10781796 | 25 | MU |
| cg11351958 | chr11 | 75277791 | 9 | MU | cg26539736 | chr2 | 10666585 | 25 | MU |
| cg14849578 | chr12 | 125282480 | 9 | N | cg10767193 | chr2 | 10666662 | 25 | N |
| cg25283432 | chr12 | 125282561 | 9 | MU | cg08846959 | chr2 | 10666713 | 25 | N |
| cg07805940 | chr12 | 130909013 | 9 | MU | cg17016175 | chr2 | 98507477 | 25 | MU |
| cg19946641 | chr12 | 130909093 | 9 | N | cg24586870 | chr2 | 233198590 | 25 | MU |
| cg17380943 | chr13 | 99100506 | 9 | N | cg13393809 | chr2 | 239974467 | 25 | N |
| cg15168958 | chr13 | 99100528 | 9 | MU | cg10067182 | chr2 | 239974554 | 25 | MU |
| cg20750642 | chr13 | 99100586 | 9 | N | cg21478921 | chr2 | 242617935 | 25 | MU |
| cg04843615 | chr13 | 113613229 | 9 | N | cg03301433 | chr2 | 242617946 | 25 | N |
| cg04081844 | chr13 | 113613272 | 9 | N | cg09561365 | chr22 | 45676755 | 25 | MU |
| cg26080331 | chr13 | 113613318 | 9 | MU | cg27620176 | chr22 | 47054067 | 25 | N |
| cg18110590 | chr13 | 113636679 | 9 | MU | cg26058289 | chr22 | 47054148 | 25 | MU |
| cg16325826 | chr13 | 113719136 | 9 | MU | cg17213870 | chr22 | 47054273 | 25 | N |
| cg25307724 | chr13 | 113719172 | 9 | N | cg00678709 | chr3 | 14584745 | 25 | MU |
| cg04708126 | chr13 | 113719440 | 9 | MU | cg24508976 | chr3 | 14584778 | 25 | N |
| cg18327049 | chr14 | 103470338 | 9 | MU | cg00011754 | chr3 | 14584788 | 25 | N |
| cg20640374 | chr15 | 31355304 | 9 | MU | cg05485379 | chr3 | 197121410 | 25 | MU |
| cg24957518 | chr15 | 31355362 | 9 | N | cg25611057 | chr3 | 197121419 | 25 | N |
| cg12241333 | chr15 | 31355430 | 9 | N | cg12201155 | chr4 | 1290568 | 25 | MU |
| cg21555471 | chr15 | 41060201 | 9 | MU | cg13576505 | chr4 | 12611355 | 25 | MU |
| cg03049323 | chr15 | 41060265 | 9 | N | cg09559196 | chr5 | 149375082 | 25 | MU |
| cg05620415 | chr16 | 1961808 | 9 | N | cg07858195 | chr5 | 177853481 | 25 | MU |
| cg00972522 | chr16 | 1961953 | 9 | MU | cg18009093 | chr5 | 177853574 | 25 | N |
| cg06445533 | chr16 | 2114118 | 9 | N | cg05798147 | chr6 | 127781063 | 25 | MU |
| cg06991955 | chr16 | 2114232 | 9 | MU | cg22004774 | chr6 | 137495354 | 25 | MU |
| cg06912227 | chr16 | 4053409 | 9 | MU | cg14553824 | chr6 | 137495383 | 25 | N |
| cg03057173 | chr16 | 57552056 | 9 | MU | cg20067575 | chr6 | 150437072 | 25 | MU |
| cg10246581 | chr16 | 81536171 | 9 | MU | cg04076264 | chr6 | 150437118 | 25 | N |
| cg00838040 | chr16 | 84446919 | 9 | MU | cg02152290 | chr7 | 551601 | 25 | MU |
| cg04624689 | chr16 | 85690349 | 9 | MU | cg15879716 | chr7 | 1932272 | 25 | MU |
| cg07891658 | chr16 | 87996731 | 9 | N | cg26918117 | chr7 | 101603298 | 25 | N |
| cg03755748 | chr16 | 87996816 | 9 | N | cg05855116 | chr7 | 101603335 | 25 | MU |
| cg16659510 | chr16 | 87996870 | 9 | MU | cg10243459 | chr7 | 157584081 | 25 | MU |
| cg03034934 | chr16 | 88965044 | 9 | MU | og26431815 | chr8 | 668053 | 25 | MU |
| cg02627286 | chr16 | 89590583 | 9 | MU | cg03899721 | chr8 | 8748792 | 25 | MU |
| cg13312036 | chr17 | 1386274 | 9 | MU | cg17644611 | chr8 | 17549768 | 25 | MU |
| cg02104967 | chr17 | 1386368 . | 9 | MU | cg05744888 | chr8 | 141588118 | 25 | N |
| cg06651376 | chr17 | 1690794 | 9 | MU | cg23287005 | chr8 | 141588165 | 25 | MU |
| cg03531984 | chr17 | 3500374 | 9 | MU | cg05827089 | chr8 | 141588242 | 25 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg14762845 | chr17 | 3500390 | 9 | N | cg27000496 | chr8 | 142231004 | 25 | MU |
| cg18100532 | chr17 | 3500408 | 9 | N | cg20555854 | chr8 | 142231133 | 25 | N |
| cg11106060 | chr17 | 3500417 | 9 | N | cg01716084 | chr8 | 142367700 | 25 | N |
| cg13158990 | chr17 | 21101960 | 9 | MU | cg22979083 | chr8 | 142367795 | 25 | MU |
| cg02301815 | chr17 | 44249491 | 9 | N | cg14377739 | chr9 | 139699037 | 25 | MU |
| cg26855231 | chr17 | 44249543 | 9 | MU | cg02971920 | chr1 | 1095607 | 26 | MU |
| cg14374015 | chr17 | 44249569 | 9 | N | cg06767191 | chr1 | 1095737 | 26 | N |
| cg05104283 | chr17 | 75463860 | 9 | MU | cg19900387 | chr1 | 6533368 | 26 | MU |
| cg21808045 | chr17 | 75463984 | 9 | N | cg09022584 | chr1 | 15541956 | 26 | MU |
| cg08115732 | chr17 | 76171794 | 9 | N | cg10538929 | chr1 | 22149812 | 26 | MU |
| cg00715343 | chr17 | 76171845 | 9 | MU | cg12462916 | chr1 | 220132315 | 26 | MU |
| cg05837235 | chr17 | 77043815 | 9 | MU | cg05394294 | chr10 | 3160926 | 26 | N |
| cg11274148 | chr17 | 78876656 | 9 | MU | cg15319824 | chr10 | 3160974 | 26 | N |
| cg01412400 | chr17 | 78876726 | 9 | N | cg02579140 | chr10 | 3160982 | 26 | MU |
| cg12131324 | chr17 | 78876794 | 9 | N | cg14068301 | chr10 | 3161106 | 26 | N |
| cg12602405 | chr17 | 79456824 | 9 | MU | cg18103236 | chr10 | 81060645 | 26 | MU |
| cg14247862 | chr17 | 80764631 | 9 | MU | cg05655953 | chr10 | 123810032 | 26 | MU |
| cg13280079 | chr17 | 80764739 | 9 | N | cg18049513 | chr11 | 541660 | 26 | MU |
| cg24777505 | chr17 | 80829604 | 9 | N | cg08373573 | chr11 | 1028291 | 26 | MU |
| cg22432760 | chr17 | 80829718 | 9 | MU | cg20625588 | chr11 | 1028356 | 26 | N |
| cg14393952 | chr18 | 56439236 | 9 | MU | cg01182863 | chr11 | 1464792 | 26 | MU |
| cg02854447 | chr19 | 1377113 | 9 | N | cg04925032 | chr11 | 1847227 | 26 | N |
| cg08502383 | chr19 | 1377233 | 9 | MU | cg26805905 | chr11 | 1847267 | 26 | N |
| cg12257170 | chr19 | 2222026 | 9 | MU | cg23726178 | chr11 | 1847307 | 26 | MU |
| cg06678890 | chr19 | 2222224 | 9 | MU | cg03529598 | chr11 | 1847357 | 26 | MU |
| cg22001208 | chr19 | 18782374 | 9 | MU | cg16718999 | chr11 | 64108790 | 26 | MU |
| cg12218126 | chr19 | 42912486 | 9 | MU | cg08904394 | chr11 | 64108902 | 26 | N |
| cg01044465 | chr19 | 42914685 | 9 | MU | cg08707110 | chr11 | 65620170 | 26 | N |
| cg01537765 | chr19 | 42914828 | 9 | MU | cg01068102 | chr11 | 65620199 | 26 | MU |
| cg09377572 | chr19 | 48220075 | 9 | N | cg18262591 | chr11 | 68579970 | 26 | MU |
| cg14043593 | chr19 | 48220211 | 9 | MU | cg03855388 | chr11 | 68580018 | 26 | MU |
| cg17091056 | chr2 | 54315563 | 9 | MU | cg20629021 | chr11 | 68580041 | 26 | N |
| cg11135381 | chr2 | 86255654 | 9 | N | cg01378512 | chr11 | 126055457 | 26 | MU |
| cg24062526 | chr2 | 86255804 | 9 | MU | cg02643834 | chr11 | 130060371 | 26 | N |
| cg07949777 | chr2 | 128388876 | 9 | MU | cg11069338 | chr11 | 130060459 | 26 | MU |
| cg23046231 | chr2 | 201342582 | 9 | MU | cg08162124 | chr13 | 113428094 | 26 | MU |
| cg24746666 | chr2 | 201342647 | 9 | N | cg21897676 | chr13 | 113428161 | 26 | MU |
| cg15982130 | chr2 | 236300720 | 9 | N | cg01831771 | chr13 | 113428172 | 26 | MU |
| cg07055259 | chr2 | 236300855 | 9 | MU | cg16100355 | chr13 | 113648607 | 26 | MU |
| cg14212402 | chr2 | 236300937 | 9 | N | cg12947224 | chr13 | 114189945 | 26 | MU |
| cg01879273 | chr2 | 236506413 | 9 | MU | cg09537621 | chr13 | 114189998 | 26 | N |
| cg17858500 | chr20 | 3734725 | 9 | MU | cg02034328 | chr14 | 77968467 | 26 | MU |
| cg08722200 | chr20 | 55960520 | 9 | MU | cg00931124 | chr16 | 967929 | 26 | MU |
| cg06544464 | chr22 | 39627591 | 9 | N | cg07702526 | chr16 | 967972 | 26 | MU |
| cg08255187 | chr22 | 39627689 | 9 | MU | cg02200944 | chr16 | 968024 | 26 | N |
| cg18045556 | chr22 | 39627786 | 9 | MU | cg04454872 | chr16 | 968682 | 26 | MU |
| cg05643613 | chr3 | 11592649 | 9 | MU | cg27578227 | chr16 | 968787 | 26 | N |
| cg13496790 | chr3 | 66455738 | 9 | MU | cg01149677 | chr16 | 1808230 | 26 | MU |
| cg04056669 | chr3 | 195614059 | 9 | MU | cg10108042 | chr16 | 1808309 | 26 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg10966208 | chr4 | 866710 | 9 | N | cg02520816 | chr16 | 4056403 | 26 | MU |
| cg03307425 | chr4 | 866811 | 9 | MU | cg05599155 | chr16 | 29188654 | 26 | MU |
| cg27574071 | chr4 | 1219498 | 9 | MU | cg09602020 | chr16 | 29188796 | 26 | N |
| cg00667841 | chr4 | 3308884 | 9 | N | cg00284420 | chr16 | 87311948 | 26 | MU |
| cg22770294 | chr4 | 3308933 | 9 | MU | cg01579218 | chr16 | 87312047 | 26 | N |
| cg22389275 | chr4 | 3309013 | 9 | N | cg04286195 | chr16 | 88099702 | 26 | MU |
| cg16371080 | chr4 | 3309069 | 9 | N | cg05762553 | chr16 | 88099722 | 26 | N |
| cg26065792 | chr5 | 4023752 | 9 | MU | cg06969933 | chr16 | 88099740 | 26 | N |
| cg02353814 | chr5 | 135146660 | 9 | MU | cg17633681 | chr16 | 88106987 | 26 | N |
| cg23949969 | chr5 | 180219412 | 9 | MU | cg03395651 | chr16 | 88107091 | 26 | MU |
| cg20346284 | chr6 | 3280503 | 9 | N | cg10398761 | chr16 | 89169289 | 26 | MU |
| cg12034847 | chr6 | 3280558 | 9 | N | cg10303721 | chr16 | 89674435 | 26 | N |
| cg14660539 | chr6 | 3280635 | 9 | MU | cg03664916 | chr16 | 89674560 | 26 | MU |
| cg27083825 | chr6 | 158453594 | 9 | MU | cg23098912 | chr17 | 5993686 | 26 | MU |
| cg22180298 | chr6 | 163731903 | 9 | N | cg06421238 | chr17 | 5993785 | 26 | MU |
| cg09577425 | chr6 | 163731978 | 9 | MU | cg24096540 | chr17 | 12695725 | 26 | MU |
| cg11387916 | chr6 | 169273332 | 9 | N | cg24037380 | chr17 | 73727879 | 26 | N |
| cg05740106 | chr6 | 169273346 | 9 | MU | cg26314765 | chr17 | 73727957 | 26 | MU |
| cg20876525 | chr6 | 169273382 | 9 | N | cg20942310 | chr17 | 73728061 | 26 | N |
| cg19043969 | chr7 | 1162860 | 9 | N | cg17838182 | chr17 | 76991208 | 26 | MU |
| cg24530527 | chr7 | 1162932 | 9 | MU | cg25052374 | chr17 | 76991253 | 26 | MU |
| cg05342467 | chr7 | 2029732 | 9 | MU | cg23911097 | chr17 | 76991378 | 26 | MU |
| cg19601293 | chr7 | 2029752 | 9 | MU | cg24844295 | chr17 | 78821928 | 26 | MU |
| cg18641463 | chr7 | 2144579 | 9 | MU | cg16366685 | chr17 | 80535398 | 26 | N |
| cg06231385 | chr7 | 4743056 | 9 | MU | cg03454705 | chr17 | 80535428 | 26 | MU |
| cg08447571 | chr7 | 4743074 | 9 | N | cg00137385 | chr18 | 46288005 | 26 | N |
| cg19080320 | chr7 | 4743346 | 9 | N | cg21150921 | chr18 | 46288073 | 26 | MU |
| cg11516540 | chr7 | 4743394 | 9 | MU | cg22483095 | chr19 | 1112679 | 26 | MU |
| cg14012264 | chr7 | 4743426 | 9 | N | cg24361823 | chr19 | 1112732 | 26 | N |
| cg22758834 | chr7 | 95222104 | 9 | MU | cg27594157 | chr19 | 1235003 | 26 | MU |
| cg14329833 | chr7 | 134850443 | 9 | MU | cg03284113 | chr19 | 1235093 | 26 | N |
| cg27279876 | chr8 | 49702292 | 9 | N | cg27341866 | chr19 | 2278618 | 26 | MU |
| cg04234758 | chr8 | 49702304 | 9 | MU | cg07595134 | chr19 | 2278708 | 26 | MU |
| cg10673192 | chr8 | 140749308 | 9 | N | cg08399733 | chr19 | 2278773 | 26 | N |
| cg26328150 | chr8 | 140749377 | 9 | MU | cg17437218 | chr19 | 2278847 | 26 | N |
| cg14284797 | chr9 | 136545056 | 9 | MU | cg13465645 | chr19 | 3578711 | 26 | MU |
| cg11754357 | chr1 | 1046230 | 10 | N | cg05574870 | chr19 | 4054124 | 26 | N |
| cg05527091 | chr1 | 1046324 | 10 | MU | cg27196194 | chr19 | 4054253 | 26 | MU |
| cg02300356 | chr1 | 2027983 | 10 | MU | cg12028674 | chr19 | 39306244 | 26 | MU |
| cg16264537 | chr1 | 4468051 | 10 | N | cg24478966 | chr19 | 42460930 | 26 | MU |
| cg04571522 | chr1 | 4468089 | 10 | MU | cg15706424 | chr2 | 469781 | 26 | N |
| cg13934553 | chr1 | 6530615 | 10 | MU | cg08131059 | chr2 | 469790 | 26 | MU |
| cg08091596 | chr1 | 6530743 | 10 | | cg10900049 | chr2 | 8849962 | 26 | MU |
| cg10710472 | chr1 | 8394347 | 10 | N | cg00240378 | chr2 | 8850020 | 26 | MU |
| cg25767112 | chr1 | 8394362 | 10 | N | cg03448016 | chr2 | 8850066 | 26 | N |
| cg22352169 | chr1 | 8394401 | 10 | MU | cg12793924 | chr2 | 17830624 | 26 | MU |
| cg26428731 | chr1 | 35334352 | 10 | N | cg10907356 | chr2 | 17830694 | 26 | N |
| cg04031507 | chr1 | 35334359 | 10 | MU | cg27656592 | chr2 | 17830761 | 26 | N |
| cg09758896 | chr10 | 131694575 | 10 | N | cg19953799 | chr2 | 97427733 | 26 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg20800606 | chr10 | 131694675 | 10 | N | cg12942038 | chr2 | 97427895 | 26 | N |
| cg26275858 | chr10 | 131694689 | 10 | MU | cg11464842 | chr2 | 97427975 | 26 | MU |
| cg19003390 | chr10 | 133948441 | 10 | MU | cg03532037 | chr2 | 106959205 | 26 | N |
| cg03872339 | chr10 | 133948517 | 10 | N | cg24419520 | chr2 | 106959257 | 26 | MU |
| cg27617132 | chr10 | 134543871 | 10 | MU | cg08093959 | chr2 | 106959853 | 26 | N |
| cg09563846 | chr11 | 875758 | 10 | MU | cg15139063 | chr2 | 106959868 | 26 | MU |
| cg2730915.5 | chr11 | 1300211 | 10 | MU | cg12606455 | chr2 | 106959878 | 26 | MU |
| cg14981422 | chr11 | 1300358 | 10 | N | cg09235217 | chr2 | 232263030 | 26 | MU |
| cg18403285 | chr11 | 1317034 | 10 | MU | cg12672050 | chr2 | 242585243 | 26 | N |
| cg05409391 | chr11 | 1886783 | 10 | MU | cg19445205 | chr2 | 242585285 | 26 | MU |
| cg25848652 | chr11 | 17591822 | 10 | N | cg06598544 | chr20 | 61472147 | 26 | MU |
| cg22155376 | chr11 | 17591837 | 10 | N | cg11523191 | chr20 | 62187126 | 26 | MU |
| cg07592809 | chr11 | 17591873 | 10 | MU | cg19817118 | chr3 | 31494178 | 26 | MU |
| cg25577078 | chr11 | 70644110 | 10 | MU | cg20927547 | chr3 | 31494215 | 26 | MU |
| cg05402634 | chr11 | 70644141 | 10 | MU | cg00184032 | chr3 | 31494263 | 26 | N |
| cg15624160 | chr11 | 70644185 | 10 | N | cg24724109 | chr3 | 31494362 | 26 | N |
| cg16480759 | chr11 | 119535816 | 10 | N | cg05279806 | chr3 | 175128616 | 26 | MU |
| cg01909845 | chr11 | 119535897 | 10 | MU | cg13748287 | chr3 | 184290594 | 26 | N |
| cg20182983 | chr12 | 123470916 | 10 | MU | cg19161330 | chr3 | 184290643 | 26 | N |
| cg05171224 | chr12 | 123471014 | 10 | N | cg13735557 | chr3 | 184290720 | 26 | MU |
| cg04353660 | chr12 | 123471054 | 10 | N | cg10769844 | chr3 | 184290794 | 26 | N |
| cg10200168 | chr12 | 124927713 | 10 | MU | cg19463199 | chr3 | 197401858 | 26 | N |
| cg07155331 | chr12 | 125500511 | 10 | N | cg12023911 | chr3 | 197401931 | 26 | N |
| cg01589353 | chr12 | 125500634 | 10 | MU | cg27013765 | chr3 | 197402007 | 26 | MU |
| cg15161050 | chr12 | 125500700 | 10 | N | cg09559242 | chr3 | 197402153 | 26 | N |
| cg25104118 | chr12 | 125621344 | 10 | MU | cg27309282 | chr4 | 1016081 | 26 | MU |
| cg25124228 | chr12 | 125621409 | 10 | MU | cg17095315 | chr4 | 2038352 | 26 | MU |
| cg06696911 | chr12 | 133147957 | 10 | MU | cg17630294 | chr4 | 7985238 | 26 | MU |
| cg01901624 | chr12 | 133306722 | 10 | N | cg08100122 | chr4 | 187629232 | 26 | N |
| cg19327049 | chr12 | 133306772 | 10 | MU | cg21399040 | chr4 | 187629328 | 26 | MU |
| cg11570575 | chr12 | 133306839 | 10 | N | cg24820270 | chr4 | 187629336 | 26 | MU |
| cg26261553 | chr12 | 133306893 | 10 | N | cg01040779 | chr4 | 187629339 | 26 | N |
| cg16784943 | chr14 | 93415143 | 10 | MU | cg25352342 | chr4 | 187629387 | 26 | MU |
| cg19995595 | chr14 | 93415197 | 10 | N | cg24833566 | chr4 | 187629400 | 26 | N |
| cg22808839 | chr14 | 93415226 | 10 | N | cg13454542 | chr4 | 187629427 | 26 | MU |
| cg13026782 | chr14 | 102484795 | 10 | N | cg10010780 | chr4 | 187629520 | 26 | N |
| cg21834982 | chr14 | 102484833 | 10 | MU | cg01221209 | chr5 | 554886 | 26 | MU |
| cg01678790 | chr14 | 102484867 | 10 | MU | cg19599827 | chr6 | 596273 | 26 | MU |
| cg01694276 | chr14 | 105247329 | 10 | MU | cg14815183 | chr6 | 30131613 | 26 | N |
| cg00697668 | chr14 | 105617575 | 10 | MU | cg07421969 | chr6 | 30131634 | 26 | N |
| cg20220196 | chr16 | 111753 | 10 | N | cg21988950 | chr6 | 30131691 | 26 | N |
| cg09201525 | chr16 | 111871 | 10 | MU | cg09769113 | chr6 | 30131719 | 26 | N |
| cg05791870 | chr16 | 136656 | 10 | MU | cg05664039 | chr6 | 30131755 | 26 | MU |
| cg27465384 | chr16 | 1435426 | 10 | MU | cg00628477 | chr6 | 30131821 | 26 | N |
| cg05603553 | chr16 | 1607853 | 10 | N | cg01905142 | chr6 | 30131840 | 26 | N |
| cg03315432 | chr16 | 1607889 | 10 | MU | cg07889936 | chr6 | 30616472 | 26 | MU |
| cg16757605 | chr16 | 70697712 | 10 | MU | cg10669449 | chr6 | 106676237 | 26 | MU |
| cg27403117 | chr16 | 70698053 | 10 | N | cg11532971 | chr6 | 106676381 | 26 | N |
| cg06933816 | chr16 | 70698146 | 10 | MU | cg17301053 | chr6 | 166891617 | 26 | N |

(continued)

| Illumina Probe Name | Chr. | Position | Tissue/Cell Type | MU/N | Illumina Probe Name | Chr. | Position | Tissue/Cell Type | MU/N |
|---|---|---|---|---|---|---|---|---|---|
| cg27088725 | chr16 | 72459796 | 10 | N | cg04224624 | chr6 | 166891650 | 26 | MU |
| cg07209034 | chr16 | 72459825 | 10 | MU | cg18092351 | chr7 | 960115 | 26 | MU |
| cg16557526 | chr16 | 87730518 | 10 | N | cg13687274 | chr7 | 960246 | 26 | N |
| cg08922090 | chr16 | 87730600 | 10 | MU | cg20449048 | chr7 | 1037343 | 26 | MU |
| cg09391949 | chr16 | 87730651 | 10 | N | cg25319337 | chr7 | 48008127 | 26 | MU |
| cg01018360 | chr16 | 88689494 | 10 | MU | cg16233515 | chr7 | 73408058 | 26 | MU |
| cg03233548 | chr16 | 88949985 | 10 | N | cg07243141 | chr7. | 73408060 | 26 | MU |
| cg08351911 | chr16 | 88950075 | 10 | MU | cg27099880 | chr7 | 74021938 | 26 | MU |
| cg01168962 | chr16 | 88950197 | 10 | MU | cg18771538 | chr7 | 107483786 | 26 | N |
| cg00001687 | chr16 | 89761661 | 10 | MU | cg07536914 | chr7 | 107483801 | 26 | MU |
| cg23401459 | chr17 | 1027840 | 10 | MU | cg10290200 | chr7 | 128481295 | 26 | MU |
| cg08314543 | chr17 | 8380278 | 10 | N | cg17677524 | chr7 | 150069026 | 26 | MU |
| cg13021479 | chr17 | 8380298 | 10 | MU | cg03899721 | chr8 | 8748792 | 26 | MU |
| cg18041123 | chr17 | 8380351 | 10 | N | cg03598366 | chr8 | 61789011 | 26 | MU |
| cg10940874 | chr17 | 8380417 | 10 | N | cg13568432 | chr8 | 61789077 | 26 | MU |
| cg01218720 | chr17 | 40322138 | 10 | N | cg13494954 | chr9 | 97369791 | 26 | MU |
| cg26583088 | chr17 | 40322250 | 10 | MU | cg14040532 | chr9 | 136915627 | 26 | MU |
| cg27125505 | chr17 | 43679177 | 10 | MU | cg01219549 | chr9 | 140683649 | 26 | N |
| cg04927033 | chr17 | 43679265 | 10 | N | cg14050824 | chr9 | 140683797 | 26 | MU |
| cg03730389 | chr18 | 55315731 | 10 | N | cg13244315 | chr1 | 985393 | 27 | MU |
| cg21577007 | chr18 | 55315848 | 10 | N | cg23087108 | chr1 | 1120617 | 27 | MU |
| cg16771467 | chr18 | 55315872 | 10 | MU | cg03935783 | chr1 | 1161069 | 27 | MU |
| cg07779777 | chr19 | 4047755 | 10 | N | cg24002071 | chr1 | 2519920 | 27 | MU |
| cg14017045 | chr19 | 4047785 | 10 | N | cg18594482 | chr1 | 27480543 | 27 | N |
| cg00007324 | chr19 | 4047803 | 10 | MU | cg26515162 | chr1 | 27480594 | 27 | MU |
| cg21206277 | chr19 | 4048075 | 10 | MU | cg22481448 | chr1 | 27480668 | 27 | MU |
| cg11817453 | chr19 | 8464850 | 10 | MU | cg05414613 | chr1 | 53558470 | 27 | N |
| cg15469014 | chr19 | 11032172 | 10 | MU | cg04142017 | chr1 | 53558596 | 27 | MU |
| cg00037314 | chr19 | 14274039 | 10 | MU | cg08256691 | chr1 | 155881846 | 27 | N |
| cg24530000 | chr19 | 14274172 | 10 | N | cg24337215 | chr1 | 155881944 | 27 | MU |
| cg09373983 | chr19 | 15348512 | 10 | MU | cg11109027 | chr1 | 207991720 | 27 | N |
| cg14730223 | chr19 | 18232604 | 10 | MU | cg15456742 | chr1 | 207991729 | 27 | N |
| cg21529164 | chr19 | 18313289 | 10 | N | cg03885098 | chr1 | 207991780 | 27 | MU |
| cg22617898 | chr19 | 18313388 | 10 | N | cg18929335 | chr10 | 30336504 | 27 | N |
| cg09998451 | chr19 | 18313429 | 10 | MU | cg20032789 | chr10 | 30336565 | 27 | MU |
| cg15528519 | chr19 | 36269387 | 10 | MU | cg09848445 | chr10 | 43601862 | 27 | MU |
| cg20690695 | chr19 | 40873973 | 10 | MU | cg14725719 | chr10 | 43601916 | 27 | N |
| cg06857516 | chr19 | 48205035 | 10 | MU | cg03969079 | chr10 | 63759909 | 27 | MU |
| cg24429081 | chr19 | 55741476 | 10 | MU | cg05655953 | chr10 | 123810032 | 27 | MU |
| cg14385008 | chr2 | 3469154 | 10 | MU | cg06732450 | chr10 | 134572361 | 27 | N |
| cg06529996 | chr2 | 3469212 | 10 | N | cg09768859 | chr10 | 134572388 | 27 | MU |
| cg02969343 | chr2 | 11163955 | 10 | MU | cg17994840 | chr10 | 134572417 | 27 | MU |
| cg12160850 | chr2 | 49143238 | 10 | MU | cg20090482 | chr10 | 135113191 | 27 | MU |
| cg10043829 | chr2 | 49143334 | 10 | N | cg11685186 | chr10 | 135113239 | 27 | N |
| cg22654940 | chr2 | 54856752 | 10 | MU | cg12818656 | chr11 | 1031856 | 27 | MU |
| cg10201874 | chr2 | 220412606 | 10 | MU | cg04539057 | chr11 | 1031985 | 27 | MU |
| cg10141075 | chr2 | 236708123 | 10 | MU | cg23270726 | chr11 | 1033157 | 27 | MU |
| cg26361545 | chr2 | 236708149 | 10 | N | cg09906402 | chr11 | 1033236 | 27 | N |
| cg25118192 | chr2 | 241819325 | 10 | N | cg25562530 | chr11 | 2013417 | 27 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg14992035 | chr2 | 241819352 | 10 | MU | cg01641514 | chr11 | 2013461 | 27 | N |
| cg01255646 | chr2 | 241819381 | 10 | N | cg26350007 | chr11 | 2013524 | 27 | MU |
| cg24428851 | chr2 | 242170166 | 10 | N | cg12580930 | chr11 | 2013668 | 27 | N |
| cg09235936 | chr2 | 242170211 | 10 | N | cg18896032 | chr11 | 57097718 | 27 | MU |
| cg03445663 | chr2 | 242170236 | 10 | MU | cg11179625 | chr11 | 61522741 | 27 | N |
| cg01227027 | chr20 | 61430899 | 10 | MU | cg05881566 | chr11 | 61522765 | 27 | MU |
| cg23897377 | chr21 | 46441953 | 10 | MU | cg11060910 | chr11 | 61522775 . | 27 | MU |
| cg14755417 | chr21 | 47823560 | 10 | MU | cg07486017 | chr11 | 61522799 | 27 | N |
| cg22959474 | chr22 | 42395235 | 10 | MU | cg06137032 | chr11 | 61522807 | 27 | MU |
| cg14777509 | chr3 | 52084879 | 10 | MU | cg15974867 | chr11 | 69464012 | 27 | MU |
| cg08350797 | chr3 | 52084921 | 10 | N | cg26579578 | chr11 | 69707303 | 27 | MU |
| cg01939195 | chr4 | 2277576 | 10 | N | cg01923037 | chr11 | 69707384 | 27 | N |
| cg11068784 | chr4 | 2277713 | 10 | MU | cg22397625 | chr11 | 119044725 | 27 | N |
| cg06554342 | chr4 | 2277752 | 10 | N | cg07478335 | chr11 | 119044743 | 27 | MU |
| cg07473822 | chr4 | 2277836 | 10 | N | cg22335368 | chr11 | 119044774 | 27 | N |
| cg05945584 | chr5 | 688486 | 10 | MU | cg26426437 | chr12 | 121686502 | 27 | MU |
| cg24954453 | chr5 | 780549 | 10 | MU | cg04356090 | chr12 | 129280133 | 27 | N |
| cg21544818 | chr5 | 780560 | 10 | N | cg05934015 | chr12 | 129280265 | 27 | MU |
| cg07865418, | chr5 | 6411408 | 10 | N | cg16782719 | chr12 | 129280334 | 27 | N |
| cg23366979 | chr5 | 6411510 | 10 | MU | cg02742418 | chr12 | 129280388 | 27 | N |
| cg11203538 | chr5 | 16794797 | 10 | MU | cg16682206 | chr13 | 111277501 | 27 | N |
| cg19453893 | chr5 | 16794840 | 10 | MU | cg21664614 | chr13 | 111277611 | 27 | MU |
| cg24923860 | chr5 | 149765871 | 10 | MU | cg02898500 | chr13 | 113826554 | 27 | MU |
| cg02362020 | chr6 | 31502415 | 10 | N | cg03775163 | chr13 | 114074693 | 27 | MU |
| cg24515095 | chr6 | 31502471 | 10 | N | cg13980446 | chr13 | 114312327 | 27 | MU |
| cg24477778 | chr6 | 31502511 | 10 | MU | cg10800989 | chr14 | 99786193 | 27 | MU |
| cg21545988 | chr6 | 31502516 | 10 | N | cg04741516 | chr14 | 99786208 | 27 | N |
| cg07845011 | chr6 | 31502521 | 10 | N | cg10538275 | chr14 | 99786226 | 27 | N |
| cg15541287 | chr6 | 56296129 | 10 | N | cg10533694 | chr14 | 105181370 | 27 | MU |
| cg06167524 | chr6 | 56296165 | 10 | N | cg11290775 | chr14 | 105181449 | 27 | N |
| cg27347309 | chr6 | 56296240 | 10 | MU | cg01921845 | chr14 | 105238643 | 27 | MU |
| cg12308631 | chr6 | 158454502 | 10 | N | cg10415607 | chr14 | 105238745 | 27 | N |
| cg19697584 | chr6 | 158454638 | 10 | MU | cg14184729 | chr14 | 105238773 | 27 | N |
| cg15332159 | chr6 | 158454775 | 10 | N | cg24765648 | chr14 | 105349455 | 27 | MU |
| cg05501666 | chr6 | 163558674 | 10 | N | cg07883696 | chr15 | 41628806 | 27 | MU |
| cg11724750 | chr6 | 163558823 | 10 | MU | cg18083415 | chr15 | 99669970 | 27 | N |
| cg20268208 | chr7 | 618210 | 10 | N | cg22245494 | chr15 | 99669982 | 27 | MU |
| cg26812421 | chr7 | 618226 | 10 | MU | cg00701514 | chr16 | 1183462 | 27 | MU |
| cg27462820 | chr7 | 636583 | 10 | MU | cg07812849 | chr16 | 1183588 | 27 | N |
| cg20392203 | chr7 | 636590 | 10 | MU | cg00180470 | chr16 | 11343465 | 27 | MU |
| cg06413196 | chr7 | 640338 | 10 | MU | cg09328356 | chr16 | 29913162 | 27 | MU |
| cg10623530 | chr7 | 640409 | 10 N | N | cg02488299 | chr16 | 29913223 | 27 | N |
| cg18807030 | chr7 | 931980 | 10 | MU | cg27156649 | chr16 | 30567019 | 27 | MU |
| cg24297873 | chr7 | 1993352 | 10 | MU | cg04453552 | chr16 | 57394387 | 27 | MU |
| cg25573368 | chr7 | 1993468 | 10 | N | cg16559361 | chr16 | 88543440 | 27 | MU |
| cg27483739 | chr7 | 44279162 | 10 | N | cg26884787 | chr16 | 88543447 | 27 | N |
| cg09214456 | chr7 | 44279245 | 10 | MU | cg04228827 | chr16 | 88550365 | 27 | N |
| cg03257708 | chr7 | 44279575 | 10 | N | cg02071579 | chr16 | 88550450 | 27 | MU |
| cg25305174 | chr7 | 44279680 | 10 | MU | cg00379559 | chr16 | 88564934 | 27 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg01776691 | chr7 | 44279727 | 10 | N | cg10095533 | chr16 | 88565080 | 27 | MU |
| cg01896918 | chr7 | 73938448 | 10 | N | cg06800849 | chr16 | 89180587 | 27 | MU |
| cg26307355 | chr7 | 73938543 | 10 | MU | cg10016717 | chr17 | 88246 | 27 | MU |
| cg10192483 | chr7 | 76632239 | 10 | MU | cg11751810 | chr17 | 88286 | 27 | MU |
| cg27369641 | chr7 | 100274361 | 10 | N | cg06932713 | chr17 | 910196 | 27 | MU |
| cg17854981 | chr7 | 100274472 | 10 | MU | cg19463982 | chr17 | 48614436 | 27 | MU |
| cg14794786 | chr7 | 150815320 | 10 | MU | cg25648211 | chr17 | 76312455 | 27 | MU |
| cg21657490 | chr7 | 150815391 | 10 | N | cg17838182 | chr17 | 76991208 | 27 | MU |
| cg25454298 | chr7 | 150815445 | 10 | MU | cg25052374 | chr17 | 76991253 | 27 | N |
| cg27437510 | chr7 | 150817197 | 10 | MU | cg17129554 | chr17 | 76993272 | 27 | N |
| cg21010435 | chr7 | 157208882 | 10 | MU | cg22687077 | chr17 | 76993374 | 27 | N |
| cg22412150 | chr7 | 157208980 | 10 | N | cg00902147 | chr17 | 76993394 | 27 | MU |
| cg10787000 | chr8 | 1311396 | 10 | MU | cg18186771 | chr17 | 76993506 | 27 | N |
| cg15240102 | chr8 | 1311483 | 10 | N | cg03594801 | chr17 | 77948865 | 27 | N |
| cg02192555 | chr8 | 9952926 | 10 | MU | cg19790877 | chr17 | 77948884 | 27 | MU |
| cg26880445 | chr8 | 9952965 | 10 | N | cg05853503 | chr17 | 19146661 | 27 | N |
| cg11824826 | chr8 | 9953022 | 10 | N | cg12143110 | chr17 | 79146738 | 27 | N |
| cg13959595 | chr9 | 139910220 | 10 | MU | cg16466065 | chr17 | 79146774 | 27 | MU |
| cg17165158 | chr1 | 2182612 | 11 | MU | cg07049352 | chr17 | 80654335 | 27 | MU |
| cg12268236 | chr1 | 3329292 | 11 | MU | cg13937164 | chr18 | 19780587 | 27 | N |
| cg20986726 | chr1 | 11107045 | 11 | N | cg20880080 | chr18 | 19780644 | 27 | N |
| cg12888113 | chr1 | 11107048 | 11 | MU | cg12026967 | chr18 | 19780728 | 27 | MU |
| cg25009258 | chr1 | 20480748 | 11 | MU | cg00148892 | chr18 | 19780878 | 27 | N |
| cg20741134 | chr1 | 181382639 | 11 | MU | cg18328265 | chr19 | 418899 | 27 | N |
| cg13935480 | chr1 | 228238511 | 11 | N | cg10634728 | chr19 | 418906 | 27 | MU |
| cg07204739 | chr1 | 228238553 | 11 | MU | cg08840018 | chr19 | 419048 | 27 | N |
| cg03610629 | chr11 | 611114 | 11 | MU | cg24299136 | chr19 | 2511707 | 27 | MU |
| cg09509144 | chr11 | 2860989 | 11 | N | cg18415485 | chr19 | 3546364 | 27 | MU |
| cg26896748 | chr11 | 2861064 | 11 | MU | cg08344281 | chr19 | 6042039 | 27 | MU |
| cg24761467 | chr11 | 2861204 | 11 | N | cg22707397 | chr19 | 49020528 | 27 | MU |
| cg02369448 | chr11 | 5315952 | 11 | MU | cg22518433 | chr19 | 49253931 | 27 | MU |
| cg13527922 | chr11 | 46741299 | 11 | N | cg06497736 | chr19 | 50411442 | 27 | N |
| cg26453360 | chr11 | 46741373 | 11 | MU | cg06477444 | chr19 | 50411521 | 27 | MU |
| cg09004691 | chr11 | 46741405 | 11 | MU | cg15153684 | chr2 | 10686963 | 27 | MU |
| cg13309174 | chr11 | 61102045 | 11 | N | cg13851334 | chr2 | 10686975 | 27 | N |
| cg03483654 | chr11 | 61102074 | 11 | MU | cg09761224 | chr2 | 10687042 | 27 | MU |
| cg25406518 | chr11 | 61102165 | 11 | N | cg03405781 | chr2. | 103095638 | 27 | MU |
| cg19620972 | chr11 | 65810516 | 11 | MU | cg10403518 | chr2 | 103095704 | 27 | N |
| cg00874225 | chr11 | 66458784 | 11 | N | cg13551814 | chr20 | 45280276 | 27 | N |
| cg1576234.8 | chr11 | 66458829 | 11 | MU | cg19421368 | chr20 | 45280288 | 27 | N |
| cg22707381 | chr11 | 66458942 | 11 | N | cg24649332 | chr20 | 45280359 | 27 | N |
| cg17400609 | chr11 | 68154098 | 11 | MU | cg27650212 | chr20 | 45280414 | 27 | MU |
| cg02140020 | chr12 | 2613914 | 11 | N | cg15457037 | chr20 | 62562848 | 27 | MU |
| cg12205822 | chr12 | 2613941 | 11 | MU | cg19864539 | chr22 | 25747688 | 27 | MU |
| cg24043604 | chr12 | 2613948 | 11 | N | cg10788213 | chr22 | 45719176 | 27 | MU |
| cg14672128 | chr12 | 7280912 | 11 | N | cg11314274 | chr3 | 132004064 | 27 | MU |
| cg12074585 | chr12 | 7280958 | 11 | MU | cg06726099 | chr3 | 195940674 | 27 | N |
| cg20315995 | chr12 | 7280971 | 11 | MU | cg04245305 | chr3 | 195940754 | 27 | MU |
| cg00203347 | chr12 | 108603943 | 11 | MU | cg05347334 | chr4 | 2439397 | 27 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg16956999 | chr12 | 133249241 | 11 | N | cg26149658 | chr4 | 2439423 | 27 | N |
| cg27197524 | chr12 | 133249278 | 11 | MU | cg15689795 | chr4 | 2439478 | 27 | N |
| cg20361429 | chr12 | 133249308 | 11 | MU | cg26605046 | chr4 | 2439731 | 27 | MU |
| cg02212698 | chr14 | 89628141 | 11 | MU | cg27491224 | chr4 | 2439740 | 27 | N |
| cg21898844 | chr14 | 89628169 | 11 | N | cg25469474 | chr4 | 2749501 | 27 | N |
| cg25007781 | chr14 | 105963610 | 11 | MU | cg22014887 | chr4 | 2749539 | 27 | MU |
| cg12050054 | chr14 | 105963656 | 11 | N | cg10587047 | chr4 | 2956821 | 27 | N |
| cg19879265 | chr14 | 105963772 | 11 | MU | cg05719181 | chr4 | 2956957 | 27 | MU |
| cg19525780 | chr14 | 105964243 | 11 | MU | cg24385450 | chr4 | 2957128 | 27 | MU |
| cg12651540 | chr15 | 90191926 | 11 | MU | cg00313498 | chr4 | 7787944 | 27 | N |
| cg26495393 | chr16 | 31473952 | 11 | MU | cg10099518 | chr4 | 7787960 | 27 | N |
| cg07478240 | chr16 | 87174203 | 11 | MU | cg27567206 | chr4 | 7787983 | 27 | MU |
| cg26573923 | chr16 | 87825195 | 11 | MU | cg07213482 | chr4 | 89660237 | 27 | MU |
| cg05640992 | chr16 | 87970367 | 11 | MU | cg08436224 | chr5 | 1122753 | 27 | N |
| cg03550864 | chr16 | 87970376 | 11 | MU | cg00395291 | chr5 | 1122815 | 27 | N |
| cg00379559 | chr16 | 88564934 | 11 | N | cg12301921 | chr5 | 1122879 | 27 | MU |
| cg10095533 | chr16 | 88565080 | 11 | MU | cg26224791 | chr5 | 1320734 | 27 | MU |
| cg14758740 | chr17 | 941872 | 11 | MU | cg05905124 | chr5 | 1342124 | 27 | N |
| cg26939227 | chr17 | 1372403 | 11 | N | cg07493874 | chr5 | 1342172 | 27 | MU |
| cg20771846 | chr17 | 1372516 | 11 | MU | cg14733637 | chr5 | 1342200 | 27 | MU |
| cg01453157 | chr17 | 1639375 | 11 | MU | cg18766691 | chr5 | 1489818 | 27 | N |
| cg19078123 | chr17 | 2917358 | 11 | N | cg08697251 | chr5 | 1489875 | 27 | N |
| cg03274539 | chr17 | 2917371 | 11 | MU | cg16272981 | chr5 | 1489889 | 27 | MU |
| cg18536123 | chr17 | 26694939 | 11 | MU | cg03887092 | chr6 | 1080885 | 27 | MU |
| cg03028529 | chr17 | 26695002 | 11 | N | cg00077898 | chr6 | 1081144 | 27 | MU |
| cg11316510 | chr17 | 38510570 | 11 | MU | cg03680932 | chr6 | 16306683 | 27 | MU |
| cg12190841 | chr17 | 38510610 | 11 | N | cg18015809 | chr6 | 47202254 | 27 | N |
| cg12438413 | chr17 | 38510643 | 11 | N | cg21552001 | chr6 | 47202394 | 27 | MU |
| cg20116491 | chr17 | 72999894 | 11 | MU | cg27205928 | chr7 | 1062652 | 27 | MU |
| cg03280611 | chr17 | 72999908 | 11 | N | cg16492851 | chr7 | 1062681 | 27 | N |
| cg21034531 | chr17 | 80197757 | 11 | N | cg04682977 | chr7 | 1062711 | 27 | N |
| cg22805688 | chr17 | 80197841 | 11 | N | cg18023771 | chr7 | 24874132 | 27 | MU |
| cg02744699 | chr17 | 80197898 | 11 | MU | cg04525464 | chr7 | 24874213 | 27 | N |
| cg25623174 | chr19 | 1487019 ' | 11 | MU | cg23043230 | chr7 | 97935666 | 27 | N |
| cg15470261 | chr19 | 1698699 | 11 | MU | cg15211734 | chr7 | 97935756 | 27 | N |
| cg12176815 | chr19 | 1979540 | 11 | MU | cg10592494 | chr7 | 97935764 | 27 | MU |
| cg18592294 | chr19 | 2790475 | 11 | MU | cg21962765 | chr7 | 100490061 | 27 | N |
| cg25806370 | chr19 | 2790585 | 11 | MU | cg14204266 | chr7 | 100490184 | 27 | MU |
| cg23295181 | chr19 | 2790716 | 11 | N | cg11385616 | chr7 | 100490250 | 27 | N |
| cg23499194 | chr19 | 3192601 | 11 | MU | cg23275972 | chr7 | 157224017 | 27 | N |
| cg13733923 | chr19 | 4537901 | 11 | MU | cg05331807 | chr7 | 157224035 | 27 | MU |
| cg10317145 | chr2 | 44065680 | 11 | N | cg10012512 | chr7 | 157224041 | 27 | N |
| cg00459909 | chr2 | 44065720 | 11 | N | cg27224718 | chr8 | 135614730 | 27 | MU |
| cg20926720 | chr2 | 44065725 | 11 | MU | cg21672855 | chr8 | 135614777 | 27 | N |
| cg07681696 | chr2 | 44065858 | 11 | N | cg13494954 | chr9 | 97369791 | 27 | MU |
| cg02078517 | chr2 | 75720511 | 11 | N | cg14468605 | chr9 | 137255666 | 27 | MU |
| cg00409636 | chr2 | 75720541 | 11 | MU | cg14487665 | chr9 | 140408634 | 27 | MU |
| cg00590251 | chr2 | 119980583 | 11 | MU | cg04890237 | chr1 | 2537585 | 28 | N |
| cg15356320 | chr2 | 127818000 | 11 | N | cg11707310 | chr1 | 2537719 | 28 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg08663425 | chr2 | 127818046 | 11 | N | cg21929781 | chr1 | 2537748 | 28 | N |
| cg13293246 | chr2 | 127818101 | 11 | N | cg01075559 | chr1 | 2537774 | 28 | N |
| cg10846328 | chr2 | 127818131 | 11 | MU | cg07832245 | chr1 | 3159568 | 28 | N |
| cg21427464 | chr2 | 127818226 | 11 | N | cg13175786 | chr1 | 3159615 | 28 | N |
| cg07780694 | chr2 | 238990408 | 11 | MU | cg13867039 | chr1 | 3159645 | 28 | MU |
| cg26042618 | chr20 | 56227286 | 11 | MU | cg01602345 | chr1 | 3277525 | 28 | N |
| cg14083603 | chr20 | 62366419 | 11 | MU | cg21167817 | chr1 | 3277557 | 28 | MU |
| cg22989407 | chr21 | 44590650 | 11 | MU | cg01071314 | chr1 | 3277623 | 28 | MU |
| cg05670717 | chr21 | 46368052 | 11 | N | cg02891048 | chr1 | 8044991 | 28 | MU |
| cg11988476 | chr21 | 46368090 | 11 | MU | cg14531038 | chr1 | 8045077 | 28 | N |
| cg10004976 | chr21 | 46880279 | 11 | MU | cg06634717 | chr1 | 8442507 | 28 | MU |
| cg10394047 | chr21 | 47575416 | 11 | N | cg24425531 | chr1 | 15372951 | 28 | MU |
| cg09436823 | chr21 | 47575498 | 11 | N | cg19358202 | chr1 | 41486102 | 28 | MU |
| cg18024037 | chr21 | 47575504 | 11 | MU | cg01186725 | chr1 | 53905612 | 28 | N |
| cg25322086 | chr21 | 47575547 | 11 | N | cg16038181 | chr1 | 53905714 | 28 | N |
| cg07819160 | chr22 | 37420328 | 11 | N | cg00350519 | chr1 | 53905742 | 28 | MU |
| cg12253469 | chr22 | 37420454 | 11 | MU | cg11753771 | chr1 | 214638070 | 28 | MU |
| cg18659483 | chr22 | 41630982 | 11 | MU | cg25894019 | chr10 | 115169101 | 28 | MU |
| cg21874862 | chr22 | 50644682 | 11 | N | cg17750946 | chr10 | 134559637 | 28 | N |
| cg25934700 | chr22 | 5064475.5 | 11 | MU | cg07927953 | chr10 | 134559696 | 28 | MU |
| cg16919420 | chr22 | 50644878 | 11 | N | cg14275445 | chr10 | 134560047 | 28 | N |
| cg25354127 | chr3 | 142681595 | 11 | N | cg08552167 | chr10 | 134560109 | 28 | MU |
| cg26301689 | chr3 | 142681704 | 11 | MU | cg18263591 | chr11 | 3638352 | 28 | N |
| cg21380181 | chr4 | 1198278 | 11 | MU | cg14166625 | chr11 | 3638448 | 28 | N |
| cg18406106 | chr4 | 1198308 | 11 | N | cg02453348 | chr11 | 3638458 | 28 | MU |
| cg05821186 | chr4 | 1198353 | 11 | N | cg07783183 | chr11 | 75852355 | 28 | MU |
| cg20356136 | chr4 | 7802238 | 11 | MU | cg24630516 | chr11 | 75852389 | 28 | MU |
| cg11932225 | chr4 | 7802347 | 11 | N | cg05343689 | chr11 | 75852437 | 28 | N |
| cg24716420 | chr4 | 7802369 | 11 | N | cg19846609 | chr11 | 124999394 | 28 | MU |
| cg01807825 | chr4 | 80329368 | 11 | N | cg11985341 | chr11 | 129722477 | 28 | MU |
| cg17738194 | chr4 | 80329388 | 11 | N | cg27223646 | chr11 | 129722512 | 28 | MU |
| cg25615081 | chr4 | 80329402 | 11 | N | cg01179055 | chr12 | 665829 | 28 | N |
| cg20562630 | chr4 | 80329424 | 11 | N | cg12554051 | chr12 | 665863 | 28 | MU |
| cg00177468 | chr4 | 80329447 | 11 | N | cg24984147 | chr12 | 665887 | 28 | N |
| cg18493326 | chr4 | 80329481 | 11 | N | cg02952918 | chr12 | 120173011 | 28 | MU |
| cg15063695 | chr4 | 80329483 | 11 | MU | cg14578247 | chr12 | 120173057 | 28 | MU |
| cg26226487 | chr4 | 80329486 | 11 | MU | cg17338594 | chr12 | 120173114 | 28 | N |
| cg04414629 | chr4 | 80329633 | 11 | N | cg09860804 | chr12 | 122396633 | 28 | N |
| cg26955455 | chr4 | 154727550 | 11 | MU | cg13411923 | chr12 | 122396677 | 28 | MU |
| cg03498175 | chr4 | 185724569 | 11 | MU | cg15417875 | chr12 | 122396687 | 28 | MU |
| cg08823975 | chr4 | 185724646 | 11 | N | cg14078579 | chr12 | 133120548 | 28 | N |
| cg25074170 | chr4 | 187088169 | 11 | MU | cg05053403 | chr12 | 133120676 | 28 | MU |
| cg14477780 | chr4 | 187088191 | 11 | MU | cg01362762 | chr13 | 114805400 | 28 | MU |
| cg24153754 | chr5 | 149901187 | 11 | MU | cg26070281 | chr14 | 21560028 | 28 | MU |
| cg14462307 | chr5 | 179267556 | 11 | MU | cg01813877 | chr14 | 103369717 | 28 | MU |
| cg16243359 | chr6 | 7142578 | 11 | MU | cg22779765 | chr14 | 103369786 | 28 | N |
| cg14864022 | chr6 | 7142610 | 11 | MU | cg18993641 | chr14 | 106109496 | 28 | MU |
| cg09866303 | chr6 | 7142638 | 11 | MU | cg13175736 | chr15 | 62537871 | 28 | N |
| cg17124478 | chr6 | 14284182 | 11 | MU | cg06136125 | chr15 | 62537916 | 28 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg01291920 | chr6 | 31915077 | 11 | N | cg23066057 | chr15 | 62537934 | 28 | MU |
| cg23346801 | chr6 | 31915104 | 11 | N | cg25637473 | chr15 | 62537960 | 28 | MU |
| cg25734035 | chr6 | 31915125 | 11 | N | cg17274072 | chr15 | 90904227 | 28 | MU |
| cg01595997 | chr6 | 31915184 | 11 | MU | cg01201782 | chr16 | 1652449 | 28 | N |
| cg21178851 | chr6 | 160500620 | 11 | MU | cg10141942 | chr16 | 1652482 | 28 | N |
| cg02396797 | chr6 | 160500689 | 11 | N | cg02654411 | chr16 | 1652552 | 28 | MU |
| cg03064642 | chr6 | 160500735 | 11 | N | cg09692355 | chr16 | 1677047 | 28 | MU |
| cg09511662 | chr6 | 160500971 | 11 | MU | cg03929747 | chr16 | 2282618 | 28 | MU |
| cg13992493 | chr6 | 169092333 | 11 | MU | cg10278046 | chr16 | 4802848 | 28 | N |
| cg08123673 | chr6 | 169092358 | 11 | N | cg16755475 | chr16 | 4802990 | 28 | MU |
| cg26422935 | chr6 | 169092481 | 11 | N | cg09596260 | chr16 | 50338380 | 28 | MU |
| cg26337420 | chr6 | 169092495 | 11 | N | cg09100343 | chr16 | 57147202 | 28 | N |
| cg09279942 | chr6 | 169092523 | 11 | MU | cg06880930 | chr16 | 57147300 | 28 | MU |
| cg24915751 | chr6 | 170561236 | 11 | MU | cg10464529 | chr16 | 58030730 | 28 | MU |
| cg25929139 | chr7 | 1748515 | 11 | N | cg00454856 | chr16 | 58030811 | 28 | MU |
| cg20718434 | chr7 | 1748597 | 11 | N | cg26673975 | chr16 | 66420974 | 28 | MU |
| cg17549927 | chr7 | 1748615 | 11 | N | cg02924834 | chr16 | 87744876 | 28 | N |
| cg18218112 | chr7 | 1748639 | 11 | MU | cg27459530 | chr16 | 87744906 | 28 | MU |
| cg10369896 | chr7 | 1748766 | 11 | N | cg08734237 | chr16 | 87744948 | 28 | MU |
| cg18227995 | chr7 | 1882029 | 11 | N | cg24352934 | chr17 | 1053717 | 28 | MU |
| cg24813613 | chr7 | 1882135 | 11 | MU | cg15893929 | chr17 | 3755190 | 28 | MU |
| cg08411881 | chr7 | 1963104 | 11 | MU | cg10924857 | chr17 | 20013829 | 28 | MU |
| cg10571824 | chr7 | 1963144 | 11 | MU | cg05888362 | chr17 | 20013887 | 28 | N |
| cg16773043 | chr7 | 1963149 | 11 | MU | cg21784143 | chr17 | 20460588 | 28 | MU |
| cg02943336 | chr7 | 2959067 | 11 | N | cg05058886 | chr17 | 20460627 | 28 | N |
| cg13106306 | chr7 | 2959105 | 11 | MU | cg23674202 | chr17 | 80541549 | 28 | MU |
| cg06830441 | chr7 | 2959121 | 11 | N | cg24220178 | chr17 | 80541649 | 28 | N |
| cg21232767 | chr7 | 157619282 | 11 | MU | cg25899954 | chr18 | 8660164 | 28 | MU |
| cg05439204 | chr7 | 157854693 | 11 | N | cg13513411 | chr19 | 4676202 | 28 | MU |
| cg02470440 | chr7 | 157854772 | 11 | MU | cg24868883 | chr19 | 45648302 | 28 | MU |
| cg10105718 | chr7 | 158673837 | 11 | N | cg00673724 | chr19 | 45648483 | 28 | N |
| cg08767743 | chr7 | 158673942 | 11 | MU | cg13786513 | chr19 | 45648562 | 28 | MU |
| cg13308350 | chr7 | 158673979 | 11 | N | cg15147841 | chr19 | 46033285 | 28 | MU |
| cg10702755 | chr8 | 528084 | 11 | MU | cg11276053 | chr19 | 46307734 | 28 | MU |
| cg17142066 | chr8 | 528131 | 11 | N | cg02359773 | chr19 | 46307846 | 28 | MU |
| cg20823730 | chr8 | 790768 | 11 | MU | cg20469261 | chr19 | 50361793 | 28 | MU |
| cg22658846 | chr8 | 2019760 | 11 | MU | cg26570040 | chr19 | 50361827 | 28 | N |
| cg06835018 | chr8 | 12272982 | 11 | MU | cg23985239 | chr19 | 50361910 | 28 | N |
| cg22292713 | chr8 | 29344567 | 11 | N | cg11616411 | chr19 | 54290799 | 28 | N |
| cg18557212 | chr8 | 29344583 | 11 | MU | cg24823137 | chr19 | 54290902 | 28 | N |
| cg05973488 | chr8 | 29344599 | 11 | N | cg05882315 | chr19 | 54290905 | 28 | N |
| cg01911981 | chr8 | 39380341 | 11 | N | cg20644425 | chr19 | 54290913 | 28 | MU |
| cg17527589 | chr8 | 39380435 | 11 | MU | cg08239565 | chr19 | 54290996 | 28 | N |
| cg19598713 | chr8 | 144601734 | 11 | N | cg01975502 | chr19 | 54291017 | 28 | N |
| cg18210511 | chr8 | 144601781 | 11 | N | cg18403970 | chr19 | 54291050 | 28 | N |
| cg09580859 | chr8 | 144601800 | 11 | N | cg01743020 | chr2 | 1886086 | 28 | MU |
| cg25900150 | chr8 | 144601851 | 11 | MU | cg09927251 | chr2 | 1886105 | 28 | N |
| cg00783706 | chr8 | 144601883 | 11 | N | cg17162031 | chr2 | 1886127 | 28 | N |
| cg00110724 | chr8 | 144885851 | 11 | MU | cg02381965 | chr2 | 3418480 | 28 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg03000485 | chr8 | 144973638 | 11 | MU | cg24929896 | chr2 | 3418530 | 28 | N |
| cg17740743 | chr1 | 1986220 | 12 | N | cg03239335 | chr2 | 3418641 | 28 | MU |
| cg12391964 | chr1 | 1986275 | 12 | MU | cg03445173 | chr2 | 17998337 | 28 | MU |
| cg01421021 | chr1 | 10719929 | 12 | MU | cg03269045 | chr2 | 26680478 | 28 | MU |
| cg13377965 | chr1 | 21844799 | 12 | MU | cg03427577 | chr2 | 61809639 | 28 | MU |
| cg08713722 | chr1 | 38942976 | 12 | MU | cg00102512 | chr2 | 129103162 | 28 | N |
| cg19358202 | chr1 | 41486102 | 12 | MU | cg10933428 | chr2 | 129103164 | 28 | MU |
| cg17589319 | chr1 | 43530194 | 12 | MU | cg01125214 | chr2 | 236888769 | 28 | MU |
| cg07602008 | chr1 | 86048479 | 12 | N | cg04216051 | chr2 | 240172612 | 28 | MU |
| cg20293609 | chr1 | 86048540 | 12 | MU | cg20415945 | chr2 | 240172645 | 28 | N |
| cg17499345 | chr1 | 86048585 | 12 | N | cg17257554 | chr2 | 242004824 | 28 | MU |
| cg16563929 | chr10 | 525898 | 12 | N | cg18624016 | chr2 | 242004954 | 28 | N |
| cg00195144 | chr10 | 525946 | 12 | MU | cg15707332 | chr20 | 32436359 | 28 | MU |
| cg27201679 | chr10 | 1120794 | 12 | N | cg04893833 | chr20 | 32436391 | 28 | N |
| cg11314310 | chr10 | 1120831 | 12 | MU | cg17894293 | chr20 | 32436418 | 28 | N |
| cg13473894 | chr10 | 13771383 | 12 | MU | cg00171166 | chr20 | 35444275 | 28 | MU |
| cg12656653 | chr10 | 13771427 | 12 | MU | cg06814048 | chr20 | 35444364 | 28 | N |
| cg23873415 | chr10 | 13771465 | 12 | MU | cg00734813 | chr20 | 35444512 | 28 | N |
| cg18594033 | chr10 | 112524504 | 12 | MU | cg21970473 | chr20 | 35444558 | 28 | N |
| cg06874891 | chr10 | 112524550 | 12 | N | cg08209184 | chr20 | 35444560 | 28 | MU |
| cg21193639 | chr11 | 2349510 | 12 | MU | cg19412478 | chr22 | 26883640 | 28 | MU |
| cg23267171 | chr11 | 2349553 | 12 | N | cg25931138 | chr3 | 195709698 | 28 | MU |
| cg05527468 | chr11 | 2349598 | 12 | N | cg12923728 | chr3 | 195709715 | 28 | N |
| cg10738865 | chr11 | 2356706 | 12 | N | cg26124466 | chr3 | 197347235 | 28 | N |
| cg27444290 | chr11 | 2356831 | 12 | MU | cg00133223 | chr3 | 197347256 | 28 | N |
| cg26688678 | chr11 | 2356948 | 12 | N | cg02746684 | chr3 | 197347276 | 28 | MU |
| cg14157917 | chr11 | 2965816 | 12 | MU | cg11985754 | chr4 | 1214965 | 28 | N |
| cg18553732 | chr11 | 67844184 | 12 | MU | cg12389346 | chr4 | 1215112 | 28 | MU |
| cg13091717 | chr11 | 75898195 | 12 | MU | cg11920453 | chr4 | 1411820 | 28 | MU |
| cg12471171 | chr11 | 75898203 | 12 | N | cg00313498 | chr4 | 7787944 | 28 | MU |
| cg13906874 | chr11 | 130089912 | 12 | MU | cg10099518 | chr4 | 7787960 | 28 | MU |
| cg04636406 | chr12 | 6993009 | 12 | MU | cg27567206 | chr4 | 7787983 | 28 | MU |
| cg14871584 | chr12 | 6993046 | 12 | N | cg06664054 | chr4 | 83323522 | 28 | N |
| cg21541335 | chr12 | 6993134 | 12 | N | cg18579761 | chr4 | 83323585 | 28 | MU |
| cg25015791 | chr12 | 66889655 | 12 | N | cg07979388 | chr4 | 169573255 | 28 | MU |
| cg09596707 | chr12 | 66889730 | 12 | MU | cg00612828 | chr4 | 183815971 | 28 | MU |
| cg02952918 | chr12 | 120173011 | 12 | MU | cg09557149 | chr4 | 183816164 | 28 | MU |
| cg14578247 | chr12 | 120173057 | 12 | N | cg19945912 | chr4 | 186945215 | 28 | N |
| cg17338594 | chr12 | 120173114 | 12 | N | cg09227533 | chr4 | 186945339 | 28 | MU |
| cg17211612 | chr12 | 124330647 | 12 | MU | cg10427811 | chr5 | 79647590 | 28 | MU |
| cg13541153 | chr12 | 132281761 | 12 | N | cg24526056 | chr5 | 156479556 | 28 | MU |
| cg01854803 | chr12 | 132281827 | 12 | MU | cg12943317 | chr5 | 156479607 | 28 | N |
| cg14078579 | chr12 | 133120548 | 12 | N | cg26994283 | chr5 | 177964757 | 28 | MU |
| cg05053403 | chr12 | 133120676 | 12 | MU | cg24830898 | chr5 | 177964873 | 28 | N |
| cg02592525 | chr13 | 25740027 | 12 | MU | cg06024039 | chr6 | 25726884 | 28 | N |
| cg26945748 | chr13 | 113400391 | 12 | MU | cg07077277 | chr6 | 25726912 | 28 | N |
| cg02150207 | chr13 | 113419720 | 12 | MU | cg10093738 | chr6 | 25726947 | 28 | N |
| cg19957503 | chr13 | 113419790 | 12 | N | cg25090472 | chr6 | 25726953 | 28 | MU |
| cg00033877 | chr13 | 113419845 | 12 | N | cg11761622 | chr6 | 25726956 | 28 | N |

(continued)

| Illumina Probe Name | Chr. | Position | Tissue/Cell Type | MU/N | Illumina Probe Name | Chr. | Position | Tissue/Cell Type | MU/N |
|---|---|---|---|---|---|---|---|---|---|
| cg11992426 | chr13 | 114764890 | 12 | MU | cg13778975 | chr6 | 25727019 | 28 | N |
| cg16400425 | chr14 | 35447884 | 12 | MU | cg03347739 | chr6 | 53787668 | 28 | MU |
| cg04250681 | chr14 | 71949117 | 12 | MU | cg02655824 | chr6 | 167051714 | 28 | N |
| cg25034766 | chr14 | 75747332 | 12 | MU | cg02118390 | chr6 | 167051744 | 28 | MU |
| cg16996571 | chr14 | 91691129 | 12 | MU | cg25296804 | chr6 | 167051781 | 28 | N |
| cg19873420 | chr14 | 91691179 | 12 | N | cg14764357 | chr7 | 1093718 | 28 | N |
| cg14304073 | chr14 | 91691190 | 12 | MU | cg26071556 | chr7 | 1093854 | 28 | MU |
| cg16434156 | chr14 | 94497084 | 12 | MU | cg11116288 | chr7 | 1093880 | 28 | N |
| cg10984327 | chr14 | 105683798 | 12 | N | cg13320257 | chr7 | 1093903 | 28 | N |
| cg13692222 | chr14 | 105683925 | 12 | MU | cg19016273 | chr7 | 3948024 | 28 | MU |
| cg04835431 | chr14 | 105684005 | 12 | MU | cg24872851 | chr7 | 4265468 | 28 | N |
| cg20800039 | chr15 | 58891233 | 12 | MU | cg19854900 | chr7 | 4265529 | 28 | N |
| cg22389121 | chr15 | 80217380 | 12 | MU | cg19727491 | chr7 | 4265548 | 28 | MU |
| cg06717750 | chr16 | 667157 | 12 | MU | cg18365383 | chr7 | 33725819 | 28 | MU |
| cg27060386 | chr16 | 667188 | 12 | N | cg23940732 | chr7 | 33725885 | 28 | N |
| cg06013113 | chr16 | 678127 | 12 | MU | cg01312316 | chr7 | 33725924 | 28 | N |
| cg01201782 | chr16 | 1652449 | 12 | N | cg20985897 | chr7 | 157778531 | 28 | MU |
| cg10141942 | chr16 | 1652482 | 12 | N | cg26480662 | chr7 | 157778593 | 28 | N |
| cg02654411 | chr16 | 1652552 | 12 | MU | cg13083427 | chr7 | 157778645 | 28 | N |
| cg06780216 | chr16 | 2374397 | 12 | N | cg16481556 | chr8 | 2196764 | 28 | MU |
| cg16463165 | chr16 | 2374474 | 12 | N | cg24196612 | chr8 | 21608193 | 28 | N |
| cg07753939. | chr16 | 2374515 | 12 | MU | cg15997512 | chr8 | 21608254 | 28 | MU |
| cg00794055 | chr16 | 2544909 | 12 | MU | cg23716449 | chr8 | 21608280 | 28 | MU |
| cg20364187 | chr16 | 3139060 | 12 | MU | cg02528189 | chr8 | 140931333 | 28 | N |
| cg07651316 | chr16 | 3641320 | 12 | MU | cg10440196 | chr8 | 140931443 | 28 | MU |
| cg07351059 | chr16 | 29999179 | 12 | MU | cg02235041 | chr8 | 144676053 | 28 | MU |
| cg09251197 | chr16 | 30000254 | 12 | MU | cg00083359 | chr8 | 144676110 | 28 | N |
| cg09969487 | chr16 | 49902325 | 12 | MU | cg14280283 | chr9 | 139903861 | 28 | MU |
| cg08138817 | chr16 | 49902342 | 12 | N | cg10827460 | chr1 | 879375 | 29 | N |
| cg05819154 | chr16 | 49902436 | 12 | N | cg15742605 | chr1 | 879383 | 29 | MU |
| cg02667487 | chr16 | 67920036 | 12 | MU | cg09756115 | chr1 | 879415 | 29 | N |
| cg08520423 | chr16 | 68013968 | 12 | MU | cg09234454 | chr1 | 94560697 | 29 | MU |
| cg23401756 | chr16 | 68013981 | 12 | N | cg13459217 | chr1 | 205326437 | 29 | MU |
| cg17248540 | chr16 | 69143500 | 12 | N | cg26251854 | chr1 | 229252074 | 29 | MU |
| cg04320956 | chr16 | 69143512 | 12 | MU | cg18537476 | chr1 | 229252112 | 29 | MU |
| cg02730714 | chr16 | 69143775 | 12 | MU | cg19087171 | chr1 | 234819745 | 29 | MU |
| cg03246665 | chr16 | 84271391 | 12 | MU | cg12639192 | chr10 | 1166782 | 29 | N |
| cg02893344 | chr16 | 85517269 | 12 | N | cg22747480 | chr10 | 1166867 | 29 | MU |
| cg06081580 | chr16 | 85517323 | 12 | MU | cg00779313 | chr10 | 1166994 | 29 | N |
| cg05574053 | chr16 | 85517371 | 12 | MU | cg17575314 | chr10 | 71142526 | 29 | MU |
| cg16399368 | chr16 | 85517462 | 12 | N | cg02009103 | chr10 | 102766386 | 29 | MU |
| cg21384021 | chr17 | 79952367 | 12 | MU | cg18360825 | chr10 | 102766458 | 29 | N |
| cg01502876 | chr17 | 81042947 | 12 | MU | cg08398691 | chr10 | 131217895 | 29 | N |
| cg05248652 | chr17 | 81043057 | 12 | N | cg07175255 | chr10 | 131218009 | 29 | MU |
| cg08626004 | chr19 | 2513687 | 12 | MU | cg16627236 | chr10 | 134420036 | 29 | N |
| cg19819654 | chr19 | 10370550 | 12 | MU | cg07249433 | chr10 | 134420183 | 29 | MU |
| cg11616411 | chr19 | 54290799 | 12 | N | cg11740348 | chr10 | 134420208 | 29 | MU |
| cg24823137 | chr19 | 54290902 | 12 | N | cg27437721 | chr10 | 134420300 | 29 | N |
| cg05882315 | chr19 | 54290905 | 12 | N | cg19895894 | chr10 | 134593496 | 29 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg20644425 | chr19 | 54290913 | 12 | MU | cg16832975 | chr10 | 134593532 | 29 | MU |
| cg08239565 | chr19 | 54290996 | 12 | N | cg07783889 | chr10 | 134593607 | 29 | N |
| cg01975502 | chr19 | 54291017 | 12 | N | cg05480169 | chr10 | 135067408 | 29 | MU |
| cg18403970 | chr19 | 54291050 | 12 | N | cg12587618 | chr11 | 1979879 | 29 | MU |
| cg03347837 | chr2 | 1426181 | 12 | N | cg24769295 | chr11 | 63676485 | 29 | N |
| cg05544715 | chr2 | 1426301 | 12 | MU | cg06982473 | chr11 | 63676611 | 29 | MU |
| cg23421509 | chr2 | 8978201 | 12 | MU | cg09034795 | chr11 | 63676760 | 29 | N |
| cg16333269 | chr2 | 8978225 | 12 | N | cg17676132 | chr11 | 63886204 | 29 | MU |
| cg05937392 | chr2 | 18736132 | 12 | MU | cg18093064 | chr11 | 63886229 | 29 | N |
| cg04837438 | chr2 | 95403063 | 12 | MU | cg04815835 | chr11 | 64522197 | 29 | MU |
| cg27273946 | chr2 | 232113244 | 12 | MU | cg12071131 | chr11 | 64522266 | 29 | N |
| cg17212304 | chr2 | 234394541 | 12 | MU | cg27402782 | chr11 | 65296794 | 29 | MU |
| cg25923162 | chr2 | 234394645 | 12 | N | cg16516712 | chr11 | 70557445 | 29 | MU |
| cg23246666 | chr2 | 236672684 | 12 | MU | cg23055735 | chr11 | 70557464 | 29 | N |
| cg01206017 | chr2 | 236672749 | 12 | N | cg11609668 | chr11 | 117075078 | 29 | MU |
| cg04588969 | chr20 | 36767986 | 12 | MU | cg13909661 | chr12 | 26386833 | 29 | MU |
| cg21880624 | chr21 | 40194678 | 12 | MU | cg11724984 | chr12 | 121890864 | 29 | N |
| cg20434300 | chr21 | 40194735 | 12 | N | cg02827029 | chr12 | 121890907 | 29 | MU |
| cg01414403 | chr22 | 18275680 | 12 | N | cg00634652 | chr13 | 112166963 | 29 | MU |
| cg19552441 | chr22 | 18275800 | 12 | MU | cg21561701 | chr13 | 114193150 | 29 | MU |
| cg00573338 | chr22 | 38479099 | 12 | N | cg03479204 | chr13 | 114193155 | 29 | MU |
| cg06876311 | chr22 | 38479181 | 12 | N | cg24926364 | chr13 | 114748575 | 29 | N |
| cg00111823 | chr22 | 38479215 | 12 | MU | cg00738309 | chr13 | 114748624 | 29 | MU |
| cg26069037 | chr22 | 38479240 | 12 | N | cg12441624 | chr13 | 114748733 | 29 | N |
| cg08363074 | chr22 | 38479288 | 12 | N | cg01515732 | chr14 | 76012927 | 29 | MU |
| cg07100595 | chr3 | 56503840 | 12 | MU | cg13738571 | chr14 | 76012941 | 29 | N |
| cg21425003 | chr3 | 195599105 | 12 | MU | cg00292073 | chr16 | . 1187671 | 29 | MU |
| cg00359285 | chr3 | 195599248 | 12 | N | cg07205796 | chr16 | 1187722 | 29 | MU |
| cg02891522 | chr4 | 1976232 | 12 | N | cg06328338 | chr16 | 1511419 | 29 | MU |
| cg13663170 | chr4 | 1976361 | 12 | MU | cg26923629 | chr16 | 4799203 | 29 | MU |
| cg09965668 | chr4 | 1977395 | 12 | MU | cg06480482 | chr16 | 29265720 | 29 | MU |
| cg18179964 | chr4 | 2933016 | 12 | MU | cg02227036 | chr16 | 50425329 | 29 | MU |
| cg00667841 | chr4 | 3308884 | 12 | N | cg01986767 | chr16 | 50425351 | 29 | MU |
| cg22770294 | chr4 | 3308933 | 12 | MU | cg05922453 | chr16 | 50425414 | 29 | N |
| cg22389275 | chr4 | 3309013 | 12 | N | cg27239168 | chr16 | 58192136 | 29 | MU |
| cg16371080 | chr4 | 3309069 | 12 | MU | cg27238852 | chr16 | 58192174 | 29 | MU |
| cg08206079 | chr4 | 8386188 | 12 | N | cg04832450 | chr16 | 84487078 | 29 | MU |
| cg18999185 | chr4 | 8386198 | 12 | MU | cg04510420 | chr16 | 88102758 | 29 | MU |
| cg09133485 | chr4 | 8386266 | 12 | N | cg27627876 | chr16 | 88102820 | 29 | N |
| cg27656498 | chr5 | 14492820 | 12 | N | cg10278213 | chr16 | 88966229 | 29 | MU |
| cg19800026 | chr5 | 14492945 | 12 | MU | cg08875273 | chr16 | 88966346 | 29 | N |
| cg04719634 | chr5 | 73872294 | 12 | MU | cg06656553 | chr16 | 89960601 | 29 | MU |
| cg08705647 | chr5 | 132608124 | 12 | MU | cg21289738 | chr17 | 8383742 | 29 | N |
| cg27568307 | chr6 | 10617551 | 12 | MU | cg21871213 | chr17 | 8383800 | 29 | MU |
| cg05858612 | chr6 | 10617799 | 12 | MU | cg25495024 | chr17 | 9189607 | 29 | MU |
| cg06024039 | chr6 | 25726884 | 12 | N | cg27198013 | chr17 | 17714293 | 29 | MU |
| cg07077277 | chr6 | 25726912 | 12 | MU | cg09015921 | chr17 | 17714347 | 29 | MU |
| cg10093738 | chr6 | 25726947 | 12 | N | cg22946219 | chr17 | 17714414 | 29 | N |
| cg25090472 | chr6 | 25726953 | 12 | MU | cg17876831 | chr17 | 18918410 | 29 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg11761622 | chr6 | 25726956 | 12 | MU | cg22671072 | chr17 | 18918466 | 29 | N |
| cg13778975 | chr6 | 25727019 | 12 | N | cg24209115 | chr17 | 18918472 | 29 | N |
| cg03347739 | chr6 | 53787668 | 12 | MU | cg23665668 | chr17 | 38245507 | 29 | MU |
| cg17969853 | chr6 | 108615613 | 12 | MU | cg23526824 | chr17 | 38245542 | 29 | N |
| cg16083439 | chr6 | 108615723 | 12 | N | cg10858898 | chr17 | 38245607 | 29 | N |
| cg21158664 | chr7 | 159870 | 12 | MU | cg11316510 | chr17 | 38510570 | 29 | MU |
| cg17907628 | chr7 | 2473529 | 12 | MU | cg12190841 | chr17 | 38510610 | 29 | N |
| cg09523782 | chr7 | 5272064 | 12 | N | cg12438413 | chr17 | 38510643 | 29 | N |
| cg15277378 | chr7 | 5272208 | 12 | MU | cg00439089 | chr17 | 48263150 | 29 | N |
| cg20099458 | chr7 | 5272275 | 12 | N | cg16514513 | chr17 | 48263159 | 29 | N |
| cg13877423 | chr7 | 5521770 | 12 | MU | cg11615029 | chr17 | 48263189 | 29 | MU |
| cg15357078 | chr7 | 151082220 | 12 | MU | cg11993636 | chr17 | 48263198 | 29 | N |
| cg04368808 | chr7 | 151082230 | 12 | N | cg25735490 | chr17 | 48263204 | 29 | MU |
| cg07890882 | chr8 | 232941 | 12 | MU | cg15694704 | chr17 | 78894364 | 29 | N |
| cg13474004 | chr8 | 1783980 | 12 | MU | cg06096901 | chr17 | 78894393 | 29 | N |
| cg16142313 | chr8 | 28950249 | 12 | N | cg21818807 | chr17 | 78894477 | 29 | MU |
| cg07237653 | chr8 | 28950303 | 12 | MU | cg09361653 | chr17 | 78936292 | 29 | N |
| cg23060465 | chr8 | 141625545 | 12 | MU | cg17408291 | chr17 | 78936372 | 29 | MU |
| cg22329153 | chr8 | 144984462 | 12 | N | cg02775417 | chr19 | 643457 | 29 | MU |
| cg24465224 | chr8 | 144984599 | 12 | MU | cg12062672 | chr19 | 643555 | 29 | N |
| cg20253172 | chr1 | 3107290 | 13 | MU | cg14010550 | chr19 | 1009642 | 29 | N |
| cg21865157 | chr1 | 16058422 | 13 | MU | cg24402975 | chr19 | 1009732 | 29 | N |
| cg27187669 | chr1 | 16058427 | 13 | MU | cg02922913 | chr19 | 1009764 | 29 | MU |
| cg03339674 | chr1 | 16058504 | 13 | N | cg19222405 | chr19 | 1826867 | 29 | MU |
| cg08714895 | chr1 | 17996919 | 13 | MU | cg03924776 | chr19 | 1826924 | 29 | N |
| cg00894697 | chr1 | 20826246 | 13 | MU | cg04584009 | chr19 | 1826957 | 29 | N |
| cg00352560 | chr1 | 45106266 | 13 | MU | cg00473257 | chr19 | 3964734 | 29 | N |
| cg24317827 | chr1 | 45106404 | 13 | N | cg06647026 | chr19 | 3964837 | 29 | MU |
| cg00922420 | chr1 | 55529158 | 13 | MU | cg03110167 | chr19 | 3964912 | 29 | N |
| cg03937605 | chr1 | 110167976 | 13 | N | cg24419436 | chr19 | 4446031 | 29 | MU |
| cg25110106 | chr1 | 110167983 | 13 | N | cg23304156 | chr19 | 16466421 | 29 | N |
| cg25772143 | chr1 | 110168021 | 13 | N | cg17702388 | chr19 | 16466480 | 29 | MU |
| cg21644334 | chr1 | 110168049 | 13 | MU | cg04738220 | chr19 | 16466617 | 29 | MU |
| cg06173481 | chr1 | 153658650 | 13 | MU | cg11013793 | chr19 | 16466636 | 29 | N |
| cg24131452 | chr10 | 415359 | 13 | N | cg23696432 | chr19 | 45150725 | 29 | MU |
| cg24136740 | chr10 | 415418 | 13 | MU | cg20469261 | chr19 | 50361793 | 29 | MU |
| cg06920946 | chr10 | 415489 | 13 | N | cg26570040 | chr19 | 50361827 | 29 | N |
| cg00961127 | chr10 | 526813 | 13 | MU | cg23985239 | chr19 | 50361910 | 29 | N |
| cg06338697 | chr10 | 34587539 | 13 | MU | cg22182287 | chr2 | 3452347 | 29 | N |
| cg01520297 | chr10 | 134456336 | 13 | MU | cg00548824 | chr2 | 3452407 | 29 | MU |
| cg25073705 | chr11 | 1008290 | 13 | MU | cg17738010 | chr2 | 3452437 | 29 | N |
| cg18090970 | chr11 | 11409294 | 13 | MU | cg06360976 | chr2 | 3452543 | 29 | MU |
| cg02299195 | chr11 | 48185069 | 13 | MU | cg11642891 | chr2 | 3452563 | 29 | MU |
| cg04743859 | chr11 | 67817488 | 13 | N | cg15807143 | chr2 | 3452624 | 29 | N |
| cg25439758 | chr11 | 67817606 | 13 | MU | cg23079012 | chr2 | 8343710 | 29 | MU |
| cg24659171 | chr11 | 67817691 | 13 | N | cg20944157 | chr2 | 10567750 | 29 | N |
| cg13857569 | chr11 | 75114506 | 13 | MU | cg14245471 | chr2 | 10567843 | 29 | MU |
| cg16029256 | chr11 | 75114523 | 13 | N | cg26160153 | chr2 | 27373014 | 29 | N |
| cg03252786 | chr11 | 125106056 | 13 | MU | cg24874524 | chr2 | 27373088 | 29 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg04164023 | chr11 | 125106101 | 13 | N | cg14949065 | chr2 | 27373128 | 29 | MU |
| cg07747661 | chr11 | 125106135 | 13 | N | cg02930665 | chr2 | 27373213 | 29 | N |
| cg10827094 | chr12 | 117468061 | 13 | MU | cg18703066 | chr2 | 105363536 | 29 | MU |
| cg19528797 | chr12 | 117468110 | 13 | N | cg03305746 | chr2 | 106927199 | 29 | N |
| cg24847996 | chr12 | 123340818 | 13 | MU | cg02517473 | chr2 | 106927320 | 29 | MU |
| cg22346429 | chr12 | 132504721 | 13 | MU | cg02800216 | chr2 | 237542624 | 29 | N |
| cg12068463 | chr12 | 132505658 | 13 | MU | cg20722637 | chr2 | 237542662 | 29 | MU |
| cg21075590 | chr12 | 132505731 | 13 | N | cg27104778 | chr2 | 237542706 | 29 | MU |
| cg05698020 | chr13 | 111163978 | 13 | MU | cg16326123 | chr2 | 240015886 | 29 | MU |
| cg16682206 | chr13 | 111277501 | 13 | N | cg10185853 | chr2 | 241581630 | 29 | MU |
| cg21664614 | chr13 | 111277611 | 13 | MU | cg08597733 | chr20 | 60886158 | 29 | MU |
| cg26350803 | chr13 | 113654392 | 13 | MU | cg03308831 | chr3 | 9258385 | 29 | N |
| cg02887623 | chr14 | 101531755 | 13 | N | cg07611351 | chr3 | 9258512 | 29 | MU |
| cg12782201 | chr14 | 101531768 | 13 | N | cg00897276 | chr3 | 9258573 | 29 | N |
| cg05537796 | chr14 | 101531806 | 13 | N | cg00364339 | chr3 | 126440043 | 29 | MU |
| cg14007694 | chr14 | 101531857 | 13 | N | cg25670583 | chr4 | 715865 | 29 | MU |
| cg25122402 | chr14 | 101531901 | 13 | MU | cg25712567 | chr4 | 715872 . | 29 | N |
| cg19944840 | chr14 | 101531905 | 13 | N | cg16767590 | chr4 | 715950 | 29 | N |
| cg21878393 | chr14 | 101531947 | 13 | N | cg16185996 | chr4 | 1008614 | 29 | N |
| cg01694276 | chr14 | 105247329 | 13 | MU | cg07727358, | chr4 | 1008738 | 29 | MU |
| cg02365967 | chr15 | 28473380 | 13 | N | cg13346820 | chr5 | 865318 | 29 | N |
| cg10011015 | chr15 | 28473421 | 13 | MU | cg11447335 | chr5 | 865350 | 29 | MU |
| cg07281377. | chr16 | 1113891 | 13 | MU | cg07213685 | chr5 | 2169729 | 29 | N |
| cg04262392 | chr16 | 2136746 | 13 | N | cg27369096 | chr5 | 2169769 | 29 | MU |
| cg01288350 | chr16 | 2136783 | 13 | MU | cg26943056 | chr5 | 2169815 | 29 | N |
| cg05752664 | chr16 | 2136842 | 13 | N | cg00319661 | chr5 | 2632178 | 29 | MU |
| cg03412557 | chr16 | 12096923 | 13 | N | cg25645199 | chr5 | 171533681 | 29 | MU |
| cg08015702 | chr16 | 12096947 | 13 | MU | cg11530408 | chr5 | 180028658 | 29 | N |
| cg27619813 | chr16 | 30958065 | 13 | N | cg26555052 | chr5 | 180028767 | 29 | MU |
| cg16402758 | chr16 | 30958190 | 13 | MU | cg07795168 | chr5 | 180028818 | 29 | MU |
| cg04653928 | chr16 | 30958289 | 13 | N | cg08907436 | chr6. | 152125965 | 29 | N |
| cg08401628 | chr16 | 85433676 | 13 | MU | cg07619683 | chr6 | 152126080 | 29 | MU |
| cg02893344 | chr16 | 85517269 | 13 | N | cg07189962 | chr6 | 152126092 | 29 | N |
| cg06081580 | chr16 | 85517323 | 13 | MU | cg25740208 | chr7 | 1001911 | 29 | MU |
| cg05574053 | chr16 | 85517371 | 13 | MU | cg07497218 | chr7 | 1002011 | 29 | N |
| cg16399368 | chr16 | 85517462 | 13 | N | cg18641463 | chr7 | 2144579 | 29 | MU |
| cg02622601 | chr16 | 85537527 | 13 | MU | cg16904636 | chr7 | 4857015 | 29 | N |
| cg05318705 | chr16 | 85537610 | 13 | MU | cg13972986 | chr7 | 4857070 | 29 | MU |
| cg09250149 | chr16 | 85684353 | 13 | MU | cg06947694 | chr7 | 5389271 | 29 | MU |
| cg03004860 | chr16 | 85684468 | 13 | N | cg19547972 | chr7 | 73389575 | 29 | N |
| cg26967566 | chr16 | 85743863 | 13 | MU | cg07993468 | chr7 | 73389632 | 29 | MU |
| cg15446115 | chr16 | 85743927 | 13 | N | cg12548057 | chr7 | 73389660 | 29 | MU |
| cg09051215 | chr16 | 88994192 | 13 | N | cg06746318 | chr7 | 101755186 | 29 | MU |
| cg03704006 | chr16 | 88994295 | 13 | MU | cg18541042 | chr7 | 140227195 | 29 | MU |
| cg05354904 | chr16 | 88994375 | 13 | N | cg13675721 | chr7 | 140227273 | 29 | MU |
| cg04308346 | chr16 | 89203063 | 13 | N | cg03383886 | chr7 | 156336617 | 29 | MU |
| cg00144414 | chr16 | 89203153 | 13 | MU | cg08789101 | chr7 | 156336675 | 29 | N |
| cg00553527 | chr16 | 89763816 | 13 | MU | cg04058237 | chr7 | 156337107 | 29 | N |
| cg10434344 | chr17 | 600550 | 13 | MU | cg21839504 | chr7 | 156337168 | 29 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg02954676 | chr17 | 600586 | 13 | N | cg18195333 | chr7 | 156337209 | 29 | N |
| cg14102880 | chr17 | 915634 | 13 | MU | cg14353728 | chr8 | 140937350 | 29 | N |
| cg17958132 | chr17 | 915739 | 13 | N | cg21602882 | chr8 | 140937410 | 29 | MU |
| cg10370025 | chr17 | 926264 | 13 | N | cg09541794 | chr8 | 144872852 | 29 | MU |
| cg23180978 | chr17 | 926314 | 13 | N | cg14528751 | chr9 " | 91144074 | 29 | MU |
| cg07992519 | chr17 | 926372 | 13 | MU | cg17006413 | chr1 | 942451 | 30 | N |
| cg02217367 | chr17 | 926438 | 13 | N | cg21472517 | chr1 | 942471 | 30 | MU |
| cg22006392 | chr17 | 927457 | 13 | MU | cg18019572 | chr1 | 942489 | 30 | N |
| cg04833819 | chr17 | 927551 | 13 | MU | cg22673826 | chr1 | 942498 | 30 | N |
| cg21768956 | chr17 | 927652 | 13 | N | cg01922485 | chr1 | 942696 | 30 | MU |
| cg23401459 | chr17 | 1027840 | 13 | MU | cg16097329 | chr1 | 3384827 | 30 | N |
| cg12563935 | chr17 | 63163128 | 13 | MU | cg24371534 | chr1 | 3384929 | 30 | MU |
| cg11777611 | chr17 | 63163163 | 13 | MU | cg02328660 | chr1 | 3389744 | 30 | MU |
| cg21879029 | chr17 | 78935175 | 13 | N | cg03050669 | chr1 | 3606777 | 30 | N |
| cg09491897 | chr17 | 78935269 | 13 | N | cg23163013 | chr1 | 3606892 | 30 | MU |
| cg07964113 | chr17 | 78935289 | 13 | MU | cg16401490 | chr1 | 10704953 | 30 | MU |
| cg10407177 | chr18 | 34916713 | 13 | N | cg22347357 | chr1 | 10705035 | 30 | N |
| cg20168823 | chr18 | 34916738 | 13 | MU | cg15074789 | chr1 | 16451437 | 30 | MU |
| cg12228319 | chr18 | 34916814 | 13 | N | cg20288836 | chr1 | 87995129 | 30 | MU |
| cg27349129 | chr19 | 5080888 | 13 | MU | cg21101726 | chr1 | 204124158 | 30 | MU |
| cg00878516 | chr19 | 35842853 | 13 | MU | cg10521650 | chr1 | 246930936 | 30 | N |
| cg25971548 | chr19 | 35843132 | 13 | MU | cg24633745 | chr1 | 246930955 | 30 | MU |
| cg10498798 | chr19 | 35843186 | 13 | N | cg12866090 | chr10 | 3572595 | 30 | N |
| cg18410168 | chr19 | 35843282 | 13 | N | cg07198365 | chr10 | 3572665 | 30 | N |
| cg25690202 | chr19 | 38684328 | 13 | MU | cg06636244 | chr10 | 3572706 | 30 | MU |
| cg24292487 | chr19 | 54963304 | 13 | N | cg13728376 | chr10 | 5523245 | 30 | MU |
| cg08468187 | chr19 | 54963323 | 13 | N | cg25887076 | chr10 | 5576256 | 30 | MU |
| cg02936752 | chr19 | 54963326 | 13 | MU | cg19139727 | chr10 | 35230860 | 30 | MU |
| cg26962002 | chr19 | 54963333 | 13 | MU | cg20122645 | chr10 | 128599008 | 30 | MU |
| cg20631628 | chr19 | 54963341 | 13 | MU | cg21762589 | chr10 | 133796476 | 30 | N |
| cg01336878 | chr19 | 54963396 | 13 | N | cg02835981 | chr10 | 133796494 | 30 | N |
| cg03025986 | chr2 | 3292757 | 13 | MU | cg13599131 | chr10 | 133796523 | 30 | N |
| cg05989746 | chr2 | 3425584 | 13 | N | cg27353308 | chr10 | 133796559 | 30 | MU |
| cg26058508 | chr2 | 3425687 | 13 | MU | cg21134090 | chr11 | 455955 | 30 | N |
| cg15806038 | chr2 | 3601756 | 13 | MU | cg16124920 | chr11 | 456063 | 30 | MU |
| cg22406334 | chr2 | 9788946 | 13 | MU | cg06415550 | chr11 | 456082 | 30 | N |
| cg05824818 | chr2 | 192584931 | 13 | MU | cg03690133 | chr11 | 1483833 | 30 | N |
| cg24428851 | chr2 | 242170166 | 13 | MU | cg02923888 | chr11 | 1483891 | 30 | MU |
| cg09235936 | chr2 | 242170211 | 13 | MU | cg07335189 | chr11 | 1483973 | 30 | MU |
| cg03445663 | chr2 | 242170236 | 13 | N | cg01570480 | chr11 | 64951916 | 30 | MU |
| cg10024484 | chr20 | 20435885 | 13 | MU | cg13762244 | chr11 | 64951960 | 30 | N |
| cg14455541 | chr20 | 49203350 | 13 | MU | cg03241909 | chr11 | 64952034 | 30 | N |
| cg11414263 | chr21 | 47724906 | 13 | MU | cg14139875 | chr11 | 66454595 | 30 | MU |
| cg15294341 | chr21 | 48072745 | 13 | MU | cg22306015 | chr11 | 66454625 | 30 | N |
| cg16973406 | chr22 | 43265991 | 13 | N | cg04822177 | chr11 | 66454651 | 30 | N |
| cg21220614 | chr12 | 43266108 | 13 | MU | cg20892743 | chr11 | 68133159 | 30 | N |
| cg14910055 | chr22 | 43266228 | 13 | N | cg06989074 | chr11 | 68133177 | 30 | N |
| cg19667082 | chr22 | 50898441 | 13 | MU | cg24728180 | chr11 | 68133235 | 30 | MU |
| cg16950088 | chr4 | 899775 | 13 | MU | cg07259651 | chr11 | 129802069 | 30 | MU |

(continued)

| Illumina Probe Name | Chr. | Position | Tissue/Cell Type | MU/N | Illumina Probe Name | Chr. | Position | Tissue/Cell Type | MU/N |
|---|---|---|---|---|---|---|---|---|---|
| cg08604785 | chr4 | 899970 | 13 | MU | cg19711602 | chr12 | 258355 | 30 | MU |
| cg14879397 | chr4 | 899984 | 13 | N | cg14830740 | chr12 | 258436 | 30 | N |
| cg19780308 | chr4 | 140754450 | 13 | MU | cg23884120 | chr12 | 121198406 | 30 | MU |
| cg24715412 | chr4 | 184922571 | 13 | N | cg19468883 | chr12 | 121198447 | 30 | N |
| cg19147428 | chr4 | 184922646 | 13 | MU | cg03782130 | chr12 | 132219020 | 30 | MU |
| cg26402435 | chr4 | 190661492 | 13 | N | cg04345366 | chr12 | 132219026 | 30 | N |
| cg05970547 | chr4 | 190661634 | 13 | MU | cg23244605 | chr13 | 97599787 | 30 | MU |
| cg14612133 | chr5 | 14406585 | 13 | N | cg09426957 | chr13 | 97599820 | 30 | N |
| cg26060817 | chr5 | 14406676 | 13 | N | cg20198393 | chr13 | 101194647 | 30 | MU |
| cg04646987 | chr5 | 14406732 | 13 | MU | cg04832557 | chr13 | 101194666 | 30 | N |
| cg05520500 | chr5 | 176004569 | 13 | MU | cg10130374 | chr13 | 111935412 | 30 | N |
| cg17464807 | chr7 | 1878190 | 13 | N | cg22795345 | chr13 | 111935508 | 30 | MU |
| cg22573590 | chr7 | 1878225 | 13 | N | cg14270002 | chr13 | 111935521 | 30 | MU |
| cg00815484 | chr7 | 1878297 | 13 | MU | cg22032283 | chr13 | 111936044 | 30 | N |
| cg26315617 | chr7 | 3991014 | 13 | MU | cg12512173 | chr13 | 111936059 | 30 | N |
| cg14431986 | chr7 | 3991031 | 13 | MU | cg21613549 | chr13 | 111936071 | 30 | MU |
| cg26180191 | chr7 | 3991335 | 13 | MU | cg09473364 | chr13 | 112927190 | 30 | MU |
| cg25330147 | chr7 | 127344968 | 13 | MU | cg07402939 | chr13 | 112927331 | 30 | N |
| cg01012244 | chr7 | 127344973 | 13 | N | cg13129548 | chr14 | 93405937 | 30 | MU |
| cg06903658 | chr7 | 127532361 | 13 | MU | cg05049312 | chr14 | 93406016 | 30 | N |
| cg25484712 | chr7 | 150828561 | 13 | MU | cg08087268 | chr14 | 93406082 | 30 | N |
| cg16419361 | chr7 | 150828577 | 13 | N | cg16628135 | chr16 | 1397791 | 30 | MU |
| cg06416110 | chr7 | 157190100 | 13 | MU | cg06675348 | chr16 | 11276415 | 30 | N |
| cg03955375 | chr8 | 22477237 | 13 | MU | cg26442107 | chr16 | 11276460 | 30 | MU |
| cg05418938 | chr8 | 22477283 | 13 | N | cg26673975 | chr16 | 66420974 | 30 | MU |
| cg11363615 | chr8 | 41507621 | 13 | MU | cg08842616 | chr16 | 70733772 | 30 | N |
| cg20749005 | chr8 | 140950007 | 13 | N | cg08796240 | chr16 | 70733832 | 30 | MU |
| cg21351483 | chr8 | 140950027 | 13 | MU | cg01033642 | chr16 | 81564192 | 30 | N |
| cg16927379 | chr8 | 140950075 | 13 | N | cg04897892 | chr16 | 81564314 | 30 | MU |
| cg16222790 | chr8 | 144521374 | 13 | MU | cg01753252 | chr16 | 88757588 | 30 | MU |
| cg09890746 | chr8 | 144521404 | 13 | MU | cg02089452 | chr16 | 89500152 | 30 | MU |
| cg02247300 | chr8 | 144521435 | 13 | MU | cg02192318 | chr16 | 89500212 | 30 | N |
| cg17517442 | chr8 | 144521470 | 13 | N | cg12914530 | chr16 | 89500225 | 30 | N |
| cg20008846 | chr8 | 144521520 | 13 | N | cg04237489 | chr17 | 1799551 | 30 | |
| cg11964216 | chr8 | 144521570 | 13 | N | cg14896608 | chr17 | 1799628 | 30 | MU |
| cg20781216 | chr8 | 144546287 | 13 | MU | cg09021817 | chr17 | 1799727 | 30 | N |
| cg21449445 | chr8 | 144546357 | 13 | MU | cg25588480 | chr17 | 4763240 | 30 | MU |
| cg25834632 | chr8 | 144590015 | 13 | MU | cg13483263 | chr17 | 7493150 | 30 | N |
| cg09961689 | chr8 | 144590068 | 13 | N | cg13159929 | chr17 | 7493222 | 30 | MU |
| cg13764850 | chr9 | 94499729 | 13 | MU | cg24060527 | chr17 | 9066156, | 30 | MU |
| cg14060898 | chr9 | 97939026 | 13 | MU | cg20705392 | chr17 | 9066278 | 30 | N |
| cg14406074 | chr9 | 139818362 | 13 | MU | cg12601118 | chr17 | 9859123 | 30 | MU |
| cg14593536 | chr9 | 139818475 | 13 | N | cg09123026 | chr17 | 74480528 | 30 | MU |
| cg14984126 | chr1 | 2409866 | 14 | N | cg09013599 | chr17 | 75207115 | 30 | N |
| cg04341461 | chr1 | 2410006 | 14 | MU | cg04308797 | chr17 | 75207259 | 30 | MU |
| cg00929493 | chr1 | 6314002 | 14 | N | cg17940464 | chr17 | 75207380 | 30 | N |
| cg13071306 | chr1 | 6314113 | 14 | MU | cg01449677 | chr17 | 76794888 | 30 | MU |
| cg05305413 | chr1 | 22327119 | 14 | MU | cg04117396 | chr17 | 76795028 | 30 | N |
| cg15629893 | chr1 | 22327131 | 14 | N | cg22949575 | chr17 | 79948825 | 30 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg01417692 | chr1 | 47078581 | 14 | MU | cg22528521 | chr19 | 1424814 | 30 | N |
| cg22795216 | chr1 | 47078649 | 14 | MU | cg19593255 | chr19 | 1424952 | 30 | MU |
| cg19866325 | chr1 | 47078752 | 14 | MU | cg00766482 | chr19 | 6269688 | 30 | MU |
| cg22424284 | chr1 | 118166402 | 14 | MU | cg07838427 | chr19 | 10665928 | 30 | MU |
| cg12667031 | chr1 | 169878852 | 14 | MU | cg14697425 | chr19 | 14042840 | 30 | MU |
| cg17464228 | chr1 | 169878927 | 14 | N | cg19726935 | chr19 | 17691610 | 30 | N |
| cg17019070 | chr10 | 73466639 | 14 | MU | cg10730370 | chr19 | 17691691 | 30 | MU |
| cg10505163 | chr10 | 73466728 | 14 | N | cg26961332 | chr19 | 18420631 | 30 | MU |
| cg23284338 | chr10 | 73569605 | 14 | N | cg20535805 | chr19 | 18420735 | 30 | N |
| cg26134646 | chr10 | 73569677 | 14 | MU | cg26011534 | chr19 | 54972426 | 30 | N |
| cg05294276 | chr10 | 112618918 | 14 | MU | cg11320517 | chr19 | 54972544 | 30 | MU |
| cg14905600 | chr10 | 134226361 | 14 | MU | cg10814803 | chr2 | 63273940 | 30 | MU |
| cg16334004 | chr10 | 134722168 | 14 | MU | cg24586870 | chr2 | 233198590 | 30 | MU |
| cg09252766 | chr10 | 134722243 | 14 | N | cg26126749 | chr2 | 239970075 | 30 | MU |
| cg06197024 | chr10 | 134940464 | 14 | N | cg08781549 | chr2 | 239970152 | 30 | N |
| cg09216081 | chr10 | 134940484 | 14 | N | cg26641275 | chr2 | 239971554 | 30 | MU |
| cg20559403. | chr10 | 134940595 | 14 | MU | cg13626676 | chr20 | 20377112 | 30 | MU |
| cg08065122 | chr11 | 794433 | 14 | N | cg25478712 | chr21 | 44840120 | 30 | MU |
| cg25211622 | chr11 | 794443 | 14 | MU | cg20341238 | chr21 | 44865764 | 30 | MU |
| cg23571839 | chr11 | 1431343 | 14 | MU | cg07897659 | chr22 | 50316901 | 30 | MU |
| cg07737063 | chr11 | 1486341 | 14 | MU | cg04176532 | chr22 | 50317003 | 30 | N |
| cg26913576 | chr11 | 2151698 | 14 | N | cg20746101 | chr22 | 50725470 | 30 | N |
| cg09503234 | chr11 | 2151721 | 14 | MU | cg23041250 | chr22 | 50725605 | 30 | MU |
| cg02719427 | chr11 | 2151725 | 14 | MU | cg12944146 | chr22 | 50725609 | 30 | MU |
| cg06168166 | chr11 | 47372949 | 14 | N | cg00307254 | chr3 | 126706825 | 30 | MU |
| cg10354134 | chr11 | 47372960 | 14 | MU | cg17228960 | chr3 | 126707271 | 30 | N |
| cg25492363 | chr11 | 57319872 | 14 | MU | cg05119979 | chr3 | 126707413 | 30 | MU |
| cg18159819 | chr11 | 62139609 | 14 | N | cg23340249 | chr3 | 126707501 | 30 | N |
| cg00473624 | chr11 | 62139710 | 14 | MU | cg05485379 | chr3 | 197121410 | 30 | MU |
| cg23508264 | chr11 | 62415024 | 14 | MU | cg25611057 | chr3 | 197121419 | 30 | N |
| cg14684068 | chr11 | 63894400 | 14 | N | cg17900047 | chr4 | 1214930 | 30 | N |
| cg02914667 | chr11 | 63894453 | 14 | N | cg11985754 | chr4 | 1214965 | 30 | MU |
| cg05757474 | chr11 | 63894504 | 14 | MU | cg12389346 | chr4 | 1215112 | 30 | MU |
| cg08561825 | chr11 | 64333366 | 14 | MU | cg10752575 | chr4 | 1215743 | 30 | MU |
| cg18456140 | chr11 | 67810268 | 14 | MU | cg10503298 | chr4 | 1217221 | 30 | MU |
| cg26921533 | chr11 | 67810300 | 14 | N | cg15048480 | chr4 | 1217317 | 30 | N |
| cg02387701 | chr11 | 67810303 | 14 | N | cg17924901 | chr4 | 3475233 | 30 | N |
| cg11410726 | chr11 | 69625409 | 14 | MU | cg05673770 | chr4 | 3475328 | 30 | N |
| cg08684959 | chr11 | 70031709 | 14 | MU | cg21333208 | chr4 | 3475372 | 30 | MU |
| cg23190190 | chr11 | 70031725 | 14 | N | cg05144635 | chr4 | 7218707 | 30 | MU |
| cg00203347 | chr12 | 108603943 | 14 | MU | cg14378364 | chr4 | 123591475 | 30 | MU |
| cg08085917 | chr12 | 133147010 | 14 | MU | cg01094351 | chr4 | 153179467 | 30 | MU |
| cg17902007 | chr13 | 101184847 | 14 | MU | cg11802666 | chr4 | 185345707 | 30 | MU |
| cg02688393 | chr13 | 111109497 | 14 | MU | cg06277588 | chr6 | 13489246 | 30 | N |
| cg11118697 | chr13 | 112856684 | 14 | N | cg18671057 | chr6 | 13489248 | 30 | MU |
| cg22528556 | chr13 | 112856721 | 14 | N | cg21893764 | chr6 | 33145523 | 30 | N |
| cg07949720 | chr13 | 112856747 | 14 | MU | cg12312338 | chr6 | 33145571 | 30 | MU |
| cg00747890 | chr14 | 90854534 | 14 | MU | cg00944666 | chr6 | 107926037 | 30 | MU |
| cg13114125 | chr14 | 105738426 | 14 | MU | cg03113916 | chr6 | 143660870 | 30 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg23121787 | chr15 | 42211437 | 14 | N | cg20067575 | chr6 | 150437072 | 30 | MU |
| cg04489066 | chr15 | 42211509 | 14 | N | cg04076264 | chr6 | 150437118 | 30 | MU |
| cg27276079 | chr15 | 42211520 | 14 | MU | cg16306083 | chr6 | 170449858 | 30 | N |
| cg17806437 | chr15 | 42211581 | 14 | MU | cg14218838 | chr6 | 170449884 | 30 | MU |
| cg09204108 | chr15 | 42211679 | 14 | N | cg02152290 | chr7 | 551601 | 30 | MU |
| cg04656171 | chr15 | 42211689 | 14 | N | cg13381243 | chr7 | 1103063 | 30 | N |
| cg01979489 | chr16 | 332603 | 14 | N | cg02332902 | chr7 | 1103177 | 30 | MU |
| cg27392802 | chr16 | 332705 | 14 | MU | cg09526129 | chr7 | 1103262 | 30 | N |
| cg09357140 | chr16 | 332745 | 14 | N | cg16601832 | chr7 | 1514361 | 30 | N |
| cg02428506 | chr16 | 332825 | 14 | N | cg23956190 | chr7 | 1514455 | 30 | MU |
| cg05349077 | chr16 | 630159 | 14 | MU | cg14576951 | chr7 | 1514568 | 30 | MU |
| cg19791043 | chr16 | 1116361 | 14 | N | cg00906908 | chr7 | 1514623 | 30 | N |
| cg08056755 | chr16 | 1116397 | 14 | MU | cg00035074 | chr7 | 2701567 | 30 | N |
| cg02403239 | chr16 | 1252140 | 14 | MU | cg21452471 | chr7 | 2701636 | 30 | MU |
| cg04834608 | chr16 | 3706634 | 14 | MU | cg17337874 | chr7 | 2701641 | 30 | N |
| cg05186188 | chr16 | 25228569 | 14 | N | cg13320898 | chr7 | 4801923 | 30 | MU |
| cg26807107 | chr16 | 25228603 | 14 | MU | cg23871697 | chr7 | 4801993 | 30 | N |
| cg26506947 | chr16 | 25228688 | 14 | N | cg25204742 | chr7 | 6448933 | 30 | MU |
| cg03547812 | chr16 | 25228709 | 14 | N | cg00437258 | chr7 | 6449066 | 30 | N |
| cg03934313 | chrl6 | 67696043 | 14 | N | cg02813710 | chr7 | 105079663 | 30 | MU |
| cg26685294 | chr16 | 67696047 | 14 | N | cg00415665 | chr8 | 123875036 | 30 | MU |
| cg27219874 | chr16 | 67696095 | 14 | N | cg26427777 | chr8 | 123875181 | 30 | N |
| cg04788956 | chr16 | 67696138 | 14 | MU | cg05851505 | chr8 | 142221164 | 30 | MU |
| cg00318436 | chr16 | 67696181 | 14 | N | cg07695089 | chr8 | 142221192 | 30 | N |
| cg04607024 | chr16 | 67696204 | 14 | N | cg14650228 | chr8 | 144573510 | 30 | MU |
| cg01342411 | chr16 | 75263551 | 14 | MU | cg25834632 | chr8 | 144590015 | 30 | MU |
| cg06535849 | chr16 | 81925084 | 14 | MU | cg09961689 | chr8 | 144590068 | 30 | N |
| cg01376763 | chr16 | 81925132 | 14 | N | cg03222672 | chr9 | 125878106 | 30 | MU |
| cg07124126 | chr16 | 81927346 | 14 | MU | cg14233878 | chr9 | 139546555 | 30 | MU |
| cg07397481 | chr16 | 81927366 | 14 | N | cg13407601 | chr9 | 140615230 | 30 | MU |
| cg08478121 | chr16 | 88545571 | 14 | MU | cg09022584 | chr1 | 15541956 | 31 | MU |
| cg05749855 | chr16 | 89472771 | 14 | MU | cg05398883 | chr1 | 42630335 | 31 | MU |
| cg02938172 | chr17 | 185152 | 14 | N | cg13452215 | chr1 | 42630436 | 31 | N |
| cg03379477 | chr17 | 185227 | 14 | N | cg06779393 | chr1 | 159684337 | 31 | N |
| cg05526731 | chr17 | 185263 | 14 | MU | cg08474603 | chr1 | 159684431 | 31 | MU |
| cg25732961 | chr17 | 7755877 | 14 | MU | cg14015211 | chr1 | 223315766 | 31 | MU |
| cg11903177 | chr17 | 17379358 | 14 | MU | cg05858079 | chr1 | 223315886 | 31 | N |
| cg01084215 | chr17 | 77924269 | 14 | N | cg04850059 | chr1 | 225883161 | 31 | MU |
| cg14505694 | chr17 | 77924275 | 14 | N | cg12513686 | chr10 | 709592 | 31 | MU |
| cg18749563 | chr17 | 77924371 | 14 | MU | cg16859636 | chr10 | 709610 | 31 | MU |
| cg06582663 | chr17 | 79043264 | 14 | MU | cg15247093 | chr10 | 47768476 | 31 | MU |
| cg25342872 | chr17 | 79175132 | 14 | MU | cg00820740 | chr10 | 60274755 | 31 | MU |
| cg13292542 | chr17 | 80394588 | 14 | MU | cg09869167 | chr10 | 95428731 | 31 | MU |
| cg09414987 | chr19 | 392121 | 14 | MU | cg03289548 | chr10 | 114580989 | 31 | MU |
| cg22548831 | chr19 | 398562 | 14 | MU | cg12829687 | chr10 | 114581004 | 31 | MU |
| cg24372485 | chr19 | 2078331 | 14 | MU | cg07591090 | chr10 | 114877616 | 31 | MU |
| cg05012672 | chr19 | 3189830 | 14 | MU | cg06403317 | chr10 | 114877652 | 31 | MU |
| cg24301352 | chr19 | 3189957 | 14 | N | cg12226006 | chr10 | 123350909 | 31 | MU |
| cg22551372 | chr19 | 18092728 | 14 | N | cg16244248 | chr10 | 134594987 | 31 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg05369268 | chr19 | 18092781 | 14 | MU | cg03875091 | chr10 | 134595048 | 31 | MU |
| cg03117053 | chr19 | 34263644 | 14 | MU | cg12839363 | chr11 | 12650181 | 31 | MU |
| cg10193069 | chr19 | 50058971 | 14 | N | cg24925907 | chr11 | 33715619 | 31 | MU |
| cg12149986 | chr19 | 50059027 | 14 | MU | cg09705456 | chr11 | 63839479 | 31 | MU |
| cg16028175 | chr19 | 50059164 | 14 | N | cg27456707 | chr11 | 63839511 | 31 | N |
| cg21292909 | chr19 | 50059937 | 14 | MU | cg03460534 | chr11 | 63839528 | 31 | N |
| cg04355077 | chr19 | 50059946 | 14 | MU | cg04720283 | chr11 | 103914796 | 31 | MU |
| cg24292487 | chr19 | 54963304 | 14 | N | cg14546955 | chr11 | 105959819 | 31 | MU |
| cg08468187 | chr19 | 54963323 | 14 | N | cg09688422 | chr11 | 105959824 | 31 | N |
| cg02936752 | chr19 | 54963326 | 14 | N | cg03252786 | chr11 | 125106056 | 31 | MU |
| cg26962002 | chr19 | 54963333 | 14 | MU | cg04164023 | chr11 | 125106101 | 31 | N |
| cg20631628 | chr19 | 54963341 | 14 | MU | cg07747661 | chr11 | 125106135 | 31 | N |
| cg01336878 | chr19 | 54963396 | 14 | N | cg02591564 | chr12 | 125578355 | 31 | MU |
| cg08182975 | chr2 | 7164527 | 14 | MU | cg01392656 | chr12 | 125578483 | 31 | N |
| cg20995753 | chr2 | 7164586 | 14 | N | cg00440032 | chr12 | 127257679 | 31 | MU |
| cg00049868 | chr2 | 11245407 | 14 | MU | cg14662172 | chr13 | 46679081 | 31 | MU |
| cg19246065 | chr2 | 11245457 | 14 | N | cg00049616 | chr13 | 100308186 | 31 | MU |
| cg24618467 | chr2 | 11245514 | 14 | N | cg15982595 | chr13 | 113301180 | 31 | MU |
| cg20699463 | chr2 | 23785143 | 14 | MU | cg25319564 | chr13 | 114214573 | 31 | MU |
| cg25365034 | chr2 | 23785230 | 14 | N | cg16341690 | chr14 | 23291090 | 31 | N |
| cg03170725 | chr2 | 27498214 | 14 | N | cg08319417 | chr14 | 23291214 | 31 | MU |
| cg04505439 | chr2 | 27498232 | 14 | N | cg18703243 | chr14 | 62021555 | 31 | MU |
| cg13830506 | chr2 | 27498285 | 14 | N | cg15994519 | chr14 | 71585510 | 31 | MU |
| cg20128772 | chr2 | 27498295 | 14 | MU | cg16245111 | chr14 | 96750514 | 31 | MU |
| cg16964535 | chr2 | 27498334 | 14 | N | cg07054904 | chr14 | 96750522 | 31 | MU |
| cg20585358 | chr2 | 98354223 | 14 | MU | cg10196001 | chr14 | 96750614 | 31 | MU |
| cg15679219 | chr2 | 98354261 | 14 | N | cg12195230 | chr14 | 103470300 | 31 | MU |
| cg20076826 | chr2 | 98354292 | 14 | N | cg18327049 | chr14 | 103470338 | 31 | MU |
| cg02201132 | chr2 | 242212884 | 14 | N | cg24043286 | chr14 | 104761941 | 31 | N |
| cg20333001 | chr2 | 242212896 | 14 | N | cg14191343 | chr14 | 104762027 | 31 | N |
| cg19372975 | chr2 | 242212901 | 14 | MU | cg09947795 | chr14 | 104762067 | 31 | MU |
| cg02163943 | chr20 | 42217859 | 14 | MU | cg09088406 | chr15 | 27819306 | 31 | N |
| cg18326819 | chr21 | 46801257 | 14 | MU | cg13159361 | chr15 | 27819341 | 31 | N |
| cg03863756 | chr21 | 46859983 | 14 | MU | cg07521193 | chr15 | 27819422 | 31 | MU |
| cg25060632 | chr22 | 46683277 | 14 | MU | cg09971674 | chr15 | 58853142 | 31 | MU |
| cg27309229 | chr22 | 49085091 | 14 | MU | cg24530041 | chr15 | 90349551 | 31 | N |
| cg02579469 | chr5 | 1378350 | 14 | MU | cg16914674 | chr15 | 90349662 | 31 | MU |
| cg03679715 | chr5 | 176295869 | 14 | N | cg07670266 | chr16 | 2087377 | 31 | MU |
| cg09582314 | chr5 | 176295891 | 14 | MU | cg09969487 | chr16 | 49902325 | 31 | MU |
| cg09800974 | chr5 | 178688272 | 14 | MU | cg08138817 | chr16 | 49902342 | 31 | N |
| cg13398440 | chr5 | 178688308 | 14 | N | cg05819154 | chr16 | 49902436 | 31 | N |
| cg04020211 | chr5 | 179763432 | 14 | MU | cg00263677 | chr16 | 84885230 | 31 | MU |
| cg14465148 | chr6 | 3329791 | 14 | N | cg05640992 | chr16 | 87970367 | 31 | MU |
| cg08546278 | chr16 | 3329904 | 14 | N | cg03550864 | chr16 | 87970376 | 31 | N |
| cg00132509 | chr6 | 3329936 | 14 | MU | cg04245568 | chr16 | 88453579 | 31 | MU |
| cg02964172 | chr6 | 10599459 | 14 | MU | cg10218510 | chr16 | 88453688 | 31 | N |
| cg27594095 | chr6 | 31095276 | 14 | MU | cg06489433 | chr16 | 89491229 | 31 | MU |
| cg01270038 | chr6 | 36343705 | 14 | MU | cg09632029 | chr17 | 18855506 | 31 | MU |
| cg24870688 | chr6 | 168399841 | 14 | MU | cg18536123 | chr17 | 26694939 | 31 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg23951305 | chr6 | 170581890 | 14 | N | cg03028529 | chr17 | 26695002 | 31 | N |
| cg04030444 | chr6 | 170581971 | 14 | MU | cg20875182 | chr18 | 44561130 | 31 | MU |
| cg07179981 | chr7 | 922051 | 14 | N | cg05989423 | chr18 | 44561188 | 31 | N |
| cg24902995 | chr7 | 922165 | 14 | MU | cg15084903 | chr19 | 376055 | 31 | MU |
| cg24046689 | chr7 | 922235 | 14 | MU | cg26901714 | chr19 | 376152 | 31 | N |
| cg06314184 | chr7 | 4876143 | 14 | MU | cg15470261 | chr19 | 1698699 | 31 | MU |
| cg01432945 | chr7 | 30946639 | 14 | MU | cg23931381 | chr19 | 18119847 | 31 | MU |
| cg22821931 | chr7 | 50800837 | 14 | N | cg05845141 | chr19 | 18120614 | 31 | N |
| cg00228281 | chr7 | 50800963 | 14 | MU | cg12965095 | chr19 | 18120626 | 31 | MU |
| cg11867552 | chr7 | 50801000 | 14 | N | cg03014882 | chr19 | 18120728 | 31 | MU |
| cg02468316 | chr7 | 50801032 | 14 | N | cg00474840 | chr19 | 18120819 | 31 | MU |
| cg22884541 | chr7 | 97841864 | 14 | MU | cg04583529 | chr19 | 19230594 | 31 | MU |
| cg07740640 | chr7 | 97841941 | 14 | N | cg12249345 | chr19 | 45316493 | 31 | N |
| cg13918611 | chr7 | 97841998 | 14 | N | cg21978694 | chr19 | 45316509 | 31 | MU |
| cg18550381 | chr7 | 97844391 | 14 | N | cg22683283 | chr2 | 37375983 | 31 | N |
| cg21099419 | chr7 | 97844473 | 14 | MU | cg11344073 | chr2 | 37376067 | 31 | MU |
| cg06569421 | chr7 | 101509114 | 14 | N | cg10317145 | chr2 | 44065680 | 31 | N |
| cg27324804 | chr7 | 101509161 | 14 | MU | cg00459909 | chr2 | 44065720 | 31 | N |
| cg08765199 | chr7 | 130021519 | 14 | N | cg20926720 | chr2 | 44065725 | 31 | MU |
| cg21115346 | chr7 | 130021544 | 14 | N | cg07681696 | chr2 | 44065858 | 31 | N |
| cg10663408 | chr7 | 130021651 | 14 | MU | cg02329386 | chr2 | 70055719 | 31 | MU |
| cg09499844 | chr8 | 129045722 | 14 | MU | cg02078517 | chr2 | 75720511 | 31 | MU |
| cg14229207 | chr9 | 135929747 | 14 | MU | cg00409636 | chr2 | 75720541 | 31 | MU |
| cg14570652 | chr9 | 139393736 | 14 | MU | cg00533827 | chr2 | 86163827 | 31 | MU |
| cg20253172 | chr1 | 3107290 | 15 | MU | cg06900782 | chr2 | 201168293 | 31 | MU |
| cg05656251 | chr1 | 3125906 | 15 | N | cg23585686 | chr2 | 242787243 | 31 | N |
| cg19706803 | chr1 | 3126033 | 15 | MU | cg23899450 | chr2 | 242787324 | 31 | MU |
| cg25349768 | chr1 | 3126154 | 15 | N | cg03862380 | chr20 | 43029895 | 31 | N |
| cg02769865 | chr1 | 9789982 | 15 | MU | cg11542165 | chr20 | 43029992 | 31 | N |
| cg11697440 | chr1 | 19280186 | 15 | MU | cg07064544 | chr20 | 43029994 | 31 | MU |
| cg06779393 | chr1 | 159684337 | 15 | N | cg21058973 | chr20 | .43029997 | 31 | MU |
| cg08474603 | chr1 | 159684431 | 15 | MU | cg03451557 | chr20 | 57570581 | 31 | MU |
| cg09168939 | chr1 | 164652019 | 15 | MU | cg17803226 | chr20 | 57570685 | 31 | N |
| cg24408776 | chr1 | 203098067 | 15 | MU | cg10394047 | chr21 | 47575416 | 31 | N |
| cg05803596 | chr1 | 203098085 | 15 | N | cg09436823 | chr21 | 47575498 | 31 | MU |
| cg00820740 | chr10 | 60274755 | 15 | MU | cg18024037 | chr21 | 47575504 | 31 | MU |
| cg10870587 | chr10 | 95249627 | 15 | MU | cg25322086 | chr21 | 47575547 | 31 | N |
| cg22973172 | chr10 | 105492643 | 15 | MU | cg10788213 | chr22 | 45719176 | 31 | MU |
| cg03289548 | chr10 | 114580989 | 15 | MU | cg18465245 | chr3 | 30737566 | 31 | MU |
| cg12829687 | chr10 | 114581004 | 15 | MU | cg11314274 | chr3 | 132004064 | 31 | MU |
| cg07591090 | chr10 | 114877616 | 15 | MU | cg10029905 | chr3 | 170716743 | 31 | MU |
| cg06403317 | chr10 | 114877652 | 15 | MU | cg21339581 | chr4 | 1800987 | 31 | N |
| cg02208891 | chr10 | 126205741 | 15 | MU | cg12997579 | chr4 | 1801063 | 31 | MU |
| cg14687118 | chr10 | 126205766 | 15 | MU | cg13131329 | chr4 | 1801148 | 31 | N |
| cg06139099 | chr10 | 134499505 | 15 | N | cg01795199 | chr4 | 1808807 | 31 | MU |
| cg00458454 | chr10 | 134499528 | 15 | MU | cg15697036 | chr4 | 1808923 | 31 | N |
| cg13441142 | chr10 | 134499734 | 15 | MU | cg08216584 | chr4 | 72056209 | 31 | MU |
| cg02541125 | chr10 | 134499752 | 15 | MU | cg00159379 | chr4 | 85818877 | 31 | MU |
| cg15216078 | chr10 | 134567188 | 15 | N | cg20481837 | chr4 | 151501627 | 31 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg16041156 | chr10 | 134567277 | 15 | N | cg17433772 | chr4 | 151501739 | 31 | MU |
| cg01119718 | chr10 | 134567309 | 15 | MU | cg05878390 | chr4 | 151502935 | 31 | N |
| cg03108697 | chr11 | 9732066 | 15 | MU | cg20334738 | chr4 | 151502939 | 31 | MU |
| cg12839363 | chr11 | 12650181 | 15 | MU | cg25053484 | chr4 | 187078828 | 31 | MU |
| cg24925907 | chr11 | 33715619 | 15 | MU | cg25478037 | chr5 | 55731675 | 31 | MU |
| cg20219100 | chr11 | 63776397 | 15 | N | cg12329933 | chr5 | 59968073 | 31 | MU |
| cg12573253 | chr11 | 63776419 | 15 | MU | cg24838839 | chr5 | 61031569 | 31 | MU |
| cg19960891 | chr11 | 63776653 | 15 | MU | cg06376277 | chr5 | 176520130 | 31 | N |
| cg09705456 | chr11 | 63839479 | 15 | MU | cg11112931 | chr5 | 176520269 | 31 | MU |
| cg27456707 | chr11 | 63839511 | 15 | N | cg02044145 | chr5 | 176520341 | 31 | N |
| cg03460534 | chr11 | 63839528 | 15 | N | cg22469020 | chr6 | 24240935 | 31 | MU |
| cg04743859 | chr11 | 67817488 | 15 | N | cg27613749 | chr6 | 25756473 | 31 | MU |
| cg25439758 | chr11 | 67817606 | 15 | MU | cg15138473 | chr6 | 83757182 | 31 | MU |
| cg24659171 | chr11 | 67817691 | 15 | N | cg09860783 | chr6 | 149777984 | 31 | MU |
| cg15934634 | chr11 | 68920095 | 15 | MU | cg03813151 | chr6 | 149778058 | 31 | N |
| cg01196123 | chr12 | 27414167 | 15 | MU | cg08860941 | chr6 | 167633955 | 31 | MU |
| cg03762760 | chr12 | 33031216 | 15 | MU | cg24915751 | chr6 | 170561236 | 31 | MU |
| cg00759707 | chr12 | 110788669 | 15 | MU | cg24645679 | chr7 | 2611236 | 31 | MU |
| cg01699430 | chr12 | 111484977 | 15 | MU | cg26765385 | chr7 | 2611244 | 31 | N |
| cg17304276 | chr12 | 111485005 | 15 | N | cg12964881 | chr7 | 2611301 | 31 | MU |
| cg27650540 | chr12 | 111664582 | 15 | N | cg06104667 | chr7 | 72742068 | 31 | MU |
| cg24309040 | chr12 | 111664600 | 15 | MU | cg19343600 | chr7 | 72742143 | 31 | N |
| cg05979433 | chr12 | 111664628 | 15 | MU | cg05669157 | chr7 | 72742156 | 31 | N |
| cg11813690 | chr12 | 111664723 | 15 | N | cg03743037 | chr7 | 134673090 | 31 | MU |
| cg09798033 | chr12 | 111664754 | 15 | N | cg15123087 | chr7 | 144056518 | 31 | MU |
| cg21905990 | chr12 | 129429812 | 15 | MU | cg06435374 | chr8 | 26474480 | 31 | MU |
| cg07791585 | chr13 | 73826369 | 15 | MU | cg26461905 | chr8 | 86349742 | 31 | MU |
| cg15982595 | chr13 | 113301180 | 15 | MU | cg13958215 | chr8 | 86349754 | 31 | MU |
| cg12557626 | chr13 | 113977332 | 15 | MU | cg07912402 | chr1 | 989305 | 32 | MU |
| cg14131555 | chr13 | 113977481 | 15 | N | cg04274259 | chr1 | 989392 | 32 | N |
| cg26136325 | chr14 | 88855259 | 15 | MU | cg06181703 | chr1 | 1028878 | 32 | MU |
| cg16245111 | chr14 | 96750514 | 15 | MU | cg23887776 | chr1 | 2827798 | 32 | N |
| cg07054904 | chr14 | 96750522 | 15 | MU | cg15356461 | chr1 | 2827833 | 32 | MU |
| cg10196001 | chr14 | 96750614 | 15 | MU | cg07212761 | chr1 | 3199650 | 32 | MU |
| cg09088406 | chr15 | 27819306 | 15 | N | cg00850612 | chr1 | 6131676 | 32 | MU |
| cg13159361 | chr15 | 27819341 | 15 | N | cg13898470 | chr1 | 6688607 | 32 | MU |
| cg07521193 | chr15 | 27819422 | 15 | MU | cg17517799 | chr1 | 6688679 | 32 | N |
| cg01191815 | chr15 | 63084959 | 15 | MU | cg18595639 | chr1 | 9324274 | 32 | N |
| cg01144019 | chr15 | 63188741 | 15 | MU | cg13131263 | chr1 | 9324393 | 32 | MU |
| cg21245492 | chr15 | 99250440 | 15 | MU | cg20246820 | chr1 | 9324688 | 32 | MU |
| cg27351816 | chr15 | 102157488 | 15 | MU | cg17114375 | chr1 | 9324830 | 32 | N |
| cg20983577 | chr15 | 102157577 | 15 | N | cg10494257 | chr1 | 16342123 | 32 | N |
| cg09807446 | chr16 | 1171603 | 15 | MU | cg24140574 | chr1 | 16342155 | 32 | MU |
| cg26749361 | chr16 | 30455027 | 15 | MU | cg06441514 | chr1 | 148596792 | 32 | MU |
| cg06790197 | chr16 | 73265937 | 15 | N | cg09853570 | chr1 | 228403422 | 32 | N |
| cg07178278 | chr16 | 73266047 | 15 | MU | cg22850968 | chr1 | 228403515 | 32 | MU |
| cg08401628 | chr16 | 85433676 | 15 | MU | cg03981727 | chr1 | 228470802 | 32 | MU |
| cg10019392 | chr16 | 87934324 | 15 | MU | cg12209313 | chr1 | 228470904 | 32 | N |
| cg01510696 | chr16 | 89688145 | 15 | MU | cg00532451 | chrl | 228528913 | 32 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg00487989 | chr16 | 89688244 | 15 | MU | cg13791379 | chrl | 228528979 | 32 | N |
| cg06066694 | chr16 | 89688353 | 15 | N | cg17975299 | chrl | 247270007 | 32 | N |
| cg05184882 | chr17 | 55509593 | 15 | MU | cg19356179 | chr1 | 247270087 | 32 | N |
| cg14245202 | chr17 | 76264037 | 15 | MU | cg26369133 | chrl | 247270143 | 32 | MU |
| cg08845329 | chr17 | 80932127 | 15 | MU | cg17282060 | chr10 | 49658320 | 32 | MU |
| cg24789587 | chr19 | 990235 | 15 | MU | cg12841513 | chr10 | 99329961 | 32 | N |
| cg05881698 | chr19 | 5818763 | 15 | MU | cg07748741 | chr10 | 99330056 | 32 | MU |
| cg14333565 | chr19 | 5824065 | 15 | N | cg02613380 | chr10 | 99330076 | 32 | N |
| cg25544164 | chr19 | 5824159 | 15 | MU | cg15232718 | chr10 | 99330203 | 32 | N |
| cg13361749 | chr19 | 5824196 | 15 | N | cg19126889 | chr10 | 131565313 | 32 | N |
| cg10201616 | chr19 | 11198556 | 15 | MU | cg15986238 | chr10 | 131565319 | 32 | MU |
| cg22191277 | chr19 | 33870347 | 15 | MU | cg22918560 | chr10 | 133775705 | 32 | N |
| cg02329386 | chr2 | 70055719 | 15 | MU | cg26869506 | chr10 | 133775762 | 32 | MU |
| cg07941953 | chr20 | 10508668 | 15 | MU | cg14138475 | chr10 | 133775839 | 32 | N |
| cg15010213 | chr20 | 61150489 | 15 | MU | cg16803967 | chr10 | 133872299 | 32 | N |
| cg16226106 | chr20 | 61150516 | 15 | N | cg05435315 | chr10 | 133872448 | 32 | MU |
| cg23792592 | chr20 | 61150637 | 15 | N | cg16415825 | chr10 | 133872532 | 32 | N |
| cg10628201 | chr22 | 21977597 | 15 | MU | cg27412039 | chr11 | 1947601 | 32 | MU |
| cg20680669 | chr22 | 28189870 | 15 | MU | cg06455375 | chr11 | 1947743 | 32 | N |
| cg18465245 | chr3 | 30737566 | 15 | MU | cg15432903 | chr11 | 17409602 | 32 | N |
| cg27318481 | chr3 | 100970896 | 15 | MU | cg11839944 | chr11 | 17409644 | 32 | MU |
| cg05711023 | chr3 | 156511814 | 15 | MU | cg05263649 | chr11 | 63916644 | 32 | N |
| cg01731023 | chr4 | 1363486 | 15 | MU | cg18998442 | chr11 | 63916676 | 32 | N' |
| cg20249882 | chr4 | 1364046 | 15 | MU | cg04140971 | chr11 | 63916691 | 32 | MU |
| cg20099968 | chr4 | 1364147 | 15 | MU | cg05564438 | chr11 | 64120286 | 32 | N |
| cg27240402 | chr4 | 1364170 | 15 | MU | cg04162999 | chr11 | 64120313 | 32 | MU |
| cg24232092 | chr4 | 1364344 | 15 | MU | cg24438178 | chr11 | 64120354 | 32 | N |
| cg04742135 | chr4 | 1800154 | 15 | MU | cg14476630 | chr11 | 72437731 | 32 | MU |
| cg02350535 | chr4 | 1800191 | 15 | N | cg23249399 | chr11 | 72437871 | 32 | N |
| cg24979233 | chr4 | 1800284 | 15 | N | cg05824174 | chr12 | 3142845 | 32 | MU |
| cg21339581 | chr4 | 1800987 | 15 | N | cg19862427 | chr12 | 111358545 | 32 | N |
| cg12997579 | chr4 | 1801063 | 15 | MU | cg09763086 | chr12 | 111358601 | 32 | N |
| cg13131329 | chr4 | 1801148 | 15 | N | cg23533926 | chr12 | 111358616 | 32 | N |
| cg01903185 | chr4 | 1808586 | 15 | MU | cg01197294 | chr12 | 111358627 | 32 | N |
| cg01795199 | chr4 | 1808807 | 15 | MU | cg04735123 | chr12 | 111358639 | 32 | MU |
| cg15697036 | chr4 | 1808923 | 15 | N | cg04243901 | chr12 | 125441307 | 32 | MU |
| cg08216584 | chr4 | 72056209 | 15 | MU | cg25769122 | chr12 | 132939657 | 32 | N |
| cg27442676 | chr4 | 72070667 | 15 | MU | cg10426234 | chr12 | 132939776 | 32 | MU |
| cg26850677 | chr4 | 183710344 | 15 | N | cg19444998 | chr12 | 133352427 | 32 | N |
| cg06366833 | chr4 | 183710473 | 15 | MU | cg22683624 | chr12 | 133352552 | 32 | MU |
| cg24838839 | chr5 | 61031569 | 15 | MU | cg21074877 | chr14 | 23481968 | 32 | MU |
| cg02377021 | chr5 | 71537334 | 15 | MU | cg15361373 | chr14 | 70316621 | 32 | MU |
| cg21569714 | chr5 | 153798362 | 15 | MU | cg23319601 | chr14 | 70316638 | 32 | MU |
| cg18994744 | chr5 | 166802318 | 15 | N | cg02973354 | chr14 | 70316670 | 32 | N |
| cg06651505 | chr5 | 166802425 | 15 | MU | cg03991106 | chr15 | 101661445 | 32 | MU |
| cg09800974 | chr5 | 178688272 | 15 | MU | cg13480902 | chr15 | 101661529 | 32 | N |
| cg13398440 | chr5 | 178688308 | 15 | N | cg22551145 | chr15 | 101661580 | 32 | N |
| cg22469020 | chr6 | 24240935 | 15 | MU | cg26619740 | chr16 | 971556 | 32 | N |
| cg15562022 | chr6 | 31167427 | 15 | MU | cg08214990 | chr16 | 971579 | 32 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg16887420 | chr6 | 131548063 | 15 | MU | cg02333298 | chr16 | 27052228 | 32 | N |
| cg00788354 | chr6 | 168199725 | 15 | MU | cg04172166 | chr16 | 27052337 | 32 | MU |
| cg02070077 | chr6 | 168199753 | 15 | MU | cg03790192 | chr16 | 88044375 | 32 | N |
| cg24652281 | chr6 | 168970774 | 15 | N | cg16537589 | chr16 | 88044443 | 32 | MU |
| cg13517866 | chr6 | 168970787 | 15 | MU | cg19809575 | chr16 | 88044501 | 32 | N |
| cg26912251 | chr6 | 168970824 | 15 | N | cg06320420 | chr16 | 88872998 | 32 | MU |
| cg06721601 | chr7 | 101762633 | 15 | MU | cg10155891 | chr16 | 88941576 | 32 | MU |
| cg03202545 | chr7 | 139359312 | 15 | MU | cg03233548 | chr16 | 88949985 | 32 | N |
| cg14940444 | chr7 | 141646270 | 15 | MU | cg08351911 | chr16 | 88950075 | 32 | MU |
| cg04127867 | chr7 | 156781786 | 15 | MU | cg01168962 | chr16 | 88950197 | 32 | MU |
| cg27637873 | chr8 | 102804678 | 15 | MU | cg00957684 | chr16 | 88962800 | 32 | MU |
| cg01211852 | chr8 | 144471653 | 15 | N | cg06153386 | chr16 | 89764255 | 32 | N |
| cg10643493 | chr8 | 144471690 | 15 | MU | cg08013914 | chr16 | 89764279 | 32 | MU |
| cg13601936 | chr9 | 140198751 | 15 | MU | cg02128278 | chr16 | 89764314 | 32 | N |
| cg00386984 | chr1 | 2249617 | 16 | MU | cg00627045 | chr17 | 11461244 | 32 | N |
| cg23049737 | chr1 | 8412697 | 16 | MU | cg11471805 | chr17 | 11461316 | 32 | MU |
| cg18184092 | chr1 | 8412759 | 16 | N | cg19586165 | chr17 | 37814072 | 32 | N |
| cg20163085 | chr1 | 10510396 | 16 | MU | cg00129232 | chr17 | 37814104 | 32 | MU |
| cg04256470 | chr1 | 10510465 | 16 | N | cg10533978 | chr17 | 40842804 | 32 | MU |
| cg25407372 | chr1 | 10510499 | 16 | N | cg09739017 | chr17 | 65040854 | 32 | N |
| cg26162385 | chr1 | 37271723 | 16 | N | cg01541398 | chr17 | 65040983 | 32 | MU |
| cg13934830 | chr1 | 37271822 | 16 | MU | cg04777612 | chr17 | 73805953 | 32 | MU |
| cg19640303 | chr1 | 37271960 | 16 | N | cg05647756 | chr17 | 73805997 | 32 | MU |
| cg23022656 | chr1 | 42628585 | 16 | MU | cg26525457 | chr17 | 76672843 | 32 | MU |
| cg07602008 | chr1 | 86048479 | 16 | N | cg27169819 | chr17 | 79388690 | 32 | MU |
| cg20293609 | chr1 | 86048540 | 16 | MU | cg20428133 | chr17 | 79388814 | 32 | N |
| cg17499345 | chr1 | 86048585 | 16 | N | cg26066277 | chr17 | 79792777 | 32 | MU |
| cg11659330 | chr1 | 155156300 | 16 | MU | cg23708206 | chr17 | 79792925 | 32 | N |
| cg10577713 | chr1 | 204401413 | 16 | MU | cg25885449 | chr18 | 77638415 | 32 | MU |
| cg24683255 | chr1 | 204951031 | 16 | N | cg16488953 | chr18 | 77638446 | 32 | MU |
| cg20063963 | chr1 | 204951166 | 16 | MU | cg09414987 | chr19 | 392121 | 32 | MU |
| cg14201387 | chr1 | 221737206 | 16 | MU | cg13426839 | chr19 | 539489 | 32 | MU |
| cg17802005 | chr1 | 235035687 | 16 | MU | cg17124430 | chr19 | 793925 | 32 | MU |
| cg04248461 | chr10 | 466737 | 16 | MU | cg23292266 | chr19 | 11285128 | 32 | N |
| cg24068006 | chr10 | 466825 | 16 | MU | cg07676300 | chr19 | 11285258 | 32 | MU |
| cg11295183 | chr10 | 466878 | 16 | MU | cg10389930 | chr19 | 11285331 | 32 | N |
| cg06655291 | chr10 | 30980898 | 16 | MU | cg01596520 | chr19 | 14225029 | 32 | N |
| cg10586837 | chr10 | 30980965 | 16 | N | cg19586199 | chr19 | 14225172 | 32 | MU |
| cg13525013 | chr10 | 98908962 | 16 | N | cg09776718 | chr19 | 30164820 | 32 | MU |
| cg16314733 | chr10 | 98909007 | 16 | MU | cg23620904 | chr19 | 30165018 | 32 | N |
| cg19120251 | chr10 | 105598517 | 16 | MU | cg08427305 | chr19 | 30165133 | 32 | MU |
| cg27337124 | chr10 | 131589141 | 16 | N | cg19421371 | chr19 | 45211155 | 32 | MU |
| cg15139179 | chr10 | 131589157 | 16 | N | cg26347064 | chr19 | 45211248 | 32 | N |
| cg26664254 | chr10 | 131589189 | 16 | MU | cg04770020 | chr2 | 1544321 | 32 | MU |
| cg13253210 | chr10 | 132365700 | 16 | MU | cg06574769 | chr2 | 1544352 | 32 | N |
| cg23036308 | chr10 | 132887033 | 16 | MU | cg18995558 | chr2 | 1544370 | 32 | N |
| cg07095457 | chr10 | 132887109 | 16 | N | cg07608848 | chr2 | 1647185 | 32 | N |
| cg12598767 | chr11 | 72465959 | 16 | N | cg15796818 | chr2 | 1647197 | 32 | MU |
| cg03697509 | chr11 | 72466028 | 16 | MU | cg00138926 | chr2 | 11118414 | 32 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg02119837 | chr11 | 72466113 | 16 | N | cg07702693 | chr2 | 11118564 | 32 | MU |
| cg21917524 | chr11 | 74200334 | 16 | MU | cg16145071 | chr2 | 101727897 | 32 | MU |
| cg13068653 | chr11 | 117074303 | 16 | MU | cg26650973 | chr2 | 128350306 | 32 | N |
| cg11609668 | chr11 | 117075078 | 16 | MU | cg20679271 | chr2 | 128350442 | 32 | MU |
| cg02490942 | chr13 | 113491766 | 16 | N | cg01577298 | chr2 | 128350572 | 32 | N |
| cg15495091 | chr13 | 113491819 | 16 | MU | cg05689415 | chr2 | 234386055 | 32 | MU |
| cg16277139 | chr13 | 113744320 | 16 | MU | cg24414737 | chr2 | 242616833 | 32 | N |
| cg22198179 | chr13 | 114770495 | 16 | N | cg02823437 | chr2 | 242616875 | 32 | N |
| cg00427150 | chr13 | 114770568 | 16 | MU | cg05880944 | chr2 | 242616965 | 32 | MU |
| cg04766043 | chr13 | 114770710 | 16 | N | cg15734942 | chr2 | 242617028 | 32 | N |
| cg07146723 | chr14 | 21559356 | 16 | MU | cg19513282 | chr20 | 62200711 | 32 | N |
| cg21036096 | chr14 | 54417114 | 16 | MU | cg23957118 | chr20 | 62200850 . | 32 | MU |
| cg18951582 | chr14 | 105207600 | 16 | MU | cg21520607 | chr20 | 62200854 | 32 | N |
| cg11157486 | chr14 | 105207716 | 16 | N | cg11815682 | chr21 | 44486381 | 32 | N |
| cg21555471 | chr15 | 41060201 | 16 | MU | cg11796095 | chr21 | 44486452 | 32 | MU |
| cg03049323 | chr15 | 41060265 | 16 | N | cg00775094 | chr21 | 44904824 | 32 | N |
| cg04339692 | chr15 | 48666418 | 16 | MU | cg04550439 | chr21 | 44904941 | 32 | MU |
| cg00800141 | chr16 | 4014161 | 16 | N | cg27462210 | chr21 | 44905012 | 32 | N |
| cg04122657 | chr16 | 4014295 | 16 | MU | cg12955277 | chr21 | 44905064 | 32 | N |
| cg06912227 | chr16 | 4053409 | 16 | MU | cg05670717 | chr21 | 46368052 | 32 | N |
| cg04694492 | chr16 | 4387567 | 16 | MU | cg11988476 | chr21 | 46368090 | 32 | N |
| cg02751838 | chr16 | 75148330 | 16 | N | cg00660655 | chr21 | 46368151 | 32 | MU |
| cg00004883 | chr16 | 75148460 | 16 | MU | cg14026113 | chr22 | 48977177 | 32 | N |
| cg10246581 | chr16 | 81536171 | 16 | MU | cg22504180 | chr22 | 48977277 | 32 | MU |
| cg05540639 | chr16 | 85352976 | 16 | MU | cg04377908 | chr22 | 48977382 | 32 | N |
| cg09798202 | chr16 | 85353755 | 16 | MU | cg07047131 | chr3 | 12856650 | 32 | N |
| cg10074498 | chr16 | 85353838 | 16 | N | cg03181573 | chr3 | 12856729 | 32 | N |
| cg05285352 | chr16 | 87598762 | 16 | MU | cg18797950 | chr3 | 12856746 | 32 | N |
| cg08822215 | chr16 | 89438651 | 16 | N | cg15730967 | chr3 | 12856795 | 32 | MU |
| cg00432497 | chr16 | 89438714 | 16 | N | cg02217386 | chr3 | 12856862 | 32 | N |
| cg01881136 | chr16 | 89438758 | 16 | MU | cg24097241 | chr3 | 71247280 | 32 | MU |
| cg09537792 | chr16 | 89438892 | 16 | N | cg15556723 | chr3 | 71247394. | 32 | MU |
| cg03169557 | chr16 | 89598950 | 16 | MU | cg06856329 | chr3 | 71247442 | 32 | MU |
| cg05390141 | chr17 | 727706 | 16 | N | cg23186787 | chr4 | 4380004 | 32 | MU |
| cg02824818 | chr17 | 727734 | 16 | MU | cg17069541 | chr4 | 4380019 | 32 | N |
| cg16333078 | chr17 | 727791 | 16 | N | cg08577953 | chr4 | 100574455 | 32 | MU |
| cg03846283 | chr17 | 2226871 | 16 | MU | cg12097672 | chr4 | 100574527 | 32 | N |
| cg23098912 | chr17 | 5993686 | 16 | MU | cg09176893 | chr4 | 100574596 | 32 | N |
| cg06421238 | chr17 | 5993785 | 16 | MU | cg00428692 | chr5 | 194530 | 32 | N |
| cg21637561 | chr17 | 37074902 | 16 | N | cg26466258 | chr5 | 194607 | 32 | MU |
| cg26912592 | chr17 | 37074981 | 16 | MU | cg25522232 | chr5 | 195132 | 32 | MU |
| cg23559053 | chr17 | 37075016 | 16 | N | cg24883374 | chr5 | 195171 | 32 | N |
| cg01806442 | chr17 | 37075068 | 16 | MU | cg00084435 | chr5 | 195276 | 32 | N |
| cg01348970 | chr17 | 42581598 | 16 | MU | cg11024341 | chr5 | 2041368 | 32 | MU |
| cg00439089 | chr17 | 48263150 | 16 | N | cg06460652 | chr5 | 176519331 | 32 | MU |
| cg16514513 | chr17 | 48263159 | 16 | N | cg27320658 | chr5 | 176519389 | 32 | N |
| cg11615029 | chr17 | 48263189 | 16 | N | cg20725716 | chr5 | 176519455 | 32 | N |
| cg11993636 | chr17 | 48263198 | 16 | N | cg20768313 | chr5 | 179267916 | 32 | MU |
| cg25735490 | chr17 | 48263204 | 16 | MU | cg26844954 | chr6 | 26867420 | 32 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg17901038 | chr17 | 74385781 | 16 | MU | cg02820566 | chr7 | 187254 | 32 | N |
| cg20035319 | chr17 | 76102438 | 16 | N | cg20865618 | chr7 | 187333 | 32 | MU |
| cg18748888 | chr17 | 76102450 | 16 | MU | cg25980689 | chr7 | 187339 | 32 | MU |
| cg16337457 | chr17 | 76989680 | 16 | MU | cg24883607 | chr7 | 609506 | 32 | MU |
| cg06420480 | chr17 | 78923244 | 16 | N | cg18241094 | chr7 | 609545 | 32 | N |
| cg19443023 | chr17 | 78923344 | 16 | MU | cg09702334 | chr7 | 610337 | 32 | MU |
| cg14247862 | chr17 | 80764631 | 16 | MU | cg02665399 | chr7 | 611060 | 32 | MU |
| cg13280079 | chr17 | 80764739 | 16 | N | cg27495643 | chr7 | 611397 | 32 | MU |
| cg14907788 | chr19 | 2555976 | 16 | N | cg01105362 | chr7 | 1059627 | 32 | MU |
| cg08172637 | chr19 | 2556024 | 16 | MU | cg10074347 | chr7 | 2182153 | 32 | MU |
| cg01895482 | chr19 | 2556145 | 16 | N | cg13504346 | chr7 | 2182182 | 32 | N |
| cg22018329 | chr19 | 3116716 | 16 | MU | cg14397500 | chr7 | 2214759 | 32 | N |
| cg08486065 | chr19 | 3464875 | 16 | MU | cg18207099 | chr7 | 2214790 | 32 | MU |
| cg05021842 | chr19 | 3464940 | 16 | | cg04380800 | chr7 | 100491337 | 32 | MU |
| cg04640975 | chr19 | 3464991 | 16 | | cg08192683 | chr7 | 157551275 | 32 | MU |
| cg25401482 | chr19 | 5148023 | 16 | | cg17665357 | chr8 | 2024249 | 32 | MU |
| cg02842850 | chr19 | 5148130 | 16 | MU | cg23588952 | chr8 | 2024300 | 32 | N |
| cg27644247 | chr19 | 45540766 | 16 | MU | cg02618355 | chr8 | 2024368 | 32 | N |
| cg08408433 | chr19 | 47127358 | 16 | MU | cg24139520 | chr8 | 28573832 | 32 | MU |
| cg04057485 | chr19 | 47127365 | 16 | | cg14546712 | chr8 | 41478669 | 32 | MU |
| cg03344109 | chr19 | 50046477 | 16 | MU | cg13446043 | chr9 | 4804648 | 32 | MU |
| cg00812580 | chr19 | 50080543 | 16 | MU | cg07850533 | chr9 | 4804779 | 32 | N |
| cg02384543 | chr19 | 58879857 | 16 | MU | cg23087108 | chr1 | 1120617 | 33 | MU |
| cg03781262 | chr19 | 58879871 | 16 | N | cg21285198 | chr1 | 1360970 | 33 | N |
| cg08084812 | chr19 | 58879943 | 16 | N | cg00305774 | chr1 | 1361077 | 33 | MU |
| cg07608848 | chr2 | 1647185 | 16 | MU | cg22529900 | chr1 | 8044553 | 33 | MU |
| cg15796818 | chr2 | 1647197 | 16 | MU | cg12446447 | chr1 | 8044581 | 33 | N |
| cg20499506 | chr2 | 3173204 | 16 | MU | cg18861810 | chr1 | 204985932 | 33 | MU |
| cg11062387 | chr2 | 3173253 | 16 | N | cg19707004 | chr1 | 220271059 | 33 | MU |
| cg07948095 | chr2 | 3173279 | 16 | N | cg15393025 | chr1 | 244318867 | 33 | MU |
| cg20430826 | chr2 | 3173292 | 16 | N | cg25466396 | chr1 | 244319008 | 33 | N |
| cg11135381 | chr2 | 86255654 | 16 | N | cg10809560 | chr10 | 79936959 | 33 | MU |
| cg24062526 | chr2 | 86255804 | 16 | MU | cg15602037 | chr10 | 79937002 | 33 | N |
| cg05643613 | chr3 | 11592649 | 16 | MU | cg26109030 | chr10 | 79937095 | 33 | N |
| cg16270079 | chr3 | 48456686 | 16 | MU | cg03410667 | chr10 | 81145959 | 33 | N |
| cg27112062 | chr3 | 48456733 | 16 | N | cg13500206 | chr10 | 81146051 | 33 | N |
| cg19458233 | chr3 | 100148679 | 16 | MU | cg25485956 | chr10 | 81146099 | 33 | MU |
| cg23085666 | chr3 | 125820924 | 16 | MU | cg19957119 | chr10 | 102766322 | 33 | N |
| cg18548199 | chr3 | 129281892 | 16 | MU | cg02009103 | chr10 | 102766386 | 33 | MU |
| cg05944173 | chr3 | 129281962 | 16 | N | cg18360825 | chr10 | 102766458 | 33 | N |
| cg18945794 | chr4 | 3319669 | 16 | MU | cg09758896 | chr10 | 131694575 | 33 | N |
| cg06018666 | chr4 | 10079364 | 16 | MU | cg20800606 | chr10 | 131694675 | 33 | N |
| cg24464101 | chr4 | 38690487 | 16 | MU | cg26275858 | chr10 | 131694689 | 33 | MU |
| cg21953508 | chr4 | 38690502 | 16 | MU | cg12470014 | chr10 | 133878016 | 33 | MU |
| cg22051776 | chr4 | 38690557 | 16 | MU | cg00782697 | chr10 | 134454481 | 33 | MU |
| cg23974120 | chr5 | 14366002 | 16 | MU | cg22979615 | chr11 | 1510644 | 33 | MU |
| cg05575213 | chr6 | 1621807 | 16 | MU | cg26892308 | chr11 | 1510724 | 33 | N |
| cg17983632 | chr6 | 1621930 | 16 | N | cg23822643 | chr12 | 123341665 | 33 | N |
| cg00741954 | chr6 | 1766046 | 16 | N | cg04107639 | chr12 | 123341681 | 33 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg04695233 | chr6 | 1766129 | 16 | MU | cg24631609 | chr12 | 124850781 | 33 | MU |
| cg17356964 | chr6 | 106582638 | 16 | MU | cg10283674 | chr12 | 124850836 | 33 | N |
| cg25581448 | chr6 | 106582669 | 16 | N | cg24336338 | chr12 | 131452238 | 33 | N |
| cg22060817 | chr6 | 126080723 | 16 | MU | cg03776878 | chr12 | 131452292 | 33 | N |
| cg27083825 | chr6 | 158453594 | 16 | MU | cg23617848 | chr12 | 131452297 | 33 | MU |
| cg08362144 | chr6 | 161558085 | 16 | MU | cg07964097 | chr12 | 131708081 | 33 | N |
| cg00004806 | chr6 | 161558105 | 16 | N | cg23969380 | chr12 | 131708174 | 33 | MU |
| cg21496803 | chr6 | 167029080 | 16 | MU | cg19198993 | chr12 | 131708300 | 33 | N |
| cg25930662 | chr6 | 168121243 | 16 | MU | cg06004166 | chr12 | 133102136 | 33 | N |
| cg17531889 | chr6 | 169568517 | 16 | N | cg12967384 | chr12 | 133102272 | 33 | MU |
| cg12956380 | chr6 | 169568536 | 16 | MU | cg09663836 | chr12 | 133102331 | 33 | N |
| cg26074411 | chr6 | 169568564 | 16 | N | cg03571073 | chr13 | 113464332 | 33 | N |
| cg18185116 | chr7 | 598729 | 16 | MU | cg02067585 | chr13 | 113464357 | 33 | MU |
| cg21643916 | chr7 | 616051 | 16 | MU | cg19484164 | chr13 | 113464412 | 33 | N |
| cg14236519 | chr7 | 1152144 | 16 | N | cg11724557 | chr14 | 105965005 | 33 | MU |
| cg13644549 | chr7 | 1152192 | 16 | MU | cg20384325 | chr15 | 49268750 | 33 | MU |
| cg02288552 | chr7 | 1152401 | 16 | MU | cg09077332 | chr16 | 595794 | 33 | N |
| cg19043969 | chr7 | 1162860 | 16 | N | cg13113599 | chr16 | 595848 | 33 | N |
| cg24530527 | chr7 | 1162932 | 16 | MU | cg03614373 | chr16 | 595904 | 33 | MU |
| cg23843484 | chr7 | 1560248 | 16 | MU | cg02278319 | chr16 | 596009 | 33 | N |
| cg25510838 | chr7 | 1560381 | 16 | N | cg08816443 | chr16 | 926409 | 33 | MU |
| cg08513622 | chr7 | 1889785 | 16 | MU | cg26619740 | chr16 | 971556 | 33 | N |
| cg06624731 | chr7 | 1889793 | 16 | MU | cg08214990 | chr16 | 971579 | 33 | MU |
| cg01611679 | chr7 | 99051465 | 16 | | cg02384338 | chr16 | 19883969 | 33 | MU |
| cg05058103 | chr7 | 99051500 | 16 | MU | cg04600253 | chr16 | 50897243 | 33 | N |
| cg15145985 | chr7 | 99051547 | 16 | N | cg00900231 | chr16 | 50897271 | 33 | MU |
| cg21129976 | chr7 | 100421502 | 16 | MU | cg27088725 | chr16 | 72459796 | 33 | N |
| cg22644163 | chr7 | 100421510 | 16 | N | cg07209034 | chr16 | 72459825 | 33 | MU |
| cg05196487 | chr8 | 28929078 | 16 | N | cg02619656 | chr16 | 72460083 | 33 | MU |
| cg19888654 | chr8 | 28929168 | 16 | MU | cg05347108 | chr16 | 72460114 | 33 | N |
| cg07563765 | chr8 | 28929242 | 16 | N | cg00940248 | chr16 | 81929358 | 33 | N |
| cg13754915 | chr9 | 140174272 | 16 | MU | cg03147430 | chr16 | 81929395 | 33 | MU |
| cg24090393 | chr1 | 1560804 | 17 | MU | cg05684569 | chr16 | 81929465 | 33 | MU |
| cg02492207 | chr1 | 1560894 | 17 | N | cg04850875 | chr16 | 85698376 | 33 | MU |
| cg16652920 | chr1 | 7432614 | 17 | MU | cg26573923 | chr16 | 87825195 | 33 | MU |
| cg05650453 | chr1 | 7432644 | 17 | N | cg05058069 | chr16 | 88091975 | 33 | MU |
| cg20274267 | chr1 | 12164477 | 17 | MU | cg08822215 | chr16 | 89438651 | 33 | N |
| cg10494257 | chr1 | 16342123 | 17 | N | cg00432497 | chr16 | 89438714 | 33 | N |
| cg24140574 | chr1 | 16342155 | 17 | MU | cg01881136 | chr16 | 89438758 | 33 | MU |
| cg01782059 | chr1 | 44715942 | 17 | MU | cg09537792 | chr16 | 89438892 | 33 | N |
| cg16710124 | chr1 | 44716058 | 17 | N | cg20931195 | chr17 | 708009 | 33 | MU |
| cg00352560 | chr1 | 45106266 | 17 | MU | cg08903077 | chr17 | 4013000 | 33 | MU |
| cg24317827 | chr1 | 45106404 | 17 | N | cg20544516 | chr17 | 17717183 | 33 | N |
| cg08249608 | chr1 | 94090449 | 17 | MU | cg24161106 | chr17 | 17717208 | 33 | MU |
| cg18564849 | chr1 | 230958741 | 17 | MU | cg11393407 | chr17 | 17717239 | 33 | N |
| cg17088185 | chr10 | 1209315 | 17 | N | cg09494646 | chr17 | 17717252 | 33 | N |
| cg06965169 | chr10 | 1209379 | 17 | MU | cg13245539 | chr17 | 17717265 | 33 | N |
| cg00632125 | chr10 | 1209447 | 17 | MU | cg07415388 | chr17 | 17717276 | 33 | N |
| cg18698819 | chr10 | 13736003 | 17 | N | cg21564965 | chr17 | 36626180 | 33 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg15658978 | chr10 | 13736020 | 17 | N | cg01443071 | chr17 | 40120250 | 33 | MU |
| cg15777335 | chr10 | 13736109 | 17 | MU | cg14376548 | chr17 | 40120327 | 33 | N |
| cg04426802 | chr10 | 126314680 | 17 | MU | cg27125505 | chr17 | 43679177 | 33 | MU |
| cg26725552 | chr10 | 126314758 | 17 | N | cg04927033 | chr17 | 43679265 | 33 | N |
| cg07984614 | chr10 | 126314798 | 17 | MU | cg14110885 | chr17 | 48911141 | 33 | N |
| cg06252276 | chr11 | 34852392 | 17 | MU | cg13639451 | chr17 | 48911157 | 33 | MU |
| cg13052101 | chr11 | 68517529 | 17 | MU | cg13375248 | chr17 | 48911181 | 33 | N |
| cg10553894 | chr11 | 68550534 | 17 | MU | cg06852605 | chr17 | 76422640 | 33 | MU |
| cg22653345 | chr11 | 69307400 | 17 | MU | cg10251094 | chr17 | 76454984 | 33 | N |
| cg11170552 | chr11 | 69307431 | 17 | N | cg05561386 | chr17 | 76455102 | 33 | MU |
| cg08914150 | chr11 | 70256556 | 17 | N | cg20930294 | chr17 | 76455269 | 33 | MU |
| cg02011981 | chr11 | 70256681 | 17 | MU | cg27343663 | chr17 | 79082811 | 33 | MU |
| cg00449344 | chr12 | 658824 | 17 | MU | cg04242349 | chr17 | 79082840 | 33 | MU |
| cg01179055 | chr12 | 665829 | 17 | N | cg09591867 | chr17 | 79098558 | 33 | MU |
| cg12554051 | chr12 | 665863 | 17 | MU | cg11779900 | chr17 | 80519722 | 33 | MU |
| cg24984147 | chr12 | 665887 | 17 | N | cg16891318 | chr18 | 72201590 | 33 | N |
| cg19859729 | chr12 | 3281556 | 17 | N | cg02840828 | chr18 | 72201655 | 33 | N |
| cg02052242 | chr12 | 3281658 | 17 | MU | cg05851240 | chr18 | 72201663 | 33 | N |
| cg10575652 | chr12 | 123351652 | 17 | N | cg03148084 | chr18 | 72201665 | 33 | MU |
| cg03261428 | chr12 | 123351782 | 17 | N | cg12811033 | chr18 | 72201716 | 33 | N |
| cg09178970 | chr12 | 123351798 | 17 | MU | cg03775901 | chr18 | 72201764 | 33 | N |
| cg04701618 | chr12 | 131443231 | 17 | MU | cg15441535 | chr18 | 72201775 | 33 | N |
| cg19026802 | chr12 | 131443260 | 17 | N | cg00226849 | chr18 | 72201785 | 33 | N |
| cg06894723 | chr13 | 110398142 | 17 | N | cg27172769 | chr19 | 1255283 | 33 | MU |
| cg12746120 | chr13 | 110398188 | 17 | MU | cg08176140 | chr19 | 1255392 | 33 | N |
| cg24687655 | chr13 | 110398293 | 17 | N | cg24330746 | chr19 | 7913785 | 33 | MU |
| cg22010909 | chr13 | 113383754 | 17 | MU | cg14232365 | chr19 | 7913869 | 33 | N |
| cg04570540 | chr13 | 113383816 | 17 | MU | cg08337184 | chr19 | 11558539 | 33 | MU |
| cg06377362 | chr13 | 113500867 | 17 | MU | cg15804426 | chr19 | 38993534 | 33 | MU |
| cg21834032 | chr13 | 114108433 | 17 | N | cg20469261 | chr19 | 50361793 | 33 | MU |
| cg00242786 | chr13 | 114108471 | 17 | MU | cg26570040 | chr19 | 50361827 | 33 | N |
| cg00873678 | chr14 | 91770052 | 17 | N | cg23985239 | chr19 | 50361910 | 33 | N |
| cg23138608 | chr14 | 91770145 | 17 | MU | cg10393338 | chr19 | 55798418 | 33 | N |
| cg19465605 | chr14 | 91770229 | 17 | N | cg22577385 | chr19 | 55798477 | 33 | MU |
| cg19745541 | chr14 | 102245559 | 17 | MU | cg13309510 | chr2 | 3479637 | 33 | MU |
| cg16992599 | chr15 | 28752121 | 17 | MU | cg19057227 | chr2 | 3479762 | 33 | N |
| cg17355791 | chr15 | 85798843 | 17 | N | cg06886081 | chr2 | 3479778 | 33 | N |
| cg12522049 | chr15 | 85798898 | 17 | MU | cg09868768 | chr2 | 70188605 | 33 | N |
| cg03310087 | chr15 | 99427016 | 17 | MU | cg05298696 | chr2 | 70188691 | 33 | MU |
| cg06912227 | chr16 | 4053409 | 17 | MU | cg15365028 | chr2 | 236442817 | 33 | MU |
| cg07442105 | chr16 | 66962501 | 17 | MU | cg23551605 | chr2 | 236442942 | 33 | N |
| cg08988156 | chr16 | 68732213 | 17 | N | cg27309098 | chr2 | 236619748 | 33 | MU |
| cg06849452 | chr16 | 68732297 | 17 | MU | cg14930335 | chr2 | 236619767 | 33 | MU |
| cg08640475 | chr16 | 85551478 | 17 | MU | cg21672829 | chr2 | 236619825 | 33 | N |
| cg10099827 | chr16 | 85551514 | 17 | N | cg19759366 | chr2 | 236715747 | 33 | MU |
| cg26586384 | chr17 | 12526632 | 17 | MU | cg14165096 | chr2 | 236715897 | 33 | N |
| cg07673807 | chr17 | 12526662 | 17 | N | cg07642035 | chr2 | 236760940 | 33 | N |
| cg01443071 | chr17 | 40120250 | 17 | MU | cg22373519 | chr2 | 236761021 | 33 | MU |
| cg14376548 | chr17 | 40120327 | 17 | N | cg20351405 | chr2 | 236761068 | 33 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg24771017 | chr17 | 48647138 | 17 | MU | cg01125214 | chr2 | 236888769 | 33 | MU |
| cg00915372 | chr17 | 75209460 | 17 | N | cg07780694 | chr2 | 238990408 | 33 | MU |
| cg13817725 | chr17 | 75209578 | 17 | MU | cg18959169 | chr2 | 240239988 | 33 | N |
| cg07166159 | chr17 | 75209644 | 17 | N | cg09549949 | chr2 | 240240119 | 33 | MU |
| cg02864619 | chr17 | 78933961 | 17 | MU | cg00397274 | chr2 | 240240189 | 33 | N |
| cg27455613 | chr17 | 80820699 | 17 | N | cg27459437 | chr20 | 37464594 | 33 | MU |
| cg01045697 | chr17 | 80820757 | 17 | N | cg19513282 | chr20 | 62200711 | 33 | N |
| cg08605840 | chr17 | 80820763 | 17 | MU | cg23957118 | chr20 | 62200850 | 33 | MU |
| cg13937164 | chr18 | 19780587 | 17 | N | cg21520607 | chr20 | 62200854 | 33 | N |
| cg20880080 | chr18 | 19780644 | 17 | N | cg04461566 | chr22 | 22288352 | 33 | N |
| cg12026967 | chr18 | 19780728 | 17 | MU | cg12894883 | chr22 | 22288473 | 33 | MU |
| cg00148892 | chr18 | 19780878 | 17 | N | cg26285749 | chr22 | 22288508 | 33 | N |
| cg14393952 | chr18 | 56439236 | 17 | MU | cg00489795 | chr22 | 24041104 | 33 | MU |
| cg11890453 | chr18 | 67959378 | 17 | MU | cg17201947 | chr3 | 42700007 | 33 | N |
| cg10406526 | chr19 | 1051079 | 17 | MU | cg27351619 | chr3 | 42700108 | 33 | MU |
| cg00874873 | chr19 | 1051160 | 17 | N | cg24169875 | chr3 | 42700203 | 33 | N |
| cg00869989 | chr19 | 2774197 | 17 | MU | cg06163701 | chr3 | 51994615 | 33 | MU |
| cg07586345 | chr19 | 2774332 | 17 | N | cg21144338 | chr3 | 51994712 | 33 | N |
| cg00473257 | chr19 | 3964734 | 17 | N | cg00071984 | chr3 | 60617504 | 33 | MU |
| cg06647026 | chr19 | 3964837 | 17 | MU | cg21856334 | chr3 | 196754664 | 33 | MU |
| cg03110167 | chr19 | 3964912 | 17 | N | cg16185996 | chr4 | 1008614 | 33 | N |
| cg16617758 | chr19 | 5114202 | 17 | MU | cg07727358 | chr4 | 1008738 | 33 | MU |
| cg11575644 | chr19 | 10464711 | 17 | N | cg12379954 | chr4 | 144272609 | 33 | MU |
| cg00590639 | chr19 | 10464771 | 17 | MU | cg12982893 | chr4 | 144272716 | 33 | N |
| cg22001208 | chr19 | 18782374 | 17 | MU | cg11599887 | chr5 | 1552064 | 33 | MU |
| cg02384543 | chr19 | 58879857 | 17 | MU | cg27039593 | chr5 | 1552182 | 33 | N |
| cg03781262 | chr19 | 58879871 | 17 | N , | cg25654517 | chr6 | 29627131 | 33 | N |
| cg08084812 | chr19 | 58879943 | 17 | N | cg02589899 | chr6 | 29627167 | 33 | MU |
| cg00138926 | chr2 | 11118414 | 17 | N | cg14656245 | chr6 | 29627290 | 33 | N |
| cg07702693 | chr2 | 11118564 | 17 | MU | cg25017994 | chr6 | 29627296 | 33 | N |
| cg00746446 | chr2 | 23879137 | 17 | MU | cg20637405 | chr6 | 42046377 | 33 | MU |
| cg11135381 | chr2 | 86255654 | 17 | N | cg18116267 | chr6 | 158418527 | 33 | MU |
| cg24062526 | chr2 | 86255804 | 17 | MU | cg05987787 | chr6 | 158448508 | 33 | MU |
| cg14330593 | chr2 | 99194088 | 17 | MU | cg02272278 | chr6 | 158448578 | 33 | N |
| cg02078598 | chr2 | 99194199 | 17 | N | cg12465250 | chr6 | 163521043 | 33 | MU |
| cg10096519 | chr2 | 236777077 | 17 | MU | cg05501666 | chr6 | 163558674 | 33 | N |
| cg27276994 | chr2 | 236777122 | 17 | N | cg11724750 | chr6 | 163558823 | 33 | MU |
| cg19504661 | chr2 | 239542427 | 17 | MU | cg09883659 | chr6 | 164520746 | 33 | N |
| cg09549949 | chr2 | 240240119 | 17 | MU | cg25862072 | chr6 | 164520845 | 33 | MU |
| cg00397274 | chr2 | 240240189 | 17 | N | cg22549545 | chr6 | 166836358 | 33 | MU |
| cg11960435 | chr20 | 31434982 | 17 | MU | cg08770734 | chr6 | 166836393 | 33 | N |
| cg24531396 | chr22 | 36013560 | 17 | N | cg24873410 | chr6 | 166836643 | 33 | N |
| cg01945840 | chr22 | 36013675 | 17 | MU | cg26725047 | chr6 | 166836730 | 33 | N |
| cg15161225 | chr22 | 36013678 | 17 | N | cg17292669 | chr6 | 166836791 | 33 | MU |
| cg01815090 | chr22 | 36013708 | 17 | N | cg24692358 | chr6 | 168550512 | 33 | N |
| cg15212895 | chr22 | 49064512 | 17 | MU | cg16545749 | chr6 | 168550568 | 33 | MU |
| cg19458233 | chr3 | 100148679 | 17 | MU | cg09328444 | chr6 | 169901471 | 33 | N |
| cg13592780 | chr3 | 123010034 | 17 | MU | cg25823893 | chr6 | 169901484 | 33 | MU |
| cg14696064 | chr3 | 123010055 | 17 | N | cg18714887 | chr6 | 169901503 | 33 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg10843210 | chr3 | 195613589 | 17 | N | cg27462820 | chr7 | 636583 | 33 | MU |
| cg15010544 | chr3 | 195613641 | 17 | N | cg20392203 | chr7 | 636590 | 33 | MU |
| cg09628877 | chr3 | 195613739 | 17 | MU | cg02635482 | chr7 | 672108 | 33 | MU |
| cg14030878 | chr3 | 195615302 | 17 | N | cg07266412 | chr7 | 101723521 | 33 | N |
| cg04214520 | chr3 | 195615359 | 17 | N | cg17713751 | chr7 | 101723568 | 33 | MU |
| cg18473978 | chr3 | 195615386 | 17 | MU | cg06010390 | chr7 | 101849120 | 33 | MU |
| cg02296904 | chr4 | 877321 | 17 | MU | cg19310653 | chr7 | 101849184 | 33 | N |
| cg11174998 | chr4 | 47885045 | 17 | MU | cg25484712 | chr7 | 150828561 | 33 | MU |
| cg16316054 | chr4 | 185480838 | 17 | N | cg16419361 | chr7 | 150828577 | 33 | N |
| cg19745553 | chr4 | 185480871 | 17 | MU | cg15264162 | chr7 | 154035993 | 33 | MU |
| cg17678847 | chr4 | 186933824 | 17 | N | cg19130610 | chr7 | 156190025 | 33 | MU |
| cg20468325 | chr4 | 186933893 | 17 | MU | cg17515932 | chr7 | 156190074 | 33 | N |
| cg10896676 | chr4 | 187517794 | 17 | MU | cg18686937 | chr7 | 156190095 | 33 | N |
| cg03064793 | chr4 | 187517847 | 17 | N | cg20588703 | chr7 | 156371613 | 33 | MU |
| cg03211527 | chr5 | 1121021 | 17 | MU | cg02192555 | chr8 | 9952926 | 33 | MU |
| cg08436224 | chr5 | 1122753 | 17 | N | cg26880445 | chr8 | 9952965 | 33 | N |
| cg00395291 | chr5 | 1122815 | 17 | MU | cg11824826 | chr8 | 9953022 | 33 | N |
| cg12301921 | chr5 | 1122879 | 17 | N | cg24512544 | chr8 | 141588943 | 33 | MU |
| cg27656498 | chr5 | 14492820 | 17 | N | cg17792192 | chr9 | 138774526 | 33 | MU |
| cg19800026 | chr5 | 14492945 | 17 | MU | cg14105458 | chr9 | 139917436 | 33 | MU |
| cg02379684 | chr5 | 154181831 | 17 | MU | cg10827460 | chr1 | 879375 | 34 | N |
| cg23939866 | chr5 | 154181852 | 17 | N | cg15742605 | chr1 | 879383 | 34 | MU |
| cg16577707 | chr5 | 154181866 | 17 | N | cg09756115 | chr1 | 879415 | 34 | N |
| cg01114405 | chr6 | 504249 | 17 | MU | cg17469240 | chr1 | 1190096 | 34 | MU |
| cg16199907 | chr6 | 504351 | 17 | MU | cg19081571 | chr1 | 1190137 | 34 | N |
| cg18185178 | chr6 | 1516613 | 17 | MU | cg13689260 | chr1 | 1190238 | 34 | N |
| cg01682157 | chr6 | 1516643 | 17 | N | cg10623802 | chr1 | 1913544 | 34 | MU |
| cg09088836 | chr6 | 2510144 | 17 | N | cg27442613 | chr1 | 2801085 | 34 | N |
| cg02666566 | chr6 | 2510173 | 17 | MU | cg03543952 | chr1 | 2801150 | 34 | MU |
| cg17947992 | chr6 | 2510180 | 17 | MU | cg02096887 | chr1 | 10699831 | 34 | MU |
| cg27215236 | chr7 | 878040 | 17 | MU | cg02720091 | chr1 | 10699958 | 34 | N |
| cg23656906 | chr7 | 878067 | 17 | N | cg17836913 | chr1 | 54723679 | 34 | N |
| cg07815491 | chr7 | 878073 | 17 | N | cg24672056 | chr1 | 54723730 | 34 | MU |
| cg06293719 | chr7 | 889564 | 17 | MU | cg08231577 | chr1 | 117077088 | 34 | MU |
| cg11852646 | chr7 | 1956546 | 17 | MU | cg02974976 | chr1 | 117077219 | 34 | N |
| cg08722586 | chr7 | 2046821 | 17 | N | cg10245330 | chr1 | 243652681 | 34 | MU |
| cg03043709 | chr7 | 2046920 | 17 | MU | cg09879526 | chr1 | 243652762 | 34 | N |
| cg08305371 | chr7 | 2046963 | 17 | MU | cg11803859 | chr10 | 125770124 | 34 | N |
| cg17760049 | chr7 | 4774448 | 17 | MU | cg05342655 | chr10 | 125770239 | 34 | MU |
| cg22153931 | chr7 | 43917675 | 17 | MU | cg08398691 | chr10 | 131217895 | 34 | N |
| cg23043230 | chr7 | 97935666 | 17 | N | cg07175255 | chr10 | 131218009 | 34 | MU |
| cg15211734 | chr7 | 97935756 | 17 | N | cg20241059 | chr10 | 133768242 | 34 | MU |
| cg10592494 | chr7 | 97935764 | 17 | MU | cg05796331 | chr10 | 133768294 | 34 | N |
| cg06466782 | chr7 | 101049300 | 17 | MU | cg05480169 | chr10 | 135067408 | 34 | MU |
| cg01380946 | chr7 | 101049372 | 17 | N | cg12587618 | chr11 | 1979879 | 34 | MU |
| cg06823965 | chr7 | 101632645 | 17 | MU | cg17676132 | chr11 | 63886204 | 34 | MU |
| cg12937183 | chr7 | 151421916 | 17 | MU | cg18093064 | chr11 | 63886229 | 34 | N |
| cg04058237 | chr7 | 156337107 | 17 | N | cg01256440 | chr11 | 63886459 | 34 | MU |
| cg21839504 | chr7 | 156337168 | 17 | MU | cg06910673 | chr11 | 63971624 | 34 | N |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg18195333 | chr7 | 156337209 | 17 | N | cg19352801 | chr11 | 63971766 | 34 | MU |
| cg18753341 | chr7 | 157202405 | 17 | MU | cg04815835 | chr11 | 64522197 | 34 | MU |
| cg19839956 | chr8 | 1444021 | 17 | MU | cg12071131 | chr11 | 64522266 | 34 | N |
| cg13367644 | chr8 | 1444085 | 17 | N | cg27402782 | chr11 | 65296794 | 34 | MU |
| cg21620107 | chr8 | 1614053 | 17 | MU | cg09212369 | chrl 1 | 77283117 | 34 | MU |
| cg20812772 | chr8 | 1614107 | 17 | N | cg11609668 | chr11 | 117075078 | 34 | MU |
| cg03613433 | chr8 | 1614182 | 17 | N | cg07263694 | chr12 | 111748299 | 34 | N |
| cg17665357 | chr8 | 2024249 | 17 | MU | cg17125804 | chr12 | 111748346 | 34 | MU |
| cg23588952 | chr8 | 2024300 | 17 | N | cg11550862 | chr12 | 124856981 | 34 | MU |
| cg06769094 | chr8 | 41151382 | 17 | MU | cg16277139 | chr13 | 113744320 | 34 | MU |
| cg26282299 | chr8 | 142189729 | 17 | N . | cg21561701 | chr13 | 114193150 | 34 | MU |
| cg23637880 | chr8 | 142189756 | 17 | MU | cg03479204 | chr13 | 114193155 | 34 | MU |
| cg13133827 | chr8 | 142189806 | 17 | N | cg01515732 | chr14 | 76012927 | 34 | MU |
| cg02095498 | chr8 | 142368264 | 17 | N | cg13738571 | chr14 | 76012941 | 34 | N |
| cg10787911 | chr8 | 142368316 | 17 | N | cg14531093 | chr14 | 102973717 | 34 | MU |
| cg20325921 | chr8 | 142368332 | 17 | MU | cg27542341 | chr15 | 75248086 | 34 | MU |
| cg23016632 | chr1 | 1396169 | 18 | N | cg06349762 | chr16 | 888938 | 34 | MU |
| cg10972351 | chr1 | 1396279 | 18 | N | cg01125617 | chr16 | 889011 | 34 | N |
| cg20951194 | chr1 | 1396296 | 18 | MU | cg07205796 | chr16 | 1187722 | 34 | MU |
| cg22031783 | chr1 | 3128606 | 18 | MU | cg02261541 | chr16 | 4050315 | 34 | MU |
| cg01602345 | chr1 | 3277525 | 18 | N | cg06171406 | chr16 | 4050400 | 34 | N |
| cg21167817 | chr1 | 3277557 | 18 | MU | cg06480482 | chr16 | 29265720 | 34 | MU |
| cg01071314 | chr1 | 3277623 | 18 | MU | cg26796743 | chr16 | 85547467 | 34 | MU |
| cg16245035 | chr1 | 10684392 | 18 | MU | cg26953293 | chr16 | 85684108 | 34 | MU |
| cg20287790 | chr1 | 53359982 | 18 | MU | cg03187934 | chr16 | 90166344 | 34 | MU |
| cg16553083 | chr1 | 224698080 | 18 | MU | cg20899146 | chr17 | 1897348 | 34 | MU |
| cg26251854 | chr1 | 229252074 | 18 | MU | cg10709026 | chr17 | 3716247 | 34 | MU |
| cg18537476 | chr1 | 229252112 | 18 | MU | cg02317397 | chr17 | 3716279 | 34 | N |
| cg18381395 | chr1 | 236924323 | 18 | N | cg25382652 | chr17 | 3716426 | 34 | MU |
| cg20282303 | chr1 | 236924392 | 18 | MU | cg04248364 | chr17 | 3716558 | 34 | N |
| cg17219656 | chr1 | 236924443 | 18 | N | cg21289738 | chr17 | 8383742 | 34 | N. |
| cg24633745 | chr1 | 246930955 | 18 | MU | cg21871213 | chr17 | 8383800 | 34 | MU |
| cg13728376 | chr10 | 5523245 | 18 | MU | cg25495024 | chr17 | 9189607 | 34 | MU |
| cg03398910 | chr10 | 35080979 | 18 | MU | cg27198013 | chr17 | 17714293 | 34 | MU |
| cg01176720 | chr10 | 35081047 | 18 | N | cg09015921 | chr17 | 17714347 | 34 | N · |
| cg14588422 | chr10 | 35081084 | 18 | MU | cg22946219 | chr17 | 17714414 | 34 | N |
| cg15247093 | chr10 | 47768476 | 18 | MU | cg17876831 | chr17 | 18918410 | 34 | MU |
| cg09287096 | chr10 | 86998622 | 18 | MU | cg22671072 | chr17 | 18918466 | 34 | N |
| cg05848463 | chr10 | 88982562 | 18 | N | cg24209115 | chr17 | 18918472 | 34 | N |
| cg07505719 | chr10 | 88982663 | 18 | MU | cg23665668 | chr17 | 38245507 | 34 | MU |
| cg25467549 | chr10 | 88982701 | 18 | N | cg23526824 | chr17 | 38245542 | 34 | N |
| cg04426802 | chr10 | 126314680 | 18 | MU | cg10858898 | chr17 | 38245607 | 34 | N |
| cg26725552 | chr10 | 126314758 | 18 | N | cg20404150 | chr17 | 40705273 | 34 | MU |
| cg07984614 | chr10 | 126314798 | 18 | N | cg00439089 | chr17 | 48263150 | 34 | N |
| cg27607136 | chr11 | 1857442 | 18 | MU | cg16514513 | chr17 | 48263159 | 34 | N |
| cg22348345 | chr11 | 1857477 | 18 | N | cg11615029 | chr17 | 48263189 | 34 | MU |
| cg14642259 | chr11 | 47359017 | 18 | MU | cg11993636 | chr17 | 48263198 | 34 | N |
| cg05649391 | chr11 | 47359115 | 18 | N | cg25735490 | chr17 | 48263204 | 34 | MU |
| cg01176141 | chr11 | 70023032 | 18 | MU | cg14193007 | chr17 | 77953016 | 34 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg13089599 | chr11 | 70023060 | 18 | MU | cg04194055 | chr17 | 77953088 | 34 | MU |
| cg24832721 | chr11 | 70023142 | 18 | N | cg17602102 | chr17 | 78796159 | 34 | MU |
| cg03614381 | chr11 | 71229642 | 18 | MU | cg02910299 | chr17 | 78796166 | 34 | N |
| cg14632485 | chr11 | 113216820 | 18 | MU | cg02775417 | chr19 | 643457 | 34 | MU |
| cg13659360 | chr11 | 121023627 | 18 | MU | cg12062672 | chr19 | 643555 | 34 | N |
| cg24327383 | chr11 | 121023708 | 18 | N | cg19222405 | chr19 | 1826867 | 34 | MU |
| cg13751188 | chr11 | 121023720 | 18 | N | cg03924776 | chr19 | 1826924 | 34 | N |
| cg06193412 | chr11 | 134608672 | 18 | MU | cg04584009 | chr19 | 1826957 | 34 | N |
| cg09414985 | chr11 | 134608774 | 18 | N | cg23429986 | chr19 | 3382104 | 34 | N |
| cg10457436 | chr12 | 6745871 | 18 | MU | cg22731204 | chr19 | 3382127 | 34 | MU |
| cg04701618 | chr12 | 131443231 | 18 | MU | cg21740826 | chr19 | 4037881 | 34 | MU |
| cg19026802 | chr12 | 131443260 | 18 | N | cg26501450 | chr19 | 4038221 | 34 | MU |
| cg13251533 | chr12 | 132286017 | 18 | MU | cg22268467 | chr19 | 4361713 | 34 | MU |
| cg10130374 | chr13 | 111935412 | 18 | N | cg24419436 | chr19 | 4446031 | 34 | MU |
| cg22795345 | chr13 | ⁻111935508 | 18 | MU | cg09377855 | chr19 | 4446336 | 34 | MU |
| cg14270002 | chr13 | 111935521. | 18 | MU | cg05421550 | chr19 | 4446485 | 34 | N |
| cg22612590 | chr13 | 114187973 | 18 | N | cg02305651 | chr19 | 5997013 | 34 | N |
| cg13241681 | chr13 | 114187990 | 18 | MU | cg07572348 | chr19 | 5997117 | 34 | MU |
| cg20539175 | chr13 | 114188051 | 18 | N | cg17702388 | chr19 | 16466480 | 34 | N |
| cg03535253 | chr14 | 93706571 | 18 | MU | cg04738220 | chr19 | 16466617 | 34 | MU |
| cg00705661 | chr14 | 93706609 | 18 | MU | cg11013793 | chrl9 | 16466636 | 34 | N |
| cg22920967 | chr14 | 104047722 | 18 | | cg00029640 | chr19 | 35770141 | 34 | N |
| cg00834537 | chr14 | 104047751 | 18 | MU | cg08113595 | chr19 | 35770164 | 34 | MU |
| cg18471471 | chr15 | 100537686 | 18 | MU | cg07918620 | chr19 | 35800925 | 34 | MU |
| cg07015368 | chr15 | 100537761 | 18 | N | cg15761414 | chr19 | 35801014 | 34 | N |
| cg16628135 | chr16 | 1397791 | 18 | MU | cg22923523 | chr19 | 47549464 | 34 | MU |
| cg06698598 | chr16 | 1398226 | 18 | MU | cg22182287 | chr2 | 3452347 | 34 | N |
| cg06739469 | chr16 | 29343859 | 18 | N | cg00548824 | chr2 | 3452407 | 34 | MU |
| cg00711542 | chr16 | 29343894 | 18 | MU | cg17738010 | chr2 | 3452437 | 34 | N |
| cg00535785 | chr16 | 49766785 | 18 | N | cg06360976 | chr2 | 3452543 | 34 | N |
| cg08842616 | chr16 | 70733772 | 18 | N | cg08182975 | chr2 | 7164527 | 34 | MU |
| cg08796240 | chr16 | 70733832 | 18 | MU | cg20995753 | chr2 | 7164586 | 34 | N |
| cg02501155 | chr16 | 79263473 | 18 | MU | cg23079012 | chr2 | 8343710 | 34 | MU |
| cg10221896 | chr16 | 79263595 | 18 | N | cg23393100 | chr2 | 25457204 | 34 | N |
| cg03430116 | chr16 | 85083564 | 18 | MU | cg13828701 | chr2 | 25457215 | 34 | MU |
| cg04922015 | chr16 | 86500429 | 18 | MU | cg26160153 | chr2 | 27373014 | 34 | N |
| cg00284420 | chr16 | 87311948 | 18 | MU | cg24874524 | chr2 | 27373088 | 34 | MU |
| cg01579218 | chr16 | 87312047 | 18 | N | cg14949065 | chr2 | 27373128 | 34 | MU |
| cg01753252 | chr16 | 88757588 | 18 | MU | cg02930665 | chr2 | 27373213 | 34 | N |
| cg06359492 | chr16 | 89162377 | 18 | MU | cg25114693 | chr2 | 105695949 | 34 | MU |
| cg10158369 | chr16 | 89162475 | 18 | N | cg16326123 | chr2 | 240015886 | 34 | MU |
| cg09482780 | chr17 | 7756609 | 18 | MU | cg09669931 | chr2 | 240058920 | 34 | MU |
| cg01218720 | chr17 | 40322138 | 18 | N | cg18359321 | chr2 | 240058950 | 34 | MU |
| cg26583088 | chr17 | 40322250 | 18 | MU | cg19426572 | chr20 | 61919450 | 34 | MU |
| cg00439089 | chr17 | 48263150 | 18 | N | cg14058699 | chr21 | 47421177 | 34 | N |
| cg16514513 | chr17 | 48263159 | 18 | N | cg24310904 | chr21 | 47421270 | 34 | N |
| cg11615029 | chr17 | 48263189 | 18 | N | cg21308336 | chr21 | 47421319 | 34 | MU |
| cg11993636 | chr17 | 48263198 | 18 | N | cg13082654 | chr21 | 47811350 | 34 | MU |
| cg25735490 | chr17 | 48263204 | 18 | MU | cg26017564 | chr3 | 172034780 | 34 | MU |

(continued)

| Illumina Probe Name | Chr. | Position | Tissue /Cell Type | MU/ N | Illumina Probe Name | Chr. | Position | Tissue /Cell Type | MU/ N |
|---|---|---|---|---|---|---|---|---|---|
| cg26758486 | chr17 | 71533286 | 18 | MU | cg03999583 | chr3 | 197385493 | 34 | MU |
| cg08459186 | chr17 | 71533337 | 18 | N | cg24715682 | chr3 | 197877792 | 34 | MU |
| cg01449677 | chr17 | 76794888 | 18 | MU | cg05145898 | chr4 | 6883799 | 34 | N |
| cg04117396 | chr17 | 76795028 | 18 | N | cg07976816 | chr4 | 6883843 | 34 | MU |
| cg17602102 | chr17 | 78796159 | 18 | MU | cg14853946 | chr4 | 6883863 | 34 | N |
| cg14073057 | chr17 | 78796363 | 18 | MU | cg01592387 | chr4 | 7774382 | 34 | N |
| cg25949961 | chr17 | 79166676 | 18 | MU | cg20929387 | chr4 | 7774510 | 34 | MU |
| cg10963033 | chr17 | 79166715 | 18 | N | cg11565007 | chr4 | 106978683 | 34 | MU |
| cg20632978 | chr17 | 79167828 | 18 | MU | cg26402435 | chr4 | 190661492 | 34 | N |
| cg01977863 | chr17 | 79167836 | 18 | N | cg05970547 | chr4 | 190661634 | 34 | MU |
| cg04460362 | chr17 | 79913303 | 18 | MU | cg13346820 | chr5 | 865318 | 34 | N |
| cg25105415 | chr17 | 79913327 | 18 | MU | cg11447335 | chr5 | 865350 | 34 | MU |
| cg03654588 | chr17 | 79913374 | 18 | N | cg02493524 | chr5 | 3768103 | 34 | N |
| cg23515921 | chr17 | 80747189 | 18 | N | cg19541998 | chr5 | 3768144 | 34 | N |
| cg18602928 | chr17 | 80747240 | 18 | MU | cg23988801 | chr5 | 3768181 | 34 | MU |
| cg04934595 | chr17 | 81021419 | 18 | MU | cg09429437 | chr5 | 6755265 | 34 | N |
| cg14515064 | chr17 | 81021472 | 18 | N | cg15145174 | chr5 | 6755386 | 34 | MU |
| cg04516518 | chr18 | 77193464 | 18 | N | cg26964629 | chr5 | 6755439 | 34 | N |
| cg07759042 | chr18 | 77193502 | 18 | MU | cg08970011 | chr6 | 3293371 | 34 | MU |
| cg12524531 | chr18 | 77193581 | 18 | N | cg23927832 | chr6 | 30633356 | 34 | MU |
| cg24175580 | chr19 | 36024217 | 18 | N | cg15055187 | chr6 | 30633386 | 34 | N |
| cg27036341 | chr19 | 36024225 | 18 | MU | cg19965099 | chr6 | 31801683 | 34 | MU |
| cg21453378 | chr19 | 36024253 | 18 | N | cg19816983 | chr6 | 31937343 | 34 | N |
| cg06710596 | chr19 | 36024279 | 18 | N | cg10674805 | chr6 | 31937437 | 34 | MU |
| cg15729137 | chr19 | 36024282 | 18 | N | cg05909390 | chr6 | 31937454 | 34 | N |
| cg25681177 | chr19 | 36024313 | 18 | N | cg20480259 | chr6 | 31937462 | 34 | N |
| cg25498045 | chr19 | 36024375 | 18 | N | cg09230350 | chr6 | 31937582 | 34 | N |
| cg02457644 | chr19 | 36245325 | 18 | MU | cg09299390 | chr7 | 1866243 | 34 | MU |
| cg02384543 | chr19 | 58879857 | 18 | MU | cg13037895 | chr7 | 2262292 | 34 | N |
| cg03781262 | chr19 | 58879871 | 18 | N | cg16573836 | chr7 | 2262355 | 34 | MU |
| cg08084812 | chr19 | 58879943 | 18 | N | cg24941703 | chr7 | 2262479 | 34 | N |
| cg19361181 | chr2 | 10616043 | 18 | MU | cg02527886 | chr7 | 2968316 | 34 | MU |
| cg26539736 | chr2 | 10666585 | 18 | MU | cg10939949 | chr7 | 2968406 | 34 | N |
| cg10767193 | chr2 | 10666662 | 18 | N | cg06746318 | chr7 | 101755186 | 34 | MU |
| cg08846959 | chr2 | 10666713 | 18 | N | cg19410378 | chr7 | 152551811 | 34 | MU |
| cg00138926 | chr2 | 11118414 | 18 | N | cg04058237 | chr7 | 156337107 | 34 | N |
| cg07702693 | chr2 | 11118564 | 18 | MU | cg21839504 | chr7 | 156337168 | 34 | MU |
| cg07622575 | chr2 | 11487955 | 18 | MU | cg18195333 | chr7 | 156337209 | 34 | N |
| cg24586870 | chr2 | 233198590 | 18 | MU | cg19237063 | chr7 | 157666168 | 34 | MU |
| cg17365725 | chr2 | 242698748 | 18 | N | cg16142313 | chr8 | 28950249 | 34 | N |
| cg02698622 | chr2 | 242698811 | 18 | MU | cg07237653 | chr8 | 28950303 | 34 | MU |
| cg25478712 | chr21 | 44840120 | 18 | MU | cg25987871 | chr8 | 141231538 | 34 | MU |
| cg20341238 | chr21 | 44865764 | 18 | MU | cg14487379 | chr9 | 135481870 | 34 | MU |
| cg22247792 | chr21 | 47548991 | 18 | MU | cg14008164 | chr9 | 141105202 | 34 | MU |

**Table 3: 500 CpGs iteratively added to distinguish between similar pairs of tissues/cell types**

| | Illumina Probe Name | Chr. | Position | Tissue/Cell Type 1 | % Meth. | Tissue/Cell Type 2 | % Meth. |
|---|---|---|---|---|---|---|---|
| 1 | cg01156249 | 16 | 4714794 | Monocytes | 3.2% | B-cells | 90.9% |

(continued)

| | Illumina Probe Name | Chr. | Position | Tissue/Cell Type 1 | % Meth. | Tissue/Cell Type 2 | % Meth. |
|---|---|---|---|---|---|---|---|
| 2 | cg25574765 | 11 | 70211531 | NK-cells | 0.0% | Breast | 91.5% |
| 3 | cg08960448 | 16 | 4838658 | Neurons | 99.0% | Panc duct cells | 1.7% |
| 4 | cg14088921 | 8 | 98944936 | Panc beta cells | 83.8% | Panc duct cells | 2.2% |
| 5 | cg26872907 | 6 | 161796854 | Monocytes | 9.3% | Neutrophils | 94.8% |
| 6 | cg12213680 | 6 | 24877462 | Eosinophils | 15.9% | Neutrophils | 78.4% |
| 7 | cg13910785 | 6 | 32549849 | Colon | 42.8% | Rectum | 4.6% |
| 8 | cg06768361 | 12 | 117480333 | B-cells | 96.8% | Eryth Prog | 1.4% |
| 9 | cg00660167 | 17 | 80200974 | CD4+ T-cells | 96.6% | Neutrophils | 8.2% |
| 10 | cg22505006 | 1 | 154981829 | CD4+ T-cells | 9.7% | CD8+ T-cells | 82.8% |
| 11 | cg02380585 | 17 | 33776683 | NK-cells | 28.6% | CD8+ T-cells | 80.5% |
| 12 | cg18121066 | 17 | 80794274 | Breast | 31.4% | Uterus | 86.3% |
| 13 | cg26686732 | 20 | 22562737 | Colon | 38.3% | Rectum | 6.1% |
| 14 | cg19384241 | 2 | 55393977 | Cervix | 20.0% | Uterus | 67.1% |
| 15 | cg23905789 | 6 | 32549935 | Adipocytes | 12.7% | Bladder | 84.8% |
| 16 | cg06125903 | 20 | 57583328 | B-cells | 3.8% | NK-cells | 85.0% |
| 17 | cg12911732 | 1 | 50744322 | Breast | 9.2% | Head and neck | 87.2% |
| 18 | cg13763287 | 9 | 97856079 | Colon | 64.1% | Rectum | 95.7% |
| 19 | cg24011073 | 2 | 11917787 | NK-cells | 37.4% | CD8+ T-cells | 86.8% |
| 20 | cg20504791 | 20 | 22562478 | Colon | 43.1% | Rectum | 11.8% |
| 21 | cg16953816 | 12 | 123349952 | Kidney | 26.7% | Uterus | 92.3% |
| 22 | cg17480035 | 6 | 32552670 | Cervix | 83.5% | Uterus | 38.1% |
| 23 | cg17830515 | 7 | 1151900 | Vasc endo cells | 17.2% | Bladder | 81.0% |
| 24 | cg07984133 | 13 | 28544592 | Colon | 22.7% | Rectum | 53.7% |
| 25 | cg21498547 | 8 | 1651128 | Esophagus | 35.7% | Stomach | 85.1% |
| 26 | cg04168675 | 2 | 225809655 | NK-cells | 76.8% | CD8+ T-cells | 27.8% |
| 27 | cg04650948 | 17 | 46798298 | Colon | 62.1% | Rectum | 31.2% |
| 28 | cg08691577 | 5 | 14668489 | CD4+ T-cells | 76.1% | CD8+ T-cells | 15.4% |
| 29 | cg20240243 | 15 | 100154002 | Eosinophils | 19.9% | Neutrophils | 82.0% |
| 30 | cg08061524 | 22 | 46472568 | Bladder | 69.4% | Uterus | 14.9% |
| 31 | cg24051749 | 1 | 39340282 | Colon | 52.3% | Rectum | 21.5% |
| 32 | cg24888257 | 6 | 31785597 | Breast | 7.5% | Uterus | 61.7% |
| 33 | cg24586205 | 1 | 222162028 | Cervix | 4.1% | Uterus | 44.7% |
| 34 | cg22210145 | 13 | 28545263 | Colon | 25.8% | Rectum | 56.5% |
| 35 | cg13563298 | 9 | 95948059 | Bladder | 88.6% | Colon | 18.0% |
| 36 | cg14011789 | 17 | 75452044 | NK-cells | 76.7% | CD8+ T-cells | 27.8% |
| 37 | cg10244976 | 16 | 1014818 | Breast | 87.9% | Prostate | 17.6% |
| 38 | cg14825735 | 6 | 1594066 | Colon | 29.1% | Rectum | 59.6% |
| 39 | cg22933800 | 6 | 32605704 | Cervix | 82.3% | Uterus | 43.9% |
| 40 | cg27091865 | 2 | 3826610 | Kidney | 22.4% | Uterus | 88.0% |

(continued)

| | Illumina Probe Name | Chr. | Position | Tissue/Cell Type 1 | % Meth. | Tissue/Cell Type 2 | % Meth. |
|---|---|---|---|---|---|---|---|
| 41 | cg04855216 | 5 | 118609467 | Panc beta cells | 85.5% | Panc acinar cells | 5.0% |
| 42 | cg06665913 | 11 | 113947036 | Eosinophils | 22.2% | Neutrophils | 83.4% |
| 43 | cg06935361 | 13 | 32964454 | CD4+ T-cells | 81.0% | CD8+ T-cells | 21.1% |
| 44 | cg03635532 | 19 | 40376883 | Colon | 84.0% | Rectum | 53.9% |
| 45 | cg24441810 | 2 | 120436039 | NK-cells | 59.0% | CD8+ T-cells | 10.5% |
| 46 | cg00103771 | 6 | 32525805 | Cervix | 81.4% | Uterus | 46.0% |
| 47 | cg12374721 | 17 | 46799640 | Colon | 38.9% | Rectum | 9.1% |
| 48 | cg02159489 | 17 | 79459563 | Esophagus | 88.0% | Stomach | 39.2% |
| 49 | cg11128983 | 5 | 175297531 | Colon | 70.8% | Rectum | 41.1% |
| 50 | cg18173726 | 15 | 95713100 | Cervix | 31.4% | Uterus | 65.9% |
| 51 | cg17311865 | 14 | 22975521 | NK-cells | 63.4% | CD8+ T-cells | 15.0% |
| 52 | cg05222982 | 13 | 28545214 | Colon | 31.8% | Rectum | 60.8% |
| 53 | cg08467103 | 2 | 65593933 | Cervix | 10.5% | Uterus | 44.6% |
| 54 | cg16871561 | 12 | 98986887 | CD4+ T-cells | 15.7% | CD8+ T-cells | 74.1% |
| 55 | cg18833880 | 10 | 131650741 | Colon | 60.9% | Rectum | 89.4% |
| 56 | cg18236982 | 1 | 236065291 | Cervix | 46.8% | Uterus | 80.0% |
| 57 | cg18236877 | 1 | 10733511 | Colon | 63.9% | Rectum | 35.4% |
| 58 | cg13668207 | 12 | 65904452 | Bladder | 73.1% | Uterus | 20.4% |
| 59 | cg15564619 | 1 | 2163437 | NK-cells | 82.0% | CD8+ T-cells | 34.4% |
| 60 | cg16514085 | 6 | 32552152 | Colon | 22.2% | Rectum | 50.5% |
| 61 | cg05078091 | 11 | 129984345 | Eosinophils | 21.0% | Neutrophils | 81.2% |
| 62 | cg18001714 | 5 | 14871894 | Esophagus | 4.7% | Stomach | 51.0% |
| 63 | cg27659049 | 12 | 115173713 | Colon | 59.6% | Rectum | 31.8% |
| 64 | cg05509190 | 2 | 177503639 | Cervix | 24.0% | Uterus | 56.4% |
| 65 | cg02582848 | 1 | 224075899 | Breast | 77.1% | Uterus | 23.7% |
| 66 | cg01263942 | 10 | 695859 | Esophagus | 19.0% | Head and neck | 79.6% |
| 67 | cg05222995 | 12 | 115124973 | Colon | 50.8% | Rectum | 23.2% |
| 68 | cg13453904 | 10 | 133484610 | Bladder | 7.8% | Esophagus | 66.6% |
| 69 | cg17113883 | 6 | 128239611 | NK-cells | 80.6% | CD8+ T-cells | 33.0% |
| 70 | cg01774894 | 17 | 46674395 | Cervix | 80.2% | Uterus | 48.1% |
| 71 | cg21596498 | 19 | 42618407 | B-cells | 11.2% | NK-cells | 91.1% |
| 72 | cg25045219 | 17 | 263096 | CD4+ T-cells | 10.3% | CD8+ T-cells | 67.0% |
| 73 | cg15424233 | 2 | 9865477 | Colon | 52.2% | Rectum | 79.8% |
| 74 | cg07077965 | 2 | 177503592 | Cervix | 26.2% | Uterus | 58.3% |
| 75 | cg19602081 | 4 | 111553161 | Colon | 39.2% | Rectum | 12.6% |
| 76 | cg26103369 | 14 | 75153307 | Eosinophils | 20.1% | Neutrophils | 80.2% |
| 77 | cg19256731 | 20 | 1874527 | Colon | 67.3% | Rectum | 40.8% |
| 78 | cg19626138 | 8 | 37731969 | NK-cells | 19.1% | CD8+ T-cells | 66.6% |
| 79 | cg25006077 | 3 | 152176018 | Cervix | 41.8% | Uterus | 73.8% |

(continued)

| | Illumina Probe Name | Chr. | Position | Tissue/Cell Type 1 | % Meth. | Tissue/Cell Type 2 | % Meth. |
|---|---|---|---|---|---|---|---|
| 80 | cg25958911 | 8 | 145495665 | Esophagus | 72.6% | Stomach | 27.4% |
| 81 | cg06774703 | 17 | 39647583 | Colon | 73.5% | Rectum | 47.0% |
| 82 | cg01543583 | 14 | 59947673 | Colon | 53.0% | Rectum | 79.3% |
| 83 | cg19543867 | 7 | 112124979 | Cervix | 22.4% | Uterus | 54.4% |
| 84 | cg04329870 | 2 | 87048747 | CD4+ T-cells | 84.1% | CD8+ T-cells | 29.9% |
| 85 | cg03943773 | 4 | 111552308 | Colon | 33.7% | Rectum | 7.6% |
| 86 | cg05995465 | 2 | 240313602 | Cervix | 44.0% | Uterus | 75.9% |
| 87 | cg05263838 | 2 | 227659406 | Colon | 64.5% | Rectum | 38.5% |
| 88 | cg19351954 | 7 | 156259207 | Breast | 75.2% | Head and neck | 16.2% |
| 89 | cg05849676 | 12 | 10171151 | Bladder | 29.3% | Uterus | 80.9% |
| 90 | cg17920646 | 8 | 216578 | Colon | 60.7% | Rectum | 35.0% |
| 91 | cg04778178 | 7 | 27162294 | Bladder | 89.8% | Esophagus | 32.1% |
| 92 | cg18348836 | 1 | 60282400 | Eosinophils | 19.1% | Neutrophils | 78.3% |
| 93 | cg10708271 | 3 | 112692897 | NK-cells | 57.9% | CD8+ T-cells | 10.4% |
| 94 | cg24938632 | 17 | 46830125 | Colon | 28.4% | Rectum | 53.8% |
| 95 | cg02123534 | 7 | 965485 | CD4+ T-cells | 70.2% | CD8+ T-cells | 17.5% |
| 96 | cg08042322 | 12 | 104233595 | Colon | 73.9% | Rectum | 48.6% |
| 97 | cg05797224 | 1 | 94277532 | Cervix | 37.0% | Uterus | 68.7% |
| 98 | cg16966496 | 12 | 115172598 | Colon | 64.1% | Rectum | 38.8% |
| 99 | cg22676075 | 6 | 135203613 | Esophagus | 30.2% | Stomach | 75.2% |
| 100 | cg14565903 | 19 | 14260591 | Kidney | 74.9% | Uterus | 14.2% |
| 101 | cg21229570 | 12 | 115124768 | Colon | 40.2% | Rectum | 15.0% |
| 102 | cg23244761 | 6 | 161796850 | Monocytes | 13.9% | Neutrophils | 96.9% |
| 103 | cg24527636 | 1 | 27849295 | NK-cells | 70.6% | CD8+ T-cells | 23.2% |
| 104 | cg27169860 | 2 | 66218712 | Cervix | 18.7% | Uterus | 50.3% |
| 105 | cg10606698 | 6 | 76203642 | Colon | 68.6% | Rectum | 43.4% |
| 106 | cg04696274 | 20 | 43977531 | Cervix | 36.9% | Uterus | 68.1% |
| 107 | cg21175246 | 14 | 95964097 | Colon | 65.6% | Rectum | 40.4% |
| 108 | cg01782486 | 1 | 154986512 | CD4+ T-cells | 34.6% | CD8+ T-cells | 87.1% |
| 109 | cg18992570 | 5 | 134662888 | Colon | 83.7% | Rectum | 58.6% |
| 110 | cg05528899 | 17 | 57120 | Cervix | 36.8% | Uterus | 67.7% |
| 111 | cg07930673 | 2 | 2650283 | NK-cells | 64.0% | CD8+ T-cells | 17.2% |
| 112 | cg22022580 | 19 | 37266605 | Colon | 75.3% | Rectum | 50.2% |
| 113 | cg00584840 | 3 | 112903054 | Esophagus | 21.0% | Head and neck | 80.2% |
| 114 | cg18349241 | 5 | 89772071 | Eosinophils | 21.5% | Neutrophils | 80.0% |
| 115 | cg02220965 | 16 | 31128310 | Colon | 29.1% | Rectum | 54.2% |
| 116 | cg22645201 | 7 | 120701829 | Cervix | 42.6% | Uterus | 73.4% |
| 117 | cg25225073 | 14 | 90528983 | Esophagus | 10.4% | Stomach | 55.4% |
| 118 | cg24925400 | 4 | 111552353 | Colon | 40.2% | Rectum | 15.1% |

(continued)

|  | Illumina Probe Name | Chr. | Position | Tissue/Cell Type 1 | % Meth. | Tissue/Cell Type 2 | % Meth. |
|---|---|---|---|---|---|---|---|
| 119 | cg06579465 | 17 | 30152101 | Cervix | 36.4% | Uterus | 67.2% |
| 120 | cg26681770 | 20 | 56247302 | NK-cells | 24.5% | CD8+ T-cells | 70.7% |
| 121 | cg04528477 | 2 | 176932458 | Colon | 22.3% | Rectum | 47.3% |
| 122 | cg03634833 | 7 | 965534 | CD4+ T-cells | 67.6% | CD8+ T-cells | 15.5% |
| 123 | cg11323985 | 8 | 18541497 | Breast | 70.2% | Uterus | 17.6% |
| 124 | cg14202783 | 9 | 130662561 | Colon | 45.9% | Rectum | 70.8% |
| 125 | cg01405040 | 2 | 176964137 | Cervix | 57.4% | Uterus | 26.7% |
| 126 | cg22198853 | 6 | 1594411 | Colon | 41.5% | Rectum | 66.4% |
| 127 | cg08879910 | 6 | 29974319 | Esophagus | 5.4% | Stomach | 49.8% |
| 128 | cg04011338 | 2 | 238777587 | Cervix | 35.0% | Uterus | 65.6% |
| 129 | cg23336481 | 6 | 32634344 | Colon | 21.9% | Rectum | 46.7% |
| 130 | cg08223760 | 1 | 43291209 | Eosinophils | 21.5% | Neutrophils | 79.9% |
| 131 | cg00263248 | 14 | 98444151 | NK-cells | 64.8% | CD8+ T-cells | 18.9% |
| 132 | cg03686593 | 18 | 11149470 | Colon | 40.4% | Rectum | 15.7% |
| 133 | cg17185710 | 3 | 151984822 | Cervix | 28.0% | Uterus | 58.6% |
| 134 | cg05613718 | 7 | 38355100 | Monocytes | 82.8% | Neutrophils | 7.2% |
| 135 | cg04497820 | 5 | 158086454 | Colon | 56.0% | Rectum | 31.4% |
| 136 | cg20676788 | 13 | 49379791 | Cervix | 33.9% | Uterus | 64.4% |
| 137 | cg14989243 | 6 | 76203530 | Colon | 62.3% | Rectum | 37.7% |
| 138 | cg10581012 | 11 | 110384827 | Kidney | 83.9% | Uterus | 24.7% |
| 139 | cg23685155 | 12 | 54396440 | Colon | 44.9% | Rectum | 20.3% |
| 140 | cg23264547 | 11 | 44257668 | Cervix | 33.4% | Uterus | 63.7% |
| 141 | cg13120534 | 14 | 50063923 | Colon | 52.0% | Rectum | 76.5% |
| 142 | cg18637238 | 12 | 10543073 | CD4+ T-cells | 86.5% | CD8+ T-cells | 36.2% |
| 143 | cg09885622 | 5 | 1504991 | NK-cells | 16.0% | CD8+ T-cells | 61.7% |
| 144 | cg03455736 | 22 | 49882497 | Cervix | 34.7% | Uterus | 65.0% |
| 145 | cg20518446 | 11 | 62315034 | Colon | 66.7% | Rectum | 42.2% |
| 146 | cg10888111 | 2 | 1637068 | Colon | 64.3% | Rectum | 88.8% |
| 147 | cg18673341 | 7 | 22481962 | Cervix | 64.7% | Uterus | 34.5% |
| 148 | cg26107890 | 3 | 124860686 | Bladder | 5.1% | Esophagus | 57.3% |
| 149 | cg18450582 | 7 | 95546539 | Esophagus | 30.7% | Head and neck | 86.7% |
| 150 | cg23478225 | 2 | 46113875 | Colon | 73.5% | Rectum | 49.0% |
| 151 | cg08077807 | 14 | 62001072 | Eosinophils | 20.3% | Neutrophils | 78.5% |
| 152 | cg02196256 | 7 | 148321082 | NK-cells | 29.8% | CD8+ T-cells | 75.2% |
| 153 | cg16053651 | 6 | 168857803 | Colon | 55.1% | Rectum | 79.6% |
| 154 | cg23238315 | 7 | 156259194 | Breast | 84.8% | Head and neck | 27.2% |
| 155 | cg11777506 | 2 | 114880656 | Colon | 50.4% | Rectum | 74.9% |
| 156 | cg26986871 | 12 | 10545083 | CD4+ T-cells | 57.2% | CD8+ T-cells | 7.6% |
| 157 | cg23936031 | 7 | 27183133 | Cervix | 61.6% | Uterus | 31.5% |

(continued)

| | Illumina Probe Name | Chr. | Position | Tissue/Cell Type 1 | % Meth. | Tissue/Cell Type 2 | % Meth. |
|---|---|---|---|---|---|---|---|
| 158 | cg00555456 | 7 | 96745696 | Colon | 42.4% | Rectum | 17.9% |
| 159 | cg03339817 | 2 | 128458281 | Monocytes | 22.1% | Neutrophils | 97.0% |
| 160 | cg15857470 | 7 | 104945248 | Colon | 39.0% | Rectum | 63.4% |
| 161 | cg03260021 | 12 | 128170477 | Cervix | 37.1% | Uterus | 67.2% |
| 162 | cg16596052 | 10 | 121271746 | NK-cells | 91.0% | CD8+ T-cells | 46.3% |
| 163 | cg05803631 | 1 | 7844446 | Esophagus | 3.4% | Stomach | 47.4% |
| 164 | cg10443315 | 8 | 7947282 | Colon | 67.4% | Rectum | 43.3% |
| 165 | cg27592331 | 18 | 19756582 | Adipocytes | 79.8% | Vasc endo cells | 8.4% |
| 166 | cg24730157 | 1 | 984428 | Colon | 43.6% | Rectum | 67.5% |
| 167 | cg09696385 | 7 | 127528039 | Eosinophils | 22.7% | Neutrophils | 80.7% |
| 168 | cg08329754 | 17 | 78797015 | Cervix | 40.1% | Uterus | 70.1% |
| 169 | cg09728393 | 6 | 17851756 | Colon | 65.7% | Rectum | 41.9% |
| 170 | cg16810279 | 17 | 76254129 | CD4+ T-cells | 13.8% | CD8+ T-cells | 63.2% |
| 171 | cg12589143 | 6 | 29894341 | Colon | 34.2% | Rectum | 10.5% |
| 172 | cg06518838 | 6 | 170403264 | Esophagus | 73.3% | Stomach | 31.0% |
| 173 | cg12465678 | 1 | 27953336 | NK-cells | 13.8% | CD8+ T-cells | 58.5% |
| 174 | cg25503695 | 4 | 798983 | Colon | 57.1% | Rectum | 80.7% |
| 175 | cg15864691 | 7 | 27217606 | Cervix | 29.9% | Uterus | 59.8% |
| 176 | cg01951086 | 4 | 111551642 | Colon | 41.5% | Rectum | 17.9% |
| 177 | cg20485607 | 1 | 120217696 | Cervix | 77.8% | Uterus | 47.9% |
| 178 | cg10041379 | 6 | 33221464 | Colon | 82.6% | Rectum | 59.1% |
| 179 | cg09893465 | 10 | 134588040 | Esophagus | 40.7% | Head and neck | 96.5% |
| 180 | cg13058457 | 15 | 38422160 | Kidney | 67.7% | Uterus | 9.4% |
| 181 | cg16786640 | 4 | 3485263 | Colon | 31.7% | Rectum | 55.1% |
| 182 | cg04365699 | 3 | 177320997 | Eosinophils | 18.6% | Neutrophils | 76.4% |
| 183 | cg02561103 | 7 | 115862891 | Cervix | 47.9% | Uterus | 77.7% |
| 184 | cg14255824 | 9 | 71795999 | Vasc endo cells | 16.1% | Uterus | 81.4% |
| 185 | cg20156237 | 8 | 144543750 | NK-cells | 76.4% | CD8+ T-cells | 32.2% |
| 186 | cg06830784 | 2 | 183731824 | Colon | 43.9% | Rectum | 20.5% |
| 187 | cg13600149 | 9 | 98079129 | CD4+ T-cells | 7.3% | CD8+ T-cells | 56.5% |
| 188 | cg05359518 | 15 | 57508437 | Colon | 76.3% | Rectum | 52.9% |
| 189 | cg11846905 | 12 | 86342281 | Cervix | 36.5% | Uterus | 66.2% |
| 190 | cg24314564 | 3 | 188668940 | Monocytes | 12.7% | Neutrophils | 86.9% |
| 191 | cg07227024 | 2 | 202163482 | Esophagus | 28.2% | Stomach | 70.0% |
| 192 | cg00040312 | 12 | 54424679 | Colon | 44.8% | Rectum | 21.6% |
| 193 | cg06430465 | 16 | 1014935 | Colon | 54.8% | Rectum | 31.7% |
| 194 | cg26923863 | 4 | 1221838 | NK-cells | 38.1% | CD8+ T-cells | 82.1% |
| 195 | cg09001356 | 17 | 78797061 | Cervix | 45.2% | Uterus | 74.6% |
| 196 | cg19236493 | 17 | 46629596 | Breast | 86.6% | Head and neck | 30.8% |

(continued)

| | Illumina Probe Name | Chr. | Position | Tissue/Cell Type 1 | % Meth. | Tissue/Cell Type 2 | % Meth. |
|---|---|---|---|---|---|---|---|
| 197 | cg06434344 | 16 | 3515991 | Colon | 60.0% | Rectum | 83.1% |
| 198 | cg24345747 | 2 | 87015813 | CD4+ T-cells | 66.4% | CD8+ T-cells | 17.7% |
| 199 | cg05877497 | 2 | 66667946 | Colon | 25.9% | Rectum | 48.8% |
| 200 | cg27224986 | 4 | 53906430 | Eosinophils | 21.9% | Neutrophils | 79.6% |
| 201 | cg12502325 | 7 | 111901177 | Cervix | 46.5% | Uterus | 75.9% |
| 202 | cg24538625 | 11 | 51579523 | Colon | 61.3% | Rectum | 38.4% |
| 203 | cg25485875 | 10 | 134600998 | Esophagus | 10.1% | Stomach | 51.5% |
| 204 | cg15986668 | 1 | 41156730 | Colon | 40.0% | Rectum | 62.9% |
| 205 | cg27243507 | 3 | 101808873 | Cervix | 40.7% | Uterus | 70.0% |
| 206 | cg12754495 | 3 | 151102703 | Adipocytes | 12.9% | Uterus | 84.2% |
| 207 | cg08704934 | 3 | 194826585 | Colon | 70.1% | Rectum | 47.3% |
| 208 | cg09232358 | 14 | 23015657 | NK-cells | 62.0% | CD8+ T-cells | 18.0% |
| 209 | cg10065209 | 1 | 1267559 | Colon | 68.5% | Rectum | 91.3% |
| 210 | cg03318654 | 2 | 87034200 | CD4+ T-cells | 74.5% | CD8+ T-cells | 26.2% |
| 211 | cg03653399 | 8 | 142233436 | Bladder | 82.1% | Uterus | 31.1% |
| 212 | cg20202760 | 8 | 53499558 | Cervix | 44.3% | Uterus | 73.6% |
| 213 | cg11146821 | 1 | 62664360 | Colon | 50.4% | Rectum | 73.2% |
| 214 | cg02827046 | 11 | 125649030 | Colon | 75.5% | Rectum | 52.7% |
| 215 | cg02695349 | 7 | 38269241 | Cervix | 31.9% | Uterus | 61.1% |
| 216 | cg05390968 | 16 | 86598792 | Colon | 59.3% | Rectum | 36.9% |
| 217 | cg07355841 | 16 | 67427339 | Esophagus | 6.8% | Stomach | 48.0% |
| 218 | cg06706813 | 3 | 194826411 | Colon | 76.2% | Rectum | 53.8% |
| 219 | cg07795928 | 1 | 226900896 | Eosinophils | 17.9% | Neutrophils | 75.3% |
| 220 | cg03810198 | 17 | 74679597 | Cervix | 56.8% | Uterus | 27.6% |
| 221 | cg04416414 | 19 | 14260587 | Kidney | 73.1% | Uterus | 14.9% |
| 222 | cg01733176 | 4 | 111561070 | Colon | 37.1% | Rectum | 14.7% |
| 223 | cg02902412 | 18 | 77087900 | CD4+ T-cells | 91.6% | CD8+ T-cells | 43.9% |
| 224 | cg00826640 | 7 | 157381078 | Colon | 50.4% | Rectum | 72.7% |
| 225 | cg10092377 | 1 | 200880981 | Cervix | 78.3% | Uterus | 49.2% |
| 226 | cg11753157 | 14 | 99734018 | NK-cells | 62.0% | CD8+ T-cells | 18.0% |
| 227 | cg23651889 | 3 | 188115336 | Colon | 39.1% | Rectum | 61.3% |
| 228 | cg10293282 | 16 | 50977896 | Cervix | 46.9% | Uterus | 76.0% |
| 229 | cg04689773 | 14 | 92199710 | Colon | 61.5% | Rectum | 39.3% |
| 230 | cg25953239 | 2 | 176982342 | Vasc endo cells | 3.4% | Bladder | 64.1% |
| 231 | cg14676272 | 8 | 49836160 | Breast | 90.3% | Head and neck | 35.7% |
| 232 | cg04131969 | 2 | 33951647 | Cervix | 76.5% | Uterus | 47.5% |
| 233 | cg06449334 | 20 | 17593441 | CD4+ T-cells | 63.0% | CD8+ T-cells | 15.7% |
| 234 | cg13292042 | 4 | 111535334 | Colon | 40.5% | Rectum | 18.3% |
| 235 | cg00804179 | 3 | 13083974 | Breast | 95.1% | Uterus | 42.6% |

(continued)

| | Illumina Probe Name | Chr. | Position | Tissue/Cell Type 1 | % Meth. | Tissue/Cell Type 2 | % Meth. |
|---|---|---|---|---|---|---|---|
| 236 | cg12377972 | 5 | 132009630 | Eosinophils | 21.4% | Neutrophils | 7.8.6% |
| 237 | cg23443098 | 10 | 13987636 | Cervix | 41.5% | Uterus | 70.4% |
| 238 | cg27111890 | 21 | 43823749 | NK-cells, | 61.0% | CD8+ T-cells | 17.3% |
| 239 | cg23052826 | 2 | 232479675 | Colon | 17.0% | Rectum | 39.0% |
| 240 | cg01102073 | 3 | 128204813 | Colon | 35.3% | Rectum | 57.3% |
| 241 | cg00375132 | 10 | 22768370 | Cervix | 40.4% | Uterus | 69.3% |
| 242 | cg23697546 | 12 | 54424085 | Colon | 30.9% | Rectum | 8.9% |
| 243 | cg27499850 | 16 | 73127462 | Colon | 45.5% | Rectum | 67.4% |
| 244 | cg24037651 | 8 | 143449800 | Monocytes | 14.4% | Neutrophils | 88.5% |
| 245 | cg12583572 | 7 | 1967976 | Colon | 72.4% | Rectum | 50.6% |
| 246 | cg13468685 | 2 | 68592737 | NK-cells | 12.3% | CD8+ T-cells | 55.8% |
| 247 | cg22772386 | 6 | 167763320 | Cervix | 47.9% | Uterus | 76.6% |
| 248 | cg05609780 | 3 | 24001171 | Colon | 70.4% | Rectum | 48.6% |
| 249 | cg14477767 | 4 | 170195438 | CD4+ T-cells | 26.5% | CD8+ T-cells | 73.7% |
| 250 | cg20075156 | 4 | 11430717 | Esophagus | 1.7% | Stomach | 42.9% |
| 251 | cg26937434 | 16 | 21245151 | Breast | 36.3% | Head and neck | 86.4% |
| 252 | cg14269813 | 9 | 3899055 | Colon | 37.0% | Rectum | 58.8% |
| 253 | cg09636715 | 7 | 27217057 | Cervix | 27.5% | Uterus | 56.2% |
| 254 | cg26708319 | 4 | 111552040 | Colon | 32.1% | Rectum | 10.4% |
| 255 | cg07163168 | 1 | 76899846 | Colon | 72.2% | Rectum | 50.5% |
| 256 | cg11130630 | 12 | 109899089 | NK-cells | 18.1% | CD8+ T-cells | 61.5% |
| 257 | cg13488570 | 1 | 2222253 | Kidney | 63.6% | Uterus | 5.6% |
| 258 | cg18380211 | 5 | 98247404 | Eosinophils | 18.7% | Neutrophils | 75.8% |
| 259 | cg27424906 | 1 | 62662166 | Colon | 61.4% | Rectum | 83.1% |
| 260 | cg07232063 | 1 | 86953050 | Cervix | 36.8% | Uterus | 65.5% |
| 261 | cg13353717 | 6 | 32634276 | Colon | 15.1% | Rectum | 36.8% |
| 262 | cg25967170 | 12 | 67127781 | Cervix | 34.6% | Uterus | 63.2% |
| 263 | cg04846710 | 1 | 145478286 | Colon | 39.1% | Rectum | 17.4% |
| 264 | cg18857618 | 2 | 87048489 | CD4+ T-cells | 81.4% | CD8+ T-cells | 34.5% |
| 265 | cg04615460 | 12 | 115125098 | Colon | 49.6% | Rectum | 28.0% |
| 266 | cg08061598 | 6 | 112081538 | Cervix | 22.5% | Uterus | 51.1% |
| 267 | cg13751386 | 9 | 97856033 | Colon | 60.6% | Rectum | 82.2% |
| 268 | cg24506221 | 1 | 110230401 | Esophagus | 53.2% | Stomach | 12.0% |
| 269 | cg01802772 | 1 | 55014160 | Colon | 30.5% | Rectum | 8.9% |
| 270 | cg07769588 | 19 | 10655622 | Colon | 45.0% | Rectum | 66.5% |
| 271 | cg11945507 | 8 | 142233382 | Bladder | 73.1% | Uterus | 23.4% |
| 272 | cg19579080 | 17 | 66588601 | Cervix | 39.3% | Uterus | 67.9% |
| 273 | cg19301114 | 6 | 161796785 | Monocytes | 10.4% | Neutrophils | 84.3% |
| 274 | cg26827987 | 16 | 86232457 | Breast | 3.9% | Head and neck | 53.1% |

(continued)

| | Illumina Probe Name | Chr. | Position | Tissue/Cell Type 1 | % Meth. | Tissue/Cell Type 2 | % Meth. |
|---|---|---|---|---|---|---|---|
| 275 | cg23705108 | 4 | 27102875 | Colon | 63.5% | Rectum | 42.0% |
| 276 | cg12910830 | 5 | 162929657 | Eosinophils | 20.9% | Neutrophils | 77.9% |
| 277 | cg18303397 | 3 | 129160135 | CD4+ T-cells | 24.0% | CD8+ T-cells | 70.4% |
| 278 | cg23580725 | 14 | 99707721 | NK-cells | 65.9% | CD8+ T-cells | 22.7% |
| 279 | cg12569246 | 6 | 76203666 | Colon | 64.2% | Rectum | 42.7% |
| 280 | cg25294526 | 17 | 7786177 | Cervix | 43.0% | Uterus | 71.4% |
| 281 | cg17030488 | 12 | 115172748 | Colon | 80.6% | Rectum | 59.2% |
| 282 | cgI9304088 | 4 | 111547790 | Colon | 39.9% | Rectum | 18.6% |
| 283 | cg09884146 | 2 | 65593908 | Cervix | 19.0% | Uterus | 47.4% |
| 284 | cg10617784 | 2 | 71134586 | Colon | 24.3% | Rectum | 45.6% |
| 285 | cg23027574 | 6 | 30043194 | Esophagus | 4.8% | Stomach | 45.7% |
| 286 | cg25020969 | 7 | 1967802 | Colon | 81.1% | Rectum | 59.9% |
| 287 | cg01806427 | 5 | 123737813 | NK-cells | 29.5% | CD8+ T-cells | 72.7% |
| 288 | cg14727763 | 10 | 78961643 | Cervix | 41.8% | Uterus | 70.2% |
| 289 | cg21091766 | 5 | 176898673 | CD4+ T-cells | 85.0% | CD8+ T-cells | 38.7% |
| 290 | cg05218882 | 3 | 132440131 | Colon | 59.8% | Rectum | 81.0% |
| 291 | cg01189072 | 1 | 1610660 | Bladder | 87.8% | Esophagus | 35.7% |
| 292 | cg12626036 | 5 | 134662980 | Colon | 77.6% | Rectum | 56.5% |
| 293 | cg20701689 | 2 | 183731832 | Colon | 40.5% | Rectum | 19.4% |
| 294 | cg14170999 | 17 | 79380515 | Cervix | 25.5% | Uterus | 53.8% |
| 295 | cg01055099 | 16 | 3516099 | Colon | 41.8% | Rectum | 62.9% |
| 296 | cg00779602 | 3 | 66002927 | Breast | 87.3% | Head and neck | 38.4% |
| 297 | cg18254848 | 19 | 40227949 | Eosinophils | 23.7% | Neutrophils | 80.6% |
| 298 | cg01403655 | 13 | 43702940 | Cervix | 47.2% | Uterus | 75.5% |
| 299 | cg07058086 | 8 | 29120186 | Colon | 15.4% | Rectum | 36.4% |
| 300 | cg14338425 | 8 | 120220799 | Esophagus | 9.2% | Stomach | 50.1% |
| 301 | cg22911650 | 19 | 51645423 | NK-cells | 27.8% | CD8+ T-cells | 70.8% |
| 302 | cg08890690 | 4 | 111553018 | Colon | 31.0% | Rectum | 10.0% |
| 303 | cg18174654 | 2 | 87019612 | CD4+ T-cells | 81.0% | CD8+ T-cells | 34.9% |
| 304 | cg07284231 | 8 | 700689 | Bladder | 79.2% | Uterus | 29.6% |
| 305 | cg05668703 | 17 | 79380493 | Cervix | 26.8% | Uterus | 55.0% |
| 306 | cg22244192 | 6 | 33943837 | Colon | 50.0% | Rectum | 29.1% |
| 307 | cg17144149 | 17 | 46656572 | Colon | 47.5% | Rectum | 68.4% |
| 308 | cg18179075 | 7 | 129913008 | Colon | 52.6% | Rectum | 31.7% |
| 309 | cg08013737 | 17 | 35480754 | Cervix | 39.1% | Uterus | 67.3% |
| 310 | cg24820809 | 22 | 36019252 | Colon | 65.8% | Rectum | 86.7% |
| 311 | cg01898661 | 16 | 90114063 | Esophagus | 8.8% | Stomach | 49.6% |
| 312 | cg12279419 | 6 | 33943662 | Colon | 49.3% | Rectum | 28.4% |
| 313 | cg05154454 | 9 | 113100053 | Cervix | 40.5% | Uterus | 68.6% |

(continued)

| | Illumina Probe Name | Chr. | Position | Tissue/Cell Type 1 | % Meth. | Tissue/Cell Type 2 | % Meth. |
|---|---|---|---|---|---|---|---|
| 314 | cg09654116 | 7 | 158816213 | CD4+ T-cells | 87.0% | CD8+ T-cells | 41.4% |
| 315 | cg01757124 | 17 | 76320916 | Colon | 39.9% | Rectum | 19.1% |
| 316 | cg21261709 | 7 | 104887042 | Eosinophils | 21.4% | Neutrophils | 78.0% |
| 317 | cg00910015 | 2 | 3826621 | Kidney | 35.2% | Uterus | 92.9% |
| 318 | cg11551216 | 12 | 115125619 | Colon | 48.7% | Rectum | 28.0% |
| 319 | cg03787603 | 7 | 101768610 | Cervix | 40.1% | Uterus | 68.2% |
| 320 | cg14987787 | 12 | 115105220 | Colon | 38.1% | Rectum | 17.4% |
| 321 | cg00031277 | 4 | 101943443 | Cervix | 42.7% | Uterus | 70.8% |
| 322 | cg10752315 | 3 | 150238463 | Colon | 68.8% | Rectum | 89.5% |
| 323 | cg03687070 | 2 | 16210108 | Bladder | 91.2% | Esophagus | 39.1% |
| 324 | cg23597015 | 15 | 68932319 | Bladder | 81.1% | Uterus | 31.6% |
| 325 | cg17945429 | 1 | 158174027 | NK-cells | 60.4% | CD8+ T-cells | 17.5% |
| 326 | cg03051836 | 20 | 22563319 | Colon | 53.9% | Rectum | 33.2% |
| 327 | cg26146569 | 15 | 31637592 | CD4+ T-cells | 60.6% | CD8+ T-cells | 15.3% |
| 328 | cg05552183 | 6 | 42928497 | Esophagus | 1.6% | Stomach | 42.2% |
| 329 | cg09273483 | 1 | 244511482 | Cervix | 22.4% | Uterus | 50.5% |
| 330 | cg00968270 | 16 | 87471788 | Colon | 71.5% | Rectum | 50.8% |
| 331 | cg11354682 | 19 | 10978833 | Monocytes | 11.2% | Neutrophils | 84.9% |
| 332 | cg17965712 | 5 | 2757802 | Breast | 68.8% | Uterus | 16.6% |
| 333 | cg02959678 | 19 | 1047604 | Colon | 52.6% | Rectum | 73.3% |
| 334 | cg13600632 | 9 | 129169002 | Cervix | 44.9% | Uterus | 73.0% |
| 335 | cg08822897 | 11 | 64258103 | Colon | 51.5% | Rectum | 72.1% |
| 336 | cg12192145 | 14 | 55849528 | Eosinophils | 20.6% | Neutrophils | 77.1% |
| 337 | cg08707875 | 1 | 224575077 | Colon | 68.6% | Rectum | 48.0% |
| 338 | cg12608692 | 5 | 16742020 | Cervix | 33.5% | Uterus | 61.5% |
| 339 | cg13818708 | 6 | 30291314 | Colon | 86.4% | Rectum | 65.8% |
| 340 | cg06419846 | 11 | 66083697 | CD4+ T-cells | 94.8% | CD8+ T-cells | 49.6% |
| 341 | cg10682299 | 4 | 111550154 | Colon | 48.4% | Rectum | 27.8% |
| 342 | cg02042623 | 12 | 104227548 | Cervix | 44.1% | Uterus | 72.1% |
| 343 | cg15912390 | 3 | 194826514 | Colon | 75.1% | Rectum | 54.5% |
| 344 | cg13673837 | 19 | 15306670 | Esophagus | 3.0% | Stomach | 43.6% |
| 345 | cg13016773 | 12 | 72081013 | Colon | 45.5% | Rectum | 66.1% |
| 346 | cg05355436 | 10 | 63520873 | Cervix | 36.3% | Uterus | 64.2% |
| 347 | cg02866149 | 20 | 35405989 | Colon | 59.9% | Rectum | 39.3% |
| 348 | cg18760534 | 2 | 2964910 | Bladder | 91.9% | Uterus | 43.7% |
| 349 | cg03117976 | 18 | 11149435 | Colon | 32.8% | Rectum | 12.3% |
| 350 | cg02225720 | 17 | 3623472 | Cervix | 17.3% | Uterus | 45.1% |
| 351 | cg24798398 | 2 | 176937524 | Colon | 18.9% | Rectum | 39.4% |
| 352 | cg00842595 | 17 | 263193 | CD4+ T-cells | 34.2% | CD8+ T-cells | 79.4% |

(continued)

| | Illumina Probe Name | Chr. | Position | Tissue/Cell Type 1 | % Meth. | Tissue/Cell Type 2 | % Meth. |
|---|---|---|---|---|---|---|---|
| 353 | cg15899743 | 14 | 22985112 | NK-cells | 69.4% | CD8+ T-cells | 26.6% |
| 354 | cg12744859 | 17 | 46669492 | Breast | 75.8% | Head and neck | 27.1% |
| 355 | cg00723994 | 12 | 115105290 | Colon | 44.3% | Rectum | 23.9% |
| 356 | cg16139202 | 5 | 134662788 | Colon | 88.0% | Rectum | 67.6% |
| 357 | cg26975184 | 7 | 140267040 | Esophagus | 42.1% | Head and neck | 96.8% |
| 358 | cg17470397 | 3 | 193308169 | Eosinophils | 20.8% | Neutrophils | 77.2% |
| 359 | cg03144922 | 4 | 111540992 | Colon | 34.2% | Rectum | 13.9% |
| 360 | cg01401824 | 2 | 159902017 | Cervix | 47.6% | Uterus | 75.4% |
| 361 | cg06796779 | 3 | 128204927 | Colon | 43.2% | Rectum | 63.5% |
| 362 | cg00892792 | 8 | 142297584 | Colon | 79.0% | Rectum | 58.7% |
| 363 | cg14054883 | 9 | 93619467 | NK-cells | 58.7% | CD8+ T-cells | 16.2% |
| 364 | cg05617307 | 10 | 121413182 | CD4+ T-cells | 33.0% | CD8+ T-cells | 78.2% |
| 365 | cg07249765 | 7 | 4244643 | Cervix | 93.0% | Uterus | 65.3% |
| 366 | cg21150839 | 20 | 62209634 | Colon | 57.5% | Rectum | 77.8% |
| 367 | cg26992028 | 7 | 1030287 | Colon | 70.6% | Rectum | 50.3% |
| 368 | cg07702235 | 2 | 71983542 | Cervix | 20.1% | Uterus | 47.8% |
| 369 | cg20559385 | 6 | 106049591 | Monocytes | 11.6% | Neutrophils | 84.2% |
| 370 | cg23207527 | 6 | 17283113 | Colon | 61.2% | Rectum | 40.9% |
| 371 | cg22355889 | 11 | 107461585 | Esophagus | 12.9% | Stomach | 53.4% |
| 372 | cg22486928 | 12 | 96251729 | Colon | 63.7% | Rectum | 43.4% |
| 373 | cg19019345 | 11 | 8888814 | Cervix | 20.2% | Uterus | 47.9% |
| 374 | cg10111816 | 16 | 22384715 | NK-cells | 54.9% | CD8+ T-cells | 12.5% |
| 375 | cg14783123 | 8 | 68022262 | Eosinophils | 23.3% | Neutrophils | 79.6% |
| 376 | cg24896021 | 8 | 6630930 | Colon | 73.1% | Rectum | 52.9% |
| 377 | cg02058628 | 1 | 2164542 | CD4+ T-cells | 38.4% | CD8+ T-cells | 83.4% |
| 378 | cg02208793 | 11 | 84634615 | Colon | 68.2% | Rectum | 48.0% |
| 379 | cg00666623 | 8 | 105375683 | Colon | 81.5% | Rectum | 61.3% |
| 380 | cg16345566 | 6 | 32633102 | Colon | 46.1% | Rectum | 66.3% |
| 381 | cg21780711 | 5 | 122765476 | Cervix | 49.2% | Uterus | 76.7% |
| 382 | cg26400750 | 14 | 22992073 | NK-cells | 60.7% | CD8+ T-cells | 18.4% |
| 383 | cg22986569 | 5 | 2659008 | Colon | 79.7% | Rectum | 59.6% |
| 384 | cg12145488 | 10 | 134514630 | Colon | 58.4% | Rectum | 78.5% |
| 385 | cg12673499 | 10 | 104412063 | CD4+ T-cells | 59.2% | CD8+ T-cells | 14.2% |
| 386 | cg20663852 | 20 | 50312386 | Colon | 47.4% | Rectum | 27.3% |
| 387 | cg13495373 | 7 | 28474813 | Cervix | 47.8% | Uterus | 75.3% |
| 388 | cg27491759 | 10 | 131988874 | Esophagus | 2.4% | Stomach | 42.8% |
| 389 | cg19373813 | 2 | 176989586 | Colon | 39.4% | Rectum | 59.5% |
| 390 | cg00664697 | 16 | 80061290 | Bladder | 14.7% | Uterus | 62.0% |
| 391 | cg08767838 | 20 | 62209642 | Colon | 54.6% | Rectum | 74.7% |

(continued)

| | Illumina Probe Name | Chr. | Position | Tissue/Cell Type 1 | % Meth. | Tissue/Cell Type 2 | % Meth. |
|---|---|---|---|---|---|---|---|
| 392 | cg25643644 | 11 | 118210017 | NK-cells | 52.7% | CD8+ T-cells | 10.4% |
| 393 | cg10590657 | 3 | 192538384 | Colon | 85.9% | Rectum | 65.9% |
| 394 | cg26508164 | 2 | 182650931 | Cervix | 40.4% | Uterus | 67.9% |
| 395 | cg15402992 | 20 | 44714513 | Colon | 52.6% | Rectum | 72.6% |
| 396 | cg02346442 | 3 | 186194046 | Eosinophils | 19.4% | Neutrophils | 75.7% |
| 397 | cg13939204 | 9 | 130667057 | Colon | 51.9% | Rectum. | 31.9% |
| 398 | cg02202923 | 10 | 135040250 | Breast | 27.1% | Uterus | 78.2% |
| 399 | cg23497611 | 2 | 37985316 | Cervix | 37.1% | Uterus | 64.5% |
| 400 | cg10585941 | 1 | 214461479 | CD4+ T-cells | 60.4% | CD8+ T-cells | 15.9% |
| 401 | cg23255141 | 15 | 51199395 | Colon | 40.8% | Rectum | 60.8% |
| 402 | cg00012522 | 15 | 98503952 | Esophagus | 7.3% | Stomach | 47.4% |
| 403 | cg25283205 | 12 | 58333626 | Colon | 74.3% | Rectum | 54.3% |
| 404 | cg10509607 | 14 | 99658541 | NK-cells | 65.4% | CD8+ T-cells | 23.2% |
| 405 | cg13038847 | 22 | 28073997 | Cervix | 38.5% | Uterus | 65.9% |
| 406 | cg02645340 | 13 | 28553783 | Colon | 38.1% | Rectum | 58.1% |
| 407 | cg23426945 | 10 | 77190054 | Cervix | 61.3% | Uterus | 34.0% |
| 408 | cg12582008 | 13 | 53603286 | Colon | 60.3% | Rectum | 80.3% |
| 409 | cg03469682 | 3 | 100889156 | Colon | 51.9% | Rectum | 31.9% |
| 410 | cg05979457 | 8 | 105373628 | Cervix | 18.9% | Uterus | 46.2% |
| 411 | cg00705730 | 2 | 106438120 | Monocytes | 83.8% | Neutrophils | 11.6% |
| 412 | cg17818435 | 12 | 53497138 | CD4+ T-cells | 65.8% | CD8+ T-cells | 21.4% |
| 413 | cg08864645 | 6 | 33943929 | Colon | 56.7% | Rectum | 36.8% |
| 414 | cg09953583 | 14 | 22974144 | NK-cells | 76.6% | CD8+ T-cells | 34.6% |
| 415 | cg19759549 | 3 | 128211096 | Colon | 15.2% | Rectum | 35.1% |
| 416 | cg20282837 | 10 | 3508379 | Eosinophils | 24.5% | Neutrophils | 80.6% |
| 417 | cg24845296 | 3 | 142775293 | Cervix | 53.9% | Uterus | 81.2% |
| 418 | cg04060356 | 20 | 52226170 | Colon | 45.2% | Rectum | 25.3% |
| 419 | cg17997684 | 2 | 38151490 | Colon | 52.7% | Rectum | 72.6% |
| 420 | cg17632579 | 4 | 119274013 | Esophagus | 3.7% | Stomach | 43.8% |
| 421 | cg10901805 | 11 | 84634620 | Colon | 76.9% | Rectum | 57.0% |
| 422 | cg27006252 | 14 | 23391205 | Cervix | 47.6% | Uterus | 74.9% |
| 423 | cg05585149 | 10 | 695844 | Esophagus | 29.6% | Head and neck | 83.5% |
| 424 | cg07039378 | 7 | 73645723 | NK-cells | 28.9% | CD8+ T-cells | 70.8% |
| 425 | cg23256802 | 5 | 133313281 | Colon | 44.1% | Rectum | 63.9% |
| 426 | cg08506127 | 2 | 87018958 | CD4+ T-cells | 63.8% | CD8+ T-cells | 19.5% |
| 427 | cg27217253 | 17 | 8648562 | Colon | 45.9% | Rectum | 65.7% |
| 428 | cg19047868 | 17 | 46669485 | Cervix | 83.0% | Uterus | 55.8% |
| 429 | cg20521198 | 4 | 27102907 | Colon | 48.4% | Rectum | 28.6% |
| 430 | cg17346176 | 6 | 119239700 | Colon | 76.8% | Rectum | 57.0% |

(continued)

| | Illumina Probe Name | Chr. | Position | Tissue/Cell Type 1 | % Meth. | Tissue/Cell Type 2 | % Meth. |
|---|---|---|---|---|---|---|---|
| 431 | cg02046247 | 12 | 1662459 | Cervix | 42.7% | Uterus | 69.9% |
| 432 | cg26858152 | 8 | 140971523 | Colon | 70.9% | Rectum | 51.1% |
| 433 | cg13677149 | 7 | 27284789 | Colon | 38.6% | Rectum | 58.3% |
| 434 | cg23172995 | 5 | 150486801 | Eosinophils | 19.8% | Neutrophils | 75.8% |
| 435 | cg20852378 | 4 | 184680264 | Colon | 67.4% | Rectum | 47.7% |
| 436 | cg16582660 | 6 | 11329463 | Cervix | 42.4% | Uterus | 69.6% |
| 437 | cg20885910 | 11 | 48922741 | Colon | 86.2% | Rectum | 66.5% |
| 438 | cg02891314 | 5 | 179741120 | Esophagus | 53.8% | Stomach | 93.8% |
| 439 | cg23717186 | 1 | 226924311 | NK-cells | 64.9% | CD8+ T-cells | 23.1% |
| 440 | cg05059607 | 17 | 65488100 | CD4+ T-cells | 75.8% | CD8+ T-cells | 32.2% |
| 441 | cg17242937 | 4 | 111561114 | Colon | 53.5% | Rectum | 33.8% |
| 442 | cg15676528 | 2 | 9865398 | Colon | 58.2% | Rectum | 77.9% |
| 443 | cg06570967 | 10 | 96989650 | Cervix | 38.0% | Uterus | 65.2% |
| 444 | cg25595431 | 6 | 166261900 | Colon | 73.1% | Rectum | 92.7% |
| 445 | cg00225902 | 3 | 66444122 | Bladder | 62.1% | Uterus | 15.3% |
| 446 | cg05211868 | 4 | 184718046 | Colon | 31.6% | Rectum | 51.2% |
| 447 | cg01714284 | 4 | 185714524 | Cervix | 36.3% | Uterus | 63.5% |
| 448 | cg25373630 | 18 | 60984656 | Colon | 46.1% | Rectum | 65.7% |
| 449 | cg13163833 | 2 | 176954533 | Colon | 53.1% | Rectum | 72.7% |
| 450 | cg01476222 | 11 | 36522143 | Monocytes | 17.3% | Neutrophils | 89.4% |
| 451 | cg11067179 | 11 | 66083541 | CD4+ T-cells | 83.5% | CD8+ T-cells | 40.1% |
| 452 | cg08599635 | 16 | 78496508 | Breast | 83.5% | Head and neck | 35.7% |
| 453 | cg24410546 | 8 | 26371551 | Esophagus | 3.0% | Stomach | 42.8% |
| 454 | cg26239495 | 11 | 122791447 | Colon | 65.7% | Rectum | 85.3% |
| 455 | cg27656658 | 17 | 46628224 | Cervix | 78.9% | Uterus | 51.8% |
| 456 | cg04911180 | 3 | 176775716 | Colon | 57.3% | Rectum | 76.9% |
| 457 | cg20327324 | 4 | 24590014 | Eosinophils | 25.2% | Neutrophils | 80.9% |
| 458 | cg19854437 | 14 | 99891629 | Colon | 52.2% | Rectum | 71.7% |
| 459 | cg14689338 | 5 | 16742054 | Cervix | 34.7% | Uterus | 61.8% |
| 460 | cg07339236 | 20 | 50312490 | Colon | 48.2% | Rectum | 28.7% |
| 461 | cg15996644 | 12 | 54370352 | Colon | 44.9% | Rectum | 25.5% |
| 462 | cg26376241 | 2 | 65594021 | Cervix | 14.5% | Uterus | 41.6% |
| 463 | cg05408649 | 12 | 54446019 | CD4+ T-cells | 16.3% | CD8+ T-cells | 59.6% |
| 464 | cg13980266 | 9 | 97022269 | Colon | 60.4% | Rectum | 79.8% |
| 465 | cg20602300 | 15 | 40846077 | Colon | 74.4% | Rectum | 55.0% |
| 466 | cg13404054 | 19 | 15311666 | Esophagus | 14.4% | Stomach | 54.1% |
| 467 | cg10326726 | 10 | 51549505 | Esophagus | 34.8% | Head and neck | 88.6% |
| 468 | cg18498565 | 10 | 3173604 | Colon | 61.7% | Rectum | 81.1% |
| 469 | cg21185355 | 3 | 41975014 | Cervix | 24.3% | Uterus | 51.4% |

(continued)

| | Illumina Probe Name | Chr. | Position | Tissue/Cell Type 1 | % Meth. | Tissue/Cell Type 2 | % Meth. |
|---|---|---|---|---|---|---|---|
| 470 | cg08198540 | 14 | 22963867 | NK-cells | 72.5% | CD8+ T-cells | 30.9% |
| 471 | cg14526718 | 2 | 176932840 | Colon | 21.1% | Rectum | 40.4% |
| 472 | cg01016191 | 12 | 130707332 | Cervix | 82.0% | Uterus | 55.0% |
| 473 | cg04683418 | 17 | 21848728 | Colon | 89.8% | Rectum | 70.5% |
| 474 | cg26934362 | 5 | 1519265 | CD4+ T-cells | 78.0% | CD8+ T-cells | 34.7% |
| 475 | cg14384685 | 20 | 62209427 | Colon | 57.1% | Rectum | 76.4% |
| 476 | cg06775149 | 5 | 67818678 | Cervix | 45.5% | Uterus | 72.5% |
| 477 | cg05527785 | 17 | 46656543 | Colon | 39.8% | Rectum | 59.1% |
| 478 | cg18476766 | 2 | 240529742 | Breast | 79.6% | Uterus | 28.7% |
| 479 | cg13001142 | 6 | 147528521 | Eosinophils | 10.4% | Neutrophils | 66.1% |
| 480 | cg11196529 | 13 | 107182539 | Colon | 58.3% | Rectum | 77.5% |
| 481 | cg02979703 | 8 | 37558858 | Colon | 61.2% | Rectum | 80.4% |
| 482 | cg12101498 | 1 | 244829264 | NK-cells | 15.4% | CD8+ T-cells | 56.9% |
| 483 | cg15831212 | 5 | 139672796 | Colon | 62.7% | Rectum | 81.9% |
| 484 | cg08693997 | 15 | 95713013 | Cervix | 33.7% | Uterus | 60.7% |
| 485 | cg06400704 | 1 | 240256644 | Colon | 27.8% | Rectum | 8.6% |
| 486 | cg12422199 | 3 | 101284166 | Monocytes | 13.5% | Neutrophils | 85.5% |
| 487 | cg01756381 | 4 | 11430693 | Esophagus | 4.3% | Stomach | 43.8% |
| 488 | cg22801992 | 3 | 128210000 | Colon | 22.8% | Rectum | 42.0% |
| 489 | cg17636707 | 10 | 30970470 | Cervix | 46.4% | Uterus | 73.3% |
| 490 | cg10792302 | 12 | 54376019 | Colon | 71.5% | Rectum | 52.4% |
| 491 | cg03465513 | 13 | 28550046 | Colon. | 23.2% | Rectum | 42.3% |
| 492 | cg14089436 | 9 | 20624421 | Colon | 44.5% | Rectum | 63.6% |
| 493 | cg08033722 | 12 | 54370458 | Colon | 40.8% | Rectum | 21.7% |
| 494 | cg01961086 | 1 | 3086487 | Esophagus | 43.6% | Head and neck | 96.5% |
| 495 | cg10032599 | 16 | 51560612 | Cervix | 27.3% | Uterus | 54.2% |
| 496 | cg07917644 | 15 | 101766122 | Eosinophils | 16.8% | Neutrophils | 72.4% |
| 497 | cg07679948 | 12 | 5.6329641 | NK-cells | 88.4% | CD8+ T-cells | 47.0% |
| 498 | cg26783428 | 1 | 41156791 | Colon | 32.0% | Rectum | 51.1% |
| 499 | cg05120668 | 1 | 78275707 | Cervix | 42.9% | Uterus | 69.8% |
| 500 | cg20325547 | 1 | 9431594 | Colon | 60.6% | Rectum | 41.5% |

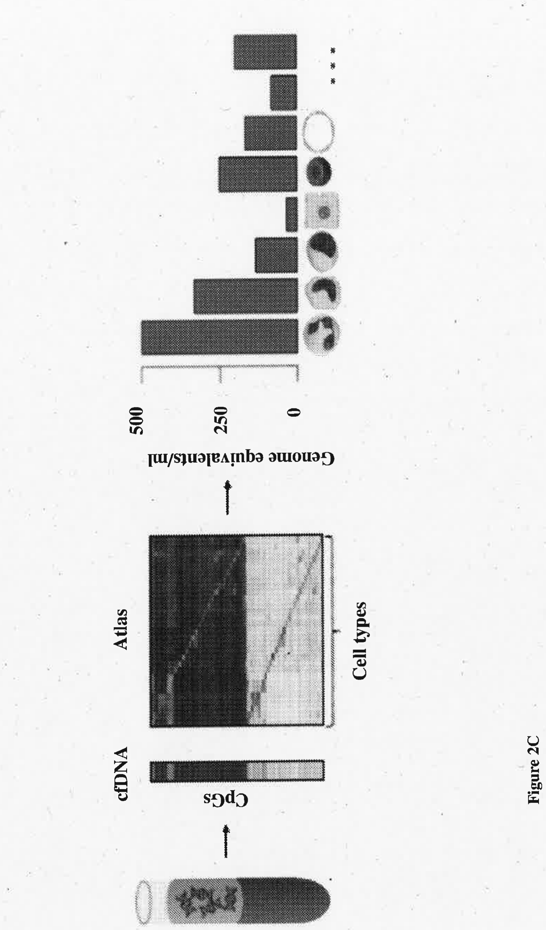

For deconvolution, it was assumed that a cfDNA methylation profile is a linear combination of the methylation profiles of the cell types which contribute to cfDNA. Under this assumption, the relative contributions of different cell types can be determined using non-negative least squares regression (NNLS) (see illustration of the process in **Figure 2C)**.

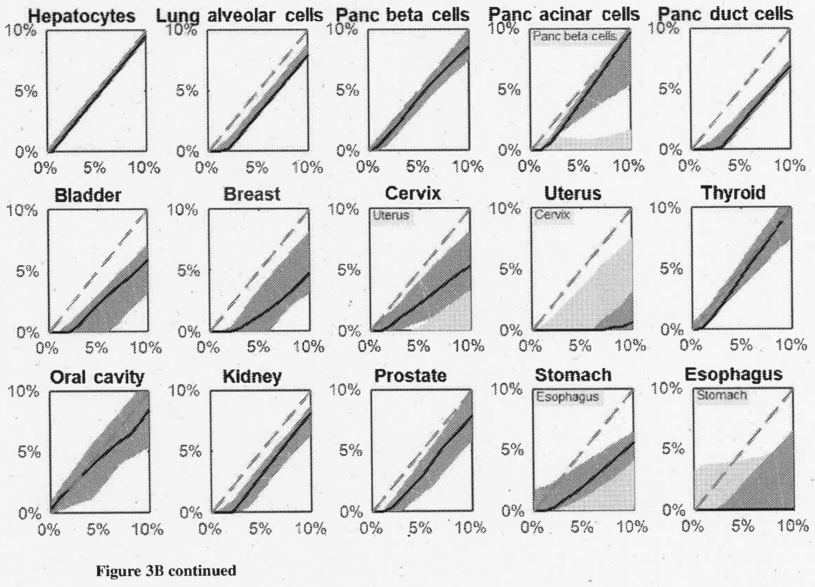

*in silico* experiments were performed initially to assess performance of the deconvolution approach in determining the relative contributions of various cell types to a methylation profile of DNA from a heterogeneous mixture of cell types. DNA methylation profiles of individual samples of cell types and tissues were computationally mixed with DNA methylation profiles of leukocytes **(Fig. 3A)** and the performance of the deconvolution algorithm on these mixes was assessed (when using a reference atlas not including the specific methylomes mixed *in silico*). It was found that every cell type in the analysis (whose methylome is present in the atlas) could be detected when composing > 1% of the mixture, with many cell types detected even when present at <1% in the mixture. Importantly, very little presence of non-leukocyte cells was detected when leukocytes alone were analyzed **(Fig. 3A).** When an atlas lacking the feature-selection described herein was used, detection was far less accurate and required the cell or tissue to comprise a greater percent of the cfDNA total **(Fig. 3B).**

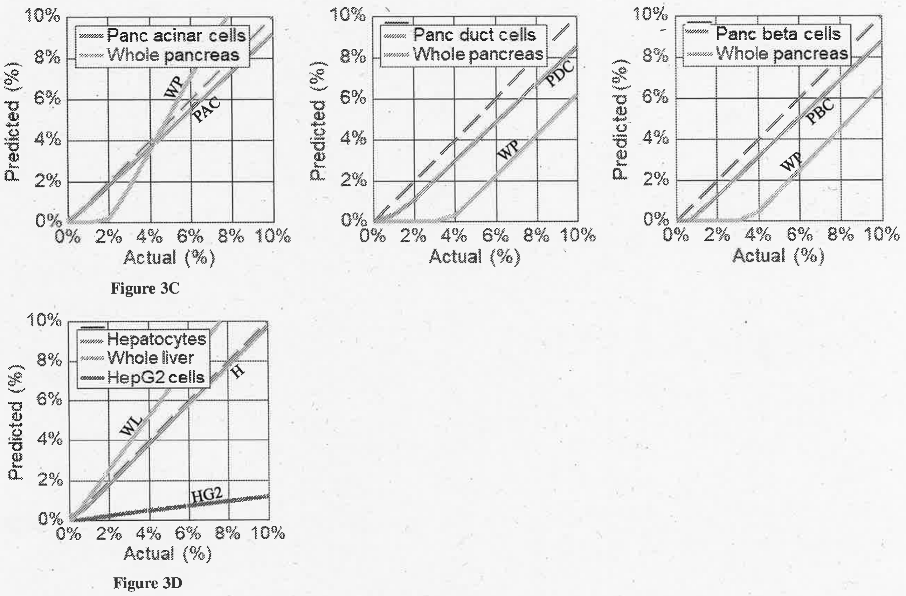

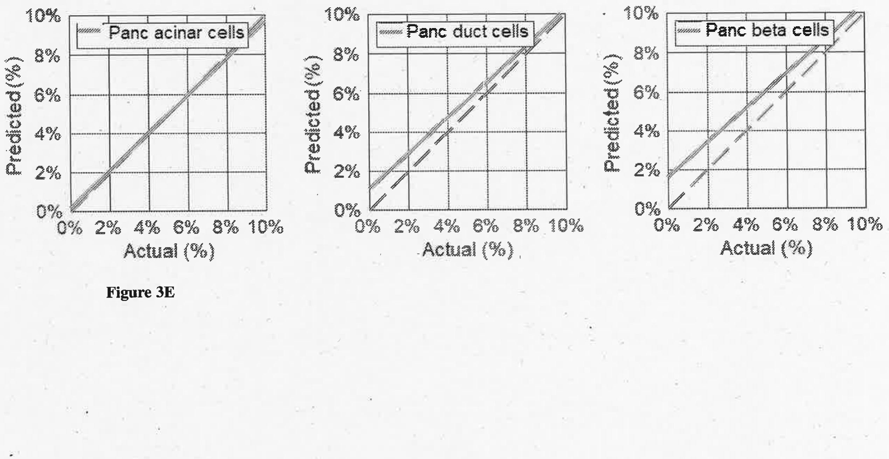

As expected, purified cell types mixed into blood methylomes were more easily detected than whole tissues, which represents heterogeneous mixtures of different cell types **(Fig. 3C-D).** An atlas containing the methylomes of purified pancreatic cells was superior in detecting pancreatic DNA within blood compared to a reference atlas containing the methylome of the whole pancreas **(Fig. 3C).** Similarly, use of a hepatocyte methylome in place of whole liver or HepG2 cells was superior in detecting cfDNA **(Fig. 3D).** Importantly, the advantage of using a cell type-specific reference rather than a tissue-specific reference was most evident when the tissue of interest was contributing <2% to the mixed methylome. Lastly, a pancreas only atlas, comprising healthy cfDNA methylomes and methylomes from three pancreatic cell types (acinar, beta and duct) was capable of identifying each cfDNA of each of those 3 cell types **(Fig. 3E).** These findings support the feasibility of highly sensitive deconvolution of the plasma methylome, and highlight the importance of using a comprehensive, cell type specific, methylome atlas for sensitive detection of rare contributors to mixed methylomes.

## Example 3: Healthy cfDNA contributors

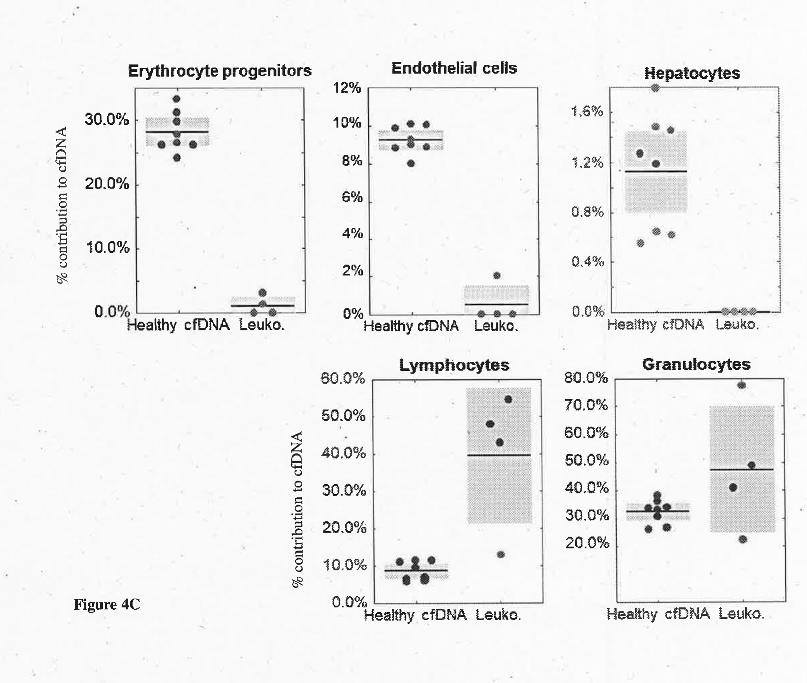

To determine the main contributors to cfDNA in healthy individuals, plasma was collected from multiple healthy donors, and the donors were classified as male or female, young (age 19-30) or elder (age 75+), and cfDNA present in plasma according to these groups was pooled, to reach 250ng cfDNA in each pool. Additionally, in a few cases blood draws of ~100ml from a single donor were possible, so as to avoid pooling. Methylation profiles of the samples were then obtained using the Illumina EPIC arrays and deconvolution analysis was performed. The predicted distribution of tissue sources was very similar among the different pools and between individual healthy donors **(Fig. 4A-B).** As has been previously reported, it was found that the main contributors to cfDNA were of hematopoietic origin. On average, 30.5% of cfDNA came from granulocytes, 31.8% from erythrocyte progenitors (likely representing the process by which erythrocyte progenitor cell lose their DNA during differentiation in the bone marrow), 18.3% from monocytes, and 7.3% from lymphocytes. The main solid tissue contributions to cfDNA were from vascular endothelial cells (~10%) and hepatocytes (~1.3%). The signal from erythrocyte progenitors, endothelial cells and hepatocytes is expected to be present in cfDNA but not in DNA isolated from leukocytes. Indeed, deconvolution of blood methylomes predicted signals from these tissues at much lower levels than in plasma, supporting validity of the algorithm **(Fig. 4C).** While the young and elder samples showed similar relative contributions of the different cell types, the plasma of older people showed higher absolute cfDNA levels for the all cell types (genome equivalents/ml). While this could indicate increased cell death in older individuals, the finding that cfDNA from all tissues was essentially affected equally suggests a slower clearance rate of circulating DNA in elder individuals **(Fig. 4B).** These findings provide a first detailed description of the composition of cfDNA in healthy subjects.

## Example 4: Deconvolution of cfDNA in pancreatic islet transplant recipients

As the analysis of cfDNA is emerging as an important tool in the monitoring of organ transplant recipients, the potential for a cfDNA methylome deconvolution approach to identify DNA from a transplanted organ in circulation was evaluated. The methylome profile generated from pooled cfDNA of type 1 diabetes patients 1 hour after receiving a cadaveric islet transplant was determined. The total concentration of cfDNA in these samples was ~20x higher than healthy control levels, suggesting a massive process of cell death shortly after islet transplantation. The deconvolution algorithm identified that a large proportion of the DNA (~17%) was of pancreatic origin, both beta cells and acinar cells **(Fig. 5A),** which had never been observed in healthy plasma. Each cell type was significantly increased on its own and also all pancreatic cells were increases when the two were taken together. These findings provide further support to the validity of the deconvolution procedure. More strikingly, it was found that most of the increase in cfDNA levels in islet transplant recipients was of an immune cell origin (granulocytes, monocytes and lymphocytes, but not erythrocyte progenitors), suggesting an acute immune response to transplantation resulting in massive immune cell death **(Fig. 5B).**

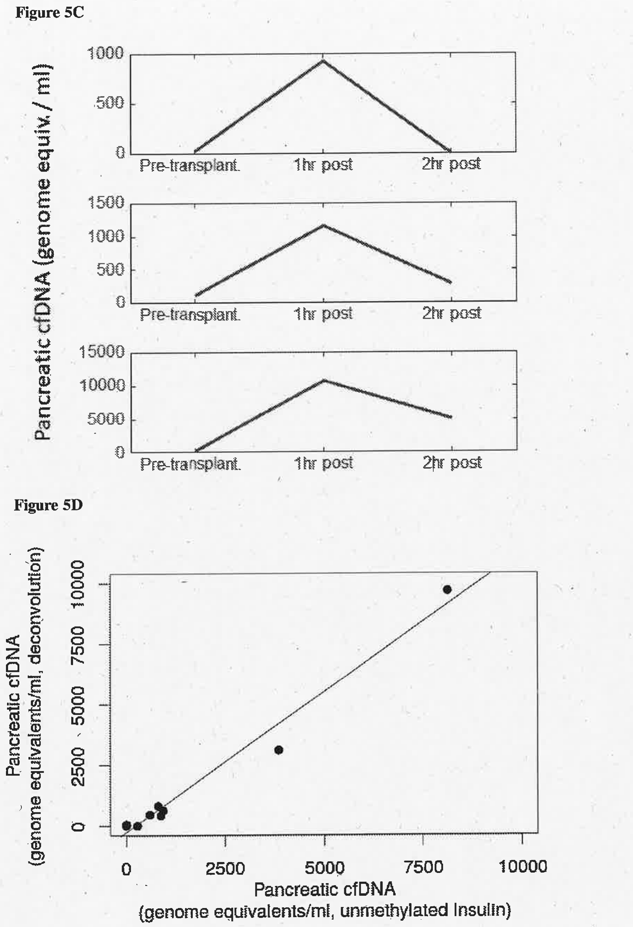

Additional control experiments were performed to assess assay validity. First, the predictions of the deconvolution algorithm for pancreatic cfDNA in the plasma of patients was assessed before (<1 day), 1 hour after, and 2 hours after transplantation. Negligible levels of pancreas cfDNA were found before islet transplantation, and a large increase immediately after transplantation, and a subsequent decrease in levels of pancreatic cfDNA, was also observed as expected **(Fig. 5C).** Second, the levels of beta cell cfDNA predicted by the deconvolution algorithm were correlated to the levels measured by specific analysis of the unmethylated insulin gene as has been reported in the art. The prediction of deconvolution agreed well with the method based on PCR and sequencing **(Fig. 5D).**

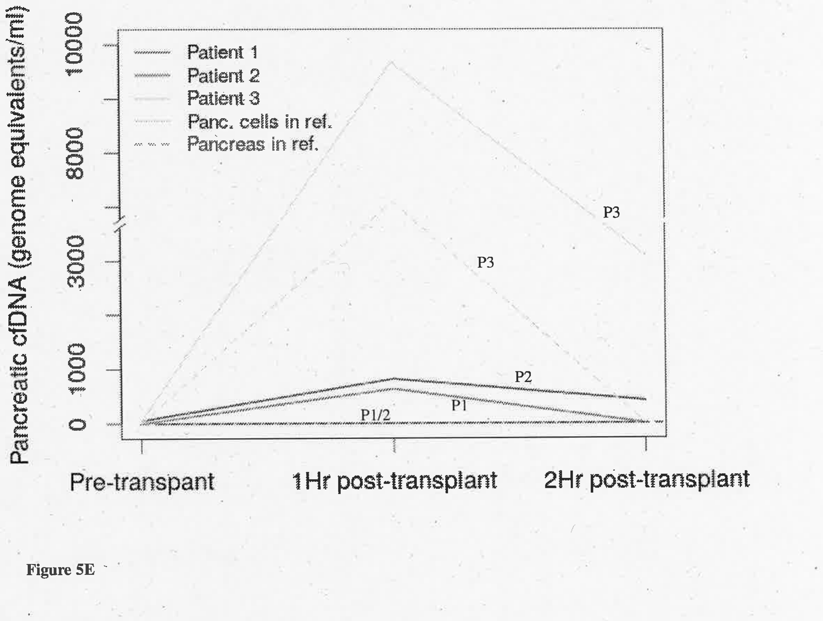

Finally, the performance of the deconvolution algorithm was tested when using a reference matrix containing either a whole pancreas methylome or pancreatic cell specific methylomes (acinar, duct and beta cells). Consistent with results from deconvolution of *in silico* mixes **(Fig. 3A-D),** a reference matrix containing cell type-specific methylomes proved highly sensitive in the detection of rare pancreas cell contribution to plasma in the transplant setting; more sensitive than when the reference matrix contained a whole pancreas methylome **(Fig. 5E).** Of great importance, two of the patients tested only showed an increase in pancreatic cfDNA with the cell type-specific methylome atlas (blue and red solid lines), whereas when analysis was performed with a reference matrix from whole pancreases no change in pancreatic cfDNA was observed at any time point (blue and red dashed lines).

### Example 5: Cellular contributors to cfDNA in sepsis

An increase in total cfDNA levels in septic patients has been previously documented, and even shown to have a prognostic value. However, it is unclear which cell types are contributing to elevated cfDNA. Therefore, the cfDNA methylation profile of 15 samples from patients with sepsis were analyzed. The main contributors to the increase in cfDNA in these patients relative to healthy levels were leukocytes (mainly neutrophils) in most cases (12/15) **(Fig. 6A-B).** In one case, a large amount of DNA of gastrointestinal origin was detected, consistent with the fact the donor had developed sepsis after an intestinal perforation during surgery to remove a tumor of intestinal origin **(Fig. 6C).** Hepatocyte cfDNA was also detected likely owing to remaining metastases in the liver. In other cases, varying amounts of hepatocyte cfDNA were detected **(Fig. 6A,** and **6D).** Importantly, the levels of hepatocyte cfDNA were strongly correlated with levels of Alanine Transferase (ALT) in circulation, a marker of hepatocyte damage **(Fig. 6E).**

### Example 6: Identifying tumor origins by cfDNA methylation

cfDNA methylation profiles of three patients with metastatic colon cancer were analyzed, all of whom presented with elevated overall levels of cfDNA compared to healthy individuals. In these cases, most of the increase in cfDNA could be defined as gastrointestinal in origin **(Fig. 7A).** Next an attempt was made to determine whether an unbiased deconvolution approach could be useful in identifying a cancer tissue of origin in patients with metastatic disease, even in the absence of an identifiable primary tumor. To this end, the cfDNA of three patients with Cancer of Unknown Primary (CUP) were analyzed. All patients had metastatic disease with no clear pathological identification of the primary source of cancer. In each case the suspected origin of the tumor, based on clinical history of the patient (e.g. a local bladder carcinoma that was previously treated is suspected as the source of new bone metastases) showed a strong signal in the deconvolution analysis **(Fig..7B).** Importantly, in all 3 cases the clinically suspected tissue gave a signal only in the cfDNA of the relevant patient. All CUP patients also had a strong stomach signal in cfDNA; it is not clear if this reflects an error of the deconvolution algorithm or true (possibly treatment-associated) intestinal damage. These findings indicate that cfDNA methylation deconvolution can be the basis of a new non-invasive approach to identify the origin of cancer.

### Example 7: Reproducibility

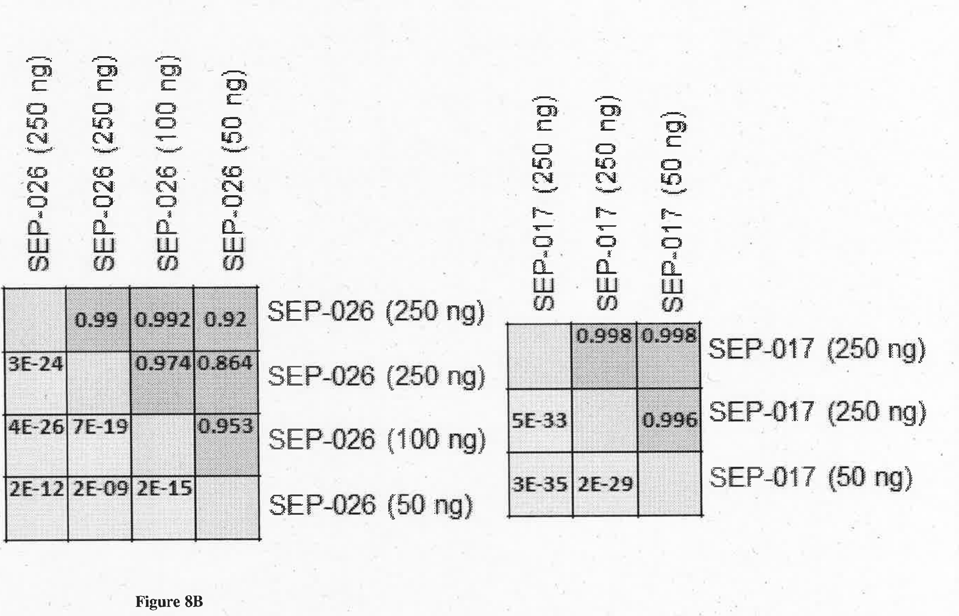

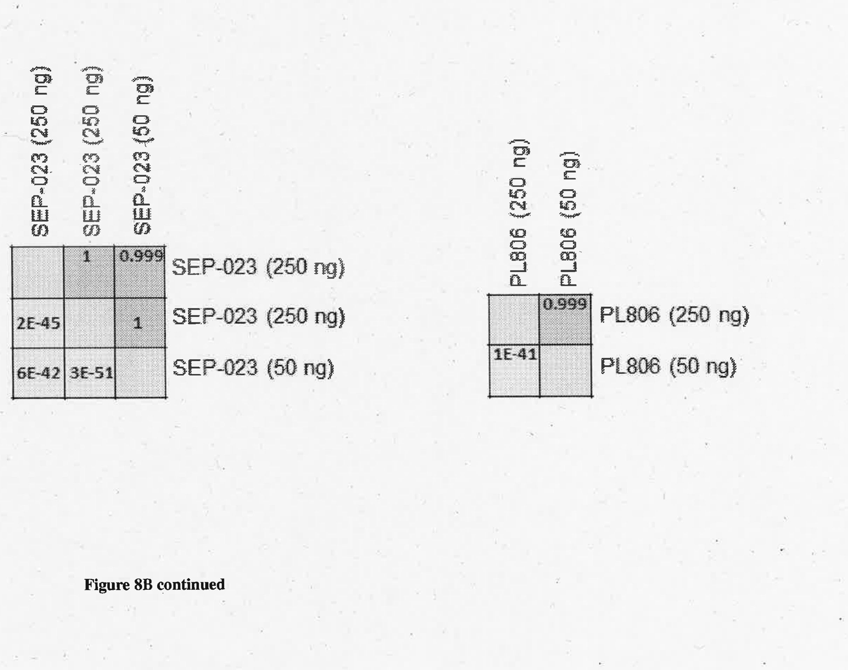

To assess the reproducibility of the deconvolution analysis, three cfDNA samples were assayed in duplicate (Fig. 8A). The predicted proportions of cell types contributing to the samples were very highly correlated (r > 0.995) (Fig. 8B). Furthermore, as limited amounts of cfDNA are often available, the possibility of using less than the 250 ng DNA recommended by Illumina for analysis with the Illumina methylation array was evaluated. The results were reproducible with as little as 50 ng of cfDNA (average r = 0.964) (Fig. 8B).

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

Further optional embodiments are set out below by way of the following clauses:

1. A method of determining the cell type or tissue of origin of cell free DNA (cfDNA) comprising:

a. providing cfDNA;

b. measuring DNA methylation of said cfDNA; and

c. assigning a cfDNA molecule from said cfDNA to a cell type or tissue of origin by comparing the methylation of said molecule to a methylome atlas of at least 1 cell type or tissue, wherein said atlas comprises at least 25 of the 100 most uniquely methylated sites and at least 25 of the 100 most uniquely unmethylated sites in each of said at least 1 cell type or tissue;

thereby determining the cell or tissue of origin of cfDNA.

2. The method of clause 1, for detecting death of a cell type or tissue in a subject, wherein said cfDNA is from said subject, and wherein determining the origin cell type or tissue of said cfDNA indicates death of said origin cell type or tissue in said subject; optionally for use in detecting a disease state in a subject in need the thereof, wherein death of said cell type or tissue is indicative of said disease state.

3. The method of clause 2, wherein said disease state is selected from organ transplantation, sepsis, and cancer; optionally wherein said method determines the cell or tissue of origin of said cancer.

4. The method of any one of clauses 1 to 3, wherein at least 50 ng of cfDNA are provided.

5. The method of any one of clauses 1 to 4, wherein said providing comprises providing a bodily fluid and isolating said cfDNA from said bodily fluid.

6. The method of any one of clauses 1 to 5, wherein said measuring DNA methylation comprises bisulfite conversion of said cfDNA; optionally wherein said measuring further comprises performing a methylome array or chip on said bisulfite converted cfDNA.

7. The method of any one of clauses 1 to 6, wherein said methylome atlas comprises only data from purified cell types; optionally wherein said purified cell types are only non-blood derived purified cell types.

8. The method of any one of clauses 1 to 7, wherein said methylome atlas comprises methylation data from at least 5 of the following 34 tissues or cell types: monocytes, B-cells, CD4+ T-cells, NK-cells, CD8+ T-cells, eosinophils, neutrophils, erythrocyte progenitors, adipocytes, neurons,
hepatocytes, lung alveolar cells, pancreatic beta cells, pancreatic acinar cells, pancreatic duct cells, vascular endothelial cells, left atrium, bladder, breast, cervix, colon, esophagus, oral cavity, kidney, prostate, rectum, stomach, thyroid, uterus, lung bronchial cells, cholangiocytes, muscle, oligodendrocytes, and ovary; optionally wherein said methylome atlas comprises data from all of said 34 tissues or cell types.

9. The method of any one clauses 1 to 8, wherein said methylome atlas comprises at least the 100 most uniquely methylated or unmethylated sites in each tissue or cell type.

10. The method of any one of clauses 1 to 9, wherein said methylome atlas further comprises any CpG sites within at least 150 base pairs upstream and downstream of said most uniquely methylated and most uniquely unmethylated sites in each tissue or cell type; optionally wherein said CpG sites within at least 150 base pairs upstream and downstream are selected from Tables 1 and 2.

11. The method of any one of clauses 1 to 10, wherein said methylome atlas further comprises at least one of the 500 CpG sites that best differentiate between the most similar pairs of tissues and cell types; optionally wherein said 500 CpG sites that best differentiate between the most similar pairs of tissues and cell types are selected from Table 3.

12. The method of any one of clauses 1 to 11, wherein said most uniquely methylated sites are selected from Table 1 and wherein said most uniquely hypomethylated sites are selected from Table 2.

13. The method of any one of clauses 1 to 12, wherein cfDNA of said tissue or cell type comprises as little 1 % of all of said cfDNA.

14. A method of constructing a methylome atlas, comprising:

    a. providing DNA methylation data from at least 5 tissues and/or cell types; b. selecting at least the top 100 uniquely hyper-methylated and at least the top 100 uniquely hypomethylated CpG sites per a tissue and/or cell type as compared to the other tissues and/or cell types; and .

    c. combining said uniquely hyper-methylated and uniquely hypo-methylated sites for all said tissues and/or cell types;

thereby constructing a methylome atlas.

15. The method of clause 14, further comprising:

d. selecting all neighboring CpG sites within at least 150 base pairs upstream and/or downstream of each of said uniquely hyper-methylated and uniquely hypomethylated CpG site; and

e. combining said neighboring CpG sites with said uniquely hyper-methylated and uniquely hypomethylated CpG sites.

16. The method of clause 14 or 15, further comprising:

f. selecting at least the top 100 CpG sites that best differentiate between the most similar pair of tissues and/or cell types; and

g. combining said CpG sites that best differentiate with said uniquely hyper- methylated and uniquely hypo-methylated CpG sites.

17. A computerized method of determining the cell type or tissue of origin of cell free DNA (cfDNA) comprising:
using at least one hardware processor to:

a receive a methylation sequencing data acquired from a cfDNA sample and a methylome atlas, wherein the methylome atlas comprises (i) a first set of uniquely methylated sites, (ii) a plurality of neighboring methylated sites, wherein at least some of the plurality of neighboring methylated sites are within 500 base units to each element of the first set of uniquely methylated sites, and (iii) a second set of uniquely methylated sites comprising more than double the number of sites of the first set, and comprising less than half the number of tissue types in each comparison as the first set;

b. compare the methylation sequencing data with a methylome atlas to determine a set of candidate tissues, wherein the comparing comprises identifying at least some elements of the first set of uniquely methylated sites in both the methylation sequencing data and the methylome atlas;

c. compare the plurality of neighboring methylated sites of the methylation sequencing data with the atlas entries of the set of candidate tissues to determine a first subset of candidate tissues;

d. compare the second set of uniquely methylated sites of the methylome atlas within the methylation sequencing data to determine a probability ranking of candidate tissues; and

e. assign at least one of the highest-ranking tissues of the probability ranking of candidate tissues to the cfDNA sample.

18. The method of clause 17, wherein the first set comprises between 25 and 100 most uniquely methylated sites.

19. The method of clause 17 or 18, wherein the first set comprises a plurality of most uniquely methylated sites and wherein the plurality of neighboring methylated sites comprises any CpG sites within between 150 and 500 base pairs upstream and downstream of said most uniquely methylated sites in each tissue or cell type.

20. The method of any one of clauses 17 to 19, wherein the second set comprises between 100 and 500 most uniquely methylated sites.

21. The method of any one of clauses 17 to 20, wherein the second set of uniquely methylated sites compares a plurality of specific pairs or triplets of similar tissue types.

22. The method of any one of clauses 17 to 21, wherein the first set of uniquely methylated sites are uniquely methylated as compared to all cell types and tissues of the atlas, and wherein said second set of uniquely methylated sites are uniquely methylated in one cell type or tissue as compared to a second most similar cell type or tissue.

23. The method of any one of clauses 17 to 22, wherein the comparing comprises using a latent probabilistic model.

24. A computer program product for determining the cell or tissue of origin of cell free DNA (cfDNA), comprising a non-transitory computer-readable storage medium having program code embodied thereon, the program code executable by at least one hardware processor to

a. measure or access measurements of DNA methylation of cfDNA;

b. assign a cfDNA molecule from said cfDNA to a cell type or tissue of origin by comparing the methylation of said molecule to a methylome atlas of at least 5 cell types or tissues, wherein said atlas comprises at least 25 of the 100 most uniquely methylated sites and at least 25 of the 100 most uniquely unmethylated sites in each of said 5 cell types or tissues; and

c. provide an output regarding the cell or tissue of origin of cfDNA.

25. A computer program product for determining the cell or tissue of origin of cell free DNA (cfDNA), comprising a non-transitory computer-readable storage medium having program code embodied thereon, the program code executable by at least one hardware processor to:

a. receive a methylation sequencing data acquired from a cfDNA sample and a methylome atlas, wherein the methylome atlas comprises (i) a first set of uniquely methylated sites, (ii) a plurality of neighboring methylated sites, wherein at least some of the plurality of neighboring methylated sites are within 500 base units to each element of the first set of uniquely methylated sites, and (iii) a second set of uniquely methylated sites comprising more than double the number of sites of the first set, and comprising less than half the number of tissue types in each comparison as the first set;

b. compare the methylation sequencing data with a methylome atlas to determine a set of candidate tissues, wherein the comparing comprises identifying at least some elements of the first set of uniquely methylated sites in both the methylation sequencing data and the methylome atlas;

c. compare the plurality of neighboring methylated sites of the methylation sequencing data with the atlas entries of the set of candidate tissues to determine a first subset of candidate tissues;

d. compare the second set of uniquely methylated sites of the methylome atlas within the methylation sequencing data to determine a probability ranking of candidate tissues; and

e. assign at least one of the highest-ranking tissues of the probability ranking of candidate tissues to the cfDNA sample.

26. The computer program product of clause 25, wherein the first set comprises between 25 and 100 most uniquely methylated sites.

27. The computer program product of clause 25 or 26, wherein the first set comprises a plurality of most uniquely methylated sites and wherein the plurality of neighboring methylated sites comprises any CpG sites within between 150 and 500 base pairs upstream and downstream of said most uniquely methylated sites in each tissue or cell type.

28. The computer program product of any one of clauses 25 to 27, wherein the second set comprises between 100 and 500 most uniquely methylated sites.

29. The computer program product of any one of clauses 25 to 28, wherein the second set of uniquely methylated sites compares a plurality of specific pairs or triplets of similar tissue types.

30. The computer program product of any one of clauses 25 to 29, wherein the first set of uniquely methylated sites are uniquely methylated as compared to all cell types and tissues of the atlas, and wherein said second set of uniquely methylated sites are uniquely methylated in one cell type or tissue as compared to a second most similar cell type or tissue.

31. The computer program product of any one of clauses 25 to 30, wherein the comparing comprises using a latent probabilistic model.

32. A computerized system for determining the cell or tissue of origin of cell free DNA (cfDNA), comprising:

at least one hardware processor; and

a non-transitory computer-readable storage medium having program code embodied thereon, the program code executable by the at least one hardware processor to:

a. receive a methylation sequencing data acquired from a cfDNA sample and a methylome atlas, wherein the methylome atlas comprises (i) a first set of uniquely methylated sites, (ii) a plurality of neighboring methylated sites, wherein at least some of the plurality of neighboring methylated sites are within 500 base units to each element of the first set of uniquely methylated sites, and (iii) a second set of uniquely methylated sites comprising more than double the number of sites of the first set, and comprising less than half the number of tissue types in each comparison as the first set;

b. compare the methylation sequencing data with a methylome atlas to determine a set of candidate tissues, wherein the comparing comprises identifying at least some elements of the first set of uniquely methylated sites in both the methylation sequencing data and the methylome atlas;

c. compare the plurality of neighboring methylated sites of the methylation sequencing data with the atlas entries of the set of candidate tissues to determine a first subset of candidate tissues;

d. compare the second set of uniquely methylated sites of the methylome atlas within the methylation sequencing data to determine a probability ranking of candidate tissues; and

e. assign at least one of the highest-ranking tissues of the probability ranking of candidate tissues to the cfDNA sample.

33. The computerized system product of clause 32, wherein the first set comprises between 25 and 100 most uniquely methylated sites.

34. The computerized system product of clause 32 or 33, wherein the first set comprises a plurality of most uniquely methylated sites and wherein the plurality of neighboring methylated sites comprises any CpG sites within between 150 and 500 base pairs upstream and downstream of said most uniquely methylated sites in each tissue or cell type.

35. The computerized system product of any one of clauses 32 to 34, wherein the second set comprises between 100 and 500 most uniquely methylated sites.

36. The computerized system product of any one of clauses 32 to 35, wherein the second set of uniquely methylated sites compares a plurality of specific pairs of tissue types.

37.The computerized system product of any one of clauses 32 to 36, wherein the first set of uniquely methylated sites are uniquely methylated as compared to all cell types and tissues of the atlas, and wherein said second set of uniquely methylated sites are uniquely methylated in one cell type or tissue as compared to a second most similar cell type or tissue.

**Claims**

1. A method for determining a cell type or tissue of origin of cell-free DNA (cfDNA), comprising:

(a) obtaining cfDNA from a biological sample;
(b) determining DNA methylation values at a plurality of CpG sites in the cfDNA; and
(c) determining a relative contribution of a plurality of cell types or tissues to the cfDNA by comparing the DNA methylation values with a methylome atlas,

wherein the methylome atlas comprises DNA methylation data derived from purified cell populations.

2. The method of claim 1, wherein the purified cell populations are obtained from tissues by cell separation.

3. The method of claim 1 or 2, wherein the methylome atlas comprises DNA methylation data from at least 5 different cell types or tissues.

4. The method of any one of claims 1-3, wherein the purified cell populations comprise non-blood-derived cell populations.

5. The method of any one of claims 1-4, wherein a contribution of a cell type or tissue representing 1% or less of total cfDNA is detected.

6. The method of any one of claims 1-5, wherein determining the relative contribution comprises solving a deconvolution problem.

7. The method of claim 6, wherein the deconvolution comprises non-negative least squares analysis.

8. The method of any one of claims 1-7, wherein determining DNA methylation values comprises bisulfite conversion of the cfDNA.

9. The method of claim 8, wherein determining DNA methylation values further comprises sequencing or array-based analysis.

10. The method of any one of claims 1-9, wherein the biological sample comprises blood or plasma.

11. The method of any one of claims 1-10, wherein the plurality of CpG sites comprises CpG sites selected based on differential methylation between the plurality of cell types or tissues.

12. The method of claim 11, wherein the CpG sites are selected such that methylation levels distinguish one cell type or tissue from at least one other cell type or tissue.

13. The method of any one of claims 1-12, wherein determining the cell type or tissue of origin indicates cell death in said cell type or tissue.

14. The method of claim 13, wherein the method is used for detecting or monitoring a disease selected from cancer, organ transplantation, or sepsis.

15. A computer-implemented method comprising:

   (a) receiving DNA methylation data from a cfDNA sample;
   (b) comparing the DNA methylation data with a methylome atlas comprising DNA methylation data derived from purified cell populations; and
   (c) determining a relative contribution of a plurality of cell types or tissues to the cfDNA based on the comparison.

Figure 1A

Figure 1B

Figure 1C

Figure 1C Continued

Figure 1D

Figure 1E

Figure 2A

**Figure 2B**

**Figure 2B continued_1**

Sensitivity (at 0.1%)

Sensitivity (at 0.1%)
300bp blocks

Sensitivity (at 0.1%)
300bp blocks + 500 pairwise CpGs

Figure 2B continued _2

Sensitivity (at 1%)

Sensitivity (at 1%)
300bp blocks

Sensitivity (at 1%)
300bp blocks + 500 pairwise CpGs

Figure 2C

Figure 3A

EP 4 741 515 A2

Figure 3A continued

Figure 3B

Figure 3B continued

Figure 3C

Figure 3D

Figure 3E

**Figure 4A**

**Figure 4B**

Figure 4C

**Figure 5A**

**Figure 5B**

**Figure 5C**

**Figure 5D**

Figure 5E

**Figure 6A**

**Figure 6B**

**Sepsis** (SEP-031)

- Monocytes
- Granulocytes
- Endothelial
- Panc. acinar

**Figure 6C**

**Sepsis/Colon cancer** (SEP-002)

- Monocytes
- Granulocytes
- Colon
- Rectum
- Stomach

**Figure 6D**

**Sepsis/Liver damage** (SEP-017)

- Monocytes
- Lymphocytes
- Granulocytes
- Erythro. prog
- Hepatocytes
- Neurons
- Panc. acinar
- Rectum
- Stomach

Figure 6E

Figure 7A

**Colorectal cancer cfDNA**

Figure 7B

Cancer of Unknown Primary cfDNA

| | Presumed origin | Metastases | |
|---|---|---|---|
| Patient 1 | Lungs | Brain, lungs, bones | |
| Patient 2 | Prostate | Bones | |
| Patient 3 | Bladder | Bones, lungs | |

**Figure 8A**

**Figure 8A continued_1**

**Figure 8A continued_2**

**Figure 8A continued_3**

|  | SEP-017 (250 ng) | SEP-017 (250 ng) | SEP-017 (50 ng) |
|---|---|---|---|
| SEP-017 (50 ng) | 0.998 | 0.998 |  |
| SEP-017 (250 ng) | 5E-33 | 0.996 |  |
| SEP-017 (250 ng) | 3E-35 | 2E-29 |  |

|  | SEP-026 (250 ng) | SEP-026 (250 ng) | SEP-026 (100 ng) | SEP-026 (50 ng) |
|---|---|---|---|---|
| SEP-026 (50 ng) | 0.92 | 0.864 | 0.953 |  |
| SEP-026 (100 ng) | 0.992 | 0.974 |  | 2E-15 |
| SEP-026 (250 ng) | 0.99 | 7E-19 |  | 2E-09 |
| SEP-026 (250 ng) | 3E-24 | 4E-26 |  | 2E-12 |

Figure 8B

| | SEP-023 (250 ng) | SEP-023 (250 ng) | SEP-023 (50 ng) | |
|---|---|---|---|---|
| | | 1 | 0.999 | SEP-023 (250 ng) |
| | 2E-45 | | 1 | SEP-023 (250 ng) |
| | 6E-42 | 3E-51 | | SEP-023 (50 ng) |

| | PL806 (250 ng) | PL806 (50 ng) | |
|---|---|---|---|
| | | 0.999 | PL806 (250 ng) |
| | 1E-41 | | PL806 (50 ng) |

Figure 8B continued

**EP 4 741 515 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62631791
- US 62661179
- US 4666828 A
- US 4683202 A
- US 4801531 A
- US 5192659 A
- US 5272057 A

### Non-patent literature cited in the description

- **SAMBROOK et al.** *Molecular Cloning: A laboratory Manual*, 1989
- *Current Protocols in Molecular Biology*, 1994
- **AUSUBEL et al.** Current Protocols in Molecular Biology. John Wiley and Sons, 1989
- **PERBAL**. A Practical Guide to Molecular Cloning. John Wiley & Sons, 1988
- **WATSON et al.** Recombinant DNA. *Scientific American*
- Genome Analysis: A Laboratory Manual Series. Cold Spring Harbor Laboratory Press, 1998, vol. 1-4
- Cell Biology: A Laboratory Handbook. 1994
- **FRESHNEY**. Culture of Animal Cells - A Manual of Basic Technique. Wiley-Liss, 1994
- Current Protocols in Immunology. 1994
- Basic and Clinical Immunology. Appleton & Lange, Norwalk, 1994
- Strategies for Protein Purification and Characterization - A Laboratory Course Manual. CSHL. Press, 1996